(19) Europäisches Patentamt
European Patent Office
Office européen des brevets

(11) EP 2 391 711 B1

(12) **EUROPEAN PATENT SPECIFICATION**

(45) Date of publication and mention of the grant of the patent:
**08.04.2015 Bulletin 2015/15**

(21) Application number: **10736548.8**

(22) Date of filing: **01.02.2010**

(51) Int Cl.:
***C12N 5/00*** *(2006.01)* ***C12N 5/07*** *(2010.01)*
***G01N 33/50*** *(2006.01)*

(86) International application number:
**PCT/US2010/022781**

(87) International publication number:
**WO 2010/088633 (05.08.2010 Gazette 2010/31)**

(54) **NOVEL CELL LINES AND METHODS**

NEUE ZELLLINIEN UND VERFAHREN

NOUVELLES LIGNÉES CELLULAIRES ET PROCÉDÉS

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority:
**02.02.2009 US 149311 P**
**02.02.2009 US 149318 P**
**02.02.2009 US 149321 P**
**31.07.2009 US 230536 P**
**19.08.2009 US 235181 P**
**02.02.2009 US 149324 P**

(43) Date of publication of application:
**07.12.2011 Bulletin 2011/49**

(60) Divisional application:
**15156204.8**

(73) Proprietor: **Chromocell Corporation**
**North Brunswick, NJ 08902 (US)**

(72) Inventors:
- **SHEKDAR, Kambiz**
**New York, NY 10010 (US)**
- **SAWCHUK, Dennis**
**Fanwood, NJ 07023 (US)**
- **SHAH, Purvi, Manoj**
**North Brunswick, NJ 08902 (US)**

(74) Representative: **Jennings, Tara Romaine**
**FRKelly**
**27 Clyde Road**
**Ballsbridge**
**Dublin 4 (IE)**

(56) References cited:
**WO-A2-2009/102569**
**US-A1- 2005 032 158**
**US-A1- 2006 147 937**
**US-A1- 2008 262 087**

- **TOYONO ET AL: "CCAAT/Enhancer-binding protein beta regulates expression of human T1R3 taste receptor gene in the bile duct carcinoma cell line, HuCCT1", BIOCHIMICA ET BIOPHYSICA ACTA . GENE STRUCTURE AND EXPRESSION, ELSEVIER, AMSTERDAM, NL, vol. 1769, no. 11-12, 1 November 2007 (2007-11-01), pages 641-648, XP022349465, ISSN: 0167-4781**

EP 2 391 711 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

## Field of the Invention

The invention relates to novel cells and cell lines, and methods for isolating them. In particular embodiments, the invention relates to cells and cell lines stably expressing complex targets. Disclosed herein are methods of making and using such cells and cell lines. The cells and cell lines provided herein are useful in identifying modulators of such complex targets.

## Background of the Invention

Currently, the industry average failure rate for drug discovery programs in pharmaceutical companies is reported to be approximately 98%. Although this includes failures at all stages of the process, the high failure rate points to a dire need for any improvements in the efficiency of the process.

One factor contributing to the high failure rate is the lack of cell lines expressing therapeutic targets for used in cell-based functional assays during drug discovery. Indisputably, research using cell-based assays, especially drug discovery research, would benefit from cells and cell lines for use in cell-based assays.

Consequently, there is a great need for rapid and effective establishment of cell based assays for more rapid discovery of new and improved drugs. Preferably, for more effective drug discovery, the assay system should provide a more physiologically relevant predictor of the effect of a modulator *in vivo.*

Beyond the need for cell-based assays is a need for improved cells for protein production, cell-based therapy and a variety of other uses.

Accordingly, there is an urgent need for cells and cell lines that express a functional protein or RNA of interest.

In the mouth, taste receptor cells (TRCs) can be found in several specialized zones that include the tongue, part of the palate, epiglottis, larynx and pharynx. On the tongue, TRCs are organized into groups of cells called taste buds. Taste buds consist of a single apical pore where microvilli of TRCs come into contact with tastants present within the oral cavity. On the tongue, taste buds are embedded in three types of specialized epidermal structures. The fungiform papillae are distributed over the anterior two-thirds of the tongue. The foliate papillae, which are well developed at birth but regress with age, are found on the sides of the posterior one-third of the tongue. Seven to nine circumvallate papillae are located far back on the posterior tongue close to the terminal sulcus. In addition to the 'classical' TRCs organized in taste buds, chemosensory cell clusters or solitary chemosensory cells are found in non-lingual epithelia in the lung and the intestine.

US 2008/262087 recites that co-expression of T1R1 and T1R3 results in a taste receptor that responds to umami taste stimuli, including monosodium glutamate. Also, it recites that co-expression of the T1R2 and T1R3 receptors results in a taste receptor that responds to sweet taste stimuli including naturally occurring and artificial sweeteners. Also, it refers to the use of hetero-oligomeric taste receptors comprising T1R1/T1R3 and T1R2/T1R3 in assays to identify compounds that respectively respond to umami taste stimuli and sweet taste stimuli. Further, it refers to cell lines that co-express a combination of T1R1 and T1R3; or T1R2 and T1R3.

WO 2009/102569 refers to cells and cell lines, and methods for making and using them, including a method for isolating a cell that expresses a sweet taste receptor.

Toyono et al, "CCAAT/Enhancer-binding protein beta regulates expression of human T1R3 taste receptor gene in the bile duct carcinoma cell line, HuCCT1", Biochemica et Biophysica Acta 1769 (2007) 641 - 648, refers to, *inter alia,* the determination of whether a population of cells expresses T1R3.

US 2006/147937 refers to the detection of mRNAs as well as other RNAs in living cells, and also refers to methods for identifying and, if desired, separating cells based on their desired characteristics.

### Sweet Taste Receptor

Sweet perception is mediated by a heteromeric G-protein coupled receptor (GPCR) composed of two subunits TASR2 (T1R2) and TASR3 (T1 R3). The receptor is named the sweet taste receptor. Both subunits of the receptor are members of the class C GPCR subfamily and possess a large N-terminal extracellular domain, often referred to as the Venus flytrap domain. The T1 R subunits can couple to the G proteins alpha transducin or alpha gustducin, through which they can activate a phospholipase C (PLC) $\beta_2$-dependent pathway to increase intracellular $Ca^{2+}$ concentration. They may also activate a cAMP-dependent pathway.

Sweet taste receptors detect a wide variety of sweet chemicals including simple carbohydrates (such as sugars), amino acids, peptides, proteins, and synthetic sweeteners. Sweet taste receptors are sensitive to both natural and artificial sweeteners. Given the wide diversity of chemical structures known to activate the receptors, multiple binding sites in the receptors have been proposed, including a site in the transmembrane region and a site on T1 R3, which serves as a shared subunit with umami taste receptors.

Sweet taste receptors have also been implicated in conditions such as obesity and diabetes, as these receptors appear to play an important role in nutrient detection and sensing. Taste receptors are expressed in nutrient detection regions of the proximal small intestine in humans, where evidence suggests that they play a role in the detection of nutrients in the intestinal lumen. There is a highly coordinated expression of sweet taste receptors and gustducin, a G-protein implicated in intracellular taste signal transduction, in this region and, more specifically, in the endocrine cells of the gut. The function of these sweet taste receptors thus may show similar ligand-mediated control as other G-protein coupled receptors, that is, they will lose their activity and or expression in the presence of high concentrations of their ligands. This would make intestinal 'taste' signaling responsive to the dynamic metabolic changes in glucose concentrations in the blood and lumen. Accordingly, sweet taste receptors and their modulation in the gut may have important roles in diet, appetite and in the treatment of various diseases, such as obesity and diabetes.

Activation of intestinal sweet taste receptors by natural sugars and artificial sweeteners also leads to increased expression of the apical glucose transporter, GLUT2, and other glucose transporters. For example, artificial sweeteners are nutritionally active, because they can signal a functional taste reception system to increase sugar absorption during a meal, a finding that may have important implications in nutrition and appetite, and thus in the potential treatment of malnutrition and eating disorders. Consistently elevated apical GLUT2 levels result in increased sugar absorption and are a characteristic of experimental diabetes and of insulin-resistant states induced by fructose and fat. Additionally, sweet taste receptor activation in neuroendocrine cells leads to the release of glucagon like peptide (GLP-1) and perhaps other modulators of digestion. Overall, sweet taste receptors in the intestine play an important role in sensing the nutritional value of luminal content and help coordinate the body's response via regulated absorption and digestion. These findings suggest that sweet taste receptors could serve as possible targets for modulators useful in treating obesity and diabetes.

Umami Taste Receptor

Savory (umami) perception is mediated by a heteromeric GPCR composed of two subunits TASR1 (T1 R1) and TASR3 (T1 R3). The receptor is named the umami taste receptor. Both subunits of the receptor are members of the class C GPCR subfamily and possess a large N-terminal extracellular domain, often referred to as the Venus flytrap domain. The T1 R subunits can be coupled to the G proteins alpha transducin or alpha gustducin, through which they can activate a phospholipase C (PLC) 2-dependent pathway to increase intracellular Ca2+ concentration. They may also activate a cAMP-dependent pathway. These receptors can detect a wide variety of savory chemicals including L-amino acids and monosodium glutamate (MSG). T1 R1 has also been shown to bind disodium 5'-inosinate (IMP) and other nucleotides, known potentiators of umami taste.

Umami taste receptors are also expressed in nutrient detection regions of the proximal small intestine in humans, where they are thought to play a role in the detection of nutrients in the intestinal lumen. There is a highly coordinated expression of umami taste receptors and gustducin, a G-protein implicated in intracellular taste signal transduction in this region and in specific, in neuroendocrine cells. Activation of intestinal umami taste receptors by amino acids leads to modulation of the apical oligopetide transporter PepT1. Overall, umami taste receptors in the intestine play an important role in sensing the nutritional value of luminal content and help coordinate the body's response via regulated absorption and digestion. These findings suggest that umami taste receptors could serve as possible targets for modulators useful in treating obesity and diabetes.

Bitter Taste Receptor

Bitter receptors are G protein coupled receptors (GPCRs) expressed at the surface of taste receptor cells and are coupled to secondary messenger pathways. TAS2R receptors can be coupled to transducin (*e.g.,* GNAT1, GNAT2, and guanine nucleotide-binding protein G(t)) or gustducin (e.g., GNAT3 guanine nucleotide binding protein and a transducin 3), for example, through which they can activate both phospodiesterases and a phospholipase C (PLC)β2-dependent pathway to increase intracellular $Ca^{2+}$ concentration. TAS2R receptors can also be coupled to human GNA15 (guanine nucleotide binding protein (G protein) α15 (Gq class; synonym GNA16) and mouse Gα15, and their chimera proteins Gα15-GNA15 (also known as Gα15-Gα16).

Human bitter taste is mediated by about 25 members of the human TAS2 receptor (hTAS2R) gene family. In addition to their role in taste, bitter receptors are also important in a series of physiological contexts. For example, taste receptor agonists elicit a secretory response in enteroendocrine cells in vitro and in animals in vivo, and induce neuronal activation. Therefore, all of the bitter receptor family members are important clinical targets for managing a variety of conditions associated with detection of bitter tastants.

The discovery of new and improved compounds that specifically target taste receptors (*e.g.*, sweet taste receptors, umami taste receptors, and bitter taste receptors) and thus modulate their activity has been hampered by the lack of robust, physiologically relevant, cell-based systems and more especially such systems that are amenable to high through-put formats for identifying and testing taste receptor modulators (*e.g.,* sweet taste receptor modulators, umami taste receptor modulators, and bitter taste receptor modulators). Such cell-based systems are preferred for drug discovery and validation because they provide a functional assay for a compound as opposed to cell-free systems, which only provide a binding assay. Moreover, cell-based systems have the advantage of simultaneously testing cytotoxicity. Ideally, cell-based systems should also stably and constitutively express the target protein. It is also desirable for a cell-based system to be reproducible. The present invention addresses these problems in various embodiments in the context of providing cells and cell lines that stably express taste receptors, *e.g.,* sweet taste receptors, bitter taste receptors, or umami taste receptors, in a physiologically relevant form and in methods of using those cells and cell lines to identify modulators of taste receptors, *e.g.,* sweet taste receptors, bitter taste receptors, or umami taste receptors.

**Summary of the Invention**

The invention provides methods as specified in claims 1 and 3. Disclosed herein is a cell that expresses a heterodimeric protein of interest from an introduced nucleic acid encoding at least one of the subunits of the heterodimeric protein of interest, said cell being characterized in that it produces the heterodimeric protein of interest in a form suitable for use in a functional assay, wherein said protein of interest does not comprise a protein tag, or said protein is produced in that form consistently and reproducibly such that the cell has a Z' factor of at least 0.4 in the functional assay, or said cell is cultured in the absence of selective pressure, or any combinations thereof.

Also disclosed herein is a cell that expresses a heterodimeric protein of interest, wherein the cell is engineered to activate transcription of an endogenous nucleic acid encoding at least one of the subunits of the heterodimeric protein of interest, said cell being characterized in that it produces the heterodimeric protein of interest in a form suitable for use in a functional assay, wherein said protein of interest does not comprise a protein tag, or said protein is produced in that form consistently and reproducibly such that the cell has a Z' factor of at least 0.4 in the functional assay, or said cell is cultured in the absence of selective pressure, or any combinations thereof.

Also disclosed herein is a cell that expresses a heterodimeric protein of interest from an introduced nucleic acid encoding at least one of the subunits of the heterodimeric protein of interest, said cell being characterized in that it produces the protein of interest in a form that is or is capable of becoming biologically active, wherein the cell is cultured in the absence of selective pressure.

Also disclosed herein is a cell that expresses a heterodimeric protein of interest wherein the cell is engineered to activate transcription of an endogenous nucleic acid encoding at least one of the subunits of the heterodimeric protein of interest, said cell being characterized in that it produces the protein of interest in a form that is or is capable of becoming biologically active, wherein the cell is cultured in the absence of selective pressure.

Optionally, the nucleic acid encoding the second subunit of the heterodimeric protein of interest is endogenous. Further optionally, the nucleic acid encoding the second subunit of the heterodimeric protein of interest is introduced. Still further optionally, the protein of interest does not comprise a protein tag.

Optionally, the heterodimeric protein of interest is selected from the group consisting of: an ion channel, a G protein coupled receptor (GPCR), tyrosine receptor kinase, cytokine receptor, nuclear steroid hormone receptor, antibody, biologic, and immunological receptor. Optionally, the heterodimeric protein is an antibody or a biologic. Optionally, the heterodimeric protein of interest is selected from the group consisting of: a sweet taste receptor and an umami taste receptor. Optionally, the heterodimeric protein of interest has no known ligand. Further optionally, there is no known assay to detect functional expression of the heterodimeric protein of interest.

Optionally, the heterodimeric protein of interest is not expressed in a cell of the same type. Optionally the cell is a mammalian cell.

Optionally, the cell is further characterized in that it has an additional desired property selected from the group consisting of: a signal to noise ratio greater than 1, being stable over time, growth without selective pressure without losing expression, physiological EC50 values, and physiological IC50 values. Optionally, the heterodimeric protein of interest is produced in a form consistently and reproducibly for a period of time selected from: at least one week, at least two weeks, at least three weeks, at least one month, at least two months, at least three months at least four months, at least five months, at least six months, at least seven months, at least eight months, and at least nine months. Optionally , the functional assay is selected from the group consisting of : a cell-based assay, a fluorescent cell-based assay, a high throughput screening assay, a reporter cell-based assay, a G protein mediated cell-based assay, and a calcium flux cell-based assay. Optionally, the functional assay is a membrane potential assay, ELISA, mass spectrometry, biochemical characterization of the protein of interest, a cell growth assay, a viability assay, a cell specification assay, or capacity for protein production. Further optionally, the cell is suitable for utilization in a cell based high throughput screening.

Optionally, the selective pressure is an antibiotic. Further optionally, the cell expresses the heterodimeric protein in the absence of selective pressure for at least 15 days, 30 days, 45 days, 60 days, 75 days, 100 days, 120 days, or 150 days.

Also disclosed herein is a cell that expresses a heteromultimeric protein of interest wherein said heteromultimeric protein comprises at least 3 subunits, wherein at least one subunit of the heteromultimeric protein interest is encoded by an introduced nucleic acid, said cell being characterized in that it produces the heteromultimeric protein of interest in a form suitable for use in a functional assay, wherein said protein of interest does not comprise a protein tag, or said protein produced in that form consistently and reproducibly such that the cell has a Z' factor of at least 0.4 in the functional assay, or said cell is cultured in the absence of selective pressure, or any combinations thereof.

Also disclosed herein is a cell that expresses a heteromultimeric protein of interest wherein said heteromultimeric protein comprises at least 3 subunits, wherein the cell is engineered to activate transcription of an endogenous nucleic acid encoding at least one of the subunits of the heteromultimeric protein of interest, said cell being characterized in that it produces the heteromultimeric protein of interest in a form suitable for use in a functional assay, wherein said protein of interest does not comprise a protein tag, or said protein produced in that form consistently and reproducibly such that the cell has a Z' factor of at least 0.4 in the functional assay, or said cell is cultured in the absence of selective pressure, or any combinations thereof.

Also disclosed herein is a cell that expresses a heteromultimeric protein of interest wherein said heteromultimeric protein comprises at least 3 subunits, wherein at least one subunit of the heteromultimeric protein interest is encoded by an introduced nucleic acid, said cell being characterized in that it produces the protein of interest in a form that is or is capable of becoming biologically active.

Also disclosed herein is a cell that expresses a heteromultimeric protein of interest wherein said heteromultimeric protein comprises at least 3 subunits, wherein the cell is engineered to activate transcription of an endogenous nucleic acid encoding at least one of the subunits of the heteromultimeric protein of interest, said cell being characterized in that it produces the protein of interest in a form that is or is capable of becoming biologically active.

Optionally, the nucleic acid encoding at least one of the subunits of the heteromultimeric protein of interest is endogenous.

Optionally, the nucleic acid encoding at least one of the subunits of the heteromultimeric protein of interest is introduced.

Optionally, the protein of interest does not comprise a protein tag.

Optionally, the heteromultimeric protein of interest is selected from the group consisting of: an ion channel, a G protein coupled receptor (GPCR), tyrosine receptor kinase, cytokine receptor, nuclear steroid hormone receptor and immunological receptor. Optionally, the heteromultimeric protein of interest is an antibody or a biologic. Further optionally, the heteromultimeric protein of interest is selected from the group consisting of: GABA, ENaC and NaV. Optionally, the heteromultimeric protein of interest has no known ligand. Further optionally, there is no known assay to detect functional expression of said heteromultimeric protein of interest.

Optionally, the heteromultimeric protein of interest is not expressed in a cell of the same type. Further optionally, the cell is a mammalian cell.

Optionally, the cell is further characterized in that it has an additional desired property selected from the group consisting of: a signal to noise ratio greater than 1, being stable over time, growth without selective pressure without losing expression, physiological EC50 values, and physiological IC50 values. Further optionally, the heteromultimeric protein of interest is produced in a form consistently and reproducibly for a period of time selected from: at least one week, at least two weeks, at least three weeks, at least one month, at least two months, at least three months at least four months, at least five months, at least six months, at least seven months, at least eight months, and at least nine months.

Optionally, the functional assay is selected from the group consisting of: a cell-based assay, a fluorescent cell-based assay, a high throughput screening assay, a reporter cell-based assay, a G protein mediated cell-based assay, and a calcium flux cell-based assay. Optionally, the functional assay is a membrane potential assay, ELISA, mass spectrometry, biochemical characterization of the protein of interest, a cell growth assay, a viability assay, a cell specification assay, or capacity for protein production. Further optionally, the cell expressing the heteromultimeric protein is suitable for utilization in a cell based high throughput screening.

Optionally, the cells expressing the heteromultimeric protein are cultured in the absence of selective pressure. Optionally, the selective pressure is an antibiotic. Further optionally, the cell according to claim 35 or 36, wherein the cell expresses the heteromultimeric protein in the absence of selective pressure for at least 15 days, 30 days, 45 days, 60 days, 75 days, 100 days, 120 days or 150 days.

Also disclosed herein is a cell that expresses two or more proteins of interest from an introduced nucleic acid encoding at least one of the proteins of interest, said cell being characterized in that it produces the proteins of interest in a form suitable for use in a functional assay, wherein said proteins of interest do not comprise a protein tag, or said proteins are produced in that form consistently and reproducibly such that the cell has a Z' factor of at least 0.4 in the functional assay, or said cell is cultured in the absence of selective pressure, or any combinations thereof.

Also disclosed herein is a cell that expresses two or more proteins of interest, wherein the cell is engineered to activate transcription of an endogenous nucleic acid encoding at least one of the proteins of interest, said cell being characterized in that it produces the proteins of interest in a form suitable for use in a functional assay, wherein said proteins of interest do not comprise a protein tag, or said proteins are produced in that form consistently and reproducibly such that the cell has a Z' factor of at least 0.4 in the functional assay, or said cell is cultured in the absence of selective pressure, or any combinations thereof.

Also disclosed herein is a cell that expresses two or more proteins of interest from an introduced nucleic acid encoding at least one of the proteins of interest, said cell being characterized in that it produces the proteins of interest in a form that is or is capable of becoming biologically active.

Also disclosed herein is a cell that expresses two or more proteins of interest, wherein the cell is engineered to activate transcription of an endogenous nucleic acid encoding at least one of the proteins of interest, said cell being characterized in that it produces the proteins of interest in a form that is or is capable of becoming biologically active.

Optionally, at least one of the two or more proteins of interest is a dimeric protein. Further optionally, the dimeric protein of interest is a homodimeric protein. Still further optionally, the dimeric protein of interest is a heterodimeric protein. Optionally, at least one of the two or more proteins of interest is a multimeric protein. Further optionally, the multimeric protein of interest is a homomultimeric protein. Still further optionally the multimeric protein of interest is a heteromultimeric protein.

Optionally, one of the two or mor proteins of interest is encoded by an endogenous nucleic acid. Further optionally, one of the two or more proteins of interest is encoded by an introduced nucleic acid. Still further optionally, the proteins of interest do not comprise a protein tag.

Optionally, one of the two or more proteins of interest is selected from the group consisting of: an ion channel, a G protein coupled receptor (GPCR), tyrosine receptor kinase, cytokine receptor, nuclear steroid hormone receptor and immunological receptor. Optionally, the two or more proteins of interest are independently antibodies or biologics. Further optionally one of the proteins of interest has no known ligand. Still further optionally, there is no known assay to detect functional expression of the two or more protein of interest.

Optionally, one of the two or more proteins of interest is not expressed in a cell of the same type. Optionally, the cell expressing the two or more proteins is a mammalian cell.

Optionally, the cell expressing the two or more proteins is further characterized in that it has an additional desired property selected from the group consisting of: a signal to noise ratio greater than 1, being stable over time, growth without selective pressure without losing expression, physiological EC50 values, and physiological IC50 values.

Optionally, the two or more proteins of interest are produced in a form consistently and reproducibly for a period of time selected from: at least one week, at least two weeks, at least three weeks, at least one month, at least two months, at least three months at least four months, at least five months, at least six months, at least seven months, at least eight months, and at least nine months.

Optionally, the functional assay is selected from the group consisting of: a cell-based assay, a fluorescent cell-based assay, a high throughput screening assay, a reporter cell-based assay, a G protein mediated cell-based assay, and a calcium flux cell-based assay. Optionally, the functional assay is a membrane potential assay, ELISA, mass spectrometry, biochemical characterization of the protein of interest, a cell growth assay, a viability assay, a cell specification assay, or capacity for protein production. Optionally, the cell expressing the two or more proteins is suitable for utilization in a cell based high throughput screening.

Optionally, the cell expressing the two or more proteins is cultured in the absence of selective pressure. Optionally, the selective pressure is an antibiotic. Optionally, the cell expresses the two or more proteins in the absence of selective pressure for at least 15 days, 30 days, 45 days, 60 days, 75 days, 100 days, 120 days, or 150 days.

Also disclosed herein is a cell that expresses at least one RNA of interest, wherein said RNA of interest is encoded by an introduced nucleic acid, said cell being characterized in that it produces the at least one RNA of interest in a form suitable for use in a functional assay, wherein said RNA of interest do not comprise a tag, or said RNA is produced in that form consistently and reproducibly such that the cell has a Z' factor of at least 0.4 in the functional assay, or said cell is cultured in the absence of selective pressure, or any combinations thereof.

Optionally, the invention provides a cell that expresses at least one RNA of interest, wherein the cell is engineered to activate transcription of an endogenous nucleic acid encoding the at least one RNA of interest, said cell being characterized in that it produces the at least one RNA of interest in a form suitable for use in a functional assay, wherein said RNA of interest do not comprise a tag, or said RNA is produced in that form consistently and reproducibly such that the cell has a Z' factor of at least 0.4 in the functional assay or said cell is cultured in the absence of selective pressure, or any combinations thereof.

Optionally, the cell expresses at least two RNAs of interest. Further optionally, the cell expresses at least three RNAs of interest. Optionally, the cell further expresses a RNA encoded by an introduced nucleic acid. Optionally, the RNA of interest is selected from the group consisting of : a RNA encoding an ion channel, a RNA encoding a G protein coupled receptor (GPCR), a RNA encoding a tyrosine receptor kinase, a RNA encoding a cytokine receptor, a RNA encoding a nuclear steroid hormone receptor and a RNA encoding an immunological receptor. Further optionally, the RNA of interest is a RNA encoding an antibody or a RNA encoding a biologic.

Optionally, the RNA of interest is not expressed in a cell of the same type. Optionally, the cell expressing the RNA of interest is a mammalian cell.

Optionally, the cell expressing the RNA of interest is further characterized in that it has an additional desired property selected from the group consisting of: a signal to noise ratio greater than 1, being stable over time, growth without selective pressure without losing expression, physiological EC50 values, and physiological IC50 values. Optionally, the RNA of interest is produced in a form consistently and reproducibly for a period of time selected from: at least one week, at least two weeks, at least three weeks, at least one month, at least two months, at least three months at least four months, at least five months, at least six months, at least seven months, at least eight months, and at least nine months.

Optionally, the functional assay is selected from the group consisting of: a cell-based assay, a fluorescent cell-based assay, a high throughput screening assay, a reporter cell-based assay, a G protein mediated cell-based assay, and a calcium flux cell-based assay. Further optionally, the functional assay is a membrane potential assay, ELISA, mass spectrometry, biochemical characterization of the protein of interest, a cell growth assay, a viability assay, a cell specification assay, or capacity for protein production.

Optionally, the cell expressing the RNA of interest is suitable for utilization in a cell based high throughput screening.

Also disclosed herein is a cell line produced from a cell described herein.

Also disclosed herein is a method for producing a cell that expresses a protein of interest, wherein the cell has at least one desired property that is consistent over time, comprising the steps of:

a) providing a plurality of cells that express mRNA encoding the protein of interest;
b) dispersing cells individually into individual culture vessels, thereby providing a plurality of separate cell cultures
c) culturing the cells under a set of desired culture conditions using automated cell culture methods characterized in that the conditions are substantially identical for each of the separate cell cultures, during which culturing the number of cells per separate cell culture is normalized, and wherein the separate cultures are passaged on the same schedule;
d) assaying the separate cell cultures for at least one desired characteristic of the protein of interest at least twice; and
e) identifying a separate cell culture that has the desired characteristic in both assays. Optionally, the cell produced by the method described herein is a differentiated cell. Optionally, the cell produced by the method described herein is a dedifferentiated cell. Optionally, the dedifferentiated cell is a stem cell selected from the group consisting of a multipotent stem cell, a pluripotent stem cell, an omnipotent stem cell, an induced pluripotent stem cell, an embryonic stem cell, a cancer stem cell, an organ-specific stem cell, and a tissue-specific stem cell.

Also disclosed herein is a method for producing a cell that expresses a protein of interest, wherein the cell has at least one desired property that is consistent over time, comprising the steps of:

a) providing at least two cells that express RNA encoding the protein of interest;
b) dispersing cells individually into individual culture vessels, thereby providing a plurality of separate cell cultures
c) culturing the cells under a set of desired culture conditions using automated cell culture methods characterized in that the conditions are substantially identical for each of the separate cell cultures, during which culturing the number of cells per separate cell culture is normalized, and wherein the separate cultures are passaged on the same schedule;
d) assaying the separate cell cultures for at least one desired characteristic of the protein of interest at least twice; and
e) identifying a separate cell culture that has the desired characteristic in both assay. Optionally, the cell produced by the method described herein is a differentiated cell. Optionally, the cell produced by the method described herein is a dedifferentiated cell. Optionally, the dedifferentiated cell is a stem cell selected from the group consisting of a multipotent stem cell, a pluripotent stem cell, an omnipotent stem cell, an induced pluripotent stem cell, an embryonic stem cell, a cancer stem cell, an organ-specific stem cell and a tissue-specific stem cell.

Optionally, the plurality of cells in step a) of the methods disclosed herein are cultured for some period of time prior to the dispersing in step b).

Optionally, the individual culture vessels used in the methods disclosed herein are selected from the group consisting of: individual wells of a multiwell plate and vials.

Optionally, the method further comprises the step of determining the growth rate of a plurality of the separate cell cultures and grouping the separate cell cultures by their growth rates into groups such that the difference between the fastest and slowest growth rates in any group is no more than 1, 2, 3, 4 or 5 hours between steps b) and c).

Optionally, the method further comprises the step of preparing a stored stock of one or more of the separate cultures. Optionally, the method further comprises the step of one or more replicate sets of the separate cell cultures and culturing the one or more replicate sets separately from the source separate cell cultures.

Optionally, the assaying in step d) of the method of this invention is a functional assay for the protein.
Optionally, the at least one characteristic that has remained constant in step e) is protein function.
Optionally, the culturing in step c) of the methods of this invention is in a robotic cell culture apparatus. Optionally, the robotic cell culture apparatus comprises a multi-channel robotic pipettor. Optionally, the multi-channel robotic pipettor comprises at least 96 channels. Optionally, the robotic cell culture apparatus further comprises a cherrypicking arm.
Optionally, the automated methods include one or more of: media removal, media replacement, cell washing, reagent addition, removal of cells, cell dispersal, and cell passaging.
Optionally, the plurality of separate cell cultures used in the methods of this invention is at least 50 cultures. Further optionally, the plurality of separate cell cultures is at least 100 cultures. Further optionally, the plurality of separate cell cultures is at least 500 cultures. Still further optionally, the plurality of separate cell cultures is at least 1000 cultures.
Optionally, the growth rate is determined by a method selected from the group consisting of: measuring ATP, measuring cell confluency, light scattering, optical density measurement. Optionally, the difference between the fastest and slowest growth rates in a group is no more than 1, 2, 3, 4, or 5 hours.
Optionally, the culturing in step c) of the methods of this invention is for at least 2 days.
Optionally, the growth rates of the plurality of separate cell cultures are determined by dispersing the cells and measuring cell confluency. Optionally, the cells in each separate cell culture of the methods of this invention are dispersed prior to measuring cell confluency. Optionally, the dispersing step comprises adding trypsin to the well and to eliminate clumps. Optionally, the dispersing step comprises adding a cell dissociation reagent to the well and to eliminate clumps Optionally, the cell confluency of the plurality of separate cell cultures is measured using an automated microplate reader.
Optionally, at least two confluency measurements are made before growth rate is calculated. Optionally, the cell confluency is measured by an automated plate reader and the confluency values are used with a software program that calculates growth rate.
Optionally, the separate cell cultures in step d) are characterization for a desired trait selected from one or more of: fragility, morphology, adherence to a solid surface; lack of adherence to a solid surface and protein function. Further optionally, the desired trait is UPR, cell viability, capacity for improved protein production, yield, folding, assembly, secretion, integration into a cell membrane, post-translational modification, or glycosylation or any combination thereof.
Optionally, the cells used in the methods disclosed herein are eukaryotic cells. Optionally, the eukaryotic cells used in the methods of this invention are mammalian cells. Optionally, the mammalian cell line is selected from the group consisting of: NS0 cells, CHO cells, COS cells, HEK-293 cells, HUVECs, 3T3 cells and HeLa cells. Further optionally, the mammalian cell line is Perc6.
Optionally, the protein of interest expressed in the methods disclosed herein is a human protein. Optionally, the protein of interest is a heteromultimer. Optionally, the protein of interest is a G protein coupled receptor. Further optionally, the protein has no known ligand. Further optionally, there is no known assay to detect functional expression of said protein.
Optionally, the method disclosed herein, further comprises after the identifying step, the steps of:

a) expanding a stored aliquot of the cell culture identified in step e) under desired culture conditions; and
b) determining if the expanded cell culture of a) has the desired characteristic.

Optionally, the invention provides a matched panel of clonal cell lines, wherein the clonal cell lines are of the same cell type, and wherein each cell line in the panel expresses a protein of interest, and wherein the clonal cell lines in the panel are matched to share the same physiological property to allow parallel processing.
Also disclosed herein is a matched panel of clonal cell lines, wherein the clonal cell lines are of the same cell type, and wherein at least two cell lines in the panel express a protein of interest, and wherein the clonal cell lines in the panel are matched to share the same physiological property to allow parallel processing.
Also disclosed herein is a combinatorial matched panel of clonal cell lines wherein the clonal cell lines are the of the same type and wherein at least two of the cell lines in the express a multi-subunit protein of interest and wherein each of said clonal cell lines comprises a different combination of subunits of the multi-subunit protein of interest; and wherein the clonal cell lines of the panel are matched such that they are grown under the same cell culture conditions in parallel.
Optionally, the physiological property is growth rate. Further optionally embodiments, the physiological property is adherence to a tissue culture surface. Further optionally, the physiological property is Z' factor. Further optionally, the physiological property is expression level of RNA encoding the protein of interest. Still further optionally, the physiological property is expression level of the protein of interest. Still further optionally, the physiological property is activity level of RNA encoding the protein of interest. Optionally, the growth rates of the clonal cell lines in the panel are within 1, 2, 3, 4, or 5 hours of each other. Further optionally, the culture conditions used for the matched panel are the same for all clonal cell lines in the panel.
Optionally, the clonal cell line used in the matched panels is a eukaryotic cell line. Optionally, the eukaryotic cell line is a mammalian cell line. Optionally, the cell line cells used in the matched panels are selected from the group consisting of: primary cells and immortalized cells.

Optionally, the cell line cells used in the matched panels are prokaryotic or eukaryotic. Optionally, the cell line cells used in the matched panels are eukaryotic and are selected from the group consisting of: fungal cells, insect cells, mammalian cells, yeast cells, algae, crustacean cells, arthropod cells, avian cells, reptilian cells, amphibian cells and plant cells. Optionally, the cell line cells used in the matched panels are mammalian and are selected from the group consisting of: human, non-human primate, bovine, porcine, feline, rat, marsupial, murine, canine, ovine, caprine, rabbit, guinea pig hamster.

Optionally, the cells in the cell line of the matched panels are engineered to express the protein of interest. Optionally, the cells in the cell line of the matched panels express the protein of interest from an introduced nucleic acid encoding the protein or, in the case of a multimeric protein, encoding a subunit of the protein. Optionally, the cells express the protein of interest from an endogenous nucleic acid and wherein the cell is engineered to activate transcription of the endogenous protein or, in the case of a multimeric protein, activates transcription of a subunit of the protein.

Optionally, the panel comprises at least four clonal cell lines. Further optionally, the panel comprises at least six clonal cell lines. Still further optionally, the panel comprises at least twenty five clonal cell lines.

Optionally, two or more of the clonal cell lines in the panel express the same protein of interest. Further optionally, two or more of the clonal cell lines in the panel express a different protein of interest.

Optionally, the cell lines in the panel express different forms of a protein of interest, wherein the forms are selected from the group consisting of: isoforms, amino acid sequence variants, splice variants, truncated forms, fusion proteins, chimeras, or combinations thereof. Further optionally, the forms are active forms, modified forms, glycosylated forms, proteolyzed forms, or functional forms, or combinations thereof. Still further optionally, the forms are selected from the group consisting of: isoforms, amino acid sequence variants, splice variants, truncated forms, fusion proteins, chimeras, active forms, modified forms, glycosylated forms, proteolyzed forms, functional forms or combinations thereof.

Optionally, the cell lines in the panel express different proteins in a group of proteins of interest, wherein the groups of proteins of interest are selected from the group consisting of: proteins in the same signaling pathway, expression library of similar proteins, monoclonal antibody heavy chain library, monoclonal antibody light chain library and SNPs.

Optionally, the protein of interest expressed in the panel is a single chain protein. Optionally, the single chain protein is a G protein coupled receptor. Optionally, the G protein coupled receptor is a taste receptor. Optionally, the taste receptor is selected from the group consisting of: a bitter taste receptor, a sweet taste receptor, a salt taste receptor and an umami taste receptor.

Optionally, the protein of interest expressed in the panel is a multimeric protein. Optionally, the protein is a heterodimer or a heteromultimer.

Optionally, the protein of interest expressed in the panel is selected from the group consisting of: an ion channel, an ion channel, a G protein coupled receptor (GPCR), tyrosine receptor kinase, cytokine receptor, nuclear steroid hormone receptor and immunological receptor. Further optionally, the protein of interest expressed in the panel is an antibody or a biologic. Optionally, the protein expressed in the matched panel is Epithelial sodium Channel (ENaC). Optionally, the ENaC comprises an alpha subunit, a beta subunit and a gamma subunit. Further optionally, the cell lines in the panel express different ENaC isoforms. Further optionally, the cell lines in the panel comprise different proteolyzed isoforms of ENaC. Optionally, the ENaC is human ENaC. Optionally the protein expressed in the matched panel is voltage gated sodium channel (NaV). Optionally, the NaV comprises an alpha subunit and two beta subunits. Optionally, the NaV is human NaV.

Optionally, the protein expressed in the matched panel is selected from the group consisting of: gamma-aminobutyric acid A receptor (GABA $_A$ receptor), gamma-aminobutyric acid B receptor (GABA $_B$ receptor) and gamma-aminobutyric acid C receptor (GABA c receptor). Optionally, the protein is GABA $_A$ receptor. Optionally, the GABA $_A$ receptor comprises two alpha subunits, two beta subunits and a gamma or delta subunit.

Optionally, the clonal cell lines in the panel are produced simultaneously, or within no more than 4 weeks of each other. Further optionally, the clonal cell lines in the panel were produced using substantially identical methods for isolation, maintenance or testing of the clonal cell lines of the panel.

Also disclosed herein is a cell that expresses a monomeric protein of interest from an introduced nucleic acid encoding said monomeric protein of interest, characterized in that it produces the protein of interest in a form that is or is capable of becoming biologically active, wherein the cell is cultured in the absence of selective pressure and wherein the expression of the protein does not vary by more than 5% over 3 months. Optionally the expression of the protein does not vary by more than 5% over 6 months. Optionally, the monomeric protein of interest has no known ligand.

Also disclosed herein is a cell that expresses a monomeric protein of interest from an introduced nucleic acid encoding said monomeric protein of interest, characterized in that it produces the protein of interest in a form that is or is capable of becoming biologically active, wherein the cell is cultured in the absence of selective pressure and wherein the expression of the protein does not vary by more than 30% over 3 months. Optionally the expression of the protein does not vary by more than 30% over 6 months.

Also disclosed herein is a cell that expresses at least one RNA of interest, wherein said RNA of interest is encoded by an introduced nucleic acid, characterized in that it produces the RNA of interest in a form that is or is capable of becoming biologically active, wherein the cell is cultured in the absence of selective pressure and wherein the expression of the RNA does not vary by more than 30% over 3 months. Optionally the expression of the RNA does not vary by more than 30% over 6 months.
Also disclosed herein is a cell that expresses a protein of interest, wherein said protein of interest is encoded by an introduced nucleic acid, characterized in that it produces the protein of interest in a form that is or is capable of becoming biologically active, wherein the cell is cultured in the absence of selective pressure and wherein the expression of the protein does not vary by more than 30% over 3 months. Optionally the expression of the protein does not vary by more than 30% over 6 months.
Also disclosed herein is a cell that expresses at least one protein of interest, wherein the protein of interest has no known ligand or wherein there is no known assay to detect functional expression of said protein of interest; and wherein said protein of interest does not comprise a protein tag.
Also disclosed herein is a method for identifying a modulator of a protein of interest comprising the steps of:

a) contacting a cell according to any one of the above-described cell embodiments with a test compound; and
b) detecting a change in the activity of the protein of interest in the cell contacted with the test compound compared to the activity of the protein in a cell not contacted by the test compound;

wherein a compound that produces a difference in the activity in the presence compared to in the absence is a modulator of the protein of interest.
Also disclosed herein is a modulator identified by the method of the preceding paragraph.
Also disclosed herein is a cell that expresses at least one protein of interest from an introduced nucleic acid encoding the at least one protein of interest, wherein the at least one protein of interest alters a physiological property of the cell, and wherein the physiological property of the cell does not vary by more than 25% over 3 months under constant cell culture conditions.
Also disclosed herein is a cell that expresses a protein of interest, wherein the cell is engineered to activate transcription of an endogenous nucleic acid encoding the protein of interest, wherein the protein of interest alters a physiological property of the cell, and wherein the physiological property of the cell does not vary by more than 25% over 3 months under constant cell culture conditions.
Also disclosed herein is a cell that expresses an RNA of interest, wherein the RNA of interest is encoded by an introduced nucleic acid, wherein the at least one RNA of interest alters a physiological property of the cell, and wherein the physiological property of the cell does not vary by more than 25% over 3 months under constant cell culture conditions.
A cell that expresses at least one protein of interest from an introduced nucleic acid encoding the at least one protein of interest, said cell being characterized in that it produces the protein of interest in a form that is or is capable of becoming biologically active, and wherein the cell consistently and reproducibly expresses at least 500, 2,500, 5,000, or 100,000 picograms of protein per cell per day.
A cell that expresses a protein of interest, wherein the cell is engineered to activate transcription of an endogenous nucleic acid encoding the protein of interest, said cell being characterized in that it produces the protein of interest in a form that is or is capable of becoming biologically active, and wherein the cell consistently and reproducibly expresses at least 500, 2,500, 5,000, or 100,000 picograms of protein per cell per day.
Optionally, the cell is produced in a period of time selected from less than 1 week, less than 2 weeks, less than 3 weeks, less than 4 weeks, less than 1 month, less than 2 months, less than 3 months, less than 4 months, less than 5 months, less than 6 months, less than 7 months, less than 8 months or less than 9 months.
Also disclosed herein is a cell that expresses at least one protein of interest from an introduced nucleic acid encoding the at least one protein of interest, said cell being characterized in that it produces the protein of interest in a form that is or is capable of becoming biologically active, wherein the cell is produced in a period of time selected from less than 7 months, less than 8 months or less than 9 months, and wherein the cell consistently and reproducibly expresses at least 0.5, 1.0, 5.0 or 10 g/L or protein.
Also disclosed herein is a cell that expresses a protein of interest, wherein the cell is engineered to activate transcription of an endogenous nucleic acid encoding the protein of interest, said cell being characterized in that it produces the protein of interest in a form that is or is capable of becoming biologically active, wherein the cell is produced in a period of time selected from less than 7 months, less than 8 months or less than 9 months, and wherein the cell consistently and reproducibly expresses at least 0.5, 1.0, 5.0 or 10 g/L of protein.
Optionally, the cell is produced in a period of time selected from less than 3 months, less than 4 months or less than 6 months. Optionally, the protein is a monomeric protein. Further optionally, the protein is a multimeric protein. Optionally, the protein of interest does not comprise a protein tag or said cell is cultured in the absence of selective pressure or a combination thereof. Optionally, the multimeric protein of interest comprises at least 2, 3, 4, 5, or at least 6 subunits. Optionally, the multimeric protein of interest is selected from the group consisting of: an ion channel, a G protein coupled receptor (GPCR), tyrosine receptor kinase, cytokine receptor, nuclear steroid hormone receptor, antibody, biologic and immunological receptor. Optionally, the multimeric protein of interest is an ion channel and the cell physiological property is selected from a membrane potential, UPR, cell viability, a capacity for improved protein production, yield, folding assembly, secretion, integration into a cell membrane, post-translational modification, glycosylation, or any combination thereof.

Also disclosed herein is a cell line produced from a cell described herein.

Also disclosed herein is a method for identifying a modulator of a protein of interest comprising the steps of:

a) contacting a cell described herein (*e.g.,* a cell that expresses at least one protein or RNA of interest) with a test compound; and
b) detecting a change in the activity of the protein of interest in the cell contacted with the test compound compared to the activity of the protein in a cell not contacted by the test compound;

wherein a compound that produces a difference in the activity in the presence compared to in the absence is a modulator of the protein of interest.

Also disclosed herein is a matched panel of cells or clonal cell lines comprising at least two cells described herein (*e.g.,* a cell that expresses at least one protein or RNA of interest) or two clonal cell lines described herein (*e.g.,* a cell line produced from a cell described herein), wherein the at least two cells or the at least two clonal cell lines are matched such that they are grown under the same cell culture conditions in parallel.

Optionally, the matched panel comprises at least 10 cells 10 clonal cell lines and the at least 10 cells or the 10 clonal cell lines are matched such that they are grown under identical cell culture conditions in parallel. In other embodiments, the panel comprises at least 100 cells or at least 100 clonal cell lines and the at least 100 cells or the at least 100 clonal cell lines are grown under identical cell culture conditions in parallel.

Also disclosed herein is a matched panel of clonal cell lines wherein the clonal cell lines are of the same type and comprises a first and a second protein of interest; wherein the first protein of interest is the same in each clonal cell line; wherein the second protein of interest is a component of a functional biological pathway; and wherein:

a) the panel comprises at least 5 cell lines;
b) the panel is produced in less than 6 months;
c) the first and second proteins of interest do not have a protein tag;
d) the clonal cell lines are cultured in the absence of selective pressure; or
e) any combination of a)-d).

Also disclosed herein is, the first protein of interest is an antibody and the functional biological pathway is a glycosylation pathway.

Optionally a method for generating an in vitro correlate for an in vivo physiological property, wherein the method comprises:

a) contacting a compound or a plurality of compounds that have the physiological property with a first cell that expresses a first protein of interest;
b) assaying the effect of the compound or plurality of compounds on the first protein in a functional assay;
c) contacting the compound or plurality of compounds with a second cell that expresses a second protein of interest;
d) assaying the effect of the compound or plurality of compounds on the second protein in a functional assay;

wherein the first and second proteins independently i) do not comprise a protein tag, ii) are produced consistently and reproducibly in a form suitable for use in a functional assay such that the cells have a Z' factor of at least 0.4 in the functional assay, iii) are expressed in cells cultured in the absence of selective pressure, iv) alter a physiological property of the cell and wherein the physiological property of the cell does not vary by more than 25% over 3 months under constant cell culture conditions; v) are stably expressed in cells cultured in the absence of selective pressure and wherein the expression of the protein does not vary by more than 30% over 3 months, vi) are expressed in a cell further expressing another protein and said cell is cultured in the absence of selective pressure or vii) any combination thereof; and wherein the profile obtained in steps a) to d) provides an in vitro correlate for the in vivo physiological property.

Optionally, the first and second proteins of interest are independently selected from a monomeric protein or a multimeric protein. Optionally, the multimeric protein comprises at least 2, 3, 4, 5, or 6 subunits. Optionally, the multimeric protein is a heteromultimeric protein. Optionally, the first and second proteins of interest are independently selected from the group consisting of: ENaC, NaV, GABAA, sweet taste receptor, umami taste receptor, bitter taste receptor, CFTR and GCC.

Optionally, the first cell and the second cell are cells within a panel of cells further comprising at least one other cell; each cell in the panel of cells is engineered to express a different protein, and is contacted by the compound or plurality of compounds; the effect of the compound or plurality of compounds on each protein expressed in each cell in the panel of cells is assayed in a functional assay; and the activity profile of the compound or plurality of compounds in each cell is used to generate the in vitro correlate for the physiological property.

Optionally, each protein is independently selected from a monomeric protein or a multimeric protein. Optionally, the multimeric protein comprises at least 2, 3, 4, 5, or 6 subunits. Optionally, the multimeric protein is a heteromultimeric protein. Optionally, each protein is independently selected from the group consisting of: ENaC, NaV, GABAA, sweet taste receptor, umami taste receptor, bitter taste receptor, CFTR and GCC.

Also disclosed herein is a method for predicting a physiological property of a test compound, wherein the method comprises:

a) contacting the test compound or a plurality of test compounds with a first cell that expresses a first protein of interest described hereinabove (*e.g.,* a first protein of interest as described in the method for generating an in vitro correlate for an in vivo physiological property);
b) assaying the effect of the test compound or plurality of test compounds on the first protein in a functional assay;
c) contacting the test compound or plurality of test compounds with a second cell that that expresses a second protein of interest described hereinabove (*e.g.,* a second protein of interest as described in the method for generating an in vitro correlate for an in vivo physiological property);
d) assaying the effect of the test compound or plurality of test compounds on the second protein in a functional assay;
e) comparing the activity profile of the compound obtained in steps a) to d) with an in vitro correlate as generated by the method described hereinabove (*e.g.,* a method for generating an in vitro correlate for an in vivo physiological property),

wherein the first and second proteins independently i) do not comprise a protein tag, ii) are produced consistently and reproducibly in a form suitable for use in a functional assay such that the cells have a Z' factor of at least 0.4 in the functional assay, iii) are expressed in cells cultured in the absence of selective pressure, iv) alter a physiological property of the cell and wherein the physiological property of the cell does not vary by more than 25% over 3 months under constant cell culture conditions; v) are stably expressed in cells cultured in the absence of selective pressure and wherein the expression of the protein does not vary by more than 30% over 3 months, vi) are expressed in a cell further expressing another protein and said cell is cultured in the absence of selective pressure or vii) any combination thereof; and wherein the test compound or plurality of test compounds are predicted to have the physiological property of the in vitro correlate if the activity profile of the test compound or compounds and the activity profile of the in vitro correlate are at least 90% identical.

Also disclosed herein is a method for confirming a physiological property of a test compound or plurality of test compounds, wherein the method comprises:

a) contacting the test compound or a plurality of test compounds with a first cell that expresses a first protein of interest described hereinabove (*e.g.,* a first protein of interest as described in the method for generating an in vitro correlate for an in vivo physiological property);
b) assaying the effect of the test compound or plurality of test compounds on the first protein in a functional assay;
c) contacting the test compound or plurality of test compounds with a second cell that that expresses a second protein of interest described hereinabove (*e.g.,* a second protein of interest as described in the method for generating an in vitro correlate for an in vivo physiological property);
d) assaying the effect of the test compound or plurality of test compounds on the second protein in a functional assay;
e) comparing the activity profile of the test compound or plurality of test compounds obtained in steps a) to d) with an in vitro correlate for the physiological property as generated by the method described hereinabove (*e.g.,* a method for generating an in vitro correlate for an in vivo physiological property), wherein the first and second proteins independently i) do not comprise a protein tag, ii) are produced consistently and reproducibly in a form suitable for use in a functional assay such that the cells have a Z' factor of at least 0.4 in the functional assay, iii) are expressed in cells cultured in the absence of selective pressure, iv) alter a physiological property of the cell and wherein the physiological property of the cell does not vary by more than 25% over 3 months under constant cell culture conditions; v) are stably expressed in cells cultured in the absence of selective pressure and wherein the expression of the protein does not vary by more than 30% over 3 months, vi) are expressed in a cell further expressing another protein and said cell is cultured in the absence of selective pressure or vii) any combination thereof; and wherein the compound is confirmed to have the physiological property if the activity profile of the test compound or plurality of test compounds and the activity profile of the in vitro correlate are at least 90% identical.

Optionally, the first and second proteins are independently selected from a monomeric protein or a multimeric protein. Optionally, the multimeric protein comprises at least 2, 3, 4, 5, or at least 6 subunits. Optionally, the multimeric protein is a heteromultimeric protein.

Optionally, the first cell and the second cell are cells within a panel of cells further comprising at least one other cell; each cell in the panel of cells is engineered to express a different protein, and is contacted by the test compound or plurality of test compounds; the effect of the test compound or plurality of test compounds on each protein of interest expressed in each cell in the panel of cells is assayed in a functional assay; and the activity profile of the test compound or plurality of test compounds in each cell is used to compare with the profile of the in vitro correlate.

Optionally, at least one of the first multimeric protein of interest and the second multimeric protein of interest is a heteromeric protein. Optionally, at least one of the first protein of interest and the second protein of interest is a dimeric protein. Further optionally, at least one of the first protein of interest and the second protein of interest is a trimeric protein. Optionally, the first protein of interest and the second protein of interest are different forms of a multimeric protein. Optionally, the multimeric protein is GABA A receptor.

Optionally, at least one of the first or second protein of interest is part of a functional biological pathway. Optionally, the functional biological pathway is selected from the group consisting of: glycosylation, protein synthesis, UPR, ER, ribosomal, mitochondrial activity, RNA synthesis, post-translational modification, cell signaling, cell growth and cell death.

Optionally, the physiological property is a therapeutic effect. Optionally, the physiological property is an adverse effect. Optionally, the effect of the compound or plurality of compounds on the physiological property is assayed by high throughput screening. Optionally, the step comparing described hereinabove is implemented in a computer system.

Also disclosed herein is a computer-implemented method for determining a physiological property of a test compound or plurality of test compounds, wherein the method comprises:

(a) receiving a first activity profile of said test compound or plurality of test compounds, wherein said first activity profile is generated by the method described hereinabove, and wherein said first activity profile provides an in vitro correlate for the physiological property of said test compound or plurality of test compounds;
(b) comparing said first activity profile to a plurality of landmark activity profiles stored in a database to determine a measure of similarity between said first activity profile and each said landmark activity profile in said plurality of landmark activity profiles, wherein each said landmark activity profile provides an in vitro correlate for a known physiological property of a respective known compound or plurality of known compounds;
(c) determining one or more landmark activity profiles most similar to said first activity profile based on the measures of similarity determined in step (b); and
(d) identifying the known physiological property associated with the one or more landmark activity profiles determined to be most similar to said first activity profile in step (c) as the physiological property of said test compound or plurality of test compounds; wherein steps (a), (b), (c), and (d) are implemented on a suitably programmed computer.

Optionally, the one or more landmark activity profiles are most similar to said first activity profile if said measures of similarity are above a predetermined threshold.

Also disclosed herein is a computer-implemented method for characterizing a test compound or plurality of test compounds as being associated with a particular physiological property, wherein the method comprises:

(a) receiving a first activity profile of said test compound or plurality of test compounds, wherein said first activity profile is generated by the method described hereinabove, and wherein said first activity profile provides an in vitro correlate for the physiological property of said test compound or plurality of compounds;
(b) clustering a plurality of activity profiles, which plurality comprises said first activity profile and a plurality of landmark activity profiles, wherein each said landmark activity profile provides an in vitro correlate for a known physiological property of a respective known compound or plurality of known compounds;
(c) identifying one or more landmark activity profiles in said plurality of landmark activity profiles that cluster with the first activity profile; and
(d) characterizing the test compound or plurality of test compounds as being associated with said known physiological property of the respective known compound or plurality of known compounds corresponding to the one or more landmark activity profiles identified as clustered with said first activity profile in step (c);
wherein steps (a), (b), (c), and (d) are implemented on a suitably programmed computer.

Also disclosed herein is a computer-implemented method of classifying a test compound or a plurality of test compounds as to a physiological property using a classifier, wherein the method comprises:

(a) training a classifier for classifying a test compound or a plurality of test compounds as to a pharmacological property using a plurality of landmark activity profiles stored in a database, wherein each said landmark activity profile provides an in vitro correlate for a known physiological property of a respective known compound or plurality of know compounds; and
(b) processing, using said classifier, a first activity profile generated by the method described hereinabove to classify said test compound or plurality of test compounds as to a physiological property;

wherein steps (a) and (b) are implemented on a suitably programmed computer.

Also disclosed herein is a computer-implemented method of classifying a test compound or a plurality of test compounds as to a physiological property using a classifier, wherein the method comprises:

(a) training a classifier for classifying the compound or plurality of compounds as to a pharmacological property using a plurality of landmark activity profiles stored in a database, wherein each said landmark activity profile provides an in vitro correlate for a known in vivo pharmacological property of a respective compound; and
(b) processing, using said classifier, a first activity profile generated by the method described hereinabove, to classify said test compound or plurality of test compounds as to the physiological property,
(c) training said classifier for classifying a test compound or plurality of test compounds as to a physiological property using a plurality of landmark activity profiles stored in a database, wherein each said landmark activity profile provides an in vitro correlate for a known physiological property of a respective known compound or plurality of compounds;
(d) wherein steps (a) and (b) are implemented on a suitably programmed computer.

Also disclosed herein is a method for characterizing an active subunit combination of a multimeric protein of interest in a cell, wherein the method comprises:

(a) contacting a first cell that expresses a first subunit of the multimeric protein of interest with a test compound or a plurality of test compounds;
(b) contacting a second cell that expresses a second subunit of the multimeric protein of interest with the test compound or plurality of test compounds;
(c) contacting a third cell that expresses the first subunit and the second subunit of the multimeric protein of interest with the test compound or plurality of test compounds;
(d) assaying the effect of the test compound or plurality of test compounds on the multimeric protein as it would be expressed in the first cell, the second cell, and the third cell in a functional assay;
(e) deducing whether the first and/or second subunits are part of the biologically active multimeric protein and wherein the profile obtained in steps a) to d) provides an in vitro correlate for the in vivo physiological property, and wherein the first and second subunits of the multimeric protein independently do not comprise a protein tag, are expressed in cells cultured in the absence of selective pressure or any combination thereof.

Optionally the multimeric protein of interest is a heterodimer. In other embodiments, the multimeric protein of interest is a heterotrimer.

Also disclosed herein is a method for characterizing an active subunit combination of a multimeric protein of interest in a cell, wherein the method comprises:

(a) contacting a first cell that expresses a first subunit of the multimeric protein of interest with a test compound or a plurality of test compounds;
(b) contacting a second cell that expresses a second subunit of the multimeric protein of interest with the test compound or plurality of test compounds;
(c) contacting a third cell that expresses a third subunit of the multimeric protein of interest with the test compound or plurality of test compounds;
(d) contacting a fourth cell that expresses the first subunit, second and third subunits of the multimeric protein of interest with the test compound or plurality of test compounds;
(e) assaying the effect of the test compound or plurality of test compounds on the multimeric protein as it would be expressed in the first cell, the second cell, the third cell and fourth cell in a functional assay;
(f) deducing whether the first, second and/or third subunits are part of the biologically active multimeric protein;

wherein the first, second and third subunits of the multimeric protein independently do not comprise a protein tag, are expressed in cells cultured in the absence of selective pressure or any combination thereof.

Optionally, the multimeric protein is a heterotrimer. Optionally, the multimeric protein is a GABA A receptor.

Also disclosed herein is a panel of cells, wherein the panel comprises a first cell and a second cell, wherein the first cell and the second cell have been engineered to express the same subunit of a multimeric protein of interest, wherein the physiological profile of the multimeric protein of interest in the first cell differs from the physiological profile of the multimeric protein in the second cell, and wherein the first cell and the second cell originate from the same host cell line; wherein the subunits of the multimeric protein of interest do not comprise a protein tag, are expressed in cells cultured in the absence of selective pressure or any combination thereof.

Also disclosed herein is a panel of clonal cell lines, wherein each cell line has been engineered to express the same subunit of a multimeric protein of interest, and wherein the physiological profiles of the multimeric protein in each cell line is different from the physiological profile of the multimeric protein of interest in the other cell lines of the panel, and wherein the cell lines in the panel of cell lines originate from the same host cell line; wherein the subunits of the multimeric protein of interest do not comprise a protein tag, are expressed in cells cultured in the absence of selective pressure or any combination thereof.

Optionally, the panel comprises 2 cell lines. Optionally, the panel comprises 5 cell lines. Optionally, the panel comprises 10 cell lines.

Optionally, the multimeric protein of interest is NaV.

Also disclosed herein is a cell that has been engineered to express all component proteins of a functional biological pathway.

Optionally, the pathway has at least five protein components. Optionally, the cell is cultured in the absence of selective pressure. Optionally, the component proteins of the biological pathway do not comprise a protein tag.

Also disclosed herein is a panel of clonal cell lines comprising a plurality of clonal cell lines, wherein each clonal cell line of the plurality of clonal cell lines has been engineered to express a different odorant receptor; wherein the odorant receptor does not comprise a protein tag, or the odorant receptor is produced consistently and reproducibly in a form suitable for use in a functional assay such that the cells have a Z' factor of at least 0.4 in the functional assay, or the clonal cell lines are cultured in the absence of selective pressure, or any combination thereof.

Optionally, the plurality of clonal cell lines comprises at least 10 cell lines. Optionally, the different odorant receptors are human odorant receptors or insect odorant receptors.

Optionally, the different human odorant receptors are selected from the group consisting of OR10A1, OR10A3, OR10A4, OR10A5, OR10A6, OR10A7, OR10C1, OR10C2, OR10D4, OR10G2, OR10G3, OR10G4, OR10G7, OR10G8, OR10G9, OR10H1, OR10H2, OR10H3, OR10H4, OR10H5, OR10J1, OR10J3, OR10J5, OR10J6, OR10K1, OR10K2, OR10Q1, OR10R2, OR10S1, OR10T2, OR10V1, OR10Z1, OR11A1, OR11G2, OR11H1, OR11H4, OR11H6, OR11H7P, OR11L1, OR12D3, OR13A1, OR13C2, OR13C3, OR13C4, OR13C5, OR13C7, OR13C8, OR13C9, OR13D1, OR13E2, OR13F1, OR13G1, OR13H1, OR13J1, OR14A16, OR14A2, OR14C36, OR14J1, OR1A1, OR1A2, OR1A2, OR1 B1, OR1 C1, OR1D2, OR1D4, OR1D5, OR1 E1, OR1 E2, OR1 E2, OR1E5, OR1 E5, OR1E6, OR1E7, OR1F1, OR1F10, OR1F11, OR1F12, OR1F2, OR1G1, OR111, OR1J1, OR1J2, OR1J2, OR1J4, OR1J5, OR1K1, OR1L1, OR1L3, OR1L4, OR1 L6, OR1 L8, OR1 M1, OR1 M1, OR1N1, OR1 N2, OR1 N3, OR1Q1, OR1 S1, OR1 S2, OR2A1, OR2A10, OR2A19, OR2A20, OR2A21, OR2A4, OR2A42, OR2A5, OR2A6, OR2A7, OR2AE1, OR2AJ1, OR2AK2, OR2B1, OR2B2, OR2B3, OR2B6, OR2B9, OR2C1, OR2D1, OR2D2, OR2D3, OR2F1, OR2F2, OR2F3, OR2G2, OR2G3, OR2H1, OR2H2, OR2H3, OR2J2, OR2J3, OR2K1, OR2K2, OR2L1, OR2L2, OR2L3, OR2L5, OR2L8, OR2M1, OR2M2, OR2M4, OR2S2, OR2T1, OR2T3, OR2T4, OR2T5, OR2T6, OR2T7, OR2T8, OR2V1, OR2V2, OR2V3, OR2W1, OR2W3, OR2Y1, OR2Z1, OR3A1, OR3A2, OR3A3, OR3A4, OR4A15, OR4A16, OR4A4, OR4A5, OR4B1, OR4C12, OR4C13, OR4C15, OR4C16, OR4C3 , OR4C6, OR4D1, OR4D2, OR4D5, OR4D6 , OR4D9, OR4E2, OR4F10, OR4F15, OR4F16, OR4F16, OR4F17, OR4F18, OR4F19, OR4F3, OR4F6, OR4K1, OR4K13, OR4K14, OR4K15, OR4K17, OR4K2, OR4K3, OR4K5, OR4L1, OR4M1, OR4M2, OR4N2, OR4N4, OR4N5, OR4P4, OR4Q3, OR4S1, OR4X1, OR4X2, OR51A2, OR51A4, OR51 A7, OR51B2, OR51 B4, OR51D1, OR51E1, OR51E2, OR51 F2, OR51G1, OR51G2, OR51H1, OR5111, OR5112, OR51L1, OR51M1, OR51Q1, OR51S1, OR51T1, OR52A1, OR52A2, OR52B2, OR52B4, OR52B4, OR52B4 , OR52B6, OR52D1, OR52E2, OR52E4, OR52E5, OR52E6, OR52E8, OR52H1, OR5211, OR5212, OR52J3, OR52K1, OR52K2, OR52L1, OR52L2, OR52N1, OR52N2, OR52N4, OR52N5, OR52P1, OR52R1, OR56A4, OR56A6, OR56B2, OR56B4, OR5A1, OR5A2, OR5AC2, OR5AK2, OR5AK3, OR5AN1, OR5AP2, OR5AR1, OR5AS1, OR5AU1, OR5AU1, OR5B13, OR5B16, OR5B17, OR5B2, OR5B3, OR5C1, OR5D13, OR5D14, OR5D16, OR5D18, OR5F1, OR5G3, OR5H1, OR5H2, OR5H6, OR511, OR5K1, OR5K2, OR5L1, OR5L2, OR5M1, OR5M10, OR5M11, OR5M11, OR5M3, OR5M3, OR5M8, OR5M9, OR5P2, OR5P3, OR5T2, OR5T3, OR5V1, OR6A1, OR6B1, OR6B2, OR6C1, OR6C2, OR6C3, OR6F1, OR6J2, OR6K3, OR6K6, OR6M1, OR6N1, OR6N2, OR6P1, OR6Q1, OR6S1, OR6T1, OR6V1, OR6X1, OR6Y1, OR7A10, OR7A17, OR7A2 , OR7A5, OR7C1, OR7C2, OR7D2, OR7D2, OR7D4P, OR7E102, OR7E120, OR7G1, OR7G2, OR7G3, OR8A1, OR8B12, OR8B2, OR8B3, OR8B4, OR8B8, OR8D1, OR8D2, OR8D4, OR8G1, OR8G2, OR8H1, OR8H2, OR8H3, OR812, OR8J1, OR8J3, OR8K1, OR8K3, OR8K5, OR9A2, OR9A4, OR9G1, OR9G4, OR9G5, OR9I1, OR9K2, and OR9Q1.

Optionally, the different insect odorant receptors are mosquito odorant receptors selected from the group consisting of IOR100, IOR101, IOR102, IOR103, IOR104, IOR105, IOR106, IOR107, IOR108, IOR109, IOR110, IOR111, IOR112, IOR113, IOR114, IOR115, IOR116, IOR117, IOR118, IOR119, IOR120, IOR121, IOR122, IOR123, IOR124, IOR125, IOR126, IOR127, IOR49, IOR50, IOR51, IOR52, IOR53, IOR54, IOR55, IOR56, IOR57, IOR58, IOR59, IOR60, IOR61, IOR62, IOR63, IOR64, IOR65, IOR66, IOR67, IOR68, IOR69, IOR70, IOR71, IOR72, IOR73, IOR74, IOR75, IOR76, IOR77, IOR78, IOR79, IOR80, IOR81, IOR82, IOR83, IOR84, IOR85, IOR86, IOR87, IOR88, IOR89, IOR90, IOR91, IOR92, IOR93, IOR94, IOR95, IOR96, IOR97, IOR98, IOR99, ORL7077, ORL7078, ORL7079, ORL7080, ORL7081, ORL7082, ORL7083, ORL7084, ORL7085, ORL7086, ORL7087, ORL7088, ORL7089, ORL7090, ORL7091, ORL7092, ORL7093, ORL7094, ORL7095, ORL7096, ORL7097, ORL7098, ORL7099, ORL7100, ORL7101, ORL7102, ORL7103, ORL7104, ORL7105, ORL7106, ORL7107, ORL7108, ORL7109, ORL7110, ORL7111, ORL7112, ORL7113, ORL7114, ORL7115, ORL7116, ORL7117, ORL7118, ORL7119, ORL7120, ORL7121, ORL7122, ORL7123, ORL7124, ORL7125, TPR2307, TPR2308, TPR2309, TPR2310, TPR2312, TPR2314, TPR2315, TPR2316, TPR2317, TPR2318, TPR2319, TPR2320, TPR2321, TPR2321, TPR698, TPR699, TPR700, TPR701, TPR702, TPR703, TPR704, TPR705, TPR706, TPR707, TPR708, TPR709, TPR710, TPR711, TPR712, TPR713, TPR714, TPR715, TPR716, TPR717, TPR718, TPR719, TPR720, TPR721, TPR722, TPR723, TPR724, TPR725, TPR725, TPR726, TPR727, TPR728, TPR729, TPR730, TPR731, TPR732, TPR733, TPR734, TPR735, TPR736, TPR737, TPR738, TPR739, TPR740, TPR741, TPR742, TPR743, TPR744, TPR745, TPR746, TPR747, TPR748, TPR749, TPR750, TPR751, TPR752, TPR753, TPR754, TPR755, TPR756, TPR757, TPR758, TPR759, TPR760, TPR761, TPR762, TPR763, TPR764, TPR765, TPR766, TPR767, TPR768, TPR769, TPR770, TPR771, and TPR772.

Also disclosed herein is a method for generating an odorant activity profile of a test compound or composition, wherein the method comprises:

i. contacting the panel described herein (e.g., a panel of clonal cell lines comprising a plurality of clonal cell lines, wherein each clonal cell line of the plurality of clonal cell lines has been engineered to express a different odorant receptor) with the test compound or composition; and
ii. measuring the effect of the test compound or composition on the activity in a functional assay of at least 2 different odorant receptors in the panel, wherein the activities measured in step (ii) provide the odorant activity profile of the test compound or composition.

Also disclosed herein is a method for identifying a second test compound that mimics the odor of a first test compound or composition, wherein the method comprises:

i. contacting the panel described herein (*e.g.,* a panel of clonal cell lines comprising a plurality of clonal cell lines, wherein each clonal cell line of the plurality of clonal cell lines has been engineered to express a different odorant receptor) with the second test compound;
ii. testing the effect of the second test compound on the activity in a functional assay of at least 2 odorant receptors in the panel;
iii. comparing the odorant activity profile of the second test compound obtained in step (ii) with the odorant activity profile of the first test compound or composition; wherein the second test compound mimics the odor of the first test compound or composition if the odorant activity profile of the second test compound is similar to the odorant activity profile of the first test compound or composition.

Also disclosed herein is a method to identify a second test compound that modifies the odorant activity profile of a first test compound or composition, wherein the method comprises:

i. generating the odorant activity profile of a second test compound in the presence of the first test compound or composition in accordance with the method described herein (*e.g.,* a method for generating an odorant activity profile of a test compound or composition);
ii. comparing the odorant activity profile obtained in step (i) with the odorant activity profile of the first test compound or composition in the absence of the second test compound; wherein the second test compound modifies the odorant activity profile of the first test compound or composition if the odorant activity profile of the first test compound or composition alone differs from the odorant activity profile of the second test compound in the presence with the first test compound or composition.

Also disclosed herein is a computer-implemented method for identifying an odor associated with a test compound, wherein the method comprises:

(a) receiving a first odorant activity profile of the test compound, wherein said first odorant activity profile is generated by the method described herein *(e.g.,* a method for generating an odorant activity profile of a test compound or composition);
(b) comparing said first odorant activity profile to a plurality of landmark odorant activity profiles stored in a database to determine a measure of similarity between said first odorant activity profile and each said landmark odorant activity profile in said plurality of landmark odorant activity profiles, wherein each said landmark odorant activity profile corresponds to a respective known compound having a known odor, and wherein each said landmark odorant activity profile is generated by the method described herein *(e.g.,* a method for generating an odorant activity profile of a test compound or composition);

(c) determining one or more landmark odorant activity profiles most similar to said first odorant activity profile based on the measures of similarity determined in step (b); and

(d) identifying the odor associated with the one or more landmark odorant activity profiles determined to be most similar to said first odorant activity profile in step (c) as the odor associated with said known compound;

wherein steps (a), (b), (c), and (d) are implemented on a suitably programmed computer.

Optionally, the one or more landmark odorant activity profiles are most similar to said first odorant activity profile if said measures of similarity are above a predetermined threshold.

Also disclosed herein is a computer-implemented method for characterizing a compound as being associated with a particular odor, wherein the method comprises:

(a) receiving a first odorant activity profile of said compound, wherein said first odorant activity profile is generated by the method described herein (*e.g.,* a method for generating an odorant activity profile of a test compound or composition);

(b) clustering a plurality of odorant activity profiles, which plurality comprises said first odorant activity profile and a plurality of landmark odorant activity profiles, wherein each said landmark odorant activity profile corresponds to a respective known compound having a known odor, and wherein each said landmark odorant activity profile is generated by the method described herein (*e.g.,* a method for generating an odorant activity profile of a test compound or composition);

(c) identifying one or more landmark odorant activity profiles in said plurality of landmark odorant activity profiles that cluster with the first odorant activity profile; and

(d) characterizing the compound as being associated with said known odor associated with the respective compound corresponding to the one or more landmark odorant activity profiles identified as clustered with said first odorant activity profile in step (c);

wherein steps (a), (b), (c), and (d) are implemented on a suitably programmed computer.

Also disclosed herein is a computer-implemented method of classifying a test compound as having an odor using a classifier, wherein the method comprises:

(a) training a classifier for classifying a test compound as to an odor using a plurality of landmark odorant activity profiles stored in a database, wherein each said landmark odorant activity profile corresponds to a respective known compound having a known odor, and wherein each said landmark odorant activity profile is generated by the method described herein (*e.g.,* a method for generating an odorant activity profile of a test compound or composition); and

(b) processing, using said classifier, a first odorant activity profile of said compound generated by the method described herein (*e.g.,* a method for generating an odorant activity profile of a test compound or composition), to classify said compound as to a known odor;

wherein steps (a) and (b) are implemented on a suitably programmed computer.

Also disclosed herein is a computer-implemented method of classifying a test compound as having an odor using a classifier, wherein the method comprises:

processing, using said classifier, a first odorant activity profile of said compound generated by the method described herein (*e.g.,* a method for generating an odorant activity profile of a test compound or composition), to classify said test compound as to a known odor, wherein said classifier is trained according to a method comprising:

training said classifier for classifying a test compound as to an odor using a plurality of landmark odorant activity profiles stored in a database, wherein each said landmark odorant activity profile corresponds to a respective known compound having a known odor, and wherein each said landmark odorant activity profile is generated by the method described herein (*e.g.,* a method for generating an odorant activity profile of a test compound or composition);

wherein the processing is implemented on a suitably programmed computer.

Also disclosed herein is a computer-implemented method for associating one or more test compounds with an odor, wherein the method comprises:

(a) receiving a first odorant activity profile of a first test compound, wherein said first odorant activity profile is generated by the method described herein (*e.g.,* a method for generating an odorant activity profile of a test compound or composition), and wherein said first test compound has a known odor;
(b) comparing said first odorant activity profile to a plurality of landmark odorant activity profiles stored in a database to determine a measure of similarity between said first odorant activity profile and each of said landmark odorant activity profile in said plurality of landmark odorant activity profiles, wherein each said landmark odorant activity profile corresponds to a respective known compound, and wherein each said landmark odorant activity profile is generated by the method described herein (*e.g.,* a method for generating an odorant activity profile of a test compound or composition);
(c) determining one or more landmark odorant activity profiles most similar to said first odorant activity profile based on the measures of similarity determined in step (b); and
(d) characterizing the respective test compound corresponding to the one or more landmark odorant activity profiles determined to be most similar to said first odorant activity profile in step (c) as being associated with said known odor;

wherein steps (a), (b), (c), and (d) are implemented on a suitably programmed computer.

Optionally, the one or more landmark odorant activity profiles are most similar to said first odorant activity profile if said measures of similarity are above a predetermined threshold.

Also disclosed herein is a computer-implemented method for characterizing one or more test compounds as being associated with a particular odor, wherein the method comprises:

(a) receiving a first odorant activity profile of a first test compound, wherein said first odorant activity profile is generated by the method described herein (*e.g.,* a method for generating an odorant activity profile of a test compound or composition), and wherein said first test compound has a known odor;
(b) clustering a plurality of odorant activity profiles, which plurality comprises said first odorant activity profile and a plurality of landmark odorant activity profiles, wherein each said landmark odorant activity profile corresponds to a respective known compound, and wherein each said landmark odorant activity profile is generated by the method described herein (*e.g.,* a method for generating an odorant activity profile of a test compound or composition);
(c) identifying one or more landmark odorant activity profiles in said plurality of landmark odorant activity profiles that cluster with the first odorant activity profile; and
(d) characterizing the respective compound corresponding to the one or more landmark odorant activity profiles identified as clustered with said first odorant activity profile in step (c) as being associated with said known odor; wherein steps (a), (b), (c), and (d) are implemented on a suitably programmed computer.

Also disclosed herein is a computer-implemented method of classifying one or more test compounds as having an odor using a classifier, wherein the method comprises:

processing, using said classifier, a first odorant activity profile generated by the method described herein *(e.g.,* a method for generating an odorant activity profile of a test compound or composition), wherein said first odorant activity profile corresponds to a first test compound having a known odor, to classify one or more landmark odorant activity profiles of a plurality of landmark odorant activity profiles stored in a database as having said known odor, wherein said classifier is trained according to a method comprising:

training said classifier using said plurality of landmark odorant activity profiles for classifying said one or more landmark odorant activity profiles as having an odor, wherein each said landmark odorant activity profile corresponds to a respective known compound, and wherein each said landmark odorant activity profile is generated by the method described herein (*e.g.,* a method for generating an odorant activity profile of a test compound or composition);

wherein the processing is implemented on a suitably programmed computer.

Optionally the RNA of interest is siRNA or an antisense RNA. Optionally, the protein of interest comprises at least 2, 3, 4,5, or 6 subunits.

Optionally, the protein of interest is an orphan receptor identified by a Human Gene Symbol as shown in Table 8 selected from the group consisting of BRS3, GPR42P, FPRL2, GPR81, OPN3, GPR52, GPR21, GPR78, GPR26, GPR37, GPR37L1, GPR63, GPR45, GPR83, GRCAe, GPR153, P2RY5, P2RY10, GPR174, GPR142, GPR139, ADMR, CMKOR1, LGR4, LGR5, LGR6, GPR85, GPR27, GPR173, CCRL2, MAS1, MAS1L, MRGPRE, MRGPRF, MRGPRG, MRGX3e, MRGX4e, GPR50, GPR87, TRAR3f, TRAR4, TRAR5, PNRe, GPR57g, GPR58, EBI2, GPR160, GPRe, GPR1, GPR101, GPR135, OPN5, GPR141, GPR146, GPR148, GPR149, GPR15, GPR150, GPR152, GPR161, GPR17, GPR171, GPR18, GPR19, GPR20, GPR22, GPR25, GPR31 ,GPR32, GPR33, GPR34, GPR55, GPR61, GPR62, GPR79h, GPR82, GPR84, GPR88, GPR92, P2RY8, GPR15, GPR64, GPR56, GPR115, GPR114, BAI1, BAI2, BAI3, CELSR1, CELSR2, CELSR3, EMR1, EMR2, GPR97, GPR110, GPR111, GPR112, GPR113, GPR116, MASS1, ELTD1, GPR123, GPR124, GPR125, GPR126, GPR128, GPR144, EMR3, EMR4b, CD97, LPHN2, LPHN3, LPHN1, GPR157, GPR51, GPR156, GPRC6A, GPRC5A, GPRC5B, GPRC5C, GPRC5D, GPR158 and GPR158L1.

Optionally, at least one subunit of the protein of interest is expressed by gene activation. Further optionally, at least one subunit of the protein of interest is expressed from an introduced nucleic acid.

Also disclosed herein is a method for generating a cell line, wherein the method comprises culturing a plurality of cell lines in a plurality of parallel cultures under the same culture conditions, and identifying a cell line that has at least one property that remains consistent over time.

Optionally, the plurality of parallel cultures comprises at least 50 cell cultures. Further optionally, the plurality of parallel cultures comprises at least 100 cell cultures. Still further optionally, the plurality of parallel cultures comprises at least 200 cell cultures.

Also disclosed herein is a protein or plurality of proteins that is/are an in vitro correlate for an in vivo protein of interest or a plurality of proteins of interest, wherein the in vitro correlate is predictive of the function or activity of the corresponding protein or plurality of proteins of interest expressed in vivo; wherein the in vitro correlate is a biologically active protein or plurality of proteins expressed under non-physiological conditions in vitro; wherein the in vitro correlate comprises at least one functional or pharmacological or physiological profile that corresponds to the in vivo protein or plurality of proteins of interest; and wherein at least 10% of compounds identified in a high throughput screening using said in vitro correlate are capable of having a therapeutic effect in vivo.

Optionally, the in vitro correlate comprises at least 2, 3, 4, 5, or 6 subunits. Optionally, at least one protein of the in vitro correlate comprises at least 2, 3, 4, 5, or 6 subunits. Optionally, the in vitro correlate comprises at a heteromultimer. Optionally, at least one protein of the in vitro correlate comprises a heteromultimer. Optionally, the protein or plurality of proteins of the in vitro correlate does not comprise a protein tag.

Optionally, the in vitro correlate is stably expressed in cells cultured in the absence of selective pressure. Optionally, the in vitro correlate is expressed in a cell line without causing cytotoxicity. Optionally, the in vitro correlate is expressed in a cell that does not endogenously express the protein or plurality of proteins.

Optionally, the protein or plurality of proteins may be produced by a cell of the present invention.

Also disclosed herein is a cell expressing the protein or plurality of proteins as described hereinabove.

Also disclosed herein is a cell line produced from the cell expressing the protein or plurality of proteins as described hereinabove.

Also disclosed herein is a method for identifying a modulator of an in vivo protein of interest comprising the steps of

a) contacting a cell expressing the protein or plurality of proteins as described hereinabove with a test compound; and
b) detecting a change in the activity of the protein or plurality of proteins of the in vitro correlate in the cell contacted with the test compound

compared to the activity of the protein or plurality of proteins of the in vitro correlate in a cell not contacted by the test compound;
wherein a compound that produces a difference in the activity in the presence compared to in the absence is a modulator of the in vivo protein of interest.

Also disclosed herein is a modulator identified by the method described in the preceding paragraph.

Optionally, the cell described in any one of the preceding
paragraphs is a differentiated cell. Optionally, the cell described in any one of the preceding paragraphs is a dedifferentiated cell. Further optionally, the dedifferentiated cell is a stem cell selected from the group consisting of a multipotent stem cell, a pluripotent stem cell, an omnipotent stem cell, an induced pluripotent stem cell, an embryonic stem cell, a cancer stem cell, an organ-specific stem cell and a tissue-specific stem cell.

Also disclosed herein is a method for generating a stem cell comprising the step of: dedifferentiating a differentiated cell into a stem cell, wherein the differentiated cell is a cell described herein or a cell produced by a method described herein. Optionally, the stem cell is selected from the group consisting of a multipotent stem cell, a pluripotent stem cell, an omnipotent stem cell, an induced pluripotent stem cell, an embryonic stem cell, a cancer stem cell, an organ-specific stem cell and a tissue-specific stem cell.

Also disclosed herein is, a method for generating a redifferentiated cell, comprising the steps of:

a) dedifferentiating a cell described in any one of the preceding paragraphs or a cell produced by a method described herein, to produce a stem cell; and

b) redifferentiating the stem cell to produce the redifferentiated cell. In particular embodiments, the stem cell is selected from the group consisting of a multipotent stem cell, a pluripotent stem cell, an omnipotent stem cell, an induced pluripotent stem cell, an embryonic stem cell, a cancer stem cell, an organ-specific stem cell and a tissue-specific stem cell. In certain embodiments, the redifferentiated cell is of a different type than the differentiated cell that has not undergone dedifferention.

Also disclosed herein is a method for generating a non-human organism comprising the steps of:

a) dedifferentiating a differentiated cell described herein or a differentiated cell produced by the method described herein, to produce a stem cell, wherein the stem cell is an embryonic stem cell or an induced pluripotent stem cell; and
b) redifferentiating the stem cell to produce a non-human organism. Optionally, the organism is a mammal. Further optionally, the mammal is a mouse.

Also disclosed herein is a redifferentiated cell produced by a method described herein.
Also disclosed herein is a non-human organism produced by a method described herein. Optionally, the organism is a mammal. Optionally, the mammal is a mouse.
Optionally, cells that endogenously express the protein of interest can be isolated from a population of cells as described herein. Such isolated cells can be used with the methods and compositions described herein, such as the screening methods and panels
In certain aspects, provided herein are cells or cell lines stably expressing a sweet taste receptor comprising a sweet taste receptor T1 R2 subunit and a sweet taste receptor T1 R3 subunit, the expression of at least one of the subunits resulting from introduction of a nucleic acid encoding the subunit into a host cell or gene activation of a nucleic acid encoding the subunit already present in a host cell, the cell or cell line being derived from the host cell. Optionally, the cell or cell line may also be engineered to produce a G protein.
In specific embodiments, at least one sweet taste receptor subunit is expressed from a nucleic acid encoding that subunit that is introduced into the host cell. In other specific embodiments, at least one sweet taste receptor subunit is expressed from a nucleic acid present in the host cell by gene activation. In other specific embodiments, the host cell: a) is a eukaryotic cell; b) is a mammalian cell; c) does not express at least one subunit of a sweet taste receptor or a G protein endogenously; or d) any combination of (a), (b) and (c). In other specific embodiments, the host cell is an HEK-293 cell. In other specific embodiments, the sweet taste receptor a) is mammalian; b) is human; c) comprises subunits from different species; d) comprises one or more subunits that are chimeric; or e) any combination of (a) - (d). In other specific embodiments, the sweet taste receptor is functional. In other specific embodiments, the cell or cell line described herein, has a Z' value of at least 0.3 in an assay. In other specific embodiments,the cell or cell line described herein has a Z' value of at least 0.7 in an assay. In other specific embodiments, the cell or cell line described herein stably expresses the sweet taste receptor in culture media in the absence of selective pressure. In other specific embodiments, the sweet taste T1 R2 receptor subunit is selected from the group consisting of:

a) a sweet taste receptor subunit comprising the amino acid sequence of SEQ ID NO: 34 or a counterpart amino acid sequence of another species;
b) a sweet taste receptor subunit comprising an amino acid sequence that is at least 85% identical to the amino acid sequence of SEQ ID NO: 34 or a counterpart amino acid sequence of another species;
c) a sweet taste receptor subunit comprising an amino acid sequence encoded by a nucleic acid that hybridizes under stringent conditions to SEQ ID NO: 31 or a nucleic acid that encodes the amino acid of SEQ ID NO: 34 or a counterpart amino acid sequence of another species; and
d) a sweet taste receptor subunit comprising an amino acid sequence encoded by a nucleic acid that is at least 85% identical to SEQ ID NO: 31 or a nucleic acid that encodes the amino acid of SEQ ID NO: 34 or a counterpart amino acid sequence of another species.

In particular embodiments, the sweet taste receptor subunit T1 R2 of the cell of cell line described herein is encoded by a nucleic acid selected from the group consisting of:

a) a nucleic acid comprising SEQ ID NO: 31:
b) a nucleic acid comprising a nucleotide sequence that encodes a polypeptide comprising the amino acid sequence of SEQ ID NO: 34 or a counterpart amino acid sequence of another species;
c) a nucleic acid comprising a nucleotide sequence that hybridizes to the nucleic acid of a) or b) under stringent conditions; and
d) a nucleic acid comprising a nucleotide sequence that is at least 95% identical to SEQ ID NO: 31 or a nucleic acid that encodes the amino acid of SEQ ID NO: 34 or a counterpart amino acid sequence of another species. In other particular embodiments, the sweet taste receptor subunit T1 R3 is selected from the group consisting of:
e) a sweet taste receptor subunit comprising an amino acid sequence of SEQ ID NO: 35 or a counterpart amino acid sequence of another species;

f) a sweet taste receptor subunit that comprising an amino acid sequence that is at least 85% identical to the amino acid sequence of SEQ ID NO: 35 or a counterpart amino acid sequence of another species;

g) a sweet taste receptor subunit comprising an amino acid sequence encoded by a nucleic acid that hybridizes under stringent conditions to SEQ ID NO: 32 or a nucleic acid that encodes the amino acid sequence of SEQ ID NO: 35 or a counterpart amino acid sequence of another species; and

h) a sweet taste receptor subunit comprising an amino acid sequence encoded by a nucleic acid that is at least 85% identical to SEQ ID NO: 32 or a nucleic acid that encodes the amino acid sequence of SEQ ID NO: 35 or a counterpart amino acid sequence of another species.

In other particular embodiments, the sweet taste receptor T1 R3 subunit is encoded by a nucleic acid selected from the group consisting of:

a) a nucleic acid comprising SEQ ID NO: 32;

b) a nucleic acid comprising a nucleotide sequence that encodes the polypeptide comprising the amino acid of SEQ ID NO: 35 or a counterpart amino acid sequence of another species;

c) a nucleic acid comprising a nucleotide sequence that hybridizes to the nucleic acid of a) or b) under stringent conditions; and

d) a nucleic acid comprising a nucleotide sequence that is at least 85% identical to SEQ ID NO: 32 or a nucleic acid that encodes the amino acid of SEQ ID NO: 35 or a counterpart amino acid sequence of another species.

In other particular embodiments, the G protein is selected from the group consisting of:

a) a G protein comprising the amino acid sequence of SEQ ID NO: 36 or 37 or a counterpart amino acid sequence of another species;

b) a G protein comprising an amino acid sequence that is at least 85% identical to SEQ ID NO: 36 or 37 or a counterpart amino acid sequence of another species;

c) a G protein comprising an amino acid sequence encoded by a nucleic acid that hybridizes under stringent conditions to SEQ ID NO: 33 or a nucleic acid that encodes the amino acid of SEQ ID NO: 36 or 37 or a counterpart amino acid sequence of another species; and

d) a G protein comprising an amino acid sequence encoded by a nucleic acid sequence that is at least 85% identical to SEQ ID NO: 33 or a nucleic acid that encodes the amino acid of SEQ ID NO: 36 or 37 or a counterpart amino acid sequence of another species.

In other particular embodiments, the G protein is encoded by a nucleic acid selected from the group consisting of:

a) a nucleic acid comprising SEQ ID NO: 33;

b) a nucleic acid comprising a nucleotide sequence that encodes a polypeptide of SEQ ID NO: 36 or 37 or a counterpart amino acid sequence of another species;

c) a nucleic acid comprising a nucleotide sequence that hybridizes to the nucleic acid sequence of a) or b) under stringent conditions and;

d) a nucleic acid comprising a nucleotide sequence that is at least 95% sequence identical to SEQ ID NO: 33 or a nucleic acid sequence that encodes the amino acid of SEQ ID NO: 36 or 37 or a counterpart amino acid sequence of another species.

Also disclosed herein are cells or cell lines stably expressing an umami taste receptor comprising an umami taste receptor T1 R1 subunit and an umami taste receptor T1 R3 subunit, the expression of at least one of the subunits resulting from introduction of a nucleic acid encoding the subunit into a host cell or gene activation of a nucleic acid encoding the subunit already present in a host cell, the cell or cell line being derived from the host cell. Optionally, the cell or cell line may also be engineered to produce a G protein.

Optionally, at least one umami taste receptor subunit is expressed from a nucleic acid encoding that subunit that is introduced into the host cell. Further optionally, at least one umami taste receptor subunit is expressed from a nucleic acid present in the host cell by gene activation. Further optionally, the host cell: a) is a eukaryotic cell; b) is a mammalian cell; c) does not express at least one subunit of a umami taste receptor or a G protein endogenously; or d) any combination of (a), (b) and (c). Further optionally, the host cell is an HEK-293 cell. Further optionally, the umami taste receptor a) is mammalian; b) is human; c) comprises subunits from different species; d) comprises one or more subunits that are chimeric; or e) any combination of (a) - (d). Further optionally, the umami taste receptor is functional. Further optionally, the cell or cell line described herein, has a Z' value of at least 0.3 in an assay. Further optionally, the cell or cell line described herein has a Z' value of at least 0.7 in an assay. Further optionally, the cell or cell line described herein stably expresses the umami taste receptor in culture media in the absence of selective pressure. Further optionally, the umami taste T1 R1 receptor subunit is selected from the group consisting of:

a) an umami taste receptor subunit comprising the amino acid sequence of any one of SEQ ID NOS: 42-45 or a counterpart amino acid sequence of another species;
b) a umami taste receptor subunit comprising an amino acid sequence that is at least 85% identical to the amino acid sequence of any one of SEQ ID NOS: 42-45 or a counterpart amino acid sequence of another species;
c) an umami taste receptor subunit comprising an amino acid sequence encoded by a nucleic acid that hybridizes under stringent conditions to SEQ ID NO: 41 or a nucleic acid that encodes the amino acid of any one of SEQ ID NOS: 42-45 or a counterpart amino acid sequence of another species; and
d) an umami taste receptor subunit comprising an amino acid sequence encoded by a nucleic acid that is at least 85% identical to SEQ ID NO: 41 or a nucleic acid that encodes the amino acid of any one of SEQ ID NOS: 42-45 or a counterpart amino acid sequence of another species.

Optionally, the umami taste receptor subunit T1 R1 of the cell of cell line described herein is encoded by a nucleic acid selected from the group consisting of:

a) a nucleic acid comprising SEQ ID NO: 41:
b) a nucleic acid comprising a nucleotide sequence that encodes a polypeptide comprising the amino acid sequence of any one of SEQ ID NOS: 42-45 or a counterpart amino acid sequence of another species;
c) a nucleic acid comprising a nucleotide sequence that hybridizes to the nucleic acid of a) or b) under stringent conditions; and
d) a nucleic acid comprising a nucleotide sequence that is at least 95% identical to SEQ ID NO: 31 or a nucleic acid that encodes the amino acid of SEQ ID NO: 34 or a counterpart amino acid sequence of another species. In other particular embodiments, the umami taste receptor subunit T1 R3 is selected from the group consisting of:
e) an umami taste receptor subunit comprising an amino acid sequence of SEQ ID NO: 35 or a counterpart amino acid sequence of another species;
f) an umami taste receptor subunit that comprising an amino acid sequence that is at least 85% identical to the amino acid sequence of SEQ ID NO: 35 or a counterpart amino acid sequence of another species;
g) an umami taste receptor subunit comprising an amino acid sequence encoded by a nucleic acid that hybridizes under stringent conditions to SEQ ID NO: 32 or a nucleic acid that encodes the amino acid sequence of SEQ ID NO: 35 or a counterpart amino acid sequence of another species; and
h) an umami taste receptor subunit comprising an amino acid sequence encoded by a nucleic acid that is at least 85% identical to SEQ ID NO: 32 or a nucleic acid that encodes the amino acid sequence of SEQ ID NO: 35 or a counterpart amino acid sequence of another species.

Further optionally, the umami taste receptor T1R3 subunit is encoded by a nucleic acid selected from the group consisting of:

a) a nucleic acid comprising SEQ ID NO: 32;
b) a nucleic acid comprising a nucleotide sequence that encodes the polypeptide comprising the amino acid of SEQ ID NO: 35 or a counterpart amino acid sequence of another species;
c) a nucleic acid comprising a nucleotide sequence that hybridizes to the nucleic acid of a) or b) under stringent conditions; and
d) a nucleic acid comprising a nucleotide sequence that is at least 85% identical to SEQ ID NO: 32 or a nucleic acid that encodes the amino acid of SEQ ID NO: 35 or a counterpart amino acid sequence of another species.

Further optionally, the G protein is selected from the group consisting of:

a) a G protein comprising the amino acid sequence of SEQ ID NO: 36 or 37 or a counterpart amino acid sequence of another species;
b) a G protein comprising an amino acid sequence that is at least 85% identical to SEQ ID NO: 36 or 37 or a counterpart amino acid sequence of another species;
c) a G protein comprising an amino acid sequence encoded by a nucleic acid that hybridizes under stringent conditions to SEQ ID NO: 33 or a nucleic acid that encodes the amino acid of SEQ ID NO: 36 or 37 or a counterpart amino acid sequence of another species; and
d) a G protein comprising an amino acid sequence encoded by a nucleic acid sequence that is at least 85% identical to SEQ ID NO: 33 or a nucleic acid that encodes the amino acid of SEQ ID NO: 36 or 37 or a counterpart amino acid sequence of another species.

Further optionally, the G protein is encoded by a nucleic acid selected from the group consisting of:

a) a nucleic acid comprising SEQ ID NO: 33;
b) a nucleic acid comprising a nucleotide sequence that encodes a polypeptide of SEQ ID NO: 36 or 37 or a counterpart amino acid sequence of another species;
c) a nucleic acid comprising a nucleotide sequence that hybridizes to the nucleic acid sequence of a) or b) under stringent conditions and;
d) a nucleic acid comprising a nucleotide sequence that is at least 95% sequence identical to SEQ ID NO: 33 or a nucleic acid sequence that encodes the amino acid of SEQ ID NO: 36 or 37 or a counterpart amino acid sequence of another species.

Optionally the cells and cell lines of this invention produce umami taste receptors or sweet taste receptors that are functional and physiologically relevant. They are, thus, useful to identify and selector modulators of the umami taste receptor or sweet taste receptor.

Further optionally, the cells and cell lines of this invention stably express an umami taste receptor or a sweet taste receptor over 1 to 4 weeks, 1 to 9 months or any time in between.

Further optionally, the cells and cell lines of the invention express an umami taste receptor or a sweet taste receptor at substantially the same level over a period of 1 to 4 weeks, 1 to 9 months or any time in between.

Optionally, the expression levels are measured using a functional assay.

Also disclosed herein are modulators of umami taste receptors or sweet taste receptors identified using the cells and cell lines of this invention and the use of those modulators in modifying the tastes of products, including foods and pharmaceuticals, or in treating diseases, including diabetes and obesity, where umami taste receptors or sweet taste receptors are implicated.

Also disclosed herein is a method for producing the cell or cell line described herein (*e.g.*, cell or cell line stably expressing a sweet taste receptor) comprising the steps of:

a) introducing a first vector comprising a nucleic acid encoding a sweet taste receptor T1 R2 subunit, a second vector comprising a nucleic acid encoding a sweet taste receptor T1 R3 subunit and optionally a third vector comprising a nucleic acid encoding a G protein into a host cell;
b) introducing a first molecular beacon that detects the expression of the sweet taste receptor T1 R2 subunit, a second molecular beacon that detects the expression of the sweet taste receptor T1 R3 subunit and optionally a third molecular beacon that detects the expression of the G protein, into the host cell produced in step a); and
c) isolating a cell that expresses the T1 R2 subunit, the T1R3 subunit and optionally the G protein.

Also disclosed herein is a method for producing the cell or cell line described herein (*e.g.*, a cell or cell line stably expressing an umami taste receptor) comprising the steps of:

a) introducing a first vector comprising a nucleic acid encoding an umami taste receptor T1 R1 subunit, a second vector comprising a nucleic acid encoding an umami taste receptor T1 R3 subunit and optionally a third vector comprising a nucleic acid encoding a G protein into a host cell;
b) introducing a first molecular beacon that detects the expression of the umami taste receptor T1 R1 subunit, a second molecular beacon that detects the expression of the umami taste receptor T1 R3 subunit and optionally a third molecular beacon that detects the expression of the G protein, into the host cell produced in step a); and
c) isolating a cell that expresses the T1 R1 subunit, the T1 R3 subunit and optionally the G protein.

Optionally, the method described herein (e.g., method for producing a cell or cell line stably expressing a sweet taste receptor or an umami taste receptor) further comprises the step of generating a cell line from the cell isolated in step c). Further optionally, the host cell:

a) is a eukaryotic cell;
b) is a mammalian cell;
c) does not express at least a subunit of a sweet taste receptor or umami taste receptor or a G protein endogenously; or
d) any combination of a), b) and c).

Further optionally, the method described herein (*e.g.,* method for producing a cell or cell line stably expressing a sweet taste receptor or an umami taste receptor) further comprises the steps of:

a) culturing the cells for a period of time, selected from the group of 1 to 4 weeks, 1 to 9 months, or any time in between;
b) assaying the expression of the sweet taste receptor or the umami taste receptor or its subunits periodically over those times, the expression being assayed at the RNA or protein level; and
c) selecting the cells or cell lines that are characterized by substantially stable expression of the sweet taste receptor or the umami taste receptor or its subunits over a period of time selected from the group of 1 to 4 weeks, 1 to 9 months, or any time in between.

Further optionally, the method described herein (*e.g.*, method for producing a cell or cell line stably expressing a sweet taste receptor or an umami taste receptor) further comprises the steps of:

a) culturing the cells for a period of time, selected from the group of 1 to 4 weeks, 1 to 9 months or any time in between;
b) measuring the expression levels of the sweet taste receptor or the umami taste receptor or its subunits periodically over those times, the expression
being assayed at the RNA or protein level; and
c) selecting the cells or cell lines that are characterized by substantially the same level of the expression of the sweet taste receptor or the umami taste receptor or its subunits over a period of time selected from the group of 1 to 4 weeks, 1 to 9 months or any time in between.

Optionally, measuring of the protein expression levels of the sweet taste receptor is carried out using a functional assay. Optionally, the isolating step utilizes a fluorescence activated cell sorter (Beckman Coulter, Miami, FL).

Also disclosed herein is a method for identifying a modulator of a sweet taste receptor function comprising the step of exposing at least one cell or cell line described herein stably expressing a sweet taste receptor to at least one test compound and detecting a change in sweet taste receptor function. Optionally, the modulator is selected from the group consisting of a sweet taste receptor inhibitor, a sweet taste receptor antagonist, a sweet taste receptor blocker, a sweet taste receptor activator, a sweet taste receptor agonist or a sweet taste receptor potentiator. Further optionally, the sweet taste receptor is human sweet taste receptor. Further optionally, the test compound is a small molecule, a chemical moiety, a polypeptide, or an antibody. Further optionally, the test compound is a library of compounds. Further optionally, the library is a small molecule library, a combinatorial library, a peptide library or an antibody library. Optionally, the modulator is selective for an enzymatically modified form of a sweet taste receptor.

Also disclosed herein is a method for identifying a modulator of an umami taste receptor function comprising the step of exposing at least one cell or cell line described herein stably expressing an umami taste receptor to at least one test compound and detecting a change in umami taste receptor function. Optionally, the modulator is selected from the group consisting of an umami taste receptor inhibitor, an umami taste receptor antagonist, an umami taste receptor blocker, an umami taste receptor activator, an umami taste receptor agonist or an umami receptor potentiator. Further optionally, the umami taste receptor is human umami taste receptor. Further optionally, the test compound is a small molecule, a chemical moiety, a polypeptide, or an antibody. Further optionally, the test compound is a library of compounds. Further optionally, the library is a small molecule library, a combinatorial library, a peptide library or an antibody library. Optionally, the modulator is selective for an enzymatically modified form of an umami taste receptor.

Also disclosed herein is a modulator identified by the method described herein (*e.g.*, a method for identifying a modulator of a sweet taste receptor function or an umami taste receptor function).

Also disclosed herein is a cell or cell line described herein (e.g., cell or cell line stably expressing a sweet taste receptor or an umami taste receptor) which is produced by the method described herein for producing such cell or cell line. Optionally, the cell (e.g., cell stably expressing a sweet taste receptor or an umami taste receptor) of such method has at least one desired property that is consistent over time, and such method comprises the steps of:

(a) providing a plurality of cells that express mRNA encoding the subunits of the taste receptor (*e.g.*, sweet taste receptor or umami taste receptor) and optionally a G protein;
(b) dispersing cells individually into individual culture vessels, thereby providing a plurality of separate cell cultures;
(c) culturing the cells under a set of desired culture conditions using automated cell culture methods characterized in that the conditions are substantially identical for each of the separate cell cultures, during which culturing the number of cells per well in each separate cell culture is normalized, and wherein the separate cultures are passaged on the same schedule;
(d) assaying the separate cell cultures for at least one desired characteristic of the taste receptor (*e.g.*, sweet taste receptor or umami taste receptor) or of a cell producing that receptor at least twice; and
(e) identifying a separate cell culture that has the desired characteristic in both assays. In certain aspects, provided herein is a cell or cell line producing a taste receptor (*e.g.*, sweet taste receptor or umami taste receptor) and having at least one desired property that is consistent over time, the cell or cell line being produced by such method.

In certain embodiments, cells that endogenously express the sweet receptor, umami receptor, and / or G protein can be isolated from a population of cells as described herein. Such isolated cells can be used with the methods and compositions described herein, such as the screening methods and panels

Also disclosed herein is a cell or cell line which is engineered to stably express a bitter receptor. Optionally, the bitter receptor is expressed from a nucleic acid introduced into the cell or cell line. Further optionally, the bitter receptor is expressed from an endogenous nucleic acid by engineered gene activation. Optionally, the cell or cell line stably expresses at least one other bitter receptor. Optionally, the at least one other bitter receptor is endogenously expressed. Further optionally, the at least one other bitter receptor is expressed from a nucleic acid introduced into the cell or cell line. Still further optionally, the bitter receptor and the at least one other bitter receptor are expressed from separate nucleic acids introduced into the cell or cell line. Still further optionally, the bitter receptor and the at least one other bitter receptor are both expressed form a single nucleic acid introduced into the cell or cell line.

Optionally, the cell or cell line stably expresses an endogenous G protein. Further optionally, the cell or cell line stably expresses a heterologous G protein. Still further optionally, the cell or cell line stably expresses both an endogenous G protein and a heterologous G protein. Optionally, the G protein is a heteromultimeric G protein comprising three different subunits. Optionally, at least one subunit of a heteromultimeric G protein is expressed from a nucleic acid introduced into the cell or cell line. Further optionally, at least two different subunits are expressed from different nucleic acids introduced into the cell or cell line. Still further optionally, at least two different subunits are expressed from the same nucleic acid introduced into the cell or cell line. Still further optionally embodiments, the three different subunits are each expressed from the same nucleic acid introduced into the cell or cell line.

Optionally, the cell or the cells in the cell line are eukaryotic cells. Further optionally, the cell or the cells in the cell line are mammalian cells. Still further optionally, the cell or the cells in the cell line do not express an endogenous bitter receptor. Still further optionally, the cell or the cells in the cell line are eukaryotic cells of a cell type that does not express an endogenous bitter receptor. Still further optionally, the cell or the cells in the cell line are mammalian cells of a cell type that does not express an endogenous bitter receptor. Optionally, the cell or the cells in the cell line are human embryonic kidney 293T cells.

Optionally, the bitter receptor is mammalian. Further optionally, the bitter receptor is human. Still further optionally, the bitter receptor does not have a polypeptide tag at its amino terminus or carboxyl terminus. Still further optionally, the bitter receptor is a mammalian bitter receptor that does not have a polypeptide tag at its amino terminus or carboxyl terminus. Still further optionally, the bitter receptor is a human bitter receptor that does not have a polypeptide tag at its amino terminus or carboxyl terminus.

Optionally, the cell or cell line produces a Z' value of at least 0.45 in an assay selected from the group consisting of: a cell-based assay, a fluorescent cell-based assay, a high throughput screening assay, a reporter cell-based assay, a G protein mediated cell-based assay, and a calcium flux cell-based assay. Further optionally, the cell or cell line produces a Z' value of at least 0.5 in an assay selected from the group consisting of: a cell-based assay, a fluorescent cell-based assay, a high throughput screening assay, a reporter cell-based assay, a G protein mediated cell-based assay, and a calcium flux cell-based assay. Still further optionally, the cell or cell line produces a Z' value of at least 0.6 in an assay selected from the group consisting of: a cell-based assay, a fluorescent cell-based assay, a high throughput screening assay, a reporter cell-based assay, a G protein mediated cell-based assay, and a calcium flux cell-based assay.

Optionally, the cell or cell line stably expresses the bitter receptor in culture media without antibiotics for at least 2 weeks. Further optionally, the cell or cell line stably expresses the bitter receptor in culture media without antibiotics for at least 4 weeks. Still further optionally, the cell or cell line stably expresses the bitter receptor in culture media without antibiotics for at least 6 weeks. Still further optionally, the cell or cell line stably expresses the bitter receptor in culture media without antibiotics for at least 3 months. Still further optionally, the cell or cell line stably expresses the bitter receptor in culture media without antibiotics for at least 6 months. Still further optionally, the cell or cell line stably expresses the bitter receptor in culture media without antibiotics for and at least 9 months.

Optionally, the bitter receptor comprises the amino acid sequence of any one of SEQ ID NOS: 77-101. Further optionally, the bitter receptor comprises an amino acid sequence that is at least 95% identical to the amino acid sequence of any one of SEQ ID NOS: 77-101. Still further optionally, the bitter receptor comprises an amino acid sequence encoded by a nucleic acid that hybridizes to a nucleic acid comprising the reverse-complement sequence of any one of SEQ ID NOS: 51-75 under stringent conditions. Still further optionally, the bitter receptor comprises an amino acid sequence encoded by a nucleic acid that is an allelic variant of any one of SEQ ID NOS: 51-75.

Optionally, the bitter receptor comprises an amino acid sequence encoded by the nucleotide sequence of any one of SEQ ID NOS: 51-75. Further optionally, the bitter receptor comprises an amino acid sequence encoded by a nucleotide sequence that is at least 95% identical to any one of SEQ ID NOS: 51-75. Still further optionally, the bitter receptor comprises an amino acid sequence encoded by the sequence of a nucleic acid that hybridizes to a nucleic acid comprising the reverse-complement sequence of any one of SEQ ID NOS: 51-75 under stringent conditions. Still further optionally, the bitter receptor comprises an amino acid sequence encoded by the sequence of a nucleic acid that is an allelic variant of any one of SEQ ID NOS: 51-75.

Optionally, the bitter receptor is a functional bitter receptor. Optionally, the cell or cell line has a change in the concentration of intracellular free calcium when contacted with isoproterenol. Optionally, the isoproterenol has an EC50 value of between about 1 nM and about 20 nM in a dose response curve conducted with the cell or cell line. Optionally, the cell or cell line has a signal to noise ratio of greater than 1.

Also disclosed herein is a collection of cell or cell line that is engineered to stably express a bitter receptor. Optionally, the collection comprises two or more cell or cell lines, each cell or cell line stably expresses a different bitter receptor or an allelic variant thereof. Optionally, the collection additionally comprises a cell or cell line engineered to stably express a bitter receptor with a known ligand. Optionally, the allelic variant is a single-nuelcotide polymorphism (SNP). Still further optionally, each of the cells or cell lines has a change in the concentration of intracellular free calcium when contacted with isoproterenol. Optionally, the isoproterenol has an EC50 value of between about 1 nM and about 20 nM in a dose response curve conducted with each cell or cell line.

Optionally, the cells or cell lines are matched to share the same physiological property to allow parallel processing. Optionally, the physiological property is growth rate. Further optionally, the physiological property is adherence to a tissue culture surface. Still further optionally, the physiological property is Z' factor. Still further optionally, the physiological property is expression level of the bitter receptor.

Further optionally the collection comprises two or more cell or cell lines, each cell or cell line stably expresses a same bitter receptor or an allelic variant thereof. Optionally, the collection additionally comprises a cell or cell line engineered to stably express a bitter receptor with a known ligand. Optionally, the allelic variant is a SNP. Still further optionally, each of the cells or cell lines has a change in the concentration of intracellular free calcium when contacted with isoproterenol. Optionally, the isoproterenol has an EC50 value of between about 1 nM and about 20 nM in a dose response curve conducted with each cell or cell line.

Optionally, the cells or cell lines are matched to share the same physiological property to allow parallel processing. Optionally, the physiological property is growth rate. Further optionally, the physiological property is adherence to a tissue culture surface. Still further optionally the physiological property is Z' factor. Still further optionally, the physiological property is expression level of the bitter receptor.

Also disclosed herein is a method of producing a cell stably expressing a bitter receptor. The method comprises: a) introducing a nucleic acid encoding the bitter receptor into a plurality of cells; b) introducing a molecular beacon that detects expression of the bitter receptor into the plurality of cells provided in step (a); and c) isolating a cell that expresses the bitter receptor. Optionally, the method further comprises the step of generating a cell line from the cell isolated in step (c). Optionally, the generated cell line stably expresses the bitter receptor in culture media without any antibiotic selection for at least 2 weeks. Further optionally, the generated cell line stably expresses the bitter receptor in culture media without any antibiotic selection for at least 4 weeks. Still further optionally, the generated cell line stably expresses the bitter receptor in culture media without any antibiotic selection for at least 6 weeks. Still further optionally, the generated cell line stably expresses the bitter receptor in culture media without any antibiotic selection for at least 3 months. Still further optionally, the generated cell line stably expresses the bitter receptor in culture media without any antibiotic selection for at least 6 months. Still further optionally, the generated cell line stably expresses the bitter receptor in culture media without any antibiotic selection for at least 9 months.

Optionally, the cells that are used to produce a cell stably expressing a bitter receptor are eukaryotic cells. Further optionally, the cells that are used to produce a cell stably expressing a bitter receptor are mammalian cells. Still further optionally, the cells that are used to produce a cell stably expressing a bitter receptor do not express an endogenous bitter receptor. Still further optionally, the cells that are used to produce a cell stably expressing a bitter receptor are eukaryotic cells of a cell type that does not express an endogenous bitter receptor. Still further optionally, the cells that are used to produce a cell stably expressing a bitter receptor are mammalian cells of a cell type that does not express an endogenous bitter receptor. Optionally, the cells that are used to produce a cell stably expressing a bitter receptor are human embryonic kidney 293T cells.

Optionally, the bitter receptor is mammalian. Further optionally, the bitter receptor is human. Still further optionally, the bitter receptor does not have a polypeptide tag at its amino terminus or carboxyl terminus. Still further optionally, the bitter receptor is a mammalian bitter receptor that does not have a polypeptide tag at its amino terminus or carboxyl terminus. Still further optionally, the bitter receptor is a human bitter receptor that does not have a polypeptide tag at its amino terminus or carboxyl terminus.

Optionally, the bitter receptor comprises the amino acid sequence of any one of SEQ ID NOS: 77-101. Further optionally, the bitter receptor comprises an amino acid sequence that is at least 95% identical to the amino acid sequence of any one of SEQ ID NOS: 77-101. Still further optionally, the bitter receptor comprises an amino acid sequence encoded by a nucleic acid that hybridizes to a nucleic acid comprising the reverse-complement sequence of any one of SEQ ID NOS: 51-75 under stringent conditions. Still further optionally, the bitter receptor comprises an amino acid sequence encoded by a nucleic acid that is an allelic variant of any one of SEQ ID NOS: 51-75.

Optionally, the bitter receptor comprises an amino acid sequence encoded by the nucleotide sequence of any one of SEQ ID NOS: 51-75.

Further optionally, the bitter receptor comprises an amino acid sequence encoded by a nucleotide sequence that is at least 95% identical to any one of SEQ ID NOS: 51-75. Still further optionally, the bitter receptor comprises an amino acid sequence encoded by the sequence of a nucleic acid that hybridizes to a nucleic acid comprising the reverse-complement sequence of any one of SEQ ID NOS: 51-75 under stringent conditions. Still further optionally, the bitter receptor comprises an amino acid sequence encoded by the sequence of a nucleic acid that is an allelic variant of any one of SEQ ID NOS: 51-75.

Optionally, the bitter receptor is a functional bitter receptor. Optionally, the cell isolated in step (c) has a change in the concentration of intracellular free calcium when contacted with isoproterenol. Optionally, the isoproterenol has an EC50 value of between about 1 nM and about 20 nM in a dose response curve conducted with the cell.

Optionally, the isolating utilizes a fluorescence activated cell sorter.

Optionally, the cells that are used to produce a cell stably expressing a bitter receptor stably express an endogenous G protein. Further optionally, the cells that are used to produce a cell stably expressing a bitter receptor stably express a heterologous G protein. Still further optionally, the cells that are used to produce a cell stably expressing a bitter receptor stably express an endogenous G protein and a heterologous G protein.

Optionally, the method of producing a cell line expressing a bitter receptor further comprises introducing into the cells a nucleic acid encoding a G protein. Optionally, the nucleic acid encoding the G protein is introduced into the cells before introducing the nucleic acid encoding the bitter receptor. Further optionally, the nucleic acid encoding the G protein is introduced into the cells after introducing the nucleic acid encoding the bitter receptor. Still further optionally, the nucleic acid encoding the G protein is introduced simultaneously with introducing the nucleic acid encoding the bitter receptor. Optionally, the nucleic acid encoding the bitter receptor and the nucleic acid encoding the G protein are on a single vector. Optionally, the method further comprises introducing into the cells a molecular beacon that detects expression of the G protein before introducing the molecular beacon that detects expression of the bitter receptor. Further optionally, the method further comprises introducing into the cells a molecular beacon that detects expression of the G protein after introducing the molecular beacon that detects expression of the bitter receptor. Still further optionally, the method further comprises introducing into the cells a molecular beacon that detects expression of the G protein simultaneously with introducing the molecular beacon that detects expression of the bitter receptor. Optionally, the molecular beacon that detects expression of the bitter receptor and the molecular beacon that detects expression of the G protein are different molecular beacons. Further optionally, the molecular beacon that detects expression of the bitter receptor and the molecular beacon that detects expression of the G protein are a same molecular beacon. Optionally, the method further comprises isolating a cell that expresses the G protein before isolating the cell that expresses the bitter receptor, thereby isolating a cell that expresses both the bitter receptor and the G protein. Further optionally, the method further comprises isolating a cell that expresses the G protein after isolating the cell that expresses the bitter receptor, thereby isolating a cell that expresses both the bitter receptor and the G protein. Still further optionally, the method further comprises isolating a cell that expresses the G protein simultaneously with isolating the cell that expresses the bitter receptor, thereby isolating a cell that expresses both the bitter receptor and the G protein.

Also disclosed herein is a method of identifying a modulator of a bitter receptor function comprising a) exposing a cell or cell line that stably expresses a bitter receptor to a test compound; and b) detecting a change in a function of the bitter receptor. Optionally, the detecting utilizes an assay that measures intracellular free calcium. Optionally, the intracellular free calcium is measured using one or more calcium-sensitive fluorescent dyes, a fluorescence microscope, and optionally a fluorescent plate reader, wherein at least one fluorescent dye binds free calcium. Further optionally, the intracellular free calcium is monitored by real-time imaging using one or more calcium-sensitive fluorescent dyes, wherein at least one fluorescent dye binds free calcium.

Optionally, the cells or the cells in the cell line are eukaryotic cells. Further optionally, the cells or the cells in the cell line are mammalian cells. Still further optionally, the cells or the cells in the cell line do not express an endogenous bitter receptor. Still further optionally, the cells or the cells in the cell line are eukaryotic cells of a cell type that does not express an endogenous bitter receptor. Still further optionally, the cells or the cells in the cell line are mammalian cells of a cell type that does not express an endogenous bitter receptor. Optionally, the cells or the cells in the cell line are human embryonic kidney 293T cells.

Optionally, the bitter receptor comprises the amino acid sequence of any one of SEQ ID NOS: 77-101. Further optionally, the bitter receptor comprises an amino acid sequence that is at least 95% identical to the amino acid sequence of any one of SEQ ID NOS: 77-101. Still further optionally, the bitter receptor comprises an amino acid sequence encoded by a nucleic acid that hybridizes to a nucleic acid comprising the reverse-complement sequence of any one of SEQ ID NOS: 51-75 under stringent conditions. Still further optionally, the bitter receptor comprises an amino acid sequence encoded by a nucleic acid that is an allelic variant of any one of SEQ ID NOS: 51-75.

Optionally, the bitter receptor comprises an amino acid sequence encoded by the nucleotide sequence of any one of SEQ ID NOS: 51-75. Further optionally, the bitter receptor comprises an amino acid sequence encoded by a nucleotide sequence that is at least 95% identical to any one of SEQ ID NOS: 51-75. Still further optionally, the bitter receptor comprises an amino acid sequence encoded by the sequence of a nucleic acid that hybridizes to a nucleic acid comprising the reverse-complement sequence of any one of SEQ ID NOS: 51-75 under stringent conditions. Still further optionally, the bitter receptor comprises an amino acid sequence encoded by the sequence of a nucleic acid that is an allelic variant of any one of SEQ ID NOS: 51-75.

Optionally, the cell or cell line has a change in the concentration of intracellular free calcium. Optionally, the isoproterenol has an EC50 value of between 1 nM and 20 nM in a dose response curve conducted with the cell or cell line.

Optionally, the test compound is a bitter receptor inhibitor. Optionally, the method further comprises exposing the cell or cell line to a known agonist of the bitter receptor prior to the step of exposing the cell or cell line to the test compound. Further optionally, the method further comprises exposing the cell or cell line to a known agonist of the bitter receptor simultaneously with the step of exposing the cell or cell line to the test compound.

Optionally, the test compound is a bitter receptor agonist. Optionally, the method further comprises exposing the cell or cell line to a known inhibitor of the bitter receptor prior to the step of exposing the cell or cell line to the test compound. Further optionally, the method further comprises exposing the cell or cell line to a known inhibitor of the bitter receptor simultaneously with the step of exposing the cell or cell line to the test compound.

Optionally, the test compound is a small molecule. Optionally, the test compound is a chemical moiety. Optionally, the test compound is a polypeptide. Optionally, the test compound is an antibody. Optionally, the test compound is a food extract.

Also disclosed herein is a method of identifying a modulator of bitter receptor function comprising a) exposing a collection of cell lines to a library of different test compounds, wherein the collection of cell lines comprises two or more cell lines, each cell line stably expressing a same bitter receptor or an allelic variant thereof, and wherein each cell line is exposed to one or more test compounds in the library; and b) detecting a change in a function of the bitter receptor or allelic variant thereof stably expressed by each cell line. Optionally, the detecting utilizes an assay that measures intracellular free calcium. Optionally, the intracellular free calcium is measured using one or more calcium-sensitive fluorescent dyes, a fluorescence microscope, and optionally a fluorescent plate reader, wherein at least one fluorescent dye binds free calcium. Further optionally, the intracellular free calcium is monitored by real-time imaging using one or more calcium-sensitive fluorescent dyes, wherein at least one fluorescent dye binds free calcium.

Optionally, the library is a small molecule library. Optionally, the library is a combinatorial library. Optionally, the library is a peptide library. Optionally, the library is an antibody library.

Optionally, the test compounds are small molecules. Optionally, the test compounds are chemical moieties. Optionally, the test compounds are polypeptides. Optionally, the test compounds are antibodies. Optionally, the test compounds are food extracts.

Optionally, the method further comprises exposing the collection of cell lines to a known bitter receptor agonist prior to or concurrently with step (a). Further optionally, the method further comprises exposing the collection of cell lines to a known bitter receptor inhibitor prior to or concurrently with step (a).

Also disclosed herein is a method of identifying a modulator of bitter receptor function comprising. a) exposing a collection of cell lines to a test compound, wherein the collection of cell lines comprises two or more cell lines, each cell line stably expressing a different bitter receptor or an allelic variant thereof; and b) detecting a change in a function of the bitter receptor stably expressed by each cell line. Optionally, the detecting utilizes an assay that measures intracellular free calcium. Optionally, the intracellular free calcium is measured using one or more calcium-sensitive fluorescent dyes, a fluorescence microscope, and optionally a fluorescent plate reader, wherein at least one fluorescent dye binds free calcium. Further optionally, the intracellular free calcium is monitored by real-time imaging using one or more calcium-sensitive fluorescent dyes, wherein at least one fluorescent dye binds free calcium.

Optionally, the test compound is a small molecule. Optionally, the test compound is a chemical moiety. Optionally, the test compound is a polypeptide. Optionally, the test compound is an antibody. Optionally, the test compound is a food extract.

Optionally, the method further comprises exposing the collection of cell lines to a known bitter receptor agonist prior to or concurrently with step (a). Further optionally, the method further comprises exposing the collection of cell lines to a known bitter receptor inhibitor prior to or concurrently with step (a).

Also disclosed herein is a cell engineered to stably express a bitter receptor at a consistent level over time which is made by a method comprising the steps of: a) providing a plurality of cells that express mRNAs encoding the bitter receptor; b) dispersing the cells individually into individual culture vessels, thereby providing a plurality of separate cell cultures; c) culturing the cells under a set of desired culture conditions using automated cell culture methods characterized in that the conditions are substantially identical for each of the separate cell cultures, during which culturing the number of cells per separate cell culture is normalized, and wherein the separate cultures are passaged on the same schedule; d) assaying the separate cell cultures to measure expression of the bitter receptor at least twice; and e) identifying a separate cell culture that expresses the bitter receptor at a consistent level in both assays, thereby obtaining said cell.

Optionally, cells that endogenously express a bitter receptor can be isolated from a population of cells as described herein. Such isolated cells can be used with the methods and compositions described herein, such as the screening methods and panels

Also disclosed herein is a method for defining the chemical space of compounds that modulate a protein complex, wherein the method comprises:

a) contacting a plurality of chemically diverse compounds separately with a cell that has been engineered to express the protein complex;
b) assaying the effects of the compounds on the activity of the protein complex;
c) correlating the effects obtained in step b) with structural commonalities of the compounds.

Also disclosed herein is a method for identifying a structural commonality among compounds that modulate a protein complex, wherein the method comprises:

(a) identifying compounds that modulate a protein complex according to steps a) and b) of the method described above;
(b) constructing a structure-activity relationship (SAR) model for each said compounds using a molecular representation of the respective compound and an activity profile of the respective compound, wherein said molecular representation of each said compounds comprises structural descriptors of the respective compound, wherein each said activity profile comprises quantitative measures of the effect of the respective compound on the biological activity of the protein complex, and wherein each said SAR model correlates structural features of the respective compound with the activity profile of the respective compound;
(c) identifying one or more structural features of each said compounds that correlates with the activity profile of the respective compound based on said SAR model for the respective compound; and
(d) identifying at least one structural feature common to said compounds from among the one or more structural features of each said compounds identified in step (c).

Also disclosed herein is a method for identifying a structural commonality among compounds that modulates a protein complex, wherein the method comprises:

(a) contacting, separately, a plurality of candidate compounds with a cell that has been engineered to express said protein complex;
(b) assaying the effects of each candidate compound of said plurality of compounds on the activity of said protein complex to provide an activity profile of each said candidate compound, wherein each said activity profile comprises quantitative measures of the effect of the respective candidate compound on the biological activity of the protein complex;
(c) identifying one or more candidate compounds that modulate the activity of said protein complex based on the activity profiles of said candidate compounds;
(d) constructing a structure-activity relationship (SAR) model for each said one or more candidate compounds identified in step (c) using a molecular representation of the respective candidate compound and the activity profile of the respective candidate compound, wherein said molecular representation of each said one or more candidate compounds comprises structural descriptors of the respective candidate compound, and wherein each said SAR model correlates structural features of the respective candidate compound with the activity profile of the respective candidate compound;
(e) identifying one or more structural features of each said one or more candidate compounds that correlates with the activity profile of the respective candidate compound based on said SAR model for the respective candidate compound; and
(f) identifying at least one structural feature common to said one or more candidate compounds from among the one or more structural features of each said respective candidate compound identified in step (e).

Optionally, said molecular representation of each said compounds further comprises physicochemical data of the respective compound, spatial data of the respective compound, topological data of the respective compound, or a combination thereof.

Optionally, the protein complex is a bitter receptor.

Optionally, said constructing said SAR model for each said compounds comprises applying a regression method to determine a relationship between said molecular representation of the respective compound and said activity profile of the respective compound.

Optionally, said SAR model for each said compounds is independently a receptor-dependent free energy force field QSAR (FEFF-QSAR) model, a receptor-independent three-dimensional QSAR (3D-QSAR), or a receptor-dependent or receptor-independent four-dimensional QSAR (4D-QSAR).

Also disclosed herein are kits that can be used in the methods described herein. Also disclosed herein are kits comprising one or more cells or cell lines stably expressing one or more complex targets. Optionally, kits provided herein comprise one or more signaling probes described herein. Optionally, a kit may comprise one or more vectors encoding one or more complex targets. Optionally, a kit comprises one or more dyes for use in functional cell-based assays (*e.g.,* calcium flux assay, membrane potential assay) to screen and select cells stably expressing one or more complex targets.

Also disclosed herein are kits comprising one or more containers filled with one or more of the reagents and/or cells described herein, such as one or more cells, vectors, and/or signaling probes provided herein. Optionally associated with such container(s) can be a notice containing instructions for using the components in the kit.

**Brief Description of the Drawings**

**Figures 1-3** are each a diagram of computer program modules that can be used in accordance with the present invention.

**Figure 4** contains four panels each showing dose response curves for several compounds each of which was tested against 4 different NaV 1.7 cell lines expressing NaV $\alpha$, $\beta$1 and $\beta$2 subunits.

**Figure 5** is a table showing the responses of different NaV 1.7 cell lines expressing NaV $\alpha$, $\beta$1 and $\beta$2 subunits to different doses of different compounds as grouped by bins.

**Figure 6** is a bar graph representation of the data obtained following an umami taste receptor-expressing (RNA) cell-based assay in which umami taste receptor-expressing cells were treated with either 12.5 mM fructose (a sweet agonist) or 25 mM MSG (an umami agonist) and were grown in various conditions, and the assay results from aliquots of the same cells tested in three of these conditions (1, 2 and "Final") are illustrated in this figure.

**Figure 7** is a graphic representation of a quantitative gene expression analysis of a cell line of this invention expressing umami receptor T1 R1 and T1 R3 subunits and a G$\alpha$15 protein. Total RNA was extracted from the cell line for TaqMan analysis of gene expression using gene-specific primers and probes for T1 R1, T1 R3, and G$\alpha$15. Relative expression levels over control cells are presented.

**Figure 8** displays representative gels that analyze the stability of umami receptor expression in cell lines after nine months in culture. Single endpoint RT-PCR was used to assess T1 R1, T1 R3 and G$\alpha$15 gene expression in one and nine month cultures of an umami taste receptor-expressing cell line. Reactions with and without reverse transcriptase ("+RT" or "-RT") and using control cells ("Control") or water only ("None") samples were also performed, and as a positive control the expression of a plasmid encoded neomycin resistance gene ("neo") was used. Arrows indicate reactions where positive results were expected. Due to the large number of reactions, data from different gels have been digitally juxtaposed. Data for T1 R3 are shown in its own panel as these reactions required independent PCR conditions.

**Figure 9** is a series of representative response traces generated after performing an umami taste receptor-expressing cell-based assay. The umami taste receptor-expressing cell-based assay was performed using buffer alone as a control (boxed wells) and the umami taste receptor agonist MSG at 33mM in the remaining wells The cell line yields a Z' value of > 0.8.

**Figure 10** is a line graph representing the data obtained following a dose response curve experiment using the umami taste receptor agonist MSG in an umami taste receptor-expressing cell-based assay. The responses of the umami taste receptor-expressing cell line and the control cells were plotted as a function of the agonist concentration.

**Figure 11** is a series of representative response traces generated after performing an umami taste receptor-expressing cell-based assay in the presence of various concentrations of MSG (1 mM-100mM, *right to left*) and the potentiator IMP (0mM-30mM, *bottom to top*).

**Figure 12** is a series of representative response traces generated after performing an umami taste receptor-expressing cell-based assay in the presence of various concentrations of sodium cyclamate.

**Figure 13** is a graph showing distinct functional activity ("Assay response" on y-axis) of native (circles) and tagged (squares) human bitter receptors in the presence of a range of concentrations of a bitter extract (x-axis).

**Figure 14** is a table showing that cell lines expressing a human bitter receptor showed up to 89% positive rate for functional receptor response. Each cell represents a well in a 96-well plate. Black boxes represent no cells/too few cells present. White boxes represent cells present, but no agonist signal above the background signal of that well. Gray boxes represent cells present, with agonist signal above background signal of that well.

**Figure 15** is a series of fluorescent micrographs of real-time imaging of bitter receptor response to a bitter agonist in (1) a bitter receptor-expressing cell line isolated according to the methods of the present invention (top panel) and in (2) drug-selected cells (bottom panel).

**Figure 16** is a graph showing dose response curves of relative responses to isoproterenol in 25 human bitter receptor-Ga15 cell lines.

**Figure 17** is a table showing broadly tuned, moderately tuned, and selective bitter receptors as identified in transient transfection assays.

**Figure 18** is a table showing the activity of different compounds at the 25 different human bitter receptors measured in functional cell-based assays and expressed as percent activity above the basal activity of the receptors.

**Figure 19** is a table showing different bitter receptor assignments using native cell lines (top row) and tagged cell lines (bottom row).

**Figure 20** is a bar graph representation of the sweet taste receptor-expressing (RNA) cell-based assay using either 12.5mM fructose (a sweet agonist) or 25mM MSG (an umami agonist) as a test compound. Cultures were grown in various conditions, and the assay results from aliquots of the same cells tested in three of these conditions (1, 2 and "Final") are illustrated in this figure. Assay response is normalized to control cell values.

**Figure 21** is a graphic representation of a quantitative gene expression analysis of a cell line of this invention expressing sweet taste receptor human T1 R2 and T1 R3 subunits (SEQ ID NOS: 31 and 32, respectively) and a mouse G$\alpha$15 protein (SEQ ID NO: 33). Total RNA was extracted from the cell line and analyzed by TaqMan for gene expression using gene-specific primers and probes for the human T1 R2 and T1 R3, and the mouse G$\alpha$15. Relative expression levels over control cells are presented.

**Figure 22** displays representative gels that analyze the stability of sweet receptor expression in cell lines of this invention after nine months in culture. Single endpoint RT-PCR was used to assess human T1 R2, human T1 R3 and mouse G$\alpha$15 gene expression in one and nine month cultures of a sweet taste receptor-expressing cell line. Reactions with and without reverse transcriptase ("+RT" or "-RT") and using control cells ("Control") or water only ("None") samples were also performed, and as a positive control the expression of a plasmid encoded-neomycin resistance gene ("neo") was used. Arrows indicate the lanes in which positive results were expected. Due to the large number of reactions, data from different gels have been digitally juxtaposed. Data for the human T1 R3 subunit are shown in its own panel as these reactions required independent PCR conditions.

**Figure 23** is a series of representative response traces generated after performing a sweet taste receptor-expressing cell-based assay (using cells of this invention) of a known agonist of the sweet taste receptor, fructose, at 75mM in alternating wells with buffer alone used as a control in the other wells. The cell line yields a Z' value of $\geq$ 0.8.

**Figure 24** (A-C) is series of line graphs representing the data obtained in various dose response experiments using sweet taste receptor agonists in a sweet taste receptor-expressing cell-based assay (using cells of this invention). (A) The responses of a sweet taste receptor-expressing cell line to natural caloric sweeteners were plotted as a function of the agonist concentration. (B) Dose response curves of common artificial sweeteners in a sweet taste receptor-expressing cell-based assay. (C) Dose response curves of natural high-intensity sweeteners in a sweet taste receptor-expressing cell-based assay.

**Figure 25** is a series of representative response traces generated after performing a sweet taste receptor-expressing cell-based assay using cells of this invention. In contrast to the typical bell-shaped GPCR response seen with most agonists, the stevia agonist showed extended response in the calcium flux FDSS assay.

**Figure 26** (A-B) depicts the genomic locus of T1 R2. **Figure 26A** depicts the position of the T1 R2 locus within Chromosome 1. **Figure 26B** depicts one possible intron-exon coding structure for the T1 R2 gene. The information was obtained from the genome browser of the Univresity of California, Santa Cruz. Exons and introns corresponding to T1 R2 are indicated numerically from the 5' to 3' direction. Exon numbers are indicated in black and intron numbers are indicated in grey. Scale and chromosomal position are indicated. The information was obtained from the genome browser of the Univresity of California, Santa Cruz.

**Figure 27** (A-B) depicts the genomic locus of T1 R3. **Figure 27A** depicts the position of the T1 R3 locus within Chromosome 1. The information was obtained from the genome browser of the University of California, Santa Cruz. **Figure 27B** depicts one possible intron-exon coding structure for the T1 R3 gene. Exons and introns corresponding to T1 R3 are indicated numerically from the 5' to 3' direction. Exon numbers are indicated in black and intron numbers are indicated in grey. Scale and chromosomal position are indicated. The information was obtained from the genome browser of the University of California, Santa Cruz.

**Figure 28** (A-B) depicts representative traces of the functional cell-based response of cells of the invention to the addition of fructose (to a final concentration of 15mM) compared to background. Cells cultured from individual isolated cells were tested (black traces) compared to control cells (gray traces). The cells demonstrated a higher response to fructose as demonstrated by subtracting the control response, or background, from both test and control cell samples. The cell-based assay was designed to report calcium flux using a fluorescent calcium signaling dye. Fluorescent assay response is plotted along the Y-axis and time is indicated along the X-axis. Arrows indicate the timepoint at which fructose was added. **Figure 28A** depicts traces from cells cultured from an individual HuTu cell compared to control. **Figure 28B** depicts traces from cells cultured from an individual H716 cell compared to control. **Figure 28C** depicts traces from cells cultured from an individual 293T cell compared to control.

**Figure 29** (A-B) depicts representative traces of the functional cell-based response of cells expressing a human odorant receptor to Helional and to Bourgeonal compared to background. **Figure 29A** depicts representative traces of the functional cell-based response of a cell line described herein expressing human odorant receptor OR3A1 to Helional (to a final concentration of 4.5 mM) compared to DMSO vehicle background signal as control. Test (black) and control (gray) traces are overlaid. The cells demonstrated a response to Helional over background. The cell-based assay was designed to report calcium flux using a fluorescent calcium signaling dye. Fluorescent assay response is plotted along the Y-axis and time is indicated along the X-axis. Arrows indicate the timepoint at which Helional or DMSO was added.

**Figure 29B** depicts representative traces of the functional cell-based response of a cell line described herein expressing human odorant receptor OR1 D2 to Bourgeonal (to a final concentration of 0.3 mM) compared to DMSO vehicle background signal as control. Test (black) and control (gray) traces are overlaid. The cells demonstrated a response to Bourgeonal over background. The cell-based assay was designed to report calcium flux using a fluorescent calcium signaling dye. Fluorescent assay response is plotted along the Y-axis and time is indicated along the X-axis. Arrows indicate the timepoint at which Bourgeonal or DMSO was added.

## Detailed Description

Unless otherwise defined, all technical and scientific terms used herein have the same meaning as commonly understood by one of ordinary skill in the art to which this invention belongs. Exemplary methods and materials are described below, although methods and materials similar or equivalent to those described herein can also be used in the practice or testing of the present invention. In case of conflict, the present specification, including definitions, will control.

Although a number of documents are cited herein, this citation does not constitute an admission that any of these documents forms part of the common general knowledge in the art.

Throughout this specification and claims, the word "comprise," or variations such as "comprises" or "comprising" will be understood to imply the inclusion of a stated integer or group of integers but not the exclusion of any other integer or group of integers. Unless otherwise required by context, singular terms shall include pluralities and plural terms shall include the singular. The materials, methods, and examples are illustrative only and not intended to be limiting.

In order that the present invention may be more readily understood, certain terms are first defined. Additional definitions are set forth throughout the detailed description.

The term "stable" or "stably expressing" is meant to distinguish the cells and cell lines of the invention from cells that transiently express proteins as the terms "stable expression" and "transient expression" would be understood by a person of skill in the art. As used herein, "not expressed in a cell of the same type" includes not expressed in any other cell of the same type, or in at least 99% of the cells of the same type, or in at least 99% of the cells of the same type, or in at least 98% of the cells of the same type, or in at least 97% of the cells of the same type, or in at least 96% of the cells of the same type, or in at least 95% of the cells of the same type, or in at least 94% of the cells of the same type, or in at least 93% of the cells of the same type, or in at least 92% of the cells of the same type, or in at least 91 % of the cells of the same type, or in at least 90% of the cells of the same type, or in at least 85% of the cells of the same type, or in at least 80% of the cells of the same type, or in at least 75% of the cells of the same type, or in at least 70% of the cells of the same type, or in at least 60% of the cells of the same type, or in at least 50% of the cells of the same type.

As used herein, a "functional" RNA or protein of interest is one that has a signal to noise ratio greater than 1:1 in a cell based assay. Optionally, a "functional" RNA or protein of interest has a signal to noise ratio is greater than 2. Optionally, a "functional" RNA or protein of interest has a signal to noise ratio is greater than 3. Optionally, a "functional" RNA or protein of interest has a signal to noise ratio is greater than 4. Optionally, a "functional" RNA or protein of interest has a signal to noise ratio is greater than 5. Optionally, a "functional" RNA or protein of interest has a signal to noise ratio is greater than 10. Optionally, a "functional" RNA or protein of interest has a signal to noise ratio is greater than 15. Optionally, a "functional" RNA or protein of interest has a signal to noise ratio is greater than 20. Optionally, a "functional" RNA or protein of interest has a signal to noise ratio is greater than 30. Optionally, a "functional" RNA or protein of interest has a signal to noise ratio is greater than 40. Optionally, a signal to noise ratio does not vary by more than 10%, 20% ,30%, 40%, 50%, 60% or 70%. Optionally, a signal to noise ratios does not vary by more than 10%, 20% ,30%, 40%, 50%, 60% or 70% from experiment to experiment. Optionally, a signal to noise ratio does not vary by more than 1%, 2%, 3%, 4%, 5%, 6%, 7%, 8%, 9% or 10% from experiment to experiment. Optionally, a signal to noise ratios does not vary by more than 10%, 20% ,30%, 40%, 50%, 60% or 70% between 2 to 20 different replicates of an experiment. Optionally, a signal to noise ratio does not vary by more than 1%, 2%, 3%, 4%, 5%, 6%, 7%, 8%, 9% or 10% between 2 to 20 different replicates of an experiment. Optionally, a signal to noise ratios does not vary by more than 10%, 20% ,30%, 40%, 50%, 60% or 70% for cells that are tested from 1 to 5, 5 to 10, 10 to 15, 15 to 20, 20 to 25, 25 to 30, 30 to 40, 40 to 50, 50 to 60, 60 to 70 or more than 70 days wherein the cells are in continuous culture. Optionally, a signal to noise ratio does not vary by more than 1%, 2%, 3%, 4%, 5%, 6%, 7%, 8%, 9% or 10% for cells that are tested from 1 to 5, 5 to 10, 10 to 15, 15 to 20, 20 to 25, 25 to 30, 30 to 40, 40 to 50, 50 to 60, 60 to 70 or more than 70 days wherein the cells are in continuous culture. Optionally, a functional protein or RNA of interest has one or more of the biological activities of the naturally occurring or endogenously expressed protein or RNA.

The term "cell line" or "clonal cell line" refers to a population of cells that is the progeny of a single original cell. As used herein, cell lines are maintained *in vitro* in cell culture and may be frozen in aliquots to establish banks of clonal cells.

The term "stringent conditions" or "stringent hybridization conditions" describe temperature and salt conditions for hybridizing one or more nucleic acid probes to a nucleic acid sample and washing off probes that have not bound specifically to target nucleic acids in the sample. Stringent conditions are known to those skilled in the art and can be found in, for example, Current Protocols in Molecular Biology, John Wiley & Sons, N.Y. (1989), 6.3.1-6.3.6. Aqueous and nonaqueous methods are described in the Protocols and either can be used. One example of stringent hybridization conditions is hybridization in 6X SSC at about 45°C, followed by at least one wash in 0.2X SSC, 0.1% SDS at 60°C. Another example of stringent hybridization conditions is hybridization in 6X SSC at about 45°C, followed by at least one wash in 0.2X SSC, 0.1% SDS at 65°C. Stringent hybridization conditions also include hybridization in 0.5M sodium phosphate, 7% SDS at 65°C, followed by at least one wash at 0.2X SSC, 1 % SDS at 65°C.

The phrase "percent identical" or "percent identity" in connection with amino acid and/or nucleic acid sequences refers to the similarity between at least two different sequences. The percent identity can be determined by standard alignment algorithms, for example, the Basic Local Alignment Tool (BLAST) described by Altshul et al. ((1990) J. Mol. Biol., 215: 403-410); the algorithm of Needleman et al. ((1970) J. Mol. Biol., 48: 444-453); or the algorithm of Meyers et al. ((1988) Comput. Appl. Biosci., 4: 11-17). A set of parameters may be the Blosum 62 scoring matrix with a gap penalty of 12, a gap extend penalty of 4, and a frameshift gap penalty of 5. The percent identity between two amino acid or nucleotide sequences can also be determined using the algorithm of E. Meyers and W. Miller ((1989) CABIOS, 4:11-17) that has been incorporated into the ALIGN program (version 2.0), using a PAM120 weight residue table, a gap length penalty of 12 and a gap penalty of 4. The percent identity is usually calculated by comparing sequences of similar length.

Protein analysis software matches similar amino acid sequences using measures of similarity assigned to various substitutions, deletions and other modifications, including conservative amino acid substitutions. For instance, the GCG Wisconsin Package (Accelrys, Inc.) contains programs such as "Gap" and "Bestfit" that can be used with default parameters to determine sequence identity between closely related polypeptides, such as homologous polypeptides from different species or between a wild type protein and a mutein thereof. *See, e.g.,* GCG Version 6.1. Polypeptide sequences also can be compared using FASTA using default or recommended parameters. A program in GCG Version 6.1. FASTA (*e.g.,* FASTA2 and FASTA3) provides alignments and percent sequence identity of the regions of the best overlap between the query and search sequences (Pearson, Methods Enzymol. 183:63-98 (1990); Pearson, Methods Mol. Biol. 132:185-219 (2000)).

The length of polypeptide sequences compared for identity will generally be at least about 16 amino acid residues, usually at least about 20 residues, more usually at least about 24 residues, typically at least about 28 residues, and preferably more than about 35 residues. The length of a DNA sequence compared for identity will generally be at least about 48 nucleic acid residues, usually at least about 60 nucleic acid residues, more usually at least about 72 nucleic acid residues, typically at least about 84 nucleic acid residues, and preferably more than about 105 nucleic acid residues.

The phrase "substantially as set out," "substantially identical" or "substantially homologous" in connection with an amino acid or nucleotide sequence means that the relevant amino acid or nucleotide sequence will be identical to or have insubstantial differences (*e.g.,* conserved amino acid substitutions or nucleic acids encoding such substitutions) in comparison to the comparator sequences. Insubstantial differences include minor amino acid changes, such as 1 or 2 substitutions in a 50 amino acid sequence of a specified region and the nucleic acids that encode those sequences.

Modulators include any substance or compound that alters an activity of a protein of interest, for example, a taste receptor (*e.g.,* bitter taste receptor, umami taste receptor, or sweet taste receptor). The modulator can be an agonist (potentiator or activator) or antagonist (inhibitor or blocker), including partial agonists or antagonists, selective agonists or antagonists and inverse agonists, and can also be an allosteric modulator. A substance or compound is a modulator even if its modulating activity changes under different conditions or concentrations or with respect to different forms of a protein of interest, for example, a taste receptor (*e.g.,* bitter taste receptor, umami taste receptor, or sweet taste receptor). In other aspects, a modulator may change the ability of another modulator to affect the function of a protein of interest, for example, a taste receptor (*e.g.,* bitter taste receptor, umami taste receptor, or sweet taste receptor). In specific embodiments, a modulator can alter the structure, conformation, biochemical or biophysical properties or functionality of a taste receptor (*e.g.,* bitter taste receptor, umami taste receptor, or sweet taste receptor), either positively or negatively.

The terms "potentiator", "agonist" or "activator" refer to a compound or substance that increases one or more activities of a protein of interest, for example, a taste receptor *(e.g.,* bitter taste receptor, umami taste receptor, or sweet taste receptor). In specific embodiments, terms "potentiator", "agonist" or "activator" in the context of taste receptors, refer to a compound or substance that increases the downstream signaling response associated with the taste receptor (*e.g.,* bitter taste receptor, umami taste receptor, or sweet taste receptor). Optionally, increasing taste receptor activity can result in change in the amount or distribution of an intracellular molecule or the activity of an enzyme which is part of the intracellular signaling pathway for the bitter receptor. Examples of the intracellular molecule include, but are not limited to, free calcium, cyclic adenosine monophosphate (cAMP), inositol mono-, di- or tri-phosphate. Examples of the enzyme include, but are not limited to, adenylate cyclase, phospholipase-C, G-protein coupled receptor kinase.

The terms "inhibitor", "antagonist" or "blocker" refer to a compound or substance that decreases or blocks one or more activities of a protein of interest, for example, a taste receptor (*e.g.,* bitter taste receptor, umami taste receptor, or sweet taste receptor). In specific embodiments, terms "inhibitor", "antagonist" or "blocker" in the context of taste receptors, refer to a compound or substance that decreases the downstream signaling response associated with the taste receptor (*e.g.,* bitter taste receptor, umami taste receptor, or sweet taste receptor). Optionally, decreasing taste receptor activity can result in change in the amount or distribution of an intracellular molecule or the activity of an enzyme which is part of the intracellular signaling pathway for the bitter receptor. Examples of the intracellular molecule include, but are not limited to, free calcium, cyclic adenosine monophosphate (cAMP), inositol mono-, di- or tri-phosphate. Examples of the enzyme include, but are not limited to, adenylate cyclase, phospholipase-C, G-protein coupled receptor kinase.

A sweet taste receptor is a protein that is present in many mammalian tissues, including epithelial cells of the mouth, the lung and the intestine. Without being bound by theory, we believe that sweet taste receptor dysregulation or dysfunction may be linked to many disease states including diabetes and obesity.

The phrase "functional sweet taste receptor" refers to a sweet taste receptor that comprises at least a T1 R2 and a T1 R3 subunit and that responds to a known activator, such as fructose, glucose, sucrose, monellin/miraculin, mogroside, steviva, rebaudioside A, saccharin, asparatame, sodium cyclamate, sucralose, sorbital, acesulfame K or Gymnema Sylvestre, or a known inhibitor, such as methyl 4,6-dichloro-4,6-dideoxy-$\alpha$-D-galactopyranoside (MAD-diCl-Gal), PNP/lactisole (may act differently at different concentrations), Gymnemic acid 1, hoduloside, Ziziphin, and gurmarin in a similar way (*i.e.* at least 50%, 60%, 70%, 80% 90% and 95% the same) as a sweet taste receptor produced in a cell that normally expresses that receptor without genetic engineering of the cell to produce it. Sweet taste receptor behavior can be determined by, for example, physiological activities or pharmacological responses. Physiological activities include, but are not limited to activation of a G protein and associated downstream signaling. Pharmacological responses include, but are not limited to, inhibition, activation, and modulation of the receptor. Such responses can, for example, be assayed in an assay that monitors intracellular calcium release from the endoplasmic reticulum upon $G_{\alpha q}$ protein activation by an activated sweet taste receptor.

An umami taste receptor is a protein that is present in many mammalian tissues, including epithelial cells of the mouth, the lung and the intestine. Without being bound by theory, we believe that umami taste receptor dysregulation or dysfunction may be linked to many disease states including diabetes and obesity.

The phrase "functional umami taste receptor" refers to an umami taste receptor that comprises at least a T1 R1 and a T1 R3 subunit and that responds to a known activator, such as monosodium glutamate (MSG), or a known inhibitor, such as lactisole, which is known to act as an umami taste receptor inhibitor at specific concentrations, or PMP (2-(4-methoxyphenoxy)-propionic acid) in a similar way (*i.e.* at least 50%, 60%, 70%, 80% 90% and 95% the same) as an umami taste receptor produced in a cell that normally expresses that receptor without genetic engineering of the cell to produce it. Umami taste receptor behavior can be determined by, for example, physiological activities or pharmacological responses. Physiological activities include, but are not limited to activation of a G protein and associated downstream signaling. Pharmacological responses include, but are not limited to, inhibition, activation, and modulation of the receptor. Such responses can, for example, be assayed in an assay that monitors intracellular calcium release from the endoplasmic reticulum upon $G_{\alpha q}$ protein activation by an activated umami taste receptor.

The phrase "functional bitter receptor" refers to a bitter receptor that responds to a known activator or a known inhibitor in substantially the same way as the bitter receptor in a cell that normally expresses the bitter receptor without engineering. Bitter receptor behavior can be determined by, for example, physiological activities and pharmacological responses. Physiological activities include, but are not limited to, the sense of bitter taste. Pharmacological responses include, but are not limited to, a change in the amount or distribution of an intracellular molecule or the activity of an enzyme which is part of the intracellular signaling pathway for the bitter receptor when a bitter receptor is contacted with a modulator. For example, a pharmacological response may include an increase in intracellular free calcium when the bitter receptor is activated, or a decrease in intracellular free calcium when the bitter receptor is blocked.

The term "bitter receptor", as used herein, refers to any one of the G protein coupled receptors that is expressed at the surface of a taste receptor cell and that mediates bitter taste perception via secondary messenger pathways.

A "heterologous" or "introduced" protein of interest, for example, a taste receptor subunit (*e.g.,* bitter taste receptor subunit, umami taste receptor subunit, or sweet taste receptor subunit) or G protein, means that the protein of interest, for example, a taste receptor subunit (*e.g.,* bitter taste receptor subunit, umami taste receptor subunit, or sweet taste receptor subunit) or G protein is encoded by a nucleic acid introduced into a host cell.

A "gene activated" protein of interest, for example, a taste receptor subunit (*e.g.,* bitter taste receptor subunit, umami taste receptor subunit, or sweet taste receptor subunit) or G protein, means that an endogenous nucleic acid encoding the subunit or protein has been activated for expression by the introduction and operative linking of an expression control sequence to that nucleic acid.

Disclosed herein are novel cetts and cell tines produced from the cells that meet the urgent need for cells that stably express a functional RNA of interest or a functional protein of interest, including complex proteins such as heteromultimeric proteins and proteins for which no ligand is known. The cells and cell lines disclosed herein are suitable for any use in which consistent, functional expression of an RNA or protein of interest are desirable. Applicants have produced cell lines meeting this description for a variety of proteins, both single subunit and heteromultimeric (including heterodimeric and proteins with more than two different subunits), including membrane proteins, cytosolic proteins and secreted proteins, as well as various combinations of these.

Examples of a protein of interest include, but are not limited to: receptor (*e.g.,* cytokine receptor, immunoglobulin receptor family member, ligand-gated ion channel, protein kinase receptor, G-protein coupled receptor (GPCR), nuclear hormone receptor and other receptors), signaling molecule (*e.g.,* cytokine, growth factor, peptide hormone, chemokine, membrane-bound signaling molecule and other signaling molecules), kinase (*e.g.,* amino acid kinase, carbohydrate kinase, nucleotide kinase, protein kinase and other kinases), phosphatase (*e.g.,* carbohydrate phosphatase, nucleotide phosphatase, protein phosphatase and other phosphatases), protease (*e.g.,* aspartic protease, cysteine protease, metalloprotease, serine protease and other proteases), regulatory molecule (*e.g.,* G-protein modulator, large G-protein, small GTPase, kinase modulator, phosphatase modulator, protease inhibitor and other enzyme regulator), calcium binding protein (*e.g.,* annexin, calmodulin related protein and other select calcium binding proteins), transcription factor (*e.g.,* nuclear hormone receptor, basal transcription factor, basic helix-loop-helix transcription factor, creb transcription factor, hmg box transcription factor, homeobox transcription factor, other transcription factor, transcription cofactor and zinc finger transcription factor), nucleic acid binding protein (*e.g.,* helicase, DNA ligase, DNA methyltransferase, RNA methyltransferase, double-stranded DNA binding protein, endodeoxyribonuclease, replication origin binding protein, reverse transcriptase, ribonucleoprotein, ribosomal protein, single-stranded DNA-binding protein, centromere DNA-binding protein, chromatin/chromatin-binding protein, DNA glycosylase, DNA photolyase, DNA polymerase processivity factor, DNA strand-pairing protein, DNA topoisomerase, DNA-directed DNA polymerase, DNA-directed RNA polymerase, damaged DNA-binding protein, histone, primase, endoribonuclease, exodeoxyribonuclease, exoribonuclease, translation elongation factor, translation initiation factor, translation release factor, mRNA polyadenylation factor, mRNA splicing factor, other DNA-binding proteins, other RNA-binding proteins and other nucleic acid binding proteins), ion channel (*e.g.,* anion channel, ligand-gated ion channel, voltage-gated ion channel and other ion channels), transporter (*e.g.,* cation transporter, ATP-binding cassette (ABC) transporter, amino acid transporter, carbohydrate transporter and other transporters), transfer/carrier protein (*e.g.,* apolipoprotein, mitochondrial carrier protein and other transfer/carrier proteins), cell adhesion molecule (*e.g.,* cam family adhesion molecule, cadherin and other cell adhesion molecule), cytoskeletal protein (*e.g.,* actin and actin related protein, actin binding motor protein, non-motor actin binding protein, other actin family cytoskeletal protein, intermediate filament, microtubule family cytoskeletal protein and other cytoskeletal proteins), extracellular matrix (*e.g.,* extracellular matrix glycoprotein, extracellular matrix linker protein, extracellular matrix structural protein and other extracellular matrix), cell junction protein (*e.g.,* gap junction protein, tight junction protein and other cell junction proteins), synthase, synthetase, oxidoreductase (*e.g.,* dehydrogenase, hydroxylase, oxidase, oxygenase, peroxidase, reductase and other oxidoreductase), transferase (*e.g.,* methyltransferase, acetyltransferase, acyltransferase, glycosyltransferase, nucleotidyltransferase, phosphorylase, transaldolase, transaminase, transketolase and other transferase), hydrolyase (*e.g.,* deacetylase, deaminase, esterase, galactosidase, glucosidase, glycosidase, lipase, phosphodiesterase, pyrophosphatase, amylase and other hydrolase), lysase (*e.g.,* adenylate cyclase, guanylate cyclase, aldolase, decarboxylase, dehydratase, hydratase and other lyases), isomerase (*e.g.,* epimerase/racemase, mutase and other isomerases), ligase (*e.g.,* DNA ligase, ubiquitin-protein ligase and other ligases), defense/immunity protein *(e.g.,* antibacterial response protein, complement component, immunoglobulin, immunoglobulin receptor family member, major histocompatibility complex antigen and other defense and immunity proteins), membrane traffic protein (*e.g.,* membrane traffic regulatory protein, SNARE protein, vesicle coat protein and other membrane traffic proteins), chaperone (*e.g.,* chaperonin, hsp 70 family chaperone, hsp 90 family chaperone and other chaperones), viral protein (*e.g.,* viral coat protein and other viral proteins), myelin protein, other miscellaneous function protein, storage protein, structural protein, surfactant, and transmembrane receptor regulatory/adaptor protein. Other examples of proteins and their functions include those identified in Paul D. Thomas, Michael J. Campbell, Anish Kejariwal, Huaiyu Mi, Brian Karlak, Robin Daverman, Karen Diemer, Anushya Muruganujan, Apurva Narechania. 2003. PANTHER: a library of protein families and subfamilies indexed by function. Genome Res., 13: 2129-2141, which is incorporated herein by reference in its entirety.

Examples of GPCRs include, but are not limited to:

Class A GPCRs, including, but not limited to:

5-Hydroxytryptamine receptors (*e.g.,* HTR1A, HTR1B, HTR1D, HTR1E, HTR1F, HTR2A, HTR2B, HTR2C, HTR4, HTR5A, HTR6, and HTR7), Muscarinic acetylcholine receptors (*e.g.,* CHRM1, CHRM2, CHRM3, CHRM4, and CHRM5), Adenosine receptors (*e.g.,* ADORA1, ADORA2A, ADORA2B, and ADORA3), Alpha-Adrenoceptors (*e.g.,* ADRA1A, ADRA1B, ADRA1D, ADRA2A, ADRA2B, and ADRA2C), Beta-Adrenoceptors (*e.g.,* ADRB1,ADRB2, and ADRB3), Anaphylatoxin receptors (*e.g.,* GPR77, C5R1, and C3AR1), Angiotensin receptors *(e.g.,* AGTR1 and AGTR2), Apelin receptor (*e.g.,* AGTRL1), Bile acid receptor *(e.g.,* GPBAR1), Bombesin receptors (*e.g.,* NMBR, GRPR, and BRS3), Bradykinin receptors *(e.g.,* BDKRB1 and BDKRB2), Cannabinoid receptors (*e.g.,* CNR1 and CNR2), Chemokine receptors- Interleukin (*e.g.,* 8IL8RA and IL8RB), Chemokine receptors (*e.g.,* CXCR3, CXCR4, CXCR5, CCR1, CCR2, CCR3, CCR4, CCR5, CCR6, CCR7, CCR8, CCR9, CCR10, CX3CR1, XCR1, and CXCR6), Cholecystokinin receptors (*e.g.,* CCKAR and CCKBR), Dopamine receptors (*e.g.,* DRD1, DRD2, DRD3, DRD4, and DRD5), Endothelin receptors (*e.g.,* EDNRA and EDNRB), Estrogen receptor (*e.g.,* GPER), Formylpeptide receptors (*e.g.,* FPR2, FPR3, and FPR1), Free fatty acid receptors (*e.g.,* FFAR1, FFAR3, FFAR2, and GPR42), Galanin receptors *(e.g.,* GALR1, GALR2, and GALR3), Ghrelin receptor (*e.g.,* GHSR), Glycoprotein hormone receptors *(e.g.,* FSHR, LHCGR, and TSHR), Gonadotrophin-releasing hormone receptors *(e.g.,* GNRHR and GNRHR2), Histamine receptors (*e.g.,* HRH1, HRH2, HRH3, and HRH4), KiSS1-derived peptide receptor (*e.g.,* KISS1R), Leukotriene receptors (*e.g.,* LTB4R2, FPRL1, OXER1, LTB4R, CYSLTR1, and CYSLTR2), Lysophospholipid receptors (*e.g.,* LPAR1, LPAR2, LPAR3, S1 PR1, S1 PR2, S1 PR3, S1 PR4, and S1 PR5), Melanin-concentrating hormone receptors (*e.g.,* MCHR1 and MCHR2), Melanocortin receptors (*e.g.,* MC1 R, MC2R, MC3R, MC4R, and MC5R), Melatonin receptors *(e.g.,* MTNR1A and MTNR1B), Motilin receptor *(e.g.,* MLNR), Neuromedin U receptors (*e.g.,* NMUR1 and NMUR2), Neuropeptide FF/neuropeptide AF receptors (*e.g.,* NPFFR1 and NPFFR2), Neuropeptide S receptor *(e.g.,* NPSR1), Neuropeptide W/neuropeptide B receptors (*e.g.,* NPBWR1 and NPBWR2), Neuropeptide Y receptors (*e.g.,* NPY1 R, NPY2R, PPYR1, and NPY5R), Neurotensin receptors (*e.g.,* NTSR1 and NTSR2), Nicotinic acid receptor family (*e.g.,* GPR109B, GPR109A, and GPR81), Non-signalling 7TM chemokine-binding proteins (*e.g.,* DARC, CCBP2, and CCRL1), Opioid receptors (*e.g.,* OPRM1, OPRD1, OPRK1, and OPRL1), Orexin receptors (*e.g.,* HCRTR1 and HCRTR2), P2Y receptors (*e.g.,* P2RY1, P2RY2, P2RY4, P2RY6, P2RY11, P2RY12, P2RY14, and P2RY13), Peptide P518 receptor (*e.g.,* QRFPR), Platelet-activating factor receptor (*e.g.,* PTAFR), Prokineticin receptors (*e.g.,* PROKR1 and PROKR2), Prolactin-releasing peptide receptor (*e.g.,* PRLHR), Prostanoid receptors (*e.g.,* PTGDR, PTGER1, PTGER2, PTGER3, PTGER4, PTGFR, PTGIR, TBXA2R, and GPR44), Protease-activated receptors (*e.g.,* Thrombin (F2R)), Protease-activated receptors (*e.g.,* F2RL1, F2RL2, and F2RL3), Relaxin family peptide receptors (*e.g.,* RXFP1, RXFP2, RXFP3, and RXFP4), Somatostatin receptors (*e.g.,* SSTR2, SSTR5, SSTR3, SSTR1, and SSTR4), Tachykinin receptors (*e.g.,* TACR1, TACR2, and TACR3), Thyrotropin-releasing hormone receptor (*e.g.,* TRHR), Trace amine receptor (*e.g.,* TAAR1), Urotensin receptor (*e.g.,* UTS2R), Vasopressin and oxytocin receptors (*e.g.,* AVPR1A, AVPR2, AVPR1B, and OXTR), Class A Orphans(*e.g.,* GPR82, GPR182, CCRL2, CMKLR1, CMKOR1, GPR183, GPR1, GPR3, GPR4, GPR6, GPR12, GPR15, GPR17, GPR18, GPR19, GPR20, GPR21, GPR22, GPR23, GPR25, GPR26, GPR27, GPR31, GPR32, GPR33, GPR34, GPR35, GPR37, GPR37L1, GPR39, GPR45, GPR50, GPR52, GPR55, GPR61, GPR62, GPR63, GPR65, GPR68, GPR75, GPR78, GPR79, GPR83, GPR84, GPR85, GPR87, GPR88, GPR92, GPR101, GPR119, GPR120, GPR132, GPR135, GPR139, GPR141, GPR142, GPR146, GPR148, GPR149, GPR150, GPR151, GPR152, GPR153, GPR160, GPR161, GPR171, GPR173, GPR174, GPR162, LGR4, LGR5, LGR6, MAS1, MAS1L, MRGPRD, MRGPRE, MRGPRF, MRGPRG, MRGPRX1, MRGPRX2, MRGPRX3, MRGPRX4, OPN3, OPN5, OXGR1, P2RY10, P2RY5, P2RY8, SUCNR1, TAAR2, TAAR3, TAAR5, TAAR6, TAAR8, TAAR9, and GPR42);

Class B GPCRs, including, but not limited to:

Calcium-sensing receptors *(e.g.,* CASR and GPRC6A),GABA-B receptors *(e.g.,* GABBR1 and GABBR2), GPRC5 receptors (*e.g.,* GPRC5A, GPRC5B, GPRC5C, and GPRC5D), Metabotropic glutamate receptors (*e.g.,* GRM1, GRM2, GRM3, GRM4, GRM5, GRM6, GRM7, and GRM8,), Class C Orphans (*e.g.,* GPR156, GPR158, GPR179, GPRC5A, GPRC5B, GPRC5C, and GPRC5D);

Class C GPCRs, including, but not limited to:

Calcitonin receptors *(e.g.,* CALCR/CT, AMY1, AMY2, AMY3, CALCRL, CGRP, AM1, and AM2), Corticotropin-releasing factor receptors (*e.g.,* CRHR1 and CRHR2), Glucagon receptor family (*e.g.,* GCGR, GLP1R, GLP2R, GIPR, SCTR, and GHRHR), Parathyroid hormone receptors (*e.g.,* PTH1 R and PTHR2), VIP and PACAP receptors *(e.g.,* ADCYAP1R1, VIPR1, and VIPR2), Class B Orphans *(e.g.,* BAI1, BAI2, BAI3, CD97, CELSR1, CELSR2, CELSR3, ELTD1, EMR1, EMR2, EMR3, EMR4, GPR56, GPR64, GPR97, GPR110, GPR111 ,GPR112, GPR113, GPR114, GPR115, GPR116, GPR123, GPR124, GPR125, GPR126, GPR128, GPR133, GPR143, GPR144, GPR157, LPHN1, LPHN2, LPHN3, and GPR98);

Class D GPCRs, including, but not limited to, fungal mating pheromone receptors (*e.g.,* STE2 and STE3);
Class E GPCRs, including, but not limited to, cAMP receptors (*e.g.,* Dictyostelium);
Class F GPCRs, including, but not limited to, frizzled receptors (*e.g.,* FZD1, FZD2, FZD3, FZD4, FZD5, FZD6, FZD7, FZD8, FZD9, FZD10, and SMO); and

Unclassified GPCRs (*e.g.,* OPCML,OGFR, OGFRL1, and OPRS1).
Examples of voltage-gated ion channels include, but are not limited to:

Calcium-activated potassium channels, including, but not limited to, KCNMA1, KCNN1,KCNN2, KCNN3, KCNN4, KCNT1, KCNT2, and KCNU1;
CatSper and two-pore channels, including, but not limited to, CATSPER1, CATSPER2, CATSPER3, CATSPER4, TPCN1, and TPCN2;
Cyclic nucleotide-regulated channels, including, but not limited to, CNGA1, CNGA2, CNGA3, CNGA4, CNGB1, CNGB3, HCN1, HCN2, HCN3, and HCN4;
Inwardly rectifying potassium channels, including, but not limited to, KCNJ1, KCNJ2, KCNJ12, KCNJ4, KCNJ14, KCNJ3, KCNJ6, KCNJ9, KCNJ5, KCNJ10, KCNJ15, KCNJ16, KCNJ8, KCNJ11, and KCNJ13;
Transient receptor potential channels, including, but not limited to, TRPA1, TRPC1, TRPC2, TRPC3, TRPC4, TRPC5, TRPC6, TRPC7, TRPM1, TRPM2, TRPM3, TRPM4, TRPM5, TRPM6, TRPM7, TRPM8, MCOLN1, MCOLN2, MCOLN3, PKD2, PKD2L1, PKD2L2, TRPV1, TRPV2, TRVP3, TRPV4, TRPV5, and TRPV6;
Two-P potassium channels, including, but not limited to, KCNK1, KCNK2, KCNK3, KCNK4, KCNK5, KCNK6, KCNK7, KCNK9, KCNK10, KCNK12, KCNK13, KCNK15, KCNK16, KCNK17, and KCNK18;
Voltage-gated calcium channels, including, but not limited to, CACNA1S, CACNA1C, CACNA1D, CACNA1F, CACNA1A, CACNA1B, CACNA1E, CACNA1G, CACNA1H, and CACNA1I;
Voltage-gated potassium channels, including, but not limited to, KCNA1, KCNA2, KCNA3, KCNA4, KCNA5, KCNA6, KCNA7, KCNA10, KCNB1, KCNB2, KCNC1, KCNC2, KCNC3, KCNC4, KCND1, KCND2, KCND3, KCNF1, KCNG1, KCNG2, KCNG3, KCNG4, KCNQ1, KCNQ2, KCNQ3, KCNQ4, KCNQ5, KCNV1, KCNV2, KCNS1, KCNS2, KCNS3, KCNH1, KCNH5, KCNH2, KCNH6, KCNH7, KCNH8, KCNH3, and KCNH4;
Voltage-gated sodium channels, including, but not limited to, SCN1 A, SCN2A, SCN3A, SCN4A, SCN5A, SCN8A, SCN9A, SCN10A, and SCN11 A; and
Other voltage-gated ion channels, including, but not limited to, KCNE1, KCNE1L, KCNE2, KCNE3, KCNIP1, KCNIP2, KCNIP3, KCNIP4, KCNMB1, KCNMB2, KCNMB3, CNMB3L, and KCNMB4.
Examples of ligand gated ion channels include, but are not limited to:
Serotonin receptor subunits, including, but not limited to, 5HT3Acapo, 5HT3Ahosa, 5HT3Amumu, 5HT3Amupu, 5HT3Aorcu, 5HT3Apatr, 5HT3Arano, 5HT3Bhosa, 5HT3Bmumu, 5HT3Borcu, 5HT3Bpatr, 5HT3Brano, 5HT3Chosa, 5HT3Cpatr, 5HT3Dhosa, 5HT3Dpatr, 5HT3Ehosa, 5HT3gaga, and 5HTmod1cael; 5-HT receptors including: 5-HT1 A; 5-HT1 B; 5-HT1D; 5-HT1 E; 5-HT1 F; 5-HT2A; 5-HT2B; 5-HT2C; 5HT4 splice isoforms a, b, c, d, e, f, g, n; 5-HT5A; 5-HT6; 5-HT7 splice forms a, b, c.
Acetylcholine receptor subunits, including, but not limited to, ACHa10gaga, ACHa10hosa, ACHa10mumu, ACHa10patr, ACHa10rano, ACHa1anga, ACHa1apca, ACHa1ataru, ACHa1axela, ACHa1bota, ACHa1btaru, ACHa1bxela, ACHa1cafa, ACHa1dare, ACHa1gaga, ACHa1hevi, ACHa1hosa, ACHa1lomi, ACHa1mumu, ACHa1mype, ACHa1naha, ACHa1nana, ACHa1nate, ACHa1patr, ACHa1rano, ACHa1 toca, ACHa1 toma, ACHa2anga, ACHa2apca, ACHa2apgo, ACHa2dare, ACHa2gaga, ACHa2hevi, ACHa2hosa, ACHa2lomi, ACHa2mamu, ACHa2mumu, ACHa2mype, ACHa2patr, ACHa2rano, ACHa2taru, ACHa3anga, ACHa3apme, ACHa3bota, ACHa3caau, ACHa3drme, ACHa3gaga, ACHa3hevi, ACHa3hosa, ACHa3lomi, ACHa3mamu, ACHa3mumu, ACHa3mype, ACHa3patr, ACHa3rano, ACHa3taru, ACHa4anga, ACHa4drme, ACHa4gaga, ACHa4hosa, ACHa4mamu, ACHa4mumu, ACHa4mype, ACHa4patr, ACHa4rano, ACHa4taru, ACHa5anga, ACHa5drme, ACHa5gaga, ACHa5hosa, ACHa5mamu, ACHa5mumu, ACHa5mype, ACHa5patr, ACHa5rano, ACHa6ataru, ACHa6btaru, ACHa6drme, ACHa6gaga, ACHa6hosa, ACHa6mamu, ACHa6mumu, ACHa6patr, ACHa6rano, ACHa7_1hevi, ACHa7_2hevi, ACHa7anga, ACHa7ataru, ACHa7bota, ACHa7btaru, ACHa7ctaru, ACHa7dare, ACHa7drme, ACHa7gaga, ACHa7hosa, ACHa7mamu, ACHa7mumu, ACHa7patr, ACHa7rano, ACHa7rasp, ACHa8ataru, ACHa8btaru, ACHa8gaga, ACHa9ataru, ACHa9btaru, ACHa9ctaru, ACHa9dare, ACHa9dtaru, ACHa9gaga, ACHa9hosa, ACHa9mumu, ACHa9patr, ACHa9rano, ACHaassu, ACHacr10cael, ACHacr11cael, ACHacr12cael, ACHacr13cael, ACHacr14cael, ACHacr15cael, ACHacr16cael, ACHacr17cael, ACHacr18cael, ACHacr19cael, ACHacr20cael, ACHacr21cael, ACHacr22cael, ACHacr23cael, ACHacr2cael, ACHacr3cael, ACHacr4cael, ACHacr5cael, ACHacr6cael, ACHacr7cael, ACHacr8cael, ACHacr9cael, ACHahaco, ACHal1acdo, ACHal1scgr, ACHalsdrme, ACHalsmase, ACHalyst, ACHarddrme, ACHb1ataru, ACHb1bota, ACHb1btaru, ACHb1hevi, ACHb1hosa, ACHb1mase, ACHb1mumu, ACHb1patr, ACHb1rano, ACHb1toca, ACHb1xela, ACHb2caau, ACHb2gaga, ACHb2hosa, ACHb2mamu, ACHb2mumu, ACHb2patr, ACHb2rano, ACHb3acaau, ACHb3adare, ACHb3bcaau, ACHb3gaga, ACHb3hosa, ACHb3mamu, ACHb3mumu, ACHb3patr, ACHb3rano, ACHb4bota, ACHb4gaga, ACHb4hosa, ACHb4mamu, ACHb4mumu, ACHb4patr, ACHb4rano, ACHb4taru, ACHb5taru, ACHb6taru, ACHb7taru, ACHblomi, ACHblyst, ACHc04c3_2cael, ACHc15a7_1cael, ACHcg7589drme, ACHclyst, ACHcup4cael, ACHdbota, ACHddare, ACHdeg3cael, ACHdgaga, ACHdhosa, ACHdlyst, ACHdmumu, ACHdpatr, ACHdrara, ACHdtaru, ACHdtoca, ACHdxela, ACHeat2cael, ACHebota, ACHehosa, ACHelyst, ACHemumu, ACHepatr, ACHerara, ACHetaru, ACHexela, ACHf11c7_1cael, ACHf17e9_7cael, ACHf17e9_8cael, ACHf18g5_4cael, ACHf21a3_7cael, ACHf58h7_3cael, ACHflyst, ACHgbota, ACHggaga, ACHghosa, ACHglyst, ACHgmumu, ACHgpatr, ACHgrara, ACHgtaru, ACHgtoca, ACHgxela, ACHhlyst, ACHilyst, ACHjlyst, ACHklyst, ACHlev1cael, ACHllyst, ACHnaapca, ACHr03e1_3cael, ACHr13a5_4cael, ACHronvo, ACHsaddrme, ACHsbddrme, ACHssu1osci, ACHssu2osci, ACHt01h10_1cael, ACHt01h10_2cael, ACHt01h10_3cael, ACHt01h10_5cael, ACHt01 h10_6cael, ACHt01h10_7cael, ACHt05b4_1cael, ACHtar1trco, ACHunc29cael, ACHunc38cael, ACHunc63cael, ACHy44a6e 1 cael, ACHy57g11c_2cael, ACHy57g11c_49cael, ACHy58g8a_1cael, ACHy73b6bl_26cael, and ACHy73f8a_30cael;

GABAA receptor subunits, inclucing, but not limited to, GABa1bota, GABa1gaga, GABa1hosa, GABa1mumu, GABa1rara, GABa2bota, GABa2hosa, GABa2mumu, GABa2rara, GABa3bota, GABa3hevi, GABa3hosa, GABa3mumu, GABa3rara, GABa4bota, GABa4hosa, GABa4mumu, GABa4rara, GABa5hosa, GABa5rara, GABa6caau, GABa6hosa, GABa6mumu, GABa6rara, GABb1bota, GABb1hosa, GABb1mumu, GABb1rasp, GABb2dare, GABb2hosa, GABb2mumu, GABb2rasp, GABb3gaga, GABb3hosa, GABb3mumu, GABb3rasp, GABb4gaga, GABbdrme, GABblyst, GABbseof, GABc09g5_1 cael, GABc27h5_4cael, GABc39b10_2cael, GABc53d6_3cael, GABdhosa, GABdmumu, GABdrara, GABehosa, GABemumu, GABerano, GABf09c12_1cael, GABf11h8_2cael, GABf47a4_1cael, GABf55d10_5cael, GABf58g6_4cael, GABg1gaga, GABg1hosa, GABg1mumu, GABg1rano, GABg2bota, GABg2gaga, GABg2hosa, GABg2mumu, GABg2rara, GABg3hosa, GABg3mumu, GABg3rano, GABg4gaga, GABg4hosa, GABg4mumu, GABgbr2cael, GABgrddrme, GABhg1haco, GABk10d6_1cael, GABphosa, GABpmumu, GABr1amoam, GABr1bmoam, GABr1hosa, GABr1mumu, GABr1rano, GABr2amoam, GABr2bmoam, GABr2hosa, GABr2mumu, GABr2rara, GABr3hosa, GABr3moam, GABr3mumu, GABr3rano, GABrdlaeae, GABrdlceca, GABrdldrme, GABt20b12_9cael, GABt21f2_1cael, GABt24d8_1cael, GABthosa, GABtmumu, GABtrano, GABunc49bcael, GABunc49cael, and GABzlyst;

Glycine/Histamine receptor subuntis, including, but not limited to, GLYa1dare, GLYa1hosa, GLYa1mumu, GLYa1rano, GLYa2dare, GLYa2hosa, GLYa2mumu, GLYa2rano, GLYa3dare, GLYa3hosa, GLYa3moam, GLYa3mumu, GLYa3rano, GLYa4adare, GLYa4bdare, GLYa4hosa, GLYa4mumu, GLYbdare, GLYbhosa, GLYbmumu, GLYbrano,and HIScl1drme;

ATP receptor subunits, including, but not limited to, ATPp2x1 hosa, ATPp2x1 rano, ATPp2x2capo, ATPp2x2hosa, ATPp2x2mumu, ATPp2x2rano, ATPp2x3hosa, ATPp2x3mumu, ATPp2x3rano, ATPp2x4bota, ATPp2x4gaga, ATPp2x4hosa, ATPp2x4mumu, ATPp2x4orcu, ATPp2x4rano, ATPp2x5bota, ATPp2x5hosa, ATPp2x5mumu, ATPp2x5rano, ATPp2x6hosa, ATPp2x6mumu, ATPp2x6rano, ATPp2x7bota, ATPp2x7hosa, ATPp2x7mumu, ATPp2x7rano, and ATPp2xscma;

Glutamate receptor subunits, including, but not limited to, GLU1_1arth, GLU1_2arth, GLU1_3arth, GLU1_4arth, GLU2_1arth, GLU2_2arth, GLU2_3arth, GLU2_4arth, GLU2_5arth, GLU2_6arth, GLU2_7arth, GLU2_8arth, GLU2_9arth, GLU3_1arth, GLU3_2arth, GLU3_3arth, GLU3_4arth, GLU3_5arth, GLU3_6arth, GLU3_7arth, GLUd1hosa, GLUd1mumu, GLUd1rano, GLUd2hosa, GLUd2mumu, GLUd2rano, GLUglr10cael, GLUglr1cael, GLUglr2cael, GLUglr4cael, GLUglr5cael, GLUglr6cael, GLUglr7cael, GLUglr8cael, GLUglr9cael, GLUk10d3_1cael, GLUka1hosa, GLUka1mumu, GLUka1rano, GLUka2hosa, GLUka2mumu, GLUka2rano, GLUka4mumu, GLUkbpacaau, GLUkbpansp, GLUkbpbcaau, GLUkbpgaga, GLUkbprapi, GLUkbpxela, GLUnr1anpl, GLUnr1aple, GLUnr1caau, GLUnr1drme, GLUnr1hosa, GLUnr1mumu, GLUnr1rano, GLUnr1susc, GLUnr1xela, GLUnr2ahosa, GLUnr2amumu, GLUnr2arano, GLUnr2bhosa, GLUnr2bmumu, GLUnr2brano, GLUnr2chosa, GLUnr2cmumu, GLUnr2crano, GLUnr2dhosa, GLUnr2dmumu, GLUnr2drano, GLUnr3ahosa, GLUnr3amumu, GLUnr3arano, GLUnr3bhosa, GLUnr3bmumu, GLUnr3brano, GLUr0, GLUr1gaga, GLUr1hosa, GLUr1moch, GLUr1mumu, GLUr1rano, GLUr2acorni, GLUr2borni, GLUr2coli, GLUr2gaga, GLUr2hosa, GLUr2mumu, GLUr2rano, GLUr3aormo, GLUr3caau, GLUr3coli, GLUr3gaga, GLUr3hosa, GLUr3mumu, GLUr3rano, GLUr4caau, GLUr4coli, GLUr4gaga, GLUr4hosa, GLUr4mumu, GLUr4rano, GLUr5daae, GLUr5hosa, GLUr5mumu, GLUr5rano, GLUr6hosa, GLUr6mumu, GLUr6rano, GLUr6xela, GLUr7hosa, GLUr7mumu, GLUr7rano, GLUrldrme, GLUrllAdrme, GLUrllBdrme, GLUrlllyst, GLUrllyst, GLUrk1lyst, GLUcl3cael, GLUclacael, GLUclbcael, GLUclbhaco, GLUcldrme, GLUclhaco, GLUclxcael, and GLUclxonvo.

Examples of other channel proteins include, but are not limited to:

ENaC/DEG family proteins, including, but not limited to, SCNN1 A, SCNN1 B, SCNN1 G, SCNN1D, ACCN2, ACCN1, ACCN3, ACCN4, and ACCN5;

Aquaporins, including, but not limited to, AQP1, AQP2, AQP3, AQP4, AQP5, AQP6, AQP7, AQP7P1, AQP7P2, AQP7P3, AQP7P4, AQP8, AQP9, AQP10, AQP11, AQP12A, and AQP12B; and

Chloride channels, including, but not limited to, CLCA1, CLCA2, CLCA3P, CLCA4, CLCC1, CLCF1, CLCN1, CLCN2, CLCN3, CLCN4, CLCN5, CLCN6, CLCN7, CLCNKA, and CLCNKB.

Examples of membrane carrier/transporter proteins include, but are not limited to:

ABCC family of proteins, including, but not limited to, ABCA1, ABCA2, ABCA3, ABCA4, ABCA5, ABCA6, ABCA7, ABCA8, ABCA9, ABCA10, ABCA11 P, ABCA12, ABCA13, ABCA17P, ABCB1, ABCB4, ABCB5, ABCB6, ABCB7, ABCB8, ABCB9, ABCB10, ABCB10P1, ABCB11, ABCC1, ABCC2, ABCC3, ABCC4, ABCC5, ABCC6, ABCC6P1, ABCC6P2, ABCC8, ABCC9, ABCC10, ABCC11, ABCC12, ABCC13, ABCD1, ABCD1 P1, ABCD1 P2, ABCD1 P3, ABCD1 P4, ABCD2, ABCD3, ABCD4, ABCE1, ABCF1, ABCF2, ABCF3, ABCG1, ABCG2, ABCG4, ABCG5, ABCG8, TAP1, TAP2, CFTR TAPBP, and TAPBPL;

Soluble carrier family of proteins, including, but not limited to, SLC1 A1, SLC1A2, SLC1A3, SLC1A4, SLC1A5, SLC1A6, SLC1A7, SLC2A1, SLC2A2, SLC2A3, SLC2A3P1, SLC2A3P2, SLC2A3P4, SLC2A4, SLC2A4RG, SLC2A5, SLC2A6, SLC2A7, SLC2A8, SLC2A9, SLC2A10, SLC2A11, SLC2A12, SLC2A13, SLC2A14, SLC2AXP1, SLC3A1, SLC3A2, SLC4A1, SLC4A1AP, SLC4A2, SLC4A3, SLC4A4, SLC4A5, SLC4A7, SLC4A8, SLC4A9, SLC4A10, SLC4A11, SLC5A1, SLC5A2, SLC5A3, SLC5A4, SLC5A5, SLC5A6, SLC5A7, SLC5A8, SLC5A9, SLC5A10, SLC5A11, SLC5A12, SLC6A1, SLC6A2, SLC6A3, SLC6A4, SLC6A5, SLC6A6, SLC6A6P, SLC6A7, SLC6A8, SLC6A9, SLC6A10P, SLC6A11, SLC6A12, SLC6A13, SLC6A14, SLC6A15, SLC6A16, SLC6A17, SLC6A18, SLC6A19, SLC6A20, SLC6A21P, SLC7A1, SLC7A2, SLC7A3, SLC7A4, SLC7A5, SLC7A5P1, SLC7A6, SLC7A6OS, SLC7A7, SLC7A8, SLC7A9, SLC7A10, SLC7A11, SLC7A13, SLC7A14, SLC8A1, SLC8A2, SLC8A3, SLC9A1, SLC9A2, SLC9A3, SLC9A3P, SLC9A3P2, SLC9A3P3, SLC9A3P4, SLC9A3R1, SLC9A3R2, SLC9A4, SLC9A5, SLC9A6, SLC9A7, SLC9A8, SLC9A9, SLC9A10, SLC9A11, SLC10A1, SLC10A2, SLC10A3, SLC10A4, SLC10A5, SLC10A6, SLC10A7, SLC11A1, SLC11A2, SLC12A1, SLC12A2, SLC12A3, SLC12A4, SLC12A5, SLC12A6, SLC12A7, SLC12A8, SLC12A9, SLC13A1, SLC13A2, SLC13A3, SLC13A4, SLC13A5, SLC14A1, SLC14A2, SLC15A1, SLC15A2, SLC15A3, SLC15A4, SLC15A5, SLC16A1, SLC16A2, SLC16A3, SLC16A4, SLC16A5, SLC16A6, SLC16A7, SLC16A8, SLC16A9, SLC16A10, SLC16A11, SLC16A12, SLC16A13, SLC16A14, SLC17A1, SLC17A2, SLC17A3, SLC17A4, SLC17A5, SLC17A6, SLC17A7, SLC17A8, SLC17A9, SLC18A1, SLC18A2, SLC18A3, SLC19A1, SLC19A2, SLC19A3, SLC20A1, SLC20A1P1, SLC20A2, SLC22A1, SLC22A2, SLC22A3, SLC22A4, SLC22A5, SLC22A6, SLC22A7, SLC22A8, SLC22A9, SLC22A10, SLC22A11, SLC22A12,SLC22A13, SLC22A14, SLC22A15, SLC22A16, SLC22A17, SLC22A18, SLC22A18AS, SLC22A20, SLC22A23, SLC22A24, SLC22A25, SLC23A1, SLC23A2, SLC23A3, SLC23A4, SLC24A1, SLC24A2, SLC24A3, SLC24A4, SLC24A5, SLC24A6, SLC25A1, SLC25A2, SLC25A3, SLC25A4, SLC25A5, SLC25A5P1, SLC25A5P2, SLC25A5P3, SLC25A5P4, SLC25A5P5, SLC25A5P6, SLC25A5P7, SLC25A5P8, SLC25A5P9, SLC25A6, SLC25A6P1, SLC25A10, SLC25A11, SLC25A12, SLC25A13, SLC25A14, SLC25A15, SLC25A15P, SLC25A16, SLC25A17, SLC25A18, SLC25A19, SLC25A20, SLC25A20P, SLC25A21, SLC25A22, SLC25A23, SLC25A24, SLC25A25, SLC25A26, SLC25A27, SLC25A28, SLC25A29, SLC25A30, SLC25A31, SLC25A32, SLC25A33, SLC25A34, SLC25A35, SLC25A36, SLC25A37, SLC25A38, SLC25A39, SLC25A40, SLC25A41, SLC25A42, SLC25A43, SLC25A44, SLC25A45, SLC25A46, SLC26A1, SLC26A2, SLC26A3, SLC26A4, SLC26A5, SLC26A6, SLC26A7, SLC26A8, SLC26A9, SLC26A10, SLC26A11, SLC27A1, SLC27A2, SLC27A3, SLC27A4, SLC27A5, SLC27A6, SLC28A1, SLC28A2, SLC28A3, SLC29A1, SLC29A2, SLC29A3, SLC29A4, SLC30A1, SLC30A2, SLC30A3, SLC30A4, SLC30A5, SLC30A6, SLC30A7, SLC30A8, SLC30A9, SLC30A10, SLC31A1, SLC31 A1 P, SLC31 A2, SLC32A1, SLC33A1, SLC34A1, SLC34A2, SLC34A3, SLC35A1, SLC35A2, SLC35A3, SLC35A4, SLC35A5, SLC35B1, SLC35B2, SLC35B3, SLC35B4, SLC35C1, SLC35C2, SLC35D1, SLC35D2, SLC35D3, SLC35E1, SLC35E2, SLC35E3, SLC35E4, SLC35F1, SLC35F2, SLC35F3, SLC35F4, SLC35F5, SLC36A1, SLC36A2, SLC36A3, SLC36A4, SLC37A1, SLC37A2, SLC37A3, SLC37A4, SLC38A1, SLC38A2, SLC38A3, SLC38A4, SLC38A5, SLC38A6, SLC38A7, SLC38A8, SLC38A9, SLC38A10, SLC38A11, SLC39A1, SLC39A2, SLC39A3, SLC39A4, SLC39A5, SLC39A6, SLC39A7, SLC39A8, SLC39A9, SLC39A10, SLC39A11, SLC39A12, SLC39A13, SLC39A14, SLC40A1, SLC41A1, SLC41 A2, SLC41A3, SLC43A1, SLC43A2, SLC43A3, LC44A1, SLC44A2, SLC44A3, SLC44A4, SLC44A5, SLC45A1, SLC45A2, SLC45A3, SLC45A4, SLC46A1, SLC46A2, SLC46A3, SLC47A1, SLC47A2, SLC48A1, SLCO1A2, SLCO1B1, SLCO1B3, SLCO1C1, SLCO2A1, SLCO2B1, SLCO3A1, SLCO4A1, SLCO4C1, SLCO5A1 and SLCO6A1;

ATP transporters, including, but not limited to, ATP1A1,ATP1A2, ATP1 A3, ATP1 A4, ATP1B1, ATP1 B2, ATP1 B3, ATP1 B3P1, ATP1 B4, ATP1L1, ATP2A1, ATP2A2, ATP2A3, ATP2B1, ATP2B2, ATP2B3, ATP2B4, ATP2C1, ATP2C2, ATP3, ATP4A, ATP4B, ATP5A1, ATP5AL1, ATP5AP1, ATP5AP2, ATP5AP3, ATP5AP4, ATP5B, ATP5BL1, ATP5BL2, ATP5C1, ATP5C2, ATP5D, ATP5E, ATP5EP1, ATP5EP2, ATP5F1, ATP5G1, ATP5G2, ATP5G3, ATP5GP1, ATP5GP2, ATP5GP3, ATP5GP4, ATP5H, ATP5HP1, ATP5I, ATP5J, ATP5J2, ATP5J2LP, ATP5J2P2, ATP5J2P3, ATP5J2P4, ATP5J2P5, ATP5J2P6, ATP5L, ATP5LP1, ATP5LP2, ATP5LP3, ATP5O, ATP5S, ATP5SL, ATP6AP1, ATP6AP1L, ATP6AP2, ATP6V1A, ATP6V1 B1, ATP6V1 B2, ATP6V1 C1, ATP6V1 C2, ATP6V1 D, ATP6V1 E1, ATP6V1 E2, ATP6V1 EL1, ATP6V1 EP1, ATP6V1 EP2, ATP6V1 F, ATP6V1 G1, ATP6V1G2, ATP6V1G3, ATP6V1GP1, ATP6V1GP2, ATP6V1H, ATP6V0A1, ATP6V0A2, ATP6V0A4, ATP6V0B, ATP6V0C, ATP6V0D1,ATP6V0D2, ATP6V0E1,ATP6V0E2, ATP7A, ATP7B, and ATP8A1; and

Fatty acid binding proteins, including, but not limited to, FABP1, FABP2, FABP3, FABP3P2, FABP4, FABP5, FABP5L1, FABP5L2, FABP5L3, FABP5L4, FABP5L5, FABP5L6, FABP5L7, FABP5L8, FABP5L9, FABP5L10, FABP5L11, FABP5L12, FABP6, FABP7, FABP9, and FABP12;

Insulin-like growth factors, including, but not limited to, IGF1, IGF1R, IGF2, IGF2AS, IGF2BP1, IGF2BP2, IGF2BP3, IGF2R, IGFALS, IGFBP1, IGFBP2, IGFBP3, IGFBP4, IGFBP5, IGFBP6, IGFBP7, IGFBPL1, IGFL1, IGFL1P1, IGFL1P2, IGFL2, IGFL3, IGFL4, and IGFN1;

Transforming growth factors, including, but not limited to, TGFA, TGFB1, TGFB1I1, TGFB2, TGFB3, TGFBI, TGFBR1, TGFBR2, TGFBR3, TGFBRAP1, TGFBRE, LEFTY1, LEFTY2, BMPR1 A, BMPR1APS1, BMPR1APS2, BMPR1B, BMPR2, ACVR1, ACVR1 B, ACVR1 C, ACVR2A, ACVR2B, and ACVRL1;

Nuclear receptors, including, but not limited to, NR1D1, NR1D2, NR1H2, NR1H3, NR1H4, NR1H5P, NR111,NR112, NR113, NR114, NR2A1, NR2A2, NR2B1, NR2B2, NR2B3, NR2C1, NR2C2, NR2C2AP, NR2E1, NR2E3, NR2F1, NR2F2, NR2F6, NR3C1, NR3C1P, NR3C2, NR3C3, NR3C4, NR4A1, NR4A2, NR4A3, NR5A1, NR5A2, NR6A1, NRAP, NRARP, NR0B1, NR0B2, NRBF2, NRBP1, NRBP2, NRCAM, NRIP1, NRIP2, and NRIP3;

Retinoic acid receptors, including, but not limited to, RARA, RARB, RARG, RARRES1, RARRES2, and RARRES3;

Receptor tyrosine kinase orphan and RAR related proteins, including, but not limited to, ROR1, ROR2, RORA, RORB, and RORC;

Peroxisome proliferator activated receptors, including, but not limited to, PPARA, PPARD, PPARG, PPARGC1A, and PPARGC1 B;

Thyroid hormone receptors, including, but not limited to, THRA, THRAP3, THRAP3L, THRB, and THRSP;

Estrogen receptors, epithelial splicing regulatory proteins, and estrogen related receptors, including, but not limited to, ESR1, ESR2, ESRP1, ESRP2, ESRRA, ESRRAP1, ESRRAP2, ESRRB, and ESRRG;

Etrythroblastic leukemia viral oncogenes, including, but not limited to, ERBB2, ERBB2IP, ERBB3, ERBB4, and EGFR;

Platelet derived growth factors, including, but not limited to, PDGFA, PDGFB, PDGFC, PDGFD, PDGFRA, PDGFRB, and PDGFRL;

Fibroblast derived growth factors, including, but not limited to, FGFR1, FGFR10P, FGFR10P2, FGFR2, FGFR3, FGFR3P, FGFR4, FGFR6, and FGFRL1;

Latent transforming growth factor β binding proteins, including, but not limited to, LTBP1,LTBP2, LTBP3, and LTBP4;

Vitamin carrier proteins, including, but not limited to, RBP1, RBP2, RBP3, RBP4,RBP5,RBP7,RBPJ, RBPJL, RBPJP1, RBPJP2, RBPJP3, RBPJP4, RBPMS, RBPMS2, and RBPMSLP;

Steroidogenic acute regulatory proteins, including, but not limited to, STAR, STARD3, STARD3NL, STARD4, STARD5, STARD6, STARD7, STARD8, STARD9, STARD10,STARD13, and STARP1;

Sterol carrier proteins, including, but not limited to, SCP2 and SCPEP1;

Glycolipid transfer proteins, including, but not limited to, GLTP, GLTPD1, GLTPD2, and GLTPP1; and

Other transport proteins, *e.g.,* CETP.

Other examples of proteins of interest include, but are not limited to:

T cell receptor β constant 1, including, but not limited to, TRBC1, TRBC2, TRBD1, TRBD2, TRBJ1-1, TRBJ1-2,TRBJ1-3,TRBJ1-4,TRBJ1-5,TRBJ1-6,TRBJ2-1, TRBJ2-2, TRBJ2-2P,TRBJ2-3, TRBJ2-4, TRBJ2-5, TRBJ2-6, TRBJ2-7,TRBV1, TRBV2, TRBV3-1, TRBV3-2, TRBV4-1, TRBV4-2, TRBV4-3, TRBV5-1, TRBV5-2, TRBV5-3, TRBV5-4, TRBV5-5, TRBV5-6, TRBV5-7, TRBV5-8, TRBV6-1, TRBV6-2, TRBV6-3, TRBV6-4, TRBV6-5, TRBV6-6, TRBV6-7, TRBV6-8, TRBV6-9, TRBV7-1, TRBV7-2, TRBV7-3, TRBV7-4, TRBV7-5, TRBV7-6, TRBV7-7, TRBV7-8, TRBV7-9, TRBV8-1,TRBV8-2,TRBV9, TRBV10-1, TRBV10-2, TRBV10-3, TRBV11-1, TRBV11-2, TRBV11-3, TRBV12-1, TRBV12-2, TRBV12-3, TRBV12-4, TRBV12-5, TRBV13, TRBV14, TRBV15, TRBV16, TRBV17, TRBV18, TRBV19, TRBV20-1, TRBV20OR9-2, TRBV21-1, TRBV21 OR9-2, TRBV22-1, TRBV22OR9-2, TRBV23-1, TRBV23OR9-2, TRBV24-1, TRBV24OR9-2, TRBV25-1, TRBV25OR9-2, TRBV26, TRBV26OR9-2, TRBV27, TRBV28, TRBV29-1, TRBV29OR9-2, TRBV30, TRBVA, TRBVAOR9-2,TRBVB, and TRBVOR9;

Disintegrins, including, but not limited to, ADAM1, ADAM2, ADAM3A,ADAM3B,ADAM5P,ADAM6, ADAM7, ADAM8,ADAM9,ADAM10, ADAM11, ADAM12, ADAM15, ADAM17, ADAM18, ADAM19, ADAM20, ADAM21, ADAM21 P, ADAM22, ADAM23, ADAM28, ADAM29, ADAM30, ADAM32, ADAM33, ADAMDEC1, ADAMTS1, ADAMTS2, ADAMTS3, ADAMTS4, ADAMTS5, ADAMTS6, ADAMTS7, ADAMTS8, ADAMTS9, ADAMTS10, ADAMTS12, ADAMTS13, ADAMTS14, ADAMTS15, ADAMTS16, ADAMTS17, ADAMTS18,ADAMTS19, ADAMTS20, ADAMTSL1, ADAMTSL2,ADAMTSL3,ADAMTSL4, and ADAMTSL5;

Integrins, including, but not limited to, ITGA1, ITGA2,ITGA2B, ITGA3, ITGA4, ITGA5, ITGA6, ITGA7, ITGA8, ITGA9, ITGA10, ITGA11, ITGAD, ITGAE, ITGAL, ITGAM, ITGAV, ITGAW, ITGAX, ITGB1, ITGB1BP1, ITGB1BP2, ITGB1BP3, ITGB2, ITGB3, ITGB3BP, ITGB4, ITGB5, ITGB6, ITGB7, ITGB8, and ITGBL1;

Cell adhesion molecules, including, but not limited to, NCAM1, NCAM2, VCAM1, ICAM1, ICAM2, ICAM3, ICAM4, ICAM5, PECAM1, L1CAM, CHL1, MAG, CADM1, CADM2, CADM3, and CADM4;

Human odorant receptors, including, but not limited to, OR10A1, OR10A3 , OR10A4, OR10A5 , OR10A6 , OR10A7, OR10C1, OR10C2, OR10D4 , OR10G2 , OR10G3, OR10G4, OR10G7, OR10G8, OR10G9 , OR10H1, OR10H2, OR10H3 , OR10H4 , OR10H5 , OR10J1, OR10J3, OR10J5 , OR10J6 , OR10K1, OR10K2, OR10Q1, OR10R2, OR10S1, OR10T2, OR10V1, OR10Z1, OR11A1, OR11G2, OR11H1, OR11H4, OR11 H6 , OR11 H7P , OR11 L1 , OR12D3 , OR13A1 , OR13C2, OR13C3 , OR13C4 , OR13C5 , OR13C7 , OR13C8 , OR13C9 , OR13D1 , OR13E2, OR13F1, OR13G1, OR13H1, OR13J1, OR14A16, OR14A2, OR14C36, OR14J1, OR1A1, OR1A2, OR1A2, OR1B1, OR1C1, OR1D2, OR1D4 , OR1D5, OR1E1, OR1E2, OR1E2, OR1E5, OR1E5, OR1E6, OR1E7, OR1F1, OR1F10, OR1F11, OR1F12 , OR1F2, OR1G1, OR111, OR1J1, OR1J2, OR1J2, OR1J4, OR1J5, OR1K1, OR1L1, OR1 L3 , OR1 L4 , OR1 L6 , OR1 L8 , OR1M1 , OR1M1, OR1N1, OR1 N2 , OR1 N3 , OR1Q1, OR1S1, OR1 S2 , OR2A1, OR2A10 , OR2A19 , OR2A20, OR2A21, OR2A4 , OR2A42, OR2A5 , OR2A6 , OR2A7 , OR2AE1 , OR2AJ1, OR2AK2, OR2B1, OR2B2, OR2B3 , OR2B6 , OR2B9 , OR2C1 , OR2D1 , OR2D2 , OR2D3 , OR2F1 , OR2F2 , OR2F3 , OR2G2 , OR2G3 , OR2H1 , OR2H2 , OR2H3 , OR2J2 , OR2J3 , OR2K1 , OR2K2, OR2L1, OR2L2, OR2L3 , OR2L5, OR2L8, OR2M1, OR2M2 , OR2M4, OR2S2, OR2T1 , OR2T3 , OR2T4 , OR2T5 , OR2T6, OR2T7, OR2T8, OR2V1, OR2V2 , OR2V3 , OR2W1, OR2W3, OR2Y1, OR2Z1, OR3A1, OR3A2 , OR3A3 , OR3A4 , OR4A15 , OR4A16 , OR4A4 , OR4A5, OR4B1, OR4C12, OR4C13, OR4C15, OR4C16, OR4C3 , OR4C6 , OR4D1 , OR4D2 , OR4D5 , OR4D6 , OR4D9 , OR4E2 , OR4F10 , OR4F15 , OR4F16 , OR4F16 , OR4F17 , OR4F18 , OR4F19 , OR4F3 , OR4F6 , OR4K1 , OR4K13 , OR4K14, OR4K15 , OR4K17 , OR4K2 , OR4K3 , OR4K5 , OR4L1 , OR4M1 , OR4M2 , OR4N2 , OR4N4 , OR4N5 , OR4P4 , OR4Q3, OR4S1, OR4X1, OR4X2, OR51A2 , OR51A4 , OR51A7 , OR51B2 , OR51B4 , OR51D1, OR51E1, OR51E2, OR51F2, OR51G1, OR51G2, OR51H1, OR51I1, OR51I2, OR51L1, OR51M1, OR51Q1, OR51S1, OR51T1, OR52A1, OR52A2, OR52B2, OR52B4, OR52B4, OR52B4, OR52B6, OR52D1, OR52E2, OR52E4, OR52E5, OR52E6, OR52E8 , OR52H1 , OR52I1 , OR52I2, OR52J3 , OR52K1 , OR52K2 , OR52L1 , OR52L2 , OR52N1 , OR52N2 , OR52N4 , OR52N5 , OR52P1 , OR52R1 , OR56A4 , OR56A6 , OR56B2, OR56B4 , OR5A1 , OR5A2 , OR5AC2 , OR5AK2 , OR5AK3 , OR5AN1 , OR5AP2 , OR5AR1 , OR5AS1, OR5AU1 , OR5AU1 , OR5B13, OR5B16 , OR5B17 , OR5B2 , OR5B3 , OR5C1, OR5D13 , OR5D14 , OR5D16 , OR5D18 , OR5F1 , OR5G3, OR5H1 , OR5H2 , OR5H6 , OR511 , OR5K1, OR5K2 , OR5L1 , OR5L2 , OR5M1 , OR5M10 , OR5M11 , OR5M11 , OR5M3 , OR5M3 , OR5M8 , OR5M9 , OR5P2 , OR5P3 , OR5T2 , OR5T3, OR5V1 , OR6A1 , OR6B1 , OR6B2, OR6C1 , OR6C2 , OR6C3 , OR6F1 , OR6J2 , OR6K3 , OR6K6 , OR6M1 , OR6N1 , OR6N2 , OR6P1 , OR6Q1 , OR6S1, OR6T1 , OR6V1 , OR6X1 , OR6Y1 , OR7A10 , OR7A17 , OR7A2 , OR7A5 , OR7C1 , OR7C2 , OR7D2 , OR7D2 , OR7D4P, OR7E102 , OR7E120 , OR7G1 , OR7G2 , OR7G3 , OR8A1 , OR8B12, OR8B2 , OR8B3 , OR8B4 , OR8B8 , OR8D1 , OR8D2 , OR8D4 , OR8G1 , OR8G2 , OR8H1 , OR8H2 , OR8H3 , OR812 , OR8J1 , OR8J3 , OR8K1, OR8K3 , OR8K5 , OR9A2 , OR9A4 , OR9G1 , OR9G4 , OR9G5 , OR911 , OR9K2 , and OR9Q1; and

Mosquito (anopheles gambiae) odorant receptors, including, but not limited to, IOR100, IOR101, IOR102, IOR103, IOR104, IOR105, IOR106, IOR107, IOR108, IOR109, IOR110, IOR111, IOR112, IOR113, IOR114, IOR115, IOR116, IOR117, IOR118, IOR119, IOR120, IOR121, IOR122, IOR123, IOR124, IOR125, IOR126, IOR127, IOR49, IOR50, IOR51, IOR52, IOR53, IOR54, IOR55, IOR56, IOR57, IOR58, IOR59, IOR60, IOR61, IOR62, IOR63, IOR64, IOR65, IOR66, IOR67, IOR68, IOR69, IOR70, IOR71, IOR72, IOR73, IOR74, IOR75, IOR76, IOR77, IOR78, IOR79, IOR80, IOR81, IOR82, IOR83, IOR84, IOR85, IOR86, IOR87, IOR88, IOR89, IOR90, IOR91, IOR92, IOR93, IOR94, IOR95, IOR96, IOR97, IOR98, IOR99, ORL7077, ORL7078, ORL7079, ORL7080, ORL7081, ORL7082, ORL7083, ORL7084, ORL7085, ORL7086, ORL7087, ORL7088, ORL7089, ORL7090, ORL7091, ORL7092, ORL7093, ORL7094, ORL7095, ORL7096, ORL7097, ORL7098, ORL7099, ORL7100, ORL7101, ORL7102, ORL7103, ORL7104, ORL7105, ORL7106, ORL7107, ORL7108, ORL7109, ORL7110, ORL7111, ORL7112, ORL7113, ORL7114, ORL7115, ORL7116, ORL7117, ORL7118, ORL7119, ORL7120, ORL7121, ORL7122, ORL7123, ORL7124, ORL7125, TPR2307, TPR2308, TPR2309, TPR2310, TPR2312, TPR2314, TPR2315, TPR2316, TPR2317, TPR2318, TPR2319, TPR2320, TPR2321, TPR2321, TPR698, TPR699, TPR700, TPR701, TPR702, TPR703, TPR704, TPR705, TPR706, TPR707, TPR708, TPR709, TPR710, TPR711, TPR712, TPR713, TPR714, TPR715, TPR716, TPR717, TPR718, TPR719, TPR720, TPR721, TPR722, TPR723, TPR724, TPR725, TPR725, TPR726, TPR727, TPR728, TPR729, TPR730, TPR731, TPR732, TPR733, TPR734, TPR735, TPR736, TPR737, TPR738, TPR739, TPR740, TPR741, TPR742, TPR743, TPR744, TPR745, TPR746, TPR747, TPR748, TPR749, TPR750, TPR751, TPR752, TPR753, TPR754, TPR755, TPR756, TPR757, TPR758, TPR759, TPR760, TPR761, TPR762, TPR763, TPR764, TPR765, TPR766, TPR767, TPR768, TPR769, TPR770, TPR771, and TPR772.

Further examples of proteins of interest can be found in Tables 7-22 hereinbelow. Optionally, spliced forms and/or SNPs of the proteins listed in Tables 7-22 may be expressed. Optionally, any combination of any of the proteins listed in Tables 7-22 may be co-expresped in cells.

Optionally, the cells and cell lines disclosed herein are suitable for use in a cell-based assay. Such cells and cell lines provide consistent and reproducible expression of the protein of interest over time and, thus, are particularly advantageous in such assays.

Disclosed herein are cells and cell lines that are suitable for the production of biological molecules. The cells and cell lines for such use are characterized, for example, by consistent expression of a protein or polypeptide that is functional or that is capable of becoming functional. Also disclosed herein is a method for producing cells and cell lines that stably express an RNA or a protein of interest. Using the method disclosed herein, one can produce cells and cell lines that express any desired protein in functional form, including complex proteins such as multimeric proteins, (*e.g.,* heteromultimeric proteins) and proteins that are cytotoxic. The method disclosed herein makes possible the production of engineered cells and cell lines stably expressing functional proteins that prior to this disclosure herein have not previously been produced. Without being bound by theory, it is believed that because the method permits investigation of very large numbers of cells or cell lines under any desired set of conditions, it makes possible the identification of rare cells that would not have been produced in smaller populations or could not otherwise be found and that are optimally suited to express a desired protein in a functional form under desired conditions. Without being limited by theory, many RNAs and proteins of interest are normally expressed in specialized cells (*e.g.* cells of specific tissues, cells of specialized tissues, cells of specialized functions, cells with specialized cellular domains or compartments, sensory cells, neurons, taste buds, epithelial cells, stem cells, cancer cells, muscle cells, cells of the eye, cells that produce antibodies, cells that produce high levels of proteins, as well as the various cell types disclosed herein). The specialized cells may provide a specific biological or cellular context, background or genetic make-up for non-cyctotoxic or native functional or physiological expression of the RNAs or proteins of interest. For instance, the specialized cells may provide factors including accessory factors or chaperones or specialized cellular compartments for sufficient, proper or optimal expression, stoichiometry, production, folding, assembly, post-translational modification, targeting, membrane integration, secretion, function, pharmacology or physiology of the RNAs or proteins of interest. Engineering of cells or cell lines to express RNAs or proteins of interest that they do not normally express may result in the production of cells or cell lines where these conditions are not recapitulated nor approximated for optimal expression or function of RNAs or proteins of interest without associated cytotoxicity.

Many populations of cells that may be engineered to express an RNA or protein of interest are comprised of genetically diverse populations of individual cells where even the number of chromosomes may vary between cells. Rare cells included in these populations (compared to the average cell of such populations) may provide a biological or cellular context or background or genetic make-up that is sufficient, preferred, above average, improved, or optimal for native or non-cytotoxic expression, function, pharmacology or physiology of RNAs or proteins of interest that are not normally expressed in the average cell of the population of cells.

Optionally, the disclosure herein allows the analysis of millions of individual cells of populations of cells engineered to comprise an RNA or protein of interest such that individual cells that are compatible for the expression of the RNAs or proteins of interest can be rapidly or individually detected or isolated, even if this represents only rare cells in the population of cells. Optionally, rare cells that are compatible with viable, non-cytotoxic, functional or native expression of an RNA or protein of interest that normally results in cytotoxicity or cell death in the average cell of a population of cells that is engineered to express the RNA or protein of interest may be detected and isolated. Optionally, each positive cell that is detected or isolated from a population of cells engineered to express an RNA or protein of interest may comprise different absolute or relative levels of each RNA or protein of interest. Optionally, each positive cell may further provide or comprise a different cellular or genetic context (*e.g.* different number of chromosomes or fragments of chromosomes, genes, gene sequences, expression profiles, or endogenously expressed proteins or RNAs including mRNAs or siRNAs, or accessory factors for the RNAs or proteins of interest) as the cellular background for the expression or function of the RNA or protein of interest. Disclosed herein is the isolation of numerous engineered cells positive for the expression of an RNA or protein of interest coupled with novel methods that enable the maintenance of the isolated cells in culture. Optionally, the maintenance of the isolated cells in culture may be performed using conditions that are substantially identical for all of the cells that are maintained. Optionally, this in turn enables testing including functional testing of the isolated cells over time in culture to identify and produce functional stable cells or cell lines comprising desired or improved expression, function, physiology or pharmacology of the expressed RNA or protein of interest. Optionally, by isolating, maintaining and functionally testing numerous cells positive for the expression of an RNA or protein of interest, the methods allow identification or production of cells functionally, viably and stably expressing the RNAs or proteins of interest even for RNAs or proteins of interest that are not normally expressed in the average cell of the populations of cells engineered to express the RNAs or proteins of interest.

In fact, optionally, the methods were found to result in functional and stable expression of RNAs or proteins that had previously been considered to be cytotoxic when expressed in engineered cells, demonstrating the effectiveness with which the methods may be used to produce cells comprising the conditions that are required and compatible with non-cytotoxic expression of and function of such proteins which could not previously be modeled in engineered cells.

Also disclosed herein is a matched panel of cell lines, *i.e.,* a collection of clonal cell lines that are matched for one or more physiological properties. Because the disclosed method permits maintenance and characterization of large numbers of cell lines under identical conditions, it is possible to identify any number of cell lines with similar physiological properties. Using the disclosed method, it is possible to make matched panels comprising any desired number of cell lines or make up Such matched panels may be maintained under identical conditions, including cell density and, thus, are useful for high throughput screening and other uses where it is desired to compare and identify differences between cell lines. Also disclosed are matched panels of cell lines that are matched for growth rate.

Also disclosed herein is a method for producing cells or cell lines that express a protein of previously unknown function and/or for which no ligand had previously been identified. Such a protein may be a known naturally occurring protein, a previously unknown naturally occurring protein, a previously unknown form of a known naturally occurring protein or a modified form of any of the foregoing.

Any desired cell type may be used for the cells disclosed herein. The cells may be prokaryotic or eukaryotic. The cells may express the protein of interest in their native state or not. Eukaryotic cells that may be used include but are not limited to fungi cells such as yeast cells, plant cells, insect cells and animal cells. Animal cells that can be used include but are not limited to mammalian cells. Primary or immortalized cells may be derived from mesoderm, ectoderm or endoderm layers of eukaryotic organisms. The cells may be endothelial, epidermal, mesenchymal, neural, renal, hepatic, hematopoietic, or immune cells. For example, the cells may be intestinal crypt or villi cells, clara cells, colon cells, intestinal cells, goblet cells, enterochromafin cells, enteroendocrine cells. Mammalian cells that are useful in the method include but are not limited to human, non-human primate, cow, horse, goat, sheep, pig, rodent (including rat, mouse, hamster, guinea pig), marsupial, rabbit, dog and cat. The cells can be differentiated cells or stem cells, including embryonic stem cells.

Cells disclosed herein can be primary, transformed, oncogenically transformed, virally transformed, immortalized, conditionally transformed, explants, cells of tissue sections, animals, plants, fungi, protists, archaebacteria and eubacteria, mammals, birds, fish, reptiles, amphibians, and arthropods, avian, chicken, reptile, amphibian, frog, lizard, snake, fish, worms, squid, lobster, sea urchin, sea slug, sea squirt, fly, squid, hydra, arthropods, beetles, chicken, lamprey, ricefish, zebra finch, pufferfish, and Zebrafish,

Additionally, cells such as blood/immune cells, endocrine (thyroid, parathyroid, adrenal), GI (mouth, stomach, intestine), liver, pancreas, gallbladder, respiratory (lung, trachea, pharynx), Cartilage, bone, muscle, skin, hair, urinary (kidney, bladder), reproductive (sperm, ovum, testis, uterus, ovary, penis, vagina), sensory (eye, ear, nose, mouth, tongue, sensory neurons), Blood/immune cells such as_B cell, T cell (Cytotoxic T cell, Natural Killer T cell, Regulatory T cell, T helper cell, γδ Tcell , Natural killer cell; granulocytes (basophil granulocyte, eosinophil granulocyte, neutrophil granulocyte/hypersegmented neutrophil), monocyte/macrophage, red blood cell (reticulocyte), mast cell, thrombocyte/Megakaryocyte, dendritic cell; endocrine cells such as: thyroid (thyroid epithelial cell, parafollicular cell), parathyroid (parathyroid chief cell, oxyphil cell), adrenal (chromaffin cell), nervous system cells such as: glial cells (astrocyte, microglia), magnocellular neurosecretory cell, stellate cell, nuclear chain cell, boettcher cell, pituitary, (gonadotrope, corticotrope, thyrotrope, somatotrope, lactotroph), respiratory system cells such as pneumocyte (type I pneumocyte, type II pneumocyte), clara cell, goblet cell; circulatory system cells such as myocardiocyte pericyte; digestive system cells such as stomach (gastric chief cell, parietal cell), goblet cell, paneth cell, G cells, D cells, ECL cells, I cells, K cells, ,enteroendocrine cells, enterochromaffin cell, APUD cell, liver (hepatocyte, kupffer cell), pancreas (beta cells, alpha cells), gallbladder; cartilage/bone/muscle/integumentary system cells such as osteoblast, osteocyte, osteoclast, tooth cells (cementoblast, ameloblast), cartilage cells: chondroblast, chondrocyte, skin/hair cells: trichocyte, keratinocyte, melanocyte, muscle cells: myocyte, adipocyte, fibroblast, urinary system cells such as podocyte, juxtaglomerular cell, intraglomerular mesangial cell/extraglomerular mesangial cell, kidney proximal tubule brush border cell, macula densa cell; reproductive system cells such as spermatozoon, sertoli cell, leydig cell, ovum, ovarian follicle cell; sensory cells such as organ of corti cells, olfactory epithelium, temperature sensitive sensory neurons, merckel cells, olfactory receptor neuron, pain sensitive neurons, photoreceptor cells, taste bud cells, hair cells of the vestibular apparatus, carotid body cells are useful to make cells or cell lines of the invention.

Plant cells that are useful include roots, stems and leaves and plant tissues include meristematic tissues, parenchyma collenchyma, sclerenchyma, secretory tissues, xylem, phloem, epidermis, periderm (bark).

Cells that are useful for the cells and cell lines disclosed herein also include but are not limited to: Chinese hamster ovary (CHO) cells, established neuronal cell lines, pheochromocytomas, neuroblastomas fibroblasts, rhabdomyosarcomas, dorsal root ganglion cells, NS0 cells, CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171), L-cells, HEK-293 (ATCC CRL1573) and PC12 (ATCC CRL-1721), HEK293T (ATCC CRL-11268), RBL (ATCC CRL-1378), SH-SY5Y (ATCC CRL-2266), MDCK (ATCC CCL-34), SJ-RH30 (ATCC CRL-2061), HepG2 (ATCC HB-8065), ND7/23 (ECACC 92090903), CHO (ECACC 85050302), Vero (ATCC CCL 81), Caco-2 (ATCC HTB 37), K562 (ATCC CCL 243), Jurkat (ATCC TIB-152), Per.C6 (Crucell, Leiden, The Netherlands), Huvec (ATCC Human Primary PCS 100-010, Mouse CRL 2514, CRL 2515, CRL 2516), HuH-7D12 (ECACC 01042712), 293 (ATCC CRL 10852), A549 (ATCC CCL 185), IMR-90 (ATCC CCL 186), MCF-7 (ATC HTB-22), U-2 OS (ATCC HTB-96), T84 (ATCC CCL 248), or any established cell line (polarized or nonpolarized) or any cell line available from repositories such as American Type Culture Collection (ATCC, 10801 University Blvd. Manassas, Va. 20110-2209 USA) or European Collection of Cell Cultures (ECACC, Salisbury Wiltshire SP4 0JG England).

Further, cells that are useful in the method disclosed herein are mammalian cells amenable to growth in serum containing media, serum free media, fully defined media without any animal-derived products, and cells that can be converted from one of these conditions to another..

Cells disclosed herein include cells into which a nucleic acid that encodes the protein of interest (or in the case of a heteromultimeric protein, a nucleic acid that encodes one or more of the subunits of the protein) has been introduced. Engineered cells also include cells into which nucleic acids for transcriptional activation of an endogenous sequence encoding a protein of interest (or for transcriptional activation of endogenous sequence encoding one or more subunits of a heteromultimeric protein) have been introduced. Engineered cells also include cells comprising a nucleic acid encoding a protein of interest that is activated by contact with an activating compound or following post-translational modification, treatment with or contact with an enzyme including but not limited to a protease. Engineered cells further include combinations of the foregoing, that is, cells that express one or more subunits of a heteromultimeric protein from an introduced nucleic acid encoding it and that express one or more subunits of the protein by gene activation.

Any of the nucleic acids may be introduced into the cells using known means. Techniques for introducing nucleic acids into cells are well-known and readily appreciated by the skilled worker. The methods include but are not limited to transfection, viral delivery, protein or peptide mediated insertion, coprecipitation methods, lipid based delivery reagents (lipofection), cytofection, lipopolyamine delivery, dendrimer delivery reagents, electroporation or mechanical delivery. Examples of transfection reagents are GENEPORTER, GENEPORTER2, LIPOFECTAMINE, LIPOFECTAMINE 2000, FUGENE 6, FUGENE HD, TFX-10, TFX-20, TFX-50, OLIGOFECTAMINE, TRANSFAST, TRANSFECTAM, GENESHUTTLE, TROJENE, GENESILENCER, X-TREMEGENE, PERFECTIN, CYTOFECTIN, SIPORT, UNIFECTOR, SIFECTOR, TRANSIT-LT1, TRANSIT-LT2, TRANSIT-EXPRESS, IFECT, RNAI SHUTTLE, METAFECTENE, LYOVEC, LIPOTAXI, GENEERASER, GENEJUICE, CYTOPURE, JETSI, JETPEI, MEGAFECTIN, POLYFECT, TRANSMESSANGER, RNAIFECT, SUPERFECT, EFFECTENE, TF-PEI-KIT, CLONFECTIN, and METAFECTINE.

Where two or more nucleotide sequences are introduced, such as sequences encoding two or more subunits of a heteromultimeric protein or sequences encoding two or more different proteins of interest, the sequences may be introduced on the same vector or, preferably, on separate vectors. The DNA can be genomic DNA, cDNA, synthetic DNA or mixtures of them. Optionally, nucleic acids encoding a protein of interest or a partial protein of interest do not include additional sequences such that the protein of interest is expressed with additional amino acids that may alter the function of the cells compared to the physiological function of the protein.

Optionally, the nucleic acid encoding the protein of interest comprises one or more substitutions, insertions, mutations or deletions, as compared to a nucleic acid sequence encoding the wild-type protein. In a nucleic acid comprising a mutation disclosed herein, the mutation may be a random mutation or a site-specific mutation. These nucleic acid changes may or may not result in an amino acid substitution. Optionally, the nucleic acid is a fragment of the nucleic acid that encodes the protein of interest. Nucleic acids that are fragments or have such modifications encode polypeptides that retain at least one biological property of the protein of interest.

Also disclosed herein are cells and cell lines stably expressing a nucleic acid, whose sequence is at least about 85% identical to the "wild type" sequence encoding the protein of interest, or a counterpart nucleic acid derived from a species other than human or a nucleic acid that encodes the same amino acid sequence as any of those nucleic acids. Optionally, the sequence identity is at least 85%, 90%, 95%, 96%, 97%, 98%, 99% or higher compared to those sequences. Also disclosed herein are cells and cell lines wherein the nucleic acid encoding a protein of interest hybridizes under stringent conditions to the wild type sequence or a counterpart nucleic acid derived from a species other than human, or a nucleic acid that encodes the same amino acid sequence as any of those nucleic acids.

Optionally, the cell or cell line comprises a protein-encoding nucleic acid sequence comprising at least one substitution as compared to the wild-type sequence or a counterpart nucleic acid derived from a species other than human or a nucleic acid that encodes the same amino acid sequence as any of those nucleic acids. The substitution may comprise less than 10, 20, 30, or 40 nucleotides or, up to or equal to 1 %, 5%, 10% or 20% of the nucleotide sequence. Optionallly, the substituted sequence may be substantially identical to the wild-type sequence or a counterpart nucleic acid derived from a species other than human a nucleic acid that encodes the same amino acid sequence as any of those nucleic acids (*e.g.,* a sequence at least 85%, 90%, 95%, 96%, 97%, 98%, 99% or higher identical thereto), or be a sequence that is capable of hybridizing under stringent conditions to the wild type sequence or a counterpart nucleic acid derived from a species other than human or a nucleic acid that encodes the same amino acid sequence as any one of those nucleic acids.

Optionally, the cell or cell line comprises protein-encoding nucleic acid sequence comprising an insertion into or deletion from the wild type sequence or a counterpart nucleic acid derived from a species other than human or a nucleic acid that encodes the same amino acid sequence as any of those nucleic acids. The insertion or deletion may be less than 10, 20, 30, or 40 nucleotides or up to or equal to 1 %, 5%, 10% or 20% of the nucleotide sequence. Optionally, the sequences of the insertion or deletion may be substantially identical to the wild type sequence or a counterpart nucleic acid derived from a species other than human or a nucleic acid that encodes the same amino acid sequence as any of those nucleic acids (*e.g.,* a sequence at least 85%, 90%, 95%, 96%, 97%, 98%, 99% or higher identical thereto), or be a sequence that is capable of hybridizing under stringent conditions to the wild-type sequence or a counterpart nucleic acid derived from a species other than human, or a nucleic acid that encodes the same amino acid sequence as any of those nucleic acids.

Optionally, the nucleic acid substitution or modification results in an amino acid change, such as an amino acid substitution. For example, an amino acid residue of the wild type protein of interest or a counterpart amino acid derived from a species other than human may be replaced by a conservative or a non-conservative substitution. Optionally, the sequence identity between the original and modified amino acid sequence can differ by about 1%, 5%, 10% or 20% or from a sequence substantially identical thereto (*e.g.,* a sequence at least 85%, 90%, 95%, 96%, 97%, 98%, 99% or higher identical thereto).

A "conservative amino acid substitution" is one in which an amino acid residue is substituted by another amino acid residue having a side chain R group with similar chemical properties to the parent amino acid residue (*e.g.,* charge or hydrophobicity). In cases where two or more amino acid sequences differ from each other by conservative substitutions, the percent sequence identity or degree of similarity may be adjusted upwards to correct for the conservative nature of the substitution. Means for making this adjustment are well-known to those of skill in the art. *See, e.g.,* Pearson, Methods Mol. Biol. 243:307-31 (1994).

Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Preferred conservative amino acids substitution groups are: valine-leucine-isoleucine, phenylalanine-tyrosine, lysine-arginine, alanine-valine, glutamate-aspartate, and asparagine-glutamine. Alternatively, a conservative amino acid substitution is any change having a positive value in the PAM250 log-likelihood matrix disclosed in Gonnet et al., Science 256:1443-45 (1992). A "moderately conservative" replacement is any change having a nonnegative value in the PAM250 log-likelihood matrix.

Conservative modifications in the protein of interest will produce proteins having functional and chemical characteristics similar (*i.e.* at least 50%, 60%, 70%, 80%, 90% or 95% the same) to those of the unmodified protein.

Optionally, the host cell is an embryonic stem cell that is then used as the basis for the generation of transgenic animals that produce the protein of interest. Embryonic stem cells stably expressing a functional protein of interest, may be implanted into organisms directly, or their nuclei may be transferred into other recipient cells and these may then be implanted, or they may be used to create transgenic animals. Optionally the protein may be expressed in the animal with desired temporal and/or tissue specific expression.

As will be appreciated by those of skill in the art, any vector that is suitable for use with a chosen host cell may be used to introduce a nucleic acid encoding a protein of interest into a host cell. Where more than one vector is used, for example, to introduce two or more different subunits or two or more proteins of interest, the vectors may be the same type or may be of different types.

Examples of vectors that may be used to introduce the nucleic acids into host cells include but are not limited to plasmids, viruses, including retroviruses and lentiviruses, cosmids, artificial chromosomes and may include, for example, pCMVScript, pcDNA3.1 Hygro, pcDNA3.1 neo, pcDNA3.1 puro, pSV2neo, pIRES puro, pSV2 zeo. Exemplary mammalian expression vectors that are useful to make the cells and cell lines disclosed herein include: pFN11A (BIND) Flexi®, pGL4.31, pFC14A (HaloTag® 7) CMV Flexi®, pFC14K (HaloTag® 7) CMV Flexi®, pFN24A (HaloTag® 7) CMVd3 Flexi®, pFN24K (HaloTag® 7) CMVd3 Flexi®,HaloTag™ pHT2,pACT , pAdVAntage™, pALTER®-MAX,pBIND, pCAT®3-Basic, pCAT®3-Control, pCAT®3-Enhancer, pCAT®3-Promoter, pCI, pCMVTNT™ , pG5luc, pSI, pTARGET™, pTNT™, pF12A RM Flexi®, pF12K RM Flexi®, pReg neo,pYES2/GS, pAcKDMVA/5-DEST Gateway® Vector, pAd/PL-DEST™ Gateway® Vector,Gateway® pDEST™27 Vector, Gateway® pEF-DEST51 Vector, Gateway® pcDNA™-DEST47 vector, pCMV/Bsd Vector, pEF6/His A, B, & c,pcDNA™6.2-DEST, pLenti6/TR, pLP-AcGFP1-C, pLPS-AcGFP1-N,pLP-IRESneo, pLP-TRE2, pLP-RevTRE, pLP-LNCX, pLP-CMV-HA, pLP-CMV-Myc, pLP-RetroQ and pLP-CMVneo.

Optionally, the vectors comprise expression control sequences such as constitutive or conditional promoters, preferably, constitutive promoters are used. One of ordinary skill in the art will be able to select such sequences. For example, suitable promoters include but are not limited to CMV, TK, SV40 and EF-1α. Optionally, the promoters are inducible, temperature regulated, tissue specific, repressible, heat-shock, developmental, cell lineage specific, eukaryotic, prokaryotic or temporal promoters or a combination or recombination of unmodified or mutagenized, randomized, shuffled sequences of any one or more of the above. Further optionally, the protein of interest is expressed by gene activation or episomally.

Optionally, the vector lacks a selectable marker or drug resistance gene. Further optionally, the vector optionally comprises a nucleic acid encoding a selectable marker, such as a protein that confers drug or antibiotic resistance or more generally any product that exerts selective pressure on the cell. Where more than one vector is used, each vector may have the same or a different drug resistance or other selective pressure marker. If more than one of the drug resistance or selective pressure markers are the same, simultaneous selection may be achieved by increasing the level of the drug. Suitable markers are well-known to those of skill in the art and include but are not limited to polypeptides products conferring resistance to any one of the following: Neomycin/G418, Puromycin, hygromycin, Zeocin, methotrexate and blasticidin. Although drug selection (or selection using any other suitable selection marker) is not a required step in producing the cells and cell lines disclosed herein, it may be used to enrich the transfected cell population for stably transfected cells, provided that the transfected constructs are designed to confer drug resistance. If subsequent selection of cells expressing the protein of interest is accomplished using signaling probes, selection too soon following transfection can result in some positive cells that may only be transiently and not stably transfected. However, this effect can be minimized by allowing sufficient cell passage to allow for dilution of transient expression in transfected cells.

Selective pressure can be applied to cells using a variety of compounds or treatments that would be known to one of skill in the art. Without being limited by theory, selective pressure can be applied by exposing cell to conditions that are suboptimal for or deleterious to cell growth, progression of the cell cycle or viability, such that cells that are tolerant or resistant to these conditions are selected for compared to cells that are not tolerant or resistant to these conditions. Conditions that can be used to exert or apply selective pressure include but are not limited to antibiotics, drugs, mutagens, compounds that slow or halt cell growth or the synthesis of biological building blocks, compounds that disrupt RNA, DNA or protein synthesis, deprivation or limitation of nutrients, amino acids, carbohydrates or compounds required for cell growth and viability from cell growth or culture media, treatments such as growth or maintenance of cells under conditions that are suboptimal for cell growth, for instance at suboptimal temperatures, atmospheric conditions (*e.g.,* %carbon dioxide, oxygen or nitrogen or humidity) or in deprived media conditions. Without being limited by theory, selective pressure can be used to select a marker, factor or gene that confers or encodes resistance or tolerance to the selective pressure. For instance, (i) a population of cells can first be exposed to or introduced with such a marker, factor or gene that confers resistance or tolerance to a selective pressure such that each cell will uptake or be modified to comprise a different level or none of the marker, factor or gene, and (ii) the population can then be exposed to the selective pressure for which the marker, factor or gene confers resistance or tolerance such that cells that comprise the marker, factor or gene comprise a growth advantage compared to cells that do not. Without being limited by theory, cells comprising increased levels of the marker, factor or gene will exhibit a proportionally increased tolerance to the corresponding selective pressure. Selective pressure can be used to select for cells comprising a desired property, RNA or protein of interest by association of the property, RNA or protein with a marker, factor or gene that confers tolerance or resistance to the corresponding selective pressure. Without being limited by theory, cells with proportionally increased levels of the desired property, RNA or protein of interest may be selected by applying proportionally increased levels or amounts or selective pressure during the selection process. If cells comprising multiple properties, RNAs or proteins are desired, each of these can be associated a marker, factor or gene that confers resistance to the same or a different form of selective pressure and selection using all of these selective pressures may be used to select for cells comprising all of the desired properties, RNAs or proteins of interest. After selection of cells with desired properties, RNAs or proteins of interest, the selected cells may be maintained under the same, increased or decreased levels, concentrations, doses, or treatments of selective pressure that was used during selection. In some cases, periodic increases of the levels, concentrations, doses, or treatments of selective pressure can be used to select for amplification of cells comprising correspondingly increasing levels of the desired property, RNA or protein of interest. In some cases, following the selection of cells using selective pressure, the selected cells are maintained using reduced levels, concentrations, doses, or treatments of the selective pressure to help ensure that the desired property, RNA or protein that was selected for is maintained in the cells that are maintained.

The level of selective pressure that is used can be determined by one of skill in the art. This can be done for instance by performing a kill curve experiment, where control cells and cells that comprise resistance markers, factors or genes are tested with increasing levels, doses, concentrations or treatments of the selective pressure and the ranges that selected against the negative cells only or preferentially over a desired range of time (*e.g.,* from 1 to 24 hours, 1 to 3 days, 3 to 5 days, 4 to 7 days, 5 to 14 days, 1 to 3 weeks, 2 to 6 weeks, 1 to 2 months, 1 to 3 months, 4, 5, 6, 7, 8, 9, or more than 10 months). The exact levels, concentrations, doses, or treatments of selective pressure that can be used depends on the cells that are used, the desired properties themselves, the markers, factors or genes that confer resistance or tolerance to the selective pressure as well as the levels of the desired properties that are desired in the cells that are selected and one of skill in the art would readily appreciate how to determine appropriate ranges based on these considerations. In some cases following selection, less than 1, 5, 10, 20, 30, 40, 50, 60, 70, 80, 90 or 100% of the levels, concentrations, doses, or treatments of selective pressure used during the selection process are used in the subsequent maintenance of the cells that are selected. In some cases where multiple different selective pressures are used, the levels, concentrations, doses, or treatments of each selective pressure used during the selection process can be reduced in the subsequent maintenance of the cells that are selected, *e.g.,* to less than 1, 5, 10, 20, 30, 40, 50, 60, 70, 80,90 or 100% of those used during the selection process itself. In some embodiments, these reduced levels, concentrations, doses, or treatments are selected such that the cells that are selected to comprise desired properties, RNAs or proteins continue to comprise the desired properties over time in culture. In some embodiments, no more than the levels, concentrations, doses, or treatments necessary to prevent a loss or diminishment of the desired properties in the selected cells is used during the maintenance of the cells over time in culture, for instance to minimize the exposure of the cells to any possible deleterious effects to the cells from the use of higher levels, concentrations, doses, or treatments than necessary for the cells to maintain the properties, RNAs or proteins for which they were selected.

Optionally, cells and cell lines of the present invention are capable of maintaining the properties, RNAs or proteins for which they are selected for (*e.g.,* expression of the proteins or RNAs of interest) in the absence of selective pressure for at least 15 days, 30 days, 45 days, 60 days, 75 days, 100 days, 120 days, or 150 days. Such cells and cell lines may be cultured in the presence of the same, increased, or reduced levels, concentrations, doses, or treatments of selective pressure, as compared to the those used in the selection process. Such cells and cell lines can also be cultured in the absence of any selective pressure. In the case where the levels, concentrations, doses or treatments are reduced, they can be reduced to less than 1, 5, 10, 20, 30, 40, 50, 60, 70, 80, 90 or 100% of the respective levels, concentrations, doses or treatments used during the selection process. In the case where the cells and cell lines express more than one protein or RNA of interest, and expression of each protein or RNA of interest is selected for using a different selective pressure, the levels, concentrations, doses or treatments of each selective pressure may be independently chosen during culturing of the cells and cell lines following selection, *e.g.,* each selective pressure may be independently chosen to be absent in the cell culture, or to be at the same, or an increased or reduced level, concentration, dose or treatment as compared to its respective level, concentration, dose or treatment used during the selection process. In the case where the levels, concentrations, doses or treatments are reduced, they can be reduced to less than 1, 5, 10, 20, 30, 40, 50, 60, 70, 80, 90 or 100% of the respective levels, concentrations, doses or treatments used during the selection process.

Optionally, the protein-encoding nucleic acid sequence further comprises a tag. Such tags may encode, for example, a HIS tag, a myc tag, a hemagglutinin (HA) tag, protein C, VSV-G, FLU, yellow fluorescent protein (YFP), green fluorescent protein, FLAG, BCCP, maltose binding protein tag, Nus-tag, Softag-1, Softag-2, Strep-tag, S-tag, thioredoxin, GST, V5, TAP or CBP. A tag may be used as a marker to determine protein expression levels, intracellular localization, protein-protein interactions, regulation of the protein of interest, or the protein's function. Tags may also be used to purify or fractionate proteins.

In the case of cells and cell lines expressing an RNA of interest, the RNA can be of any type including antisense RNA, short interfering RNA (siRNA), transfer RNA (tRNA), structural RNA, ribosomal RNA, heterogeneous nuclear RNA (hnRNA) and small nuclear RNA (snRNA), messenger RNA (mRNA), RNA that adopts a stem-loop structure, RNA that adopts a hairpin structure, RNA that comprises single stranded RNA, RNA that comprises double stranded RNA, RNA that binds protein, RNA that binds a fluorescent compound, RNA that has biological activity, RNA that encodes a biologically active product, catalytic RNA, RNA oligonucleotide, RNA that can mediate RNAi or RNA that can regulate the level or activity of at least a second RNA.

Optionally, wherein the cells and cell lines of the invention express a functional protein of interest, the protein can be any protein including but not limited to single chain proteins, multi-chain proteins, hetero-multimeric proteins. In the case of multimeric proteins, optionally the cells express all of the subunits that make up the native protein. The protein can have a "wild type" sequence or may be a variant. Optionally, the cells express a protein that comprises a variant of one or more of the subunits including allelic variants, splice variants, truncated forms, isoforms, different stoichiometries of subunits, different assemblies of subunits, differentially folded forms, differentially active forms, forms with different functionalities, forms with different binding properties, forms associated with different accessory factors, forms expressed in different cell backgrounds, forms expressed in different cellular genetic backgrounds, forms expressed in cells with different endogenous expression profiles, differentially localized forms, chimeric or chemically modified forms, enzymatically modified forms, post-translationally modified forms, glycosylated forms, proteolyzed forms, chimeric subunits and mutated forms that comprise amino acid substitutions (conservative or non-conservative), modified amino acids including chemically modified amino acids, and non-naturally occurring amino acids, and combinations thereof. A heteromultimeric protein expressed by cells or cell lines of the invention may comprise subunits from two or more species, such as from species homologs of the protein of interest.

Optionally, the cells disclosed herein express two or more functional proteins of interest. Such expression can be from the introduction of a nucleic acid encoding all or part of a protein of interest, from the introduction of a nucleic acid that activates the transcription of all or part of a protein of interest from an endogenous sequence or from any combination thereof. The cells may express any desired number of proteins of interest. Optionally, the cells express three, four, five, six, or more proteins of interest. For example, cells and cell lines that stably express functional proteins in a pathway of interest, proteins from intersecting pathways including enzymatic pathways, signaling pathways regulatory pathways and the like. Optionally, the cells or cell lines disclosed herein stably express one or more functional RNAs and/or proteins involved in a biological pathway of interest, e.g., protein and/or RNA components of the biological pathway and protein and/or RNA regulators of the biological pathway and/or one or more of its components. Optionally, the biological pathway consists of at least 2, 3, 4, 5, 6, 7, 8, 9, 10, 11, 12, 13, 14, 15, 16, 17 18, 9, 20, 25, 30, 35, 40, 45, or at least 50 protein components. Optionally, the biological pathway consists of at least 2, 3, 4, 5, 6, 7, 8, 9, 10, 11, 12, 13, 14, 15, 16, 17, 18, 19, 20, 25, 30, 35, 40, 45, or at least 50 RNA components. Examples of biological pathways in which functional proteins can be stably expressed by the cells and cell lines disclosed herein include, but are not limited to: 2-arachidonoylglycerol biosynthesis pathway, 5-hydroxytryptamine biosynthesis pathway, 5-hydroxytryptamine degredation pathway, 5ht1 type receptor mediated signaling pathway, 5ht2 type receptor mediated signaling pathway, 5ht3 type receptor mediated signaling pathway, 5ht4 type receptor mediated signaling pathway, acetate utilization pathway, adenine and hypoxanthine salvage pathway, adrenaline and noradrenaline biosynthesis pathway, alanine biosynthesis pathway, allantoin degradation pathway, alpha adrenergic receptor signaling pathway, alzheimer disease-amyloid secretase pathway, alzheimer disease-presenilin pathway, aminobutyrate degradation pathway, anandamide biosynthesis pathway, anandamide degradation pathway, androgen/estrogen/progesterone biosynthesis pathway, angiogenesis pathway, angiotensin ii-stimulated signaling pathway through G proteins and beta-arrestin, apoptosis signaling pathway, arginine biosynthesis pathway, ascorbate degradation pathway, asparagine and aspartate biosynthesis pathway, ATP synthesis pathway, axon guidance mediated by netrin, axon guidance mediated by semaphorins, axon guidance mediated by slit/robo, B cell activation pathway, beta1 adrenergic receptor signaling pathway, beta2 adrenergic receptor signaling pathway, beta3 adrenergic receptor signaling pathway, biotin biosynthesis pathway, blood coagulation pathway, bupropion degradation pathway, cadherin signaling pathway, carnitine metabolism pathway, cell cycle pathway, cholesterol biosynthesis pathway, chorismate biosynthesis pathway, circadian clock system pathway, cobalamin biosynthesis pathway, coenzyme A biosynthesis pathway, coenzyme A linked carnitine metabolism pathway, corticotropin releasing factor receptor signaling pathway, cysteine biosynthesis pathway, cytoskeletal regulation by rho gtpase, de novo purine biosynthesis pathway, de novo pyrimidine deoxyribonucleotide biosynthesis pathway, de novo pyrimidine ribonucleotides biosynthesis pathway, DNA replication, dopamine receptor mediated signaling pathway, egf receptor signaling pathway, endogenous cannabinoid signaling pathway, endothelin signaling pathway, enkephalin release pathway, fas signaling pathway, fgf signaling pathway, flavin biosynthesis pathway, formyltetrahydroformate biosynthesis pathway, fructose galactose metabolism pathway, GABA-b receptor ii signaling pathway, gamma-aminobutyric acid synthesis pathway, general transcription by RNA polymerase I, general transcription regulation, glutamine glutamate conversion pathway, glycolysis pathway, hedgehog signaling pathway, heme biosynthesis pathway, heterotrimeric G-protein signaling pathway-gi alpha and gs alpha mediated pathway, heterotrimeric G-protein signaling pathway-gq alpha and go alpha mediated pathway, heterotrimeric G-protein signaling pathway-rod outer segment phototransduction pathway, histamine h1 receptor mediated signaling pathway, histamine h2 receptor mediated signaling pathway, histamine synthesis pathway, histidine biosynthesis pathway, huntington disease, hypoxia response via hif activation, inflammation mediated by chemokine and cytokine signaling pathway, insulin/igf pathway-mitogen activated protein kinase kinase/map kinase cascade, insulin/igf pathway-protein kinase B signaling cascade, integrin signalling pathway, interferon-gamma signaling pathway, interleukin signaling pathway, ionotropic glutamate receptor pathway, isoleucine biosynthesis pathway, jak/stat signaling pathway, leucine biosynthesis pathway, lipoate biosynthesis pathway, lysine biosynthesis pathway, mannose metabolism pathway, metabotropic glutamate receptor group i pathway, metabotropic glutamate receptor group ii pathway, metabotropic glutamate receptor group iii pathway, methionine biosynthesis pathway, methylcitrate cycle, methylmalonyl pathway, mRNA splicing, muscarinic acetylcholine receptor 1 and 3 signaling pathway, muscarinic acetylcholine receptor 2 and 4 signaling pathway, n-acetylglucosamine metabolism, nicotine degradation pathway, nicotinic acetylcholine receptor signaling pathway, notch signaling pathway, o-antigen biosynthesis pathway, opioid prodynorphin pathway, opioid proenkephalin pathway, opioid proopiomelanocortin pathway, ornithine degradation pathway, oxidative stress response pathway, oxytocin receptor mediated signaling pathway, p38 mapk pathway, p53 pathway, p53 pathway by glucose deprivation, p53 pathway feedback loops 1, p53 pathway feedback loops 2, pantothenate biosynthesis pathway, parkinson disease, PDGF signaling pathway, pentose phosphate pathway, peptidoglycan biosynthesis pathway, phenylacetate degradation pathway, phenylalanine biosynthesis pathway, phenylethylamine degradation pathway, phenylpropionate degradation pathway, pi3 kinase pathway, plasminogen activating cascade, proline biosynthesis pathway, prpp biosynthesis pathway, purine metabolism, pyridoxal phosphate salvage pathway, pyridoxal-5-phosphate biosynthesis pathway, pyrimidine metabolism, pyruvate metabolism, ras pathway, s-adenosylmethionine biosynthesis pathway, salvage pyrimidine deoxyribonucleotides, salvage pyrimidine ribonucleotides, serine glycine biosynthesis pathway, succinate to proprionate conversion, sulfate assimilation pathway, synaptic vesicle trafficking, T cell activation pathway, TCA cycle, tetrahydrofolate biosynthesis pathway, TGF-beta signaling pathway, thiamin biosynthesis pathway, thiamin metabolism, threonine biosynthesis pathway, thyrotropin-releasing hormone receptor signaling pathway, toll receptor signaling pathway, transcription regulation by bzip transcription factor, triacylglycerol metabolism, tryptophan biosynthesis pathway, tyrosine biosynthesis pathway, ubiquitin proteasome pathway, valine biosynthesis pathway, vasopressin synthesis pathway, VEGF signaling pathway, vitamin B6 biosynthesis pathway, vitamin B6 metabolism, vitamin D metabolism and pathway, wnt signaling pathway, and xanthine and guanine salvage pathway.

Other examples of biological pathways in which functional proteins can be stably expressed by the cells and cell lines disclosed herein include, but are not limited to, the following biological processes: amino acid metabolism (*e.g.,* amino acid biosynthesis, amino acid catabolism, amino acid metabolism regulation, amino acid transport and other amino acid metabolism), transport (*e.g.,* amino acid transport, carbohydrate transport, vitamin/cofactor transport, anion transport, cation transport, lipid and fatty acid transport, nucleoside, nucleotide and nucleic acid transport, phosphate transport, extracellular transport and import, small molecule transport and other transports), apoptosis (*e.g.,* induction of apoptosis, inhibition of apoptosis other apoptosis, and other apoptotic processes), blood circulation and gas exchange, carbohydrate metabolism (*e.g.,* carbohydrate transport, disaccharide metabolism, gluconeogenesis, glycogen metabolism, glycolysis, monosaccharide metabolism, other carbohydrate metabolism, other polysaccharide metabolism, pentose-phosphate shunt, regulation of carbohydrate metabolism and tricarboxylic acid pathway), cell adhesion, cell cycle (*e.g.,* cell cycle control, DNA replication, mitosis and other cell cycle processes), cell proliferation and differentiation, cell structure and motility, coenzyme and prosthetic group metabolism (*e.g.,* coenzyme metabolism, porphyrin metabolism, pterin metabolism, vitamin/cofactor transport, vitamin biosynthesis, vitamin catabolism and other coenzyme and prosthetic group metabolism), developmental processes (*e.g.,* ectoderm development, anterior/posterior patterning, determination of dorsal/ventral axis, embryogenesis, endoderm development, fertilization, meiosis, mesoderm development, segment specification, sex determination, oogenesis, spermatogenesis and motility, and other developmental processes), electron transport (*e.g.,* ferredoxin metabolism, oxidative phosphorylation and other pathways of electron transport), homeostasis (calcium ion homeostasis, glucose homeostasis, growth factor homeostasis and other homeostasis activities), immunity and defense (*e.g.,* antioxidation and free radical removal, B-cell- and antibody-mediated immunity, blood clotting, complement-mediated immunity, cytokine/chemokine mediated immunity, detoxification, granulocyte-mediated immunity, interferon-mediated immunity, macrophage-mediated immunity, natural killer cell mediated immunity, stress response, T-cell mediated immunity and other immune and defense processes), intracellular protein traffic (*e.g.,* exocytosis, endocytosis, general vesicle transport, lysosome transport, mitochondrial transport, nuclear transport, peroxisome transport and other intracellular protein traffic), lipid, fatty acid and steroid metabolism (*e.g.,* acyl-coa metabolism, fatty acid beta-oxidation, fatty acid biosynthesis, fatty acid desaturation, lipid and fatty acid binding, lipid and fatty acid transport, lipid metabolism, and other lipid, fatty acid and steroid metabolism, phospholipid metabolism, regulation of lipid, fatty acid and steroid metabolism, bile acid metabolism, cholesterol metabolism, steroid hormone metabolism and other steroid metabolism), muscle contraction, neuronal activities (*e.g.,* action potential propagation, nerve-nerve synaptic transmission, neuromuscular synaptic transmission, neurotransmitter release and other neuronal activities), nitrogen metabolism (*e.g.,* nitric oxide biosynthesis, nitrogen utilization and other nitrogen metabolism), non-vertebrate process, nucleoside, nucleotide and nucleic acid metabolism (*e.g.,* DNA replication, DNA degradation, DNA recombination, DNA repair, chromatin packaging and remodeling, metabolism of cyclic nucleotides, nucleoside, nucleotide and nucleic acid transport, other nucleoside, nucleotide and nucleic acid metabolism, purine metabolism, pyrimidine metabolism, RNA catabolism, RNA localization, regulation of nucleoside, nucleotide metabolism, reverse transcription, RNA metabolism, tRNA metabolism, mRNA capping, mRNA end-processing and stability, mRNA polyadenylation, mRNA splicing, general mRNA transcription activities, other mRNA transcription, mRNA transcription elongation, mRNA transcription initiation, mRNA transcription regulation and mRNA transcription termination), oncogenesis (*e.g.,* oncogene, tumor suppressor and other oncogenesis-related processes), phosphate metabolism *(e.g.,* phosphate transport, polyphosphate biosynthesis, polyphosphate catabolism, regulation of phosphate metabolism and other phosphate metabolism), protein metabolism and modification (*e.g.,* proteolysis, amino acid activation and other protein metabolism, protein biosynthesis, protein complex assembly, protein folding, translational regulation, protein ADP-ribosylation, protein acetylation, protein disulfide-isomerase reaction, protein glycosylation, protein methylation, protein phosphorylation, protein-lipid modification), protein targeting and localization (*e.g.,* asymmetric protein localization and other protein targeting and localization), sensory perception *(e.g.,* olfaction, taste, hearing, pain sensation, pheromone response, vision and other sensory perception), sulfur metabolism (*e.g.,* sulfur redox metabolism and other sulfur metabolism), cell communication (*e.g.,* cell adhesion-mediated signaling, extracellular matrix protein-mediated signaling, ligand-mediated signaling and steroid hormone-mediated signaling), cell surface receptor mediated signal transduction (*e.g.,* cytokine and chemokine mediated signaling pathway, G-protein mediated signaling, receptor protein serine/threonine kinase signaling pathway, receptor protein tyrosine kinase signaling pathway and other receptor mediated signaling pathway), intracellular signaling cascade (*e.g.,* calcium mediated signaling, jak-stat cascade, JNK cascade, MAPKKK cascade, NF-kappaB cascade, NO mediated signal transduction and other intracellular signaling cascade), and other signal transduction processes.

Proteins and/or RNA components of the various biological pathways disclosed herein and their relationship to each other are known to those skilled in the art, and can be found, *e.g.,* at the KEGG pathway database on the internet (http://www.genome.jp/kegg/pathway.html).

Optionally, the cells or cell lines disclosed herein express at least one functional RNA or protein involved in a biological pathway. Optionally embodiments, the cells or cell lines disclosed herein expressing an RNA or protein of interest also express at least one functional RNA or protein component of a biological pathway. Optionally, the cells or cell lines disclosed herein express at least one functional RNA or protein component of a biological pathway, which may or may not be expressed in the cells or cell lines of the same type that are not engineered. Optionally, the cells or cell lines disclosed herein express at least two functional RNA or protein components of a biological pathway that is sufficient to impart at least one activity of the pathway in the cells or cell lines, also referred to herein as "expression of a functional biological pathway.". Optionally, expression of a biological pathway can comprise expression of at least 1, 2, 3, 4, 5, 6, 7, 8, 9 or 10 components of the biological pathway. Optionally, expression of a biological pathway can comprise expression of at least 1%, 2%, 3%, 4%, 5%, 6%, 7%, 8%, 9%, 10%, 20%, 30%, 40%, 50%, 60%, 70%, 80% or 90% or all components of the biological pathway. Expression of at least one functional RNA or protein component of a biological pathway in a cell or cell line in which the biological pathway does not naturally exist may result in reconstitution of at least one activity of the functional biological pathway in the cell or cell line. Expression of at least one functional RNA or protein component of a biological pathway in a cell or cell line in which the biological pathway naturally exists may result in an increase in at least one activity of the functional biological pathway in the cell or cell line. Expression of at least one functional RNA or protein component of a biological pathway in a cell or cell line in which the biological pathway naturally exists may result in an alteration of the net or overall activity of the pathway in the cell. Optionally, a protein of interest expressed in cells or cell lines disclosed herein may be modified, post-translationally modified, glycosylated or altered by co-expression of at least one of the RNA or protein components of a biological pathway. Optionally the protein of interest is an IgG or antibody and the pathway is a glycosylation pathway. Optionally, cell lines in a panel of cell lines each expressing the same protein of interest (*e.g.* an antibody) also each express at least one of the RNA or protein components of a biological pathway (*e.g.,* a glycosylation pathway). Optionally, at least one of the functional RNA or protein components of a biological pathway interacts with (*e.g.,* modifies, alters, glycosylates or binds, either transiently or for an extended period of time) an expressed protein of interest in the cell. However, protein-protein interaction between the expressed functional proteins is not a requirement. For example, cells and cell lines of the invention can express two or more functional proteins related to each other by being components of a same biological pathway, although the two or more functional proteins may not directly interact with each other. A biological pathway may or may not naturally exist in cells or cell lines disclosed herein expressing two or more functional protein components of the biological pathway. In the first case, expression of the two or more functional protein components of the biological pathway may result in an increase in at least one activity of the biological pathway in the cells or cell lines. In the latter case, expression of the two or more functional protein components of the biological pathway may result in reconstitution of at least one activity of or the entire biological pathway in the cells or cell lines. Optionally, at least one or more components of the biological pathway are naturally expressed by the cells or cell lines.

Also disclosed herein is a cell or cell line stably expressing at least one functional protein involved in a biological pathway of interest, wherein the cell or cell line is cultured in the absence of selective pressure and wherein the cell or cell line consistently expresses the at least one functional protein as described herein. Examples of biological pathways that may be used in accordance with the present invention include, but are not limited to, those biological pathways involved in unfolded protein response ("UPR"); cell growth, cell viability, cell death, cell health; protein expression, production, folding, secretion, membrane integration, modification or post-translational modification including glycosylation or enzymatic modification; pathways enriched or more highly expressed or active in antibody producing cells, mammary gland cells salivary cells, or cells that highly express engineered proteins, as compared to other cell types.

Any cell described herein may be used as the host cell to express functional RNAs or proteins involved in a biological pathway. Examples of cells that may be used to express at least one functional RNA or protein involved in a biological pathway include, but are not limited to: CHO, CHOK1, CHOKiSV, PerC6, NS0, 293, 293T and insect cells. Optionally, CHO cells may be used to express at least one component of the UPR pathway. Optionally, CHO cells may be used to express at least one component of the a glycosylation pathway. In some further embodiments, these cells may further express a protein of interest (*e.g.*; an antibody) that is glycosylated.

Optionally, prior to the introduction into a host cell of a nucleic acid that encodes or activates the transcription of a functional protein of interest involved in a biological pathway, the host cell expresses none of the components of the pathway. Further optionally, prior to the introduction into a host cell of a nucleic acid that encodes or activates the transcription of a functional protein of interest involved in a biological pathway, the host cell expresses at least 1, 2, 5, 10, 15, 20, or at least 25 components of the pathway.

Optionally, in addition to expressing one or more functional protein components of a biological pathway, the cells and cell lines disclosed herein also express one or more functional proteins that regulate the biological pathway and/or at least one of its components, *e.g.,* by affecting the expression or function of one or more of the functional protein components in the biological pathway. Such effect(s) may include, but are not limited to: the post-translational modification (*e.g.,* glycosylation), yield, folding, assembly and/or secretion of the one or more functional proteins in the biological pathway. Examples of genes or RNAs (including mutated, spliced, and processed forms) and expression products encoded by these that may be involved in biological pathways *(e.g.,* those involved in UPR, cell viability, protein production, folding, assembly, modification, glycosylation, proteolysis, secretion, integration into membrane of a cell, cell surface presentation or a combination of these) include, but are not limited to: ATF6a spliced, IRE1 a, IRE1b, PERCDC, ATF4, YYI, NF-YA, NF-YB, NF-YC, XBP1 spliced, and EDEM1 (UPR genes); NRF2, HERP, XIAP, GADD34, PPI a, b and g, and DNAJC3 (switch-off genes); BLIMP-1 and XBP1 spliced (genes expressed in B cells); CRT (CaBP3), CNX, ERp57 (PDIA3), BiP, BAP, ERdj3, CaBP1, GRP94 (CaBP4), ERp72 (PDIA4), and cyclophilin B (folding/secretion genes - Class 1 Chaperones); BiP, BAP, ERdj3, CaBP1, GRP94 (CaBP4), ERp72 (PDIA4) and cyclophilin B (Class 2 Chaperones); SDF2-L (glycosylation gene); ERO1a and b, ERAD, mannosidase 1, HRD1 (oxidation genes); STC1 and 2, SERCA1 and 2, COD1 (calcium pumps); INO1, SREBP1 DC, SREBP2DC, and PYC (lipogenesis/metabolism genes); Sec61 Pa,b and g (transport/membrane integration genes); and Bcl-2sp, Bcl-xL, Bim, Ku70, VDAC2, BAP31 and 14-3-3 (cell viability/anti-apoptosis genes).:

Optionally, in addition to the expression of a first protein of interest, or a functional biological pathway or one or more components thereof, the cells and cell lines disclosed herein also express at least a second protein of interest, wherein the expression of the second protein of interest is affected or altered by the expression of the first protein of interest or a functional biological pathway or one or more components thereof. Examples of such effects include, but are not limited to, those at the level of mRNA transcription, splicing, transport, protein translation, post-translational modification (*e.g.,* glycosylation), and protein transport, folding, assembly, membrane integration, secretion and overall production (yield). For example, expression of the first protein of interest or a functional biological pathway or one or more components thereof may result in increased or more efficient or correct mRNA transcription, splicing, transport, protein translation, post-translational modification (*e.g.,* glycosylation), and protein transport, folding, assembly, membrane integration, secretion and/or overall production (yield) of the second protein of interest. Optionally, the second protein of interest is a biologic. Examples of biologics are further provided hereinbelow. Optionally, the cells or cell lines disclosed herein co-express an antibody and a glycosylation pathway.

Optionally, in particular, the protein expressed by the cells or cell lines used in the method are proteins for which functional cell lines have not previously been available in cells of a cell type that does not normally express the protein without cell or genetic engineering. Without being bound by theory, it is believed that some reasons why such cell lines have not heretofore been possible include that the protein is highly complex or only expressed in specialized or rare cells in the absence of cell or genetic engineering and without preparing a large number of cells expressing the protein, it has not been possible to identify one of the possible rare engineered cells in which the protein may be properly or expressed, assembled, modified, localized, functional, associated with accessory factors, or is not associated with cyctotoxicity; or because no ligand or modulator of the protein is known for use in identifying a cell or cell line that expresses the protein in functional form; or because the protein is cytotoxic when expressed outside its natural context, such as in a content that does not naturally express it.

Cells and cell lines disclosed herein can be made that consistently express any protein of interest including but not limited to proteins that are localized in the cytoplasm, proteins that are integrated or associated with at least one membrane of the cell, proteins that are cell-surface localized or proteins that are secreted, or any combination of these. Such proteins include heteromultimeric ion channels, ligand gated (such as GABA A receptor), ion channels (such as CFTR), heteromultimeric ion channels, voltage gated (such as NaV), heteromultimeric ion channel, non-ligand gated (Epithelial sodium channel, ENaC), heterodimeric GPCRs (such as opioid receptors, taste receptors including sweet, umami and bitter), other GPCRs, Orphan GPCRs, GCC, opioid receptors, growth hormone receptors, estrogen/hgh, nuclear or membrane bound, TGF receptors, PPAR nuclear hormone receptor, nicotinics/Ach, immune receptors such as B-cell/T-cell receptors, chemosensory receptors such as receptors for sour, cool, cold, warm, hot, fat, fatty acid or lipid taste or feel, creamyness, touch, pain, mouthfeel and tingle.

Cells and cell lines disclosed herein can express functional proteins including any protein or combination of proteins listed in Tables 7-22 (Mammalian G proteins, Human orphan GPCRs, Human opioid receptors, Human olfactory receptors, Canine olfactory receptors, Mosquito olfactory receptors, Other heteromultimeric receptors and GABA receptors.

The cells and cell lines disclosed herein have a number of attributes that make them particularly advantageous for any use where it is desired that cells provide consistent expression of a functional protein of interest over time. The terms "stable" or "consistent" as applied to the expression of the protein and the function of the protein is meant to distinguish the cells and cell lines disclosed herein from cells with transient expression or variable function, as the terms "stable expression" and "transient expression" would be understood by a person of skill in the art. A cell or cell line disclosed herein may have stable or consistent expression of a functional protein that has less than 1, 2, 3, 4, 5, 6, 7, 8, 9,10, 11, 12,13, 14, 15 or 20 % variation for at least 2-4 days.

Optionally, stability of the cells or cell lines disclosed herein can be differentiated from transient expression.

Optionally, stability of cells or cell lines disclosed herein can be maintained in the absence of selective pressure for one or more of the RNAs or proteins of interest. Optionally, the stability of the cells or cell lines disclosed herein can be maintained in minimal or reduced levels or amounts of selective pressure or drug compared to the levels or amounts that are normally used or used immediately following introduction of nucleic acids encoding the RNAs or proteins of interest into the population of cells that are engineered or that are normally used during methods to select for cells or cell lines with amplified copy numbers of the nucleic acids.

Optionally, the level of stability that is observed in cells or cell lines disclosed herein is higher compared to normal levels that may be achieved in cells or cell lines produced in an average or in most cells of the same cell type.

Optionally, the level of stability that is observed in cells or cell lines disclosed herein is characterized by lower variability in the expression levels, activity or function of the RNA or protein of interest compared to normal values that could be achieved in cells or cell lines produced in an average or in most cells of the same cell type.

Optionally, the length of time for the duration of stability of expression of an RNA or protein of interest in cells or cell lines disclosed herein is longer compared to normal values that could be achieved in the average or in most cells of the same cell type.

Optionally, stability of cells or cell lines disclosed herein can be maintained with minimal or in the absence of observed cyctotoxicity associated with expression of the RNA or protein of interest compared to values that could be achieved in normal or most cells of the same cell type.

Optionally, stability of cells or cell lines of the invention can be maintained with minimal or no alteration of the functional form, function, physiology, pharmacology, assembly, localization, post-translational modification, glycosylation, enzymatic modification, proteolytic modification or stoichiometry of the RNA or protein of interest over time in culture compared to values that could be achieved in normal or most cells of the same cell type. Optionally, these properties may be determined by characterizing the pharmacological profile or response of the cells or cell lines in a cell based assay using compounds that modulate the expression or function of the RNA or protein of interest.

Optionally, the cells or cell lines disclosed herein express the functional RNA or protein of interest, *i.e.,* the cells are consistently functional after growth for at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 110, 120, 130, 140, 150, 160, 170, 180, 190, or 200 days, where consistent expression or consistently functional refers to a level of expression that does not vary by more than: 1%, 2%, 3%, 4%, 5%, 6%, 7%, 8% 9% or 10% over 2 to 4 days of continuous cell culture; 1%, 2%, 4%, 6%, 8%, 10% or 12% over 5 to 15 days of continuous cell culture; 1%, 2%, 4%, 6%, 8%, 10%, 12%, 14%, 16%, 18% or 20% over 16 to 20 days of continuous cell culture; 1%, 2%, 4%, 6%, 8%, 10%, 12%, 14%, 16%, 18%, 20%, 22%, 24% over 21 to 30 days of continuous cell culture; 1%, 2%, 4%, 6%, 8%, 10%, 12%, 14%, 16%, 18%, 20%, 22%, 24%, 26%, 28% or 30% over 30 to 40 days of continuous cell culture; 1%, 2%, 4%, 6%, 8%, 10%, 12%, 14%, 16%, 18%, 20%, 22%, 24%, 26%, 28% or 30% over 41 to 45 days of continuous cell culture; 1%, 2%, 4%, 6%, 8%, 10%, 12%, 14%, 16%, 18%, 20%, 22%, 24%, 26%, 28% or 30% over 45 to 50 days of continuous cell culture; 1 %, 2%, 4%, 6%, 8%, 10%, 12%, 14%, 16%, 18%, 20%, 22%, 24%, 26%, 28%, 30% or 35% over 45 to 50 days of continuous cell culture; 1%, 2%, 4%, 6%, 8%, 10%, 12%, 14%, 16%, 18%, 20%, 22%, 24%, 26%, 28% or 30% or 35% over 50 to 55 days of continuous cell culture; 1%, 2%, 4%, 6%, 8%, 10%, 12%, 14%, 16%, 18%, 20%, 22%, 24%, 26%, 28%, 30% or 35% over 50 to 55 days of continuous cell culture; 1%, 2%, 3%, 4%, 5%, 10%, 15%, 20%, 25%, 30%, 35% or 40% over 55 to 75 days of continuous cell culture; 1%, 2%, 3%, 4%, 5%, 5%, 10%, 15%, 20%, 25%, 30%, 35%, 40% or 45% over 75 to 100 days of continuous cell culture; 1%, 2%, 3%, 4%, 5%, 5%, 10%, 15%, 20%, 25%, 30%, 35%, 40% or 45% over 101 to 125 days of continuous cell culture; 1%, 2%, 3%, 4%, 5%, 5%, 10%, 15%, 20%, 25%, 30%, 35%, 40% or 45% over 126 to 150 days of continuous cell culture; 1%, 2%, 3%, 4%, 5%, 5%, 10%, 15%, 20%, 25%, 30%, 35%, 40% or 45% over 151 to 175 days of continuous cell culture; 1%, 2%, 3%, 4%, 5%, 5%, 10%, 15%, 20%, 25%, 30%, 35%, 40% or 45% over 176 to 200 days of continuous cell culture; 1%, 2%, 3%, 4%, 5%, 5%, 10%, 15%, 20%, 25%, 30%, 35%, 40% or 45% over more than 200 days of continuous cell culture.

Optionally, the cells or cell lines disclosed herein are stable, i.e., the cells or cell lines maintain consistent expression, amount, yield, function or activity of an RNA or protein of interest for at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 110, 120, 130, 140, 150, 160, 170, 180, 190, or 200 days, where "consistent" refers to a level of expression, amount, yield, function or activity of the RNA or protein of interest that does not vary by more than: 1%, 2%, 3%, 4%, 5%, 6%, 7%, 8% 9% or 10% over 2 to 4 days of continuous cell culture; 1%, 2%, 4%, 6%, 8%, 10% or 12% over 5 to 15 days of continuous cell culture; 1%, 2%, 4%, 6%, 8%, 10%, 12%, 14%, 16%, 18% or 20% over 16 to 20 days of continuous cell culture; 1%, 2%, 4%, 6%, 8%, 10%, 12%, 14%, 16%, 18%, 20%, 22%, 24% over 21 to 30 days of continuous cell culture; 1%, 2%, 4%, 6%, 8%, 10%, 12%, 14%, 16%, 18%, 20%, 22%, 24%, 26%, 28% or 30% over 30 to 40 days of continuous cell culture; 1%, 2%, 4%, 6%, 8%, 10%, 12%, 14%, 16%, 18%, 20%, 22%, 24%, 26%, 28% or 30% over 41 to 45 days of continuous cell culture; 1%, 2%, 4%, 6%, 8%, 10%, 12%, 14%, 16%, 18%, 20%, 22%, 24%, 26%, 28% or 30% over 45 to 50 days of continuous cell culture; 1%, 2%, 4%, 6%, 8%, 10%, 12%, 14%, 16%, 18%, 20%, 22%, 24%, 26%, 28%, 30% or 35% over 45 to 50 days of continuous cell culture; 1%, 2%, 4%, 6%, 8%, 10%, 12%, 14%, 16%, 18%, 20%, 22%, 24%, 26%, 28% or 30% or 35% over 50 to 55 days of continuous cell culture; 1%, 2%, 4%, 6%, 8%, 10%, 12%, 14%, 16%, 18%, 20%, 22%, 24%, 26%, 28%, 30% or 35% over 50 to 55 days of continuous cell culture; 1%, 2%, 3%, 4%, 5%, 10%, 15%, 20%, 25%, 30%, 35% or 40% over 55 to 75 days of continuous cell culture; 1%, 2%, 3%, 4%, 5%, 5%, 10%, 15%, 20%, 25%, 30%, 35%, 40% or 45% over 75 to 100 days of continuous cell culture; 1%, 2%, 3%, 4%, 5%, 5%, 10%, 15%, 20%, 25%, 30%, 35%, 40% or 45% over 101 to 125 days of continuous cell culture; 1%, 2%, 3%, 4%, 5%, 5%, 10%, 15%, 20%, 25%, 30%, 35%, 40% or 45% over 126 to 150 days of continuous cell culture; 1%, 2%, 3%, 4%, 5%, 5%, 10%, 15%, 20%, 25%, 30%, 35%, 40% or 45% over 151 to 175 days of continuous cell culture; 1%, 2%, 3%, 4%, 5%, 5%, 10%, 15%, 20%, 25%, 30%, 35%, 40% or 45% over 176 to 200 days of continuous cell culture; 1%, 2%, 3%, 4%, 5%, 5%, 10%, 15%, 20%, 25%, 30%, 35%, 40% or 45% over more than 200 days of continuous cell culture.

Optionally, the cells or cell lines disclosed herein are stable, *i.e.,* the cells or cell lines maintain consistent expression, amount, yield, function or activity of at least two RNAs or proteins of interest for at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 110, 120, 130, 140, 150, 160, 170, 180, 190, or 200 days, where "consistent" refers to a level of expression, stoichiometry, amount, yield, function or activity of the RNAs or proteins of interest that does not vary by more than: 1%, 2%, 3%, 4%, 5%, 6%, 7%, 8% 9% or 10% over 2 to 4 days of continuous cell culture; 1%, 2%, 4%, 6%, 8%, 10% or 12% over 5 to 15 days of continuous cell culture; 1%, 2%, 4%, 6%, 8%, 10%, 12%, 14%, 16%, 18% or 20% over 16 to 20 days of continuous cell culture; 1%, 2%, 4%, 6%, 8%, 10%, 12%, 14%, 16%, 18%, 20%, 22%, 24% over 21 to 30 days of continuous cell culture; 1%, 2%, 4%, 6%, 8%, 10%, 12%, 14%, 16%, 18%, 20%, 22%, 24%, 26%, 28% or 30% over 30 to 40 days of continuous cell culture; 1%, 2%, 4%, 6%, 8%, 10%, 12%, 14%, 16%, 18%, 20%, 22%, 24%, 26%, 28% or 30% over 41 to 45 days of continuous cell culture; 1%, 2%, 4%, 6%, 8%, 10%, 12%, 14%, 16%, 18%, 20%, 22%, 24%, 26%, 28% or 30% over 45 to 50 days of continuous cell culture; 1 %, 2%, 4%, 6%, 8%, 10%, 12%, 14%, 16%, 18%, 20%, 22%, 24%, 26%, 28%, 30% or 35% over 45 to 50 days of continuous cell culture; 1%, 2%, 4%, 6%, 8%, 10%, 12%, 14%, 16%, 18%, 20%, 22%, 24%, 26%, 28% or 30% or 35% over 50 to 55 days of continuous cell culture; 1%, 2%, 4%, 6%, 8%, 10%, 12%, 14%, 16%, 18%, 20%, 22%, 24%, 26%, 28%, 30% or 35% over 50 to 55 days of continuous cell culture; 1 %, 2%, 3%, 4%, 5%, 10%, 15%, 20%, 25%, 30%, 35% or 40% over 55 to 75 days of continuous cell culture; 1%, 2%, 3%, 4%, 5%, 5%, 10%, 15%, 20%, 25%, 30%, 35%, 40% or 45% over 75 to 100 days of continuous cell culture; 1%, 2%, 3%, 4%, 5%, 5%, 10%, 15%, 20%, 25%, 30%, 35%, 40% or 45% over 101 to 125 days of continuous cell culture; 1%, 2%, 3%, 4%, 5%, 5%, 10%, 15%, 20%, 25%, 30%, 35%, 40% or 45% over 126 to 150 days of continuous cell culture; 1%, 2%, 3%, 4%, 5%, 5%, 10%, 15%, 20%, 25%, 30%, 35%, 40% or 45% over 151 to 175 days of continuous cell culture; 1%, 2%, 3%, 4%, 5%, 5%, 10%, 15%, 20%, 25%, 30%, 35%, 40% or 45% over 176 to 200 days of continuous cell culture; 1%, 2%, 3%, 4%, 5%, 5%, 10%, 15%, 20%, 25%, 30%, 35%, 40% or 45% over more than 200 days of continuous cell culture.

Cells may be selected that have desirable properties in addition to the stable expression of functional protein. Any desired property that can be detected may be selected for. Those of skill in the art will aware of such characteristics. By way of non-limiting example, such properties include:

fragility, morphology and adherence to a solid surface, monodispersion by trypsin or cell dissociation reagent, adaptability to the automated culture conditions, performance under serum-containing conditions, performance in serum-free conditions, convertability to serum-free suspension conditions, propensity to form clumps, propensity to form monodisperse cell layers following passaging, resilience, propensity to remain attached to growth chamber surfaces under fluid addition steps of different force, non-fragmented nucleus, lack of intracellular vacuoles, lack of microbial contamination, lack of mycoplasma, lack of viral contamination, clonality, consistency of gross physical properties of cells within wells, propensity for growth below/at/above room temperature, propensity for tolerance of various temperatures for various time periods, propensity of cells to evenly uptake plasmid/oligonucleotides/fluorogenic probes/peptides/proteins/compounds, propensity of cells to withstand incubation with DMSO/EtOH/MeOH, organic solvent/detergent, propensity of cells to withstand maintained UPR induction, propensity of cells to withstand exposure to DTT, propensity of cells to be infected with viral/lentiviral/cosmid vectors, endogenous expression of desired RNA(s)/protein(s) or lack thereof, chromosomal number, chromosomal aberrations, amenable to growth at 5/6/7/8/9pH, tolerance to UV/mutagen/radiation, ability to maintain the above characteristics under altered/manual/scaled-up growth conditions (*i.e.,* including reactors).

Cells and cell lines disclosed herein have enhanced properties as compared to cells and cell lines made by conventional methods. For example, the cells and cell lines disclosed herein have enhanced stability of expression and/or levels of expression (even when maintained in cultures without selective pressure, including, for example, antibiotics and other drugs). Further optionally, the cells and cell lines disclosed herein have high Z' values in various assays. Still further optionally, the cells and cell lines disclosed herein are improved in context of their expression of a physiologically relevant protein activity as compared to more conventionally engineered cells. These properties enhance and improve the ability of the cells and cell lines disclosed herein to be used for any use, whether in assays to identify modulators, for cell therapy, for protein production or any other use and improve the functional attributes of the identified modulators.

Optionally, a further advantageous property of the cells and cell lines disclosed herein is that they stably express the protein of interest in the absence or with reduced drug or other selective pressure that is typically used or that may be used immediately following introduction of nucleic acids encoding the protein of interest into cells or that may be used during procedures to select for cells with amplified copy number of the nucleic acids. Without being bound by theory, cyctotoxicity associated with expression or RNAs or proteins of interest in cells of a cell type that do not normally express the RNAs or proteins of interest may reflect that conditions that are sufficient for non-cytotoxic expression of the RNAs or proteins of interest have not been approximated or recapitulated in the engineered cells. Optionally engineered cells with diminished or that lack cytotoxicity associated with expression of such RNAs or proteins of interest are preferred as diminished or the lack of cytotoxicity could indicate that improved conditions for expression or function of the RNAs or proteins of interest have been achieved. Optionally, engineered cells or cell lines may express an RNA or protein of interest that is not naturally expressed or that is not naturally expressed in the absence of associated cytotoxicity in the same type of cell in the absence of cell or genetic engineering. Optionally, engineered cells or cell lines may express an RNA or protein of interest that is not naturally functionally or properly expressed, folded, assembled, modified, post-translationally modified, localized or active in the same type of cell in the absence of cell or genetic engineering. Optionally, the methods disclosed herein result in cells or cell lines comprising functional, stable, viable or non-cytotoxic expression of RNAs or proteins of interest that had previously been considered to be cytotoxic when expressed in average or most cells of the same cell types. Thus, preferably, the cells and cell lines of the invention are maintained in culture without any selective pressure. Further optionally, cells and cell lines are maintained without any drug or antibiotics. As used herein, cell maintenance refers to culturing cells after they have been selected as described for protein expression. Maintenance does not refer to the optional step of growing cells under selective pressure (e.g., an antibiotic) prior to cell sorting where marker(s) introduced into the cells allow enrichment of stable transfectants in a mixed population.

Drug-free and selective pressure-free cell maintenance of the cells and cell lines disclosed herein provides a number of advantages. For example, drug-resistant cells may not express the co-transfected transgene of interest at adequate levels, because the selection relies on survival of the cells that have taken up the drug resistant gene, with or without the transgene. Further, cytotoxicity or the requirement for drug selection or other selective pressure to maintain expression or function of a RNA or protein of interest could indicate that optimal conditions for expression or function of the RNA of protein of interest have not been achieved. Further, selective drugs and other selective pressure factors are often mutagenic or otherwise interfere with the physiology of the cells, leading to skewed results in cell-based assays. For example, selective drugs may decrease susceptibility to apoptosis (Robinson et al., Biochemistry, 36(37):11169-11178 (1997)), increase DNA repair and drug metabolism (Deffie et al., Cancer Res. 48(13):3595-3602 (1988)), increase cellular pH (Thiebaut et al., J Histochem Cytochem. 38(5):685-690 (1990); Roepe et al., Biochemistry. 32(41):11042-11056 (1993); Simon et al., Proc Natl Acad Sci USA. 91 (3):1128-1132 (1994)), decrease lysosomal and endosomal pH (Schindler et al., Biochemistry. 35(9):2811-2817 (1996); Altan et al., J Exp Med. 187(10):1583-1598 (1998)), decrease plasma membrane potential (Roepe et al., Biochemistry. 32(41):11042-11056 (1993)), increase plasma membrane conductance to chloride (Gill et al., Cell. 71(1):23-32 (1992)) and ATP (Abraham et al., Proc Natl Acad Sci USA. 90(1):312-316 (1993)), and increase rates of vesicle transport (Altan et al., Proc Natl Acad Sci USA. 96(8):4432-4437 (1999)). Thus, the cells and cell lines disclosed herein allow screening assays that are free from the artifacts caused by selective pressure. Preferably, the cells and cell lines of this invention are not cultured with selective pressure factors, such as antibiotics, before or after cell sorting, so that cells and cell lines with desired properties are isolated by sorting, even when not beginning with an enriched cell population.

The cells and cell lines disclosed herein have enhanced stability as compared to cells and cell lines produced by conventional methods in the context of expression and expression levels (RNA or protein). To identify cells and cell lines characterized by such stable expression, a cell or cell line's expression of a protein of interest is measured over a timecourse and the expression levels are compared. Stable cell lines will continue expressing (RNA or protein) throughout the timecourse. In some aspects of the invention, the timecourse may be for at least one week, two weeks, three weeks, *etc.,* or at least one month, or at least two, three, four, five, six, seven, eight or nine months, or any length of time in between.

Isolated cells and cell lines may be further characterized, such as by PCR, RT-PCR, qRT-PCR and single end-point RT-PCR to determine the absolute amounts and relative amounts (in the case of multisubunit proteins or multiple proteins of interest) being expressed (RNA). Preferably, the expansion levels of the subunits of a multi-subunit protein are substantially the same in the cells and cell lines disclosed herein.

Further optionally, the expression of a functional protein of interest is assayed over time. In these cases, stable expression is measured by comparing the results of functional assays over a timecourse. The assay of cell and cell line stability based on a functional assay provides the benefit of identifying cells and cell lines that not only stably express the protein (RNA or protein), but also stably produce and properly process (*e.g.,* post-translational modification, subunit assembly, and localization within the cell) the protein to produce a functional protein.

Cells and cell lines disclosed herein have the further advantageous property of providing assays with high reproducibility as evidenced by their Z' factor. See Zhang JH, Chung TD, Oldenburg KR, "A Simple Statistical Parameter for Use in Evaluation and Validation of High Throughput Screening Assays." J. Biomol. Screen. 1999;4(2):67-73, which is incorporated herein by reference in its entirety. *Z*' values relate to the quality of a cell or cell line because it reflects the degree to which a cell or cell line will respond consistently to modulators. Z' is a statistical calculation that takes into account the signal-to-noise range and signal variability (*i.e.,* from well to well) of the functional response to a reference compound across a multiwell plate. is Z' calculated using Z' data obtained from multiple wells with a positive control and multiple wells with a negative control. The ratio of their combined standard deviations multiplied by three to the difference factor, in their mean values is subtracted from one to give the Z' according the equation below:

$$Z' \text{ factor} = 1 - ((3\sigma_{\text{positive control}} + 3\sigma_{\text{negative control}}) / (\mu_{\text{positive control}} - \mu_{\text{negative control}}))$$

If the factor is 1.0, which would indicate an ideal assay with theoretical maximum Z' no variability and limitless dynamic range. As used herein, a "high Z'" refers to a Z' factor of Z' of at least 0.6, at least 0.7, at least 0.75 or at least 0.8, or any decimal in between 0.6 and 1.0. In the case of a complex target, a high Z' means a Z' of at least 0.4 or greater. A score of close to 0 is undesirable because it indicates that there is overlap between positive and negative controls. In the industry, for simple cell-based assays, Z' scores up to 0.3 are considered marginal scores, Z' scores between 0.3 and 0.5 are considered acceptable, and Z' scores above 0.5 are considered excellent. Cell-free or biochemical assays may approach scores for cell-based systems tend to be lower because higher Z' scores, but Z' cell-based systems are complex.

As those of ordinary skill in the art will recognize, cell-based assays using conventional cells expressing even a single chain protein do not typically achieve a Z' score higher than 0.5 to 0.6. Z' scores of cells with engineered expression (either from introduced coding sequences or gene activation) of multi-subunit proteins, if even reported in the art, would be lower due to their added complexity. Such cells would not be reliable for use in assays because the results would not be reproducible. Cells and cell lines disclosed herein, on the other hand, have higher Z' values and advantageously produce consistent results in assays. Indeed, the cells and cell lines disclosed herein provide the basis for high throughput screening (HTS) compatible assays because they generally have higher Z' score values than conventionally produced cells. In some aspects disclosed herein, the cells and cell lines result in Z' of at least 0.3, at least 0.4, at least 0.5, at least 0.6, at least 0.7, or at least 0.8. Even Z' values of at least 0.3 - 0.4 for the cells and cell lines of the invention are advantageous because the proteins of interest are multigene targets. In other aspects disclosed herein, the cells and cell lines of the invention result in a Z' of at least 0.7, at least 0.75 or at least 0.8 even after the cells are maintained for multiple passages, *e.g.*, between 5-20 passages, including any integer in between 5 and 20. In some aspects disclosed herein, the cells and cell lines result in a Z' of at least 0.7, at least 0.75 or at least 0.8 in cells and cell lines maintained for 1, 2, 3, 4 or 5 weeks or 2, 3, 4, 5, 6, 7, 8 or 9 months, including any period of time in between.

Optionally, the cells and cell lines disclosed herein express a protein of interest wherein one or more physiological properties remain(s) substantially constant over time.

A physiological property includes any observable, detectable or measurable property of cells or cell lines apart from the expression of the protein of interest.

Optionally, the expression of a protein of interest can alter one or more physiological properties. Alteration of a physiological property includes any change of the physiological property due to the expression of the protein of interest, *e.g.,* a stimulation, activation, or increase of the physiological property, or an inhibition, blocking, or decrease of the physiological property. Without being bound by theory, in these embodiments, the one or more constant physiological properties indicate that the functional expression of the protein of interest also remains constant. Optionally, one or more constant physical properties associated with a taste receptor (*e.g.*, sweet taste receptor, umami taste receptor, or bitter taste receptor), which are discussed in more detail below, can be used to monitor the expression of functional taste receptors.

Also disclosed herein is a method for culturing a plurality of cells or cell lines expressing a protein of interest under constant culture conditions, wherein cells or cell lines can be selected that have one or more desired properties, such as stable expression of the protein of interest and/or one or more substantially constant physiological properties.

Optionally where a physiological property can be measured, the physiological property is determined as an average of the physiological property measured in a plurality of cells or a plurality of cells of a cell line. In certain specific embodiments, a physiological property is measured over at least 10; 100; 1,000; 10,000; 100,000; 1,000,000; or at least 10,000,000 cells and the average remains substantially constant over time.

Optionally, the average of a physiological property is determined by measuring the physiological property in a plurality of cells or a plurality of cells of a cell line wherein the cells are at different stages of the cell cycle. In other embodiments, the cells are synchronized with respect to cell cycle.

Optionally, a physiological property is observed, detected, measured or monitored on a single cell level. In certain embodiments, the physiological property remains substantially constant over time on a single cell level.

Optionally, a physiological property remains substantially constant over time if it does not vary more than 0.1%, 0.5%, 1%, 2.5%, 5%, 10%, 15%, 20%, 25%, 30%, 35%, 40%, 45%, or no more than 50% over 12 hours. Optionally, a physiological property remains substantially constant over time if it does not vary more than 0.1%, 0.5%, 1%, 2.5%, 5%, 10%, 15%, 20%, 25%, 30%, 35%, 40%, 45%, or no more than 50% over 1 day. Optionally, a physiological property remains substantially constant over time if it does not vary more than 0.1%, 0.5%, 1%, 2.5%, 5%, 10%, 15%, 20%, 25%, 30%, 35%, 40%, 45%, or no more than 50% over 2 days. Optionally, a physiological property remains substantially constant over time if it does not vary more than 0.1%, 0.5%, 1%, 2.5%, 5%, 10%, 15%, 20%, 25%, 30%, 35%, 40%, 45%, or no more than 50% over 5 days. Optionally, a physiological property remains substantially constant over time if it does not vary more than 0.1%, 0.5%, 1%, 2.5%, 5%, 10%, 15%, 20%, 25%, 30%, 35%, 40%, 45%, or no more than 50% over 10 days. Optionally, a physiological property remains substantially constant over time if it does not vary more than 0.1 %, 0.5%, 1%, 2.5%, 5%, 10%, 15%, 20%, 25%, 30%, 35%, 40%, 45%, or no more than 50% over 20 days. Optionally, a physiological property remains substantially constant over time if it does not vary more than 0.1 %, 0.5%, 1%, 2.5%, 5%, 10%, 15%, 20%, 25%, 30%, 35%, 40%, 45%, or no more than 50% over 30 days. Optionally, a physiological property remains substantially constant over time if it does not vary more than 0.1 %, 0.5%, 1%, 2.5%, 5%, 10%, 15%, 20%, 25%, 30%, 35%, 40%, 45%, or no more than 50% over 40 days. Optionally, a physiological property remains substantially constant over time if it does not vary more than 0.1 %, 0.5%, 1%, 2.5%, 5%, 10%, 15%, 20%, 25%, 30%, 35%, 40%, 45%, or no more than 50% over 50 days. Optionally, a physiological property remains substantially constant over time if it does not vary more than 0.1%, 0.5%, 1%, 2.5%, 5%, 10%, 15%, 20%, 25%, 30%, 35%, 40%, 45%, or no more than 50% over 60 days. Optionally, a physiological property remains substantially constant over time if it does not vary more than 0.1%, 0.5%, 1%, 2.5%, 5%, 10%, 15%, 20%, 25%, 30%, 35%, 40%, 45%, or no more than 50% over 70 days. Optionally, a physiological property remains substantially constant over time if it does not vary more than 0.1 %, 0.5%, 1%, 2.5%, 5%, 10%, 15%, 20%, 25%, 30%, 35%, 40%, 45%, or no more than 50% over 80 days. Optionally, a physiological property remains substantially constant over time if it does not vary more than 0.1 %, 0.5%, 1%, 2.5%, 5%, 10%, 15%, 20%, 25%, 30%, 35%, 40%, 45%, or no more than 50% over 90 days. Optionally, a physiological property remains substantially constant over time if it does not vary more than 0.1%, 0.5%, 1%, 2.5%, 5%, 10%, 15%, 20%, 25%, 30%, 35%, 40%, 45%, or no more than 50% over the course of 1 passage, 2 passages, 3 passages, 5 passages, 10 passages, 25 passages, 50 passages, or 100 passages.

Examples of cell physiological properties include, but are not limited to: growth rate, size, shape, morphology, volume; profile or content of DNA, RNA, protein, lipid, ion, carbohydrate or water; endogenous, engineered, introduced, gene-activated or total gene, RNA or protein expression or content; propensity or adaptability to growth in adherent, suspension, serum-containing, serum-free, animal-component free, shaken, static or bioreactor growth conditions; propensity or adaptability to growth in or on chips, arrays, microarrays, slides, dishes, plates, multiwell plates, high density multiwell plates, flasks, roller bottles, bags or tanks; propensity or adaptability to growth using manual or automated or robotic cell culture methodologies; abundance, level, number, amount or composition of at least one cell organelle, compartment or membrane, including, but not limited to cytoplasm, nucleoli, nucleus, ribosomes, rough endoplasmic reticulum, Golgi apparatus, cytoskeleton, smooth endoplasmic reticulum, mitochondria, vacuole, cytosol, lysosome, centrioles, chloroplasts, cell membrane, plasma cell membrane, nuclear membrane, nuclear envelope, vesicles (*e.g.*, secretory vesicles), or membrane of at least one organelle; having acquired or having the capacity or propensity to acquire at least one functional or gene expression profile (of one or more genes) shared by one or more specific cell types or differentiated, undifferentiated or dedifferentiated cell types, including, but not limited to: a stem cell, a pluripotent cell, an omnipotent cell or a specialized or tissue specific cell including one of the liver, lung, skin, muscle (including but not limited to: cardiac muscle, skeletal muscle, striatal muscle), pancreas, brain, testis, ovary, blood, immune system, nervous system, bone, cardiovascular system, central nervous system, gastro-intestinal tract, stomach, thyroid, tongue, gall bladder, kidney, nose, eye, nail, hair, taste bud cell or taste cell, neuron, skin, pancreas, blood, immune, red blood cell, white blood cell, killer T-cell, enteroendocrine cell, secretory cell, kidney, epithelial cell, endothelial cell, a human, animal or plant cell; ability to or capacity to uptake natural or synthetic chemicals or molecules including, but not limited to: nucleic acids, RNA, DNA, protein, small molecules, probes, dyes, oligonucleotides (including modified oligonucleotides) or fluorogenic oligonucleotides; resistance to or capacity to resist negative or deleterious effects of chemicals or substances that negatively affect cell growth, function or viability, including, but not limited to: resistance to infection, drugs, chemicals, pathogens, detergents, UV, adverse conditions, cold, hot, extreme temperatures, shaking, perturbation, vortexing, lack of or low levels of oxygen, lack of or low levels of nutrients, toxins, venoms, viruses or compound, treatment or agent that has an adverse effect on cells or cell growth; suitability for use in in vitro tests, cell based assays, biochemical or biological tests, implantation, cell therapy or secondary assays, including, but not limited to: large scale cell culture, miniaturized cell culture, automated cell culture, robotic cell culture, standardized cell culture, drug discovery, high throughput screening, cell based assay, functional cell based assay (including but not limited to membrane potential assays, calcium flux assays, reporter assays, G-protein reporter assays), ELISA, in vitro assays, in vivo applications, secondary testing, compound testing, binding assays, panning assays, antibody panning assays, phage display, imaging studies, microscopic imaging assays, immunofluorescence studies, RNA, DNA, protein or biologic production or purification, vaccine development, cell therapy, implantation into an organism, animal, human or plant, isolation of factors secreted by the cell, preparation of cDNA libraries, or infection by pathogens, viruses or other agent; and other observable, measurable, or detectable physiological properties such as: biosynthesis of at least one metabolite, lipid, DNA, RNA or protein; chromosomal silencing, activation, heterochromatization, euchromoatinization or recombination; gene expression, gene silencing, gene splicing, gene recombination or gene-activation; RNA production, expression, transcription, processing splicing, transport, localization or modification; protein production, expression, secretion, folding, assembly, transport, localization, cell surface presentation, secretion or integration into a cell or organelle membrane; protein modification including but not limited to post-translational modification, processing, enzymatic modification, proteolysis, glycosylation, phosphorylation, dephosphorylation; cell division including mitosis, meiosis or fission or cell fusion; high level RNA or protein production or yield.

Physiological properties may be observed, detected or measured using routine assays known in the art, including but not limited to tests and methods described in reference guides and manuals such as the Current Protocols series. This series includes common protocols in various fields and is available through the Wiley Publishing House. The protocols in these reference guides are illustrative of the methods that can be used to observe, detect or measure physiological properties of cells. The skilled worker would readily recognize any one or more of these methods may be used to observe, detect or measure the physiological properties disclosed herein.

Many markers, dyes or reporters, including proteins markers expressed as fusion proteins comprising an autofluorescent protein, that can be used to measure the level, activity or content of cellular compartments or organelles including but not limited to ribosomes, mitochondria, ER, rER, golgi, TGN, vesicles, endosomes and plasma membranes in cells are compatible with the testing of individual viable cells. Optionally fluorescence activated cell sorting or a cell sorter can be used. Optionally, cells or cell lines isolated or produced to comprise an RNA or protein of interest can be tested using these markers, dyes or reporters at the same time, subsequent, or prior to isolation, testing or production of the cells or cell lines comprising the RNA or protein of interest. Optionally, the level, activity or content of one or more of the cellular compartments or organelles can be correlated with improved, increased, native, non-cytotoxic, viable or optimal expression, function, activity, folding, assembly modification, post-translational modification, secretion, cell surface presentation, membrane integration, pharmacology, yield or physiology of the RNA or protein of interest. In some embodiments, cells or cell lines comprising the level, activity or content of at least one cellular compartment or organelle that is correlated with improved, increased, native, non-cytotoxic, viable or optimal expression, function, activity, folding, assembly modification, post-translational modification, secretion, cell surface presentation, membrane integration, pharmacology, yield or physiology of the RNA or protein of interest can be isolated. In some embodiments, cells or cell lines comprising the RNA or protein of interest and the level, activity or content of at least one cellular compartment or organelle that is correlated with improved, increased, native, non-cytotoxic, viable or optimal expression, function, activity, folding, assembly modification, post-translational modification, secretion, cell surface presentation, membrane integration, pharmacology, yield or physiology of the RNA or protein of interest can be isolated. In some embodiments the isolation of the cells is performed using cell sorting or fluorescence activated cell sorting.

Optionally, populations of diverse cells that can be engineered to comprise an RNA or protein of interest can be exposed to, introduced with or engineered to comprise at least 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 125, 150, 175 or 200 additional nucleic acid sequences at the same time, prior or subsequent to isolation, testing or production of cells or cell lines engineered to comprise the RNA or protein of interest. Optionally the additional nucleic acids can be selected from the group consisting of: RNAs, DNAs or genes encoding regulators of the unfolded protein response (UPR); RNAs, DNAs, or genes that are regulated in or in the state of UPR; RNAs, DNAs, or genes that regulate cell growth, cell viability, cell death, cell health; RNAs, DNAs, or genes that regulate RNA or protein expression, production, folding, secretion, yield, membrane integration, modification or post-translational modification including glycosylation or enzymatic modification; RNAs, DNAs or genes that are enriched in antibody producing cells compared to other cell types.

Optionally, the expression of at least one of the nucleic acids in cells can be correlated with improved, increased, native, non-cytotoxic, viable or optimal expression, function, activity, folding, assembly modification, post-translational modification, secretion, cell surface presentation, membrane integration, pharmacology, yield or physiology of the RNA or protein of interest. Optionally, cells or cell lines comprising at least one nucleic acid that is correlated with improved, increased, native, non-cytotoxic, viable or optimal expression, function, activity, folding, assembly modification, post-translational modification, secretion, cell surface presentation, membrane integration, pharmacology, yield or physiology of the RNA or protein of interest can be isolated. In some embodiments, populations of diverse cells that can be engineered to comprise an RNA or protein of interest can be exposed to, introduced with or engineered to comprise at least 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 125, 150, 175 or 200 additional nucleic acid sequences at the same time, prior or subsequent to isolation, testing or production of cells or cell lines engineered to comprise the RNA or protein of interest and cells or cell lines comprising the RNA or protein of interest and the level, activity or content of at least one cellular compartment or organelle that is correlated with improved, increased, native, non-cytotoxic, viable or optimal expression, function, activity, folding, assembly modification, post-translational modification, secretion, cell surface presentation, membrane integration, pharmacology, yield or physiology of the RNA or protein of interest can be isolated. In some embodiments the isolation of the cells is performed using cell sorting or fluorescence activated cell sorting.

Cells or preparations made from cells may be tested to analyze DNA, RNA, protein content, organization, expression or profiles using methods and tests including sequencing, PCR methods including PCR, RT-PCR, qRT-PCR, RACE, hybridization methods including northern and southern blots, FISH, in situ hybridization, microscopy including fluorescence, electron, confocal or immunofluorescence microscopy or array or microarray tests such as genechips or protein arrays, for instance to identify expression profiles or one or more genes whose expression impacts, benefits or is detrimental to the expression of a target, for instance by affecting its functional, viable or stable expression, or to a cell-physiological property. Optionally such tests are conducted to identify one or more endogenous factors that influence, benefit or affect the functional, viable or stable expression of a protein of interest or physiological property of interest.

Cells or preparations of made from cells may be tested to analyze or characterize cellular content including metabolites, DNA, RNA, protein, membranes, lipids, carbohydrates or organelles using methods and tests including centrifugation, ultracentrifugation, floating, sucrose gradients, HPLC, FPLC, subcellular fractionation, metabolite analysis, chemical composition analysis, chromosomal spreads, DAPI labeling, NMR, enzyme assays, ELISAs;

Protein produced by cells may be tested to assess its sequence, function, form, folding, membrane integration, abundance, yield, post-translational modification, glycosylation, phosphorylation, cleavage, proteolysis or degradation using sequencing, antibody binding ELISA or activity assays.

Cells may be tested and characterized by tests for cell growth, mitosis, meiosis, gene integration, gene activation, gene introduction or gene expression or silencing. Optionally, such tests are conducted to identify sites of integration of any transgenes in the genome of the cell.

Optionally, the expression profile (*e.g.*, profile of gene expression or protein expression) of a cell or cell line disclosed herein can be compared to the expression profile of a reference cell or cell line. Any method known to the skilled artisan can be used to measure the expression profile of one or more nucleic acid or amino acid sequences. Exemplary methods are gene chip, protein chip *etc.* Optionally, the expression of at least 0.1%, 0.5%, 1%, 2.5%, 5%, 10%, 15%, 20%, 25%, 30%, 35%, 40%, 45%, 50%, 60%, 70%, 80%, 90%, 95%, 98%, 99%, or 100% of the genes in a genome are assayed. Optionally, at least 0.1%, 0.5%, 1%, 2.5%, 5%, 10%, 15%, 20%, 25%, 30%, 35%, 40%, 45%, 50%, 60%, 70%, 80%, 90%, 95%, 98%, 99%, or 100% of the nucleic acid or amino acid sequences in a cell are assayed. Optionally, the expression of at least 1, 2, 3, 4, 5, 10, 15, 20, 25, 30, 35, 40, 50, 60, 70, 80, 90 or 100 or more than 100 of the genes in a genome are assayed. Optionally, at least at least 1, 2, 3,4, 5,10,15, 20, 25, 30, 35, 40, 50, 60, 70, 80, 90 or 100 or more than 100 of the nucleic acid or amino acid sequences in a cell are assayed.

Optionally, the reference cell or cell line is the host cell from which the cell or cell line disclosed herein was generated. Further optionally, the cell or cell line and the reference cell or cell line are derived from the same parent clone. Optionally, the cell or cell line and the reference cell or cell line are derived from the same parent cell. Further optionally, the reference cell or cell line is a cell or cell line of a cell type that the cell or cell line in accordance with the invention was designed to approximate. Such cell types include, but are not limited to: epidermal keratinocyte (differentiating epidermal cell), epidermal basal cell (stem cell), keratinocyte of fingernails and toenails, nail bed basal cell (stem cell), medullary hair shaft cell, cortical hair shaft cell, cuticular hair shaft cell, cuticular hair root sheath cell, hair root sheath cell of Huxley's layer, hair root sheath cell of Henle's layer, external hair root sheath cell, hair matrix cell (stem cell), surface epithelial cell of stratified squamous epithelium of cornea, tongue, oral cavity, esophagus, anal canal, distal urethra and vagina, basal cell (stem cell) of epithelia of cornea, tongue, oral cavity, esophagus, anal canal, distal urethra and vagina, urinary epithelium cell (lining urinary bladder and urinary ducts), salivary gland mucous cell (polysaccharide-rich secretion), salivary gland serous cell (glycoprotein enzyme-rich secretion), von Ebner's gland cell in tongue (washes taste buds), mammary gland cell (milk secretion), lacrimal gland cell (tear secretion), ceruminous gland cell in ear (wax secretion), eccrine sweat gland dark cell (glycoprotein secretion), eccrine sweat gland clear cell (small molecule secretion), apocrine sweat gland cell (odoriferous secretion, sex-hormone sensitive), gland of Moll cell in eyelid (specialized sweat gland), sebaceous gland cell (lipid-rich sebum secretion), bowman's gland cell in nose (washes olfactory epithelium), Brunner's gland cell in duodenum (enzymes and alkaline mucus), seminal vesicle cell (secretes seminal fluid components, including fructose for swimming sperm), prostate gland cell (secretes seminal fluid components), bulbourethral gland cell (mucus secretion), Bartholin's gland cell (vaginal lubricant secretion), gland of Littre cell (mucus secretion), uterus endometrium cell (carbohydrate secretion), isolated goblet cell of respiratory and digestive tracts (mucus secretion), stomach lining mucous cell (mucus secretion), gastric gland zymogenic cell (pepsinogen secretion), gastric gland oxyntic cell (hydrochloric acid secretion), pancreatic acinar cell (bicarbonate and digestive enzyme secretion), paneth cell of small intestine (lysozyme secretion), type II pneumocyte of lung (surfactant secretion), clara cell of lung, anterior pituitary cells, somatotropes, lactotropes, thyrotropes, gonadotropes, corticotropes, intermediate pituitary cell, secreting melanocyte-stimulating hormone, magnocellular neurosecretory cells (secreting oxytocin and/or secreting vasopressin), gut and respiratory tract cells (secreting serotonin, secreting endorphin, secreting somatostatin, secreting gastrin, secreting secretin, secreting cholecystokinin, secreting insulin, secreting glucagons, and/or secreting bombesin), thyroid gland cells, thyroid epithelial cell, parafollicular cell, parathyroid gland cells, parathyroid chief cell, oxyphil cell, adrenal gland cells, chromaffin cells, adrenal gland secreting steroid hormones (mineralocorticoids and glucocorticoids), Leydig cell of testes secreting testosterone, theca interna cell of ovarian follicle secreting estrogen, corpus luteum cell of ruptured ovarian follicle secreting progesterone (Granulosa lutein cells, and Theca lutein cells), juxtaglomerular cell (renin secretion), macula densa cell of kidney, peripolar cell of kidney, mesangial cell of kidney, hepatocyte (liver cell), white fat cell, brown fat cell, liver lipocyte, kidney glomerulus parietal cell, kidney glomerulus podocyte, kidney proximal tubule brush border cell, loop of Henle thin segment cell, kidney distal tubule cell, kidney collecting duct cell, type I pneumocyte (lining air space of lung), pancreatic duct cell (centroacinar cell), nonstriated duct cell (of sweat gland, salivary gland, mammary gland, *etc.)* such as principal cell and intercalated cell, duct cell (of seminal vesicle, prostate gland, *etc.*), intestinal brush border cell (with microvilli), exocrine gland striated duct cell, gall bladder epithelial cell, ductulus efferens nonciliated cell, epididymal principal cell, epididymal basal cell, blood vessel and lymphatic vascular endothelial fenestrated cell, blood vessel and lymphatic vascular endothelial continuous cell, blood vessel and lymphatic vascular endothelial splenic cell, synovial cell (lining joint cavities, hyaluronic acid secretion), serosal cell (lining peritoneal, pleural, and pericardial cavities), squamous cell (lining perilymphatic space of ear), squamous cell (lining endolymphatic space of ear), columnar cell of endolymphatic sac with microvilli (lining endolymphatic space of ear), columnar cell of endolymphatic sac without microvilli (lining endolymphatic space of ear), dark cell (lining endolymphatic space of ear), vestibular membrane cell (lining endolymphatic space of ear), stria vascularis basal cell (lining endolymphatic space of ear), stria vascularis marginal cell (lining endolymphatic space of ear), cell of Claudius (lining endolymphatic space of ear), cell of Boettcher (lining endolymphatic space of ear), choroid plexus cell (cerebrospinal fluid secretion), pia-arachnoid squamous cell, pigmented ciliary epithelium cell of eye, nonpigmented ciliary epithelium cell of eye, corneal endothelial cell, respiratory tract ciliated cell, oviduct ciliated cell (in female), uterine endometrial ciliated cell (in female), rete testis ciliated cell (in male), ductulus efferens ciliated cell (in male), ciliated ependymal cell of central nervous system (lining brain cavities), ameloblast epithelial cell (tooth enamel secretion), planum semilunatum epithelial cell of vestibular apparatus of ear (proteoglycan secretion), organ of Corti interdental epithelial cell (secreting tectorial membrane covering hair cells), loose connective tissue fibroblasts, corneal fibroblasts (corneal keratocytes), tendon fibroblasts, bone marrow reticular tissue fibroblasts, other nonepithelial fibroblasts, pericyte, nucleus pulposus cell of intervertebral disc, cementoblast/cementocyte (tooth root bonelike cementum secretion), ontoblast/odontocyte (tooth dentin secretion), hyaline cartilage chondrocyte, fibrocartilage chondrocyte, elastic cartilage chondrocyte, oteoblast/osteocyte, osteoprogenitor cell (stem cell of osteoblasts), hyalocyte of vitreous body of eye, stellate cell of perilymphatic space of ear, hepatic stellate cell (Ito cell), pancreatic stellate cell, skeletal muscle cells (such as Red skeletal muscle cell (slow), white skeletal muscle cell (fast), intermediate skeletal muscle cell, nuclear bag cell of muscle spindle, and nuclear chain cell of muscle spindle), satellite cell (stem cell), heart muscle cells (such as ordinary heart muscle cell, nodal heart muscle cell, and purkinje fiber cell), smooth muscle cell (various types), myoepithelial cell of iris, myoepithelial cell of exocrine glands, erythrocyte (red blood cell), megakaryocyte (platelet precursor), monocytes, connective tissue macrophage (various types), epidermal Langerhans cell, osteoclast (in bone), dendritic cell (in lymphoid tissues), microglial cell (in central nervous system), neutrophil granulocyte, eosinophil granulocyte, basophil granulocyte, mast cell, helper T cell, suppressor T cell, cytotoxic T cell, natural Killer T cell, B cell, natural killer cell, reticulocyte, stem cells and committed progenitors for the blood and immune system (various types), auditory outer hair cell of organ of Corti, basal cell of olfactory epithelium (stem cell for olfactory neurons), cold-sensitive primary sensory neurons, heat-sensitive primary sensory neurons, merkel cell of epidermis (touch sensor), olfactory receptor neuron, pain-sensitive primary sensory neurons (various types), photoreceptor cells of retina in eye (such as photoreceptor rod cells, photoreceptor blue-sensitive cone cell of eye, photoreceptor green-sensitive cone cell of eye, photoreceptor red-sensitive cone cell of eye), proprioceptive primary sensory neurons (various types), touch-sensitive primary sensory neurons (various types), type I carotid body cell (blood pH sensor), type II carotid body cell (blood pH sensor), type I hair cell of vestibular apparatus of ear (acceleration and gravity), type II hair cell of vestibular apparatus of ear (acceleration and gravity), type I taste bud cell, cholinergic neural cell (various types), adrenergic neural cell (various types), peptidergic neural cell (various types), inner pillar cell of organ of Corti, outer pillar cell of organ of Corti, inner phalangeal cell of organ of Corti, outer phalangeal cell of organ of Corti, border cell of organ of Corti, hensen cell of organ of Cortim vestibular apparatus supporting cell, type I taste bud supporting cell, olfactory epithelium supporting cell, schwann cell, satellite cell (encapsulating peripheral nerve cell bodies), enteric glial cell, astrocyte (various types), neuron cells (large variety of types, still poorly classified), oligodendrocyte, spindle neuron, anterior lens epithelial cell, crystallin-containing lens fiber cell, melanocyte, retinal pigmented epithelial cell, oogonium/oocyte, spermatid, spermatocyte, spermatogonium cell (stem cell for spermatocyte), spermatozoon, ovarian follicle cell, sertoli cell (in testis), thymus epithelial cell, and interstitial kidney cells. For example, if the protein of interest that is expressed in the cell or cell line is an ion channel that is normally expressed in a particular type of neurons, the reference cell could be a neuron of that particular type. Particular types of neurons include, but are not limited to: sensory neurons, neurons of the central nervous system, unipolar neurons, pseudounipolar neurons, bipolar neurons, multipolar neurons, Golgi I neurons, pyramidal cells, purkinje cells, anterior horn cells, Golgi II neurons, granule cells, basket cells, betz cells, large motor neurons, medium spiny neurons, Renshaw cells, alpha motor neurons, afferent neurons, efferent neurons, motor neurons and interneurons.

Optionally, the reference cell is a cell of a cell type that normally expresses or functionally expresses the RNA or protein of interest without genetic engineering. Optionally, the reference cell is a cell that has the capacity to stably express the RNA or protein of interest. Optionally the reference cell is a cell that has the capacity to express the RNA or protein of interest without associated cyctotoxicity.

Also disclosed herein is a method for producing the cells and cell lines of the invention. Optionally, the method comprises the steps of:

a) providing a plurality of cells at least two of which express an RNA of interest or an mRNA encoding a protein of interest;
b) dispersing cells individually into individual culture vessels, thereby providing a plurality of separate cell cultures;
c) culturing the cells under a set of desired culture conditions using automated cell culture methods characterized in that the conditions are substantially identical for each of the separate cell cultures, during which culturing the number of cells in each separate cell culture is normalized, and wherein the separate cultures are passaged on the same schedule;
d) assaying the separate cell cultures for at least one desired characteristic of the RNA of interest or the protein of interest at least twice; and
e) identifying a separate cell culture that has the desired characteristic in both assays.

According to the method, the cells are cultured under a desired set of culture conditions. The conditions can be any desired conditions. Those of skill in the art will understand what parameters are comprised within a set of culture conditions. For example, culture conditions include but are not limited to: the media (Base media (DMEM, MEM, RPMI, serum-free, with serum, fully chemically defined, without animal-derived components), mono and divalent ion (sodium, potassium, calcium, magnesium) concentration, additional components added (amino acids, antibiotics, glutamine, glucose or other carbon source, HEPES, channel blockers, modulators of other targets, vitamins, trace elements, heavy metals, co-factors, growth factors, anti-apoptosis reagents), fresh or conditioned media, with HEPES, pH, depleted of certain nutrients or limiting (amino acid, carbon source)), level of confluency at which cells are allowed to attain before split/passage, feeder layers of cells, or gamma-irradiated cells, $CO_2$, a three gas system (oxygen, nitrogen, carbon dioxide), humidity, temperature, still or on a shaker, and the like, which will be well known to those of skill in the art.

The cell culture conditions may be chosen for convenience or for a particular desired use of the cells. Advantageously, disclosed herein are cells and cell lines that are optimally suited for a particular desired use. That is, in cases in which cells are cultured under conditions for a particular desired use, cells are selected that have desired characteristics under the condition for the desired use.

By way of illustration, if cells will be used in assays in plates where it is desired that the cells are adherent, cells that display adherence under the conditions of the assay may be selected. Similarly, if the cells will be used for protein production, cells may be cultured under conditions appropriate for protein production and selected for advantageous properties for this use.

Optionally, the method comprises the additional step of measuring the growth rates of the separate cell cultures. Growth rates may be determined using any of a variety of techniques means that will be well known to the skilled worker. Such techniques include but are not limited to measuring ATP, cell confluency, light scattering, optical density (*e.g.*, OD 260 for DNA). Preferably growth rates are determined using means that minimize the amount of time that the cultures spend outside the selected culture conditions.

Optionally, cell confluency is measured and growth rates are calculated from the confluency values. Optionally, cells are dispersed and clumps removed prior to measuring cell confluency for improved accuracy. Means for monodispersing cells are well-known and can be achieved, for example, by addition of a dispersing reagent to a culture to be measured. Dispersing agents are well-known and readily available, and include but are not limited to enzymatic dispersing agents, such as trypsin or other protease, and non-enzymatic cell dissociation reagents, including but not limited to EDTA-based dispersing agents. Growth rates can be calculated from confluency date using commercially available software for that purpose such as HAMILTON VECTOR. Automated confluency measurement, such as using an automated microscopic plate reader is particularly useful. Plate readers that measure confluency are commercially available and include but are not limited to the CLONE SELECT IMAGER (Genetix). Typically, at least 2 measurements of cell confluency are made before calculating a growth rate. The number of confluency values used to determine growth rate can be any number that is convenient or suitable for the culture. For example, confluency can be measured multiple times over *e.g.,* a week, 2 weeks, 3 weeks or any length of time and at any frequency desired.

When the growth rates are known, according to the method, the plurality of separate cell cultures are divided into groups by similarity of growth rates. By grouping cultures into growth rate bins, one can manipulate the cultures in the group together, thereby providing another level of standardization that reduces variation between cultures. For example, the cultures in a bin can be passaged at the same time, treated with a desired reagent at the same time, *etc.* Further, functional assay results are typically dependent on cell density in an assay well. In some embodiments, a true comparison of individual clones is only accomplished by having them plated and assayed at the same density. Grouping into specific growth rate cohorts enables the plating of clones at a specific density that allows them to be functionally characterized in a high throughput format

The range of growth rates in each group can be any convenient range. It is particularly advantageous to select a range of growth rates that permits the cells to be passaged at the same time and avoid frequent renormalization of cell numbers. Growth rate groups can include a very narrow range for a tight grouping, for example, average doubling times within an hour of each other. But according to the method, the range can be up to 2 hours, up to 3 hours, up to 4 hours, up to 5 hours or up to 10 hours of each other or even broader ranges. The need for renormalization arises when the growth rates in a bin are not the same so that the number of cells in some cultures increases faster than others. To maintain substantially identical conditions for all cultures in a bin, it is necessary to periodically remove cells to renormalize the numbers across the bin. The more disparate the growth rates, the more frequently renormalization is needed.

In step d) the cells and cell lines may be tested for and selected for any physiological property including but not limited to: a change in a cellular process encoded by the genome ;a change in a cellular process regulated by the genome; a change in a pattern of chromosomal activity; a change in a pattern of chromosomal silencing; a change in a pattern of gene silencing; a change in a pattern or in the efficiency of gene activation; a change in a pattern or in the efficiency of gene expression; a change in a pattern or in the efficiency of RNA expression; a change in a pattern or in the efficiency of RNAi expression; a change in a pattern or in the efficiency of RNA processing; a change in a pattern or in the efficiency of RNA transport; a change in a pattern or in the efficiency of protein translation; a change in a pattern or in the efficiency of protein folding; a change in a pattern or in the efficiency of protein assembly; a change in a pattern or in the efficiency of protein modification; a change in a pattern or in the efficiency of protein transport; a change in a pattern or in the efficiency of transporting a membrane protein to a cell surface change in growth rate; a change in cell size; a change in cell shape; a change in cell morphology; a change in % RNA content; a change in % protein content; a change in % water content; a change in % lipid content; a change in ribosome content; a change in mitochondrial content; a change in ER mass; a change in plasma membrane surface area; a change in cell volume; a change in lipid composition of plasma membrane; a change in lipid composition of nuclear envelope; a change in protein composition of plasma membrane; a change in protein; composition of nuclear envelope; a change in number of secretory vesicles; a change in number of lysosomes; a change in number of vacuoles; a change in the capacity or potential of a cell for: protein production, protein secretion, protein folding, protein assembly, protein modification, enzymatic modification of protein, protein glycosylation, protein phosphorylation, protein dephosphorylation, metabolite biosynthesis, lipid biosynthesis, DNA synthesis, RNA synthesis, protein synthesis, nutrient absorption, cell growth, mitosis, meiosis, cell division, to dedifferentiate, to transform into a stem cell, to transform into a pluripotent cell, to transform into a omnipotent cell, to transform into a stem cell type of any organ (*i.e.* liver, lung, skin, muscle, pancreas, brain, testis, ovary, blood, immune system, nervous system, bone, cardiovascular system, central nervous system, gastro-intestinal tract, stomach, thyroid, tongue, gall bladder, kidney, nose, eye, nail, hair, taste bud), to transform into a differentiated any cell type (*i.e.* muscle, heart muscle, neuron, skin, pancreatic, blood, immune, red blood cell, white blood cell, killer T-cell, enteroendocrine cell, taste, secretory cell, kidney, epithelial cell, endothelial cell, also including any of the animal or human cell types already listed that can be used for introduction of nucleic acid sequences), to uptake DNA, to uptake small molecules, to uptake fluorogenic probes, to uptake RNA, to adhere to solid surface, to adapt to serum-free conditions, to adapt to serum-free suspension conditions, to adapt to scaled-up cell culture, for use for large scale cell culture, for use in drug discovery, for use in high throughput screening, for use in a functional cell based assay, for use in membrane potential assays, for use in calcium flux assays, for use in G-protein reporter assays, for use in reporter cell based assays, for use in ELISA studies, for use in in vitro assays, for use in vivo applications, for use in secondary testing, for use in compound testing, for use in a binding assay, for use in panning assay, for use in an antibody panning assay, for use in imaging assays, for use in microscopic imaging assays, for use in multiwell plates, for adaptation to automated cell culture, for adaptation to miniaturized automated cell culture, for adaptation to large-scale automated cell culture, for adaptation to cell culture in multiwell plates (6, 12, 24, 48, 96, 384, 1536 or higher density), for use in cell chips, for use on slides, for use on glass slides, for microarray on slides or glass slides, for immunofluorescence studies, for use in protein purification, for use in biologics production, for use in the production of industrial enzymes, for use in the production of reagents for research, for use in vaccine development, for use in cell therapy, for use in implantation into animals or humans, for use in isolation of factors secreted by the cell, for preparation of cDNA libraries, for purification of RNA, for purification of DNA, for infection by pathogens, viruses or other agent, for resistance to infection by pathogens, viruses or other agents, for resistance to drugs, for suitability to be maintained under automated miniaturized cell culture conditions, for use in the production of protein for characterization, including: protein crystallography, vaccine development, stimulation of the immune system, antibody production or generation or testing of antibodies. Those of skill in the art will readily recognize suitable tests for any of the above-listed properties.

Tests that may be used to characterize cells and cell lines disclosed herein and/or matched panels disclosed herein include but are not limited to: Amino acid analysis, DNA sequencing, Protein sequencing, NMR, A test for protein transport, A test for nucelocytoplasmic transport, A test for subcellular localization of proteins, A test for subcellular localization of nucleic acids, Microscopic analysis, Submicroscopic analysis, Fluorescence microscopy, Electron microscopy, Confocal microscopy, Laser ablation technology, Cell counting and Dialysis. The skilled worker would understand how to use any of the above-listed tests.

When collections or panels of cells or cell lines are produced, *e.g.*, for drug screening, the cells or cell lines in the collection or panel may be matched such that they are the same (including substantially the same) with regard to one or more selective physiological properties. The "same physiological property" in this context means that the selected physiological property is similar enough amongst the members in the collection or panel such that the cell collection or panel can produce reliable results in drug screening assays; for example, variations in readouts in a drug screening assay will be due to, *e.g.*, the different biological activities of test compounds on cells expressing different forms of a protein, rather than due to inherent variations in the cells. For example, the cells or cell lines may be matched to have the same growth rate, *i.e.,* growth rates with no more than one, two, three, four, or five hour difference amongst the members of the cell collection or panel. Optionally, this may be achieved by, for example, binning cells by their growth rate into five, six, seven, eight, nine, or ten groups, and creating a panel using cells from the same or different binned group. Optionally, cells can be binned by growth rate into 11, 12, 13, 14, 15, 16, 17, 18, 19 or 20 groups. Optionally, cells can be binned by growth rate into at least 20, 25,30, 35, 40, 45, 50, 60 , 70, 80, 90, 100 or more than 100 groups. Optionally, a panel of cell lines can comprise cell lines binned into the same group based on their growth rate. Optionally, a panel of cell lines can comprise cell lines binned into different groups based on their growth rate. Methods of determining cell growth rate are well known in the art. The cells or cell lines in a panel also can be matched to have the same Z' factor (*e.g.*, Z' factors that do not differ by more than 0.1), protein expression level *(e.g.,* CFTR expression levels that do not differ by more than 5%, 10%, 15%, 20%, 25%, or 30%), RNA expression level, adherence to tissue culture surfaces, and the like. Matched cells and cell lines can be grown under identical conditions, achieved by, *e.g.*, automated parallel processing, to maintain the selected physiological property. Optionally, cells or cell lines of the invention can be binned into groups based on physiological properties of the cells or cell lines including but not limited to growth rates. Optionally, a matched panel of cells or cell lines can comprise cells or cell lines of one or more bins grouped by at least one physiological property of a cell including but not limited to growth rate.

Optionally, the panel is matched for growth rate under the same set of conditions. Such a panel, also referred to herein as a matched panel, are highly desirable for use in a wide range of cell-based studies in which it is desirable to compare the effect of an experimental variable across two or more cell lines. Cell lines that are matched for growth rate maintain roughly the same number of cells per well over time thereby reducing variation in growth conditions, such as nutrient content between cell lines in the panel

Matched panels disclosed herein may have growth rates within any desired range, depending on a number of factors including the characteristics of the cells, the intended use of the panel, the size of the panel, the culture conditions, and the like. Such factors will be readily appreciated by the skilled worker.

Growth rates may be determined by any suitable and convenient means, the only requirement being that the growth rates for all of the cell lines for a matched panel are determined by the same means. Numerous means for determining growth rate are known as described herein.

A matched panel disclosed herein can comprise any number of clonal cell lines. The maximum number of clonal cell lines in the panel will differ for each use and user and can be as many as can be maintained. Optionally, the panel may comprise 2, 3, 4, 5, 6, 7, 8, 9, 10 or more clonal cell lines, for example, at least 12, at least 15, at least 20, at least 24, at least 25, at least 30, at least 35, at least 40, at least 45, at least 48, at least 50, at least 75, at least 96, at least 100, at least 200, at least 300, at least 384, at least 400 or more clonal cell lines. Optionally, the matched panel may comprise at least 100, 150, 200, 250, 300, 350, 400, 350, 500, 550, 600, 650, 700, 750, 800, 850, 900 or 1,000 clonal cell lines. Further optionally, the matched panel may comprise at least 1,100, 1, 250, 1,500, 2,000, 3,000, 4,000, 5,000, 6,000, 7,000, 8,000, 9,000 or 10,000 clonal cell lines. Further optionally, the matched panel may comprise at least 11,000, 15,000, 20,000, 25,000, 30,000, 35,000, 40,000, 45,000, 50,000, 60,000, 70,000, 80,000, 90,000 or 100,000 clonal cell lines. Further optionally, the matched panel may comprise at least 100,000, 150,000, 200,000, 300,000, 400,000, 500,000, 600,000, 700,000, 800,000, 900,000 or 1,000,000 clonal cell lines or more than 1,000,000 clonal cell lines. Still further optionally, the matched panel may comprise at least 1,000 clonal cell lines.

The panel disclosed herein comprises a plurality of clonal cell lines, that is, a plurality of cell lines generated from a different single parent cell. Optionally the plurality of cell lines in a panel of cell lines are of the same type. Optionally the plurality of cell lines in a panel of cell lines are of at least two different types. Any desired cell type may be used in the production of a matched panel. The panel can comprise cell lines of all the same cell type or cell lines of different cell types.

The clonal cell lines in the panel stably express one or more proteins of interest. The stable expression can be for any length of time that is suitable for the desired use of the panel but at a minimum, is sufficiently long to permit selection and use in a matched panel.

The clonal cell lines in the matched panel may all express the same one or more proteins of interest or some clonal cell lines in the panel may express different proteins of interest.

Optionally, the matched panel comprises one or more clonal cell lines that express different proteins of interest.

That is, a first clonal cell line in the panel may express a first protein of interest, a second clonal cell line in the panel may express a second protein of interest, a third cell line may express a third protein of interest, *etc.* for as many different proteins of interest as are desired. The different proteins of interest may be different isoforms, allelic variants, splice variants, or mutated (including but not limited to sequence mutated or truncated), different subunit stoichiometries, different subunit assemblies, differentially folded forms, differentially active forms, forms with different functionalities, forms with different binding properties, forms associated with different accessory factors, forms expressed in different cell backgrounds, forms expressed in different cellular genetic backgrounds, forms expressed in cells with different endogenous expression profiles, differentially localized forms, chimeric or chemically including modified forms, enzymatically modified forms, post-translationally modified forms, glycosylated forms, proteolyzed forms, or combinations thereof of a protein of interest. Optionally the different proteins can be members of a functionally defined group of proteins, such as a panel of bitter taste receptors or a panel of kinases. Optionally the different proteins may be part of the same or interrelated signaling pathways. In still other panels involving heteromultimeric proteins (including heterodimers), the panel may comprise two or more different combinations of subunits up to all possible combinations of subunits. The combinations may comprise subunit sequence variants, subunit isoform combinations, interspecies combinations of subunits and combinations of subunit types.

By way of example, Gamma-aminobutyric acid $(GABA)_A$ receptors typically comprise two alpha subunits, two beta subunits and a gamma subunit. There are 6 alpha isoforms, 5 beta isoforms, 4 gamma isoforms, and a delta, a pi, a theta and an epsilon subunit. The disclosure herein contemplates panels comprising two or more combinations of any of these subunits including panels comprising every possible combination of alpha, beta, gamma, delta, pi, epsilon and theta subunit. Further, the GABA receptor family also includes $GABA_B$ and $GABA_C$ receptors. The disclosure herein also contemplates panels that comprise any combination of $GABA_A$, $GABA_B$ and $GABA_C$ subunits. Optionally, such panels comprise human GABA subunits. Further optionally mammalian GABA receptor panels may comprise non-human primate (eg, cynomolgus) GABA receptors, mouse, rat or human GABA receptor panels or mixtures thereof.

In a further example, the disclosure herein contemplates one or more epithelial sodium channel (ENaC) panels, including any mammalian ENaC panel such as a non-human primate (eg, cynomolgus) ENaC, mouse, rat or human ENaC panels or mixtures thereof. Like GABA receptors, intact ENaC comprise multiple subunits: alpha or delta, beta and gamma. The disclosure herein contemplates panels with at least two different combinations of ENaC subunits and also contemplates all possible combinations of ENaC subunits, including combinations of subunits from different species, combinations of isoforms, allelic variants, SNPs, chimeric subunits, forms comprising modified and/or non-natural amino acids and chemically modified such as enzymatically modified subunits. The disclosure herein also contemplates panels comprising any ENaC form set forth in International Application PCT/US09/31936, the contents of which are incorporated by reference in its entirety.

Disclosed herein is a matched panel of 25 bitter taste receptors comprising cell lines that express native (no tag) functional bitter receptors listed in Table 10. Optionally, the panel is matched for growth rate. Optionally the panel is matched for growth rate and an additional physiological property of interest. Optionally the cell lines in the panel were generated in parallel and/or screened in parallel.

Further exemplary but non-limited examples of panels and their uses are the following: a panel of odorant receptors (insect, canine, human, bed bug), for example to profile fragrances or to discover modulators; panels of cells expressing a gene fused to a test peptide, *i.e.,* to find a peptide that works to internalize a cargo such as a protein, including an antibody, monoclonal antibody or a non-protein drug into cells (the cargo could be a reporter such as GFP or AP). Related to this case, supernatants from cells of this panel could be added to other cells for assessment of internalization. In such a case, the panel may comprise different cell types to assess cell-type specific delivery. A panel of cell lines expressing different antibody or monoclonal antibody heavy chain/light chain combinations to identify active antibodies or monoclonal antibodies. An antibody panel also could provide a series of derivatized versions of an antibody or monoclonal antibody to identify one with improved characteristics, such as stability in serum, binding affinity and the like. Yet another panel could be used to express a target protein in the presence of various signaling molecules, such as different G-proteins. Still another type of panel could be used to test variants of a target proteins for improved activity/stability. Panels could comprise single nucleotide polymorphs (SNPs) or other mutated forms of a target protein to select modulators that act on a subset, many or all forms. Other panels could be used to define the patterns of activity of test compounds on a family of proteins or isoforms of a protein (such as $GABA_A$ or other CNS ion channels). Differentially acting compounds could then be used in further study to determine the function/role/localization of corresponding subunit combinations *in vivo.* The test compounds could be known modulators that failed in the clinic or ones that have adverse off-target effects, to determine subunit combinations that may correlate with such effects. Still other panels could be used in HTS for parallel screening for reliable assessment of compounds' activity at multiple target subtypes to assist in finding compounds active at desired targets and that have minimal off target effects.

The panels can include cells that have been engineered to express any desired group of proteins and all such panels are contemplated by the disclosure herein.

The panels can include cells that have been engineered to express any desired group of RNAs and all such panels are contemplated by the disclosure herein.

Also disclosed herein is a panel of cells or cell lines, wherein the panel comprises a plurality of cells or cell lines each expressing a different odorant receptor. These odorant receptor panels can be used to generate odorant activity profiles of compounds or compositions of interest. An odorant activity profile of a compound or composition refers to the effect of the compound or composition on the activity of a plurality of odorant receptors. To generate an odorant activity profile of a compound or composition of interest, the compound or composition is contacted with a plurality of cells or cell lines each expressing a different odorant receptor.

Optionally, the odorant receptor cell panels are used to identify compounds that modify, enhance, or reduce the odorant activity profile of a compound or composition. Compounds that are identified as modifying, enhancing, or reducing the odorant activity profile of another compound or composition are predicted to modify, enhance, or reduce the olfactory effect of the other compound or composition.

Optionally, the odorant receptor cell panels are used to identify compounds that have an odorant activity profile that is similar to a compound or composition of interest. Compounds that are identified as having an odorant activity profile that is similar to a compound or composition of interest are predicted to have an olfactory effect that is similar to the other compound or composition, *i.e.,* smell similar or identical. Odorant activity profiles can be compared as described below.

Useful odorant receptors include, but are not limited to, the olfactory receptors set forth in Tables 10-12. Optionally, the odorant receptors are of the class I human olfactory receptors or the class II human olfactory receptors or a combination thereof. An odorant receptor panel of the present invention can have at least 2, 5, 10, 25, 50, 75, 100, 250, 500, 750, 1000, 1500, 2000, or at least 2500 different cells or cell lines each expressing a different odorant receptor. The different odorant receptors may be of different species or of the same species. Optionally, an odorant receptor for use with the cells, panels and methods disclosed herein may be encoded by a pseudogene.

The activity of a compound or composition of interest on an odorant receptor can be measured by any technique known to the skilled artisan. Assays to measure the effect of a compound or composition of interest on the activity of an odorant receptor include, but are not limited to: cell based assays, fluorescent cell based assays, imaging assays, calcium flux assays, membrane potential assays, high throughput screening assays, fluorogenic assays and combination of the above. Any assays to be used with the methods of the invention can be conducted in high throughput format.

Any of the cells or cell lines disclosed herein can be used to generate an odorant receptor panel disclosed herein. Optionally, a host cell for generation of an odorant receptor panel may be a cell that has been tested and verified to endogenously express signaling or other protein factors that are desirable for functional expression of odorant receptors. RNA or protein characterization of cells by tests including RT-PCR and microarray methods including genechip analysis may be used to identify such cells.

Optionally, a cell panel of the invention comprises a plurality of cells or cell lines, wherein each cell or cell line has been engineered to express one or more insect odorant receptor. The resulting cell panel can be used in cell based assays to characterize odorants and for high throughput screening ("HTS") to identify odorant receptor modulators.

Optionally, a panel of cells or cell lines may comprise all or a subset of the odorant receptors from one species (*e.g.*, a species of mosquito). A panel of cells or cell lines may comprise at least one odorant receptor from at least two different insect species. Insect odorant receptors from any insect species may be used including insects that transmit human or animal disease, insects that afflict crop or cause agricultural damage or damage to plants, including but not limited to mosquitoes, cockroaches, beetles, bed bugs, moths, butterflies, flies, ants, crickets, bees, wasps, fruit flies, ticks, lice, genital lice, scorpions, millipedes, centipedes, grasshoppers, praying mantis, and spiders. A common method to identify a protein as an olfactory receptor is based on sequence comparisons to known olfactory receptors. Optionally, cell based assays may also be used to test for responsiveness of a transmembrane protein to known volatile or odorant compounds in order to determine its role as an odorant receptor.

Substances including compounds and extracts that attract or repel insects can be tested against a panel provided herein to identify responsive receptors or receptors whose activity is modulated by the test substance. The substances may be collected from, *e.g.,* plants, flowers, foods *(e.g.,* cheese), animals, smoke, waste products, secretions, sweat (including human sweat, *e.g*., male sweat or female sweat), industrial products, natural and synthetic chemicals and biological preparations. Substances may be tested against a subset or all odorant receptor cell lines to identify the profile of activity of a compound at against all tested receptors. The patterns of activity that result from testing of substances against the odorant receptor cell lines may be used to characterize, "fingerprint" or serve as a diagnostic.

HTS may be used to screen a cell line or panel comprising an insect odorant receptor identified to respond to a substance in order to identify other substances with similar, increased or decreased activity. HTS may be used to screen a cell line or panel comprising an insect odorant receptor that responds to a substance to identify compounds that modulate, block or potentiate the activity of the receptor in the presence of the substance. HTS may be used to screen a cell line or panel comprising an odorant receptor that does not respond to a substance to identify compounds that result in a response or activity of the receptor in the presence of the substance.

Optionally, the methods disclosed herein are used to identify a compound or a mixture of compounds that have an activity that is similar to a known substance (*e.g.,* a known compound) on an insect odorant receptor. Further optionally, the methods disclosed herein are used to identify a compound that mimics the odorant receptor activity of DEET and/or other insect repellents and attractants. Optionally, the methods disclosed herein are used to identify compounds that can be used as insect repellents. Optionally, the compound that is identified as insect repellents is volatile and non-toxic to the environment, human, animals and/or crops. Optionally, the methods disclosed herein are used to identify compounds that can be used as insects attractant. Optionally, the compound that is identified as insect attractant is a volatile compound and non-toxic to the environment, human, animals and/or crops. Such insect attractants can be used in insect traps. Optionally, the insect attractant or repellent can be specific for a particular insect species.

Optionally, the methods disclosed herein are used to identify a compound or a mixture of compounds that block the activity of a particular substance on one or more odorant receptors. Further optionally, the methods are used to identify compounds that block receptor responses to sweat, human or animal secretions or their components or carbon dioxide.

Optionally, the methods of the disclosed herein are used to identify one or a mixture of compounds that potentiate the activity of a particular substance *(e.g.,* a particular compound) on one or more odorant receptors, for instance, compounds that attract or repel insects.

Optionally, the methods disclosed herein are used to identify one or a mixture of compounds that alter the activity of a particular substance (*e.g.*, a particular compound) on one or more odorant receptors. Further optionally, the methods disclosed herein can be used to identify a combination of at least 2 compounds, wherein the at least 2 compounds activate an odorant receptor only if combined, and wherein each of the at least 2 compounds does not activate the odorant receptor individually. The receptor can be a receptor that detects an insect repellent or an insect attractant.

Optionally, the methods disclosed herein are used to generate odorant activity profiles of human sweat samples obtained from males and/or females and fractions of these or compounds isolated from these to identify responsive receptors and correlate this data with other data including for instance results for testing of the same substances against one or more insect species to identify activities or compounds that correlate with insect repulsion or attraction.

Optionally, the methods disclosed herein are used to test and compare the odorant activity profiles of at least two samples. Optionally, samples can be volatiles obtained from different plants; different species of plants or animals, or from different flowers. The odorant activity profiles of the different samples are then compared to identify responsive receptors (e.g., those receptors the activities of which are affected by the different samples). The responsive receptors are an indicator for the chemical composition of the samples. The similarity of the odorant activity profiles of the samples that are being compared is a measure for the chemical similarity of the different samples.

Optionally, the methods disclosed herein are used to identify a number of chemically diverse compounds having similar activity, for combined or sequential use or introduction to address potential insect adaptation or evolution that may render at least one of the compounds ineffective.

A panel of cell lines each comprising one or more human odorant receptors can be produced for use in cell based assays to characterize odorants and for HTS to identify odorant receptor modulators.

Substances including compounds and extracts that have a scent, odor, aroma or fragrance can be tested against a panel of human odorant receptors to identify responsive receptors or receptors whose activity is modulated by the test substance. The compounds or mixtures can be collected from plants, flowers, foods, animals, cigarettes, tobacco, truffles, musk, vanilla, mint, waste products, secretions, sweat (including human sweat, *e.g*., male sweat or female sweat), industrial products, natural and synthetic chemicals and biological preparations including disease tissues and tumors. Compounds may be tested against a subset or all human odorant receptor cell lines to identify the profile of activity of a compound against all tested receptors. The patterns of activity that result from testing of substances against the human odorant receptor cell lines may be used to characterize, "fingerprint" or serve as a diagnostic.

HTS may be used to screen a cell line or panel comprising a human odorant receptor identified to respond to a substance to identify other substances with similar, increased or decreased activity. HTS may be used to screen a cell line comprising a human odorant receptor that responds to a substance to identify compounds that modulate, block or potentiate the activity of the receptor in the presence of the substance. HTS may be used to screen a cell line comprising a human odorant receptor that does not respond to a substance to identify compounds that result in a response or activity of the receptor in the presence of the substance.

Illustrative uses of the odorant receptor panels include:

Identify one or a mixture of compounds that have similar activity on one or more odorant receptors as a known substance, for instance a synthetic compound that can mimic the activity of musk.

Identify one or a mixture of compounds that block the activity of a known substance on one or more odorant receptors, for instance compounds that block substances that are malodorous (human or animal waste).

Identify one or a mixture of compounds that potentiate the activity of a known substance on one or more odorant receptors, for instance compounds that mimic the scent of a rose or the aroma of a steak.

Identify one or a mixture of compounds that alter the activity of a known substance on one or more odorant receptors, for instance compounds that result in the activation of receptors that detect malodors in the presence of substances such as tobacco smoke that may not normally produce this effect.

Identify one or a mixture of compounds that alter the activity of a known substance on one or more odorant receptors, for instance compounds that result in the activation of receptors that detect desirable scents in the presence of substances such as malodorous substances that may normally activate other receptors not receptors corresponding to desirable scents.

Profile human sweat samples obtained from males and females and fractions of these or compounds isolated from these to identify responsive receptors and correlate this data with other data including for instance human psychophysics studies to identify activities or compounds that correlate with perceived malodor or attraction.

Test a set of samples that can be compared (for instance fragrances obtained from roses of different species, or fragrances obtained from different flowers, or fractions of one substance) to identify common or specific receptors that are responsive to these fragrances.

The cells, panels and methods described herein may be applied to odorant receptors from other species including but not limited to: mammalian species selected from the group consisting of: human, non-human primate, bovine, porcine, feline, rat, marsupial, murine, canine, ovine, caprine, rabbit, guinea pig and hamster; or insect species selected from the group consisting of: Mosquitoes including Anopheles (Anopheles gamiae and all mosquito sub-species, Aedes (including Aedes aegypti and all sub-species), Culex and Aedes mosquitoes, Simulium, black flies, Phlebotomus, sand flies, Tabanus, horse flies, Chryops, deer flies, Glossina, tsetse flies, Order Siphonaptera, Fleas, Xenopsylla, Nosopsyllus, Order Hemiptera, Triatoma and Rhodnius (kissing bugs), Order Anoplura, Sucking Lice, Pediculus humanis, Lice, Black Flies, Chiggers, Eye Gnats, Stable fly, Deer and Horse Flies, Aphid, Migratory locust, Maize planthopper, Thrips, Flies, Lepidoptera, Nematodes, Homoptera, Coleoptera, Mites, Termite, cockroaches including German cockroach, Ants, Aphididae, Acrididae, Fulgoromorpha, Fulgoroidea, Thripidae, Phlaeothripidae, Tortricidae, Noctuidae, Crambidae, Noctuidae, Plutellidae, Pyralidae, Heteroderidae, Aleyrodidae, Chrysomelidae, Tenebrionidae, Curculionoidea, the grain weevil, Sitophilus granarius, rice weevil, Sitophilus oryzae, alfalfa weevil, Hypera postica. Seed weevil on peas, Bruchus pisorum, on bean seeds, Acanthoscelides obtectus, Tetranychidae, Rhinotermitidae, Kolotermitidae, Blattellidae, Formicidae, beetles and bed bugs, and moths.

The odorant activity profile of a compound of interest and a landmark odorant activity profile may be compared through computation of a correlation between the odorant activity profiles, such as but not limited to computing a measure of similarity between the odorant activity profiles. The landmark odorant activity profile may be one of a group of odorant activity profiles in a database. The landmark odorant activity profile may be a historical profile. The odorant activity profile of the compound of interest can comprise measured amounts representing an effect of the compound on the activity of two or more odorant receptors in a panel. Each landmark odorant activity profile comprises measured amounts representing the effect of a respective compound on the activity of two or more odorant receptors in the panel.

Optionally, each respective compound corresponding to a landmark odorant activity profile can be associated with a known odor. That is, measured amounts of the effect of a respective compound on the activity of two or more odorant receptors in the panel can be assembled to produce a landmark transcript profile that can be associated with a known odor. The database of landmark odorant activity profiles can be stored on a computer readable storage medium. Optionally, the database contains at least 10 landmark odorant activity profiles, at least 50 landmark odorant activity profiles, at least 100 landmark odorant activity profiles, at least 500 landmark odorant activity profiles, at least 1,000 landmark odorant activity profiles, at least 10,000 landmark odorant activity profiles, or at least 50,000 landmark odorant activity profiles, each landmark odorant activity profile containing measured amounts of at least 2, at least 10, at least 100, at least 200, at least 500, at least 1,000, at least 2000, at least 2500, at least 7500, at least 10,000, at least 20,000, at least 25,000, or at least 35,000 components. In the foregoing case, the odorant activity profile of the compound of interest can be compared to a plurality of landmark odorant activity profiles to determine the one or more landmark odorant activity profiles that correlate with (*e.g.*, are most similar to) the odorant activity profile of the compound of interest, and the compound of interest can be characterized as having the known odor(s) associated with the respective compound corresponding to these one or more landmark odorant activity profiles.

Further optionally, a compound of interest can be associated with a known odor. In the foregoing case, the odorant activity profile of the compound of interest can be compared to a plurality of landmark odorant activity profiles to determine the one or more landmark odorant activity profiles that correlate with (*e.g.*, are most similar to) the odorant activity profile of the compound of interest, and the respective compound corresponding to these one or more landmark odorant activity profiles can be characterized as having the known odor associated with the compound of interest.

Optionally, a correlation can be computed between the odorant activity profile of a compound of interest and each landmark odorant activity profile of a plurality of landmark odorant activity profiles stored in a database. The correlation can be computed by comparing a measured amount in the odorant activity profile of the compound of interest representing an effect of the compound of interest on the activity of a selection of two or more odorant receptors in a panel to the corresponding measured amount in the landmark odorant activity profile representing an effect of a different compound on the activity of the same selection of two or more odorant receptors in the panel. The odorant activity profile of the compound of interest can be deemed to correlate with a landmark odorant activity profile if the measured amounts in the landmark odorant activity profile are within about 2%, about 5%, about 8%, about 10%, about 12%, about 15%, about 20%, about 25%, about 30%, or about 35% of the measured amounts in the odorant activity profile of the compound of interest.

The odorant activity profile of a compound of interest can be deemed to be most similar to a landmark odorant activity profile if a measure of similarity between the odorant activity profile of the compound of interest and the landmark odorant activity profile is above a predetermined threshold. Optionally, the predetermined threshold can be determined as the value of the measure of similarity which indicates that the measured amounts in a landmark odorant activity profile are within about 2%, about 5%, about 8%, about 10%, about 12%, about 15%, about 20%, about 25%, about 30%, or about 35% of the measured amounts in the odorant activity profile of the compound of interest.

Optionally, the odorant activity profile of a compound of interest can be expressed as a vector p,

$$\boldsymbol{p} = [\ \boldsymbol{p}_1, \ldots \boldsymbol{p}_i, \ldots \boldsymbol{p}_n]$$

where $p_i$ is the measured amount of the *i*'th component, for example, the effect of the compound of interest on the *i*'th biological activity of a given odorant receptor in the panel. In specific embodiments, n is more than 2, more than 10, more than 100, more than 200, more than 500, more than 1000, more than 2000, more than 2500, more than 7500, more than 10,000, more than 20,000, more than 25,000, or more than 35,000. Each landmark odorant activity profile also can be expressed as a vector *p*. In computing a correlation, the measured amount of the *i*'th component in the vector representing the odorant activity profile for the compound of interest can be compared to the corresponding measured amount of the *i*'th component of the vector representing a landmark odorant activity profile, for each component $i = 1 \ldots n$.

A correlation may be computed using any statistical method in the art for determining the probability that two datasets are related may be used in accordance with the methods of the present invention in order to identify whether there is a correlation between the odorant activity profile of a compound of interest and a landmark odorant activity profile. For example, the correlation between the odorant activity profile ($pi_1$) of the compound of interest and each landmark odorant activity profile ($pi_2$) can be computed using a similarity metric $sim(pi_1, pi_2)$. One way to compute the similarity metric $sim(pi_1, pi_2)$ is to compute the negative square of the Euclidean distance. Further optionally, metrics other than Euclidean distance can be used to compute $sim(pi_1, pi_2)$, such as a Manhattan distance, a Chebychev distance, an angle between vectors, a correlation distance, a standardized Euclidean distance, a Mahalanobis distance, a squared Pearson correlation coefficient, or a Minkowski distance. Optionally a Pearson correlation coefficient, a squared Euclidean distance, a Euclidean sum of squares, or squared Pearson correlation coefficients is used to determine similarity. Such metrics can be computed, for example, using SAS (Statistics Analysis Systems Institute, Cary, North Carolina) or S-Plus (Statistical Sciences, Inc., Seattle, Washington). Use of such metrics are described in Draghici, 2003, Data Analysis Tools for DNA Microarrays, Chapman & Hall, CRC Press London, chapter 11.

The correlation can also be computed based on ranks, where $x_i$ and $y_i$ are the ranks of the values of the measured amounts in ascending or descending numerical order. See for example, Conover, Practical Nonparametric Statistics, 2nd ed., Wiley, (1971). Shannon mutual information also can be used as a measure of similarity. See for example, Pierce, An Introduction To Information Theory: Symbols, Signals, and Noise, Dover, (1980).

Various classifiers known in the art can be trained according to the methods described in this application, and used to classify a compound of interest as having an odor. Algorithms can be used to produce classifiers capable of predicting an odor of a compound of interest using an odorant activity profile of the compound of interest.

Optionally, a classifier can be trained to classify a compound as to an odor using the measured amounts in the landmark odorant activity profile of a previously characterized compound and the known odor associated with that previously characterized compound. Each respective compound corresponding to a landmark odorant activity profile can be associated with a known odor. The classifier may be an algorithm used for classification by applying a non-supervised or supervised learning algorithm to evaluate the measured amounts in the landmark odorant activity profile of a previously characterized compound and the known odor associated with that previously characterized compound. The odorant activity profile of the compound of interest can be processed using the classifier to classify the compound of interest as to an odor. That is, the classifier can be used to classify the compound of interest as having one or more of the known odors associated with the plurality of landmark odorant activity profiles used to train the class

Optionally, a compound of interest can be associated with a known odor. In the foregoing case, a classifier can be trained to identify one or more landmark odorant activity profiles that can be associated with the known odor of the compound of interest based on the odorant activity profile of the compound of interest. The classifier may be an algorithm used for classification by applying a non-supervised or supervised learning algorithm to evaluate the measured amounts in the landmark odorant activity profile of a respective compound, and to identify the one or more landmark odorant activity profiles that can be associated with the known odor of the compound of interest based on the odorant activity profile of the compound of interest.

Any standard non-supervised or supervised learning technique known in the art can be used to generate a classifier. Below are non-limiting examples of non-supervised and supervised algorithms known in the art. Given the disclosure in this application, one of skill in the art will appreciate that other pattern classification or regression techniques and algorithms may be used for the classifier and the present invention encompasses all such techniques.

*Neural networks.* Optionally, a classifier is learned using a neural network. A neural network is a two-stage regression or classification decision rule. A neural network has a layered structure that includes a layer of input units (and the bias) connected by a layer of weights to a layer of output units. For regression, the layer of output units typically includes just one output unit. However, neural networks can handle multiple quantitative responses in a seamless fashion.

In multilayer neural networks, there are input units (input layer), hidden units (hidden layer), and output units (output layer). There is, furthermore, a single bias unit that is connected to each unit other than the input units. Neural networks are described in Duda et al., 2001, Pattern Classification, Second Edition, John Wiley & Sons, Inc., New York; and Hastie et al., 2001, The Elements of Statistical Learning, Springer-Verlag, New York. Neural networks are also described in Draghici, 2003, Data Analysis Tools for DNA Microarrays, Chapman & Hall/CRC; and Mount, 2001, Bioinformatics: sequence and genome analysis, Cold Spring Harbor Laboratory Press, Cold Spring Harbor, New York. What are discussed below are some exemplary forms of neural networks.

The basic approach to the use of neural networks is to start with an untrained network, present a training pattern to the input layer, and to pass signals through the net and determine the output at the output layer. These outputs are then compared to the target values; any difference corresponds to an error. For classification, this error can be either squared error or cross-entropy (deviation). See, for example, Hastie *et al.,* 2001, *The Elements of Statistical Learning,* Springer-Verlag, New York.

Three commonly used training protocols are stochastic, batch, and on-line. In stochastic training, patterns are chosen randomly from the training set and network weights are updated for each pattern presentation. Multilayer nonlinear networks trained by gradient descent methods such as stochastic back-propagation perform a maximum-likelihood estimation of weight values in the classifier defined by the network topology. In batch training, all patterns are presented to the network before learning takes place. Typically, in batch training, several passes are made through the training data. In online training, each pattern is presented once and only once to the net.

A recurrent problem in the use of three-layer networks is the optimal number of hidden units to use in the network. The number of inputs and outputs of a three-layer network are determined by the problem to be solved. In the present invention, the number of inputs for a given neural network will equal the number of biomarkers selected from Y. The number of output for the neural network will typically be just one. If too many hidden units are used in a neural network, the network will have too many degrees of freedom and if trained too long, there is a danger that the network will overfit the data. If there are too few hidden units, the training set cannot be learned. Generally speaking, however, it is better to have too many hidden units than too few. With too few hidden units, the classifier might not have enough flexibility to capture the nonlinearities in the date; with too many hidden units, the extra weight can be shrunk towards zero if appropriate regularization or pruning, as described below, is used. In typical cases, the number of hidden units is somewhere in the range of 5 to 100, with the number increasing with the number of inputs and number of training cases.

*Clustering.* Optionally, a classifier is learned using clustering. Optionally, select components i of the vectors representing the landmark odorant activity profiles are used to cluster the odorant activity profiles. Optionally, prior to clustering, the measured amounts are normalized to have a mean value of zero and unit variance.

Landmark odorant activity profiles that exhibit similar patterns of measured amounts across the training population will tend to cluster together. A particular combination of measured amounts of components i can be considered to be a good classifier in this aspect of the invention when the vectors form clusters for a particular known odor. See, *e.g.,* pages 211-256 of Duda and Hart, Pattern Classification and Scene Analysis, 1973, John Wiley & Sons, Inc., New York (hereinafter "Duda 1973"). As described in Section 6.7 of Duda 1973, the clustering problem is described as one of finding natural groupings in a dataset. To identify natural groupings, two issues are addressed. First, a way to measure similarity (or dissimilarity) between two odorant activity profiles is determined. This metric (similarity measure) is used to ensure that the odorant activity profiles in one cluster are more like one another than they are to other odorant activity profiles. Second, a mechanism for partitioning the data into clusters using the similarity measure is determined.

Similarity measures are discussed in Section 6.7 of Duda 1973, where it is stated that one way to begin a clustering investigation is to define a distance function and to compute the matrix of distances between pairs of odorant activity profiles. If distance is a good measure of similarity, then the distance between odorant activity profiles in the same cluster will be significantly less than the distance between odorant activity profiles in different clusters. However, as stated on page 215 of Duda 1973, clustering does not require the use of a distance metric. For example, a nonmetric similarity function s(x, x') can be used to compare two vectors x and x'. Conventionally, s(x, x') is a symmetric function whose value is large when x and x' are somehow "similar". An example of a nonmetric similarity function s(x, x') is provided on page 216 of Duda 1973.

Once a method for measuring "similarity" or "dissimilarity" between points in a dataset has been selected, clustering requires a criterion function that measures the clustering quality of any partition of the data. Partitions of the data set that extremize the criterion function are used to cluster the data. See, *e.g.*, page 217 of Duda 1973. Criterion functions are discussed in Section 6.8 of Duda 1973. More recently, Duda et al., Pattern Classification, 2nd edition, John Wiley & Sons, Inc. New York, has been published. Pages 537-563 describe clustering in detail. Additional information on clustering techniques can be found in Kaufman and Rousseeuw, 1990, Finding Groups in Data: An Introduction to ClusterAnalysis, Wiley, New York, NY; Everitt, 1993, Cluster analysis (3d ed.), Wiley, New York, NY; and Backer, 1995, Computer-Assisted Reasoning in ClusterAnalysis, Prentice Hall, Upper Saddle River, New Jersey. Particular exemplary clustering techniques that can be used in the disclosure herein include, but are not limited to, hierarchical clustering (agglomerative clustering using nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering.

*Principal component analysis.* Optionally, a classifier is learned using principal component analysis. Principal component analysis is a classical technique to reduce the dimensionality of a data set by transforming the data to a new set of variable (principal components) that summarize the features of the data. See, *e.g.,* Jolliffe, 1986, Principal Component Analysis, Springer, New York. Principal component analysis is also described in Draghici, 2003, Data Analysis Tools for DNA Microarrays, Chapman & Hall/CRC. What follows is non-limiting examples of principal components analysis.

Principal components (PCs) are uncorrelated and are ordered such that the $k^{th}$ PC has the $k^{th}$ largest variance among PCs. The $k^{th}$ PC can be interpreted as the direction that maximizes the variation of the projections of the data points such that it is orthogonal to the first $k$- 1 PCs. The first few PCs capture most of the variation in the data set. In contrast, the last few PCs are often assumed to capture only the residual 'noise' in the data.

In one approach to using PCA to learn a classifier, vectors representing landmark odorant activity profiles can be constructed in the same manner described for clustering above. In fact, the set of vectors, where each vector represents a landmark odorant activity profile, can be viewed as a matrix. Optionally, this matrix is represented in a Free-Wilson method of qualitative binary description of monomers (Kubinyi, 1990, 3D QSAR in drug design theory methods and applications, Pergamon Press, Oxford, pp 589-638), and distributed in a maximally compressed space using PCA so that the first principal component (PC) captures the largest amount of variance information possible, the second principal component (PC) captures the second largest amount of all variance information, and so forth until all variance information in the matrix has been considered.

Then, each of the vectors, where each vector represents a member of the training population (such as the landmark odorant activity profiles), is plotted. Many different types of plots are possible. Optionally, a one-dimensional plot is made. In this one-dimensional plot, the value for the first principal component from each of the members of the training population is plotted. In this form of plot, the expectation is that odorant activity profiles corresponding to an odor will cluster in one range of first principal component values and profiles corresponding to another odor will cluster in a second range of first principal component values.

Optionally, the members of the training population are plotted against more than one principal component. For example, Optionally, the members of the training population are plotted on a two-dimensional plot in which the first dimension is the first principal component and the second dimension is the second principal component.

*Nearest neighbor analysis*. Optionally, a classifier is learned using nearest neighbor analysis. Nearest neighbor classifiers are memory-based and require no classifier to be fit. Given a query point $x_0$, the $k$ training points $x_{(r)}$, $r$, ..., $k$ closest in distance to $x_0$ are identified and then the point $x_0$ is classified using the $k$ nearest neighbors. Ties can be broken at random. Optionally, Euclidean distance in feature space is used to determine distance as:

$$d_{(i)} = \left\| x_{(i)} - x_0 \right\|.$$

Typically, when the nearest neighbor algorithm is used, the abundance data from Yused to compute the linear discriminant is standardized to have mean zero and variance 1. In the disclosure herein, the members of the training population are randomly divided into a training set and a test set. For example, in one case, two thirds of the members of the training population are placed in the training set and one third of the members of the training population are placed in the test set. A select combination of vector components i represents the feature space into which members of the test set are plotted. Next, the ability of the training set to correctly characterize the members of the test set is computed. Optionally, nearest neighbor computation is performed several times for a given combination of vector components i. In each iteration of the computation, the members of the training population are randomly assigned to the training set and the test set.

The nearest neighbor rule can be refined to deal with issues of unequal class priors, differential misclassification costs, and feature selection. Many of these refinements involve some form of weighted voting for the neighbors. For more information on nearest neighbor analysis, see, *e.g.,* Duda, Pattern Classification, Second Edition, 2001, John Wiley & Sons, Inc; and Hastie, 2001, The Elements of Statistical Learning, Springer, New York.

*Linear discriminant analysis.* Optionally, a classifier is learned using linear discriminant analysis. Linear discriminant analysis (LDA) attempts to classify a subject into one of two categories based on certain object properties. In other words, LDA tests whether object attributes measured in an experiment predict categorization of the objects. LDA typically requires continuous independent variables and a dichotomous categorical dependent variable. In the disclosure herein the abundance values for the select combinations of vector components *i* across a subset of the training population serve as the requisite continuous independent variables. The trait subgroup classification (*e.g.,* an odor) of each of the members of the training population serves as the dichotomous categorical dependent variable.

LDA seeks the linear combination of variables that maximizes the ratio of between-group variance and within-group variance by using the grouping information. Implicitly, the linear weights used by LDA depend on how the measured amount of a vector component i across the training set separates in the groups of the odor. Optionally, LDA is applied to the data matrix of the members in the training population. Then, the linear discriminant of each member of the training population is plotted. Ideally, those members of the training population representing an odor will cluster into one range of linear discriminant values (for example, negative) and those members of the training population representing another odor will cluster into a second range of linear discriminant values (for example, positive). The LDA is considered more successful when the separation between the clusters of discriminant values is larger. For more information on linear discriminant analysis, see, *e.g.,* Duda, Pattern Classification, Second Edition, 2001, John Wiley & Sons, Inc; and Hastie, 2001, The Elements of Statistical Learning, Springer, New York; and Venables & Ripley, 1997, Modern Applied Statistics with s-plus, Springer, New York.

*Quadratic discriminant analysis.* Optionally, classifier is learned using quadratic discriminant analysis. Quadratic discriminant analysis (QDA) takes the same input parameters and returns the same results as LDA. QDA uses quadratic equations, rather than linear equations, to produce results. LDA and QDA are interchangeable, and which to use is a matter of preference and/or availability of software to support the analysis. Logistic regression takes the same input parameters and returns the same results as LDA and QDA.

*Support vector machine.* Optionally, a classifier is learned using a support vector machine. SVMs are described, for example, in Cristianini and Shawe-Taylor, 2000, An Introduction to Support Vector Machines, Cambridge University Press, Cambridge; Boser et al., 1992, "A training algorithm for optimal margin classifiers," in Proceedings of the 5th Annual ACM Workshop on Computational Learning Theory, ACM Press, Pittsburgh, PA, pp. 142-152; Vapnik, 1998, Statistical Learning Theory, Wiley, New York; Mount, 2001, Bioinformatics: sequence and genome analysis, Cold Spring Harbor Laboratory Press, Cold Spring Harbor, New York, Duda, Pattern Classification, Second Edition, 2001, John Wiley & Sons, Inc.; and Hastie, 2001, The Elements of Statistical Learning, Springer, New York; and Furey et al., 2000, Bioinformatics 16, 906-914. When used for classification, SVMs separate a given set of binary labeled data training data with a hyper-plane that is maximally distant from them. For cases in which no linear separation is possible, SVMs can work in combination with the technique of 'kernels', which automatically realizes a non-linear mapping to a feature space. The hyper-plane found by the SVM in feature space corresponds to a non-linear decision boundary in the input space. For more information on support vector machines see, for example, Furey et al., 2000, Bioinformatics 16, page 906-914, which is hereby incorporated by reference herein.

*Decision tree.* Optionally, a classifier is a decision tree. Decision trees are described generally in Duda, 2001, Pattern Classification, John Wiley & Sons, Inc., New York, pp. 395-396. One specific algorithm that can be used is a classification and regression tree (CART). Other specific algorithms include, but are not limited to, ID3, C4.5, MART, and Random Forests. CART, ID3, and C4.5, each described in Duda, 2001, Pattern Classification, John Wiley & Sons, Inc., New York, pp. 396-408 and pp. 411-412. CART, MART, and C4.5 are also described in Hastie et al., 2001, The Elements of Statistical Learning, Springer-Verlag, New York, Chapter 9. The Random Forests technique is described in Breiman, 1999, "Random Forests - Random Features," Technical Report 567, Statistics Department, University of California at Berkeley, September 1999.

In addition to univariate decision trees in which each split is based on measured amounts for a corresponding vector component i, or the relative measured amounts of vector components i, the classifier can be a multivariate decision tree. In such a multivariate decision tree, some or all of the decisions actually comprise a linear combination of measured amounts for a plurality of vector components i. Multivariate decision trees are described in Duda, 2001, Pattern Classification, John Wiley & Sons, Inc., New York, pp. 408-409.

*Multivariate adaptive regression splines*. Another approach that can be used to learn a pairwise probability function $g_{pq}(X, W_{pq})$ uses multivariate adaptive regression splines (MARS). MARS is an adaptive procedure for regression, and is well suited for the high-dimensional problems addressed by the disclosure herein. MARS can be viewed as a generalization of stepwise linear regression or a modification of the CART method to improve the performance of CART in the regression setting. MARS is described in Hastie et al., 2001, The Elements of Statistical Learning, Springer-Verlag, New York, pp. 283-295.

*Centroid classifier techniques.* In one case a nearest centroid classifier technique is used. Such a technique computes, for the different odors, a centroid given by the average measured amounts of vector components i in the training population (landmark odorant activity profiles), and then assigns vector representing the compound of interest to the class whose centroid is nearest. This approach is similar to k-means clustering except clusters are replaced by known classes. An example implementation of this approach is the Prediction Analysis of Microarray, or PAM. See, for example, Tibshirani et al., 2002, Proceedings of the National Academy of Science USA 99; 6567-6572.

*Regression*. Optionally, the classifier is a regression classifier, such as a logistic regression classifier. Such a regression classifier includes a coefficient for each of the odorant activity profiles used to construct the classifier. In such cases, the coefficients for the regression classifier are computed using, for example, a maximum likelihood approach. In such a computation, the measured amounts of vector components i are used.

*Other methods*. Optionally, the classifier is learned using k-nearest neighbors (k-NN), an artificial neural network (ANN), a parametric linear equation, a parametric quadratic equation, a naive Bayes analysis, linear discriminant analysis, a decision tree, or a radial basis function.

Also disclosed herein is a computer program product that contains any or all of the program modules shown in Fig. 1. Aspects of the program modules are further described hereinbelow.

Also disclosed herein are cells or cell lines, or panels of cells or cell lines that express a biologic, *e.g.,* a secreted protein. Secreted proteins may include antibodies and active fragments thereof, *e.g.*, antibodies comprising heavy and light chains, single chain antibodies, proteins having an activity in the immune system, IgA, IgD, IgE, IgG and IgM proteins or active fragments thereof, enzymes, coagulation factors or hormones or a protein corresponding to a fragment of any of these. Biologics may also include FDA-approved biologics drugs, known biologics, proteins that are therapeutically active or therapeutic biologics. Examples of biologics and their brand names, include, but are not limited to: Canakinumab (Ilaris), Abobotulinumtoxin A (Dysport), Golimumab (Simponi), Romiplostim (NPLATE), Certolizumab Pegol (Cimzia), Rilonacept (Arcalyst),methoxy polyethylene glycol-epoetin beta (Mircera), eculizumab (Soliris), panitumumab (Vectibix), idursulfase (Elaprase), ranibizumab (Lucentis), alglucosidase alfa (Myozyme), Abatacept (Orencia), Galsulfase (Naglazyme), Palifermin (Kepivance), Natalizumab (Tysabri), Bevacizumab (Avastin), Cetuximab (Erbitux), nofetumomab (Verluma), capromab pendetide (ProstaScint), epoietin alfa (Procrit, Epogen), technetium fanolesomab (NeutroSpec), arcitumomab (CEA-Scan), darbepoetin alfa (Aranesp), urokinase (Abbokinase), Ibritumomab tiuxetan (Zevalin), Rituximab (Rituxan), Aldesleukin (Proleukin), Denileukin diffitox (Ontak), Pegaspargase (Oncaspar), Filgrastim (Neupoen), Oprelvekin (Neumega), Pegfilgrastim (Nuelasta), Sargramostim, (Leukine), Palifermin (Kepivance), trastuzumab (Herceptin), Cetuximab (Eribitux), Asparginase (Elspar) Rasburicase (Elitek), Alemtuzumab (Campath), Tositumomab (Bexxar), Palivizumab (Synagis), Interferon alfa-2a (Roferon-A), Peginterferon alfa-2b (Peg-Intron), Peginterferon alfa-2a (Pegasys), interferon alfa-2b (Intron A), interferon alfacon-1 (Infergen), peginterferon alpha-2 (Copasys Copegus), Daclizumab (Zenapax), Basiliximab (Simulect), Moromonab-CD3 (Orthocolone, OKT3), interferon gamma-1 b (Actimmune), Drotrecogin alfa -activated (Xigris), Collagenase (Santyl), Becaplermin (Regranex), Efalizumab (Raptiva), Alefacept (Amevive), Interferon alfa-n3 (Alferon N), Galsulfase (Naglazyme), Agalsidase beta (Fabrazyme), Laronidase (Aldurazyme), Infliximab (Remicade), Abatacept (Orencia), Anakinra (Kineret), Adalimumab (Humira), Enteracept (Enbrel), Omalzumab (Xolair), Dornase alpha (Pulmozyme), Natalizumab (Tysabri), Interferon beta-1-a (Rebif), Botulinum Toxin Type B (Myobloc), Botulinum Toxin Type A (Botox), interferon beta-1-b (Betaseron), interferon beta-1-a (Avonex), Tenecteplase (TNKase), Streptokinase (Streptase), Reeplase (Retavase), Abciximab (ReoPro), Alteplase (Cathflo Activase, Activase), epo alpha (Abseamed, Binocrit), indunorate-2-sulfatase (Elaprase), Insulin (Exubera), HPV vaccine (Gardasil), pegaptanib (Macugen), human acid-a-glucosidase (Myozyme), galsulfase (Naglazyme), human growth hormone (Omnitrope), Parathyroid hormone (Preotach), human growth hormone (Valtropin), antithrombin (Atryn), Major capsid L1 proteins from HPV (Cervarix), erythropoietin alfa (Epotein alfa hexal), mecasermin human IGF-1 (Increlex), methoxy polyethylene glycol-epoitein beta (Mlrcera), follitropin alpha/lutrophin alpha Pergoveris, epoietin zeta (Retacrit, Silapo), Ribavirin and interferon alfa-2b (Rebetron Combination Therapy), alglucerase (Ceredase), imiglucerase (Cerezyme), human insulin (Humulin, Novolin), somatropin (Humatrope, Nutropin/Nutropin AQ), Sermorelin (Geref), somatrem (Protopin), human albumin (Albutein), and gemtuzumab (Mylotarg).

Cells and cell lines with properties optimal for expression of a secreted protein may be selected using known tests to characterize clones with respect to any of properties, including, but not limited to: post-translational processing or modification, yield, percent active product, stability of certain properties (*e.g.*, by testing the properties as described herein but over time), and stoichiometry (*e.g.*, by RT-PCR or protein analysis).

The post-translational processing or modification of a secreted protein may be characterized by tests known in the art including, but not limited to, protein sequencing, mass spectroscopy, methods to test for glycosylation or phosphorylation, or a covalent addition of a chemical group or residue to select cell lines and conditions that result in a specific or desired form of the secreted protein product. Cells and cell lines may be designed to or selected to express one or more factors that effect the post-translational processing or modification of the secreted product, for instance by introducing sequences corresponding to enzymes that catalyze the post-translational processing or modification or by testing cell lines to select for those endogenously expressing these or gene-activated to express these.

Cells and cell lines with maximal production or yield of the secreted product may be produced by testing isolated clones using methods to assess secreted protein product levels, for instance using ELISA methods (*e.g.*, ELISA that detect FC fragment to assess antibody yield).

Cells and cell lines that result in maximal percent active secreted protein compared to total yield of secreted product may be produced by testing isolated clones using methods to assess the activity or binding of the protein, for instance by using activity assays, functional assays, or binding assays such as functional cell based assays, ELISAs utilizing capture reagents that comprise the binding epitope, secondary tests, animal studies or a test that is used to measure the binding or activity of the protein.

Cell lines that additionally are optimized for growth in animal component free media conditions and/or in suspension conditions as used in reactors for scaled-up production can be produced either by operating the methods used for cell line production under these and/or similar conditions or by testing clones under these desired conditions to identify those cell lines having the desired properties. Optionally, cell lines that are additionally optimized for growth in media supplemented with compounds that normally retard or impede cell growth can be produced by selecting for cells that exhibit normal or improved growth under these conditions compared to most cells of the same cell type.

Optionally, cells or cell lines that express a protein of interest according to the present invention may be used to produce the protein of interest. Encompassed herein are methods of protein production using cells that possess certain physiological properties favorable for protein production and/or have been engineered to express one or more protein expression accessory factors. Optionally, cells that express a protein of interest consistently and reproducibly as described herein (*e.g.*, for a period of time selected from: at least one week, at least two weeks, at least three weeks, at least one month, at least two months, at least three months at least four months, at least five months, at least six months, at least seven months, at least eight months, and at least nine months) are further modified and/or selected to provide an improved environment for protein production. Optionally, a cell that is engineered to express a protein of interest and a protein expression accessory factor can be used for production of the protein of interest. Further optionally, the protein of interest and/or the protein expression accessory factor is expressed consistently and reproducibly.

Optionally, the protein of interest is a biologic, *e.g.*, an antibody for therapeutic use. Any protein of interest or fragment thereof can be produced in accordance with the methods described herein including, but not limited to, membrane proteins, transmembrane proteins, structural proteins, membrane-anchored proteins, cell surface receptors, secreted proteins, cytosolic proteins, heteromultimeric proteins, homomultimeric proteins, dimeric proteins, monomeric proteins, post-translationally modified proteins, glycosylated proteins, phosphorylated proteins, and proteolytically processed proteins. Specific examples of such proteins include, but are not limited to, antibodies (including antibody fragments such as Fab and Fab, Fab', $F(ab')_2$, Fd, Fv, dAb and the like, single chain antibodies (scFv), single domain antibodies, heavy chain, light chain, and all antibody classes, *i.e.,* IgA, IgD, IgE, IgG and IgM), enzymes, coagulation factors, hormones, cytokines, ion channels, G-protein coupled receptors (GPCRs), and transporters. Other exemplary biologics include those disclosed hereinabove.

Optionally, cells that are favorable for protein production produce at least 1%, 2%, 5%, 10%, 15%, 20%, 25%, 30%, 40%, 50%, 60%, 70%, 80%, 90%, 100%, 250%, 500%, or at least 1000% more protein of interest than a reference cell. Optionally, cells that are favorable for protein production produce a protein of interest that has at least 1%, 2%, 5%, 10%, 15%, 20%, 25%, 30%, 40%, 50%, 60%, 70%, 80%, 90%, 100%, 250%, 500%, or at least 1000% more activity than the same protein of interest expressed in a reference cell. Activity may refer, *e.g.*, to enzymatic activity or binding activity or therapeutic activity to a binding partner of the protein of interest. Optionally, cells that are favorable for protein production produce protein of interest wherein at least 1%, 2%, 5%, 10%, 15%, 20%, 25%, 30%, 40%, 50%, 60%, 70%, 80%, 90%, 100%, 250%, 500%, or at least 1000% more protein of interest is secreted relative to a reference cell. Optionally, at least 50%, 60%, 70%, 80%, 90%, 95%, 98%, or at least 99% of the protein of interest are localized in the subcellular compartment in which the protein of interest is located in a cell that expresses the protein of interest without genetic engineering. Parameters to assess protein expression in a cell include quantification/analysis of protein yield, processing, modification, localization within cell or secretion, percent total protein that is optimally folded/processed. A reference cell may be the host cell from which the cell that expresses the protein of interest was generated. Any cell disclosed herein may be used as a reference cell.

Proteins produced in accordance with the methods provided herein may be characterized using methods known in the art, including protein sequencing, mass spectroscopy, immunocytochemistry, methods to analyze the extent or type of glycosylation and/or phosphorylation, as well as methods for determining the covalent addition of chemical groups and/or residues to the protein produced.

Cells that yield the greatest overall amount of protein can be identified using methods known in the art, such as ELISA. Moreover, cells that result in the maximal percentage of active protein compared to total yield of protein can be identified using assays that detect the activity of the protein or the binding of the protein, such as activity assays, functional assays, binding assays (*e.g.*, ELISA), secondary tests, animal studies, or cell based assays.

Optionally, protein expression in a cell that expresses a protein of interest is tested using a test protein, wherein if the test protein is expressed at levels higher than in a reference cell then the protein of interest in the same cell is predicted to be expressed at higher levels compared to the reference cell. Test proteins could include any protein, membrane proteins, transmembrane proteins, membrane anchored proteins, cell surface receptors, secreted proteins, cytosolic proteins, heteromultimeric proteins, homomultimeric proteins, dimeric proteins, monomeric proteins, proteins that are post translationally modified, proteins that are glycosylated/phosphorylated/proteolytically processed, and any possible combination of the above. Optionally, specific test proteins could include antibodies, ion channels, GPCRs, transporters. Cells may be tested with just one or multiple test proteins. In the case of multiple test proteins, they may be of one or multiple types (e.g multiple GPCRs only, or GPCRs, ion channels and antibodies).

Cells that possess physiological properties that are favorable for the production of proteins (*e.g.*, cells with increased endoplasmic reticulum mass) as well as cells that have been engineered to be optimized for the production of proteins (*e.g.*, by introduction of genes that encode proteins beneficial for protein production) can be identified using methods known in the art, the methods described herein, or a combination thereof. Once such cells are identified, the cells can be cultured and cell lines that stably express one or more genes that encode proteins beneficial for protein production can be generated in accordance with the methods described herein. Such cell lines can be engineered to express a protein of interest by introduction of a transgene or by gene activation either before or after selection of the cells that are favorable for protein production. Cells that produce the protein of interest then can be identified using methods known in the art and/or the methods described herein.

Cells that possess physiological properties that are favorable for the production of proteins include, but are not limited to, cells with improved viability; increased cell size; increased production of endogenously expressed proteins; increased mitochondrial activity; improved stability; the ability to maintain the properties for which they were selected; increased size of one or more organelles involved in protein processing (*e.g.*, the endoplasmic reticulum and ribosomes); and increased content of one or more organelles involved in protein processing (*e.g.*, lysosomes and endosomes). These physiological properties can be compared relative to a reference cell or historical values for a reference cell.

Cells that possess physiological properties that are favorable for the production of proteins can be identified using FACS analysis in combination with standard approaches for fluorescently labeled probes. In particular, markers for certain physiological properties are used to quantify physiological properties that relate to protein expression and compare them to a reference cell. Such markers include molecules that detect cellular structures that are related to protein expression such as ribosomes, mitochondria, ER, rER, golgi, TGN, vesicles, endosomes, and plasma membrane. Such markers can be fluorescent stains. For example, the activity of certain cellular organelles, e.g., mitochondrial activity, could be assessed by assaying for fluorescent metabolites. Fluorescent metabolites that report the activity of the organelles/compartments (*e.g.* mitochondrial activity, or incorporation of sugars onto proteins (which eg can be detected using fluorescent lectins) can be used. Proteins markers specific to one or more cellular organelles involved in protein processing could be expressed as fusion proteins with auto-fluorescing protein tags, *e.g.*, green fluorescent protein (GFP); and membrane proteins and/or secreted proteins could be labeled with fluorescent probes. Probes (e.g antibodies) to endogenously expressed proteins may be used as markers for the output of cellular organelles/compartments. Specifically, heteromultimeric membrane proteins could be used as read-out of protein expression activity in a cell.

Without being bound by theory, an increase in fluorescence as measured by FACS would suggest that the cell possesses qualities favorable for the production of proteins, and such cells could be isolated for future use. In addition to these standard techniques, the methods described herein could be used to identify cells that naturally possess physiological properties that are favorable for the production of proteins. For example, signaling probes complementary to target sequences of endoplasmic reticulum markers (*e.g.*, ERp29, cytochrome P450, NADPH-cytochrome c reductase, Calreticulin) could be used in accordance with the methods described herein to identify cells with increased endoplasmic reticulum size. Cells that demonstrate a high degree of fluorescence, as measured by FACS, would likely have increased ER size and/or content, and thus would possess qualities favorable for the production of proteins.

Optionally, cells that possess physiological properties that are favorable for the production of proteins are identified and subsequently used to develop stable cell lines which are used to produce a protein(s) of interest. Further optionally, cells are engineered to express a protein of interest followed by the identification of cells that express the protein of interest. Cells that express the protein of interest and additionally possess physiological properties that are favorable for the production of proteins then are identified. Such cells then are used to develop stable cell lines which produce the protein(s) of interest.

Optionally, a cell is transfected with a nucleic acid encoding the protein of interest; a fluorogenic oligonucleotide capable of detecting the transcript of the nucleic acid is introduced into the cell; a fluorogenic probe for a marker of a physiological property related to protein expression is introduced into the cell; selection of a cell that expresses the protein of interest and has increased levels of the physiological property related to protein expression. A cell line that expresses the protein of interest consistently and reproducibly can then be established.

The cells used in the methods of protein production provided herein may be engineered to express a protein expression accessory factor or a combination of two or more protein expression accessory factors. Optionally, the cells are engineered to express a splice variant, mutant, or fragment of a protein expression accessory factor. Optionally, a cell is engineered to express at least 2, 5, 10, 15, 20, 25, 30, 40, 50, 75, 100, or 150 protein expression accessory factors. Illustrative protein expression accessory factors include: proteins that regulate the unfolded protein response (UPR) and genes that encode proteins that are regulated in the UPR (e.g., ATF6α (spliced), IRE1α, IRE1β, PERCΔC, ATF4, YYI, NF-YA, NF-YB, NF-YC, XBP1 (spliced), EDEM1); genes that encode proteins that switch-off the apoptotic pathway induced by the UPR (*e.g.*, NRF2, HERP XIAP, GADD34, PPIα, PPIβ, PPIγ, DNAJC3); genes that encode proteins that affect the growth of cells, the viability of cells, cell death, and cell size; B-cell genes *(e.g.,* BLIMP-1, XBP1 (spliced)); genes that encode proteins involved in protein transport *(e.g.,* Sec61 Pα, Sec61 Pβ, Sec61Pγ); genes that encode proteins involved in glycosylation (*e.g.*, SDF2-L); genes that encode proteins involved in oxidation *(e.g.,* ERO1α, ERO1β); genes that encode anti-apoptotic proteins *(e.g.,* Bcl-2sp, Bcl-xL, Bim trunk. Mut., Ku70, 14-3-3q mut., VDAC2, BAP31 mut.); genes that encode proteins implicated in endoplasmic reticulum-associated degradation *(e.g.,* mannosidase 1, HRD1); genes that encode proteins involved in calcium transport (*e.g.*, STC1, STC2, SERCA1, SERCA2, COD1); genes that encode proteins implicated in lipogenesis/metabolism *(e.g.,* INO1, PYC, SREBP1ΔC, SREBP2ΔC); and genes that encode proteins implicated in protein folding and secretion (*e.g.,* CRT (CaBP3), CNX, ERp57 (PDIA3), BiP, BAP, ERdj3, CaBP1, GRP94 (CaBP4), ERp72 (PDIA4), cyclophilin B), protein assembly, the integration of proteins into membranes, cell surface presentation of proteins, and post-translational modification of proteins. Optionally, the cells are engineered to express any one or a combination of genes implicated in the UPR. Further optionally, the cells are engineered to express any one or a combination of genes implicated in the UPR as well as at least one other gene that encodes a protein known to be beneficial for protein production. Genes that regulate UPR or are regulated in UPR; genes that alter cell growth, viability, apoptosis, cell death, cell size; genes encoding chaperones or factors implicated in protein folding, assembly, membrane integration, cell surface presentation or secretion, post-translational modification including glycosylation/phosphoylation/proteolysis can be used. Optionally, a protein expression accessory factor alters a cell physiological property.

Identification of cells that express one or a combination of genes that encode proteins known to be beneficial for protein production can be accomplished using the methods described herein, *e.g.*, signaling probes that bind to target sequences in the genes/mRNA of interest could be generated and the presence of the gene/mRNA of interest then could be verified by FACS analysis.

Optionally, a cell is transfected with a first nucleic acid encoding the protein of interest and a second nucleic acid encoding a protein expression accessory factor; a fluorogenic oligonucleotide capable of detecting the transcript of the first nucleic acid and a fluorogenic oligonucleotide capable of detecting the transcript of the second nucleic acid are introduced into the cell; selection of a cell that expresses the protein of interest and the protein expression accessory factor. A cell line that expresses the protein of interest consistently and reproducibly can then be established. The cell line can be further tested for physiological properties related to protein expression as discussed above.

Optionally, cells are first engineered to express a protein expression accessory factor; cell lines expressing the protein expression accessory factor are established, cells of the cell line are then engineered to express a protein of interest. Further optionally, cells are first engineered to express a protein of interest; cell lines expressing the protein of interest are established, cells of the cell line are then engineered to express a protein expression accessory factor. Still further optionally, cells are concurrently engineered to express a protein of interest and a protein expression accessory factor. Cell lines that express the protein of interest and / or the protein expression accessory factor consistently and reproducibly can then be established as described herein.

Optionally, a plurality of cells that have been engineered to express a protein of interest are provided. These cells are then engineered to express a protein expression accessory factor and / or a cell most favorable to protein expression is selected from the plurality by using a marker for a physiological property related to protein expression as discussed above.

Optionally, the cell lines can be optimized for growth in animal component-free media and/or in suspension conditions as used in reactors for scaled-up production in accordance with the methods provided herein. Optionally, the cell lines can be optimized for growth in media that comprises a component that slows growth.

Further optionally, a method for protein production is provided that comprises (i) identifying cells that possess physiological properties that are favorable for the production of proteins; (ii) engineering the cells to express a protein of interest; (iii) generating a cell line that stably expresses the protein of interest; (iv) culturing the cells under conditions suitable for production of the protein of interest; and (v) isolation of the protein of interest.

Further optionally, a method for protein production is provided that comprises (i) introducing to cells at least one gene that encodes a protein expression accessory factor; (ii) identifying cells that express the protein expression accessory factor; (iii) engineering the cells to express a protein of interest; (iv) generating a cell line that stably expresses the protein of interest; (v) culturing the cells under conditions suitable for production of the protein of interest; and (vi) isolation of the protein of interest.

Further optionally, a method for protein production is provided that comprises (i) introducing to cells at least one gene that encodes a protein expression accessory factor; (ii) identifying cells that express the protein expression accessory factor; (iii) identifying cells that possess physiological properties that are favorable for the production of proteins; (iv) engineering the cells to express a protein of interest; (v) generating a cell line that stably expresses the protein of interest; (vi) culturing the cells under conditions suitable for production of the protein of interest; and (vii) isolation of the protein of interest. In one aspect, steps (ii) and (iii) are performed sequentially. In another aspect, steps (ii) and (iii) are performed concurrently.

Further optionally, a method for protein production is provided that comprises (i) introducing to cells at least one gene that encodes a protein expression accessory factor and engineering the cells to express a protein of interest; (ii) identifying cells that express the protein expression accessory factor and the protein of interest; (iii) generating a cell line that stably expresses the protein expression accessory factor and the protein of interest; (iv) culturing the cells under conditions suitable for production of the protein of interest; and (v) isolation of the protein of interest.

Further optionally, a method for protein production is provided that comprises (i) introducing to cells at least one gene that encodes a protein expression accessory factor and engineering the cells to express a protein of interest; (ii) identifying cells that express the protein expression accessory factor and the protein of interest; (iii) identifying cells that possess physiological properties that are favorable for the production of proteins; (iv) generating a cell line that expresses the protein expression accessory factor and the protein of interest consistently and reproducibly; (v) culturing the cells under conditions suitable for production of the protein of interest; and (vi) isolation of the protein of interest. In one aspect, steps (ii) and (iii) are performed sequentially. In another aspect, steps (ii) and (iii) are performed concurrently.

Host cells that can be used to generate cells suitable for protein production include primary cells and immortalized cells. Optionally, host cells can be, *e.g.,* CHO cells, NS0 cells, PerC6 cells, yeast cells, insect cells, 293 cells, CACO cells, HUVEC, CHOK1, CHOKiSV, NS0, 293T cells, and insect cells.

Optionally, cells or cell lines that produce or are capable of producing 0.1, 0.2, 0.3, 0.4, 0.5, 0.6, 0.7, 0.8, 0.9, 1.0, 1 to 1.5, 1.5 to 2.0, 2.0 to 2.5, 2.5 to 3.0, 3.0 to 3.5, 3.5 to 4.0, 4.0 to 4.5, 4.5 to 5.0, 5.0 to 5.5, 5.5 to 6.0, 6.5 to 7.0, 7.0 to 7.5, 7.5 to 8.0, 8.0 to 8.5, 8.5 to 9.0, 9.0 to 9.5, 9.5 to 10.0,10 to 11, 11 to 12, 12 to 13, 13 to 14, 14 to 15, 15 to 16, 16 to 17, 17 to 18, 18 to 19, 19 to 20, 20 to 25 or more than 25 grams per liter of a protein of interest are produced in less than 1, 2, 3, or 5 weeks. Optionally, cells or cell lines that produce or are capable of producing 0.1, 0.2, 0.3, 0.4, 0.5, 0.6, 0.7, 0.8, 0.9,1.0, 1 to 1.5, 1.5 to 2.0, 2.0 to 2.5, 2.5 to 3.0, 3.0 to 3.5, 3.5 to 4.0, 4.0 to 4.5, 4.5 to 5.0, 5.0 to 5.5, 5.5 to 6.0, 6.5 to 7.0, 7.0 to 7.5, 7.5 to 8.0, 8.0 to 8.5, 8.5 to 9.0, 9.0 to 9.5, 9.5 to 10.0,10 to 11, 11 to 12, 12 to 13, 13 to 14, 14 to 15, 15 to 16, 16 to 17, 17 to 18, 18 to 19, 19 to 20, 20 to 25 or more than 25 grams per liter of a protein of interest are produced in less than 1, 2, 3, 4,5, 6, 7, 8 or 9 months. Optionally, the cells are stable over 1, 2, 3, 4, 5,6, 7, 8, 9, 10 months wherein the level of protein produce does not vary by more than 30%. Optionally, the cells are stable over 1 year, 2 years or more in continuous culture wherein the level of protein produce does not vary by more than 30%. Optionally, the protein of interest is a biologic or a protein that can be used in clinical applications. Optionally, the protein of interest is an antibody. Optionally, the protein of interest is modified, post-translationally modified or glycosylated. Optionally, the protein produced by the cell is modified, post-translationally modified or glycosylated by a second protein that the cells are engineered to comprise.

Also disclosed herein are methods to produce equivalent biologic products that match the properties of existing biologic products (e.g., bio-equivalent or biosimilar biologics) in a short period of time.

A matched panel disclosed herein may be produced by generating the different cell lines for the panel sequentially, in parallel or a combination of both. For example, one can make each cell line individually and then match them. More preferably, to minimize difference between the cell lines, sequentially generated cell lines can be frozen at the same stage or passage number and thawed in parallel. Even more preferably, the cell lines are made in parallel. Optionally, matched panels can be made by producing each cell line of the panel using conditions, protocols or cell culture steps that are substantially the same.

Preferably, the cell lines in a panel are screened or assayed in parallel.

The cell lines of the matched panel disclosed herein are maintained under the same cell culture conditions including but not limited to the same culture media, temperature, and the like. All of the cell lines in the panel are passaged at the same frequency which may be any desired frequency depending on a number of factors including cell type and growth rate. As will be appreciated, to maintain roughly equal numbers of cells from cell line to cell line of the panel, the number of cells should be normalized periodically.

According to the method, cells may be cultured in any cell culture format so long as the cells or cell lines are dispersed in individual cultures prior to the step of measuring growth rates. For example, for convenience, cells may be initially pooled for culture under the desired conditions and then individual cells separated one cell per well or vessel.

Cells may be cultured in multi-well tissue culture plates with any convenient number of wells. Such plates are readily commercially available and will be well known to a person of skill in the art. In some cases, cells may preferably be cultured in vials or in any other convenient format, the various formats will be known to the skilled worker and are readily commercially available.

In cases comprising the step of measuring growth rate, prior to measuring growth rates, the cells are cultured for a sufficient length of time for them to acclimate to the culture conditions. As will be appreciated by the skilled worker, the length of time will vary depending on a number of factors such as the cell type, the chosen conditions, the culture format and may be any amount of time from one day to a few days, a week or more.

Preferably, each individual culture in the plurality of separate cell cultures is maintained under substantially identical conditions as discussed below, including a standardized maintenance schedule. Another advantageous feature of the method is that large numbers of individual cultures can be maintained simultaneously, so that a cell with a desired set of traits may be identified even if extremely rare. For those and other reasons, the plurality of separate cell cultures are cultured using automated cell culture methods so that the conditions are substantially identical for each well. Automated cell culture prevents the unavoidable variability inherent to manual cell culture.

Any automated cell culture system may be used in the method of the invention. A number of automated systems are commercially available and will be well-known to the skilled worker. Optionally, these systems could be adapted for use to automate or standardize the culture of multiple separate cultures of cells or cell lines. Optionally, these systems could be adapted for use to automate or standardize the culture of multiple separate cultures of cells or cell lines under substantially identical conditions. Optionally, these systems could be adapted for use to automate or standardize the parallel culture of multiple separate cultures of cells or cell lines under substantially identical conditions. Optionally, these systems could be adapted for use to automate or standardize the culture of multiple separate cultures of cells or cell lines such that at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50 or more than 50 physiological properties of the cells are maintained during culture. Optionally, these systems could be adapted for use to automate or standardize the culture of multiple separate cultures of cells or cell lines such that the RNA or protein of interest is stably expressed by the cells or cell lines. Optionally, these systems could be adapted for use to automate or standardize the culture of multiple separate cultures of cells or cell lines such that at least 1, 2, 3,4, 5, 6, 7, 8, 9, 10, 11, 12, 13, 14, 15, 16, 17, 18 , 19, 20, 21, 22, 23, 24, 25, 26, 27, 28, 29, 30, 35, 40, 45, 50 or more than 50 physiological properties of the cells are maintained and such that the RNA or protein of interest is stably expressed by the cells or cell lines. Optionally, the automated system is a robotic system. Preferably, the system includes independently moving channels, a multichannel head (for instance a 96-tip head) and a gripper or cherry-picking arm and a HEPA filtration device to maintain sterility during the procedure. The number of channels in the pipettor should be suitable for the format of the culture. Convenient pipettors have, *e.g.,* 96 or 384 channels. Such systems are known and are commercially available. For example, a MICROLAB STAR™ instrument (Hamilton) may be used in the method of the invention. The automated system should be able to perform a variety of desired cell culture tasks. Such tasks will be known by a person of skill in the art. They include but are not limited to: removing media, replacing media, adding reagents, cell washing, removing wash solution, adding a dispersing agent, removing cells from a culture vessel, adding cells to a culture vessel and the like.

The production of a cell or cell line disclosed herein may include any number of separate cell cultures. However, the advantages provided by the method increase as the number of cells increases. There is no theoretical upper limit to the number of cells or separate cell cultures that can be utilized in the method. The number of separate cell cultures can be two or more but more advantageously is at least 3, 4, 5, 6, 7, 8, 9, 10 or more separate cell cultures, for example, at least 12, at least 15, at least 20, at least 24, at least 25, at least 30, at least 35, at least 40, at least 45, at least 48, at least 50, at least 75, at least 96, at least 100, at least 200, at least 300, at least 384, at least 400, at least 500, at least 1000, at least 10,000, at least 100,000, at least 500,000 or more.

Optionally, the cells and cell lines disclosed herein that are cultured as described are cells at least two of which have previously been selected as positive for a nucleic acid of interest, which can be an introduced nucleic acid encoding all or part of a protein of interest or an introduced nucleic acid that activates transcription of a sequence encoding all or part of a protein of interest. Optionally, the cells that are cultured as described herein are cells at least two of which have been selected as positive for an RNA of interest or an RNA encoding the protein of interest.

To make cells and cell lines disclosed herein, one can use, for example, the technology described in U.S. Patent 6,692,965 and WO/2005/079462. This technology provides real-time assessment of millions of cells such that any desired number of clones (from hundreds to thousands of clones) may be sorted and isolated based on RNA expression. Using cell sorting techniques, such as flow cytometric cell sorting (*e.g.*, with a FACS machine) or magnetic cell sorting (*e.g.*, with a MACS machine), one cell per well is automatically deposited with high statistical confidence in a culture vessel (such as a 96 well culture plate). The speed and automation of the technology allows multigene recombinant cell lines to be readily isolated. Optionally, cells positive for the desired signal (*i.e.,* cells that express the desired RNA) are pooled. Such a pool can then be subjected to a second round of selection. Optionally, the pool of cells is subjected to a total of at least 2, 3, 4, 5, 6, 7, 8, 9, 10, 15, 20, 25, or at least 50 rounds of selection.

Using the technology, the RNA sequence for a protein of interest may be detected using a signaling probe, also referred to as a molecular beacon or fluorogenic probe. In some embodiments, the vector containing the coding sequence has an additional sequence coding for an RNA tag sequence. "Tag sequence" refers to a nucleic acid sequence that is an expressed RNA or portion of an RNA that is to be detected by a signaling probe. Signaling probes may detect a variety of RNA sequences, any of which may be used as tags, including those encoding peptide and protein tags described above. Signaling probes may be directed against the tag by designing the probes to include a portion that is complementary to the sequence of the tag. The tag sequence may be a 3' untranslated region of the plasmid that is cotranscribed with the transcript of the protein of interest and comprises a target sequence for signaling probe binding. The tag sequence can be in frame with the protein-coding portion of the message of the gene or out of frame with it, depending on whether one wishes to tag the protein produced. Thus, the tag sequence does not have to be translated for detection by the signaling probe. The tag sequences may comprise multiple target sequences that are the same or different, wherein one signaling probe hybridizes to each target sequence. The tag sequence may be located within the RNA encoding the gene of interest, or the tag sequence may be located within a 5'- or 3'-untranslated region. The tag sequences may be an RNA having secondary structure. The structure may be a three-arm junction structure. In some embodiments, the signaling probe detects a sequence within the coding sequence for the protein of interest. The tag sequence may contain chemically modified nucleotides. The tag sequences can be generated and used as described in International Patent Application Publication No. WO2005/079462 published on September 1, 2005 (Application No. PCT/US05/005080).

Following transfection of the DNA constructs into cells and subsequent drug selection (if used), or following gene activation, molecular beacons (*e.g.*, fluorogenic probes), each of which is targeted to a different tag sequence and differentially labeled, may be introduced into the cells, and a flow cytometric cell sorter is used to isolate cells positive for their signals (multiple rounds of sorting may be carried out). Optionally, the flow cytometric cell sorter is a FACS machine. MACS (magnetic cell sorting) or laser ablation of negative cells using laser-enabled analysis and processing can also be used. Other fluorescence plate readers, including those that are compatible with high-throughput screening can also be used. Signal-positive cells take up and may integrate into their genomes at least one copy of the introduced sequence(s). Cells introduced with message for the protein of interest are then identified. By way of example, the coding sequences may be integrated at different locations of the genome in the cell. The expression level of the introduced sequence may vary based upon copy number or integration site. Further, cells comprising a protein of interest may be obtained wherein one or more of the introduced nucleic acids is episomal or results from gene activation.

Signaling probes useful in this invention are known in the art and generally are oligonucleotides comprising a sequence complementary to a target sequence and a signal emitting system so arranged that no signal is emitted when the probe is not bound to the target sequence and a signal is emitted when the probe binds to the target sequence. By way of non-limiting illustration, the signaling probe may comprise a fluorophore and a quencher positioned in the probe so that the quencher and fluorophore are brought together in the unbound probe. Upon binding between the probe and the target sequence, the quencher and fluorophore separate, resulting in emission of signal. International publication WO/2005/079462, for example, describes a number of signaling probes that may be used in the production of the present cells and cell lines. The methods described above for introducing nucleic acids into cells may be used to introduce signaling probes.

Where tag sequences are used, each vector (where multiple vectors are used) can comprise the same or a different tag sequence. Whether the tag sequences are the same or different, the signaling probes may comprise different signal emitters, such as different colored fluorophores and the like so that expression of each subunit may be separately detected. By way of illustration, the signaling probe that specifically detects a first RNA (*e.g.*, mRNA, siRNA or RNA oligonucleotide) of interest can comprise a red fluorophore, the probe that detects a second RNA (*e.g.*, mRNA, siRNA or RNA oligonucleotide) of interest can comprise a green fluorophore, and the probe that detects a third RNA (*e.g.*, mRNA, siRNA or RNA oligonucleotide) of interest can comprise a blue fluorophore. Those of skill in the art will be aware of other means for differentially detecting the expression of the three subunits with a signaling probe in a triply transfected cell.

In one embodiment, the signaling probes are designed to be complementary to either a portion of the RNA encoding the protein of interest or to portions of the 5' or 3' untranslated regions. Even if the signaling probe designed to recognize a messenger RNA of interest is able to detect spuriously endogenously expressed target sequences, the proportion of these in comparison to the proportion of the sequence of interest produced by transfected cells is such that the sorter is able to discriminate the two cell types.

The expression level of a protein of interest may vary from cell to cell or cell line to cell line. The expression level in a cell or cell line may also decrease over time due to epigenetic events such as DNA methylation and gene silencing and loss of transgene copies. These variations can be attributed to a variety of factors, for example, the copy number of the transgene taken up by the cell, the site of genomic integration of the transgene, and the integrity of the transgene following genomic integration. One may use FACS or other cell sorting methods (*i.e.,* MACS) to evaluate expression levels. Additional rounds of introducing signaling probes may be used, for example, to determine if and to what extent the cells remain positive over time for any one or more of the RNAs for which they were originally isolated.

Optionally, one or more replicate sets of cultures for one or more of the growth rate groups may be prepared. In some cases, it may be advantageous to freeze a replicate set of one or more growth bins, for example, to serve as a frozen stock. However, according to the method, frozen cell stocks can be made as often as desired and at any point and at as many points during their production. Methods for freezing cell cultures are well-known to those of skill in the art. By way of example, the replicate set can be frozen at any temperature, for example, at -70° to - 80º C. In one case, cells were incubated until 70-100% confluency was reached. Next, media was aspirated and a solution of 90% FBS and 10% media was added to the plates, insulated and frozen.

The disclosure herein contemplates performing the method with any number of replicate sets using different culture conditions. That is, the method can be performed with a first plurality (set) of separate cell cultures under a first set of culture conditions and with a second set of separate cell cultures that are cultured under a second set of conditions that are different from the first conditions, and so on for any desired number of sets of conditions. The methods using different sets of conditions can be performed simultaneously or sequentially or a combination of both (such as two sets simultaneously followed by two more sets, and so on).

One advantage of the method described herein for selecting a cell with consistent functional expression of a protein of interest is that cells are selected by function, not by the presence of a particular nucleic acid in the cell. Cells that comprise a nucleic acid encoding a protein of interest may not express it, or even if the protein is produced, for many reasons the protein may not be functional or have altered function compared to "native" function, *i.e.,* function in a cell in its normal context that naturally expresses the protein. By selecting cells based on function, the methods described herein make it possible to identify novel functional forms. For example, it is possible to identify multiple cells that have various degrees of function in the same assay, such as with the same test compound or with a series of compounds. The differential function provides a series of functional "profiles". Such profiles are useful, for example, to identify compounds that differentially affect different functional forms of a protein. Such compounds are useful to identify the functional form of a protein in a particular tissue or disease state, and the like.

A further advantage of the method for making cells and cell lines disclosed herein including, cells that express complex proteins or multiple proteins of interest is that the cells can be produced in significantly less time that by conventional methods. For example, depending on a number of factors including the number of cells required for the functional assay, whether growth rate binning is done and other factors, cells expressing a demonstrably functional protein may be produced in as little as 2 days, or a week but even production time of 2 weeks, 3 weeks, 1 month, 2 months, 3 months or even 6 months are significantly faster than was possible by conventional methods, even for complex or multiple proteins.

Also disclosed herein are methods of using the cells and cell lines of the invention. The cells and cell lines disclosed herein may be used in any application for which the functional protein of interest are needed. The cells and cell lines may be used, for example, in an *in vitro* cell-based assay or an *in vivo* assay where the cells are implanted in an animal (*e.g.,* a non-human mammal) to, *e.g.*, screen for modulators; produce protein for crystallography and binding studies; and investigate compound selectivity and dosing, receptor/compound binding kinetic and stability, and effects of receptor expression on cellular physiology (*e.g.*, electrophysiology, protein trafficking, protein folding, and protein regulation). The cells and cell lines disclosed herein also can be used in knock down studies to examine the roles of the protein of interest.

Cells and cell lines disclosed herein also may be used to identify soluble biologic competitors, for functional assays, bio-panning (*e.g.*, using phage display libraries), gene chip studies to assess resulting changes in gene expression, two-hybrid studies to identify protein-protein interactions, knock down of specific subunits in cell lines to assess its role, electrophysiology, study of protein trafficking, study of protein folding, study of protein regulation, production of antibodies to the protein, isolation of probes to the protein, isolation of fluorescent probes to the protein, study of the effect of the protein's expression on overall gene expression/processing, study of the effect of the protein's expression on overall protein expression and processing, and study of the effect of protein's expression on cellular structure, properties, characteristics.

The cells and cell lines disclosed herein further are useful to characterize the protein of interest (DNA, RNA or protein) including DNA, RNA or protein stoichiometry, protein folding, assembly, membrane integration or surface presentation, conformation, activity state, activation potential, response, function, and the cell based assay function, where the protein of interest comprises a multigene system, complex or pathway whether all components of these are provided by one or more target genes introduced into cells or by any combination of introduced and endogenously expressed sequences.

Cells and cell lines that have been engineered to express one or more subunits of a multimeric (dimeric, trimeric or higher orders of multimerization) protein can produce different forms of this multimeric protein. The disclosure herein provides methods to distinguish cells with different forms of a multimeric protein. The functional form of a multimeric protein can vary depending on the physiological state of the cell, alternative splicing or post-translational modification of a target including proteolysis, its association with accessory or interacting factors or its folding, assembly or integration in cell membranes. Different subunit assemblies and stoichiometries further increase the number of possible functional forms for heteromultimeric targets. Functional forms can differ with respect to their responses to test compounds or the kinetics of their activity over time. Comparative analysis of cells expressing different functional forms of a multimeric protein allows cells comprising specific functional forms to be identified.

Optionally, a multimeric protein is a physical or biochemical association of at least two protein subunits. Optionally, a multimeric protein is a physical or biochemical association of one protein subunit and an accessory factor of the protein subunit. Optionally, a multimeric protein has at least 2, 3, 4, 5, 6, 7, 8, 9, or 10 subunits. The subunits can be the same polypeptide or different polypeptides or combinations thereof. The multimeric protein can be any multimeric protein disclosed herein.

Functional activities, or pharmacological profiles, as described herein, can be defined for cell lines expressing a target of interest by testing the effect of one or more compounds on the activity of the target in the cell line. Grouping or categorizing the clones according to these pharmacological profiles can be used to result in a panel of cell lines representing each possible form of the target. Representation of all possible functional forms may be pursued by saturating the screen, that is, by testing at least a number of cell lines such that each form as defined by its pharmacological profile is represented by at least 2, 3, 5, 10, 25, 50, or at least 100 cell lines.

Optionally, the effect of a compound on a target of interest is assayed at a particular point of the cell cycle (*e.g.*, M, S, $G_1$, or $G_2$ phase). Further optionally, the effect of a compound on a target of interest is monitored over time (*e.g.,* over 1, 5, 10, 15, 20, 30, 40, 50 seconds; over 1, 5, 10, 15, 20, 30, 40, 50 minutes; over 1, 5, 10, 15, 20 hours, 1, 2, 5, 10, 20, 30 days, or over 1, 2, 5, 10 months).

Optionally, for a heteromultimeric protein, a plurality of cell lines each comprising all subunits can be used to generate a plurality of pharmacological profiles of the heteromeric protein of interest, *i.e.,* one profile per cell line. Differences in the pharmacological profiles between the cell lines distinguish different forms of the heteromeric protein of interest, *e.g.*, variable assemblies or stoichiometries of the subunits.

Comparisons to cells that express different or fewer than all subunits can be used to ascribe functionality to individual subunits.

Examples of heteromultimeric proteins wherein different subunit combinations may be obtained and functionally tested include, but are not limited to:

GABA(A) receptor heteromultimeric combinations (see, *e.g.*, Olsen and Sieghart, "International Union of Pharmacology. LXX. Subtypes of γ-Aminobutyric AcidA Receptors: Classification of the Basis of Subunit Composition, Pharmacology, and Function. Update", Pharmacological Reviews, 60:243-260, 2008:

GABA(A) subunits include, but are not limited to, GABRA1 (α1), GABRA2 (α2), GABRA3 (α3), GABRA4 (α4), GABRA5 (α5), GABRA6 (α6), GABRB1 (β1), GABRB2 (β2), GABRB3(β3), GABRG1 (γ1), GABRG2 (γ2), GABRG3 (γ3), GABRD (δ), GABRE (ε), GABRP (π), and GABRQ (θ);

GABA(A) subunit combinations include, but are not limited to, α1β2γ2, α2βγ2, α3βγ2, α4βγ2, α4β2δ, α4β3δ, α5βγ2, α6βγ2, α6β2δ, α6β3δ, p, α1β3γ2, α1β δ, α5β3γ2, αβ1γ, αβ1δ, αβ, α1α6βγ, α1α6βδ, ρ1, ρ2, ρ3, αβγ1, αβγ3, αβθ, αβε, and αβπ;

nAChR heteromultimeric receptor combinations (see, *e.g.,* Gotti, Zoli and Clementi, "Brain nicotinic acetylcholine receptors: native subtypes and their relevance", Trends in Pharmacological Sciences, 27:482-491, 2006, and N. Millar Neuropharmacology Volume 56, Issue 1, January 2009, Pages 237-246):

nAChR subunits include, but are not limited to, *CHRNA1* (α1), *CHRNA2 (α2), CHRNA3* (α3), *CHRNA4 (α4), CHRNA5* (α5), *CHRNA6 (α6), CHRNA7(α7), CNRNA8(α8), CHRNA9(α9)" CHRNA10(α10)" CHRNB1 (β1), CHRNB2* (β2), *CHRNB3(β3), CHRNB4(β4), CHRND* (δ), and *CHRNE* (ε);

nAChR subunit combinations include, but are not limited to, α1β1γδ, α1β1δ, α1β1δε, α2α4β2, α2α5β2, α2β2, α2β4, α2α6β2, α3α4β2, α3α4α6β2, α3α5β2, α3α4β4, α3α5β2β4, α3α5β4, α3α6β2, α3β2, α3β3, α3β2β3, α3β2β4, α3β3β4, α3β4, α4α5β2, α4α6β2β3, α4β2, α4β4, α6β2, α6β2β3, α6α4β2, α6β2β3, α6β3β4, α6β4, α7, α7α8, α8, and α9α10;

5-$HT_3$ heteromeric receptors (see, *e.g.,* N.M. Barnes et al., Neuropharmacology 56 (2009) 273-284):

5-$HT_3$ subunits include, but are not limited to, HTR3A (A), HTR3B (B), HTR3C (C), HTR3D (D), and HTR3E (E);

5-$HT_3$ subunit combinations include, but are not limited to, AB, AC, AD, and AE;

Glycine heteromeric receptors (see, *e.g.*, JW Lynch Neuropharmacology 56 (2009) 303-309):

Glycine receptor subunits include, but are not limited to, GLRA1 (α1), GLRA2 (α2), GLRA3 (α3), GLRA4 (α4), and GLRB (β);

Glycine receptor subunit combinations include, but are not limited to, α1α3, α1β, α2β, α3β, and α4β;

Glutamate heteromeric receptors (see, *e.g.*, Perrais, Neuropharmacology 56 (2009) 131-140; Jane, Neuropharmacology 56 (2009) 90-113; and W. Lu, Neuron, 62, (2009) 2, 254-268):

Glutamate receptor subuntis include, but are not limited to, GRIK1 (K1), GRIK2 (K2), GRIK3 (K3), GRIK5 (K5), GRIA1 (A1) GRIA2 (A2), and GRIA3 (A3);

Glutamate receptor subunit combinations include, but are not limited to, K2K3, K1 K2, K1 K5, K2K5, A1A2, A1A3, and A2A3;

ATP-gated P2X heteromeric receptors (see, *e.g.,* M.F. Jarvis, B.S. Khakh, Neuropharmacology 56 (2009) 208-215; and S Robertson, Current Opinion in Neurobiology 11, 2001, 378-386:

ATP-gated P2X receptor subunits include, but are not limited to, P2RX1 (X1), P2RX2 (X2), P2RX3 (X3), P2RX4 (X4), P2RX5 (X5), P2RX6 (X6), and P2RX7 (X7);

ATP-gated P2X receptor subunit combinations include, but are not limited to, X1/X2, X1/X4, X1/X5, X2/X3, X2/X6, X4/X6, and X4/X7;

Taste receptors:

Taste receptor subunits include, but are not limited to, TAS1 R1 (T1R1), TAS1 R2 (T1 R2), TAS1 R3 (T1 R3);

Taste receptor subunit combinations include, but are not limited to, T1R2/T1R3 (sweet receptor), T1R1/T1R3 (umami receptor);

GPCR heteromultimers, *e.g.,* GPCR heterodimers (see, *e.g.,* Prinster, Hague and Hall, "Heterodimerization of G Protein-Coupled Receptors : Specificity and Functional Significance", Pharmacological Reviews, 57:289-298, 2005), including, but not limited to: HTR1 B (5-HT1B) / HTR1 D (5HT1 D), ADORA1 (Adenosine A1)/ DRD1 (Dopamine D1), ADORA1 (Adenosine A1)/ P2RY1 (P2Y1), ADORA1 (Adenosine A1)/ GRM1 (mGluR1 {alpha}), ADORA2a( Adenosine A2A)/ DRD2 (Dopamine D2), ADORA2a(Adenosine A2A)/ GRM5 (mGluR5), AGTR1 (Angiotensin 1)/AGTR2 (Angiotensin 2), AGTR1 (Angiotensin 1)/ADRB2({beta}2AR), AGTR1 (Angiotensin 1)/ BDKRB2 (Bradykinin B2), CASR (Calcium sensing receptor) / GRM1 (mGluR1), CASR (Calcium sensing receptor) / GRM5(mGluR5), CCR2 /CXCR4, CCR2 / CCR5, CCR5/OPRD1 (opioid receptor delta), CCR5/OPRK1 (opioid receptor kappa), CCR5/OPRM1 (opioid receptor mu), CCKRA (Cholecystokinin A)/ CCKRB (Cholecystokinin B), DRD1 (Dopamine D1) / DRD2(Dopamine D2), DRD2(Dopamine D2) /SSTR5, DRD2(Dopamine D2) / DRD3 Dopamine D3, EDNRA (Endothelin A)/ EDNRB (Endothelin B), GABABR1 /GABABR2, MTNR1A (Melatonin MT1)/ MTNR1B (Melatonin MT2), CHRM2 (Muscarinic M2)/ CHRM3 (Muscarinic M3), OXTR(Oxytocin)/AVPR1A (Vasopressin V1a), OXTR(Oxytocin)/AVPR2(Vasopressin V2), S1 PR1 (S1 P1)/ S1 PR2 (S1 P2), S1 PR1 (S1 P1)/ S1 PR3 (S1 P3), SSTR1 /SSTR5, SSTR2A /SSTR3, SSTR2A /OPRM1 ($\mu$-OPR), TACR1 (Substance P )/OPRM1 ($\mu$-OPR ), TRHR1 /TRHR2, AVPR1A (Vasopressin V1a)/ AVPR2(Vasopressin V2), ADRA1B(alpha1BAR)/ADRA1A (alpha1AAR), ADRA1 B(alpha1 BAR)/HRH1 (Histamine H1), ADRA1B(alpha1 BAR)/ ADRA1 D(alpha1 DAR), ADRA1 D(alpha1 DAR)/ ADRB2D(beta2AR), ADRA2A(alpha2AAR)/ADRB1(beta 1AR), ADRA2A(alpha2AAR)/OPRM1 ($\mu$-OPR), ADRB1 (beta 1AR) /ADRB2(beta2AR), ADRB2(beta2AR)/OPRD1 ($\delta$-OPR ), ADRB2(beta2AR)/ OPRK1 ($\kappa$-OPR ), ADRB2(beta2AR)/ADRB3(beta3AR), ADRB2(beta2AR)/ M71-OR, OPRK1 ($\kappa$-OPR )/ OPRD1 ($\delta$-OP)R, and OPRM1 ($\mu$-OPR )/OPRD1

($\delta$-OPR );

Voltage-gated calcium channel (CaV) multisubunit complexes (see, *e.g.,* WA Catterall et al. Pharmacol Rev 2005 57 411-425, and J Arikkath and K Campbell, Current Opinion in Neurobiology 2003,13:298-307):

CaV subunits include, but are not limited to, CACNA1 S ($\alpha_{1S}$), CACNA1 C ($\alpha_{1C}$), CACNA1D ($\alpha_{1D}$), CACNA1 F ($\alpha_{1F}$), CACNA1 A ($\alpha_{1A}$), CACNA1 B ($\alpha_{1B}$), CACNA1 E ($\alpha_{1E}$), CACNB1 ($\beta$1), CACNB2 ($\beta$2), CACNB3 ($\beta$3), CACNB4 ($\beta$4), CACNA2D1 ($\beta$2$\delta$), CACNG1 ($\gamma$1), and CACNG2 ($\gamma$2);

CaV subunit combinations include, but are not limited to, $\alpha_{1S}\beta_{1a}\alpha_2\delta\gamma_1$, $\alpha_{1S}\beta_{1a}\alpha_2\delta$, $\alpha_{1C}\beta_2\alpha_2\delta\gamma$, $\alpha_{1C}\beta_3\alpha_2\delta_\gamma$, $\alpha_{1D}\beta\alpha_2\delta$, $\alpha_{1F}\beta_{32}\delta$, $\alpha_{1F\beta 2}\alpha_2\delta$, $\alpha_{1A}\beta_3\alpha_2\delta$, $\alpha_{1A}\beta_4\alpha_2\delta$, $\alpha_{1A}\beta_3\alpha_2\delta\gamma_1$, $\alpha_{1A}\beta_3\alpha_2\delta\gamma_2$, $\alpha_{1A}\beta_4\alpha_\delta\gamma_1$, $\alpha_{1A}\beta_4\alpha_2\delta\gamma_2$, $\alpha_{1B}\beta_1\alpha_2\delta$, $\alpha_{1B}\beta_3\alpha_2\delta$, $\alpha_{1B}\beta_4\alpha_2\delta$, $\alpha_{1B}\beta_3\alpha_2\delta\gamma_2$, and $\alpha_{1E}\beta\alpha_2\delta$;

Voltage-gated sodium channel (NaV) multisubunit complexes (see, e.g., WA Catterall et al. Pharmacol. Rev., 2005 57 397-409):

NaV subunits include, but are not limited to, SCN1A ($\alpha$1), SCN2A ($\alpha$2), SCN3A($\alpha$3), SCN4A($\alpha$4), SCN5A($\alpha$5), SCN8A($\alpha$6), SCN9A($\alpha$7), SCN1 B ($\alpha$1), SCN2B ($\beta$2), SCN3B($\beta$3), and SCN4B($\beta$4);

NaV subunit combinations include, but are not limited to, $\alpha_1$ /$\beta_1$, $\alpha_1$ /$\alpha\beta_2$,$\alpha_1$ /$\beta_3$, $\alpha_1$ /$\beta_4$, $\alpha_1$ /$\beta_1$/$\beta_2$, $\alpha$1 /$\beta_1$/$\beta_3$, $\alpha$1 /$\beta_1$/$\beta$4, $\alpha$1 /$\beta_2$/$\beta_3$, $\alpha$1 /$\beta_2$/$\beta_4$, $\alpha$1 /$\beta_3$/$\beta_3$, $\alpha_2$/$\beta_1$, $\alpha_2$/$\beta_2$, $\alpha_2$/$\beta_3$, $\alpha_2$/$\beta_4$, $\alpha_2$/$\beta_1$/$\beta_2$, $\alpha_2$/$\beta_1$/$\beta_3$, $\alpha_2$ /$\beta_1$/$\beta_4$, $\alpha_2$/$\beta_2$/$\beta_3$, $\alpha_2$/$\beta_2$/$\beta_4$, $\alpha_2$/$\beta_3$/$\beta_4$, $\alpha_3$/$\beta_1$, $\alpha_3$/$\beta_3$, $\alpha_4$/$\beta_1$, $\alpha_5$/$\beta_1$, $\alpha_5$/$\beta_2$, $\alpha_5$/$\beta_3$, $\alpha_5$/$\beta_4$, $\alpha_5$ /$\beta_1$/$\beta_2$, $\alpha_5$/$\beta_1$/$\beta_3$, $\alpha_5$ /$\beta_1$/$\beta_4$, $\alpha_5$/$\beta_2$/$\beta_3$, $\alpha_5$/$\beta_2$/$\beta_4$, $\alpha_5$/$\beta_3$/$\beta_4$, $\alpha_6$/$\beta_1$, $\alpha_6$/$\beta_2$, $\alpha_6$ /$\beta_1$/$\beta_2$, $\alpha_7$ /$\beta_1$, $\alpha_7$ /$\beta_2$, and $\alpha_7$/$\beta_1$/$\beta_2$;

Inwardly Rectifying Potassium Channels - heteromeric/multisubunit complexes (see, *e.g.,* Y Kubo et al. Pharmacol. Rev., 2005 57 509-526):

Inwardly Rectifying Potassium Channel subunits include, but are not limited to, KCNJ2 ($K_{ir}$2.1) KCNJ12 ($K_{ir}$2.2), KCNJ4 ($K_{ir}$2.3), KCNJ14 ($K_{ir}$2.4), KCNJ3 ($K_{ir}$3.1), KCNJ6 ($K_{ir}$3.2), KCNJ9 ($K_{ir}$3.3), KCNJ5 ($K_{ir}$3.4), and KCNJ10 ($K_{ir}$4.1);

Inwardly Rectifying Potassium Channel subunit combinations include, but are not limited to, those listed in the table (Table 1) below :

**Table 1**

| Kir Receptor combinations | Auxiliary subunits |
|---|---|
| Kir 2.1/Kir2.2 | DLG4 (PSD-95), DLG1 (SAP97), AKAP5(AKAP79) |
| Kir 2.1/Kir2.3 | |
| Kir 2.1/Kir4.1 | |

(continued)

| Kir Receptor combinations | Auxiliary subunits |
|---|---|
| Kir2.2/ Kir2.3, | DLG1(SAP97), LIN7A(Veli1), LIN7C (Veli-3), DLG4 (PSD-95), DLG2 (Chapsyn-110), DLG3 (SAP102), CASK, MPP6 (Pals2), ABLIM (actin-binding LIM protein), SNTA1, SNTB1, SNTB2 ($\alpha$1, $\beta$1, and $\beta$2 syntrophin), DMD (dystrophin), DAG1 (Dp71), DTNA1 ($\alpha$-dystrobrevin-1) and DTNA2 ($\alpha$-dystrobrevin-2) |
| Kir2.1/Kir 2.4 | |
| Kir3.1/Kir3.2 | |
| Kir3.1/Kir3.3 | |
| Kir3.1/Kir3.4 | |
| Kir3.2/Kir3.3 | |
| Kir3.2/Kir3.4 | |
| Kir3.3/ Kir3.4 | |

Voltage-Gated Potassium Channels - heteromeric/multisubunit complexes (see, *e.g.,* G Gutman et al. Pharmacol. Rev., 2005 57 473-508):

Voltage-Gated Potassium Channel subunits include, but are not limited to, KCNA1 ($K_v$1.1), KCNA2 ($K_v$1.2), KCNA3 ($K_v$1.3), KCNA5 ($K_v$1.5), KCNA6 ($K_v$1.6), KCNA10 ($K_v$1.8), KCNB1 ($K_v$2.1) KCNB2 ($K_v$2.2), KCNC4($K_v$3.4), KCND1 ($K_v$4.1), KCND2 ($K_v$4.2), KCND3 ($K_v$4.3), KCNF1 ($K_v$5.1) KCNG1 ($K_v$6.1), KCNG2 ($K_v$6.2), KCNG3($K_v$6.3), KCNG4 ($K_v$6.4), KCNQ1 ($K_v$7.1), KCNQ2 ($K_v$7.2), KCNQ3($K_v$7.3), KCNQ4 ($K_v$7.4), KCNQ5 ($K_v$7.5), KCNV1($K_v$8.1), KCNV2 ($K_v$8.2), KCNS1 ($K_v$9.1), KCNS2 ($K_v$9.2), KCNS3 ($K_v$9.3), KCNH1 ($K_v$10.1), KCNH5 ($K_v$10.2), KCNH2 ($K_v$11.1), KCNH6 ($K_v$11.2), KCNH7 ($K_v$11.3), KCNAB1 ($K_v\beta$1), KCNAB2 ($K_v\beta$2), KCNAB3 ($K_v\beta$3) KCNE1 (minK), KCNE2 (MiRP1), KCNE3 (MiRP2), KCNE4(MiRP3), KCNE1L (KCNE1-like), KCNIP1, KCNIP2, KCNIP3, and KCNIP4 ;

Voltage-Gated Potassium Channel subunit combinations include, but are not limited to, those listed in the table (Table 2) below:

**Table 2**

| Kv Receptor combinations | Auxiliary proteins |
|---|---|
| $K_v$1.1/$K_v\beta$1 | DLG4 (PSD-95), DLG1(SAP97), SNAP25 |
| $K_v$1.1/$K_v\beta$2 | |
| Kv1.2/$K_v\beta$1 | DLG4 (PSD-95), DLG1(SAP97), SNAP25, CASPR2, RHOA |
| Kv1.2//$K_v\beta$2 | |
| Kv1.3/$K_v\beta$ | DLG1, ITGB1 ($\beta$1 integrin), PIAS3 |
| $K_v$1.4/$K_v$1.2/$K_v\beta$, | DLG4 (PSD-95), DLG1(SAP97), SAP90, ACTN2 ($\alpha$-actinin-2), PIAS3 (KChaP) |
| $K_v$1.5/$K_v\beta$1, $K_v\beta_{2,\,,}$ | CSK (Src tyrosine kinase), FYN, PIAS3 (KChaP), ACTN2 ($\alpha$-actinin-2), CAV1 (caveolin), DLG1(SAP97) |
| $K_v$1.5/$K_v\beta$2 | |
| $K_v$1.5/ KCNA3B | |
| $K_v$1.6/$K_v\beta$1, | CASPR2 |
| $K_v$1.6/$K_v\beta$2, | |
| $K_v$1.8/KCNA4B | |

(continued)

| Kv Receptor combinations | Auxiliary proteins |
|---|---|
| $K_v2.1/K_v5.1$ | PIAS3 (KChaP), FYN |
| $K_v2.1$/Kv6.1 | |
| $K_v2.1$/Kv6.2 | |
| $K_v2.1$/Kv6.3 | |
| $K_v2.1$/Kv8.1 | |
| $K_v2.1$/Kv9.1 | |
| $K_v2.1$/Kv9.2 | |
| $K_v2.1$/Kv9.3 | |
| $K_v2.2/K_v\beta4$ | PIAS3(KChaP) |
| $K_v2.2/K_v8.1$ | |
| $K_v2.2/K_v9$ | |
| $K_v3.4$/MiRP2 | |
| Kv4.1/$K_v4.2$ | |
| Kv4.2/Kv4.3 | KCNIP2 |
| Kv4.3/ $K_v\beta2$ | KCNIP1, KCNIP4a, DPP10 |
| KvS.1/$K_v2.2$ | |
| $K_v6.1/K_v2.2$ | |
| $K_v6.2/K_v2.1$ | |
| $K_v6.2/K_v2.2$ | |
| $K_v6.3/K_v2.1$ | |
| $K_v6.4/K_v2.1$ | |
| $K_v7.1$/minK | |
| $K_v7.1$/MiRP2 | |
| $K_v7.2$/KCNQ3 | |
| $K_v7.2$/KCNE2 | |
| $K_v7.3$/KCNQ2 | |
| $K_v7.3$/KCNQ5 | |
| $K_v7.4$/KCNQ32 | |
| $K_v7.5$/KCNQ3 | |
| $K_v8.1/K_v2.1$ | |
| $K_v8.1/K_v2.2$ | |
| $K_v8.2/K_v2.1$ | |
| $K_v8.2/K_v2.1$ | |
| $K_v9.1$ /Kv2.1 | |
| $K_v9.1$ /$K_v2.2$ | |
| $K_v9.2$ /$K_v2.1$ | |
| $K_v9.2$ /$K_v2.2$ | |
| $K_v9.3$ /$K_v2.1$ | |

(continued)

| Kv Receptor combinations | Auxiliary proteins |
|---|---|
| $K_v$9.3 /Kv2.2 | |
| $K_v$10.1/$K_v$10.2 | HK (Hyperkinetic), CALM1 (Calmodulin), Slob, EPN1 (epsin), KCR1 (potassium channel requlator) |
| $K_v$11.1/minK | |
| $K_v$11.1/MiRP1 | |
| $K_v$11.1/$K_v$11.2, and $K_v$11.3 can form heteromultimers | |
| $K_v$11.1/$K_v$11.3 | |
| $K_v$11.2/$_v$11.3 | |

Calcium-Activated Potassium Channels - heteromeric and multisubunit complexes (see, *e.g.,* A Wei et al. Pharmacol. Rev., 2005; 57:463-472):

Calcium-Activated Potassium Channel subunits include, but are not limited to, KCNMA1 ($K_{Ca}$1.1), KCNN1($K_{Ca}$2.1), KCNN2 ($K_{Ca}$2.2), KCNN3 ($K_{Ca}$2.3), KCNN4 ($K_{Ca}$3.1), KCNT1 ($K_{Ca}$4.1), KCNT2 ($K_{Ca}$4.2), and KCNU1($K_{Ca}$5.1);

Calcium-Activated Potassium Channel subunit combinations include, but are not limited to, those listed in the table (Table 3) below:

**Table 3**

| KCa Receptor combinations | Auxiliary proteins |
|---|---|
| $K_{Ca}$1.1/ KCNMB1 | KCNT1 (Slack), ADRB2 (β2-adrenergic receptor) |
| $K_{Ca}$1.1/KCNMB2 | |
| $K_{Ca}$1.1/ KCNMB3 | |
| $K_{Ca}$1.1/ KCNMB4 | |
| $K_{Ca}$1.1/BK-β | |
| $K_{Ca}$2.1 | CALM1 (Calmodulin) |
| $K_{Ca}$2.2 | CALM1 (Calmodulin), protein kinase CK2 and PPP2CA (protein phosphatase 2A) |
| $K_{Ca}$2.3 | CALM1 (Calmodulin) |
| $K_{Ca}$3.1 | CALM1 (Calmodulin) |
| $K_{Ca}$4.1 | KCNT1 (Slack) and |
| | KCNMA1 (Slo1) |
| $K_{Ca}$4.2 | DLG4 (PSD-95) |

Transient Receptor Potential channels - heteromeric/multisubunit complexes (see, *e.g.,* DE Clapham et al. Pharmacol. Rev., 2005; 57(4): 427-450):

Transient Receptor Potential Channel subunits include, but are not limited to, TRPC1, TRPC3, TRPC4, TRPC5, TRPC6, TRPC7, TRPV-5, TRPM1, and TRPM1-S;

Transient Receptor Potential Channel subunit combinations include, but are not limited to, those listed in the table (Table 4) below:

**Table 4**

| TRP Receptor combinations | Auxiliary proteins |
|---|---|
| TRPC1/TRPC4 | CALM1 (calmodulin), ITPR, CAV1 (caveolin-1), GNAQ (Gq/11), ATP2B1 (PMCA), GRM1 (mGluR1) |
| TRPC1/TRPC5 | |
| TRPC5/TRPC3 | |
| TRPC3/TRPC6 | FKBP1A (FKBP12), VAMP2 (synaptobrevin 2), NCX1 (Sodium/calcium exchanger), NTRK2 (TrkB) |
| TRPC3/TRPC7 | |
| TRPC4/TRPC1 | |
| TRPC4/TRPC5 | |
| TRPC6/TRPC7 | CALM1 (calmodulin), FKBP1A (FKBP12), |
| | CALM1 (calmodulin) |
| TRPV5/TRPV6, | SLC9A3R1 (NHERF), S100A1 |
| TRPM1/ TRPM1-S | |
| | PKD1, HAX1, CTTN (cortactin), TPM1 (tropomyosin) |

Cyclic Nucleotide-Regulated Channels - heteromeric/multisubunit complexes (see, *e.g.,* F Hofmann et al. Pharmacol. Rev., 2005; 57(4): 455-462);

Cyclic Nucleotide-Regulated Channel subunits include, but are not limited to, CNGA, CNGB1a, CNGA2, CNGB1b, CNGA4, CNGA3, CNGB3, CNG4A, and CNGA1;

Cyclic Nucleotide-Regulated Channel subunit combinations include, but are not limited to, CNGA1/CNGB1a, CNGA2/CNGB1 b/ CNGA4, CNGA3/CNGB3, CNG4A/CNGA2/CNGB1 b, CNGB1a/CNGA1, CNGB1 b/CNGA2/CNGA4, and CNGB3/CNGA3;

Epithelial Sodium Channel/Degenerin - heteromeric/multisubunit complexes (see, *e.g.,* A Staruschenko et al Biophys J, 2005; 88, 3966-3975, H Yamamura et al European J Pharm., 2008, 600, 32-36, and S Kellenberger and L Schild Physiol Rev, 2002, 82, 735-767):

Epithelial Sodium Channel/Degenerin subunits include, but are not limited to, SCNN1 A (ENaCa); SCNN1 B (β),SCNN1G (γ),SCNN1D (ENaCδ), ACCN1 (ASIC2, two splice variants 2a and 2b), ACCN3 (ASIC3);

Epithelial Sodium Channel/Degenerin subunit combinations include, but are not limited to, ENaCαβγ, ENaCαδ βγ, ENaCδβγ, ASIC2a/ASIC2b, ASIC2a/ASIC3, and ASIC2b/ASIC3.

For the heteromeric proteins listed above and those disclosed elsewhere herein, many more combinations of subunits yet to be defined may exist. Cell lines comprising the combinations listed above may be used as references against those cell lines that may express novel combinations, so that the novel combinations may be ascertained. For example, different subunit combinations may be characterized by different response profiles of different cell lines each expressing a same set of subunits to a same set of compounds. See, *e.g.,* Example 23 hereinbelow.

Large scale application of the method applied to heteromultimeric targets defined by large gene families (*e.g.,* GABA or the acetylcholine ion channels) allows cross comparative analysis of multiple cell lines for all or a subset of possible subunit combinations.

Optionally, gene activation is used with the methods, cells, and cell lines of the invention. Gene activation is described, *e.g.,* in International Application Publication WO 94/12650. Optionally, homologous recombination can be used to genetically modify a regulatory region of one or more endogenous genes in the cell that encodes for a receptor or a receptor subunit of ENaC (epithelial sodium channel), $GABA_A$ (Gamma-aminobutyric acid type A), NaV (voltage-gated sodium ion channel), a sweet taste receptor, an umami taste receptor, a bitter taste receptor, CFTR (cystic fibrosis transmembrane-conductance regulator), or GCC (guanylyl cyclase C) such that the genetic modification results in increased expression of the receptor or receptor subunit of ENaC, $GABA_A$, NaV, sweet taste receptor, umami taste receptor, bitter taste receptor, CFTR, or GCC relative to the expression levels of the receptor or receptor subunit in the cell before the genetic modification. In certain embodiments, the genetic modification results in an increase of expression by at least 10%, 50%, 100%, 500%, 1000%, 5000%, or at least 10,000%. Optionally, the cell expresses the receptor or receptor subunit at background levels before gene activation.

Optionally, a promoter is introduced via homologous recombination to be operatively linked with the coding sequence that is endogenous to the cell for a receptor or one or more receptor subunits of ENaC, $GABA_A$, NaV, a sweet taste receptor, an umami taste receptor, a bitter taste receptor, CFTR, or GCC. The promoter can be a constitutively active promoter. In particular, the promoter can be a promoter that is constitutively active in the cell. Further optionally, the promoter is a conditional promoter. Cells that can be used with these methods are described above. Optionally, promotors can be randomly inserted into the genome of the cell. Fluorogenic oligonucleotides can then be used to select a cell in which the randomly inserted promoter activates the expression of an RNA of interest. Optionally, the RNA of interest may be for a receptor or one or more subunits of a receptor of ENaC, $GABA_A$, NaV, a sweet taste receptor, an umami taste receptor, a bitter taste receptor, CFTR, or GCC. Optionally, regulatory DNA elements, such as enhancers or repressors, are inserted at random positions into the genome and a cell in which an RNA of interest is upregulated or downregulated is selected.

Optionally, homologous recombination is used to introduce DNA into the genome of a cell such that the endogenous gene is expressed. Optionally, the endogenous gene may be a gene encoding a receptor or one or more receptor subunits of ENaC, $GABA_A$, NaV, a sweet taste receptor, an umami taste receptor, a bitter taste receptor, CFTR, or GCC. Optionally the introduced DNA includes a selectable marker, such as a gene encoding for antibiotic resistance (*e.g.,* DHFR). Optionally, the endogenous gene is amplified in the genome of the cell. Optionally, the endogenous gene is amplified such that the genome of the cell comprises 2 copies, 3 copies, 4 copies, 5 copies, 6 copies, 7 copies, 8 copies, 9 copies, 10 copies, at least 2 copies, at least 5 copies, at least 10 copies, at least 15 copies, at least 20 copies, or at least 25 copies of the endogenous gene. Optionally, a cell with stable functional expression of the amplified gene is selected.

Regulatory sequences can also be modified and expression achieved by the genetic variation methods described below. Genetic variability may be generated in a cell line and subsequently a cell that expresses a receptor or one or more subunits of a receptor of ENaC, $GABA_A$, NaV, a sweet taste receptor, an umami taste receptor, a bitter taste receptor, CFTR, or GCC is selected using a fluorogenic oligonucleotide as described herein.

In certain embodiments, a cell expresses a receptor or one or more receptor subunits of ENaC, $GABA_A$, NaV, a sweet taste receptor, an umami taste receptor, a bitter taste receptor, CFTR, or GCC before application of the gene activation or generation of genetic variability. Gene activation and / or generation of genetic variability and selection is then used to generate and select a cell that expresses another receptor or at least one additional subunit of the same receptor and / or that has a reduced level of expression of the receptor or of one or more receptor subunits that was expressed before application of the gene activation or generation of genetic variability.

In certain embodiments, an accessory factor is also expressed in the cell. The accessory factor can be expressed in the cell without genetic modification; the accessory factor can be expressed as a transgene; or the accessory factor can be expressed via gene activation and / or generation of genetic variability and selection as described above. The accessory factor can be a factor that facilitates the transcription and / or translation and / or folding and / or subcellular localization and / or function of a receptor or subunit of a receptor of interest. The accessory factor can facilitate the assembly of a multi-subunit receptor.

In certain embodiments, provided herein is a cell line that expresses a complete receptor, or that expresses one, two, three, four, or five subunits of a multisubunit receptor of interest. The multisubunit receptor of interest may be, *e.g.,* ENaC, $GABA_A$, NaV, a sweet taste receptor, an umami taste receptor, or a bitter taste receptor. In certain embodiments, neither the receptor nor any receptor subunits of a multisubunit receptor of interest is expressed from a transgene. In certain embodiments, one, two, three, four, or five subunits of the multisubunit receptor of interest is / are not expressed from a transgene. In certain embodiments, a complete receptor, or one, two, three, four, or five subunits of a multisubunit receptor of interest is / are expressed from amplified genomic region of the endogenous gene encoding the receptor or the receptor subunit(s).

Optionally, genomic DNA is transfected or microinjected into a population of cells and a cell that expresses an RNA of interest is selected. Optionally, the RNA of interest may be for a receptor or one or more subunits of a receptor of ENaC, $GABA_A$, NaV, a sweet taste receptor, an umami taste receptor, a bitter taste receptor, CFTR, or GCC. Genomic DNA may be obtained from a cell that expresses the RNA of interest. Optionally, the donor cell of genomic DNA is of the same species as the acceptor cell. Optionally, the donor cell is a different species than the acceptor cell.

Optionally, the donor cell of genomic DNA can be a human, mouse, insect, dog, donkey, horse, rat, guinea pig, avian or monkey cell. Optionally, genomic DNA from 2, 3, 4, 5, 6, 7, 8, 9, or 10 different species is introduced. Optionally, the different donor species of genomic DNA have orthologs of the gene of interest. Optionally, the donor species do not have orthologs of the gene of interest.

Optionally, the genomic DNA fragment is a defined region of the genome and includes the gene of interest. Optionally, the genomic DNA fragment is at least 1 kb, 5 kb, 10 kb, 100 kb, 500 kb, or 1000 kb in size.

Any method known to the skilled artisan can be used to extract genomic DNA from a cell. An illustrative method for isolating genomic DNA is described in Unit 2.2. of Short Protocols in Molecular Biology, Ausubel et al. (editors), John Wiley & Sons, Inc., 1999. Optionally, the genomic DNA is treated very gently to avoid shearing of the DNA. Further optionally, the genomic DNA is sheared to obtain smaller DNA fragments. Optionally, the DNA is treated with DNAse-free protease to remove any proteinaceous substances from the DNA. Further optionally, the genomic DNA is not treated with protease, and instead care is taken to leave undisturbed the proteins associated with the genomic DNA. Optionally, the DNA is treated with DNAse free RNAse.

The genomic DNA can be introduced into cells using any method known to the skilled artisan. Optionally, the genomic DNA is transfected into a cell. Optionally, the genomic DNA is transfected into the cells using lipofection. Illustrative methods for introducing the genomic DNA into cells are described in Chapter 9 of Short Protocols in Molecular Biology, Ausubel et al. (editors), John Wiley & Sons, Inc., 1999.

The optimal amount of genomic DNA to be introduced into a cell can be determined by identifying the number of cells expressing the RNA of interest. Optionally, the amount of genomic DNA introduced per cell corresponds to at least the equivalent of 1 genome, at least the equivalent of $10^{-1}$ genome, at least the equivalent of $10^{-2}$ genome, at least the equivalent of $10^{-3}$ genome, at least the equivalent of $10^{-4}$ genome, at least the equivalent of $10^{-5}$ genome, at least the equivalent of $10^{-6}$ genome, or at least the equivalent of $10^{-7}$ genome. Optionally, the amount of genomic DNA introduced per cell corresponds to at most the equivalent of 1 genome, at most the equivalent of $10^{-1}$ genome, at most the equivalent of $10^{-2}$ genome, at most the equivalent of $10^{-3}$ genome, at most the equivalent of $10^{-4}$ genome, at most the equivalent of $10^{-5}$ genome, at most the equivalent of $10^{-6}$ genome, or at most the equivalent of $10^{-7}$ genome.

Optionally, the genomic DNA can be amplified by any technique known to the skilled artisan. Optionally, the genomic DNA is amplified by Whole Genome Amplification.

Any method can be used to identify and isolate those cells in which genomic DNA has been introduced. Optionally, DNA that encodes a marker gene is introduced concurrently with the genomic DNA into cells. Cells that are positive for the marker gene also harbor the genomic DNA. Any marker gene known to the skilled artisan can be used. Illustrative examples of marker genes include genes whose gene products confer resistance to a particular antibiotic to the cells (e.g., neomycin resistance), genes whose gene products enable a cell to grow on a medium that lacks a substance that is normally required by this cell for growth, or genes whose gene products encode a visual marker. A visual marker that can be used with the methods of the invention is, *e.g.,* GFP. Cells in which the DNA encoding the visual marker and the genomic DNA have been introduced can be isolated using FACS.

Optionally, the genomic DNA is introduced into cells using microinjection.

Optionally, fragments of the genomic DNA are packaged into vectors for propagation of the genomic DNA. Such vectors include, but are not limited to, bacteriophages, cosmids or YACs. Any method known to the skilled artisan can be used to package and propagate the genomic DNA.

Also disclosed herein is a cell that expresses a multisubunit receptor of interest with the same stoichiometry of subunits as the stoichiometry of subunits of the multisubunit receptor of interest in a non-recombinant organism, wherein the cell from which the cell line was derived does not express the multisubunit receptor of interest. Optionally, provided herein is a cell line that expresses a receptor, including a cell line that expresses a multisubunit receptor, wherein the pharmacological profile of the receptor in the cell line matches the pharmacological profile of the receptor in a cell that normally expresses the target in an organism. The receptor or multisubunit receptor of interest can be, *e.g.,* ENaC, $GABA_A$, NaV, a sweet taste receptor, an umami taste receptor, a bitter taste receptor, CFTR, or GCC. Optionally, the cell line expresses the receptor or multisubunit receptor stably in the absence of selective pressure as described hereinabove.

In certain aspects, the invention provides for methods for identifying, isolating, and enriching cells that are naturally occurring and express a receptor or one or more receptor subunits of ENaC, $GABA_A$, NaV, a sweet taste receptor, an umami taste receptor, or a bitter taste receptor, CFTR, or GCC. In some embodiments, the naturally occuring cell expresses a receptor or one or more receptor subunits of ENaC, $GABA_A$, NaV, a sweet taste receptor, an umami taste receptor, or a bitter taste receptor, CFTR, or GCC in nature.

In certain embodiments, the methods described herein rely on the genetic variability and diversity that exists in nature. In certain embodiments, the isolated cell is represented by not more than 1 in 10, 1 in 100, 1 in 1000, 1 in 10,000, 1 in 100,000, 1 in 1,000,000 or 1 in 10,000,000 cells in a population of cells. The population of cells can be primary cells harvested from organisms. In certain embodiments, genetic variability and diversity may also be increased using natural processes known to a person skilled in the art. Any suitable methods for creating or increasing genetic variability and/or diversity may be performed on host cells. Without being limited by theory, such generation of genetic variability generates modifications in regulatory regions of a gene encoding, for example, a receptor or a receptor subunit of ENaC, $GABA_A$, NaV, a sweet taste receptor, an umami taste receptor, or a bitter taste receptor, CFTR, or GCC. Cells expressing such a subunit can then be selected as described herein. In certain embodiments, a cell that endogenously expresses the protein of interest is isolated from a clonal cell line that has been subjected to at least 50, 100, 500, 750, or at least 1000 passages; or that has been subjected to at least 1, 2, 5, or 10 months, or at least 1, 2, 5, 10 years of continuous growth.

In other embodiments, genetic variability may be achieved by exposing a cell to UV light and / or x-rays (*e.g.,* gamma-rays). Genetic variability can also be achieved in a population of cells by introducing genomic DNA, cDNA, and / or mRNA into the cells of the population of cells. In particular embodiments, fragments of genomic DNA is introduced as discussed above. In particular embodiments, a library of genomic DNA is introduced. In particular embodiments, a cDNA library is introduced. In particular embodiments, an expression DNA library is introduced. In certain embodiments, the genomic DNA, cDNA, and / or mRNA is derived from a cell type different from the cell type of the recipient cells. In a particular embodiment, the genomic DNA, cDNA, and / or mRNA is derived from a taste cell.

In other embodiments, genetic variability may be achieved by exposing cells to EMS (ethyl methane sultonate). In some embodiments, genetic variability may be achieved by exposing cells to mutagens, carcinogens, or chemical agents. Non-limiting examples of such agents include deaminating agents such as nitrous acid, intercalating agents, and alkylating agents. Other non-limiting examples of such agents include bromine, sodium azide, and benzene.

In specific embodiments, genetic variability may be achieved by exposing cells to growth conditions that are sub-optimal, *e.g.,* low oxygen, low nutrients, oxidative stress or low nitrogen.

In certain embodiments, enzymes that result in DNA damage or that decrease the fidelity of DNA replication or repair (*e.g.* mismatch repair) can be used to increase genetic variability. In certain embodiments, an inhibitor of an enzyme involved in DNA repair is used. In certain embodiments, a compound that reduces the fidelity of an enzyme involved in DNA replication is used. In certain embodiments, proteins that result in DNA damage and / or decrease the fidelity of DNA replication or repair are introduced into cells (co-expressed, injected, transfected, electroporated).

The duration of exposure to certain conditions or agents depend on the conditions or agents used. In some embodiments, seconds or minutes of exposure is sufficient. In other embodiments, exposure for a period of hours, days or months are necessary. The skilled artisan will be aware what duration and intensity of the condition can be used.

Without being bound by theory, a method that increases genetic variability produces a mutation or alteration in a promoter region of a gene that leads to a change in the transcriptional regulation of the gene, *e.g.,* gene activation, wherein the gene is more highly expressed than a gene with an unaltered promoter region. In certain embodiments, the gene encodes a receptor or a receptor subunit of ENaC, GABAA, NaV, a sweet taste receptor, an umami taste receptor, or a bitter taste receptor, CFTR, or GCC. Generally, a promoter region includes a genomic DNA sequence upstream of a transcription start site that regulates gene transcription, and may include the minimal promoters and/or enhancers and/or repressor regions. A promoter region may range from about 20 basepairs (bps) to about 10,000 bps or more. In specific embodiments, a method that increases gene variability produces a mutation or alteration in an intron of a gene of interest that leads to a change in the transcriptional regulation of the gene, *e.g.,* gene activation wherein the gene is more highly expressed than gene with an unaltered intron. In certain embodiments, untranscribed genomic DNA is modified. For example, promoter, enhancer, modifier, or repressor regions can be added, deleted, or modified. In these cases, transcription of a transcript that is under control of the modified regulatory region can be used as a read-out. For example, if a repressor is deleted, the transcript of the gene that is repressed by the repressor is tested for increased transcription levels.

In certain embodiments, the genome of a cell or an organism can be mutated by site-specific mutagenesis or homologous recombination. In certain embodiments, oligonucleotide- or triplex-mediated recombination can be employed. See, *e.g.,* Faruqi et al., 2000, Molecular and Cellular Biology 20:990-1000 and Schleifman et al., 2008, Methods Molecular Biology 435:175-90.

In certain embodiments, fluorogenic oligonucleotide probes or molecular beacons can be used to select cells in which the genetic modification was successful, *i.e.,* cells in which the transgene or the gene of interest is expressed. To identify cells in which a mutagenic or homologous recombination event was successful, a fluorogenic oligonucleotide that specifically hybridizes to the mutagenized or recombined transcript can be used. In certain embodiments, where a cell is selected that endogenously expresses a protein of interest and the cell is of a cell type that does not express the protein of interest, a fluorogenic oligonucleotide can be used that specifically hybridizes to the RNA encoding the protein of interest to isolate cells that express the desired protein. The isolation of cells can be performed as described in U.S. Patent No. 6,692,965 by Shekdar et al. issued February 17, 2004 and International Application No. PCT/US2005/005080 published as WO/2005/079462). In certain embodiments, cells positive for the desired signal (*i.e.,* cells that express the desired RNA) are pooled. Such a pool can then be subjected to a second round of selection. In certain embodiments, the pool of cells is subjected to a total of at least 2, 3, 4, 5, 6, 7, 8, 9, 10, 15, 20, 25, or at least 50 rounds of selection.

Cells and cell lines engineered to express a multimeric protein may comprise multiple different functional forms of the multimeric protein. The same cell or cell line engineered to comprise a multimeric protein complex could comprise different functional forms or different proportions of the same or different functional forms when it is cultured under different cell culture conditions. Culture conditions may vary with respect to temperature, nutrient concentration, serum addition, ion concentration, cell density, synchronization of cell cycle, pH, acidity, carbonation, atmospheric conditions, percent carbon dioxide, shaking, stirring, UV exposure, activity or concentration of metabolites, serum, amino acids, sugars, carbohydrates, proteins, lipids, detergents, growth factors, co-factors, vitamins, mutagens, chemicals, compounds or trace metals. Different cells or cell lines engineered to comprise the same target may comprise different functional forms or different proportions of the same or different functional forms. The methods described may be used for comparative analysis of cells or cell lines engineered to comprise the same or different multimeric protein cultured in the same or different cell culture conditions.

Optionally, the cells or cell lines to be used with the methods for characterizing a multimeric protein and for making panels of cells or cell lines expressing different forms of a multimeric protein of interest are cells or cell lines as described herein, *e.g.,* cells or cell lines with a substantially constant physiological property, cells or cell lines that express a protein of interest which does not comprise a protein tag, or cells or cell lines having a Z' factor of at least 0.4 in a functional assay, or cells or cell lines cultured in the absence of selective pressure, or any combinations thereof. In some embodiments, the different cell lines to be used are maintained in parallel under substantially identical culture conditions. Robotic methods described herein may be used to maintain and manipulate the cells or cell lines expressing different forms of a multimeric protein of interest. Optionally, the invention provides a cell or cell line that expresses a multimeric (dimeric, trimeric or higher order of multimerization) protein of interest from an introduced nucleic acid encoding said multimeric protein of interest or one or more subunits thereof, wherein the cell is cultured in the absence of selective pressure and/or wherein the protein is expressed consistently as described herein.

Optionally, the invention provides methods for generating a panel of cell lines comprising a plurality of cell lines wherein each of the plurality of cell lines synthesizes a different form of a multimeric protein of interest. Examples of multimeric proteins of interest are further disclosed hereinabove. Without being bound by theory, the different forms of the multimeric protein differ, *e.g.,* in their subunit combination, post-translational modification of individual subunits, and/or subcellular localization (*e.g.,* with respect to the cytoskeleton; for transmembrane proteins: membrane integration/localization, exit out of the endoplasmic reticulum, exit out of the Golgi apparatus; and for secreted proteins: exit out of the endoplasmic reticulum, exit out of the Golgi apparatus). Optionally, such panels can be generated by starting with a specific clone or line of a host cell and engineering that host cell to express one or more subunits of a multimeric protein of interest. Optionally, transgenes encoding the one or more subunits are introduced. In other embodiments, gene activation is employed. Cells that have been successfully engineered to express the one or more subunit can be selected by any method known to the skilled artisan. Optionally, the positive cells are selected using fluorogenic oligonucleotides or molecular beacons (see, *e.g.,* International Application No. PCT/US2005/005080 published as WO/2005/079462). Cell lines are established from the identified cells. Optionally, the resulting cell lines are generated from the same materials (*e.g.,* the same host parental clone, the same nucleic acids for engineering the expression of the one or more subunits) and the same protocols (*e.g.,* the same cell culturing methods, the same genetic engineering methods). Without being bound by theory, the resulting cell lines may vary with regard to the insertion sites of any transgenes in the genome of the host cell and/or with regard to the copy number of any transgenes in the genome. Surprisingly, it was found that the resulting cell lines may synthesize different forms of a multimeric protein. Cell lines that synthesize different forms of a multimeric protein may be identified by establishing a pharmacological profile of each cell line. Certain compounds are tested for their effects on the multimeric protein in the various cell lines. Activities may be monitored over time. Dose-response curves can be established. Optionally, at least 2, 5,10, 25, 50,100, 250, 500, 750, 1000, 1500, 2000, 2500, 3000, 4000, 5000, 7500, or at least 10000 different compounds are tested per cell line to generate a pharmacological fingerprint for the multimeric protein synthesized by a particular cell line. The resulting pharmacological fingerprints for each cell line are then compared with each other. If the pharmacological fingerprints are substantially the same, the form of the multimeric protein in the cell lines are the same. If the pharmacological fingerprints are not substantially the same, the forms of the multimeric protein in these cell lines are different.

Optionally, the forms are the same if the quality of the response of the multimeric protein is the same for each compound (*e.g.,* activated, inhibited, or neutral).

Optionally, the forms are the same if the quantity of the response is the same for each compound within a margin of 50%, 25%, 10%, 5%, 1%, or 0.5%.

Optionally, the forms are different if the quality of the response of the multimeric protein is different for at least 1 compound (*e.g.,* activated, inhibited, or neutral). Optionally, the forms are different if the quality of the response of the multimeric protein is different for at least 50%, 25%, 10%, 5%, 1%, or 0.5% of the compounds tested (*e.g.,* activated, inhibited, or neutral). Optionally, the forms are different if the quantity of the response for at least 1 compound is not within a margin of at least 50%, 25%, 10%, 5%, 1 %, or 0.5%. Optionally, the forms are different if the quantity of the response for at least 50%, 25%, 10%, 5%, 1%, or 0.5% of the tested compounds is not within a margin of at least 50%, 25%, 10%, 5%, 1%, or 0.5%. Any algorithm known to the skilled artisan can be used to quantify and compare the pharmacological fingerprints of the multimeric proteins in the different cell lines.

Optionally, the composition of a biologically active multimeric protein of interest in a cell that co-expresses a first subunit and a second subunit can be classified by comparing a first subunit pharmacological profile, a second subunit pharmacological profile, and a mixed subunit pharmacological profile. The first subunit pharmacological profile can comprise measured amounts representing an effect of a compound on the biological activity of the multimeric protein as it would be expressed in a cell that expresses the first subunit of the multimeric protein and not the second subunit. The second subunit pharmacological profile can comprise measured amounts representing an effect of the compound on the biological activity of the multimeric protein as it would be expressed in a cell that expresses the second subunit of the multimeric protein and not the first subunit. The mixed subunit pharmacological profile can comprise measured amounts representing an effect of the compound on the biological activity of the multimeric protein is it would be expressed in a cell that expresses both the first subunit and the second subunit of the multimeric protein. Optionally, the biologically active multimeric protein of interest can be classified as (i) a homodimer of the first subunit, if the first subunit pharmacological profile has a high similarity to the mixed subunit pharmacological profile, and the second subunit pharmacological profile has a low similarity to the mixed subunit pharmacological profile; (ii) a homodimer of the second subunit, if the second subunit pharmacological profile has a high similarity to the mixed subunit pharmacological profile, and the first subunit pharmacological profile has a low similarity to the mixed subunit pharmacological profile; (iii) a heterodimer of the first subunit and the second subunit, if the first subunit pharmacological profile has a low similarity to the mixed subunit pharmacological profile, and the second subunit pharmacological profile has a low similarity to the mixed subunit pharmacological profile; or (iv) a combination of homodimers of the first subunit and homodimers of the second subunit, if the mixed subunit pharmacological profile is a combination of the first subunit pharmacological profile and the second subunit pharmacological profile.

Further optionally, the composition of a biologically active multimeric protein of interest in a cell that co-expresses a first subunit and a second subunit can be classified by comparing a pharmacological profile of the cell of interest with the first subunit pharmacological profile, the second subunit pharmacological profile, and the mixed subunit pharmacological profile. Optionally, the biologically active multimeric protein of interest can be classified as (i) a homodimer of the first subunit, if said first pharmacological profile has a low similarity to said second subunit pharmacological profile and a high similarity to both said first subunit pharmacological profile and said mixed subunit pharmacological profile; (ii) a homodimer of the second subunit, if said first pharmacological profile has a low similarity to said first subunit pharmacological profile and a high similarity to both said second subunit pharmacological profile and said mixed subunit pharmacological profile; (iii) a heterodimer of the first subunit and the second subunit, if said first pharmacological profile has a low similarity to said first subunit pharmacological profile, a low similarity to said second subunit pharmacological profile, and a low similarity to said mixed subunit pharmacological profile; or (iv) a combination of homodimers of the first subunit and homodimers of the second subunit, if said first pharmacological profile has a high similarity to said mixed subunit pharmacological profile, and said first pharmacological profile is a combination of said first subunit pharmacological profile and said second subunit pharmacological profile.

Optionally, an effect of a subunit on the biological activity of a multimeric protein in a cell can be characterized by comparing a pharmacological profile from a cell that expresses the subunit of interest to a pharmacological profile from a cell that does not express the subunit of interest. Optionally, a test pharmacological profile can be compared to a base pharmacological profile to characterize that effect. In the foregoing case, the test pharmacological profile can comprise measured amounts representing an effect of a compound on the biologically active multimeric protein in a cell that expresses a pre-selected combination of subunits and, in addition, the subunit of interest; the base pharmacological profile can comprise measured amounts representing an effect of that compound on the biologically active multimeric protein in a cell that expresses the pre-selected combination of subunits but not the subunit of interest. The subunit of interest is not one of the subunits of the pre-selected combination of subunits. The subunit of interest can be characterized as having an effect on the biological activity of the multimeric protein if the test pharmacological profile has a low similarity to the base pharmacological profile, or as having no effect on the biological activity of the multimeric protein if the test pharmacological profile has a high similarity to the base pharmacological profile.

The pharmacological profiles can be compared by computing a correlation between the pharmacological profiles, such as but not limited to, computing a measure of similarity between the pharmacological profiles.

Optionally, two pharmacological profiles can be deemed to be correlated if the measured amounts in one of the pharmacological profiles are within about 2%, about 5%, about 8%, about 10%, about 12%, about 15%, about 20%, about 25%, about 30%, or about 35% of the measured amounts in the other pharmacological profiles.

Optionally, two pharmacological profiles can be deemed to have a high similarity to each other if a measure of similarity computed between them is above a predetermined threshold, or can be deemed to have a low similarity to each other if a measure of similarity computed between them is below a predetermined threshold. Optionally, the predetermined threshold can be determined as the value of the measure of similarity which indicates that the measured amounts in one of the pharmacological profiles are within about 2%, about 5%, about 8%, about 10%, about 12%, about 15%, about 20%, about 25%, about 30%, or about 35% of the measured amounts in the other pharmacological profile.

Optionally, the pharmacological profile of the compound of interest can be expressed as a vector *p*,

$$\boldsymbol{p} = [\,\boldsymbol{p}_1, \ldots \boldsymbol{p}_i, \ldots \boldsymbol{p}_n]$$

where $\boldsymbol{p}_i$ is the measured amount of the *i*'th component, for example, the effect of a compound on the *i*'th biological activity of a cell that expresses the subunits of interest of a multimeric protein. Optionally, *n* is more than 2, more than 10, more than 100, more than 200, more than 500, more than 1000, more than 2000, more than 2500, more than 7500, more than 10,000, more than 20,000, more than 25,000, or more than 35,000. Each pharmacological profile also can be expressed as a vector *p*. In computing a correlation, the measured amount of the *i*th component in the vector representing the pharmacological profile for the compound of interest can be compared to the corresponding measured amount of the *i*th component of the vector representing a pharmacological profile, for each component $i = 1 ... n$. However, there are many ways in which a correlation can be computed. Indeed, any statistical method in the art for determining the probability that two datasets are related may be used in accordance with the methods disclosed herein in order to identify whether there is a correlation between the pharmacological profile of a compound of interest and a pharmacological profile. For example, the correlation between the pharmacological profile ($p_{i_1}$) of the compound of interest and each pharmacological profile ($p_{i_2}$) can be computed using a similarity metric $sim(p_{i_1}, p_{i_2})$. One way to compute the similarity metric $sim(p_{i_1}, p_{i_2})$ is to compute the negative square of the Euclidean distance. In alternative embodiments, metrics other than Euclidean distance can be used to compute $sim(p_{i_1}, p_{i_2})$, such as a Manhattan distance, a Chebychev distance, an angle between vectors, a correlation distance, a standardized Euclidean distance, a Mahalanobis distance, a squared Pearson correlation coefficient, or a Minkowski distance. a Pearson correlation coefficient, a squared Euclidean distance, a Euclidean sum of squares, or squared Pearson correlation coefficients is used to determine similarity. Such metrics can be computed, for example, using SAS (Statistics Analysis Systems Institute, Cary, North Carolina) or S-Plus (Statistical Sciences, Inc., Seattle, Washington). Use of such metrics are described in Draghici, 2003, Data Analysis Tools for DNA Microarrays, Chapman & Hall, CRC Press London, chapter 11.

The correlation can also be computed based on ranks, where $x_i$ and $y_i$ are the ranks of the values of the measured amounts in ascending or descending numerical order. See for example, Conover, Practical Nonparametric Statistics, 2nd ed., Wiley, (1971). Shannon mutual information also can be used as a measure of similarity. See for example, Pierce, An Introduction To Information Theory: Symbols, Signals, and Noise, Dover, (1980).

Also disclosed herein is a computer program product that contains any or all of the program modules shown in Fig. 2. Aspects of the program modules are further described hereinbelow.

Any assay known to the skilled artisan can be used to assay the activity of a multimeric protein of interest and thereby establish a pharmacological fingerprint. Examples of such assays are further described hereinbelow. Any assays to be used with the methods of the invention can be conducted in high throughput format.

Optionally, the panels of the invention may be used in HTS or in combination with medicinal chemistry to test and identify compounds that are selective for one or a set of targets; in particular, to identify compounds whose activity is specific to particular forms of a multimeric protein of interest or having certain patterns or profiles of activity. Secondary testing including in vivo tests using the compounds could be used to determine the presence, role and function of the corresponding target or targets in vivo, or its relevance as a biomarker. (Testing methods are diverse and include brain imaging studies, animal studies, binding studies, behavioral models, PET scans, NMRIs, *etc.*)

The disclosure herein provides for characterizing the composition of a multimeric protein. Without being bound by theory, multimeric proteins characterized by functional criteria could reflect specific stoichiometries of the multimeric proteins or combinations or levels of different specific stoichiometries. Optionally, a multimeric protein is expressed in a cell that has been engineered to express the multimeric protein.

Optionally, the composition of a dimer is determined using the methods disclosed herein.

Optionally, the methods disclosed herein allow the determination whether the dimer in a particular cell is a) a homodimer of a first subunit; b) a homodimer of a second subunit; or c) a heterodimer of the first and the second subunit. For example, such a method may comprise the following steps:

Step A) The activity of the first subunit in a cell that does not express the second subunit is tested.

Step B) The activity of the second subunit in a cell that does not express the first subunit is tested.

Step C) The activity of the first subunit and the second subunit is tested in a cell that expresses both the first and the second subunits.

The activities obtained in Steps A, B, and C are compared and the composition of the dimeric protein in a cell that expresses both the first and the second subunits is deduced. If the activity determined in Step A equals the activity determined in Step C, then the dimer that is formed in a cell that co-expresses the first and the second subunits is a homodimer of the first subunit. If the activity determined in Step B equals the activity determined in Step C, then the dimer that is formed in a cell that co-expresses the first and the second subunit is a homodimer of the second subunit. If the activity determined in Step A and the activity determined in Step B are both different from the activity determined in Step C, then the dimer that is formed in a cell that co-expresses the first and the second subunits is a heterodimer of the first and the second subunits. If the activity determined in Step C is a combination of activities observed in Step A and Step B, then the cell that co-expresses the first and the second subunits produces homodimers of the first subunit and homodimers of the second subunit.

Optionally, the activity profile of a compound over time is measured.

Similar steps can be taken to determine the subunit combination of trimers and other multimeric proteins with higher orders of multimerization. Also disclosed herein is a panel of cell lines comprising a plurality of cell lines wherein each cell line of the plurality of cell lines has been engineered to express the same subunit or subunits of a multimeric protein using the same protocol and the same host cells, wherein the resulting multimeric proteins differ among the cell lines. The differences between the multimeric proteins can be: different subunit combinations, different subunit stoichiometries, different post-translational modifications including proteolysis, and/or different splicing of one or more subunits.

Without being bound by theory, the differences in the multimeric proteins between cell lines may result, *e.g.,* from different insertion sites of the subunit(s) or copy number of introduced sequences that are being introduced into the cells. Optionally, a multimeric protein is characterized by generating a pharmacological profile by measuring the activity of at least 2, 5, 10, 50, 100, 250, 500, 1000, 2000, 5000, or at least 10,000 compounds against the multimeric protein. These pharmacological profiles of each cell line are compared with each other. If the pharmacological profiles are identical, the composition of the multimeric proteins are predicted to be the same. If the pharmacological profiles differ, the composition of the multimeric proteins are predicted to be different.

Also disclosed herein are panels of cell lines, wherein each panel comprises a plurality of cell lines each of which expresses a different multimeric protein as defined by different pharmacological profiles as discussed above, wherein the different cell lines were generated using the same protocol and the same host cell line. Optionally, substantially same protocols for cell culture could be used. Optionally, the cell lines of the panel could be processed in parallel or at different times but using consistent or similar cells, conditions or protocols.

Optionally, the methods can be applied to clones produced from different host cell lines. Optionally, the host cell lines differ in their gene expression profile. In these cases, the different cell lines can be used to characterize the effect of specific co-factors or endogenously provided factors on the formation of certain forms of a multimeric protein of interest.

Optionally, the activity of a multimeric protein can be tested by contacting the cell that expresses the multimeric protein with a compound that activates or inhibits the multimeric protein.

Optionally, the activity profile of a multimeric protein under certain conditions is determined. Without being bound by theory, it is believed that a same condition can have different effects on different forms of a multimeric protein. For example, a plurality of different conditions are tested for their effect on the multimeric protein. Exemplary conditions include: temperature, ion concentration in the cell media, $CO_2$ concentration, cell density, synchronization of cell cycle, pH, acidity, carbonation, atmospheric conditions, percent carbon dioxide, shaking, stirring, UV exposure, activity or concentration of metabolites, nutrients, serum, amino acids, sugars, carbohydrates, proteins, lipids, detergents, growth factors, co-factors, vitamins, mutagens, chemicals, compounds or trace metals. The activity profiles can then be used to deduce the composition of the multimeric protein.

Optionally, the pharmacological profile of a multimeric protein is determined. For example, a plurality of different compounds is tested for their effect on a multimeric protein. Exemplary compounds include compounds that are known to modulate the protein or a protein of the same class or family, compounds known to have side-effects in clinical studies, compounds with clinical efficacy, compounds that may be pharmacologically active, compounds of combinatorial compound libraries, chemical compounds, synthetic compounds, natural compounds, peptides, lipids, detergents, mutagens, fluorescent compounds or polymers. The pharmacological profiles can then be used to deduce the composition of the multimeric protein.

The disclosure herein makes possible the production of multiple cell lines expressing a protein of interest. Clonal cell lines disclosed herein will have different absolute and relative levels of such expression. A large panel of such clones can be screened for activity with a number of known reference compounds. In this way, each isolated cell line will have a "fingerprint" of responses to test compounds which represent the activities of differential functional expression of the protein. The cell lines can then be grouped based on the similarity of such responses to the compounds. At least one cell line representing each functionally distinct expression profile can be chosen for further study. A collection of these cell lines can then be used to screen a large number of compounds. In this way, compounds which selectively modulate one or more of the corresponding distinct functional forms of the protein may be identified. These modulators can then be tested in secondary assays or *in vivo* models to determine which demonstrate activity in these assays or models. In this connection, the modulators would be used as reference compounds to identify which corresponding functional forms of the protein may be present or play a role in the secondary assay or model system employed. Such testing may be used to determine the functional forms of a protein that may exist *in vivo* as well as those that may be physiologically relevant. These modulators could be used to discern which of the functionally distinct forms are involved in a particular phenotype or physiological function such as disease.

Also disclosed herein is a method for generating an in-vitro-correlate ("IVC") for an in vivo physiological property of interest. An IVC is generated by establishing the activity profile of a compound with an in vivo physiological property on different proteins, *e.g.,* a profile of the effect of a compound on the physiological property of different proteins. This activity profile is representative of the in vivo physiological property and thus is an IVC of a fingerprint for the physiological property.

Optionally, the in-vitro-correlate is an in-vitro-correlate for a negative side effect of a drug. Further optionally, the in-vitro-correlate is an in-vitro-correlate for a beneficial effect of a drug.

Optionally, the IVC can be used to predict or confirm one or more physiological properties of a compound of interest. The compound may be tested for its activity against different proteins and the resulting activity profile is compared to the activity profile of IVCs that were generated as described herein. The physiological property of the IVC with the activity profile most similar to the activity profile of a compound of interest is predicted to be and/or confirmed to be a physiological property of the compound of interest.

Optionally, an IVC is established by assaying the activities of a compound against different proteins or biological pathways, or combinations thereof. Similarly, to predict or confirm the physiological activity of a compound, the activities of the compound can be tested against different proteins or biological pathways, or combinations thereof.

Optionally, the methods disclosed herein can be used to determine and/or predict and/or confirm to what degree a particular physiological effect is caused by a compound of interest. Optionally, the methods disclosed herein can be used to determine and/or predict and/or confirm the tissue specificity of a physiological effect of a compound of interest.

The cell lines for use with the present methods can be engineered using gene activation (see, *e.g.,* PCT Application Publication WO/1994/012650) or introduction of a transgene. Cells expressing a protein of interest can be identified using molecular beacons or fluorogenic oligonucleotides (see, e.g., U.S. Patent No. 6,692,965 by Shekdar et al. issued February 17, 2004 and International Application No. PCT/US2005/005080 published as WO/2005/079462). Optionally, cells or cell lines are engineered to express a protein subunit that is part of a multimeric protein. Optionally, cells or cell lines are engineered to express at least 2, at least 3, at least 4, at least 5, at least 6, at least 7, at least 8, at least 9, at least 10, at least 11, or at least 12 subunits of a multimeric protein. Optionally, an activity profile is generated against a plurality of cell lines wherein each has been engineered to express a different combination of subunits of a multimeric protein.

Optionally, cells or cell lines to be used with the methods for generating an IVC are cells or cell lines as described herein, *e.g.,* cells or cell lines with a substantially constant physiological property, cells or cell lines that express a protein of interest which does not comprise a protein tag, or cells or cell lines having a Z' factor of at least 0.4 in a functional assay, or cells or cell lines cultured in the absence of selective pressure, or any combinations thereof. Optionally, different cells or cell lines to be used are maintained in parallel under substantially identical culture conditions. Robotic methods described herein can be used to maintain and manipulate the cells or cell lines for generating an IVC. Optionally, the invention provides a cell or cell line for generating an IVC, wherein the cell or cell line is cultured in the absence of selective pressure and wherein the expression of at least one protein of the cell does not vary by more than 1%, 5%, 10%, 15%, 20%, 25% 30% 35% or 40% over 3 months. Optionally the expression of the at least one protein does not vary by more than 1%, 5%, 10%, 15%, 20%, 25% 30% 35% or 40% over 4, 5, 6 or more months.

Optionally, the activity profile of a compound of interest is established by testing the activity of the compound in a plurality of in vitro assays using cell lines that are engineered to express a multimeric protein, wherein at least two cell lines express different multimeric proteins. Optionally, the different multimeric proteins are different combinations of subunits of a multimeric protein. Optionally, an IVC is generated by testing a compound of interest against at least 0.01%, 0.05%, 0.1%, 0.5%, 1%, 5%, 10%, 20%, 25%, 50%, 75%, 80%, 90%, 95%, 98%, or at least 99% of possible subunit combinations of a multimeric protein. Optionally, all possible subunit combinations are tested. Optionally, at least 5,10, 25, 50, 100, 150, 200, 250, 500, 1000, 2500, 5000, 7500, or at least 10000 subunit combinations are tested.

Optionally, different multimeric proteins of different cell lines are different forms of a multimeric protein, wherein different forms differ in the combination, stoichiometry, splicing, and/or post-translation modification of their subunits, including proteolysis.

Optionally, testing of failed and successful candidate drugs against a panel representing multiple functional forms of a target or related target can be used to correlate specific targets to adverse or undesired side-effects or therapeutic efficacy observed in the clinic. This information may be used to select well defined targets in HTS or during compound development towards drugs with maximal desired and minimal off-target activity.

Optionally, an IVC of a compound is generated by testing the compound against different cell lines that express different subunit combinations of multimeric proteins. Optionally such multimeric proteins include, but are not limited to receptor protein complexes such as $GABA_A$; NaV; $GABA_B$; ENaC; sweet receptor; umami receptor and other multimeric proteins described herein.

Optionally, an IVC is generated using ENaC, $GABA_A$, NaV, a sweet taste receptor, an umami taste receptor, a bitter taste receptor, CFTR, or GCC. As described below the IVC of a compound may represent the effect of the compound on a particular physiological parameter. Optionally, the physiological parameter is measured using functional magnetic resonance imaging ("fMRI"). Other imaging methods can also be used. Such other imaging methods include Computed tomography (CT); Computed Axial Tomography (CAT) scanning; diffuse optical imaging (DOI); diffuse optical tomography (DOT); event-related optical signal (EROS); near infrared spectroscopy (NIRS); magnetic resonance imaging (MRI); magnetoencephalography (MEG);positron emission tomography (PET) and single photon emission computed tomography (SPECT). Optionally, if the IVC represents an effect of the compound on the central nervous system ("CNS"), an IVC may be established that correlates with an fMRI pattern in the CNS. IVCs may be generated that correlate with activity of compounds in various tests and models, including human and animal (such as livestock and pets) testing models. Human diseases and disorders are listed, *e.g.,* in The Merck Manual 18th Edition (Hardcover) Mark H. Beers (Author) Robert S. Porter (Editor), Thomas V. Jones (Editor). Mental diseases and disorders are listed, *e.g.,* in Diagnostic and Statistical Manual of Mental Disorders DSM-IV-TR Fourth Edition (Text Revision) by American Psychiatric Association (Corporate Author).

IVCs generated using GABA may be for a physiological effect of a compound on CNS disorder, anxiety, sedation, post-traumatic stress disorder (PTSD), memory, learning, autism, epilepsy, alcoholism, mood disorders, CNS function, CNS physiology, CNS development, and / or aging of the CNS. The physiological effect can be an increase of decrease of at least one symptom of the disease.

IVCs for GABA may also be generated for an emotion, mood, state of mind, feeling, sensation of, or perception, including: happiness, satisfaction, euphoria, mania, excitement, anticipation, complacency, anxiety, depression, anger, fear, terror, doubt, love, hate, ambivalence, apathy, lethargy, guilt, devotion, sadness, unhappiness, grumpiness, irritability, drive, motivation, aggressiveness, hostility, rage, arrogance, self-assuredness or a lack of it, confidence, lack of confidence, uneasiness, hesitation; a positive, negative, cooperative, uncooperative, helpful or unhelpul attitude; a sense of freedom, well being, accomplishment, adequacy, inadequacy, or limitation; feelings of being attractive, unattractive, sexy, not sexy, likeable, dislikable, lovable, unlovable, desirable, or undesirable; feelings of loneliness or of being included / inclusiveness; of belonging or not belonging to a social group; feelings of being trapped, tricked, manipulated, misguided, helped, encouraged or discouraged; feelings of considering committing suicide; feelings of taking a risk, committing a crime, breaking the law, avoiding responsibility; or feelings of committing murder.

IVCs for GABA may also be generated for sensory stimulations that stimulate a positive or negative mood. Positive sensory stimulations may include odor sensations, such as sea breeze, forest, food, *etc.* In some embodiments, IVCs for GABA may be generated for sedation or sleep, including restful, uninterrupted, deep, light or REM sleep, restfulness, lack of sleep, insomnia, sleep disruption, sleep disturbances, or walking, talking or eating in one's sleep; memory, learning, interpretation, analysis, thinking, remembering, placing, making associations, recalling past events, short term memory, long term memory or cognition; alcohol dependence or addiction or alcoholism; CNS indications, chronic pain, epilepsy, convulsants, addiction, dependence; endocrine/hormonal indications, eye blink conditioning paradigms, lung cancer, prostate cancer, breast cancer and other cancers and carcinomas; glucose metabolic response, anoxia, prostaglandin induced thermogenesis, cardiac baro-receptor reflex and other reflex abnormalities; and mental disorders including autism.

An IVC generated using a bitter taste receptor may be for a physiological effect on obesity, sugar absorption, glucagon-like peptide (GLP) secretion, or blood glucose regulation. The physiological effect may be upregulation or downregulation of appetite, overall nutrition, degree or rate of nutrient absorption, obesity (gain or loss), degree or rate of sugar absorption, GLP secretion, or blood glucose regulation. Alternatively, the IVC may also be correlated to bitter taste in food and / or drug.

Optionally, IVCs using bitter taste receptors may be generated for activity in the gut (*e.g.,* GLP secretion or the secretion of other gut hormones), blood-glucose control or balance, diabetes, feeding, starvation, appetite, nutrient absorption, weight loss, energy. Further optionally, IVCs using bitter taste receptors may be generated for desired or undesired bitter taste / off-taste of beverage or food ingredients or drugs, including over the counter drugs and different stereoisoforms of drugs; for food preferences; for eating disorders (*e.g.* bulimia, anorexia or dieting); for the sensory or taste perception of compounds; or for quality control of the taste or bitter taste receptor or GPCR activity of beverage or food ingredients or drugs. Further optionally, IVCs using bitter taste receptors may be generated for: neuronal firing or CNS activity in response to active compounds; nausea, vomiting (including nausea caused by drugs or other compounds, *e.g.*, chemotherapy-induced nausea and vomiting (CINV)); activity of compounds at non-bitter GPCRs; bitter or bitter modulating compounds that are active in the oral cavity but not elsewhere (*e.g.,* not active in the gut), and vice versa. Optionally, compounds that activate, inhibit, or modulate bitter taste receptors in a tissue-specific manner can be used to modulate blood glucose levels, glucose absorption, and/or to prevent vomiting or CINV, where modulating activity is desirable in the gut but not in the oral cavity. Optionally, bitter modulating compounds that are only active in the oral cavity can be used in flavor applications where physiological activity in the gut is not desired.

IVCs generated using a sweet taste receptor may be for a physiological effect on appetite, nutrition, nutrient absorption, obesity, sugar absorption, GLP secretion, or blood glucose regulation. The physiological effect may be upregulation or downregulation of appetite, overall nutrition, degree or rate of nutrient absorption, obesity (gain or loss), degree or rate of sugar absorption, or GLP secretion, or blood glucose regulation.

IVCs using a sweet taste receptor may be generated for the following: lingering / long lasting / extended duration / off- or after-taste of sweet compounds including natural and artificial high intensity sweeteners (*e.g.* saccharin, aspartame, cyclamate, mogroside, stevia and its components, acesulfame K, neotame, sucralose and mixtures thereof); for food preferences; for eating disorders (*e.g.* bulimia, anorexia or dieting); activity in the gut (*e.g.* GLP secretion or the secretion of other gut hormones), blood-glucose control or balance, diabetes, feeding, starvation, appetite, nutrient absorption, weight loss or energy; desired, undesired or off-taste of beverage, food or drug ingredients, including over the counter drugs and different steroisoforms of drugs; sensory or taste perception of compounds; neuronal firing or CNS activity in response to active compounds; nausea / vomiting, including nausea caused by drugs or other compounds (*e.g.* CINV); quality control of the taste or GPCR triggering activity of beverage , food or drug ingredients; sweet or sweet modulating compounds that are active in the oral cavity but not elsewhere (*e.g.,* not active in the gut), and vice versa. In certain embodiments, compounds that activate, inhibit, or modulate sweet taste receptors in a tissue-specific manner can be used to modulate blood glucose levels, glucose absorption, and/or to prevent vomiting or CINV, where modulating activity is desirable in the gut but not in the oral cavity. In certain embodiments, bitter modulating compounds that are only active in the oral cavity can be used in flavor applications where physiological activity in the gut is not desired.

If an umami taste receptor is used to generate an IVC, the IVC may be for a physiological effect on appetite, nutrition, nutrient absorption, obesity, sugar absorption, GLP secretion, blood glucose regulation or amino acid absorption. The physiological effect may be upregulation or downregulation of appetite, overall nutrition, degree or rate of nutrient absorption, obesity (gain or loss), degree or rate of sugar absorption, or GLP secretion, or blood glucose regulation.

IVCs using an umami taste receptor can be generated for the following: food preferences; eating disorders (*e.g.* bulimia, anorexia or dieting); activity in the gut (*e.g.* GLP secretion or the secretion of other gut hormones), blood-glucose control or balance, diabetes, feeding, starvation, appetite, nutrient absorption, weight loss or energy; desired, undesired or off-taste of beverage, food or drug ingredients, including over the counter drugs and different steroisoforms of drugs; sensory or taste perception of compounds; neuronal firing or CNS activity in response to active compounds; nausea / vomiting, including nausea caused by drugs or other compounds (*e.g.* CINV); quality control of the taste or GPCR triggering activity of beverage , food or drug ingredients; umami or umami modulating compounds that are active in the oral cavity but not elsewhere (*e.g.,* not active in the gut), and vice versa. Optionally, compounds that activate, inhibit, or modulate umami taste receptors in a tissue-specific manner can be used to modulate blood glucose levels, glucose absorption, and/or to prevent vomiting or CINV, where modulating activity is desirable in the gut but not in the oral cavity. In certain embodiments, umami modulating compounds that are only active in the oral cavity can be used flavor applications where physiological activity in the gut is not desired.

An IVC generated using ENaC may be for a physiological effect of a compound on COPD (chronic obstructive pulmonary disease), CF (cystic fibrosis), fertility, IBS (irritable bowel syndrome), Crohn's disease, pulmonary edema or hypertension. The physiological effect can be worsening of the disease or reduction or amelioration of at least one symptom of the disease. The IVC may also represent different salt tastes of a compound.

IVCs using ENaC can also be generated for the following: food preferences; eating disorders (*e.g.* bulimia, anorexia or dieting); regulation, secretion, quality, clearance, production, viscosity, or thickness of mucous; water absorption, retention, balance, passing, or transport across epithelial tissues (especially lung, kidney, vascular tissues, eye, gut, small intestine and large intestine);neuronal firing or CNS activity in response to active compounds; pulmonary indications; gastrointestinal indications such as bowel cleansing, Irritable Bowel Syndrome (IBS), drug-induced (*i.e.* opioid) constipation, constipation/CIC of bedridden patients, acute infectious diarrhea, E.coli, cholera, viral gastroenteritis, rotavirus, modulation of malabsorption syndromes, pediatric diarrhea (viral, bacterial, protozoan), HIV, or short bowel syndrome; fertility indications such as sperm motility or sperm capacitation; female reproductive indications, cervical mucus/vaginal secretion viscosity *(i.e.* hostile cervical mucus); contraception, such as compounds that negatively affect sperm motility or cervical mucous quality relevant for sperm motility; or dry mouth, dry eye, glaucoma or runny nose.

IVCs using ENaC can also be generated for sensory or taste perception of compounds. In particular, the IVC can be for salt taste, such as taste of magnesium, sodium, potassium, and / or calcium salts. The salts may have different counter ions, such as sulfate chloride or other halides, bromide, phosphate, lactate and others. Optionally, the salt is potassium chloride or potassium lactate. Optionally, the IVC represents the taste perception of a combination of different salts.

An IVC generated using CFTR may be for a physiological effect of a compound on COPD (chronic obstructive pulmonary disease), CF (cystic fibrosis), fertility, IBS (irritable bowel syndrome), Crohn's disease, pulmonary edema or hypertension. The physiological effect can be an increase or decrease of at least one symptom of the disease.

IVCs using CFTR can also be generated for the following: regulation, secretion, quality, clearance, production, viscosity, or thickness of mucous; water absorption, retention, balance, passing, or transport across epithelial tissues (especially of lung, kidney, vascular tissues, eye, gut, small intestine, large intestine); sensory or taste perception of compounds; neuronal firing or CNS activity in response to active compounds; pulmonary indications; gastrointestinal indications such as bowel cleansing, Irritable Bowel Syndrome (IBS), drug-induced (*i.e.* opioid) constipation, constipation/CIC of bedridden patients, acute infectious diarrhea, E.coli, cholera, viral gastroenteritis, rotavirus, modulation of malabsorption syndromes, pediatric diarrhea (viral, bacterial, protozoan), HIV, or short bowel syndrome; fertility indications such as sperm motility or sperm capacitation; female reproductive indications, cervical mucus/vaginal secretion viscosity (*i.e.* hostile cervical mucus); contraception, such as compounds that negatively affect sperm motility or cervical mucous quality relevant for sperm motility; dry mouth, dry eye, glaucoma, runny nose; or endocrine indications, *i.e.* pancreatic function in CF patients.

An IVC generated using NaV may be for a physiological effect on pain. The physiological effect can be increase or decrease of the pain.

IVCs using NaV can also be generated for the following: pain (including chronic pain, acute pain, cardiac pain, muscle pain, bone pain, organ pain, fatigue, pain caused by overstimulation, abrasion, bodily harm or internal damage, pain from cancer, pain from physical injury, perceived pain, phantom pain, and debilitating pain); generation or propagation of action potentials, neuronal signaling, or transmission of neuronal information; or muscle or cardiac indications or for the activity of compounds on muscle or cardiac muscle in vivo.

An IVC generated using GCC can be for the following: gastrointestinal indications including: constipation, IBS (irritable bowel syndrome), including IBS-C (constipation), IBS-D (diarrhea) or IBS-M (mixed), chronic idiopathic constipation, opioid or drug induced constipation, constipation in bedridden or geriatric patients, acute infectious diarrhea (*e.g.* mediated by bacteria, E.coli, salmonella, cholera, especially traveler's diarrhea), viral gastroenteritis, clinical indications of rotavirus, malabsorption syndromes, pediatric diarrhea (viral, bacterial, protozoan), short bowel syndrome, colitis (collagenous, lymphocytic), Crohn's disease, UC, diverticulitis, cystic fibrosis or ulcers including petic ulcers; regulation/modulation of mucosal and/or epithelial fluid absorption and secretion; pulmonary indications such as cystic fibrosis, kidney function, cardiac fibrosis, cardiac hypertrophy, hypertension, eye disorders (*i.e.* autosomal dominant retinitis pigmentosa and Leber congenital amaurosis), growth disorders, short stature, stroke and other vascular injury; CNS indications such as memory or depression; or inflammatory disorders (*i.e.* rheumatoid arthritis).

Odorant receptors may be used to generate IVCs for sensory stimulations that stimulate a positive or negative mood. Sensory stimulations that stimulate a positive mood may include pleasant odors such as sea breeze, forest scents or food scents. Sensory stimulations that stimulate a positive mood may include unpleasant odors. IVCs may be generated for sensory stimulations that stimulate the following positive or negative emotions: happiness, satisfaction, euphoria, mania, excitement, anticipation, complacency, anxiety, depression, anger, fear, terror, doubt, love, hate, ambivalence, apathy, lethargy, guilt, devotion, sadness, unhappiness, grumpiness, irritability, drive, motivation, aggressiveness, hostility, rage, arrogance, self-assuredness or a lack of it, confidence, lack of confidence, uneasiness, hesitation; a positive, negative, cooperative, uncooperative, helpful or unhelpul attitude; a sense of freedom, well being, accomplishment, adequacy, inadequacy, or limitation; feeling of being attractive, unattractive, sexy, not sexy, likeable, dislikable, lovable, unlovable, desirable or undesirable; feelings of loneliness or of being included / inclusiveness, belonging or not belonging to a social group; feelings of being trapped, tricked, manipulated, misguided, helped, encouraged or discouraged; feelings of considering committing suicide; feelings of taking a risk, committing a crime, breaking the law, avoiding responsibility; or feelings of committing murder.

Acetylcholine receptors may also be used to generate IVCs for sensory stimulations that stimulate a positive or negative mood. Sensory stimulations include, but are not limited to, scents. IVCs may be generated for sensory stimulations that stimulate the following positive or negative emotions, moods, states of mind, feelings, sensations or perceptions, including: happiness, satisfaction, euphoria, mania, excitement, anticipation, complacency, anxiety, depression, anger, fear, terror, doubt, love, hate, ambivalence, apathy, lethargy, guilt, devotion, sadness, unhappiness, grumpiness, irritability, drive, motivation, aggressiveness, hostility, rage, arrogance, self-assuredness or a lack of it, confidence, lack of confidence, uneasiness, hesitation; a positive, negative, cooperative, uncooperative, helpful or unhelpul attitude; a sense of freedom, well being, accomplishment, adequacy, inadequacy, or limitation; feeling of being attractive, unattractive, sexy, not sexy, likeable, dislikable, lovable, unlovable, desirable or undesirable; feelings of loneliness or of being included / inclusiveness, belonging or not belonging to a social group; feelings of being trapped, tricked, manipulated, misguided, helped, encouraged or discouraged; feelings of considering committing suicide; feelings of taking a risk, committing a crime, breaking the law, avoiding responsibility; or feelings of committing murder.

IVCs for acetylcholine receptors may also be generated for physiological effects of a compound on a CNS disorder, anxiety, sedation, PTSD, memory, learning, autism, epilepsy, alcoholism, mood disorders, and / or CNS function, CNS physiology, CNS development, and / or aging of the CNS. The physiological effect can be increase or decrease of at least one symptom of the disease. IVCs generated using acetylcholine can also be generated for the following: sedation or sleep, including restful, uninterrupted, deep, light or REM sleep, restfulness, lack of sleep, insomnia, sleep disruption, sleep disturbances, or walking, talking or eating in one's sleep; memory, learning, interpretation, analysis, thinking, remembering, placing, making associations, recalling past events, short term memory, long term memory or cognition;alcohol dependence or addiction or alcoholism; CNS indications, chronic pain, epilepsy, convulsants, addiction, dependence; endocrine/hormonal indications, eye blink conditioning paradigms, lung cancer, prostate cancer, breast cancer and other cancers and carcinomas; glucose metabolic response, anoxia, prostaglandin induced thermogenesis, cardiac baro-receptor reflex and other reflex abnormalities; and mental disorders including autism.

Optionally, cells or cell lines for use with the methods are engineered to express one or more proteins as set forth in Tables 6 and 7-22 herein.

Optionally, an IVC represents a physiological property of a compound in a tissue of an organism, an organ of an organism, an extracellular matrix of an organism, a system of an organism (*e.g.,* the immune system of an organism), or a whole organism. The organism may be a vertebrate. The organism may be a mammal. In more specific embodiments, the organism is a mouse, rat, dog, cat, cattle, horse, donkey, goat, monkey, or human. The physiological property can be an effect on a particular cell type, tissue, organ, or organ system. For example, the physiological property can be an effect on mammalian tissue, healthy tissue, tissue, diseased tissue, cancer tissue, embryonic tissue, adult tissue, transplanted tissue, organ tissue, liver tissue, neuronal tissue, gastrointestinal tissue, muscle tissue, fat tissue, skin, urogenital tissue, neuronal tissue, the central nervous system, cardiovascular tissue, the endocrine system, skeletal tissue, bone tissue, bone, the immune system, an organ, a cell or a specialized cell, as well as any other cells disclosed herein.

Optionally, the physiological property is tissue protective activity, anti-inflammatory activity, neuro-stimulatory or has an activity similar to the activity of substances or compounds including but not limited to: adamantane antivirals, adrenergic bronchodilators, agents for hypertensive emergencies, agents for pulmonary hypertension, amebicides, analgesic combinations, analgesics, androgens and anabolic steroids, angiotensin II inhibitors, anorexiants, antacids, antihelmintics, anti-angiogenic ophthalmic agents, antiinfectives, antianginal agents, antiarrhythmic agents, antiasthmatic combinations, antibiotics/antineoplastics, anticholinergic antiemetics, anticholinergic antiparkinson agents, anticholinergic bronchodilators, anticholinergics/antispasmodics, anticoagulants, anticonvulsants, antidepressants, antidiabetic agents, antidiabetic combinations, antidiarrheals, antidotes, antiemetic/antivertigo agents, antifungals, antigout agents, antihistamines, antihyperlipidemic agents, antihyperlipidemic combinations, antihypertensive combinations, antihyperuricemic agents, antimalarial agents, antimalarial combinations, antimalarial quinolines, antimetabolites, antimigraine agents, antineoplastic detoxifying agents, antineoplastic interferons, antineoplastic monoclonal antibodies, antineoplastics, antiparkinson agents, antiplatelet agents, antipseudomonal penicillins, antipsoriatics, antipsychotics, antirheumatics, antiseptic and germicides, antitoxins and antivenins, antituberculosis agents, antituberculosis combinations, antitussives, antiviral agents, antiviral combinations, antiviral interferons, anxiolytics, sedatives, and hypnotics, bile acid sequestrants, bronchodilators, cardiac stressing agents, chelating agents, cholinergic muscle stimulants, CNS stimulants, coagulation modifiers, contraceptives, decongestants, digestive enzymes, diuretics, dopaminergic antiparkinsonism agents, drugs used in alcohol dependence, expectorants, factor Xa inhibitors, fatty acid derivative anticonvulsants, functional bowel disorder agents, gallstone solubilizing agents, general anesthetics, genitourinary tract agents, GI stimulants, glucose elevating agents, glycoprotein platelet inhibitors, growth hormone receptor blockers, hematopoietic stem cell mobilizer, heparin antagonists, hormone replacement therapy, hormones/antineoplastics, immunosuppressive agents, impotence agents, in vivo diagnostic biologicals, incretin mimetics, inotropic agents, laxatives, leprostatics, local injectable anesthetics, lung surfactants, lymphatic staining agents, lysosomal enzymes, mucolytics, muscle relaxants, mydriatics, ophthalmic glaucoma agents, ophthalmic lubricants and irrigations, spermicides, vasodilators, or vasopressors.

Optionally, an IVC represents the physiological effect of a compound of interest on a virus, bacterium, fungus, or yeast. Such compounds are useful, *e.g.*, as antibiotics against viruses, bacteria, fungi, and/or yeasts.

The assay to test and/or confirm the activity of a compound of interest against a particular multimeric protein of interest depends on the biological activity of the multimeric protein. Any assay to be used with the methods disclosed herein can be conducted in high throughput format.

Exemplary proteins, their references, and possible assays are set forth in the following Table (Table 5). These examples are non-limiting, in that the assays listed may be used for targets in addition to those listed, and the targets listed can be tested by other assays than those listed.

**Table 5**

| Assay | Protein | Reference |
|---|---|---|
| Intracellular calcium mobilization | GPCRs | Thomsen W et al (2005) Curr. Opin. Biotechnol. 16: 655-665 |
| Intracellular cAMP accumulation | GPCRs | Thomsen W et al (2005) Curr. Opin. Biotechnol. 16: 655-665 |
| Intracellular IP formation | GPCRs | Zhong H and Neubig RR (2001) J Pharmacol Exp Ther. 297:837-45 |
| Intracellular arrestin binding | GPCRs | Lattin J et al (2007) J. Leukoc. Biol. 82: 16-32 |
| GTPgammaS binding | GPCRs | Kimble RJ et al (2003) Combin. Chem. HTS 6:1-9 |
| Receptor binding | GPCRs, nuclear hormone receptors | Christopoulos A and Kenakin T (2002) Pharmacol Rev. 54: 323-74.<br>Germain P et al (2006) Pharmacol Rev 58:685-704 |
| Membrane potential | Ion channels | González JE et al (1999) Drug Discovery Tech. 4:431-439 |
| Ion transport | Ion channels / transporters | González JE et.al. (1999) Drug Discovery Tech. 4:431-439 |
| Amine transport | Transporters | Levi R, Smith NC.(2000) J Pharmacol Exp Ther. 292:825-830 |
| Glucose uptake | Transporters | Klip, A, et al (1994) FASEB J 8:43-53 |

(continued)

| Assay | Protein | Reference |
|---|---|---|
| Cell metabolism | Mitochondria | Laskowski KR and Russell RR (2008) Curr Heart Fail Rep. 5:75-9 |
| Enzyme activity | Enzymes | Estacio RO (2009) Postgrad Med.121:33-44. |
| Transcription factor induced gene expression | Nuclear hormone receptors | Willson TM and Moore JT (2002) Mol Endocrinol. 16:1135-1144. |
| Nuclear receptor translocation | Nuclear hormone receptors | Rosen J, Miner JN (2005) Endocr. Rev 26: 452-464 |
| Neurotransmitter release | Neurotransmitter receptors | von Euler US (1972) Pharmacol Rev. 24(2):365-369 |
| Bile/ enzyme secretion | Transporters | Roma MG at al (2008) World J Gastroenterol. 14:6786-6801 |
| Peptide - protein secretion | GPCRs | Tanoue A. (2009) J Pharmacol Sci. 109:50-52 |
| Free radical secretion | Enzymes | Podesser BK and Hallström S (2007) Br J Pharmacol. 151 (7):930-940 |
| Cell adhesion | Membrane proteins | Prozialeck WC and Edwards JR (2007) Pharmacol Ther. 114:74-93 |
| Cell proliferation | Toxicity assay | Brinkmann M et al (2002) Cytotechnology 38: 119-127 |
| Cell differentiation | Transcription factor | Kelloff GJ et al (2000) Cancer Epidemiol Biomarkers Prev. 9:127-137 |
| Cell migration | Toxicity assay | Goodwin AM. (2007) Microvasc Res. 74:172-183. |
| Cell viability | Toxicity assay | Schmauss D and Weis M (2008) Circulation 117:2131-2141 |
| Cell apoptosis | Toxicity assay | Fabregat I. (2009) World J Gastroenterol. 15:513-520 |
| Phagocytosis/ endocytosis | Membrane proteins | Motyl T et al (2006) J Physiol Pharmacol. 57 Suppl 7:17-32 |
| Chromosome aberration | Toxicity assay | Jacobson-Kram D et al (1993) Environ Health Perspect. 101 Suppl 3:121-125 |
| DNA synthesis | Toxicity assay | Kenyon J and Gerson SL (2007) Nucleic Acids Res. 35:7557-7565 |

The activity profile of a compound of interest and a landmark activity profile may be compared through computation of a correlation between the activity profiles, such as but not limited to computing a measure of similarity between the activity profiles. The landmark activity profile may be one of a group of activity profiles in a database. The landmark activity profile may be a historical profile. The database of landmark activity profiles can be stored on a computer readable storage medium. Optionally, the database contains at least 10 landmark activity profiles, at least 50 landmark activity profiles, at least 100 landmark activity profiles, at least 500 landmark activity profiles, at least 1,000 landmark activity profiles, at least 10,000 landmark activity profiles, or at least 50,000 landmark activity profiles, each landmark activity profile containing measured amounts of at least 2, at least 10, at least 100, at least 200, at least 500, at least 1,000, at least 2000, at least 2500, at least 7500, at least 10,000, at least 20,000, at least 25,000, or at least 35,000 components. The activity profile of the compound of interest can comprise measured amounts representing an effect of the compound of interest on the biological activity of different multimeric proteins, such as a first protein subunit and a second protein subunit. A landmark activity profile provides an in vitro correlate for a known physiological property of a previously characterized compound. The physiological property can be a pharmacological property such as, but not limited to, a negative side effect of a drug or a beneficial effect of a drug. Each landmark activity profile can comprise measured amounts representing the effect of a respective previously characterized compound on the biological activity of different multimeric proteins, such as the first protein subunit and the second protein subunit of a multimeric protein. Optionally, a correlation can be computed between the activity profile of a compound of interest and each landmark activity profile of a plurality of landmark activity profiles stored in a database. The correlation can be computed by comparing a measured amount in the activity profile of a compound of interest representing an effect of the compound of interest on the biological activity of a given multimeric protein to the corresponding measured amount in the landmark activity profile representing an effect of the previously characterized compound on the biological activity of the same multimeric protein. The activity profile of a compound of interest can be deemed to correlate with a landmark activity profile if the measured amounts in the landmark activity profile are within about 2%, about 5%, about 8%, about 10%, about 12%, about 15%, about 20%, about 25%, about 30%, or about 35% of the measured amounts in the activity profile of the compound of interest.

The activity profile of a compound of interest can be deemed to be most similar to a landmark activity profile if a measure of similarity between the activity profile of the compound of interest and the landmark activity profile is above a predetermined threshold. Optionally, the predetermined threshold can be determined as the value of the measure of similarity which indicates that the measured amounts in a landmark activity profile are within about 2%, about 5%, about 8%, about 10%, about 12%, about 15%, about 20%, about 25%, about 30%, or about 35% of the measured amounts in the activity profile of the compound of interest.

Optionally, the activity profile of a compound of interest can be expressed as a vector p,

$$\boldsymbol{p} = [\,\boldsymbol{p}_1, \ldots \boldsymbol{p}_i, \ldots \boldsymbol{p}_n]$$

where $\boldsymbol{p}_i$ is the measured amount of the *i*'th component, for example, the effect of the compound of interest on the *i*'th biological activity of a given multimeric protein. In specific embodiments, *n* is more than 2, more than 10, more than 100, more than 200, more than 500, more than 1000, more than 2000, more than 2500, more than 7500, more than 10,000, more than 20,000, more than 25,000, or more than 35,000. Each landmark activity profile also can be expressed as a vector *p*. In computing a correlation, the measured amount of the *i*'th component in the vector representing the activity profile for the compound of interest can be compared to the corresponding measured amount of the *i*'th component of the vector representing a landmark activity profile, for each component *i* = 1 ... *n.* However, there are many ways in which a correlation can be computed. Indeed, any statistical method in the art for determining the probability that two datasets are related may be used in accordance with the methods of the present invention in order to identify whether there is a correlation between the activity profile of a compound of interest and a landmark activity profile. For example, the correlation between the activity profile ($p_{i_1}$) of the compound of interest and each landmark activity profile ($p_{i_2}$) can be computed using a similarity metric $sim(p_{i_1}, p_{i_2})$. One way to compute the similarity metric $sim(p_{i_1}, p_{i_2})$ is to compute the negative square of the Euclidean distance. In alternative embodiments, metrics other than Euclidean distance can be used to compute $sim(p_{i_1}, p_{i_2})$, such as a Manhattan distance, a Chebychev distance, an angle between vectors, a correlation distance, a standardized Euclidean distance, a Mahalanobis distance, a squared Pearson correlation coefficient, or a Minkowski distance. In some embodiments a Pearson correlation coefficient, a squared Euclidean distance, a Euclidean sum of squares, or squared Pearson correlation coefficients is used to determine similarity. Such metrics can be computed, for example, using SAS (Statistics Analysis Systems Institute, Cary, North Carolina) or S-Plus (Statistical Sciences, Inc., Seattle, Washington). Use of such metrics are described in Draghici, 2003, Data Analysis Tools for DNA Microarrays, Chapman & Hall, CRC Press London, chapter 11.

The correlation can also be computed based on ranks, where $x_i$ and $y_i$ are the ranks of the values of the measured amounts in ascending or descending numerical order. See for example, Conover, Practical Nonparametric Statistics, 2nd ed., Wiley, (1971). Shannon mutual information also can be used as a measure of similarity. See for example, Pierce, An Introduction To Information Theory: Symbols, Signals, and Noise, Dover, (1980).

Various classifiers known in the art can be trained according to the methods described in this application, and used to classify a compound of interest as to a physiological property, such as but not limited to a pharmacological property. Algorithms can be used to produce classifiers capable of predicting a physiological property of a compound of interest using an activity profile of the compound of interest. Exemplary classifiers are described above. Optionally, the classifier can be trained using the measured amounts in the landmark activity profile of a previously characterized compound and the known physiological property associated with that previously characterized compound.

The classifier may be an algorithm used for classification by applying a non-supervised or supervised learning algorithm to evaluate the measured amounts in the landmark activity profile of a previously characterized compound and the known physiological property associated with that previously characterized compound. Any standard non-supervised or supervised learning technique known in the art can be used to generate a classifier. Below are non-limiting examples of non-supervised and supervised algorithms known in the art. Given the disclosure in this application, one of skill in the art will appreciate that other pattern classification or regression techniques and algorithms may be used for the classifier and the present invention encompasses all such techniques.

*Neural networks.* Neural networks (*e.g.*, a two-stage regression or classification decision rule) are described hereinabove.

*Clustering.* Optionally, the classifier is learned using clustering. Optionally, select components *i* of the vectors representing the landmark activity profiles are used to cluster the activity profiles. Optionally, prior to clustering, the measured amounts are normalized to have a mean value of zero and unit variance.

Landmark activity profiles that exhibit similar patterns of measured amounts across the training population will tend to cluster together. A particular combination of measured amounts of components *i* can be considered to be a good classifier in this aspect of the invention when the vectors cluster into the physiological property. Clustering is described on pages 211-256 of Duda 1973. As described in Section 6.7 of Duda 1973, the clustering problem is described as one of finding natural groupings in a dataset. To identify natural groupings, two issues are addressed. First, a way to measure similarity (or dissimilarity) between two activity profile is determined. This metric (similarity measure) is used to ensure that the activity profiles in one cluster are more like one another than they are to other activity profiles. Second, a mechanism for partitioning the data into clusters using the similarity measure is determined.

Similarity measures are discussed in Section 6.7 of Duda 1973, where it is stated that one way to begin a clustering investigation is to define a distance function and to compute the matrix of distances between pairs of activity profiles. If distance is a good measure of similarity, then the distance between activity profiles in the same cluster will be significantly less than the distance between activity profiles in different clusters. However, as stated on page 215 of Duda 1973, clustering does not require the use of a distance metric. For example, a nonmetric similarity function s(x, x') can be used to compare two vectors x and x'. Conventionally, s(x, x') is a symmetric function whose value is large when x and x' are somehow "similar". An example of a nonmetric similarity function s(x, x') is provided on page 216 of Duda 1973.

Other aspects of clustering are further discussed hereinabove.

*Principal component analysis ("PCA').* Optionally, the classifier is learned using principal component analysis. PCA is discussed hereinabove.

In one approach to using PCA to learn a classifier, vectors representing landmark activity profiles can be constructed in the same manner described for clustering above. In fact, the set of vectors, where each vector represents a landmark activity profile, can be viewed as a matrix. Optionally, this matrix is represented in a Free-Wilson method of qualitative binary description of monomers (Kubinyi, 1990, 3D QSAR in drug design theory methods and applications, Pergamon Press, Oxford, pp 589-638), and distributed in a maximally compressed space using PCA so that the first principal component (PC) captures the largest amount of variance information possible, the second principal component (PC) captures the second largest amount of all variance information, and so forth until all variance information in the matrix has been considered.

Then, each of the vectors, where each vector represents a member of the training population (such as the landmark activity profiles), is plotted. Many different types of plots are possible. Optionally, a one-dimensional plot is made. In this one-dimensional plot, the value for the first principal component from each of the members of the training population is plotted. In this form of plot, the expectation is that activity profiles corresponding to a physiological property will cluster in one range of first principal component values and profiles corresponding to another physiological property will cluster in a second range of first principal component values.

Optionally, the members of the training population are plotted against more than one principal component. For example, in some embodiments, the members of the training population are plotted on a two-dimensional plot in which the first dimension is the first principal component and the second dimension is the second principal component.

*Nearest neighbor analysis.* Nearest neighbor analysis is described hereinabove.

*Linear discriminant analysis.* Optionally, the classifier is learned using linear discriminant analysis. Linear discriminant analysis (LDA) attempts to classify a subject into one of two categories based on certain object properties. In other words, LDA tests whether object attributes measured in an experiment predict categorization of the objects. LDA typically requires continuous independent variables and a dichotomous categorical dependent variable. In the disclosure herein, the abundance values for the select combinations of vector components i across a subset of the training population serve as the requisite continuous independent variables. The trait subgroup classification (*e.g.,* a physiological property) of each of the members of the training population serves as the dichotomous categorical dependent variable.

LDA seeks the linear combination of variables that maximizes the ratio of between-group variance and within-group variance by using the grouping information. Implicitly, the linear weights used by LDA depend on how the measured amount of a vector component i across the training set separates in the groups of the physiological property. Optionally, LDA is applied to the data matrix of the members in the training population. Then, the linear discriminant of each member of the training population is plotted. Ideally, those members of the training population representing a physiological property will cluster into one range of linear discriminant values (for example, negative) and those members of the training population representing another physiological property will cluster into a second range of linear discriminant values (for example, positive). The LDA is considered more successful when the separation between the clusters of discriminant values is larger. For more information on linear discriminant analysis, see Duda, Pattern Classification, Second Edition, 2001, John Wiley & Sons, Inc; and Hastie, 2001, The Elements of Statistical Learning, Springer, New York; and Venables & Ripley, 1997, Modern Applied Statistics with s-plus, Springer, New York.

*Quadratic discriminant analysis.* Quadratic discriminant analysis is described hereinabove.

*Support vector machine.* Support vector machine is described hereinabove.

*Decision tree.* Decision tree is described hereinabove.

*Multivariate adaptive regression splines.* Multivariate adaptive regression splines are described hereinabove.

*Centroid classifier techniques.* Optionally a nearest centroid classifier technique is used. Such a technique computes, for different physiological properties, a centroid given by the average measured amounts of vector components i in the training population (landmark activity profiles), and then assigns vector representing the compound of interest to the class whose centroid is nearest. This approach is similar to k-means clustering except clusters are replaced by known classes. An example implementation of this approach is the Prediction Analysis of Microarray, or PAM. See, for example, Tibshirani et al., 2002, Proceedings of the National Academy of Science USA 99; 6567-6572.

*Regression.* Optionally, the classifier is a regression classifier, such as a logistic regression classifier. Such a regression classifier includes a coefficient for each of the activity profiles used to construct the classifier. Optionally, the coefficients for the regression classifier are computed using, for example, a maximum likelihood approach. In such a computation, the measured amounts of vector components i are used.

Other methods to learn a classifier are further described hereinabove.

The disclosure herein can be implemented as a computer program product that comprises a computer program mechanism embedded in a computer-readable storage medium. Further, any of the methods disclosed herein can be implemented in one or more computers or other forms of apparatus. Examples of apparatus include but are not limited to, a computer, and a measuring device (for example, an assay reader or scanner). Further still, any of the methods disclosed herein can be implemented in one or more computer program products. Some disclosures herein provide a computer program product that encodes any or all of the methods disclosed in this application. Such methods can be stored on a CD-ROM, DVD, magnetic disk storage product, or any other computer-readable data or program storage product. Such computer readable storage media are intended to be tangible, physical objects (as opposed to carrier waves). Such methods can also be embedded in permanent storage, such as ROM, one or more programmable chips, or one or more application specific integrated circuits (ASICs). Such permanent storage can be localized in a server, 802.11 access point, 802.11 wireless bridge/station, repeater, router, mobile phone, or other electronic devices. Such methods encoded in the computer program product can also be distributed electronically, via the Internet or otherwise, by transmission of a computer data signal (in which the software modules are embedded) either digitally or on a carrier wave (it will be clear that such use of carrier wave is for distribution, not storage).

Some disclosures herein provide a computer program product that contains any or all of the program modules shown in Fig. 3. These program modules can be stored on a CD-ROM, DVD, magnetic disk storage product, or any other computer-readable data or program storage product. The program modules can also be embedded in permanent storage, such as ROM, one or more programmable chips, or one or more application specific integrated circuits (ASICs). Such permanent storage can be localized in a server, 802.11 access point, 802.11 wireless bridge/station, repeater, router, mobile phone, or other electronic devices. The software modules in the computer program product can also be distributed electronically, via the Internet or otherwise, by transmission of a computer data signal (in which the software modules are embedded) either digitally or on a carrier wave.

Optionally, the computer program provides for outputting a result of the claimed method to a user, a user interface device, a computer readable storage medium, a monitor, a local computer, or a computer that is part of a network. Such computer readable storage media are intended to be tangible, physical objects (as opposed to carrier waves).

The disclosure herein also provides methods for creating cell lines for proteins that have not been well characterized. For such proteins, there is often little information regarding the nature of their functional response to known compounds. Such a lack of established functional benchmarks to assess the activity of clones may be one challenge in producing physiologically relevant cell lines. The method described above provides a way to obtain physiologically relevant cell lines even for proteins that are not well characterized where there is a lack of such information. Cell lines comprising the physiologically relevant form of a protein may be obtained by pursuing clones representing a number or all of the functional forms that may result from the expression of genes comprising a protein.

The cells and cell lines disclosed herein may be used to identify the roles of different forms of the protein of interest in different pathologies by correlating the identity of *in vivo* forms of the protein with the identity of known forms of the protein based on their response to various modulators. This allows selection of disease- or tissue-specific modulators for highly targeted treatment of pathologies associated with the protein.

To identify a modulator, one exposes a cell or cell line disclosed herein to a test compound under conditions in which the protein would be expected to be functional and then detects a statistically significant change (*e.g.,* $p<0.05$) in protein activity compared to a suitable control, *e.g.,* cells that are not exposed to the test compound. Positive and/or negative controls using known agonists or antagonists and/or cells expressing the protein of interest may also be used. One of ordinary skill in the art would understand that various assay parameters may be optimized, *e.g.,* signal to noise ratio.

Optionally, one or more cells or cell lines disclosed herein are exposed to a plurality of test compounds, for example, a library of test compounds. Such libraries of test compounds can be screened using the cell lines of the invention to identify one or more modulators of the protein of interest. The test compounds can be chemical moieties including small molecules, polypeptides, peptides, peptide mimetics, antibodies or antigen-binding portions thereof, natural compounds, synthetic compounds , extracts, lipids, detergents, and the like. In the case of antibodies, they may be non-human antibodies, chimeric antibodies, humanized antibodies, or fully human antibodies. The antibodies may be intact antibodies comprising a full complement of heavy and light chains or antigen-binding portions of any antibody, including antibody fragments (such as Fab and Fab, Fab', $F(ab')_2$, Fd, Fv, dAb and the like), single chain antibodies (scFv), single domain antibodies, all or an antigen-binding portion of a heavy chain or light chain variable region.

Optionally, prior to exposure to a test compound, the cells or cell lines disclosed herein may be modified by pretreatment with, for example, enzymes, including mammalian or other animal enzymes, plant enzymes, bacterial enzymes, protein modifying enzymes and lipid modifying enzymes. Such enzymes can include, for example, kinases, proteases, phosphatases, glycosidases, oxidoreductases, transferases, hydrolases, lyases, isomerases, ligases bacterial proteases, proteases from the gut, proteases from the GI tract, proteases in saliva, in the oral cavity, proteases from lysol cells/bacteria, and the like. Alternatively, the cells and cell lines may be exposed to the test compound first followed by enzyme treatment to identify compounds that alter the modification of the protein by the treatment.

Optionally, large compound collections are tested for protein modulating activity in a cell-based, functional, high-throughput screen (HTS), *e.g.*, using 96-well, 384-well, 1536-well or higher density formats. Optionally, a test compound or multiple test compounds, including a library of test compounds, may be screened using more than one cell or cell line disclosed herein.

Optionally, the cells and cell lines disclosed herein have increased sensitivity to modulators of the protein of interest. Cells and cell lines disclosed herein also respond to modulators with a physiological range $EC_{50}$ or $IC_{50}$ values for the protein. As used herein, $EC_{50}$ refers to the concentration of a compound or substance required to induce a half-maximal activating response in the cell or cell line. As used herein, $IC_{50}$ refers to the concentration of a compound or substance required to induce a half-maximal inhibitory response in the cell or cell line. $EC_{50}$ and $IC_{50}$ values may be determined using techniques that are well-known in the art, for example, a dose-response curve that correlates the concentration of a compound or substance to the response of the protein -expressing cell line.

A further advantageous property of the cells and cell lines disclosed herein is that modulators identified in initial screening using those cells and cell lines are functional in secondary functional assays. As those of ordinary skill in the art will recognize, compounds identified in initial screening assays typically must be modified, such as by combinatorial chemistry, medicinal chemistry or synthetic chemistry, for their derivatives or analogs to be functional in secondary functional assays. However, due to the high physiological relevance of the cells and cell lines of this invention, many compounds identified using those cells and cell lines are functional without further modification. Optionally, at least 25%, 30%, 40%, 50% or more of the modulators identified in an initial assay are functional in a secondary assay. Further, cell lines disclosed herein perform in functional assays on a par with the "gold standard" assays. For example, cell lines disclosed herein expressing GABA A receptors perform substantially the same in membrane potential assays and in electrophysiology.

In a further aspect of the disclosure herein, differentiated, adult or specialized cells generated according to the disclosure herein may be used to generate stem cells. Optionally, cells of the invention or cells identified by the methods disclosed herein wherein the cell type or specification is a differentiated, adult or specialized cell may be dedifferentiated into stems cells including but not limited to multipotent stem cells, pluripotent stem cells, omnipotent stem cells, induced pluripotent stem ("iPS") cells, embryonic stem cells, cancer stem cells, and organ or tissue specific stem cells. Methods of dedifferentiation are known to those skilled in the art. See, *e.g.,* Panagiotis A. Tsonis; Stem Cells from Differentiated Cells; Molecular Interventions 4:81-83, (2004). Stem cells generated from the cells disclosed herein or cells identified by the methods disclosed herein may be differentiated into one or more cells of a differentiated, adult, or specialized cell type or specification. Embryonic stem cells and iPS cells generated from the cells disclosed herein or cell identified by the methods disclosed herein may be used to produce a whole non-human organism, *e.g.,* a mouse. Method of producing mice using mouse embryonic stem cells are known to those skilled in the art. See, *e.g.,* Smith, "EMBRYO-DERIVED STEM CELLS: Of Mice and Men", Annu. Rev. Cell Dev. Biol. 2001, 17:435-62. Methods of producing mice using iPS cells are known to those skilled in the art. See, *e.g.,* Kang et al., "iPS Cells Can Support Full-Term Development of Tetraploid Blastocyst-Complemented Embryos", Cell Stem Cell, Jul 22, 2009 [Epub ahead of print], and Zhao et a/., "iPS cells produce viable mice through tetraploid complementation", Nature, July 23, 2009 [Epub ahead of print].

Optionally, cells disclosed herein or cells identified by the methods disclosed herein wherein the cell type or specification is a differentiated, adult or specialized cell may be dedifferentiated into stems cells including but not limited to multipotent stem cells, pluripotent stem cells, omnipotent stem cells, induced pluripotent stem cells ("iPS"), embryonic stem cells, cancer stem cells, and organ or tissue specific stem cells, and the stem cells thus produced may be differentiated into one or more cells of a differentiated, adult, or specialized cell type or specification.

Optionally, cells disclosed herein or cells identified by the methods disclosed herein wherein the cell type or specification is a differentiated, adult or specialized cell may be dedifferentiated into embryonic stem cells or iPS cells, and the stem cells thus produced may be used to produce a whole organism, *e.g.,* a mouse. Optionally, cells disclosed herein or cells identified by the methods disclosed herein wherein the cell type or specification is a differentiated, adult or specialized cell may be dedifferentiated into embryonic stem cells or iPS cells, and the stem cells thus produced may be used to produce a whole organism, *e.g.,* a mouse, wherein the cells in the organism of the same cell type or specification comprise the same properties for which the cells of the invention were selected, *e.g.,* expression of a protein or RNA of interest.

Optionally, cells of a specialized cell or tissue type comprising an RNA or protein or a functional or physiological form of an RNA or protein may be used to produce an embryonic stem cell or iPS cell that may be used to produce an organism, *e.g.,* a mouse, wherein the cells or tissues of the organism of the same type comprise the RNA or protein or the functional or physiological form of the RNA or protein. Optionally, the organism thus produced comprises RNA or protein of the same species. Further optionally, the organism thus produced comprises the RNA or protein of a different species. Optionally, the organism is mouse and the RNA or protein is of a human origin. Optionally, the organism thus produced comprises an in vitro correlate. Optionally, the organism thus produced may be used in testing, including preclinical testing. Optionally, the testing or preclinical testing is used to predict the activity of test compounds in humans.

Also disclosed herein is a method for generating an in vitro correlate for an in vivo physiological property. An in vitro correlate for an in vivo physiological property includes the effect(s) of one or more compounds on one or more proteins or RNAs expressed in the cells or cell lines of the present invention (*i.e.*, expressed in vitro) that correlate(s) to the effect(s) of the one or more compounds on one or more pharmacological properties in vivo. Without being bound by theory, a test compound may be considered to have similar or substantially same effect(s) on one or more in vivo physiological properties, as compared to a reference compound, if it is found to have similar or substantially same effect(s) on one or more proteins or RNAs expressed in vitro, as compared to the reference compound, *i.e*., the test compound is considered to have similar or substantially same in vitro correlate as compared to the reference compound (*e.g.,* at least 90% identical). Optionally, the test compound is considered to have similar or substantially the same in vitro correlate as compared to the reference compound (*e.g.,* at least 10%, 20%, 30%, 40%, 50% 60%, 70%, 80% or 90% identical). An in vitro correlate may comprise one or more activity profiles of a compound.

In a further aspect, a protein or a plurality of proteins expressed by the cells or cell lines disclosed herein provides an in vitro correlate for an in vivo protein of interest or a plurality of in vivo proteins of interest. Cells and cell lines disclosed herein may not exactly recapitulate those cells in vivo in providing exactly the same conditions for expressing one or more proteins, *e.g.,* there may be difference(s) in post-translational modification, folding, assembly, subunit combinations, transport and/or membrane integration between a protein expressed in the cells or cell lines disclosed herein, as compared to the protein expressed in vivo. In addition, the parameters used in testing the function of a protein expressed in vitro may not exactly recapitulate those parameters in vivo, *e.g.,* certain protein functional assays performed in vitro may be performed under certain pH or salt concentration that is considered non-physiological. Nonetheless, proteins expressed by the cells or cell lines of the present invention may be biologically active in these non-physiological conditions and may provide at least one functional or pharmacological or physiological profile when assayed in vitro. Such a functional or pharmacological or physiological profile may correspond to the in vivo protein. For example, a compound that regulates or alters the physiological property associated with a protein in vivo may be able to alter a biological activity of the corresponding protein expressed by the cells or cell lines of the present invention when assayed in vitro, thereby establishing an correlation between the protein expressed by the cells or cell lines of the present invention and the protein expressed in vivo, with the protein expressed by the cells or cell lines of the present invention considered as the "in vitro correlate" of the protein expressed in vivo. For example, we have found that different cell lines each expressing the same set of Nav subunits ($\alpha$, $\beta$1 and $\beta$2) can respond differently to the same set of compounds. See, *e.g.,* Example 23 hereinbelow. These distinct functional profiles are indications of different subunit combinations in these cell lines, and each of the different subunit combinations may be considered as an in vitro correlate of a corresponding subunit combination in vivo. In addition, we have also obtained profiles of compound activities against panels of cell lines expressing bitter taste receptors. Sensory human taste testing was used to compare with the compound activity profiles to see which patterns of compound activity against the panel correlated with a desired or off-taste in vivo.

Aside from response to compounds, in vitro correlates of the present invention may also be generated and/or categorized by applying other treatments and/or conditions to the proteins expressed by the cells of the present invention. For example, we have generated in vitro correlates of ENaC by proteolysis (*e.g.*, generation of different proteolyzed forms of ENac), and have generated in vitro correlates of sweet/umami receptors by applying different media conditions.

An in vitro correlate may comprise one protein or a plurality of proteins. Such an in vitro correlate may be predictive of the function or activity of its corresponding protein(s) in vivo. Such an in vitro correlate may be used in a high throughput screening to identity modulators of one or more biological activities of the protein expressed by the cells or cell lines disclosed herein (*i.e.*, the in vitro correlate), and some or all of the compounds thus identified as modulators of the in vitro correlate may also modulate the protein expressed in vivo (*e.g.,* have a therapeutic effect in vivo). In various embodiments, at least 10%, 20%, 30%, 40%, 50%, 60%, 70%, 80%, 90%, 95%, or 99% of all of the compounds identified in a high throughput screening are capable of having a therapeutic effect. Optionally, an in vitro correlate may comprise at least 2, 3, 4, 5, or 6 subunits. Optionally, an in vitro correlate may be a heteromultimer. Optionally, the in vitro correlate is stably expressed in cells cultured in the absence of selective pressure. Optionally, the in vitro correlate is expressed in a cell line without causing cytotoxicity. Optionally, the in vitro correlate is expressed in a cell that does not endogenously express the protein or plurality of proteins.

Optionally, cells or cell lines expressing the in vitro correlate disclosed herein may be used to identify a modulator of an in vivo protein of interest, *e.g.,* by contacting the cells or cell lines with a test compound; and detecting a change in the activity of the protein or plurality of proteins of the in vitro correlate in the cells or cell lines contacted with the test compound compared to the activity of the protein or plurality of proteins of the in vitro correlate in a cell not contacted by the test compound, wherein a compound that produces a difference in the activity in the presence compared to in the absence is a modulator of the in vivo protein of interest.

Also disclosed herein are kits that can be used in the methods described herein. Optionally, a kit comprises one or more containers comprising one or more reagents for use in the methods described herein.

Also disclosed herein are kits that can be used in the methods described herein. In particular, provided herein are kits comprising one or more cells or cell lines stably expressing one or more complex targets. Optionally, kits provided herein comprise one or more signaling probes described herein. Optionally, a kit may comprise one or more vectors encoding one or more complex targets. Optionally, a kit comprises one or more dyes for use in functional cell-based assays (*e.g.,* calcium flux assay, membrane potential assay) to screen and select cells stably expressing one or more complex targets.

Also disclosed herein are kits comprising one or more containers filled with one or more of the reagents and/or cells described herein, such as one or more cells, vectors, and/or signaling probes provided herein. Optionally, the kits disclosed herein further comprise a control reagent (*e.g.,* control signaling probe, dye, cell, and/or vector), wherein such control reagent may be a positive control or a negative control reagent. Optionally associated with such container(s) can be a notice containing instructions for using the components in the kit.

<u>Sweet Taste Receptor and Umami Taste Receptor</u>

This invention relates to novel cells and cell lines that have been engineered to stably express a T1 R2 and a T1 R3 subunit of a sweet taste receptor, as well as optionally a G protein. This invention also relates to novel cells and cell lines that have been engineered to stably express a T1 R1 and a T1 R3 subunit of an umami taste receptor, as well as optionally a G protein. In some embodiments, the taste receptor (*e.g.,* sweet taste receptor or umami taste receptor) produced in those cells and cell lines is functional and physiologically relevant. In other aspects, the invention provides methods of making and using these cells and cell lines. The cells and cell lines of the invention comprising a taste receptor, *e.g.,* umami taste receptor or a sweet taste receptor can be used to identify modulators of the taste receptor, *e.g.,* umami taste receptor or sweet taste receptor. These modulators are useful in modifying the taste of, *e.g.,* food stuffs and pharmaceuticals, and in the therapeutic treatment of diseases where the taste receptor, *e.g.,* umami taste receptor or sweet taste receptor, is implicated, *e.g.,* obesity and diabetes.

According to some embodiments of the invention, the novel cells and cell lines are singly or doubly transfected with a nucleic acid encoding a sweet taste receptor T1 R2 subunit and/or a nucleic acid encoding a sweet taste receptor T1 R3 subunit. In some specific embodiments, the novel cells and cell lines are singly or doubly transfected with a nucleic acid encoding an umami taste receptor T1 R1 subunit and/or a nucleic acid encoding an umami taste receptor T1 R3 subunit. The other subunit may be expressed in the cell from endogenous nucleic acids. The two nucleic acids, if present, may be in the same or in separate vectors. In another embodiment, the novel cells and cell lines of this invention are singly, doubly or triply transfected with nucleic acids encoding a sweet taste receptor T1 R2 subunit, a sweet taste receptor T1 R3 subunit and a G protein. In another embodiment, the novel cells and cell lines of this invention are singly, doubly or triply transfected with nucleic acids encoding an umami taste receptor T1 R1 subunit, an umami taste receptor T1 R3 subunit and a G protein. As before, the nucleic acids may be in the same or in separate vectors. For example, three vectors can be used; one vector can be used; or two vectors can be used. The other subunits, and optional G protein, may be expressed in the cell from endogenous nucleic acids. In another embodiment, the novel cells and cells lines have at least one taste receptor subunit, *e.g.,* umami taste receptor subunit or sweet taste receptor subunit, activated for expression by gene activation. The other taste receptor subunit, *e.g.,* umami taste receptor subunit or sweet taste receptor subunit, and/or optionally a G protein, as necessary, may be expressed from introduced nucleic acid sequences encoding those proteins or may be already expressed from endogenously active nucleic acids. The novel cell lines of the invention stably express the introduced and/or gene activated taste receptor subunits, *e.g.,* umami taste receptor subunits or sweet taste receptor subunits, and optionally the G protein.

As described above, in some embodiments of this invention, the cells and cell lines of the invention are engineered to produce a G protein, in addition to producing the two subunits of a taste receptor, *e.g.,* umami taste receptor or sweet taste receptor. The cells and cell lines are engineered to produce a G protein because they do not, in their pre-engineered state, produce a G protein, which is necessary to trigger downstream signaling from an activated taste receptor, *e.g.,* umami taste receptor or sweet taste receptor, or the cells do not produce that G protein in a sufficient amount for taste receptor induced signaling, *e.g.,* umami taste receptor induced signaling or sweet taste receptor induced signalling.

In a first aspect, the invention provides cells and cell lines that express taste receptors, *e.g.,* umami taste receptors or sweet taste receptors, which cells and cell lines have enhanced properties as compared to cells and cell lines made by conventional methods. For example, the taste receptor cells and cell lines, *e.g.,* umami taste receptor cells and cell lines or sweet taste receptor cells and cell lines, of this invention have enhanced stability of expression and/or levels of expression (even when maintained in cultures without selective pressure, including, for example, antibiotics and other drugs). In other embodiments, the cells and cell lines of the invention have high Z' values in various assays. In still other embodiments, the cells and cell lines of this invention are improved in the context of their expression of physiologically relevant taste receptor activity, *e.g.,* umami taste receptor activity or sweet taste receptor activity, as compared to more conventionally engineered cells. These properties enhance and improve the ability of the cells and cell lines of this invention to be used in assays to identify modulators of taste receptors, *e.g.,* umami taste receptors and/or sweet taste receptors, and improve the functional attributes of the identified modulators.

In various embodiments, the cells or cell lines of the invention express umami taste receptor T1 R1 and T1 R3 subunits or sweet taste receptor T1 R2 and T1 R3 subunits at a consistent level of expression for at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 110, 120, 130, 140, 150, 160, 170, 180, 190, 200 days or over 200 days, where consistent expression refers to a level of expression that does not vary by more than: 1%, 2%, 3%, 4%, 5%, 6%, 7%, 8% 9% or 10% over 2 to 4 days of continuous cell culture; 1%, 2%, 4%, 6%, 8%, 10% or 12% over 5 to 15 days of continuous cell culture; 1 %, 2%, 4%, 6%, 8%, 10%, 12%, 14%, 16%, 18% or 20% over 16 to 20 days of continuous cell culture; 1%, 2%, 4%, 6%, 8%, 10%, 12%, 14%, 16%, 18%, 20%, 22%, 24% over 21 to 30 days of continuous cell culture; 1%, 2%, 4%, 6%, 8%, 10%, 12%, 14%, 16%, 18%, 20%, 22%, 24%, 26%, 28% or 30% over 30 to 40 days of continuous cell culture; 1 %, 2%, 4%, 6%, 8%, 10%, 12%, 14%, 16%, 18%, 20%, 22%, 24%, 26%, 28% or 30% over 41 to 45 days of continuous cell culture; 1%, 2%, 4%, 6%, 8%, 10%, 12%, 14%, 16%, 18%, 20%, 22%, 24%, 26%, 28% or 30% over 45 to 50 days of continuous cell culture; 1%, 2%, 4%, 6%, 8%, 10%, 12%, 14%, 16%, 18%, 20%, 22%, 24%, 26%, 28%, 30% or 35% over 45 to 50 days of continuous cell culture; 1%, 2%, 4%, 6%, 8%, 10%, 12%, 14%, 16%, 18%, 20%, 22%, 24%, 26%, 28% or 30% or 35% over 50 to 55 days of continuous cell culture; 1%, 2%, 4%, 6%, 8%, 10%, 12%, 14%, 16%, 18%, 20%, 22%, 24%, 26%, 28%, 30% or 35% over 50 to 55 days of continuous cell culture; 1 %, 2%, 3%, 4%, 5%, 10%, 15%, 20%, 25%, 30%, 35% or 40% over 55 to 75 days of continuous cell culture; 1%, 2%, 3%, 4%, 5%, 5%, 10%, 15%, 20%, 25%, 30%, 35%, 40% or 45% over 75 to 100 days of continuous cell culture; 1%, 2%, 3%, 4%, 5%, 5%, 10%, 15%, 20%, 25%, 30%, 35%, 40% or 45% over 101 to 125 days of continuous cell culture; 1%, 2%, 3%, 4%, 5%, 5%, 10%, 15%, 20%, 25%, 30%, 35%, 40% or 45% over 126 to 150 days of continuous cell culture; 1%, 2%, 3%, 4%, 5%, 5%, 10%, 15%, 20%, 25%, 30%, 35%, 40% or 45% over 151 to 175 days of continuous cell culture; 1%, 2%, 3%, 4%, 5%, 5%, 10%, 15%, 20%, 25%, 30%, 35%, 40% or 45% over 176 to 200 days of continuous cell culture; 1%, 2%, 3%, 4%, 5%, 5%, 10%, 15%, 20%, 25%, 30%, 35%, 40% or 45% over more than 200 days of continuous cell culture.

According to the invention, the taste receptor, *e.g.,* umami taste receptor or sweet taste receptor, expressed by a cell or cell line of the invention can be from any mammal, including rat, mouse, rabbit, goat, dog, cow, pig or primate. The T1 R2 and T1 R3 subunits that together form the expressed sweet taste receptor can be from the same or different species. For example, sweet taste receptor T1 R2 subunit from any species may be co-expressed with a sweet taste receptor T1 R3 subunit from the same species or from any other species in a cell or cell line of the invention. Also, the T1 R1 and T1 R3 subunits that together form the expressed umami taste receptor can be from the same or different species. For example, umami taste receptor T1 R1 subunit from any species may be co-expressed with an umami taste receptor T1 R3 subunit from the same species or from any other species in a cell or cell line of the invention. Similarly, in the embodiments where a G protein is also expressed in the cells and cell lines of this invention, the G protein may be from any species. Among these G proteins are those referred to in Table 7. Chimera G proteins (G$\alpha$15- G$\alpha$16; GNA15-GNA16) can also be expressed in the cells and cell lines of this invention. G proteins from any species may be co-expressed with a sweet taste receptor T1 R2 subunit from any species, and a sweet taste receptor T1 R3 subunit from any species or any combination of the three may be used. In a specific embodiment, the sweet taste receptor is a human sweet taste receptor and is characterized, preferably, by human T1 R2 and T1 R3 subunits. In another specific embodiment, the umami taste receptor is a human umami taste receptor and is characterized, preferably, by human T1 R1 and T1 R3 subunits. One aspect of the invention provides a collection of clonal cells and cell lines, each expressing the same taste receptor, *e.g.,* umami taste receptor or sweet taste receptor, or different taste receptors, *e.g.,* umami taste receptors or sweet taste receptors. The collection may include, for example, cells or cell lines expressing combinations of different subunits, or of full length or fragments of those subunits.

The nucleic acid encoding taste receptor subunits, *e.g.,* umami taste receptor subunits T1 R1 and T1 R2 or sweet taste receptor subunits T1 R2 and T1 R3, and the nucleic acid encoding the optional G protein can be genomic DNA, cDNA, synthetic DNA or mixtures of them. In some embodiments, the taste receptor (*e.g.,* umami taste receptor or sweet taste receptor) subunit-encoding nucleic acid sequence and optionally the nucleic acid sequence encoding the G protein further comprise a tag. Such tags may encode, for example, a HIS tag, a myc tag, a hemagglutinin (HA) tag, protein C, VSV-G, FLU, yellow fluorescent protein (YFP), green fluorescent protein, FLAG, BCCP, maltose binding protein tag, Nus-tag, Softag-1, Softag-2, Strep-tag, S-tag, thioredoxin, GST, V5, TAP or CBP. A tag may be used as a marker to determine taste receptor (*e.g.,* umami taste receptor or sweet taste receptor) subunit and G protein expression levels, intracellular localization, protein-protein interactions, taste receptor (*e.g.,* umami taste receptor or sweet taste receptor) regulation, or taste receptor (*e.g.,* umami taste receptor or sweet taste receptor) function. Tags may also be used to purify or fractionate taste receptors (*e.g.,* umami taste receptors or sweet taste receptors) or G proteins.

In some embodiments, a cell or cell line of the invention may comprise nucleic acid (SEQ ID NO: 31), which encodes a human sweet taste receptor T1 R2 subunit. In some embodiments, a cell or cell line of the invention may comprise nucleic acid (SEQ ID NO: 41), which encodes a human umami taste receptor T1 R1 subunit. A cell or cell line of the invention may also comprise nucleic acid (SEQ ID NO: 32), which encodes a human sweet taste receptor or umami taste receptor T1 R3 subunit. In a specific embodiment, the cells or cell lines of the invention comprise both nucleic acids encoding T1 R3 and T1 R1 or T1 R2. In other embodiments, a cell or cell line of the invention may comprise nucleic acid sequence (SEQ ID NO: 33) in addition to the nucleic acids encoding the T1 R1 and T1 R3 subunits or T1 R2 and T1 R3 subunits. SEQ ID NO: 33 encodes a mouse G$\alpha$15 protein. In other embodiments, this G protein is human G$\alpha$15. See SEQ ID NO: 37.

In some embodiments, the nucleic acid encoding a taste receptor subunit (e.g., umami taste receptor subunit or sweet taste receptor subunit) and optional G protein comprises one or more substitutions, insertions, mutations or deletions, as compared to a nucleic acid sequence encoding the wild-type taste receptor subunit *(e.g.,* umami taste receptor subunit or sweet taste receptor subunit) or G protein. In embodiments comprising a nucleic acid comprising a mutation, the mutation may be a random mutation or a site-specific mutation. These nucleic acid changes may or may not result in an amino acid substitution. In some embodiments, the nucleic acid is a fragment of the nucleic acid that encodes a taste receptor subunit (*e.g.,* umami taste receptor subunit or sweet taste receptor subunit) or G protein. Nucleic acids that are fragments or have such modifications encode polypeptides that retain at least one biological property of a taste receptor subunit (*e.g.,* umami taste receptor subunit or sweet taste receptor subunit) or a G protein, *e.g.,* its ability with its other subunit to activate a G protein or its ability to be activated by a taste receptor subunit (*e.g.,* umami taste receptor subunit or sweet taste receptor), respectively.

The invention also encompasses cells and cell lines stably expressing a, subunit-encoding nucleic acid, whose sequence is at least about 85% identical to subunit sequences selected from the group of SEQ ID NO: 31, SEQ ID NO: 41, SEQ ID NO: 32 or a counterpart nucleic acid derived from a species other than human or a nucleic acid that encodes the same amino acid sequence as any of those nucleic acids. In some embodiments, the subunit-encoding sequence identity is at least 85%, 90%, 95%, 96%, 97%, 98%, 99% or higher compared to those subunit sequences. The invention also encompasses cells and cell lines wherein the nucleic acid encoding a taste receptor subunit (*e.g.,* umami taste receptor subunit or sweet taste receptor subunit) that hybridizes under stringent conditions to subunit sequences selected from the group of SEQ ID NO: 31, SEQ ID NO: 41, SEQ ID NO: 32 or a counterpart nucleic acid derived from a species other than human, or a nucleic acid that encodes the same amino acid sequence as any of those nucleic acids.

In some embodiments, the cell or cell line comprises a taste receptor subunit-encoding nucleic acid sequence (*e.g.,* umami taste receptor subunit-encoding nucleic acid sequence or sweet taste receptor subunit-encoding nucleic acid sequence) comprising at least one substitution as compared to SEQ ID NO: 31, SEQ ID NO: 41, SEQ ID NO: 32 or a counterpart nucleic acid derived from a species other than human or a nucleic acid that encodes the same amino acid sequence as any of those nucleic acids. The substitution may comprise less than 10, 20, 30, or 40 nucleotides or, up to or equal to 1%, 5%, 10% or 20% of the nucleotide sequence. In some embodiments, the substituted sequence may be substantially identical to SEQ ID NO: 31, SEQ ID NO: 41, SEQ ID NO: 32 or a counterpart nucleic acid derived from a species other than human a nucleic acid that encodes the same amino acid sequence as any of those nucleic acids (*e.g.,* a sequence at least 85%, 90%, 95%, 96%, 97%, 98%, 99% or higher identical thereto), or be a sequence that is capable of hybridizing under stringent conditions to SEQ ID NO: 31, SEQ ID NO: 41, SEQ ID NO: 32 or a counterpart nucleic acid derived from a species other than human or a nucleic acid that encodes the same amino acid sequence as any one of those nucleic acids.

In some embodiments, the cell or cell line comprises a taste receptor subunit-encoding nucleic acid sequence (*e.g.,* umami taste receptor subunit-encoding nucleic acid sequence or sweet taste receptor subunit-encoding nucleic acid sequence) comprising an insertion into or deletion from SEQ ID NO: 31, SEQ ID NO: 41, SEQ ID NO: 32 or a counterpart nucleic acid derived from a species other than human or a nucleic acid that encodes the same amino acid sequence as any of those nucleic acids. The insertion or deletion may be less than 10, 20, 30, or 40 nucleotides or up to or equal to 1%, 5%, 10% or 20% of the nucleotide sequence. In some embodiments, the sequences of the insertion or deletion may be substantially identical to SEQ ID NO: 31, SEQ ID NO: 41, SEQ ID NO: 32 or a counterpart nucleic acid derived from a species other than human or a nucleic acid that encodes the same amino acid sequence as any of those nucleic acids (*e.g.,* a sequence at least 85%, 90%, 95%, 96%, 97%, 98%, 99% or higher identical thereto), or be a sequence that is capable of hybridizing under stringent conditions to SEQ ID NO: 31, SEQ ID NO: 41, SEQ ID NO:

32 or a counterpart nucleic acid derived from a species other than human, or a nucleic acid that encodes the same amino acid sequence as any of those nucleic acids.

As described above, in some embodiments the cells and cell lines of this invention optionally express a G protein, for example, the mouse Gα15 protein (SEQ ID NO: 36) or human Gα15 protein (SEQ ID NO: 37). As with the nucleic acid sequence encoding the T1 R2 and T1 R3 subunits of a sweet taste receptor or the T1 R1 and T1 R3 subunits of an umami taste receptor, the nucleic acid sequence encoding the G protein (and the amino sequence of the G protein) may include substitutions, deletions and insertions as described above for the sweet taste receptor subunits.

In some embodiments, the nucleic acid substitution or modification results in an amino acid change, such as an amino acid substitution. For example, an amino acid residue of SEQ ID NO: 34 (human T1 R2), SEQ ID NO: 35 (human T1 R3), SEQ ID NO: 42 (umami human T1 R1 isoform 1 aa), SEQ ID NO: 43 (umami human T1 R1 isoform 2 aa), SEQ ID NO: 44 (umami human T1 R1 isoform 3 aa), SEQ ID NO: 45 (umami human T1 R1 isoform 4 aa), or a counterpart amino acid derived from a species other than human or an amino acid residue of SEQ ID NOS: 36 (mouse Gα15) and 37 (human Gα15) or a G protein from any species may be replaced by a conservative or a non-conservative substitution. In some embodiments, the sequence identity between the original and modified amino acid sequence can differ by about 1 %, 5%, 10% or 20% or from a sequence substantially identical thereto (*e.g.,* a sequence at least 85%, 90%, 95%, 96%, 97%, 98%, 99% or higher identical thereto).

A "conservative amino acid substitution" is one in which an amino acid residue is substituted by another amino acid residue having a side chain R group with similar chemical properties to the parent amino acid residue (*e.g.,* charge or hydrophobicity). In cases where two or more amino acid sequences differ from each other by conservative substitutions, the percent sequence identity or degree of similarity may be adjusted upwards to correct for the conservative nature of the substitution. Means for making this adjustment are well-known to those of skill in the art. *See, e.g.,* Pearson, Methods Mol. Biol. 243:307-31 (1994).

Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Preferred conservative amino acids substitution groups are: valine-leucine-isoleucine, phenylalanine-tyrosine, lysine-arginine, alanine-valine, glutamate-aspartate, and asparagine-glutamine. Alternatively, a conservative amino acid substitution is any change having a positive value in the PAM250 log-likelihood matrix disclosed in Gonnet et al., Science 256:1443-45 (1992). A "moderately conservative" replacement is any change having a nonnegative value in the PAM250 log-likelihood matrix.

Conservative modifications in subunits T1 R2 and T1 R3 will produce sweet taste receptors having functional and chemical characteristics similar *(i.e.* at least 50%, 60%, 70%, 80%, 90% or 95% the same) to those of the unmodified sweet taste receptor. Conservative modifications in subunits T1 R1 and T1 R3 will produce umami taste receptors having functional and chemical characteristics similar *(i.e.* at least 50%, 60%, 70%, 80%, 90% or 95% the same) to those of the unmodified umami taste receptor. The same is true for conservative modifications in a G protein.

Host cells used to produce a cell or cell line of the invention may express in their native state one or more endogenous taste receptor subunits (*e.g.,* umami taste receptor subunits or sweet taste receptor subunits) or lack expression of any taste receptor subunits (*e.g.,* umami taste receptor subunits or sweet taste receptor subunit). The same is true for a G protein. The host cell may be a primary, germ, or stem cell, including an embryonic stem cell. The host cell may also be an immortalized cell. Primary or immortalized host cells may be derived from mesoderm, ectoderm or endoderm layers of eukaryotic organisms. The host cell may be endothelial, epidermal, mesenchymal, neural, renal, hepatic, hematopoietic, or immune cells. For example, the host cells may be blood/immune cells such as B cell, T cell (Cytotoxic T cell, Natural Killer T cell, Regulatory T cell, T helper cell, gd T cell , Natural killer cell); granulocytes (Basophil granulocyte, Eosinophil granulocyte, Neutrophil granulocyte/Hypersegmented neutrophil), Monocyte/Macrophage, Red blood cell (Reticulocyte), Mast cell, Thrombocyte/Megakaryocyte, Dendritic cell; Endocrine cells such as thyroid (Thyroid epithelial cell, Parafollicular cell), parathyroid (Parathyroid chief cell, Oxyphil cell), adrenal (Chromaffin cell); Nervous system cells such as glial cells (Astrocyte, Microglia), Magnocellular neurosecretory cell, Stellate cell, Nuclear chain cell, Boettcher cell, pituitary, (Gonadotrope, Corticotrope, Thyrotrope, Somatotrope, Lactotroph), Respiratory system cells such as Pneumocyte (Type I pneumocyte, Type II pneumocyte), Clara cell, Goblet cell; Circulatory system cells (Myocardiocyte, Pericyte); Digestive system cells (stomach (Gastric chief cell, Parietal cell), Goblet cell, Paneth cell, G cells, D cells, ECL cells, I cells, K cells ,Enteroendocrine cells, Enterochromaffin cell, APUD cell, liver (Hepatocyte, Kupffer cell), pancreas (beta cells, alpha cells), gallbladder); Cartilage/bone/muscle/integumentary system cells such as osteoblast, osteocyte, osteoclast, tooth cells (Cementoblast, Ameloblast); cartilage cells such as Chondroblast, Chondrocyte, skin/hair cells such as Trichocyte, Keratinocyte; Melanocyte muscle cells such as Myocyte, Adipocyte, Fibroblast; Urinary system cells such as Podocyte, Juxtaglomerular cell, Intraglomerular mesangial cell/Extraglomerular mesangial cell, Kidney proximal tubule brush border cell, Macula densa cell; Reproductive system cells such as Spermatozoon, Sertoli cell, Leydig cell, Ovum, Ovarian follicle cell; Sensory cells such as organ of Corti cells, olfactory epithelium, temperature sensitive sensory neurons, Merckel cells, olfactory receptor neuron, pain sensitive neurons, photoreceptor cells, taste bud cells, hair cells of the vestibular apparatus, carotid body cells.. The host cells may be eukaryotic, prokaryotic, mammalian, avian, chicken, reptile, amphibian, frog, lizard, snake, fish, worms, squid, lobster, sea urchin, sea slug, sea squirt, fly, hydra, arthropods, beetles, chicken, lamprey, ricefish, zebra finch, pufferfish, and Zebrafish. Mammalian examples include human, non-human primate, bovine, porcine, feline, rat, marsupial, murine, canine, ovine, caprine, rabbit, guinea pig and hamster. The host cells may also be nonmammalian, such as yeast, insect, fungus, plant, lower eukaryotes and prokaryotes. Such host cells may provide backgrounds that are more divergent for testing taste receptor modulators, *e.g.,* umami taste receptor modulators or sweet taste receptor modulators, with a greater likelihood for the absence of expression products provided by the cell that may interact with the target. In preferred embodiments, the host cell is a mammalian cell.

Examples of host cells that may be used to produce a cell or cell line of the invention include but are not limited to: Human Embryonic Kidney-293T cells, established neuronal cell lines, pheochromocytomas, neuroblastomas fibroblasts, rhabdomyosarcomas, dorsal root ganglion cells, NS0 cells, CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171), L-cells, HEK-293 (ATCC CRL1573) and PC12 (ATCC CRL-1721), HEK293T (ATCC CRL-11268), RBL (ATCC CRL-1378), SH-SY5Y (ATCC CRL-2266), MDCK (ATCC CCL-34), SJ-RH30 (ATCC CRL-2061), HepG2 (ATCC HB-8065), ND7/23 (ECACC 92090903), CHO (ECACC 85050302), Vero (ATCC CCL 81), Caco-2 (ATCC HTB 37), K562 (ATCC CCL 243), Jurkat (ATCC TIB-152), Per.C6 (Crucell, Leiden, The Netherlands), Huvec (ATCC Human Primary PCS 100-010, Mouse CRL 2514, CRL 2515, CRL 2516), HuH-7D12 (ECACC 01042712), 293 (ATCC CRL 10852), A549 (ATCC CCL 185), IMR-90 (ATCC CCL 186), MCF-7 (ATC HTB-22), U-2 OS (ATCC HTB-96), T84 (ATCC CCL 248), or any established cell line (polarized or nonpolarized) or any cell line available from repositories such as American Type Culture Collection (ATCC, 10801 University Blvd. Manassas, Va. 20110-2209 USA) or European Collection of Cell Cultures (ECACC, Salisbury Wiltshire SP4 0JG England).

In one embodiment, the host cell is an embryonic stem cell that is then used as the basis for the generation of transgenic animals that produce a taste receptor, *e.g.,* umami taste receptor or sweet taste receptor. Embryonic stem cells stably expressing at least one taste receptor subunit (*e.g.,* umami taste receptor subunits or sweet taste receptor subunit), or both taste receptor subunits (*e.g.,* both umami taste receptor subunits or both sweet taste receptor subunits), and preferably a functional taste receptor (*e.g.,* umami taste receptor or sweet taste receptor), may be implanted into organisms directly, or their nuclei may be transferred into other recipient cells and these may then be implanted, or they may be used to create transgenic animals. In some embodiments one or more subunits may be expressed in the animal with desired temporal and/or tissue specific expression.

As will be appreciated by those of skill in the art, any vector that is suitable for use with a chosen host cell may be used to introduce a nucleic acid encoding a taste receptor subunit (*e.g.,* umami taste receptor subunits or sweet taste receptor subunit) or G protein into a host cell. The vectors comprising the nucleic acids encoding the various taste receptor subunits *(e.g.,* umami taste receptor subunits or sweet taste receptor subunits) or G protein may be the same type or may be of different types. Examples of vectors that may be used to introduce a taste receptor subunit (*e.g.,* umami taste receptor subunit or sweet taste receptor subunit) or G protein encoding nucleic acids into host cells include but are not limited to plasmids, viruses, including retroviruses and lentiviruses, cosmids, artificial chromosomes and may include, for example, pFN11 A (BIND) Flexi®,pGL4.31,pFC14A (HaloTag® 7) CMV Flexi®,pFC14K (HaloTag® 7) CMV Flexi®,pFN24A (HaloTag® 7) CMVd3 Flexi®,pFN24K (HaloTag® 7) CMVd3 Flexi®,HaloTag™ pHT2,pACT ,pAdVAntage™,pALTER®-MAX,pBIND,pCAT®3-Basic,pCAT®3-Control ,pCAT®3-Enhancer,pCAT®3-Promoter,pCI,pCMVTNT™ ,pG5luc,pSI,pTARGET™,pTNT™,pF12A RM Flexi® ,pF12K RM Flexi®,pReg neo,pYES2/GS,pAd/CMV/V5-DEST Gateway® Vector ,pAckPL-DEST™ Gateway® Vector,Gateway® pDEST™27 Vector ,Gateway® pEF-DEST51 Vector ,Gateway® pcDNA™-DEST47 vector ,pCMV/Bsd Vector ,pEF6/His A, B, & c,pcDNA™6.2-DEST,pLenti6/TR,pLP-AcGFP1-C, pLPS-AcGFP1-N,pLP-IRESneo, pLP-TRE2, pLP-RevTRE, pLP-LNCX, pLP-CMV-HA, pLP-CMV-Myc, pLP-RetroQ, pLP-CMVneo, pCMVScript, pcDNA3.1 Hygro, pCDNA3.1 neo, pcDNA3.1 puro, PSV2neo, pIRESpuro, pSV2zeo. In some embodiments, the vectors comprise expression control sequences such as constitutive or conditional promoters, preferably, constitutive promoters are used. One of ordinary skill in the art will be able to select such sequences. For example, suitable promoters include but are not limited to CMV, TK, SV40 and EF-1$\alpha$. In some embodiments, the promoters are inducible, temperature regulated, tissue specific, repressible, heat-shock, developmental, cell lineage specific, eukaryotic, prokaryotic or temporal promoters or a combination or recombination of unmodified or mutagenized, randomized, shuffled sequences of any one or more of the above. In other embodiments, a taste receptor subunit (*e.g.,* umami taste receptor subunit or sweet taste receptor subunit) or more than one of them (and optionally the G protein) is expressed by gene activation or episomally.

In some embodiments, the vector lacks a selectable marker or drug resistance gene. In other embodiments, the vector optionally comprises a nucleic acid encoding a selectable marker, such as a protein that confers drug or antibiotic resistance or more generally any product that exerts selective pressure on the cell. Each vector for introducing a sequence encoding a different taste receptor subunit (*e.g.,* umami taste receptor subunit or sweet taste receptor subunit) or G protein may have the same or a different drug resistance or other selective pressure marker. If more than one of the drug resistance or selective pressure markers are the same, simultaneous selection may be achieved by increasing the level of the drug. Suitable markers are well-known to those of skill in the art and include but are not limited to polypeptides products conferring resistance to any one of the following: Neomycin/G418, Puromycin, hygromycin, Zeocin, methotrexate and blasticidin. Although drug selection (or selection using any other suitable selection marker) is not a required step in producing the cells and cell lines of this invention, it may be used to enrich the transfected cell population for stably transfected cells, provided that the transfected constructs are designed to confer drug resistance. If subsequent selection of cells expressing both umami taste receptor subunits, and optionally a G protein, or both sweet taste receptor subunits and optionally a G protein is accomplished using signaling probes, selection too soon following transfection can result in some positive cells that may only be transiently and not stably transfected. However, this effect can be minimized by allowing sufficient cell passage to allow for dilution of transient expression in transfected cells.

In some embodiments, the vector used to introduce a nucleic acid encoding a taste receptor subunit (*e.g.,* umami taste receptor subunit or sweet taste receptor subunit) or optionally a G protein comprises a nucleic acid sequence encoding an RNA tag sequence. An "RNA Tag sequence" refers to a nucleic acid sequence that is an expressed RNA or portion of an RNA that is to be detected by a signaling probe. Signaling probes may detect a variety of RNA sequences. Any of these RNAs may be used as tags. Signaling probes may be directed against the RNA tag by designing the probes to include a portion that is complementary to the RNA sequence of the tag. The tag sequence may be a 3' untranslated region of the vector that is cotranscribed and comprises a target sequence for signaling probe binding. The RNA produced from the nucleic acid of interest may include the tag sequence or the tag sequence may be located within a 5'-untranslated region or 3'-untranslated region. In some embodiments, the tag is not part of the RNA produced from the nucleic acid of interest. The tag sequence can be in frame with the protein-coding portion of the message of the nucleic acid of interest or out of frame with it, depending on whether one wishes to tag the protein produced. Thus, the tag sequence does not have to be translated for detection by the signaling probe. The tag sequences may comprise multiple target sequences that are the same or different, wherein one signaling probe hybridizes to each target sequence. The tag sequences may encode an RNA having secondary structure. The structure may be a three-arm junction structure. Examples of tag sequences that may be used in this invention, and to which signaling probes may be prepared, include but are not limited to the RNA transcript of epitope tags such as, for example, a HIS tag, a myc tag, a hemagglutinin (HA) tag, protein C, VSV-G, FLU, yellow fluorescent protein (YFP), green fluorescent protein, various known magnetic tags, FLAG, BCCP, maltose binding protein tag, Nus-tag, Softag-1, Softag-2, Strep-tag, S-tag, thioredoxin, GST, V5, TAP or CBP. As described herein, one of ordinary skill in the art can readily prepare and use RNA tag sequences.

To make the cells and cell lines of the invention, one can use, for example, the methods described in U.S. Patent 6,692,965 and International Patent Publication WO/2005/079462. Both of these documents are incorporated herein by reference in their entirety for all purposes. These methods, which are preferred for making the cells and cell lines of this invention, provide real-time assessment of millions of cells such that any desired number of clones (from tens to hundreds to thousands of clones) expressing (*i.e.,* producing RNA) the nucleic acid sequences of interest may be selected. Using cell sorting techniques, such as flow cytometric cell sorting (*e.g.,* with a FACS machine) or magnetic cell sorting (*e.g.,* with a MACS machine), one selected cell per well may be automatically deposited with high statistical confidence in a culture vessel (such as a 96 well culture plate). The speed and automation of the technology allows multigene cell lines (*i.e.,* those expressing the T1 R2 and T1 R3 subunits of a taste receptor (*e.g.,* umami taste receptor or sweet taste receptor) and optionally a G protein) to be readily isolated.

Using this technology, the RNA sequence for each taste receptor subunit (*e.g.,* umami taste receptor subunit or sweet taste receptor subunit) (and optionally the G protein) expressed in the cell or cell line may be detected using a signaling probe, also referred to as a molecular beacon or fluorogenic probe. In some embodiments, the molecular beacon recognizes a target sequence as described above. In another embodiment, the molecular beacon recognizes a sequence within the taste receptor subunit (*e.g.,* umami taste receptor subunit or sweet taste receptor subunit) (or G protein) itself. Signaling probes may be directed against the RNA tag or a taste receptor subunit sequence (*e.g.,* umami taste receptor subunit sequence or sweet taste receptor subunit sequence) (or G protein sequence) by designing the probes to include a portion that is complementary to the RNA sequence of the tag or the taste receptor subunit (*e.g.,* umami taste receptor subunit or sweet taste receptor subunit) (or G protein), respectively.

Nucleic acids encoding a taste receptor subunit (*e.g.,* umami taste receptor subunit or sweet taste receptor subunit) (and optionally a nucleic acid encoding a G protein), or the sequence encoding a taste receptor subunit (*e.g.,* umami taste receptor subunit or sweet taste receptor subunit) (or G protein) and a tag sequence, and optionally a nucleic acid encoding a selectable marker may be introduced into selected host cells by well known methods. Gene activation sequences may be introduced into the cells in the a similar way using conventional methods well known in the art. The methods include but are not limited to transfection, viral delivery, protein or peptide mediated insertion, coprecipitation methods, lipid based delivery reagents (lipofection), cytofection, lipopolyamine delivery, dendrimer delivery reagents, electroporation or mechanical delivery. Examples of transfection reagents are GENEPORTER, GENEPORTER2, LIPOFECTAMINE, LIPOFECTAMINE 2000, FUGENE 6, FUGENE HD, TFX-10, TFX-20, TFX-50, OLIGOFECTAMINE, TRANSFAST, TRANSFECTAM, GENESHUTTLE, TROJENE, GENESILENCER, X-TREMEGENE, PERFECTIN, CYTOFECTIN, SIPORT, UNIFECTOR, SIFECTOR, TRANSIT-LT1, TRANSIT-LT2, TRANSIT-EXPRESS, IFECT, RNAI SHUTTLE, METAFECTENE, LYOVEC, LIPOTAXI, GENEERASER, GENEJUICE, CYTOPURE, JETSI, JETPEI, MEGAFECTIN, POLYFECT, TRANSMESSANGER, RNAiFECT, SUPERFECT, EFFECTENE, TF-PEI-KIT, CLONFECTIN, AND METAFECTINE. The taste receptor subunit (*e.g.,* umami taste receptor subunit or sweet taste receptor subunit) nucleic acid sequences (and potentially G protein encoding nucleic acids) may be integrated at different locations of the genome in the cell. The expression level of the introduced nucleic acids encoding the taste receptor subunits (*e.g.,* umami taste receptor subunits or sweet taste receptor subunits) and G proteins may vary based upon integration site.

Following introduction of the taste receptor subunit (*e.g.,* umami taste receptor subunit or sweet taste receptor subunit) coding sequences or the taste receptor (*e.g.,* umami taste receptor or sweet taste receptor) gene activation sequences into host cells (and optionally introducing or activating a G protein encoding sequence in those cells) and optional subsequent drug selection, molecular beacons (*e.g.,* fluorogenic probes) are introduced into the cells and cell sorting is used to isolate cells positive for their signals and thus the expression (RNA) of the desired nucleic acid sequences. The skilled worker will recognize that this sorting can be gated for any desired expression level. Multiple rounds of sorting may be carried out, if desired. In one embodiment, the flow cytometric cell sorter is a FACS machine. MACS (magnetic cell sorting) or laser ablation of negative cells using laser-enabled analysis and processing can also be used. According to this method, cells expressing sweet taste receptor subunits T1 R2 and T1 R3 (and optimally a G protein) or umami taste receptor subunits T1 R1 and T1 R3 (and optionally a G protein) are detected and recovered.

Signaling probes useful in this embodiment of the invention are known in the art. They are generally oligonucleotides comprising a sequence complementary to a target sequence and a signal emitting system so arranged that no signal is emitted when the probe is not bound to the target sequence and a signal is emitted when the probe binds to the target sequence. By way of a non-limiting illustration, the signaling probe may comprise a fluorophore and a quencher positioned in the probe so that the quencher and fluorophore are brought together in the unbound probe. Upon binding between the probe and the target sequence, the quencher and fluorophore separate, resulting in emission of signal. International publication WO/2005/079462, for example, describes a number of signaling probes that may be, and are preferably, used in the production of the cells and cell lines of this invention. Where tag sequences are used, the vector for each of the taste receptor subunit (*e.g.,* umami taste receptor subunit or sweet taste receptor subunit) (or optionally a G protein) can comprise the same or a different tag sequence. Whether the tag sequences are the same or different, the signaling probes may comprise different signal emitters, such as different colored fluorophores and the like so that (RNA) expression of each subunit (and G protein) may be separately detected. By way of illustration, the signaling probe that specifically detects sweet taste receptor T1 R2 mRNA or umami taste receptor T1 R1 mRNA can comprise a red fluorophore, the probe that detects the sweet/umami taste receptor T1 R3 subunit (RNA) can comprise a green fluorophore and, optionally, the probe that detects a G protein (RNA) can comprise a yellow fluorophore. Those of skill in the art will be aware of other means for differentially detecting the expression of the two (or optionally three) expressed RNAs with signaling probes in a transfected cell.

Nucleic acids encoding signaling probes may be introduced into the selected host cell by any of numerous means that will be well-known to those of skill in the art, including but not limited to transfection, coprecipitation methods, lipid based delivery reagents (lipofection), cytofection, lipopolyamine delivery, dendrimer delivery reagents, electroporation or mechanical delivery. Examples of transfection reagents are GENEPORTER, GENEPORTER2, LIPOFECTAMINE, LIPOFECTAMINE 2000, FUGENE 6, FUGENE HD, TFX-10, TFX-20, TFX-50, OLIGOFECTAMINE, TRANSFAST, TRANSFECTAM, GENESHUTTLE, TROJENE, GENESILENCER, X-TREMEGENE, PERFECTIN, CYTOFECTIN, SIPORT, UNIFECTOR, SIFECTOR, TRANSIT-LT1, TRANSIT-LT2, TRANSIT-EXPRESS, IFECT, RNAI SHUTTLE, METAFECTENE, LYOVEC, LIPOTAXI, GENEERASER, GENEJUICE, CYTOPURE, JETSI, JETPEI, MEGAFECTIN, POLYFECT, TRANSMESSANGER, RNAiFECT, SUPERFECT, EFFECTENE, TF-PEI-KIT, CLONFECTIN, AND METAFECTINE.

In one embodiment, the signaling probes are designed to be complementary to either a portion of the RNA encoding a taste receptor subunit (*e.g.,* umami taste receptor subunit or sweet taste receptor subunit) or to a portion of its 5' or 3' untranslated regions (or similar potions of the RNA encoding a G protein). Even if the signaling probe, designed to recognize a messenger RNA of interest, is able to detect endogenously expressed target sequences, the proportion of these sequences in comparison to the proportion of the sequence of interest produced by transfected or gene activated cells is such that the sorter is able to discriminate the two cell types.

In specific embodiments, signaling probes directed (*e.g.,* complementary) to other sequences within the same or other coding exons, non-coding introns or non-coding untranslated sequences can also be designed and used. In particular embodiments, signaling probes to components of signaling pathways including the signaling pathway of the sweet taste receptor (*e.g.,* T1 R2 and T1 R3) or the the umami taste receptor (*e.g.,* T1 R1 and T1 R3) can also be used.

The expression level of a taste receptor subunit (*e.g.,* umami taste receptor subunit or sweet taste receptor subunit) (and optionally the G protein) may vary from cell to cell or cell line to cell line. The expression level in a cell or cell line may also decrease over time due to epigenetic events such as DNA methylation and gene silencing and loss of transgene copies. These variations can be attributed to a variety of factors, for example, the copy number of the transgene taken up by the cell, the site of genomic integration of the transgene, and the integrity of the transgene following genomic integration. One may use FACS or other cell sorting methods (*i.e.,* MACS) to evaluate expression levels. Additional rounds of introducing signaling probes may be used, for example, to determine if and to what extent the cells remain positive over time for any one or more of the RNAs for which they were originally isolated.

In specific embodiments, cells with different absolute or relative fluorescence levels for at least one signaling probe can be isolated, for example by FACS, by gating subsets of cells with the suitable fluorescent levels relative to the entire cell population. For example, the top 5%, the top 10%, the top 15%, the top 20%, the top 25%, the top 30%, the top 35%, the top 40%, the top 45%, the top 50%, the top 55%, the top 60%, or the top 65%, of cells with the highest fluorescent signal for a particular signaling probe (or combination of signaling probes) can be gated and isolated by, *e.g.,* FACS. In other embodiments, the top 2% to 3%, the top 5% to 10%, the top 5% to 15%, the top 5% to 20%, the top 5% to 30%, the top 40% to 50%, the top 10% to 30%, the top 10% to 25%, or the top 10% to 50%, of cells with the highest fluorescent signal for a particular signaling probe (or combination of signaling probes) can be gated and isolated by, *e.g.,* FACS.

Once cells expressing the RNA for T1 R1 and T1 R3, or T1 R2 and T1 R3, (and optionally a G protein) are isolated, they may be cultured in media under any conditions for a length of time sufficient to produce and identify those cells stably expressing the subunits (and optionally G protein) (RNA or protein) and more preferably expressing a functional taste receptor (*e.g.,* umami taste receptor or sweet taste receptor) (and optionally G protein). In another embodiment of the invention, adherent cells can be adapted to suspension before or after cell sorting and isolating single cells. In one embodiment, isolated cells may be grown individually or pooled to give rise to populations of cells. Individual or multiple cells or cell lines may also be grown separately or pooled. If a pool of cells or cell lines is stably expressing the subunits and more preferably a functional taste receptor (*e.g.,* umami taste receptor or sweet taste receptor), it can be further fractionated until the cell or cell line or set of cells or cell lines having this characteristic is identified. This may make it easier to maintain large numbers of cells and cell lines without the requirements for maintaining each separately. Thus, a pool of cells or cell lines may be enriched for positive cells. An enriched pool is at least 50%, at least 60%, at least 70%, at least 80% or at least 90%, or 100% positive for the desired property or activity.

In a further aspect, the invention provides a method for producing the cells and cell lines of the invention. In one embodiment, the method comprises the steps of:

a) providing a plurality of cells that express mRNA encoding one or more taste receptor subunits, *e.g.,* umami taste receptor subunits or sweet taste receptor subunits, and optionally a G protein;
b) dispersing cells individually into individual culture vessels, thereby providing a plurality of separate cell cultures
c) culturing the cells under a set of desired culture conditions using automated cell culture methods characterized in that the conditions are substantially identical for each of the separate cell cultures, during which culturing the number of cells in each separate cell culture is normalized, and wherein the separate cultures are passaged on the same schedule;
d) assaying the separate cell cultures for at least one desired characteristic of the taste receptor, *e.g.,* umami taste receptor or sweet taste receptor, at least twice; and
e) identifying a separate cell culture that has the desired characteristic in both assays.

According to the method, the cells are cultured under a desired set of culture conditions. The conditions can be any desired conditions. Those of skill in the art will understand what parameters are comprised within a set of culture conditions. For example, culture conditions include but are not limited to: the media (Base media (DMEM, MEM, RPMI, serum-free, with serum, fully chemically defined, without animal-derived components), mono and divalent ion (sodium, potassium, calcium, magnesium) concentration, additional components added (amino acids, antibiotics, glutamine, glucose or other carbon source, HEPES, channel blockers, modulators of other targets, vitamins, trace elements, heavy metals, co-factors, growth factors, anti-apoptosis reagents), fresh or conditioned media, with HEPES, pH, depleted of certain nutrients or limiting (amino acid, carbon source)), level of confluency at which cells are allowed to attain before split/passage, feeder layers of cells, or gamma-irradiated cells, $CO_2$, a three gas system (oxygen, nitrogen, carbon dioxide), humidity, temperature, still or on a shaker, and the like, which will be well known to those of skill in the art.

The cell culture conditions may be chosen for convenience or for a particular desired use of the cells. Advantageously, the invention provides cells and cell lines that are optimally suited for a particular desired use. That is, in embodiments of the invention in which cells are cultured under conditions for a particular desired use, cells are selected that have desired characteristics under the condition for the desired use.

By way of illustration, if cells will be used in assays in plates where it is desired that the cells are adherent, cells that display adherence under the conditions of the assay may be selected. Similarly, if the cells will be used for protein production, cells may be cultured under conditions appropriate for protein production and selected for advantageous properties for this use.

In some embodiments, the method comprises the additional step of measuring the growth rates of the separate cell cultures. Growth rates may be determined using any of a variety of techniques means that will be well known to the skilled worker. Such techniques include but are not limited to measuring ATP, cell confluency, light scattering, optical density (*e.g.,* OD 260 for DNA). Preferably growth rates are determined using means that minimize the amount of time that the cultures spend outside the selected culture conditions.

In some embodiments, cell confluency is measured and growth rates are calculated from the confluency values. In some embodiments, cells are dispersed and clumps removed prior to measuring cell confluency for improved accuracy. Means for monodispersing cells are well-known and can be achieved, for example, by addition of a dispersing reagent to a culture to be measured. Dispersing agents are well-known and readily available, and include but are not limited to enzymatic dispering agents, such as trypsin, and EDTA-based dispersing agents. Growth rates can be calculated from confluency date using commercially available software for that purpose such as HAMILTON VECTOR. Automated confluency measurement, such as using an automated microscopic plate reader is particularly useful. Plate readers that measure confluency are commercially available and include but are not limited to the CLONE SELECT IMAGER (Genetix). Typically, at least 2 measurements of cell confluency are made before calculating a growth rate. The number of confluency values used to determine growth rate can be any number that is convenient or suitable for the culture. For example, confluency can be measured multiple times over *e.g.,* a week, 2 weeks, 3 weeks or any length of time and at any frequency desired.

When the growth rates are known, according to the method, the plurality of separate cell cultures are divided into groups by similarity of growth rates. By grouping cultures into growth rate bins, one can manipulate the cultures in the group together, thereby providing another level of standardization that reduces variation between cultures. For example, the cultures in a bin can be passaged at the same time, treated with a desired reagent at the same time, *etc.* Further, functional assay results are typically dependent on cell density in an assay well. A true comparison of individual clones is only accomplished by having them plated and assayed at the same density. Grouping into specific growth rate cohorts enables the plating of clones at a specific density that allows them to be functionally characterized in a high throughput format

The range of growth rates in each group can be any convenient range. It is particularly advantageous to select a range of growth rates that permits the cells to be passaged at the same time and avoid frequent renormalization of cell numbers. Growth rate groups can include a very narrow range for a tight grouping, for example, average doubling times within an hour of each other. But according to the method, the range can be up to 2 hours, up to 3 hours, up to 4 hours, up to 5 hours or up to 10 hours of each other or even broader ranges. The need for renormalization arises when the growth rates in a bin are not the same so that the number of cells in some cultures increases faster than others. To maintain substantially identical conditions for all cultures in a bin, it is necessary to periodically remove cells to renormalize the numbers across the bin. The more disparate the growth rates, the more frequently renormalization is needed.

In step d) the cells and cell lines may be tested for and selected for any physiological property including but not limited to: a change in a cellular process encoded by the genome; a change in a cellular process regulated by the genome; a change in a pattern of chromosomal activity; a change in a pattern of chromosomal silencing; a change in a pattern of gene silencing; a change in a pattern or in the efficiency of gene activation; a change in a pattern or in the efficiency of gene expression; a change in a pattern or in the efficiency of RNA expression; a change in a pattern or in the efficiency of RNAi expression; a change in a pattern or in the efficiency of RNA processing; a change in a pattern or in the efficiency of RNA transport; a change in a pattern or in the efficiency of protein translation; a change in a pattern or in the efficiency of protein folding; a change in a pattern or in the efficiency of protein assembly; a change in a pattern or in the efficiency of protein modification; a change in a pattern or in the efficiency of protein transport; a change in a pattern or in the efficiency of transporting a membrane protein to a cell surface change in growth rate; a change in cell size; a change in cell shape; a change in cell morphology; a change in % RNA content; a change in % protein content; a change in % water content; a change in % lipid content; a change in ribosome content; a change in mitochondrial content; a change in ER mass; a change in plasma membrane surface area; a change in cell volume; a change in lipid composition of plasma membrane; a change in lipid composition of nuclear envelope; a change in protein composition of plasma membrane; a change in protein; composition of nuclear envelope; a change in number of secretory vesicles; a change in number of lysosomes; a change in number of vacuoles; a change in the capacity or potential of a cell for: protein production, protein secretion, protein folding, protein assembly, protein modification, enzymatic modification of protein, protein glycosylation, protein phosphorylation, protein dephosphorylation, metabolite biosynthesis, lipid biosynthesis, DNA synthesis, RNA synthesis, protein synthesis, nutrient absorption, cell growth, mitosis, meiosis, cell division, to dedifferentiate, to transform into a stem cell, to transform into a pluripotent cell, to transform into a omnipotent cell, to transform into a stem cell type of any organ (*i.e.* liver, lung, skin, muscle, pancreas, brain, testis, ovary, blood, immune system, nervous system, bone, cardiovascular system, central nervous system, gastro-intestinal tract, stomach, thyroid, tongue, gall bladder, kidney, nose, eye, nail, hair, taste bud), to transform into a differentiated any cell type (*i.e.* muscle, heart muscle, neuron, skin, pancreatic, blood, immune, red blood cell, white blood cell, killer T-cell, enteroendocrine cell, taste, secretory cell, kidney, epithelial cell, endothelial cell, also including any of the animal or human cell types already listed that can be used for introduction of nucleic acid sequences), to uptake DNA, to uptake small molecules, to uptake fluorogenic probes, to uptake RNA, to adhere to solid surface, to adapt to serum-free conditions, to adapt to serum-free suspension conditions, to adapt to scaled-up cell culture, for use for large scale cell culture, for use in drug discovery, for use in high throughput screening, for use in a functional cell based assay, for use in calcium flux assays, for use in G protein reporter assays, for use in reporter cell based assays, for use in ELISA studies, for use in in vitro assays, for use in vivo applications, for use in secondary testing, for use in compound testing, for use in a binding assay, for use in panning assay, for use in an antibody panning assay, for use in imaging assays, for use in microscopic imaging assays, for use in multiwell plates, for adaptation to automated cell culture, for adaptation to miniaturized automated cell culture, for adaptation to large-scale automated cell culture, for adaptation to cell culture in multiwell plates (6, 12, 24, 48, 96, 384, 1536 or higher density), for use in cell chips, for use on slides, for use on glass slides, for microarray on slides or glass slides, for immunofluorescence studies, for use in protein purification, for use in biologics production, for use in the production of industrial enzymes, for use in the production of reagents for research,, for use in cell therapy, for use in implantation into animals or humans, for use in isolation of factors secreted by the cell, for preparation of cDNA libraries, for purification of RNA, for purification of DNA, for infection by pathogens, viruses or other agent, for resistance to infection by pathogens, viruses or other agents, for resistance to drugs, for suitability to be maintained under automated miniaturized cell culture conditions, for use in the production of protein for characterization, including: protein crystallography, stimulation of the immune system, antibody production or generation or testing of antibodies. Those of skill in the art will readily recognize suitable tests for any of the above-listed properties. In particular embodiments, one or more of these physical properties may be a constant physical property associated with a taste receptor (*e.g.,* sweet taste receptor or umami taste receptor) and can be used to monitor the expression of functional taste receptors (*e.g.,* sweet taste receptors or umami taste receptors).

Tests that may be used to characterize cells and cell lines of the invention and/or matched panels of the invention include but are not limited to: Amino acid analysis, DNA sequencing, Protein sequencing, NMR, A test for protein transport, A test for nucleocytoplasmic transport, A test for subcellular localization of proteins, A test for subcellular localization of nucleic acids, Microscopic analysis, Submicroscopic analysis, Fluorescence microscopy, Electron microscopy, Confocal microscopy, Laser ablation technology, Cell counting and Dialysis. The skilled worker would understand how to use any of the above-listed tests.

According to the method, cells may be cultured in any cell culture format so long as the cells or cell lines are dispersed in individual cultures prior to the step of measuring growth rates. For example, for convenience, cells may be initially pooled for culture under the desired conditions and then individual cells separated one cell per well or vessel.

Cells may be cultured in multi-well tissue culture plates with any convenient number of wells. Such plates are readily commercially available and will be well knows to a person of skill in the art. In some cases, cells may preferably be cultured in vials or in any other convenient format, the various formats will be known to the skilled worker and are readily commericially available.

In embodiments comprising the step of measuring growth rate, prior to measuring growth rates, the cells are cultured for a sufficient length of time for them to acclimate to the culture conditions. As will be appreciated by the skilled worker, the length of time will vary depending on a number of factors such as the cell type, the chosen conditions, the culture format and may be any amount of time from one day to a few days, a week or more.

Preferably, each individual culture in the plurality of separate cell cultures is maintained under substantially identical conditions a discussed below, including a standardized maintenance schedule. Another advantageous feature of the method is that large numbers of individual cultures can be maintained simultaneously, so that a cell with a desired set of traits may be identified even if extremely rare. For those and other reasons, according to the invention, the plurality of separate cell cultures are cultured using automated cell culture methods so that the conditions are substantially identical for each well. Automated cell culture prevents the unavoidable variability inherent to manual cell culture.

Any automated cell culture system may be used in the method of the invention. A number of automated cell culture systems are commercially available and will be well-known to the skilled worker. In some embodiments, the automated system is a robotic system. Preferably, the system includes independently moving channels, a multichannel head (for instance a 96-tip head) and a gripper or cherry-picking arm and a HEPA filtration device to maintain sterility during the procedure. The number of channels in the pipettor should be suitable for the format of the culture. Convenient pipettors have, *e.g.,* 96 or 384 channels. Such systems are known and are commercially available. For example, a MICROLAB START™ instrument (Hamilton) may be used in the method of the invention. The automated system should be able to perform a variety of desired cell culture tasks. Such tasks will be known by a person of skill in the art. They include but are not limited to: removing media, replacing media, adding reagents, cell washing, removing wash solution, adding a dispersing agent, removing cells from a culture vessel, adding cells to a culture vessel an the like.

The production of a cell or cell line of the invention may include any number of separate cell cultures. However, the advantages provided by the method increase as the number of cells increases. There is no theoretical upper limit to the number of cells or separate cell cultures that can be utilized in the method. According to the invention, the number of separate cell cultures can be two or more but more advantageously is at least 3, 4, 5, 6, 7, 8, 9, 10 or more separate cell cultures, for example, at least 12, at least 15, at least 20, at least 24, at least 25, at least 30, at least 35, at least 40, at least 45, at least 48, at least 50, at least 75, at least 96, at least 100, at least 200, at least 300, at least 384, at least 400, at least 500, at least 1000, at least 10,000, at least 100,000, at least 500,000 or more.

The cells and cell lines of the invention have enhanced stability as compared to cells and cell lines produced by conventional methods in the context of expression and expression levels (RNA or protein). To identify cells and cell lines characterized by such stable expression, a cell or cell line's expression of each taste receptor, *e.g.,* umami taste receptor or sweet taste receptor subunit, (and optionally G protein), is measured over a timecourse and the expression levels are compared. Stable cell lines will continue expressing (RNA or protein) sweet taste receptor T1 R2 and T1 R3 subunits, or umami taste receptor T1 R1 and T1 R3 subunits, (and optionally G protein) throughout the timecourse. In some aspects of the invention, the timecourse may be for at least one week, two weeks, three weeks, *etc.,* or at least one month, or at least two, three, four, five, six, seven, eight or nine months, or any length of time in between.

Isolated cells and cell lines may be further characterized, such as by qRT-PCR and single end-point RT-PCR to determine the absolute amounts and relative amounts of each taste receptor subunit, *e.g.,* umami taste receptor subunit or sweet taste receptor subunit, (or G protein) being expressed (RNA). Preferably, the expansion levels of the two subunits are substantially the same in the cells and cell lines of this invention.

In other embodiments, the expression of a functional taste receptor, *e.g.,* umami taste receptor or sweet taste receptor, (and G protein) is assayed over time. In these embodiments, stable expression is measured by comparing the results of functional assays over a timecourse. The assay of cell and cell line stability based on a functional assay provides the benefit of identifying cells and cell lines that not only stably express the T1 R1 and T1 R3 subunits of the umami taste receptor, or the T1 R2 and T1 R3 subunits of the sweet taste receptor, (and optionally the G protein) (RNA or protein), but also stably produce and properly process *(e.g.,* post-translational modification, subunit assembly, and localization within the cell) the subunits (and G protein) to produce a functional umami taste receptor or sweet taste receptor.

Cells and cell lines of the invention have the further advantageous property of providing assays with high reproducibility as evidenced by their Z' factor. See Zhang JH, Chung TD, Oldenburg KR, "A Simple Statistical Parameter for Use in Evaluation and Validation of High Throughput Screening Assays." J. Biomol. Screen. 1999;4(2):67-73. Z' values relate to the quality of a cell or cell line because it reflects the degree to which a cell or cell line will respond consistently to modulators. Z' is a statistical calculation that takes into account the signal-to-noise range and signal variability *(i.e.,* from well to well) of the functional response to a reference compound across a multiwell plate. Z' is calculated using data obtained from multiple wells with a positive control and multiple wells with a negative control. The ratio of their combined standard deviations multiplied by three to the difference in their mean values is subtracted from one to give the Z' factor, according the equation below:

$$Z' \text{ factor} = 1 - ((3\sigma_{\text{positive control}} + 3\sigma_{\text{negative control}}) / (\mu_{\text{positive control}} - \mu_{\text{negative control}}))$$

The theoretical maximum Z' factor is 1.0, which would indicate an ideal assay with no variability and limitless dynamic range. As used herein, a "high Z'" refers to a Z' factor of Z' of at least 0.6, at least 0.7, at least 0.75 or at least 0.8, or any decimal in between 0.6 and 1.0. In the case of a complex target such as taste receptor, *e.g.,* umami taste receptor or sweet taste receptor, a high Z' means a Z' of at least 0.4 or greater. A score of close to 0 is undesirable because it indicates that there is overlap between positive and negative controls. In the industry, for simple cell-based assays, Z' scores up to 0.3 are considered marginal scores, Z' scores between 0.3 and 0.5 are considered acceptable, and Z' scores above 0.5 are considered excellent. Cell-free or biochemical assays may approach higher Z' scores, but Z' scores for cell-based systems tend to be lower because cell-based systems are complex.

As those of ordinary skill in the art will recognize cell-based assays using conventional cells expressing even a single chain protein do not typically achieve a Z' higher than 0.5 to 0.6. Cells with engineered expression (either from introduced coding sequences or gene activation) of multi-subunit proteins, if even reported in the art, would be lower due to their added complexity. Such cells would not be reliable for use in assays because the results would not be reproducible. Cells and cell lines of this invention, on the other hand, have higher Z' values and advantageously produce consistent results in assays. Indeed, the taste receptor (*e.g.,* umami taste receptor or sweet taste receptor) expressing cells and cell lines of the invention provide the basis for high throughput screening (HTS) compatible assays because they generally have higher Z' values than conventionally produced cells. In some aspects of the invention, the cells and cell lines result in Z' of at least 0.3, at least 0.4, at least 0.5, at least 0.6, at least 0.7, or at least 0.8. Even Z' values of at least 0.3 - 0.4 for taste receptor (*e.g.,* umami taste receptor or sweet taste receptor)-expressing cell are advantageous because the taste receptor (*e.g.,* umami taste receptor or sweet taste receptor) is a multigene target. In other aspects of the invention, the cells and cell lines of the invention result in a Z' of at least 0.7, at least 0.75 or at least 0.8 even after the cells are maintained for multiple passages, *e.g.,* between 5-20 passages, including any integer in between 5 and 20. In some aspects of the invention, a Z' of at least 0.7, at least 0.75 or at least 0.8 is observed in cells and cell lines maintained for 1, 2, 3, 4 or 5 weeks or 2, 3, 4, 5, 6, 7, 8 or 9 months, including any period of time in between.

A further advantageous property of the taste receptor (*e.g.,* umami taste receptor or sweet taste receptor) cells and cell lines of the invention is that they stably express T1 R2 and T1 R3 subunits of the sweet taste receptor, or T1 R1 and T1 R3 subunits of the umami taste receptor, and optionally a G protein, in the absence of drug or other selective pressure. Thus, in preferred embodiments, the cells and cell lines of the invention are maintained in culture without any selective pressure. In further embodiments, cells and cell lines are maintained without any drug or antibiotics. As used herein, cell maintenance refers to culturing cells after they have been selected as described above for their sweet taste receptor subunit or umami taste receptor subunit (and optionally a G protein) expression. Maintenance does not refer to the optional step of growing cells under selective pressure (*e.g.,* an antibiotic) prior to cell sorting where marker(s) introduced into the cells allow enrichment of stable transfectants in a mixed population.

Drug-free and selective pressure-free cell maintenance of the cells and cell lines of this invention provides a number of advantages. For example, drug-resistant cells may not express the co-transfected transgene of interest at adequate levels, because the selection relies on survival of the cells that have taken up the drug resistant gene, with or without the transgene. Further, selective drugs and other selective pressure factors are often mutagenic or otherwise interfere with the physiology of the cells, leading to skewed results in cell-based assays. For example, selective drugs may decrease susceptibility to apoptosis (Robinson et al., Biochemistry, 36(37):11169-11178 (1997)), increase DNA repair and drug metabolism (Deffie et al., Cancer Res. 48(13):3595-3602 (1988)), increase cellular pH (Thiebaut et al., J Histochem Cytochem. 38(5):685-690 (1990); Roepe et al., Biochemistry. 32(41 ):11042-11056 (1993); Simon et al., Proc Natl Acad Sci USA. 91 (3):1128-1132 (1994)), decrease lysosomal and endosomal pH (Schindler et al., Biochemistry. 35(9):2811-2817 (1996); Altan et al., J Exp Med. 187(10):1583-1598 (1998)), decrease plasma membrane potential (Roepe et al., Biochemistry. 32(41):11042-11056 (1993)), increase plasma membrane conductance to chloride (Gill et al., Cell. 71(1):23-32 (1992)) and ATP (Abraham et al., Proc Natl Acad Sci U SA. 90(1):312-316 (1993)), and increase rates of vesicle transport (Altan et al., Proc Natl Acad Sci USA. 96(8):4432-4437 (1999)). Thus, the cells and cell lines of this invention allow screening assays that are free from the artifacts caused by selective pressure. In some preferred embodiments, the cells and cell lines of this invention are not cultured with selective pressure factors, such as antibiotics, before or after cell sorting, so that cells and cell lines with desired properties are isolated by sorting, even when not beginning with an enriched cell population.

In another aspect, the invention provides methods of using the cells and cell lines of the invention. The cells and cell lines of the invention may be used in any application for which functional taste receptor subunits (*e.g.,* umami taste receptor subunits or sweet taste receptor subunits) are needed. The cells and cell lines may be used, for example, in an *in vitro* cell-based assay or an *in vivo* assay where the cells are implanted in an animal (*e.g.,* a non-human mammal) to, *e.g.,* screen for taste receptor modulators (*e.g.,* umami taste receptor modulators or sweet taste receptor modulators); produce protein for crystallography and binding studies; and investigate compound selectivity and dosing, receptor/compound binding kinetic and stability, and effects of receptor expression on cellular physiology (*e.g.,* electrophysiology, protein trafficking, protein folding, and protein regulation). The cells and cell lines of the invention also can be used in knock down studies to examine the roles of specific taste receptor subunits (*e.g.,* umami taste receptor subunits or sweet taste receptor subunits).

Cells and cell lines expressing various combinations of subunits can be used separately or together to identify taste receptor modulators (*e.g.,* umami taste receptor modulators or sweet taste receptor modulators). The cells and cell lines may be used to identify the roles of different forms of a taste receptor (*e.g.,* umami taste receptor or sweet taste receptor) in different taste receptor pathologies by correlating the identity of *in vivo* forms of a taste receptor with the identity of known forms of taste receptors based on their response to various modulators. This allows selection of disease- or tissue-specific taste receptor modulators (*e.g.,* umami taste receptor modulators or sweet taste receptor modulators) for highly targeted treatment of such taste receptor-related pathologies.

To identify a taste receptor modulator (*e.g.,* umami taste receptor modulator or sweet taste receptor modulator), one exposes a cell or cell line of the invention to a test compound under conditions in which the taste receptor (*e.g.,* umami taste receptor or sweet taste receptor) would be expected to be functional and then detects a statistically significant change (*e.g.,* $p<0.05$) in taste receptor activity (*e.g.,* umami taste receptor activity or sweet taste receptor activity) compared to a suitable control, *e.g.,* cells that are not exposed to the test compound. Positive and/or negative controls using known agonists or antagonists and/or cells expressing different combinations of taste receptor subunits (*e.g.,* umami taste receptor subunits or sweet taste receptor subunits) (and optionally a G protein) may also be used. In some embodiments, the taste receptor activity (*e.g.,* umami taste receptor activity or sweet taste receptor activity) to be detected and/or measured is calcium release from the endoplasmic reticulum as a result of the down stream signaling events following taste receptor activation (*e.g.,* umami taste receptor activation or sweet taste receptor activation). One of ordinary skill in the art would understand that various assay parameters may be optimized, *e.g.,* signal to noise ratio.

In some embodiments, one or more cells or cell lines of the invention are exposed to a plurality of test compounds, for example, a library of test compounds. Such libraries of test compounds can be screened using the cell lines of the invention to identify one or more modulators of a taste receptor (*e.g.,* umami taste receptor or sweet taste receptor). The test compounds can be chemical moieties including small molecules, polypeptides, peptides, peptide mimetics, antibodies or antigen-binding portions thereof. In the case of antibodies, they may be non-human antibodies, chimeric antibodies, humanized antibodies, or fully human antibodies. The antibodies may be intact antibodies comprising a full complement of heavy and light chains or antigen-binding portions of any antibody, including antibody fragments (such as Fab and Fab, Fab', $F(ab')_2$, Fd, Fv, dAb and the like), single chain antibodies (scFv), single domain antibodies, all or an antigen-binding portion of a heavy chain or light chain variable region.

In some embodiments, prior to exposure to a test compound, the cells or cell lines of the invention may be modified by pretreatment with, for example, enzymes, including mammalian or other animal enzymes, plant enzymes, bacterial enzymes, protein modifying enzymes and lipid modifying enzymes. Such enzymes can include, for example, kinases, proteases, phosphatases, glycosidases, oxidoreductases, transferases, hydrolases, lyases, isomerases, ligases bacterial proteases, proteases from the gut, proteases from the GI tract, proteases in saliva, in the oral cavity, proteases from lysol cells/bacteria, and the like. Alternatively, the cells and cell lines may be exposed to the test compound first followed by enzyme treatment to identify compounds that alter the modification of the umami taste receptor or sweet taste receptor by the treatment.

In some embodiments, large compound collections are tested for taste receptor modulating activity (*e.g.,* umami taste receptor modulating activity or sweet taste receptor modulating activity) in a cell-based, functional, high-throughput screen (HTS), *e.g.,* using 96-well, 384-well, 1536-well or higher density formats. In some embodiments, a test compound or multiple test compounds, including a library of test compounds, may be screened using more than one cell or cell line of the invention. In the case of a cell or cell line of the invention that expresses a human sweet taste receptor, one can expose the cells or cell lines to a test compound to identify a compound that modulates sweet taste receptor activity (either increasing or decreasing) for use in the treatment of disease or condition characterized by undesired sweet taste receptor activity, or the decrease or absence of desired sweet taste receptor activity. Further, according to the methods of the invention, cells or cell lines of the invention can be used to identify compounds or substances that potentiate or inhibit sweet taste for use in ingestible substances.

In the case of a cell or cell line of the invention that expresses a human umami taste receptor, one can expose the cells or cell lines to a test compound to identify a compound that modulates umami taste receptor activity (either increasing or decreasing) for use in the treatment of disease or condition characterized by undesired umami taste receptor activity, or the decrease or absence of desired umami taste receptor activity. Further, according to the methods of the invention, cells or cell lines of the invention can be used to identify compounds or substances that potentiate or inhibit umami taste for use in ingestible substances.

In some embodiments, the cells and cell lines of the invention have increased sensitivity to modulators of a sweet taste receptor. For example, the cells and cell lines of this invention respond to all of the known classes of sweet compounds, *e.g.,* natural sweeteners (*e.g.,* glucose, fructose and sucrose); high intensity sweeteners (*e.g.,* steviva, rebaudioside A, mogroside); and artificial sweeteners (*e.g.,* asparatame, saccharin; and acesulfamek). *See, e.g.,* Figures 23 and 24. Cells and cell lines of the invention also respond to modulators and activate G proteins with a physiological range $EC_{50}$ or $IC_{50}$ values for sweet taste receptor.

In some embodiments, the cells and cell lines of the invention have increased sensitivity to modulators of an umami taste receptor. For example, the cells and cell lines of this invention respond to all of the known classes of umami compounds, *e.g.* MSG and sodium cyclamate. *See, e.g.,* Figures 9-12. Cells and cell lines of the invention also respond to modulators and activate G proteins with a physiological range $EC_{50}$ or $IC_{50}$ values for umami taste receptor.

As used herein, $EC_{50}$ refers to the concentration of a compound or substance required to induce a half-maximal activating response in the cell or cell line. As used herein, $IC_{50}$ refers to the concentration of a compound or substance required to induce a half-maximal inhibitory response in the cell or cell line. $EC_{50}$ and $IC_{50}$ values may be determined using techniques that are well-known in the art, for example, a dose-response curve that correlates the concentration of a compound or substance to the response of the umami taste receptor-expressing cell line or sweet taste receptor-expressing cell line.

A further advantageous property of the taste receptor (*e.g.,* umami taste receptor or sweet taste receptor) cells and cell lines of the invention is that modulators identified in initial screening using those cells and cell lines are functional in secondary functional assays, *e.g.,* sip and spit, taste testing and sensory evaluation. As those of ordinary skill in the art will recognize, compounds identified in initial screening assays typically must be modified, such as by combinatorial chemistry, medicinal chemistry or synthetic chemistry, for their derivatives or analogs to be functional in secondary functional assays. However, due to the high physiological relevance of the taste receptor (*e.g.,* umami taste receptor or sweet taste receptor) expressing cells and cell lines of this invention, many compounds identified using those cells and cell lines are functional without further modification.

In certain aspects, provided herein are methods that take advantage of the naturally occurring high degree of genetic diversity that exists in cells, and efficiently identify, select, and enrich for cells possessing desired gene expression profiles conferring desired properties (*e.g.,* stable and/or high expression of functional taste receptors, for example, sweet taste receptors, umami taste receptors, or bitter taste receptors). The present methods can identify, select, and enrich for cells with improved properties from a pool of genetically diverse cells faster and more efficiently than conventional methods. In particular embodiments, the cells have not been genetically modified. In other particular embodiments, the present methods allow for the generation of novel homogeneous populations of cells possessing improved properties (*e.g.,* more stable and/or higher expression of functional taste receptors, for example, sweet taste receptors, umami taste receptors, or bitter taste receptors).

In certain embodiments, the methods described herein comprise selecting naturally occurring cells with one or more desired properties (*e.g.,* stable and/or high expression of functional taste receptors, for example, sweet taste receptors, umami taste receptors, or bitter taste receptors). In specific embodiments, the methods described herein comprise selecting cells with naturally occurring variants or mutations in one or more taste receptor genes (*e.g.,* sweet taste receptor gene or umami taste receptor genes).

In specific embodiments, the methods described herein comprise selecting cells with naturally occurring variants or mutations in a promoter region of a gene or in a non-coding region of a gene (*e.g.,* intron, 5' untranslated region, and/or 3' untranslated region). Variants or mutations in a promoter region of a gene or in a non-coding region of a gene may result in higher and/or more stable expression of the gene product. In specific embodiments, a promoter region of a gene or in a non-coding region of a gene has been modified, for example by methylation or acetylation of DNA. In particular embodiments, a cell comprises epigenetic modification affecting chromatin remodeling with respect to one or more of the gene of interest. Non-limiting examples of epigenetic modifications include, but are not limited to, acetylation, methylation, ubiquitylation, phosphorylation and sumoylation.

In certain other embodiments, the present methods comprise selecting cells that underwent prior treatments. Such prior treatments may be exposure to sunlight or ultraviolet (UV) light, mutagens such as ethyl methane sulfonate (EMS), and chemical agents. In specific embodiments, such prior treatments may include exposure to undesirable growth conditions, *e.g.,* low oxygen or low nutrients conditions, or toxic conditions.

The methods described herein provide for identifying and/or selection of isolated cells (*e.g.,* eukaryotic cells) that express one or more genes of interest (*e.g.,* a taste receptor subunit gene, for example, sweet taste receptor subunit gene, umami taste receptor subunit gene, or bitter taste receptor subunit gene). In certain embodiments, the gene of interest is expressed at higher levels than other cells as a result of genetic variability. In particular embodiments, the methods described here comprise (a) introducing into a cell (*e.g.,* eukaryotic cell) one or more signaling probes that is capable of detecting an RNA of interest (*e.g.,* capable of hybridizing to a target sequence of an RNA of interest); and (b) determining whether the cell (*e.g.,* eukaryotic cells) comprises the RNA of interest. Such methods may further comprise quantifying the level of the RNA of interest. In specific embodiments, the methods described herein for identifying a cell with a desired RNA expression profile, wherein the method comprises: (a) introducing into a eukaryotic cell (*e.g.,* eukaryotic cell) a plurality of signaling probes each capable of detecting an RNA of interest; and (b) quantifying the RNA levels detected by the plurality of signaling probes. The desired gene expression profile may be determined by comparison to a reference population.

In particular embodiments, the methods described here comprise (a) introducing into a cell (*e.g.,* eukaryotic cell) one or more signaling probes that is capable of detecting an RNA of a taste receptor (*e.g.,* sweet taste receptor or umami taste receptor); and (b) determining whether the cell (*e.g.*, eukaryotic cells) comprises the RNA of a taste receptor (*e.g.,* sweet taste receptor or umami taste receptor). Such methods may further comprise quantifying the level of the RNA of a taste receptor (*e.g.,* sweet taste receptor or umami taste receptor). In specific embodiments, the methods described herein for identifying a cell with a desired RNA expression profile, wherein the method comprises: (a) introducing into a eukaryotic cell (*e.g.,* eukaryotic cell) a plurality of signaling probes each capable of detecting a plurality of RNAs of interest; and (b) quantifying the RNA levels detected by the plurality of signaling probes. The desired gene expression profile may be determined by comparison to a reference population. In particular embodiments, the plurality of RNAs of interest may comprise any combination of the following RNAs: RNA of T1 R1, RNA of T1 R2, RNA of T1 R3, RNA of a G protein, and RNA of a gene associated with a taste receptor. In certain embodiments, the plurality of RNAs of interest comprise the RNA of T1 R1 and the RNA of T1 R3 (and optionally RNA of a G protein). In some embodiments, the plurality of RNAs of interest comprise the RNA of T1 R2 and the RNA of T1 R3 (and optionally RNA of a G protein).

In specific embodiments, such method further comprises the step of comparing the quantified RNA levels of the cell with the RNA levels in a reference cell, respectively. In particular embodiments, the plurality of signaling probes comprises at least 2, 3, 4, 5, 6, 7, 8, 9, 10, 15, 20, 25, 30, 35, 40, 45, 50, 60, 70, 80, 90, 100, 125, 150, 175, 200, 250, 300, 350, 500, 600, 700, 800, 900, or at least 1000 signaling probes. In some embodiments, the RNA of interest is translated. In other embodiments, the RNA of interest is not translated. In specific embodiments, the RNA of interest is encoded by a taste receptor subunit gene (*e.g.,* sweet taste receptor subunit gene, umami taste receptor subunit gene, or bitter receptor subunit gene). In specific embodiments, the isolated cell (*e.g.,* eukaryotic cell) expresses one or more recombinant RNA of interest.

In specific embodiments, eukaryotic cells identified and/or selected by the methods described herein have not been genetically engineered (*e.g.,* do not recombinantly express one or more transgenes). In other embodiments, eukaryotic cells identified and/or selected by the methods described herein have been genetically engineered (*e.g.,* do recombinantly express one or more transgenes). In specific embodiments, such cells are somatic cells or differentiated cells.

In other embodiments, the cell comprises a desired gene expression profile. In certain embodiments, the desired gene expression profile is achieved by genetic engineering or by increasing genetic variability. In specific embodiments, a desired gene expression profile may be determined based on comparison with that of a reference population.

In some embodiments, the methods described herein are for identifying and/or selecting cells that express an RNA of interest at a level higher than the average heterologous cell population. In some embodiments, the methods described herein are for identifying and/or selecting cells that express an RNA of interest (*e.g.,* RNA of a sweet taste receptor or an umami taste receptor) at a level higher than the average heterologous cell population (*e.g.,* unsorted cell line population, for example unsorted 293T cell line population). In some embodiments, the methods described herein are for identifying and/or selecting cells that express an RNA of interest (*e.g.,* RNA of a sweet taste receptor or an umami taste receptor) at a level that is at least 20%, 30%, 40%, 50%, 60%, 70%, 80%, 90%, 95%, or 100% higher than the average heterologous cell population (*e.g.,* unsorted cell line population, for example unsorted 293T cell line population). In some embodiments, the methods described herein are for identifying and/or selecting cells that express an RNA of interest (*e.g.,* RNA of a sweet taste receptor or an umami taste receptor) at a level that is at least 1 fold, 1.5 fold, 2 fold, 3 fold, 4 fold, 5 fold, 6 fold, 7 fold, 8 fold, 9 fold, or 10 fold higher than the average heterologous cell population (*e.g.,* unsorted cell line population, for example unsorted 293T cell line population).

In specific embodiments, the methods described herein are for identifying and/or selecting cells that express an RNA of interest at a level lower than the average heterologous cell population. An exemplary heterologous cell population may be a cell population of mixed cell types of different origin, a cell population of cells of one cell type that are genetically heterologous, or a cell population of one particular cell line from which the isolated cell is obtained using the methods described herein.

In particular embodiments, a cell isolated using the methods described herein is a cell clone from a cell line. In certain embodiments, a cell isolated using the methods described herein is a primary cell. In some embodiments, a cell isolated using the methods described herein is a transformed cell.

In certain embodiments, a cell is not a human cell. In particular embodiments, a cell is a cell derived from a mouse, rat, monkey, dog, cat, pig, sheep, goat, horse, chicken, frog, worm, insect (*e.g.,* fly), fish, shellfish, or cow. In some embodiments, a cell is a mammalian cell or a eukaryotic cell. In other embodiments, a cell is a human cell. In some embodiments, a cell is a primary cell. In other embodiments, a cell is a transformed cell or a cell clone of a cell line.

In a specific aspect, provided herein is a method for isolating a cell that endogenously expresses a sweet taste receptor T1 R2 subunit and/or sweet taste receptor T1 R3 subunit, wherein the method comprises the steps of:

a) providing a population of cells;
b) introducing into the cells a signaling probe or molecular beacon that detects expression of T1 R2 and / or introducing into the cells a molecular beacon that detects expression of T1 R3; and
c) isolating cells that express a sweet taste receptor T1 R2 subunit and/or sweet taste receptor T1 R3 subunit.

In another specific aspect, provided herein is a method for isolating a cell that endogenously expresses a sweet taste receptor T1 R2 subunit and sweet taste receptor T1 R3 subunit, wherein the method comprises the steps of:

a) providing a population of cells;
b) introducing into the cells a molecular beacon that detects expression of T1 R2 and introducing into the cells a molecular beacon that detects expression of T1 R3; and
c) isolating cells that express a sweet taste receptor T1 R2 subunit and/or sweet taste receptor T1 R3 subunit.

In particular embodiments of such method, the population of cells is not known to express T1 R2 or T1 R3. In other specific embodiments of such method, any expression level of T1 R2 or T1 R3 in the isolated cell is at least 10 times, 50 times, 100 times, 250 times, 500 times, 750 times, 1000 times, 2500 times, 5000 times, 7500 times, 10000 times, 50000 times, or at least 100000 times higher than in the average cell of the population of cells. The expression level of a taste receptor subunit, for example T1 R2 or T1 R3, can be readily determined using methods known to one of skill in the art.

In a specific aspect, provided herein is a method for isolating a cell that endogenously expresses a umami taste receptor T1 R1 subunit and/or umami taste receptor T1 R3 subunit, wherein the method comprises the steps of:

a) providing a population of cells;
b) introducing into the cells a signaling probe or molecular beacon that detects expression of T1 R1 and / or introducing into the cells a molecular beacon that detects expression of T1 R3; and

c) isolating cells that express an umami taste receptor T1 R1 subunit and/or umami taste receptor T1 R3 subunit.

In another specific aspect, provided herein is a method for isolating a cell that endogenously expresses an umami taste receptor T1 R1 subunit and umami taste receptor T1 R3 subunit, wherein the method comprises the steps of:

a) providing a population of cells;

b) introducing into the cells a molecular beacon that detects expression of T1 R1 and introducing into the cells a molecular beacon that detects expression of T1 R3; and

c) isolating cells that express an umami taste receptor T1 R1 subunit and/or umami taste receptor T1 R3 subunit.

In particular embodiments of such method, the population of cells is not known to express T1 R1 or T1 R3. In other specific embodiments of such method, any expression level of T1 R1 or T1 R3 in the isolated cell is at least 10 times, 50 times, 100 times, 250 times, 500 times, 750 times, 1000 times, 2500 times, 5000 times, 7500 times, 10000 times, 50000 times, or at least 100000 times higher than in the average cell of the population of cells. The expression level of a taste receptor subunit, for example T1 R1 or T1 R3, can be readily determined using methods known to one of skill in the art.

Non-limiting examples of methods for determining protein expression level include immunoblotting (Western blotting), flow cytometry, or ELISA. Non-limiting examples of methods for determining RNA expression level include Northern blotting, RT-PCR, and real-time quantitative PCR. Such protein or RNA expression levels may be determined for for a population of cells generally to determine the average expression level in the population.

In other particular embodiments of such method, genetic variability in the population of cells had been increased prior to said isolating step. In particular embodiments, provided herein is an isolated cell generated according to such method.

<u>Bitter Taste Receptor</u>

This application discloses novel cells and cell lines that have been engineered to express one or more bitter receptors. Optionally, the novel cells or cell lines disclosed herein express one or more functional bitter receptors. Also disclosed herein are methods of making and using the novel cells and cell lines.

Optionally, the novel cells and cell lines are transfected with a nucleic acid encoding a native bitter receptor. Further optionally the novel cells and cell lines are transfected with a nucleic acid encoding an allelic variant (*i.e.,* a polymorphism) of a native bitter receptor, or a mutant bitter receptor. The novel cell lines disclosed herein stably express the introduced bitter receptor. Further optionally, the novel cells and cell lines have a bitter receptor activated for expression by gene activation.

Optionally, the novel cells and cell lines express an endogenous bitter receptor as a result of engineered gene activation, *i.e.,* activation of the expression of an endogenous gene, wherein the activation does not naturally occur in a cell without proper treatment. Engineered gene activation may turn on the expression of an endogenous bitter receptor, for example, where the endogenous bitter receptor is not expressed in the cell line without the proper treatment. Alternatively, engineered gene activation may result in increased expression level of the endogenous bitter receptor, for example, where the expression level of the endogenous gene in the cell line is undesirably low without the proper treatment, for example, not sufficient for functional assay of the bitter receptor in the cell line. Alternatively, engineered gene activation may be used to over-express an endogenous bitter receptor, for example, for isolating the endogenous bitter receptor from the cell line. Engineered gene activation can be achieved by a number of means known to those skilled in the art. For example, one or more transcription factors or transactivators of transcription of a gene can be over-expressed or induced to express by, *e.g.,* introducing nucleic acids expressing the transcription factors or transactivators into a cell under the control of a constitutive or inducible promoter. If the endogenous gene is known to be under the control of an inducible promoter, expression can be induced by exposing the cell to a known inducer of the gene. In addition, a nucleic acid encoding the endogenous gene itself can be introduced into a cell to obtain an increased level of expression of the gene due to increased copy number in the genome. Furthermore, certain known inhibitors of the expression of an endogenous gene that are expressed by the cell can be knocked down or even knocked out in the cell using techniques well known in the art, *e.g.,* RNAi, thereby increasing the expression of the endogenous gene.

The cells and cell lines disclosed herein comprising a bitter receptor, a mutant form thereof, or a naturally-occurring allelic variant thereof, can be used to identify modulators of bitter receptor function, including modulators that are specific for a particular bitter receptor mutant form or naturally-occurring allelic variant. The cells and cell lines can thus be used to obtain information about the properties, activities and roles of individual native or mutant forms or naturally-occurring allelic variants of bitter receptors and to identify bitter receptor modulators with activity for a particular native or mutant form or naturally-occurring allelic variant or for a subset of native or mutant forms or naturally-occurring allelic variants. These modulators are useful as therapeutics that target differentially modified bitter receptor forms in disease states or tissues. Because the polymorphism of bitter receptors *in vivo*, for example, may contribute to an undesired activity or disease state, cells and cell lines of this invention also can be used to screen for modulators for therapeutic use where alteration of the response of a mutant form or naturally-occurring allelic variant may be desired. The cells and cell lines are also useful to identify modulators that have activity with only subset of native or mutant forms or naturally-occurring allelic variants of a bitter receptor.

This disclosure herein also identifies and solves a difficulty in generating stable bitter receptor expressing cells and cell lines. As disclosed herein, we have discovered that expression of tagged bitter receptors led to incorrect assignment of bitter receptor agonists. It has been thought that specific tags, signal sequences, and/or chaperones were essential for the expression and/or transportation of the bitter receptors to the cell surface (Reichling, Meyerhof and Behrens, J. Neurochem. 106:1138-1148, 2008). This creates a dilemma and may lead to the identification of physiologically irrelevant modulators of tagged receptors. Accordingly, the physiologically relevant cell lines of the present invention can also facilitate identification of the ligands for orphan bitter receptors and dissection of the specificity of the various receptor-ligand interactions.

Disclosed herein are cells and cell lines that stably express one or more bitter receptors. Optionally, the expressed bitter receptors increase intracellular free calcium upon activation by an agonist. Optionally, a potentiator, agonist or activator can be a small molecule, a chemical moiety, a polypeptide, an antibody, or a food extract. Further optionally, the expressed bitter receptors decrease intracellular free calcium upon inhibition by an antagonist. Optionally, an inhibitor, antagonist or blocker can be a small molecule, a chemical moiety, a polypeptide, an antibody, or a food extract. A potentiator, agonist, activator, inhibitor, antagonist or blocker may act upon all or upon a specific subset of bitter receptors. Further optionally, the bitter receptor expressing cells and cell lines disclosed herein have enhanced properties compared to cells and cell lines made by conventional methods. For example, the bitter receptor expressing cells and cell lines have enhanced stability of expression (even when maintained in culture without selective antibiotics) and result in high Z' values. Also disclosed herein are methods of making and using the bitter receptor expressing cells and cell lines. ,

Optionally, the cell or cell line disclosed herein expresses a bitter receptor at a consistent level of expression for at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 110, 120, 130, 140, 150, 160, 170, 180, 190, 200 days or over 200 days, where consistent expression refers to a level of expression that does not vary by more than:

1 %, 2%, 3%, 4%, 5%, 6%, 7%, 8% 9% or 10% over 2 to 4 days of continuous cell culture,; 2%, 4%, 6%, 8%, 10% or 12% over 5 to 15 days of continuous cell culture; 2%, 4%, 6%, 8%, 10%, 12%, 14%, 16%, 18% or 20% over 16 to 20 days of continuous cell culture; 2%, 4%, 6%, 8%, 10%, 12%, 14%, 16%, 18%, 20%, 22%, 24% over 21 to 30 days of continuous cell culture; 2%, 4%, 6%, 8%, 10%, 12%, 14%, 16%, 18%, 20%, 22%, 24%, 26%, 28% or 30% over 30 to 40 days of continuous cell culture; 2%, 4%, 6%, 8%, 10%, 12%, 14%, 16%, 18%, 20%, 22%, 24%, 26%, 28% or 30% over 41 to 45 days of continuous cell culture; 2%, 4%, 6%, 8%, 10%, 12%, 14%, 16%, 18%, 20%, 22%, 24%, 26%, 28% or 30% over 45 to 50 days of continuous cell culture; 2%, 4%, 6%, 8%, 10%, 12%, 14%, 16%, 18%, 20%, 22%, 24%, 26%, 28%, 30% or 35% over 45 to 50 days of continuous cell culture; 2%, 4%, 6%, 8%, 10%, 12%, 14%, 16%, 18%, 20%, 22%, 24%, 26%, 28% or 30% over 50 to 55 days of continuous cell culture; 2%, 4%, 6%, 8%, 10%, 12%, 14%, 16%, 18%, 20%, 22%, 24%, 26%, 28%, 30% or 35% over 50 to 55 days of continuous cell culture; 1%, 2%, 3%, 4%, 5%, 10%, 15%, 20%, 25%, 30%, 35% or 40% over 55 to 75 days of continuous cell culture;1%, 2%, 3%, 4%, 5%, 6%, 10%, 15%, 20%, 25%, 30%, 35%, 40% or 45% over 75 to 100 days of continuous cell culture; 1%, 2%, 3%, 4%, 5%, 6%, 10%, 15%, 20%, 25%, 30%, 35%, 40% or 45% over 101 to 125 days of continuous cell culture; 1%, 2%, 3%, 4%, 5%, 6%, 10%, 15%, 20%, 25%, 30%, 35%, 40% or 45% over 126 to 150 days of continuous cell culture; 1%, 2%, 3%, 4%, 5%, 6%, 10%, 15%, 20%, 25%, 30%, 35%, 40% or 45% over 151 to 175 days of continuous cell culture; 1%, 2%, 3%, 4%, 5%, 6%, 10%, 15%, 20%, 25%, 30%, 35%, 40% or 45% over 176 to 200 days of continuous cell culture; 1%, 2%, 3%, 4%, 5%, 6%, 10%, 15%, 20%, 25%, 30%, 35%, 40% or 45% over more than 200 days of continuous cell culture.

The bitter receptor expressed by a cell or cell line can be from any mammal, including rat, mouse, rabbit, goat, dog, cow, pig or primate. Preferably, the bitter receptor is human bitter receptor.

Optionally, a cell or cell line disclosed herein may comprise: a nucleotide sequence (SEQ ID NO: 51) that encodes a human TAS2R1; a nucleotide sequence (SEQ ID NO: 52) that encodes a human TAS2R3; a nucleotide sequence (SEQ ID NO: 53) that encodes a human TAS2R4; a nucleotide sequence (SEQ ID NO: 54) that encodes a human TAS2R5; a nucleotide sequence (SEQ ID NO: 55) that encodes a human TAS2R7; a nucleotide sequence (SEQ ID NO: 56) that encodes a human TAS2R8; a nucleotide sequence (SEQ ID NO: 57) that encodes a human TAS2R9; a nucleotide sequence (SEQ ID NO: 58) that encodes a human TAS2R10; a nucleotide sequence (SEQ ID NO: 59) that encodes a human TAS2R13; a nucleotide sequence (SEQ ID NO: 60) that encodes a human TAS2R14; a nucleotide sequence (SEQ ID NO: 61) that encodes a human TAS2R16; a nucleotide sequence (SEQ ID NO: 62) that encodes a human TAS2R38; a nucleotide sequence (SEQ ID NO: 63) that encodes a human TAS2R39; a nucleotide sequence (SEQ ID NO: 64) that encodes a human TAS2R40; a nucleotide sequence (SEQ ID NO: 65) that encodes a human TAS2R41; a nucleotide sequence (SEQ ID NO: 66) that encodes a human TAS2R43; a nucleotide sequence (SEQ ID NO: 67) that encodes a human TAS2R44; a nucleotide sequence (SEQ ID NO: 68) that encodes a human TAS2R45; a nucleotide sequence (SEQ ID NO: 69) that encodes a human TAS2R46; a nucleotide sequence (SEQ ID NO: 70) that encodes a human TAS2R47; a nucleotide sequence (SEQ ID NO: 71) that encodes a human TAS2R48; a nucleotide sequence (SEQ ID NO: 72) that encodes a human TAS2R49; a nucleotide sequence (SEQ ID NO: 73) that encodes a human TAS2R50; a nucleotide sequence (SEQ ID NO: 74) that encodes a human TAS2R55; a nucleotide sequence (SEQ ID NO: 75) that encodes a human TAS2R60; or any combination thereof.

Optionally, a cell or cell line disclosed herein may comprise: a polynucleotide sequence for: human TAS2R1 (SEQ ID NO: 77); human TAS2R3 (SEQ ID NO: 78); human TAS2R4 (SEQ ID NO: 79); human TAS2R5 (SEQ ID NO: 80); human TAS2R7 (SEQ ID NO: 81); human TAS2R8 (SEQ ID NO: 82); human TAS2R9 (SEQ ID NO: 83); human TAS2R10 (SEQ ID NO: 84); human TAS2R13 (SEQ ID NO: 85); human TAS2R14 (SEQ ID NO: 86); human TAS2R16 (SEQ ID NO: 87); human TAS2R38 (SEQ ID NO: 88); human TAS2R39 (SEQ ID NO: 89); human TAS2R40 (SEQ ID NO: 90); human TAS2R41 (SEQ ID NO: 91); human TAS2R43 (SEQ ID NO: 92); human TAS2R44 (SEQ ID NO: 93); human TAS2R45 (SEQ ID NO: 94); human TAS2R46 (SEQ ID NO: 95); human TAS2R47 (SEQ ID NO: 96); human TAS2R48 (SEQ ID NO: 97); human TAS2R49 (SEQ ID NO: 98); human TAS2R50 (SEQ ID NO: 99); human TAS2R55 (SEQ ID NO: 100); human TAS2R60 (SEQ ID NO: 101); or any combination thereof.

Nucleic acids encoding bitter receptors can be genomic DNA or cDNA. Optionally, the nucleic acids comprise one or more mutations, as compared to the nucleic acid sequences encoding wild type bitter receptors, that may or may not result in an amino acid substitution. Further optionally, the nucleic acids comprise one or more naturally-occurring allelic variants, as compared to the most frequently occurring nucleic acid sequences encoding a certain bitter receptor in a given population. Naturally-occurring allelic variants" include different amino acid sequences of a same bitter receptor that are naturally-occurring, *e.g.,* those observed in a given population due to allelic variation or polymorphism.

Polymorphism is a common phenomenon in the human genome. Polymorphisms occurring within or near the bitter receptor genes may affect their expression or change their function by, *e.g.,* up-regulating or down-regulating their expression levels or by changing their amino acid sequences. Table 20 shows reference numbers for unique polymorphisms, including single nucleotide polymorphisms ("SNPs") related to human TAS2R genes, position of the SNPs in each reference sequence, and description of the SNPs. The reference numbers are searchable in the Single Nucleotide Polymorphism database ("dbSNP") of the National Center for Biotechnology Information ("NCBI"; Bethesda, MD).

Allelic variations of human bitter receptor genes resulting in coding sequence diversity have been studied and documented. See, *e.g.,* Ueda et al., "Identification of coding single-nucleotide polymorphisms in human taste receptor genes involving bitter tasting", Biochem Biophys Res Commun 285:147-151, 2001; Wooding et al., "Natural selection and molecular evolution in PTC, a bitter-taste receptor gene," Am. J. Hum, Genet. 74:637-646, 2004; and Kim et al., "Worldwide haplotype diversity and coding sequence variation at human bitter taste receptor loci", Human Mutation 26:199-204, 2005. Table 21 is a list of natural variations in the coding sequences of different human bitter receptors. The human bitter receptors, SEQ ID NOS of their coding sequences, and the protein sequences are listed in the first three columns. The nucleotide changes and their positions within each coding sequence as identified by their SEQ ID NOS are indicated in the columns under "Nucleotide change" and "Position of nucleotide change," respectively. The amino acid changes within each bitter receptor as identified by their SEQ ID NOS are indicated in the column under "Description" using single-letter abbreviations. Their positions with reference to each corresponding SEQ ID NO are indicated in the column under "Position of amino acid change." In addition, the "Description" column also contains identifiers of those variations that are searchable in dbSNP of NCBI. "Feature identifiers" are unique and stable feature identifiers assigned to some of the variations by the UniProt Protein Knowledgebase hosted by the European Bioinformatics Institute (Cambridge, United Kingdom). They are searchable within UniProt. "NA" denotes no feature identifiers assigned by UniProt yet.

Variation in human taste is a well-known phenomenon. Without wishing to be bound by theory, the variation of bitter taste may be related to polymorphisms of the bitter receptors. For example, polymorphisms in the hTAS2R38, a receptor for phenylthiocarbamide (PTC), has been linked to the ability to detect propylthiouracil (PROP) (Kim et al., "Positional cloning of the human quantitative trait locus underlying taste sensitivity to phenylthiocarbamide", Science 299:1221-1225, 2003; Wooding *et al.,* 2004). Certain polymorphisms in the hT2R43 gene allele make people very sensitive to the bitterness of the natural plant compounds aloin and aristolochic acid. People who do not possess this allele do not taste these compounds at low concentrations. Certain variations of the same hTAS2R43 gene allele make people more sensitive to the bitterness of saccharin. In addition, certain variations of a closely related gene's (hTAS2R44's) allele also make people more sensitive to the bitterness of saccharin. Furthermore, some people do not possess certain hTAS2R genes, which contribute to taste variation between individuals. Polymorphisms in bitter genes have also been linked to increased risk of certain diseases. Cell lines stably expressing a heterologous naturally-occurring bitter receptor, or an alleleic variant or polymorph thereof, or a mutant form thereof having one or more mutations (*e.g.,* random mutations or site-specific mutations) that are not naturally-occurring, are all within the scope of the present disclosure.

Optionally, the nucleic acid is a fragment of the nucleic acid sequences provided hereinabove. Such bitter receptors that are fragments or have such modifications retain at least one biological property of a bitter receptor, e.g.,

its ability to increase intracellular free calcium. The disclosure herein encompasses cells and cell lines stably expressing a bitter receptor-encoding nucleotide sequence that is at least about 85% identical to a sequence disclosed herein. Optionally, the subunit-encoding sequence identity is at least 85%, 90%, 95%, 96%, 97%, 98%, 99% or higher compared to a subunit sequence provided herein. The disclosure herein also encompasses cells and cell lines wherein a nucleic acid encoding a bitter receptor hybridizes under stringent conditions to a nucleic acid provided herein encoding the corresponding bitter receptor.

Optionally, the cell or cell line comprises a bitter receptor-encoding nucleic acid sequence comprising a substitution compared to a sequence provided herein by at least one but less than 10, 20, 30, or 40 nucleotides, up to or equal to 1 %, 5%, 10% or 20% of the nucleotide sequence or from a sequence substantially identical thereto (*e.g.*, a sequence at least 85%, 90%, 95%, 96%, 97%, 98%, 99% or higher identical thereto, or that is capable of hybridizing under stringent conditions to the sequences disclosed). Optionally, the cell or cell line comprises a bitter receptor-encoding nucleic acid sequence comprising an insertion into or deletion from the sequences provided herein by less than 10, 20, 30, or 40 nucleotides up to or equal to 1 %, 5%, 10% or 20% of the nucleotide sequence or from a sequence substantially identical thereto (e.g., a sequence at least 85%, 90%, 95%, 96%, 97%, 98%, 99% or higher identical thereto, or that is capable of hybridizing under stringent conditions to the sequences disclosed). The substitutions, insertions and deletions described herein may occur in any of the polynucleotides encoding bitter receptors in the cells or cell lines disclosed herein.

Optionally, where the nucleic acid substitution or modification results in an amino acid change, such as an amino acid substitution, the native amino acid may be replaced by a conservative or non-conservative substitution. Optionally, the sequence identity between the original and modified polypeptide sequence can differ by about 1%, 5%, 10% or 20% of the polypeptide sequence or from a sequence substantially identical thereto (*e.g.,* a sequence at least 85%, 90%, 95%, 96%, 97%, 98%, 99% or higher identical thereto). Those of skill in the art will understand that a conservative amino acid substitution is one in which the amino acid side chains are similar in structure and/or chemical properties and the substitution should not substantially change the structural characteristics of the parent sequence. In cases comprising a nucleic acid comprising a mutation, the mutation may be a random mutation or a site-specific mutation.

Conservative modifications will produce bitter receptors having functional and chemical characteristics similar to those of the unmodified bitter receptor. A "conservative amino acid substitution" is one in which an amino acid residue is substituted by another amino acid residue having a side chain R group with similar chemical properties to the parent amino acid residue (*e.g.,* charge or hydrophobicity). In general, a conservative amino acid substitution will not substantially change the functional properties of a protein. In cases where two or more amino acid sequences differ from each other by conservative substitutions, the percent sequence identity or degree of similarity may be adjusted upwards to correct for the conservative nature of the substitution. Means for making this adjustment are well-known to those of skill in the art. *See, e.g.,* Pearson, Methods Mol. Biol. 243:307-31 (1994).

Examples of groups of amino acids that have side chains with similar chemical properties include 1) aliphatic side chains: glycine, alanine, valine, leucine, and isoleucine; 2) aliphatic-hydroxyl side chains: serine and threonine; 3) amide-containing side chains: asparagine and glutamine; 4) aromatic side chains: phenylalanine, tyrosine, and tryptophan; 5) basic side chains: lysine, arginine, and histidine; 6) acidic side chains: aspartic acid and glutamic acid; and 7) sulfur-containing side chains: cysteine and methionine. Preferred conservative amino acids substitution groups are: valine-leucine-isoleucine, phenylalanine-tyrosine, lysine-arginine, alanine-valine, glutamate-aspartate, and asparagine-glutamine. Alternatively, a conservative amino acid substitution is any change having a positive value in the PAM250 log-likelihood matrix disclosed in Gonnet et al., Science 256:1443-45 (1992). A "moderately conservative" replacement is any change having a nonnegative value in the PAM250 log-likelihood matrix.

Optionally, the bitter receptor-encoding nucleic acid sequence further comprises a tag. Such tags may encode, for example, a HIS tag, a myc tag, a hemagglutinin (HA) tag, protein C, VSV-G, FLU, yellow fluorescent protein (YFP), green fluorescent protein (GFP), FLAG, BCCP, maltose binding protein tag, Nus-tag, Softag-1, Softag-2, Strep-tag, S-tag, thioredoxin, GST, V5, TAP or CBP. A tag may be used as a marker to determine bitter receptor expression levels, intracellular localization, protein-protein interactions, bitter receptor regulation, or bitter receptor function. Tags may also be used to purify or fractionate bitter receptor. Optionally, a tag is cleavable from the bitter receptor to which it tags. This can be achieved by, *e.g.,* introducing a specific protease cleavage site between the tag and the bitter receptor in the nucleic acid encoding the bitter receptor, and subjecting the tagged bitter receptor expressed by the nucleic acid to the treatment of the specific protease.

Host cells used to produce a cell or cell line of the invention may express in their native state one or more endogenous bitter receptor or lack expression of any bitter receptor. In the case where the cell or cell line expresses one or more of its own bitter receptors, also referred to as "endogenous" bitter receptors, the heterologous bitter receptor can be the same as one of the cell or cell line's endogenous bitter receptor(s). For example, a nucleic acid encoding an bitter receptor endogenous to a cell or cell line may be introduced into the cell or the cell line to increase the copy number of the gene encoding the bitter receptor in the cell or the cell line so that the bitter receptor is expressed at a higher level in the cell or cell line than without the introduced nucleic acid. The host cell may be a primary, germ, or stem cell, including an embryonic stem cell. The host cell may also be an immortalized cell. Primary or immortalized host cells may be derived from mesoderm, ectoderm or endoderm layers of eukaryotic organisms. The host cell may be endothelial, epidermal, mesenchymal, neural, renal, hepatic, hematopoietic, or immune cells. For example, the host cells may be intestinal crypt or villi cells, clara cells, colon cells, intestinal cells, goblet cells, enterochromafin cells, enteroendocrine cells. The host cells may be eukaryotic, prokaryotic, mammalian, human, primate, bovine, porcine, feline, rodent, marsupial, murine or other cells. The host cells may also be nonmammalian, such as yeast, insect, fungus, plant, lower eukaryotes and prokaryotes. Such host cells may provide backgrounds that are more divergent for testing bitter receptor modulators with a greater likelihood for the absence of expression products provided by the cell that may interact with the target. Preferably, the host cell is a mammalian cell. Examples of host cells that may be used to produce a cell or cell line disclosed herein include but are not limited to: human embryonic kidney 293T cells, established neuronal cell lines, pheochromocytomas, neuroblastomas fibroblasts, rhabdomyosarcomas, dorsal root ganglion cells, NS0 cells, CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171), L-cells, HEK-293 (ATCC CRL1573) and PC12 (ATCC CRL-1721), HEK293T (ATCC CRL-11268), RBL (ATCC CRL-1378), SH-SY5Y (ATCC CRL-2266), MDCK (ATCC CCL-34), SJ-RH30 (ATCC CRL-2061), HepG2 (ATCC HB-8065), ND7/23 (ECACC 92090903), CHO (ECACC 85050302), Vero (ATCC CCL 81), Caco-2 (ATCC HTB 37), K562 (ATCC CCL 243), Jurkat (ATCC TIB-152), Per.C6 (Crucell, Leiden, The Netherlands), Huvec (ATCC Human Primary PCS 100-010, Mouse CRL 2514, CRL 2515, CRL 2516), HuH-7D12 (ECACC 01042712), 293 (ATCC CRL 10852), A549 (ATCC CCL 185), IMR-90 (ATCC CCL 186), MCF-7 (ATC HTB-22), U-2 OS (ATCC HTB-96), T84 (ATCC CCL 248), or any established cell line (polarized or nonpolarized) or any cell line available from repositories such as American Type Culture Collection (ATCC, 10801 University Blvd. Manassas, Va. 20110-2209 USA) or European Collection of Cell Cultures (ECACC, Salisbury Wiltshire SP4 0JG England).

Optionally, the host cell is an embryonic stem cell that is then used as the basis for the generation of transgenic animals. Optionally one or more bitter receptors may be expressed with desired temporal and/or tissue specific expression. Embryonic stem cells stably expressing at least one bitter receptor, and preferably a functional heterologous bitter receptor, may be implanted into organisms directly, or their nuclei may be transferred into other recipient cells and these may then be implanted, or they may be used to create transgenic animals.

As will be appreciated by those of skill in the art, any vector that is suitable for use with the host cell may be used to introduce a nucleic acid encoding a bitter receptor into the host cell. Examples of vectors that may be used to introduce the bitter receptor encoding nucleic acids into the host cell include but are not limited to plasmids, viruses, including retroviruses and lentiviruses, cosmids, artificial chromosomes and may include, for example, pCMVScript, pcDNA3.1 Hygro, pcDNA3.1 neo, pcDNA3.1 puro, pSV2neo, pIRES puro, pSV2 zeo, pFN11 A (BIND) Flexi®, pGL4.31, pFC14A (HaloTag® 7) CMV Flexi®, pFC14K (HaloTag® 7) CMV Flexi®, pFN24A (HaloTag® 7) CMVd3 Flexi®, pFN24K (HaloTag® 7) CMVd3 Flexi®,HaloTag™ pHT2, pACT, pAdVAntage™, pALTER®-MAX, pBIND, pCAT®3-Basic, pCAT®3-Control, pCAT®3-Enhancer, pCAT®3-Promoter, pCI, pCMVTNT™, pG5luc, pSI, pTARGET™, pTNT™, pF12A RM Flexi®, pF12K RM Flexi®, pReg neo, pYES2/GS, pAd/CMV/V5-DEST Gateway® Vector, pAd/PL-DEST™ Gateway® Vector, Gateway® pDEST™27 Vector, Gateway® pEF-DEST51 Vector, Gateway® pcDNAT™-DEST47 vector, pCMV/Bsd Vector, pEF6/His A, B, & c,pcDNA™6.2-DEST, pLenti6/TR, pLP-AcGFP1-C, pLPS-AcGFP1-N, pLP-IRESneo, pLP-TRE2, pLP-RevTRE, pLP-LNCX, pLP-CMV-HA, pLP-CMV-Myc, pLP-RetroQ, pLP-CMVneo. In some embodiments, the vectors comprise expression control sequences such as constitutive or conditional promoters. One of ordinary skill in the art will be able to select such sequences. For example, suitable promoters include but are not limited to CMV, TK, SV40 and EF-1$\alpha$. Optionally, the promoters are inducible, temperature regulated, tissue specific, repressible, heat-shock, developmental, cell lineage specific, or temporal promoters or a combination or recombination of unmodified or mutagenized, randomized, shuffled sequences of any one or more of the above. Further optionally, bitter receptors are expressed by gene activation or when a gene encoding a bitter receptor is episomal. RNAs encoding bitter receptors or mutant forms or naturally-occurring allelic variants thereof are preferably constitutively expressed.

Optionally, the vector lacks a selectable marker or drug resistance gene. Further optionally, the vector optionally comprises a nucleic acid encoding a selectable marker such as a protein that confers drug or antibiotic resistance. Selection pressure may be applied in cell culture to select cells with desired sequences or traits, and is usually achieved by linking the expression of a polypeptide of interest with the expression of a selection marker that imparts to the cells resistance to a corresponding selective agent or pressure. Antibiotic selection includes, without limitation, the use of antibiotics (*e.g.,* puromycin, neomycin, G418, hygromycin, bleomycin and the like). Non-antibiotic selection includes, without limitation, the use of nutrient deprivation, exposure to selective temperatures, and exposure to mutagenic conditions where selection marker may be *e.g.,* glutamine synthetase, dihydrofolate reductase (DHFR), oabain, thymidine kinase (TK), hypoxanthine guanine phosphororibosyltransferase (HGPRT). Each vector for a sequence encoding a different bitter receptor may have the same or a different drug resistance or other selectable marker. If more than one of the drug resistance markers are the same, simultaneous selection may be achieved by increasing the level of the drug. Suitable markers will be well-known to those of skill in the art and include but are not limited to genes conferring resistance to any one of the following: Neomycin/G418, Puromycin, hygromycin, Zeocin, methotrexate and blasticidin. Although drug selection (or selection using any other suitable selection marker) is not a required step, it may be used to enrich the transfected cell population for stably transfected cells, provided that the transfected constructs are designed to confer drug resistance. If subsequent selection of cells expressing a bitter receptor is accomplished using signaling probes, selection too soon following transfection can result in some positive cells that may only be transiently and not stably transfected. However, this can be minimized by allowing sufficient cell passage allowing for dilution of transient expression in transfected cells.

Optionally, the vector comprises a nucleic acid sequence encoding an RNA tag sequence. "Tag sequence" refers to a nucleic acid sequence that is an expressed RNA or portion of an RNA that is to be detected by a signaling probe. Signaling probes may detect a variety of RNA sequences. Any of these RNAs may be used as tags. Signaling probes may be directed against the RNA tag by designing the probes to include a portion that is complementary to the sequence of the tag. Optionally, signaling probes are directed (*e.g.,* complementary) to sequences within the same or different coding exons, non-coding introns or non-coding untranslated sequences of an RNA. Optionally, signaling probes can be directed to RNA of components of signaling pathways including the signaling pathway of the bitter taste receptor. The tag sequence may be a 3' untranslated region of the plasmid that is cotranscribed and comprises a target sequence for signaling probe binding. The RNA encoding the gene of interest may include the tag sequence or the tag sequence may be located within a 5'-untranslated region or 3'-untranslated region. Optionally, the tag is not with the RNA encoding the gene of interest. The tag sequence can be in frame with the protein-coding portion of the message of the gene or out of frame with it, depending on whether one wishes to tag the protein produced. Thus, the tag sequence does not have to be translated for detection by the signaling probe. The tag sequences may comprise multiple target sequences that are the same or different, wherein one signaling probe hybridizes to each target sequence. The tag sequences may encode an RNA having secondary structure. The structure may be a three-arm junction structure. Examples of tag sequences that may be used in the disclosure herein, and to which signaling probes may be prepared, include but are not limited to the RNA transcript of epitope tags such as, for example, a HIS tag, a myc tag, a hemagglutinin (HA) tag, protein C, VSV-G, FLU, yellow fluorescent protein (YFP), green fluorescent protein (GFP), FLAG, BCCP, maltose binding protein tag, Nus-tag, Softag-1, Softag-2, Strep-tag, S-tag, thioredoxin, GST, V5, TAP or CBP. As described herein, one of ordinary skill in the art could create his or her own RNA tag sequences.

In another aspect, cells and cell lines disclosed herein stably expresses a G protein. There are two families of G proteins, heterotrimeric G proteins and monomeric G proteins. Heterotrimeric G proteins are activated by G protein coupled receptors ("GPCRs"), and include three subunits: $G_\alpha$, $G_\beta$ and $G_\gamma$. As used herein, the term G protein includes any one of these subunits, for example a $G_\alpha$, or any combination thereof, as well as a heterotrimeric G protein with all three subunits. In the inactive state, $G_\alpha$, $G_\beta$ and $G_\gamma$ form a trimer. The $\beta$ and $\gamma$ subunits are closely bound to one another and are referred to as the *beta-gamma complex.* $G_\alpha$ separates from $G_{\beta\gamma}$ after ligand binding to the GPCR. The $G_{\beta\gamma}$ complex is released from the $G_\alpha$ subunit after its GDP-GTP exchange. The $G_{\beta\gamma}$ complex can activate other second messengers or gate ion channels. The four families of G alpha include: $G_s$ (stimulatory) which increase cAMP synthesis by activating adenylate cyclase; $G_I$ (inhibitory) that inhibits adenylate cyclase; the $G_{12/13}$ family regulates various cell movement processes (*i.e.* cytoskeleton, cell junctions); and Gq, which stimulates calcium signaling and phospholipase C. The monomeric G proteins are homologous to the $\alpha$ subunit of the heterotrimeric G proteins. Any G protein may be expressed in the cells or cell lines disclosed herein, including, but not limited to, transducin (*e.g.*, GNAT1, GNAT2, and guanine nucleotide-binding protein G(t)), gustducin *(e.g.,* GNAT3 guanine nucleotide binding protein and $\alpha$ transducin 3), human GNA15 (guanine nucleotide binding protein (G protein) $\alpha$15 (Gq class; synonym GNA16) and mouse G$\alpha$15, and their chimera proteins, *e.g.* G$\alpha$15-GNA15 (also known as G$\alpha$15-G$\alpha$16). Optionally, the G protein is mouse G$\alpha$15 (SEQ ID NO: 102). Further optionally, the G protein is human GNA15 (SEQ ID NO: 50) or is a human G protein encoded by a nucleic acid comprising SEQ ID NO: 76. The G protein may also be any mammalian G protein, such as, but not limited to, any mammalian G protein listed in Table 7. The G protein stably expressed by the cell can be endogenous to the cell. Alternatively, the stable expression of the G protein may be a result of stable transfection of a nucleic acid encoding the G protein into the cell. Cells stably expressing a heterologous G protein are known in the art, *e.g.,* HEK293/G$\alpha$15 cells (Chandrashekar et al., "T2Rs function as bitter taste receptors", Cell 100:703-711, 2000; Bufe et al., "The human TAS2R16 receptor mediates bitter taste in rsponse to $\beta$-glucopyranosides", Na Genet 32:397-401). Further ptionally, a nucleic acid encoding a G protein and a nucleic acid encoding a bitter receptor can be transfected consecutively into a host cell, with either the nucleic acid encoding the G protein transfected first or the nucleic acid encoding the bitter receptor transfected first. Further optionally, a nucleic acid encoding a G protein and a nucleic acid encoding a bitter receptor can be co-transfected into a host cell on the same or different vectors. Accordingly, selection of cells stably expressing both the G protein and the bitter receptor, can likewise be carried out consecutively or simultaneously. The cells or cell lines that may be used to stably express a G protein are the same as those that may be used to stably express a bitter receptor, as explained above. Vectors for transfecting G proteins optionally comprise a nucleic acid encoding a selectable marker for antibiotic or non-antibiotic selection, as explained above.

Optionally, cells or cell lines disclosed herein co-express other proteins with the bitter receptor(s). Preferably, the other protein is at least one other taste receptor, such as a sweet (TAS1R2/TAS1R3) receptor or an umami (TAS1R1/TAS1R3) receptor. Proteins that are co-expressed with bitter receptors may be expressed by any mechanism, such as, but not limited to, endogenously in the host cell or heterologously from a vector. Further optionally, more than one type of bitter receptor may be stably expressed in a cell or cell line.

Optionally, cells and cell lines disclosed herein have enhanced stability as compared to cells and cell lines produced by conventional methods. To identify stable expression, a cell or cell line's expression of a bitter receptor is measured over a time course and the expression levels are compared. Stable cell lines will continue expressing the bitter receptor throughout the time course. In some aspects disclosed herein, the time course may be for at least one week, two weeks, three weeks, four weeks, five weeks, six weeks, *etc.,* or at least one month, or at least two, three, four, five, six, seven, eight or nine months, or any length of time in between. Isolated cells and cell lines can be further characterized, such as by qRT-PCR and single end-point RT-PCR to determine the absolute amounts and relative amounts of a bitter receptor being expressed. Optionally, stable expression is measured by comparing the results of functional assays over a time course. The measurement of stability based on functional assay provides the benefit of identifying clones that not only stably express the mRNA of the gene of interest, but also stably produce and properly process (*e.g.,* post-translational modification, subunit assembly, and localization within the cell) the protein encoded by the gene of interest that functions appropriately.

Cells and cell lines disclosed herein have the further advantageous property of providing assays with high reproducibility as evidenced by their Z' factor. See Zhang JH, Chung TD, Oldenburg KR, "A Simple Statistical Parameter for Use in Evaluation and Validation of High Throughput Screening Assays." J. Biomol. Screen. 1999;4(2):67-73. Z' values pertain to the quality of a cell or cell line because it reflects the degree to which a cell or cell line will respond consistently to modulators. Z' is a statistical calculation that takes into account the signal-to-noise range and signal variability (*i.e.,* from well to well) of the functional response to a reference compound across a multiwell plate. Z' is calculated using data obtained from multiple wells with a positive control and multiple wells with a negative control. The ratio of their combined standard deviations multiplied by three to the difference in their mean values is subtracted from one to give the Z' factor, according the equation below:

$$Z' \text{ factor} = 1 - ((3\sigma_{\text{positive control}} + 3\sigma_{\text{negative control}}) / (\mu_{\text{positive control}} - \mu_{\text{negative control}}))$$

The theoretical maximum Z' factor is 1.0, which would indicate an ideal assay with no variability and limitless dynamic range. Lower scores (i.e., scores close to 0) are undesirable because it indicates that there is overlap between positive and negative controls. In the industry, for simple cell-based assays, Z' scores up to 0.3 are considered marginal scores, Z' scores between 0.3 and 0.5 are considered acceptable, and Z' scores above 0.5 are considered excellent. Cell-free or biochemical assays may approach higher Z' scores, but Z' scores for cell-based systems tend to be lower because cell-based systems are complex.

Cells and cell lines disclosed herein have Z' values reflecting their ability to advantageously produce consistent results in assays. Bitter receptor expressing cells and cell lines of the invention provided the basis for high throughput screening (HTS) compatible assays because they generally have Z' factors of at least 0.45. In some aspects of the disclosure herein, the cells and cell lines result in Z' of at least 0.3, at least 0.4, at least 0.5, at least 0.6, at least 0.7, or at least 0.8. In other aspects of the disclosure herein, the cells and cell lines disclosed herein result in a Z' of at least 0.3, at least 0.4, at least 0.5, at least 0.6, at least 0.7, or at least 0.8 maintained for multiple passages, *e.g.,* between 5-20 passages, including any integer in between 5 and 20. In some aspects of the disclosure herein, the cells and cell lines result in a Z' of at least 0.4, at least 0.5, at least 0.6, at least 0.7, or at least 0.8 maintained for 1, 2, 3, 4 or 5 weeks or 2, 3, 4, 5, 6, 7, 8 or 9 months, including any period of time in between.

Optionally, cells and cell lines that express a form of a naturally occurring bitter receptor or a naturally-occurring allelic variant thereof, as well as cells and cell lines that express a mutant form of bitter receptor, can be characterized for intracellular free calcium levels. Optionally, the cells and cell lines disclosed herein express bitter receptor with "physiologically relevant" activity. As used herein, physiological relevance refers to a property of a cell or cell line expressing a bitter receptor whereby the bitter receptor causes an increase in intracellular free calcium as a naturally occurring bitter receptor of the same type would when activated, and responds to modulators in the same ways that naturally occurring bitter receptors of the same type would respond when modulated by the same compounds. Bitter receptor-expressing cells and cell lines disclosed herein, including some mutant forms of bitter receptor and some naturally-occurring allelic variants of bitter receptors, preferably demonstrate comparable function to cells that normally express native bitter receptor in a suitable assay, such as an assay measuring intracellular free calcium. Such assays are known to those skilled in the art (Nahorski, "Pharmacology of intracellular signaling pathways," Brit. J. Pharm. 147:S38-S45, 2000)). Such comparisons are used to determine a cell or cell line's physiological relevance. "Sip and spit" taste tests using a panel of trained taste testers also may be used to further validate bitter receptor physiological relevance in cells and cell lines disclosed herein. The results of sip and spit taste tests using modulators identified via screening of native or mutant forms of a bitter receptor or a naturally-occurring allelic variant thereof can be used to validate the physiological relevance of these different forms.

Optionally, the cells and cell lines disclosed herein have increased sensitivity to modulators of bitter receptors. Cells and cell lines disclosed herein respond to modulators and increase intracellular free calcium with physiological range $EC_{50}$ or $IC_{50}$ values for bitter receptors. As used herein, $EC_{50}$ refers to the concentration of a compound or substance required to induce a half-maximal activating response in the cell or cell line. As used herein, $IC_{50}$ refers to the concentration of a compound or substance required to induce a half-maximal inhibitory response in the cell or cell line. $EC_{50}$ and $IC_{50}$ values may be determined using techniques that are well-known in the art, for example, a dose-response curve that correlates the concentration of a compound or substance to the response of the bitter receptor-expressing cell line.

A further advantageous property of the bitter receptor expressing cells and cell lines disclosed herein, flowing from the physiologically relevant function of the bitter receptors is that modulators identified in initial screening are functional in secondary functional assays, *e.g.*,sip and spit or other taste tests, or functional magnetic resonance imaging (MRI) to scan brain activity in response to taste modulating compounds. As those of ordinary skill in the art will recognize, compounds identified in initial screening assays typically must be modified, such as by combinatorial chemistry, medicinal chemistry or synthetic chemistry, for their derivatives or analogs to be functional in secondary functional assays. However, due to the high physiological relevance of the present bitter receptor expressing cells and cell lines, many compounds identified therewith are functional without modification.

Optionally, properties of the cells and cell lines of the invention, such as stability, physiological relevance, reproducibility in an assay (Z'), or physiological EC50 or IC50 values, are achievable under specific culture conditions. Optionally, the culture conditions are standardized and rigorously maintained without variation, for example, by automation. Culture conditions may include any suitable conditions under which the cells or cell lines are grown and may include those known in the art. A variety of culture conditions may result in advantageous biological properties for any of the bitter receptors, or their mutants or allelic variants.

Further optionally, the cells and cell lines disclosed herein with desired properties, such as stability, physiological relevance, reproducibility in an assay (Z'), or physiological EC50 or IC50 values, can be obtained within one month or less. For example, the cells or cell lines may be obtained within 2, 3, 4, 5, or 6 days, or within 1, 2, 3 or 4 weeks, or any length of time in between.

Also disclosed herein is a collection of clonal cells and cell lines, each expressing the same bitter receptor, or different bitter receptors, including different mutant forms and naturally- occurring allelic variants of one or more bitter receptors. The collection may include, for example, cells or cell lines expressing combinations of different bitter receptors, or mutant forms of bitter receptors, naturally-occurring allelic variants of bitter receptors, or any combination thereof. The collection may also include cells or cell lines expressing the same bitter receptor and different G proteins, or any possible dimers or other multimers, including heteromultimers or chimeric dimers or multimers, of bitter receptors.

When collections or panels of cells or cell lines are produced, e.g., for drug screening, the cells or cell lines in the collection or panel may be matched such that they are the same (including substantially the same) with regard to one or more selective physiological properties. The "same physiological property" in this context means that the selected physiological property is similar enough amongst the members in the collection or panel such that the cell collection or panel can produce reliable results in drug screening assays; for example, variations in readouts in a drug screening assay will be due to, *e.g.,* the different biological activities of test compounds on cells expressing different native or mutant forms of bitter receptors, or allelic variants thereof, rather than due to inherent variations in the cells. For example, the cells or cell lines may be matched to have the same growth rate, *i.e.,* growth rates with no more than one, two, three, four, or five hour difference amongst the members of the cell collection or panel. This may be achieved by, for example, binning cells by their growth rate into five, six, seven, eight, nine, or ten groups, and creating a panel using cells from the same binned group. Methods of determining cell growth rate are well known in the art. The cells or cell lines in a panel also can be matched to have the same Z' factor (*e.g.,* Z' factors that do not differ by more than 0.1), bitter receptor expression level (*e.g.,* bitter receptor expression levels that do not differ by more than 5%, 10%, 15%, 20%, 25%, or 30%), adherence to tissue culture surfaces, and the like. Matched cells and cell lines can be grown under identical conditions, achieved by, *e.g.,* automated parallel processing, to maintain the selected physiological property.

Matched cell panels disclosed herein can be used to, for example, identify modulators with defined activity (*e.g.,* agonist or antagonist) on bitter receptors; to profile compound activity across different bitter receptors, or their allelic variants or mutant forms; to identify modulators active on just one type of bitter receptor, or its allelic variant or mutant form; and to identify modulators active on just a subset of bitter receptors. The matched cell panels of the invention allow high throughput screening. Screenings that used to take months to accomplish can now be accomplished within weeks.

To make cells and cell lines disclosed herein, one can use, for example, the technology described in U.S. Patent 6,692,965 and International Patent Publication WO/2005/079462.

This technology provides real-time assessment of millions of cells such that any desired number of clones (from hundreds to thousands of clones) may be selected. Using cell sorting techniques, such as flow cytometric cell sorting (*e.g.,* with a FACS machine) or magnetic cell sorting (*e.g.,* with a MACS machine), one cell per well may be automatically deposited with high statistical confidence in a culture vessel (such as a 96 well culture plate). The speed and automation of the technology allows multigene cell lines to be readily isolated.

Using the technology, the RNA sequence for each bitter receptor may be detected using a signaling probe, also referred to as a molecular beacon or fluorogenic probe. Optionally, the molecular beacon recognizes a target tag sequence as described above. Further optionally, the molecular beacon recognizes a sequence within the bitter receptor coding sequence itself. Signaling probes may be directed against the RNA tag or bitter receptor coding sequence by designing the probes to include a portion that is complementary to the RNA sequence of the tag or the bitter receptor coding sequence, respectively. These same techniques may be used to detect the RNA sequence for a G protein, if used.

Nucleic acids comprising a sequence encoding a bitter receptor, a sequence encoding a G protein, a tag sequence, or any combination thereof, and optionally further comprising a nucleic acid encoding a selectable marker may be introduced into selected host cells by well known methods. The methods include but not limited to transfection, viral delivery, protein or peptide mediated insertion, coprecipitation methods, lipid based delivery reagents (lipofection), cytofection, lipopolyamine delivery, dendrimer delivery reagents, electroporation or mechanical delivery. Examples of transfection reagents are GENEPORTER, GENEPORTER2, LIPOFECTAMINE, LIPOFECTAMINE 2000, FUGENE 6, FUGENE HD, TFX-10, TFX-20, TFX-50, OLIGOFECTAMINE, TRANSFAST, TRANSFECTAM, GENESHUTTLE, TROJENE, GENESILENCER, X-TREMEGENE, PERFECTIN, CYTOFECTIN, SIPORT, UNIFECTOR, SIFECTOR, TRANSIT-LT1, TRANSIT-LT2, TRANSIT-EXPRESS, IFECT, RNAI SHUTTLE, METAFECTENE, LYOVEC, LIPOTAXI, GENEERASER, GENEJUICE, CYTOPURE, JETSI, JETPEI, MEGAFECTIN, POLYFECT, TRANSMESSANGER, RNAiFECT, SUPERFECT, EFFECTENE, TF-PEI-KIT, CLONFECTIN, and METAFECTINE.

Following introduction of the bitter receptor coding sequences, and optionally also the G protein coding sequences, into host cells and optional subsequent drug selection, molecular beacons (*e.g.,* fluorogenic probes) are introduced into the cells and cell sorting is used to isolate cells positive for their signals. Molecular beacons may also be used to identify expression of bitter receptors, G proteins, or both. If expression of both are identified, their identification may be carried out simultaneously or sequentially. Multiple rounds of sorting may be carried out, if desired. Optionally, the flow cytometric cell sorter is a FACS machine. MACS (magnetic cell sorting) or laser ablation of negative cells using laser-enabled analysis and processing can also be used. According to this method, cells expressing at least one bitter receptor are detected and recovered. The bitter receptor (and, optionally, G protein) sequences may be integrated at different locations of the genome in the cell. The expression level of the introduced genes encoding the bitter receptors (and, if introduced, the G proteins) may vary based upon integration site. The skilled worker will recognize that sorting can be gated for any desired expression level (*i.e.,* above background or at a specific level above background). Further, stable cell lines may be obtained wherein one or more of the introduced genes encoding a bitter receptor or G protein is episomal.

Signaling probes useful in the disclosure herein are known in the art and generally are oligonucleotides comprising a sequence complementary to a target sequence and a signal emitting system so arranged that no signal is emitted when the probe is not bound to the target sequence and a signal is emitted when the probe binds to the target sequence. By way of non-limiting illustration, the signaling probe may comprise a fluorophore and a quencher positioned in the probe so that the quencher and fluorophore are brought together in the unbound probe. Upon binding between the probe and the target sequence, the quencher and fluorophore separate, resulting in emission of signal. International publication WO/2005/079462, for example, describes a number of signaling probes that may be used in the production of the cells and cell lines disclosed herein. Where tag sequences are used, the vector for each of the bitter receptors, if multiple bitter receptors are expressed in one cell, or the vectors for bitter receptors and G proteins, can comprise the same or a different tag sequence. Whether the tag sequences are the same or different, the signaling probes may comprise different signal emitters, such as different colored fluorophores and the like so that (RNA) expression of each different bitter receptor and, optionally, G protein, may be separately detected. By way of illustration, the signaling probe that specifically detects one bitter receptor mRNA can comprise a red fluorophore and the probe that detects an introduced G protein (RNA) can comprise a green fluorophore. Also by way of illustration, the signaling probe that specifically detects one bitter receptor mRNA can comprise a red fluorophore, the signaling probe that specifically detects another bitter receptor mRNA can comprise a green fluorophore, and the signaling probe that specifically detects a third bitter receptor mRNA can comprise a yellow fluorophore. Those of skill in the art will be aware of other means for differentially detecting the expression of multiple bitter receptors or bitter receptors and G proteins with a signaling probe in a cell transfected with multiple bitter receptors.

Nucleic acids encoding signaling probes may be introduced into the selected host cell by any of numerous means that will be well-known to those of skill in the art, including but not limited to transfection, coprecipitation methods, lipid based delivery reagents (lipofection), cytofection, lipopolyamine delivery, dendrimer delivery reagents, electroporation or mechanical delivery. Examples of transfection reagents are GENEPORTER, GENEPORTER2, LIPOFECTAMINE, LIPOFECTAMINE 2000, FUGENE 6, FUGENE HD, TFX-10, TFX-20, TFX-50, OLIGOFECTAMINE, TRANSFAST, TRANSFECTAM, GENESHUTTLE, TROJENE, GENESILENCER, X-TREMEGENE, PERFECTIN, CYTOFECTIN, SIPORT, UNIFECTOR, SIFECTOR, TRANSIT-LT1, TRANSIT-LT2, TRANSIT-EXPRESS, IFECT, RNAI SHUTTLE, METAFECTENE, LYOVEC, LIPOTAXI, GENEERASER, GENEJUICE, CYTOPURE, JETSI, JETPEI, MEGAFECTIN, POLYFECT, TRANSMESSANGER, RNAiFECT, SUPERFECT, EFFECTENE, TF-PEI-KIT, CLONFECTIN, and METAFECTINE.

Optionally, the signaling probes are designed to be complementary to either a portion of the RNA encoding a bitter receptor or to portions of their 5' or 3' untranslated regions. If the signaling probe designed to recognize a messenger RNA of interest is able to detect spurious endogenously existing target sequences, the proportion of these in comparison to the proportion of the sequence of interest produced by transfected cells is such that the sorter is able to discriminate the two cell types. Accordingly, the constructs comprising the nucleic acids encoding the bitter receptors do not require and do not include sequences encoding a fluorescent protein. Accordingly, heterologous fluorescent proteins are not expressed in the cells disclosed herein. The cells or cell lines disclosed herein stably express heterologous native bitter receptors. Although such protein tags/chaperones have been in use for expression of a number of bitter receptors to facilitate trafficking of a bitter receptor to the cell surface, the cells and cell lines disclosed herein advantageously functionally express novel bitter receptors at the cell surface without them.

Optionally, adherent cells can be adapted to suspension before or after cell sorting and isolating single cells. Further optionally, isolated cells may be grown individually or pooled to give rise to populations of cells. Individual or multiple cell lines may also be grown separately or pooled. If a pool of cell lines is producing a desired activity or has a desired property, it can be further fractionated until the cell line or set of cell lines having this effect is identified. Pooling cells or cell lines may make it easier to maintain large numbers of cell lines without the requirements for maintaining each separately. Thus, a pool of cells or cell lines may be enriched for positive cells. An enriched pool may have at least 50%, at least 60%, at least 70%, at least 80%, at least 90% or 100% are positive for the desired property or activity.

Also disclosed herein is a method for producing the cells and cell lines disclosed herein. Optionally, the method comprises the steps of:

a) providing a plurality of cells that express mRNA encoding a bitter receptor;
b) dispersing cells individually into individual culture vessels, thereby providing a plurality of separate cell cultures;
c) culturing the cells under a set of desired culture conditions using automated cell culture methods characterized in that the conditions are substantially identical for each of the separate cell cultures, during which culturing the number of cells in each separate cell culture is normalized, and wherein the separate cultures are passaged on the same schedule;
d) assaying the separate cell cultures for at least one desired characteristic of the bitter receptor at least twice; and
e) identifying a separate cell culture that has the desired characteristic in both assays.

According to the method, the cells are cultured under a desired set of culture conditions. The conditions can be any desired conditions. Those of skill in the art will understand what parameters are comprised within a set of culture conditions. For example, culture conditions include but are not limited to: the media (Base media (DMEM, MEM, RPMI, serum-free, with serum, fully chemically defined, without animal-derived components), mono and divalent ion (sodium, potassium, calcium, magnesium) concentration, additional components added (amino acids, antibiotics, glutamine, glucose or other carbon source, HEPES, channel blockers, modulators of other targets, vitamins, trace elements, heavy metals, co-factors, growth factors, anti-apoptosis reagents), fresh or conditioned media, with HEPES, pH, depleted of certain nutrients or limiting (amino acid, carbon source)), level of confluency at which cells are allowed to attain before split/passage, feeder layers of cells, or gamma-irradiated cells, $CO_2$, a three gas system (oxygen, nitrogen, carbon dioxide), humidity, temperature, still or on a shaker, and the like, which will be well known to those of skill in the art.

The cell culture conditions may be chosen for convenience or for a particular desired use of the cells. Also disclosed herein are cells and cell lines that are optimally suited for a particular desired use. That is, in cases in which cells are cultured under conditions for a particular desired use, cells are selected that have desired characteristics under the condition for the desired use.

By way of illustration, if cells will be used in assays in plates where it is desired that the cells are adherent, cells that display adherence under the conditions of the assay may be selected. Similarly, if the cells will be used for protein production, cells may be cultured under conditions appropriate for protein production and selected for advantageous properties for this use.

Optionally, the method comprises the additional step of measuring the growth rates of the separate cell cultures. Growth rates may be determined using any of a variety of techniques means that will be well known to the skilled worker. Such techniques include but are not limited to measuring ATP, cell confluency, light scattering, optical density (*e.g.,* OD 260 for DNA). Preferably growth rates are determined using means that minimize the amount of time that the cultures spend outside the selected culture conditions.

Optionally, cell confluency is measured and growth rates are calculated from the confluency values. Optionally, cells are dispersed and clumps removed prior to measuring cell confluency for improved accuracy. Means for monodispersing cells are well-known and can be achieved, for example, by addition of a dispersing reagent to a culture to be measured. Dispersing agents are well-known and readily available, and include but are not limited to enzymatic dispering agents, such as trypsin, and EDTA-based dispersing agents. Growth rates can be calculated from confluency date using commercially available software for that purpose such as HAMILTON VECTOR. Automated confluency measurement, such as using an automated microscopic plate reader is particularly useful. Plate readers that measure confluency are commercially available and include but are not limited to the CLONE SELECT IMAGER (Genetix). Typically, at least 2 measurements of cell confluency are made before calculating a growth rate. The number of confluency values used to determine growth rate can be any number that is convenient or suitable for the culture. For example, confluency can be measured multiple times over *e.g.,* a week, 2 weeks, 3 weeks or any length of time and at any frequency desired.

When the growth rates are known, according to the method, the plurality of separate cell cultures are divided into groups by similarity of growth rates. By grouping cultures into growth rate bins, one can manipulate the cultures in the group together, thereby providing another level of standardization that reduces variation between cultures. For example, the cultures in a bin can be passaged at the same time, treated with a desired reagent at the same time, *etc.* Further, functional assay results are typically dependent on cell density in an assay well. A true comparison of individual clones is only accomplished by having them plated and assayed at the same density. Grouping into specific growth rate cohorts enables the plating of clones at a specific density that allows them to be functionally characterized in a high throughput format

The range of growth rates in each group can be any convenient range. It is particularly advantageous to select a range of growth rates that permits the cells to be passaged at the same time and avoid frequent renormalization of cell numbers. Growth rate groups can include a very narrow range for a tight grouping, for example, average doubling times within an hour of each other. But according to the method, the range can be up to 2 hours, up to 3 hours, up to 4 hours, up to 5 hours or up to 10 hours of each other or even broader ranges. The need for renormalization arises when the growth rates in a bin are not the same so that the number of cells in some cultures increases faster than others. To maintain substantially identical conditions for all cultures in a bin, it is necessary to periodically remove cells to renormalize the numbers across the bin. The more disparate the growth rates, the more frequently renormalization is needed.
In step d) the cells and cell lines may be tested for and selected for any physiological property including but not limited to: a change in a cellular process encoded by the genome; a change in a cellular process regulated by the genome; a change in a pattern of chromosomal activity; a change in a pattern of chromosomal silencing; a change in a pattern of gene silencing; a change in a pattern or in the efficiency of gene activation; a change in a pattern or in the efficiency of gene expression; a change in a pattern or in the efficiency of RNA expression; a change in a pattern or in the efficiency of RNAi expression; a change in a pattern or in the efficiency of RNA processing; a change in a pattern or in the efficiency of RNA transport; a change in a pattern or in the efficiency of protein translation; a change in a pattern or in the efficiency of protein folding; a change in a pattern or in the efficiency of protein assembly; a change in a pattern or in the efficiency of protein modification; a change in a pattern or in the efficiency of protein transport; a change in a pattern or in the efficiency of transporting a membrane protein to a cell surface change in growth rate; a change in cell size; a change in cell shape; a change in cell morphology; a change in % RNA content; a change in % protein content; a change in % water content; a change in % lipid content; a change in ribosome content; a change in mitochondrial content; a change in ER mass; a change in plasma membrane surface area; a change in cell volume; a change in lipid composition of plasma membrane; a change in lipid composition of nuclear envelope; a change in protein composition of plasma membrane; a change in protein composition of nuclear envelope; a change in number of secretory vesicles; a change in number of lysosomes; a change in number of vacuoles; a change in the capacity or potential of a cell for: protein production, protein secretion, protein folding, protein assembly, protein modification, enzymatic modification of protein, protein glycosylation, protein phosphorylation, protein dephosphorylation, metabolite biosynthesis, lipid biosynthesis, DNA synthesis, RNA synthesis, protein synthesis, nutrient absorption, cell growth, mitosis, meiosis, cell division, to dedifferentiate, to transform into a stem cell, to transform into a pluripotent cell, to transform into a omnipotent cell, to transform into a stem cell type of any organ (*i.e.* liver, lung, skin, muscle, pancreas, brain, testis, ovary, blood, immune system, nervous system, bone, cardiovascular system, central nervous system, gastro-intestinal tract, stomach, thyroid, tongue, gall bladder, kidney, nose, eye, nail, hair, taste bud), to transform into a differentiated any cell type (*i.e.* muscle, heart muscle, neuron, skin, pancreatic, blood, immune, red blood cell, white blood cell, killer T-cell, enteroendocrine cell, taste, secretory cell, kidney, epithelial cell, endothelial cell, also including any of the animal or human cell types already listed that can be used for introduction of nucleic acid sequences), to uptake DNA, to uptake small molecules, to uptake fluorogenic probes, to uptake RNA, to adhere to solid surface, to adapt to serum-free conditions, to adapt to serum-free suspension conditions, to adapt to scaled-up cell culture, for use for large scale cell culture, for use in drug discovery, for use in high throughput screening, for use in a functional cell based assay, for use in calcium flux assays, for use in G-protein reporter assays, for use in reporter cell based assays, for use in ELISA studies, for use in in vitro assays, for use in vivo applications, for use in secondary testing, for use in compound testing, for use in a binding assay, for use in panning assay, for use in an antibody panning assay, for use in imaging assays, for use in microscopic imaging assays, for use in multiwell plates, for adaptation to automated cell culture, for adaptation to miniaturized automated cell culture, for adaptation to large-scale automated cell culture, for adaptation to cell culture in multiwell plates (6, 12, 24, 48, 96, 384, 1536 or higher density), for use in cell chips, for use on slides, for use on glass slides, for microarray on slides or glass slides, for immunofluorescence studies, for use in protein purification, for use in biologics production, for use in the production of industrial enzymes, for use in the production of reagents for research, for use in cell therapy, for use in implantation into animals or humans, for use in isolation of factors secreted by the cell, for preparation of cDNA libraries, for purification of RNA, for purification of DNA, for infection by pathogens, viruses or other agent, for resistance to infection by pathogens, viruses or other agents, for resistance to drugs, for suitability to be maintained under automated miniaturized cell culture conditions, for use in the production of protein for characterization, including: protein crystallography, stimulation of the immune system, antibody production or generation or testing of antibodies. Those of skill in the art will readily recognize suitable tests for any of the above-listed properties. Optionally, one or more of these physical properties may be a constant physical property associated with a bitter taste receptor and can be used to monitor the expression of functional bitter taste receptors.

Tests that may be used to characterize cells and cell lines disclosed herein and/or matched panels of the invention include but are not limited to: Amino acid analysis, DNA sequencing, Protein sequencing, NMR, A test for protein transport, A test for nucleocytoplasmic transport, A test for subcellular localization of proteins, A test for subcellular localization of nucleic acids, Microscopic analysis, Submicroscopic analysis, Fluorescence microscopy, Electron microscopy, Confocal microscopy, Laser ablation technology, Cell counting and Dialysis. The skilled worker would understand how to use any of the above-listed tests.

According to the method, cells may be cultured in any cell culture format so long as the cells or cell lines are dispersed in individual cultures prior to the step of measuring growth rates. For example, for convenience, cells may be initially pooled for culture under the desired conditions and then individual cells separated one cell per well or vessel.

Cells may be cultured in multi-well tissue culture plates with any convenient number of wells. Such plates are readily commercially available and will be well knows to a person of skill in the art. In some cases, cells may preferably be cultured in vials or in any other convenient format, the various formats will be known to the skilled worker and are readily commericially available.

In embodiments comprising the step of measuring growth rate, prior to measuring growth rates, the cells are cultured for a sufficient length of time for them to acclimate to the culture conditions. As will be appreciated by the skilled worker, the length of time will vary depending on a number of factors such as the cell type, the chosen conditions, the culture format and may be any amount of time from one day to a few days, a week or more.

Preferably, each individual culture in the plurality of separate cell cultures is maintained under substantially identical conditions a discussed below, including a standardized maintenance schedule. Another advantageous feature of the method is that large numbers of individual cultures can be maintained simultaneously, so that a cell with a desired set of traits may be identified even if extremely rare. For those and other reasons, the plurality of separate cell cultures are cultured using automated cell culture methods so that the conditions are substantially identical for each well. Automated cell culture prevents the unavoidable variability inherent to manual cell culture.

Any automated cell culture system may be used in the method disclosed herein. A number of automated cell culture systems are commercially available and will be well-known to the skilled worker. Optionally, the automated system is a robotic system. Preferably, the system includes independently moving channels, a multichannel head (for instance a 96-tip head) and a gripper or cherry-picking arm and a HEPA filtration device to maintain sterility during the procedure. The number of channels in the pipettor should be suitable for the format of the culture. Convenient pipettors have, *e.g.,* 96 or 384 channels. Such systems are known and are commercially available. For example, a MICROLAB STAR™ instrument (Hamilton) may be used in the method of the invention. The automated system should be able to perform a variety of desired cell culture tasks. Such tasks will be known by a person of skill in the art. They include but are not limited to: removing media, replacing media, adding reagents, cell washing, removing wash solution, adding a dispersing agent, removing cells from a culture vessel, adding cells to a culture vessel an the like.

The production of a cell or cell line disclosed herein may include any number of separate cell cultures. However, the advantages provided by the method increase as the number of cells increases. There is no theoretical upper limit to the number of cells or separate cell cultures that can be utilized in the method. The number of separate cell cultures can be two or more but more advantageously is at least 3, 4, 5, 6, 7, 8, 9, 10 or more separate cell cultures, for example, at least 12, at least 15, at least 20, at least 24, at least 25, at least 30, at least 35, at least 40, at least 45, at least 48, at least 50, at least 75, at least 96, at least 100, at least 200, at least 300, at least 384, at least 400, at least 500, at least 1000, at least 10,000, at least 100,000, at least 500,000 or more.

A further advantageous property of the bitter receptor expressing cells and cell lines disclosed herein is that they stably express one or more bitter receptors in the absence of drug selection pressure. Thus, in preferred embodiments, cells and cell lines disclosed herein are maintained in culture without any selective drug. Optionally, cells and cell lines are maintained without any antibiotics. As used herein, cell maintenance refers to culturing cells after they have been selected as described above for their bitter receptor expression. Maintenance does not refer to the optional step of growing cells in a selective drug *(e.g.,* an antibiotic) prior to cell sorting where drug resistance marker(s) introduced into the cells allow enrichment of stable transfectants in a mixed population.

Drug-free cell maintenance provides a number of advantages. For example, drug-resistant cells do not always express the co-transfected transgene of interest at adequate levels, because the selection relies on survival of the cells that have taken up the drug resistant gene, with or without the transgene. Further, selective drugs are often mutagenic or otherwise interfere with the physiology of the cells, leading to skewed results in cell-based assays. For example, selective drugs may decrease susceptibility to apoptosis (Robinson et al., Biochemistry, 36(37):11169-11178 (1997)), increase DNA repair and drug metabolism (Deffie et al., Cancer Res. 48(13):3595-3602 (1988)), increase cellular pH (Thiebaut et al., J Histochem Cytochem. 38(5):685-690 (1990); Roepe et al., Biochemistry. 32(41):11042-11056 (1993); Simon et al., Proc Natl Acad Sci USA. 91 (3):1128-1132 (1994)), decrease lysosomal and endosomal pH (Schindler et al., Biochemistry. 35(9):2811-2817 (1996); Altan et al., J Exp Med. 187(10):1583-1598 (1998)), decrease plasma membrane potential (Roepe et al., Biochemistry. 32(41):11042-11056 (1993)), increase plasma membrane conductance to chloride (Gill et al., Cell. 71(1):23-32 (1992)) and ATP (Abraham et al., Proc Natl Acad Sci USA. 90(1):312-316 (1993)), and increase rates of vesicle transport (Altan et al., Proc Natl Acad Sci USA. 96(8):4432-4437 (1999)). Thus, the cells and cell lines disclosed herein allow screening assays that are free from any artifact caused by selective drugs. Optionally, the cells and cell lines disclosed herein are not cultured with selective drugs such as antibiotics before or after cell sorting, so that cells and cell lines with desired properties are isolated by sorting, even when not beginning with an enriched cell population.

The expression level of a bitter receptor may vary from cell or cell line to cell or cell line. The expression level in a cell or cell line also may decrease over time due to epigenetic events such as DNA methylation and gene silencing and loss of transgene copies. These variations can be attributed to a variety of factors, for example, the copy number of the transgene taken up by the cell, the site of genomic integration of the transgene, and the integrity of the transgene following genomic integration. One may use FACS or other cell sorting methods *(i.e.,* MACS) to evaluate expression levels. Additional rounds of introducing signaling probes may be used, for example, to determine if and to what extent the cells remain positive over time for any one or more of the RNAs for which they were originally isolated.

Optionally, cells with different absolute or relative fluorescence levels for at least one signaling probe can be isolated, for example by FACS, by gating subsets of cells with the suitable fluorescent levels relative to the entire cell population. For example, the top 5%, the top 10%, the top 15%, the top 20%, the top 25%, the top 30%, the top 35%, the top 40%, the top 45%, the top 50%, the top 55%, the top 60%, or the top 65%, of cells with the highest fluorescent signal for a particular signaling probe (or combination of signaling probes) can be gated and isolated by, *e.g.,* FACS. Further optionally, the top 2% to 3%, the top 5% to 10%, the top 5% to 15%, the top 5% to 20%, the top 5% to 30%, the top 40% to 50%, the top 10% to 30%, the top 10% to 25%, or the top 10% to 50%, of cells with the highest fluorescent signal for a particular signaling probe (or combination of signaling probes) can be gated and isolated by, *e.g.,* FACS.

The ease with which it is possible to re-isolate cells expressing all of the desired RNAs from cells which may no longer express all of the RNAs of interest makes it possible to maintain cell lines in the presence of no drug or minimal concentrations of drug. Signaling probes may also be re-applied to cells or cell lines generated previously, for example, to determine if and to what extent the cells are still positive for any one or more of the RNAs for which they were originally isolated.

Also disclosed herein are methods of using the cells and cell lines disclosed herein. The cells and cell lines disclosed herein may be used in any application for which functional bitter receptors are needed. The cells and cell lines may be used, for example, but not limited to, in an *in vitro* cell-based assay or an *in vivo* assay where the cells are implanted in an animal (*e.g.,* a non-human mammal) to, *e.g.,* screen for bitter receptor modulators; assess bitterness of substances; produce protein for crystallography and binding studies; and investigate compound selectivity and dosing, receptor/compound binding kinetic and stability, and effects of receptor expression on cellular physiology (*e.g.,* electrophysiology, protein trafficking, protein folding, and protein regulation). The cells and cell lines disclosed herein also can be used in knock down studies to study the roles of specific bitter recepters or groups of bitter receptors.

Cells and cell lines expressing various combinations of bitter receptors can be used separately or together to identify bitter receptor modulators, including those specific for a particular bitter receptor or a mutant form or a naturally-occurring allelic variant of bitter receptor and to obtain information about the activities of individual forms.

Modulators include any substance or compound that alters an activity of a bitter receptor or a mutant form or a naturally-occurring allelic variant thereof. The modulator can be a bitter receptor agonist (potentiator or activator) or antagonist (inhibitor or blocker), including partial agonists or antagonists, selective agonists or antagonists and inverse agonists, and can be an allosteric modulator. A substance or compound is a modulator even if its modulating activity changes under different conditions or concentrations or with respect to different forms (*e.g.,* mutant forms and naturally-occurring allelic variants) of bitter receptor. In other aspects, a modulator may change the ability of another modulator to affect the function of a bitter receptor. For example, a modulator of a form of bitter receptor that is not inhibited by an antagonist may render that form of bitter receptor susceptible to inhibition by the antagonist.

The present cells and cell lines may be used to identify the roles of different forms of bitter receptors in different bitter receptors pathologies by correlating the identity of *in vivo* forms of bitter receptor with the identify of known forms of bitter receptors based on their response to various modulators. This allows selection of disease- or tissue-specific bitter receptor modulators for highly targeted treatment of such bitter receptor-related pathologies or other physiological conditions. For example, because many naturally occurring bitter compounds are toxic, bitter receptors may serve as warning sensors against the ingestion of toxic food compounds. Bitter receptors expressed in the gastrointestinal mucosa might participate in the functional detection of nutrients and harmful substances in the lumen and prepare the gut to absorb them or initiate a protective response. They might also participate in the control of food intake through the activation of gut-brain neural pathways. Accordingly, bitter receptor modulators identified using the cell lines and methods disclosed herein may be used to regulate nutrient uptake in a number of contexts, *e.g.,* to control the appetite and/or reduce nutrient uptake in the gut of the obese, or to control the hunger feeling and/or to increase the uptake of nutrients and/or energy from food in the malnourished. Bitter receptor modulators may also be useful in identifying bitter compouds, further characterizing the specific chemical or structural motifs or key residues of bitter receptors that influence their binding properties, identifying bitter receptors that are broadly, moderately or selectively tuned for ligand binding, defining groups and subgroups of bitter receptors based on their binding profiles, deorphaning orphan bitter receptors, using such data for molecular modeling or drug design for bitter receptors, and determining in which tissues various bitter receptors are active.

To identify a bitter receptor modulator, one can expose a novel cell or cell line disclosed herein to a test compound under conditions in which the bitter receptor would be expected to be functional and then detect a statistically significant change (*e.g.,* $p<0.05$) in bitter receptor activity compared to a suitable control, *e.g.,* cells that are not exposed to the test compound. Positive and/or negative controls using known agonists or antagonists and/or cells expressing different bitter receptor or mutant forms or naturally-occurring allelic variants thereof may also be used. In some embodiments, the bitter receptor activity to be detected and/or measured is change in intracellular free calcium levels. One of ordinary skill in the art would understand that various assay parameters may be optimized, *e.g.,* signal to noise ratio.

Also disclosed herein is a method of identifying ligands of other GPCRs. Any GPCR may be used in this method, including, but not limited to, mammalian or human GPCRs and orphaned or deorphaned GPCRs. A non-limiting example of deorphaned GPCRs are opioids (listed in Table 9). A cell or cell line expressing a GPCR may be screened using a compound or extract library to generate an expression profile for the receptor. Receptors with similar profiles may be grouped together and screened with compounds to identify a ligand(s) that binds a receptor(s).

Also disclosed herein is a method of identifying ligands for orphan bitter receptors, *i.e.* a method of deorphaning bitter receptors. A cell or cell line expressing a bitter receptor with no known modulator may be screened using a compound or extract library to generate an expression profile for the receptor. Optionally, receptors with similar profiles (if any) are grouped together and screened with known bitter compounds to a ligand(s) that binds a receptor(s). Once a ligand is identified, the results may be further verified with taste tests. Advantageously, the cells and cell lines disclosed herein stably express native (*i.e.* untagged) bitter receptors so the ligands identified using this method are accurate and relevant. In a related aspect, the method of deorphaning bitter receptors may be used to deorphan any orphan GPCR, including any orphan mammalian GPCR or any orphan human GPCR, such as those listed in Table 8.

Optionally, one or more cells or cell lines, including collections of cell lines, disclosed herein are exposed to a plurality of test compounds, for example, a library of test compounds. A library of test compounds can be screened using the cell lines disclosed herein to identify one or more modulators. The test compounds can be chemical moieties including small molecules, polypeptides, peptides, peptide mimetics, antibodies or antigen-binding portions thereof. In the case of antibodies, they may be non-human antibodies, chimeric antibodies, humanized antibodies, or fully human antibodies. The antibodies may be intact antibodies comprising a full complement of heavy and light chains or antigen-binding portions of any antibody, including antibody fragments (such as Fab, Fab', $F(ab')_2$, Fd, Fv, dAb and the like), single chain antibodies (scFv), single domain antibodies, all or an antigen-binding portion of a heavy chain or light chain variable region.

Cells or cell lines disclosed herein may also be used to create collections with specific variables. For example, a collection may include: cells or cell lines that all express the same bitter receptor and different G proteins to study receptor-G protein interactions; or cells or cell lines that express multiple endogenous and/or heterologous bitter receptors to study possible dimerization or formation of heteromultimers. Collections of cells or cell lines of the invention may also, preferably, include all 25 bitter receptors. Such a panel may be used to determine on-target versus off-target activity for a compound, or the role of the receptors in pure bitter versus related (*i.e.,* astringent or metallic) tastes.

The cells and cell lines disclosed herein may be used to identify players of the GPCR pathway other than the bitter receptors that they stably express. For example, nucleic acids expressing different G proteins can be introduced (either transiently or stably) into each cell line in a collection of cell lines expressing a same heterologous bitter receptor and, preferably, no endogenous bitter receptor. Any interactions between these G proteins and the bitter receptor expressed by the cell lines may be assayed by, *e.g.,* detecting a change in intracellular free calcium after the cells are exposed to a known agonist of the bitter receptor.

Optionally, large compound collections are tested for bitter receptor modulating activity in a cell-based, functional, high-throughput screen (HTS), *e.g.,* using a 96 well, 384 well, 1536 well or higher plate format. Optionally, a test compound or multiple test compounds including a library of test compounds may be screened using more than one cell or cell line, including collections of cell lines, disclosed herein. If multiple cells or cell lines, each expressing a different naturally occurring or mutant bitter receptor molecule, are used, one can identify modulators that are effective on multiple bitter receptors or mutant forms or naturally-occurring allelic variants thereof or alternatively, modulators that are specific for a particular bitter receptor or a mutant form or naturally-occurring allelic variant thereof and that do not modulate other bitter receptors or other forms of the bitter receptor. In the case of a cell or cell line disclosed herein that expresses a human bitter receptor, one can expose the cells to a test compound to identify a compound that modulates bitter receptor activity (either increasing or decreasing) for use in the treatment of disease or condition characterized by undesired bitter receptor activity, or the decrease or absence of desired bitter receptor activity.

Optionally, prior to exposure to a test compound, the cells or cell lines disclosed herein may be modified by pretreatment with, for example, enzymes, including mammalian or other animal enzymes, plant enzymes, bacterial enzymes, enzymes from lysed cells, protein modifying enzymes, lipid modifying enzymes, and enzymes in the oral cavity, gastrointestinal tract, stomach or saliva. Such enzymes can include, for example, kinases, proteases, phosphatases, glycosidases, oxidoreductases, transferases, hydrolases, lyases, isomerases, ligases and the like. Alternatively, the cells and cell lines may be exposed to the test compound first followed by treatment to identify compounds that alter the modification of the bitter receptor by the treatment.

Also disclosed herein are methods that take advantage of the naturally occurring high degree of genetic diversity that exists in cells, and efficiently identify, select, and enrich for cells possessing desired gene expression profiles conferring desired properties (*e.g.,* stable and/or high expression of functional bitter taste receptors). The present methods can identify, select, and enrich for cells with improved properties from a pool of genetically diverse cells faster and more efficiently than conventional methods. Optionally, the cells have not been genetically modified. Further optionally, the present methods allow for the generation of novel homogeneous populations of cells possessing improved properties (*e.g.,* more stable and/or higher expression of functional bitter taste receptors).

Optionally, the methods described herein comprise selecting naturally occurring cells with one or more desired properties (*e.g.,* stable and/or high expression of functional bitter taste receptors). Optionally, the methods described herein comprise selecting cells with naturally occurring variants or mutations in one or more bitter taste receptor subunit genes.

Optionally, the methods described herein comprise selecting isolated cells with naturally occurring variants or mutations in a promoter region of a bitter taste receptor gene or in a non-coding region of a bitter taste receptor gene (*e.g.,* intron, 5' untranslated region, and/or 3' untranslated region). Variants or mutations in a promoter region of a bitter taste receptor gene or in a non-coding region of a bitter taste receptor gene may result in higher and/or more stable expression of the gene product. Optionally, a promoter region of a bitter taste receptor gene or in a non-coding region of a bitter taste receptor gene has been modified, for example by methylation or acetylation of DNA. Optionally, a cell comprises epigenetic modification affecting chromatin remodeling with respect to a bitter taste receptor gene. Non-limiting examples of epigenetic modifications include, but are not limited to, acetylation, methylation, ubiquitylation, phosphorylation and sumoylation.

Further optionally, the present methods comprise selecting cells that underwent prior treatments. Such prior treatments may be exposure to sunlight or ultraviolet (UV) light, mutagens such as ethyl methane sulfonate (EMS), and chemical agents. Optionally, such prior treatments may include exposure to undesirable growth conditions, *e.g.,* low oxygen or low nutrients conditions, or toxic conditions.

The methods described herein provide for identifying and/or selection of cells (*e.g.,* eukaryotic cells) that express one or more genes of interest (*e.g.,* a bitter taste receptor subunit gene). Optionally, the gene of interest is expressed at higher levels than other cells as a result of genetic variability.

Optionally, the methods described here comprise (a) introducing into a cell (*e.g.,* eukaryotic cell) one or more signaling probes that is capable of detecting an RNA of a bitter taste receptor; and (b) determining whether the cell (*e.g.,* eukaryotic cells) comprises the RNA of a bitter taste receptor. Such methods may further comprise quantifying the level of the RNA of a bitter taste receptor. Optionally, the methods described herein for identifying a cell with a desired RNA expression profile, wherein the method comprises: (a) introducing into a eukaryotic cell (*e.g.,* eukaryotic cell) a plurality of signaling probes each capable of detecting a plurality of RNA of interest; and (b) quantifying the RNA levels detected by the plurality of signaling probes. The desired gene expression profile may be determined by comparison to a reference population. Optionally, the plurality of RNA of interest may comprise any combination of the following RNA: an RNA encoded by any one of SEQ ID NO: 50-102.

Optionally, such method further comprises the step of comparing the quantified RNA levels of the cell with the RNA levels in a reference cell, respectively. Optionally, the plurality of signaling probes comprises at least 2, 3, 4, 5, 6, 7, 8, 9, 10, 15, 20, 25, 30, 35, 40, 45, 50, 60, 70, 80, 90, 100, 125, 150, 175, 200, 250, 300, 350, 500, 600, 700, 800, 900, or at least 1000 signaling robes. Optionally, the RNA of interest is translated. Further optionally, the RNA of interest is not translated. Optionally, the RNA of interest is encoded by a bitter taste receptor subunit gene. Optionally embodiments, the isolated cell expresses one or more recombinant RNA of interest.

Optionally, isolated cells identified and/or selected by the methods described herein have not been genetically engineered (*e.g.,* do not recombinantly express one or more transgenes). Further optionally, isolated cells identified and/or selected by the methods described herein have been genetically engineered (*e.g.,* do recombinantly express one or more transgenes). Optionally, such cells are somatic cells or differentiated cells.

Further optionally, the cell comprises a desired gene expression profile. Optionally, the desired gene expression profile is achieved by genetic engineering or by increasing genetic variability. Optionally, a desired gene expression profile may be determined based on comparison with that of a reference population.

Optionally, the methods described herein are for identifying and/or selecting cells that express an RNA of interest (*e.g.,* RNA of a bitter taste receptor) at a level higher than the average heterologous cell population (*e.g.,* L-'/ unsorted cell line population, for example unsorted 293T cell line population). Optionally, the methods described herein are for identifying and/or selecting cells that express an RNA of interest (*e.g.,* RNA of a bitter taste receptor) at a level that is at least 20%, 30%, 40%, 50%, 60%, 70%, 80%, 90%, 95%, or 100% higher than the average heterologous cell population (*e.g.,* unsorted cell line population, for example unsorted 293T cell line population). Optionally, the methods described herein are for identifying and/or selecting cells that express an RNA of interest (*e.g.,* RNA of a bitter taste receptor) at a level that is at least 1 fold, 1.5 fold, 2 fold, 3 fold, 4 fold, 5 fold, 6 fold, 7 fold, 8 fold, 9 fold, or 10 fold higher than the average heterologous cell population *(e.g.,* unsorted cell line population, for example unsorted 293T cell line population).

Optionally, the methods described herein are for identifying and/or selecting cells that express an RNA of interest (*e.g.,* RNA of a bitter taste receptor) at a level lower than the average heterologous cell population. An exemplary heterologous cell population may be a cell population of mixed cell types of different origin, a cell population of cells of one cell type that are genetically heterologous, or a cell population of one particular cell line from which the isolated cell is obtained using the methods described herein.

Optionally, a cell isolated using the methods described herein is a cell clone from a cell line. Optionally, a cell isolated using the methods described herein is a primary cell. Optionally, a cell isolated using the methods described herein is a transformed cell.

Optionally, a cell is not a human cell. Optionally, a cell is a cell derived from a mouse, rat, monkey, dog, cat, pig, sheep, goat, horse, chicken, frog, worm, insect (*e.g.,* fly), fish, shellfish, or cow. Optionally, a cell is a mammalian cell or a eukaryotic cell. Further optionally, a cell is a human cell. Optionally, a cell is a primary cell. Further optionally, a cell is a transformed cell or a cell clone of a cell line.

Without being bound by theory, in their role as bitter taste, the bitter receptors presumably have evolved to detect a broad diversity of potentially physiologically active compounds as bitter tasting. Some of these receptors may be more broadly tuned than other GPCRs in their binding or interaction with a greater diversity of chemical structures. Therefore, testing of the bitter GPCRs with a library of structurally diverse compounds can be used to identify the compounds that interact with or modulate bitter receptors. Thereby identified compounds are predicted to also modulate the activity of other GPCRs.

Optionally, different bitter receptors, such as a panel of cell lines expressing at least 2, 5, 10, 50, or at least 100 different bitter receptors, may be used to identify the chemical space relevant for other GPCRs most closely related to these bitter receptors. Conversely, chemical space not found to interact with the bitter receptors could be enriched for compounds that have a lower likelihood of interacting with GPCRs.

Optionally, definition of GPCR and non-GPCR chemical space according to these methods can be used to enrich chemical libraries for likely hits during HTS depending on whether GPCR or non-GPCR targets are used. Similarly, the same information may be used to guide medicinal chemistry efforts, for instance where a compound in development for a non-GPCR target can be optimized to exclude functionalities that may underlie undesired interactions with GPCRs.

Any assays to be used with the methods disclosed herein can be conducted in hight throughput format.

Optionally, if the protein complexes are bitter receptors, the activity of the bitter receptors can be measured using a calcium mobilization test.

IDENTIFICATION OF STRUCTURAL COMMONALITIES AMONG COMPOUNDS

Systems and methods are provided for identifying a structural commonality among compounds that modulate a protein complex. Optionally, the protein complex can be a cell surface receptor is a G protein-coupled receptor. Optionally, the protein complex can be a bitter receptor. The compounds that modulate the protein complex can be identified using any of the systems and methods disclosed herein. These compounds can be identified, for example by contacting, separately, a number of candidate compounds with a cell that has been engineered to express the protein complex, and assaying the effects of each of the candidate compounds on the activity of the protein complex, where the effect can be ascertained using various quantitative measures of the effect of the respective candidate compound on the biological activity of the protein complex. Optionally, a library of candidate compound can be screened against the protein complex to identify the compounds that can modulate the protein complex. The effect of the compound of interest on the protein complex can be represented by an activity profile. Non-limiting examples of such quantitative measures include those derived from the assays discussed herein. Each compound of interest can be represented by molecular representations that describe the structure and other properties of the compound. Optionally, the molecular representation of the compound of interest can include structural descriptors that mathematically describe the structural features of the compound. Structural and other types of descriptors are discussed below. A structure-activity relationship (SAR) model can be used to correlate mathematically the one or more descriptors of a compound of interest with the biological activity of the compound, as observed from the activity profile of the compound.

The systems and methods are provided for identifying a structural commonality among compounds that modulate a protein complex can comprise constructing one or more SAR models of the compounds of interest using the molecular representations of a compound and its activity profile, identifying the one or more structural features of each of the compounds that correlates with the activity profile of the respective compound based on that SAR model, and identifying at least one structural feature common to the compounds from among the one or more structural features identified for each of the compounds. Optionally, a SAR model can be constructed for each of the compounds of interest, using the molecular representation and of the respective compound and the activity profile of the respective compound. Structure-activity relationship (SAR) models are discussed below.

From the SAR models, one or more structural features of each of the compounds of interest that correlates with the activity profile of the respective compound can be identified. From among these features, one or more structural features that are common to all of the compounds of interest (structural commonalities) can be identified. Optionally, computer modeling technology can be used to visualize the three-dimensional atomic structure of the compounds of interest. Optionally, the structural commonalities of the compounds of interest can be visualized using the computer modeling technology. A three-dimensional construct of a molecule can depend on data from x-ray crystallographic analyses or NMR imaging of the selected molecule. Force field data can be used to generate a model of the molecular dynamics of the compounds of interest, and/or the structural commonality. Examples of molecular modeling programs are the CHARMm and QUANTA programs (Accelrys, Inc., San Diego, CA).

MOLECULAR REPRESENTATION OF A COMPOUND

The molecular representation of a compound of interest can comprise various descriptors that describe features and properties of the compound. The descriptors can be topological, structural, physicochemical and/or spatial types of descriptors. A given compound can be represented by various descriptors, including sub-structural components or moieties, distance of chemical functional groups, or spatial, 2D or 3D topological, electrochemical, electro-physical, and or quantum mechanical properties of the compound. Optionally, the compound of interest can be represented by structural descriptors. Examples of structural descriptors of a compound can include, but are not limited to, atom type, molecular weight (MW), numbers of rotatable bonds (Rotlbonds), number of hydrogen bond donors (Hbond donor), number of hydrogen bond acceptors (Hbond acceptor), number of chiral centers (R or S) in a compound, 3D molecular moment, sub-structural properties, molecular properties, and quantum mechanical properties. Non-limiting examples of topological descriptors include Balaban index (Jx), kappa shape indices, flexibility, subgraph count, connectivity, Wiener index, Zagreb index, connectivity indices, Hosoya index and E-sate keys. Non-limiting examples of physicochemical (or thermodynamic) descriptors include the log of the partition coefficient (AlogP), log of the partition coefficient, atom-type value (AlogP98), the octanol/water partition coefficient (LogP), molar refractivity (MR) and molar refractivity (MolRef). Non-limiting examples of spatial descriptors include radius of gyration (RadOfGyration), Jurs charged partial surface area descriptors (Jurs), surface area projections (shadow indices), molecular surface area (Area), Density, molecular volume (Vm), and principal moments of inertia (PMI). Non-limiting examples of electronic descriptors include charge, sum of atomic polarizabilities (Apol), highest occupied molecular orbital energy (HOMO), lowest unoccupied molecular orbital energy (LUMO) and superdelocalizability (Sr). Descriptions of the various descriptors can be found at the website titled QSAR Descriptors, available from The Scripps Research Institute (La Jolla CA). Definitions can also be found, for example, at the website tutorial for the Cerius2 software (Accelrys, Inc., San Diego, CA) or Accelrys QSAR software product (Accelrys, Inc., San Diego, CA).

Values for the various descriptors for a compound of interest can be derived using a computational program for determining molecular structure. For example, the GRID program (Molecular Discovery Ltd., Middlesex, UK) can be used to determine energetically favorable binding sites on compounds of known structure, which can be used as descriptors input in QSAR analyses. Also, various programs that perform a QSAR analysis can also be used to derive values for the descriptors for a compound of interest, such as the Accelrys QSAR software product (Accelrys, Inc., San Diego, CA). In another example, information that can serve as descriptors of a compound of interest can be obtained from a database of compound structures, such as the Accelrys Chemicals Available for Purchase (CAP) database (Accelrys Inc., San Diego, CA).

STRUCTURE AND ACTIVITY RELATIONSHIP MODEL

A SAR model can be used to correlate mathematically the descriptors of a compound of interest with the biological activity of the compound. The biological activity of the compound of interest can be provided by an activity profile of the compound. A SAR model can be constructed using different combination of descriptors and algorithms to establish models that can be used to classify other compounds. For example, a SAR that predicts the structural features of a class of compounds that modulate a protein complex, that was constructed using a set of training compounds that were highly similar to one another, can be used to classify other compounds believed to be similar to the training set.

A SAR algorithm can be used to describe mathematic relationships between relevant chemical descriptors and the potencies of the observed biological activity, *i.e.* activity Y is a function of descriptors X, [Y=f(X)]. Any algorithm in the art that is useful for data interrogations can be used to construct a SAR model. As non-limiting examples, programs that can be used to construct a SAR model includes programs that can perform an ordinary multiple regression (OMR), a stepwise regression (SWR), an all possible subsets regression (PSR), and a partial least squares (PLS) regressions and genetic algorithms (GA). In a specific embodiment, a SAR model for a compound can take the form of a linear equation:

$$A0+(A1M1)+(A2M2)+(A3M3)+...(AnMn)$$

where the parameters M1 through Mn are the different descriptors in a molecular representation of the compound, A0 is a constant, and the coefficients A1 through An are calculated by fitting variations in the parameters M1 through Mn and the biological activity of the compound, such as provided by the activity profile. This SAR model can be fit using any regression technique in the art. For example, an ordinary multiple regression can be used to compute the least squares fit of the independent variables (for example, taken to be the descriptors) to the dependent variables (for example, taken to be the activity profile). Examples of software that can be used to provide a SAR model, such as by performing a quantitative structure-activity relationship analysis (QSAR), include but are not limited to, the Accelrys QSAR software (Accelrys Inc., San Diego, CA), and the Quasar 5, a 6D-QSAR software product (Biographics Laboratory 3R, Basel, Switzerland). Other SAR models are discussed in Selassie, C.D., "History of Quantitative Structure-Activity Relationships," Burger's Medicinal Chemistry and Drug Discovery, 6th Edition, vol. 1: Drug Discovery.

Examples of SAR models that can be constructed for a compound of interest include, but are not limited to, receptor-dependent free energy force field QSAR (FEFF-QSAR), receptor-independent three-dimensional QSAR (3D-QSAR), and receptor-dependent or receptor-independent four-dimensional QSAR (4D-QSAR).

Receptor-independent 3D-QSAR. An approach that can be used to provide a tool to relate the magnitude of a particular property exhibited by a compound to one or more structural characteristics and/or physical properties of the compound is a receptor-independent 3D-QSAR analysis. The receptor geometry can be unknown in the performance of a receptor-independent 3D-QSAR analysis. A receptor-independent QSAR can be applicable to a series of chemical analogs for which the dependent (*i.e.,* biological activity) property is derived from a set of intramolecular descriptors based upon the assumption that the chemical compounds share a common mechanism of action. A regression analysis can be used to fit the descriptors of a compound to the different measures of the activity profile for a receptor-independent 3D-QSAR analysis.

Receptor-dependent 3D-QSAR. Another approach can be to use a receptor-dependent 3D-QSAR, also referred to as a free energy force field QSAR (FEFF-QSAR). For a receptor-dependent 3D-QSAR analysis, the receptor geometry is known. The free energy force field ligand-receptor binding energy terms can be calculated and used as the independent variables of the QSAR scoring function. See, for example, Tokarski and Hopfinger (1997), J. Chem. Inf. Computer Sci. 37:792-811.

Receptor-dependent or receptor-independent 4D-QSAR. A 4D-QSAR model can incorporate conformational and alignment freedom with a 3D-QSAR analysis by performing molecular state ensemble averaging (the fourth dimension) on a set of training compounds. In 4D-QSAR analysis, it is considered that differences in the activity of compounds can be related to differences in the Boltzmann average spatial distribution of molecular shape with respect to the interaction pharmacophore element (IPE). A single "active" conformation can be assumed for each compound in a set of training compounds. The 4D-QSAR analysis can be used with additional molecular design applications including receptor independent 3D-QSAR and FEFF-QSAR models. The 4D-QSAR models are described in Duca and Hopfinger (2001), J Chem Inf Comput Sci 41(5): 1367-87.

APPARATUS, COMPUTER AND COMPUTER

PROGRAM PRODUCT IMPLEMENTATIONS

The disclosure herein can be implemented as a computer program product that comprises a computer program mechanism embedded in a computer-readable storage medium. Further, any of the methods disclosed herein can be implemented in one or more computers or other forms of apparatus. Examples of apparatus include but are not limited to, a computer, and a measuring device (for example, an assay reader or scanner). Further still, any of the methods disclosed herein can be implemented in one or more computer program products. Some embodiments disclosed herein provide a computer program product that encodes any or all of the methods disclosed in this application. Such methods can be stored on a CD-ROM, DVD, magnetic disk storage product, or any other computer-readable data or program storage product. Such computer readable storage media are intended to be tangible, physical objects (as opposed to carrier waves). Such methods can also be embedded in permanent storage, such as ROM, one or more programmable chips, or one or more application specific integrated circuits (ASICs). Such permanent storage can be localized in a server, 802.11 access point, 802.11 wireless bridge/station, repeater, router, mobile phone, or other electronic devices. Such methods encoded in the computer program product can also be distributed electronically, via the Internet or otherwise, by transmission of a computer data signal (in which the software modules are embedded) either digitally or on a carrier wave (it will be clear that such use of carrier wave is for distribution, not storage).

Some embodiments disclosed herein provide a computer program product. These program modules can be stored on a CD-ROM, DVD, magnetic disk storage product, or any other computer-readable data or program storage product. The program modules can also be embedded in permanent storage, such as ROM, one or more programmable chips, or one or more application specific integrated circuits (ASICs). Such permanent storage can be localized in a server, 802.11 access point, 802.11 wireless bridge/station, repeater, router, mobile phone, or other electronic devices. The software modules in the computer program product can also be distributed electronically, via the Internet or otherwise, by transmission of a computer data signal (in which the software modules are embedded) either digitally or on a carrier wave.

Optionally, the computer program provides for outputting a result of the claimed method to a user, a user interface device, a computer readable storage medium, a monitor, a local computer, or a computer that is part of a network. Such computer readable storage media are intended to be tangible, physical objects (as opposed to carrier waves).

These and other embodiments disclosed herein may be further illustrated in the following non-limiting Examples.

**EXAMPLES**

**Example 1 Venerating a Stable $GABA_A$-Expressing Cell Line (Reference Example)**

Generating Expression Vectors

Plasmid expression vectors that allowed streamlined cloning were generated based on pCMV-SCRIPT (Stratagene) and contained various necessary components for transcription and translation of a gene of interest, including: CMV and SV40 eukaryotic promoters; SV40 and HSV-TK polyadenylation sequences; multiple cloning sites; Kozak sequences; and neomycin/kanamycin resistance cassettes.

***Step 1: Transfection***

We transfected both 293T and CHO cells. The example focuses on CHO cells, where the CHO cells were cotransfected with three separate plasmids, one encoding a human GABA alpha subunit (SEQ ID NO: 1-4), one encoding the human GABA beta 3 subunit (SEQ ID NO:5) and the other encoding the human GABA gamma 2 subunit (SEQ ID NO: 6) in the following combinations: $\alpha1\beta3\gamma2s$ ($\alpha1$), $\alpha2\beta3\gamma2s$ ($\alpha2$), $\alpha3\beta3\gamma2s$ ($\alpha3$) and $\alpha5\beta3\gamma2s$ ($\alpha5$). As will be appreciated by those of skill in the art, any reagent that is suitable for use with a chosen host cell may be used to introduce a nucleic acid, *e.g.* plasmid, oligonucleotide, labeled oligonucleotide, into a host cell with proper optimization. Examples of reagents that may be used to introduce nucleic acids into host cells include but are not limited to Lipofectamine, Lipofectamine 2000, Oligofectamine, TFX reagents, Fugene 6, DOTAP/DOPE, Metafectine, or Fecturin.

Although drug selection is optional in the methods disclosed herein, we included one drug resistance marker per plasmid. The sequences were under the control of the CMV promoter. An untranslated sequence encoding a tag for detection by a signaling probe was also present along with a sequence encoding a drug resistance marker. The target sequences utilized were GABA Target Sequence 1 (SEQ ID NO: 7), GABA Target Sequence 2 (SEQ ID NO: 8) and GABA Target Sequence 3 (SEQ ID NO: 9). In these examples, the GABA alpha subunit gene-containing vector contained GABA Target Sequence 1, the GABA beta subunit gene-containing vector contained GABA Target Sequence 2 and the GABA gamma subunit gene-containing vector contained the GABA Target Sequence 3.

***Step 2: Selection step***

Transfected cells were grown for 2 days in HAMF12-FBS, followed by 14 days in antibiotic-containing HAMF12-FBS. The antibiotic containing period had antibiotics added to the media as follows: Puromycin (3.5 ug/ml), Hygromycin (150 ug/ml), and G418/Neomycin (300 ug/ml)

***Step 3: Cell passaging***

Following antibiotic selection, and prior to introduction of fluorogenic probes, cells were passaged 6 to 18 times in the absence of antibiotics to allow time for expression that is not stable over the selected period of time to subside.

***Step 4: Exposure of cells to fluorogenic probes***

Cells were harvested and transfected with GABA signaling probes (SEQ ID NO: 10-12). As will be appreciated by those of skill in the art, any reagent that is suitable for use with a chosen host cell may be used to introduce a nucleic acid, *e.g.* plasmid, oligonucleotide, labeled oligonucleotide, into a host cell with proper optimization. Examples of reagents that may be used to introduce nucleic acids into host cells include but are not limited to Lipofectamine, Lipofectamine 2000, Oligofectamine, TFX reagents, Fugene 6, DOTAP/DOPE, Metafectine, or Fecturin.
GABA Signaling Probe 1 binds GABA Target Sequence 1, GABA Signaling Probe 2 binds GABA Target Sequence 2 and GABA Signaling Probe 3 binds GABA Target Sequence 3. The cells were then collected for analysis and sorted using a fluorescence activated cell sorter (Beckman Coulter, Miami, FL) (below).
Target Sequences detected by signaling probes
**GABA Target 1**
5'-GTTCTTAAGGCACAGGAACTGGGAC-3' (SEQ ID NO: 7) (alpha subunit)
**GABA Target 2**
5'-GAAGTTAACCCTGTCGTTCTGCGAC-3' (SEQ ID NO: 8) (beta subunit)
**GABA Target 3**
5'-GTTCTATAGGGTCTGCTTGTCGCTC-3' (SEQ ID NO: 9) (gamma subunit)

Signaling probes

Supplied as 100μM stocks
**GABA Signaling probe 1 -** binds (GABA Target 1)
**5' - Cy5** GCCAGTCCCAGTTCCTGTGCCTTAAGAACCTCGC **BHQ3 quench -3'** (SEQ ID NO: 10)
**GABA Signaling probe 2** -binds (GABA Target 2) **5'- Cy5.5** GCGAGTCGCAGAACGACAGGGTTAACTTCCTCGC **BHQ3 quench -3'** (SEQ ID NO: 11)
A similar probe using a Quasar Dye (BioSearch) with spectral properties similar to Cy5 was used in certain experiments. Note also that 5-MedC and 2-aminodA mixmer probes rather than DNA probes were used in some instances. Note that BHQ3 could be substituted with BHQ2 or a gold particle in Probe 1 or Probe 2.
**GABA Signaling probe 3** -binds (GABA Target 3) **5'- Fam** GCGAGAGCGACAAGCAGACCCTATAGAACCTCGC **BHQ1 quench -3"** (SEQ ID NO: 12)
Note that BHQ1 could be substituted with BHQ2 or Dabcyl in Probe 3.

***Step 5: Isolation of positive cells***

The cells were dissociated and collected for analysis and sorting using a fluorescence activated cell sorter (Beckman Coulter, Miami, FL). Standard analytical methods were used to gate cells fluorescing above background and to isolate individual cells falling within the gate into barcoded 96-well plates. The gating hierarchy was as follows: Gating hierarchy: coincidence gate> singlets gate> live gate > Sort gate. With this gating strategy, the top 0.04-0.4% of triple positive cells were marked for sorting into barcoded 96-well plates.

***Step 6: Additional cycles of steps 1-5 and/or 3-5***

Steps 1 to 5 and/or 3-5 were repeated to obtain a greater number of cells. Two independent rounds of steps 1-5 were completed, and for each of these cycles, at least three internal cycles of steps 3-5 were performed for the sum of independent rounds.

*Step 7: Estimation of growth rates for the populations of cells*

The plates were transferred to a Hamilton Microlabstar automated liquid handler. Cells were incubated for 5-7 days in a 1:1 mix of 2-3 day conditioned growth medium:fresh growth medium (growth medium is Ham's F12/10% FBS) supplemented with 100 units penicillin/ml plus 0.1mg/ml streptomycin and then dispersed by trypsinization with 0.25% trypsin to minimize clumps and transferred to new 96-well plates. After the clones were dispersed, plates were imaged to determine confluency of wells (Genetix). Each plate was focused for reliable image acquisition across the plate. Reported confluencies of greater than 70% were not relied upon. Confluency measurements were obtained at days every 3 times over 9 days (between days 1 and 10 post-dispersal) and used to calculate growth rates.

*Step 8: Binning populations of cells according to growth rate estimates*

Cells were binned (independently grouped and plated as a cohort) according to growth rate between 10-11 days following the dispersal step in step 7. Bins were independently collected and plated on individual 96 well plates for downstream handling, and there could be more than one target plate per specific bin. Bins were calculated by considering the spread of growth rates and bracketing a range covering a high percentage of the total number of populations of cells. Depending on the sort iteration (see Step 5), between 5 and 6 growth bins were used with a partition of 1-4 days. Therefore each bin corresponded to a growth rate or population doubling time difference between 12 and 14.4 hours depending on the iteration.

*Step 9: Replica plating to speed parallel processing and provide stringent QC*

The plates were incubated under standard and fixed conditions (humidified 37°C, 5% $CO_2$/95% air) in Ham's F12 media/10%FBS without antibiotics. The plates of cells were split to produce 4 sets (the set consists of all plates with all growth bins - these steps ensure there are 4 replicates of the initial set) of target plates. Up to 2 target plate sets were committed for cryopreservation (see below), and the remaining set was scaled and further replica plated for passage and for functional assay experiments. Distinct and independent tissue culture reagents, incubators, personnel and carbon dioxide sources were used for each independently carried set of plates. Quality control steps were taken to ensure the proper production and quality of all tissue culture reagents: each component added to each bottle of media prepared for use was added by one designated person in one designated hood with only that reagent in the hood while a second designated person monitored to avoid mistakes. Conditions for liquid handling were set to eliminate cross contamination across wells. Fresh tips were used for all steps or stringent tip washing protocols were used. Liquid handling conditions were set for accurate volume transfer, efficient cell manipulation, washing cycles, pipetting speeds and locations, number of pipetting cycles for cell dispersal, and relative position of tip to plate.

*Step 10: Freezing early passage stocks of populations of cells*

At least two sets of plates were frozen at -70 to -80C. Plates in each set were first allowed to attain confluencies of 70 to 100%. Media was aspirated and 90%FBS and 10% DMSO was added. The plates were sealed with Parafilm and then individually surrounded by 1 to 5cm of foam and placed into a -80C freezer.

*Step 11: Methods and conditions for initial trans formative steps to produce VSF*

The remaining set of plates were maintained as described in step 9 (above). All cell splitting was performed using automated liquid handling steps, including media removal, cell washing, trypsin addition and incubation, quenching and cell dispersal steps.

*Step 12: Normalization methods to correct any remaining variability of growth rates*

The consistency and standardization of cell and culture conditions for all populations of cells was controlled. Any differences across plates due to slight differences in growth rates could be controlled by periodic normalization of cell numbers across plates.

*Step 13: Characterization of population of cells*

The cells were maintained for 6 to 8 weeks of cell culture to allow for their in vitro evolution under these conditions. During this time, we observed size, morphology, fragility, response to trypsinization or dissociation, roundness/average circularity post-dissociation, percentage viability, tendency towards microconfluency, or other aspects of cell maintenance such as adherence to culture plate surfaces.

***Step 14: Assessment of potential functionality of populations of cells under VSF conditions***

Populations of cells were tested using functional criteria. Membrane potential assay kits (Molecular Devices/MDS) were used according to manufacturer's instructions. Cells were tested at multiple different densities in 96 or 384-well plates and responses were analyzed. A variety of time points post plating were used, for instance 12-48 hours post plating. Different densities of plating were also tested for assay response differences.

***Step 15***

The functional responses from experiments performed at low and higher passage numbers were compared to identify cells with the most consistent responses over defined periods of time, ranging from 3 to 9 weeks. Other characteristics of the cells that changed over time are also noted, including morphology, tendency toward microconfluency, and time to attach to culture matrices post-plating.

***Step 16***

Populations of cells meeting functional and other criteria were further evaluated to determine those most amenable to production of viable, stable and functional cell lines. Selected populations of cells were expanded in larger tissue culture vessels and the characterization steps described above were continued or repeated under these conditions. At this point, additional standardization steps were introduced for consistent and reliable passages. These included different plating cell densities, time of passage, culture dish size/format and coating, fluidics optimization, cell dissociation optimization (type, volume used, and length of time), as well as washing steps. Assay Z' scores were stable when tested every few days over the course of four weeks in culture.
Also, viability of cells at each passage were determined. Manual intervention was increased and cells were more closely observed and monitored. This information was used to help identify and select final cell lines that retained the desired properties Final cell lines and back-up cell lines were selected that showed consistent growth, appropriate adherence, as well as functional response.

***Steep17: Establishment of cell banks***

The low passage frozen plates (see above) corresponding to the final cell line and back-up cell lines were thawed at 37°C, washed two times with Ham's F12/10% FBS and incubated in humidified 37°C/5% CO2 conditions. The cells were then expanded for a period of 2-3 weeks. Cell banks for each final and back-up cell line consisting of 25 vials each with 10 million cells were established.

***Step 18***

At least one vial from the cell bank was thawed and expanded in culture. The resulting cells were tested to confirm that they met the same characteristics for which they were originally selected.

**Example 2 Verification of $GABA_A$ Cell Lines Response to GABA Liqand. (Reference Example)**

The response of CHO cell lines expressing GABAA (subunit combinations of $\alpha1\beta3\gamma2s$ ($\alpha1$), $\alpha2\beta3\gamma2s$ ($\alpha2$), $\alpha3\beta3\gamma2s$ ($\alpha3$) and $\alpha5\beta3\gamma2s$ ($\alpha5$)) GABA, the endogenous $GABA_A$ ligand, was evaluated. Interaction of cell lines with GABA was evaluated by measuring the membrane potential of $GABA_A$, in response to GABA using the following protocol.
Cells were plated 24 hours prior to assay at 10-25,000 cells per well in 384 well plates in growth media (Ham's F-12 media plus FBS and glutamine). Media removal was followed by the addition of membrane potential dye diluted in load buffer (137mM NaCl, 5mMKCl,1.25mM CaCl, 25mM HEPES, 10mM Glucose). Incubation was for 1 hour, followed by plate loading onto the high throughput fluorescent plate reader (Hamamastu FDSS). GABA ligand was diluted in MP assay buffer (137mM NaCl, 5mM KGluconate,1.25mM CaCl, 25mM HEPES, 10mM Glucose) to the desired concentration (when needed, serial dilutions of GABA were generated, concentrations used: 3nM, 10nM, 30nM, 100nM, 300nM, 1 uM, 3uM, 10uM) and added to each well. The plates were read for 90 seconds.
**Table 6** (below) demonstrates that each of the cell lines generated responds to GABA ligand. These results indicate that the $GABA_A$ cell lines produced, which respond as expected to the endogenous ligand, are physiologically relevant for use in high-throughput screening assays. Further, the replicate wells produced precise $EC_{50}$ values from well to well indicating high reproducibility of the $GABA_A$ cell lines. Z' values generated using the membrane potential assay were α1β3γ2s 0.58, α2β3γ2s 0.67, α3β3γ2s 0.69 and α5β3γ2s 0.62.

## Example 3 Additional Verification of $GABA_A$ Cell Lines Using A Known $GABA_A$ Modulator. (Reference Example)

The $GABA_A$ cell lines and membrane potential assay were verified by the methods described in Example 2 using serial dilutions in assay buffer of bicuculline (a known antagonist) at 30uM, 10uM, 3uM, 1uM, 300nM, 100nM and 30nM.

Bicuculline was found to interact with all four $GABA_A$ cell lines in the presence of $EC_{50}$ concentrations of GABA. These results indicate that the $GABA_A$ cell lines produced, which respond as expected to this known modulator of $GABA_A$, are physiologically and pharmacologically relevant for use in high-throughput screening assays.

## Example 4 Characterization of Cell Line Expressing $GABA_A$ for Native $GABA_A$ Function Using Membrane Potential Assay (Reference Example)

The interaction of CHO cell lines expressing $GABA_A$ (subunit combinations of α1β3γ2s (α1), α2β3γ2s (α2), α3β3γ2s (α3) and α5β3γ2s (α5)) with 1280 compounds from the LOPAC 1280 (Library of Pharmacologically Active Compounds) was evaluated (Sigma-RBI Prod. No. LO1280). The LOPAC 1280 library contains high purity, small organic ligands with well documented pharmacological activities. Interaction of cell lines with test compounds was evaluated by measuring the membrane potential of $GABA_A$, in response to test compounds using the following protocol.

Cells were plated 24 hours prior to assay at 10-25,000 cells per well in 384 well plates in growth media (Ham's F-12 media plus FBS and glutamine). Media removal was followed by the addition of membrane potential dye diluted in load buffer (137mM NaCl, 5mMKCl,1.25mM CaCl, 25mM HEPES, 10mM Glucose). Incubation was for 1 hour, followed by plate loading onto the high throughput fluorescent plate reader (Hamamastu FDSS). Test compounds were diluted in MP assay buffer (137mM NaCl, 5mM KGluconate,1.25mM CaCl, 25mM HEPES, 10mM Glucose) to the desired concentration (when needed, serial dilutions of each test compound were generated, concentrations used: 3nM, 10nM, 30nM, 100nM, 300nM, 1uM, 3uM, 10uM) and added to each well. The plates were read for 90 seconds.

Results

The activity of each compound towards the $GABA_A$ cell lines produced was measured and compounds which exhibited similar or greater activity as GABA (the endogenous ligand) were scored as positive hits. Of the 1280 compounds screened, 34 activated at least one cell line (*i.e*., either α1, α2, α3 and α5) as well as, if not better, than GABA. The interaction of 17 of these compounds with the produced $GABA_A$ cell lines was confirmed in the following dose response studies. Modulators which require GABA to be present, partial agonists and low potency compounds were not included in the list.

The screening assay identified each of the $GABA_A$ agonists in the LOPAC library: GABA (endogenous ligand), propofol, isoguvacine hydrochloride, muscimol hydrobromide, piperidine-4-sulphonic acid, 3-alpha,21-dihydroxy-5-alpha-pregnan-20-one (a neurosteroid), 5-alpha-pregnan-3alpha-ol-11,20-dione (a neurosteroid), 5-alpha-pegnan-3alpha-ol-20-one (a neurosteroid), and tracazolate. The results indicate that the produced $GABA_A$ cell lines respond in a physiologically relevant manner (*e.g.,* they respond to agonists of the endogenous receptor). $EC_{50}$ values for these eight agonists were determined and are included in **Table 6** (below).

The screening assay also identified four compounds in the LOPAC library not described as GABA agonist but known to have other activities associated with $GABA_A$ which we noted: etazolate (a phospodiesterase inhibitor), androsterone (a steroid hormone), chlormezanone (a muscle relaxant), and ivermectin (an anti-parasitic known to effect chlorine channels). $EC_{50}$ values for these four compounds were determined and are summarized in **Table 6** (below).

The screening assay further identified four compounds in the LOPAC library which, until now, were not known to interact with $GABA_A$. These novel compounds include: dipyrimidole (an adenosine deaminase inhibitor), niclosamide (an anti-parasitic), tyrphosin A9 (a PDGFR inhibitor), and I-Ome-Tyrphosin AG 538 (an IGF RTK inhibitor). EC50 values for these four compounds were determined and are summarized in **Table 6** (below).

The results of the screening assays summarized in **Table 6:**

| Compound | Description | Chromocell Target | $EC_{50}$ Values |
|---|---|---|---|
| GABA | endogenous ligand | α1, α2, α3, α5 | α1 3.29μM<br>α2 374nM<br>α3 131 nM<br>α5 144nM |
| Muscimol | agonist | α1, α2, α3, α5 | α1 4μM<br>α2 675nM<br>α3 367nM<br>α5 80nM |
| Propofol | agonist | α1, α2, α3, α5 | α1 33.4μM<br>α2 42.8μM<br>α3 12.9μM<br>α5 2.0μM |
| Isoguvacine hydrochloride | agonist | α1, α2, α3, α5 | α1 3.57μM<br>α2 3.42μM<br>α3 6.78μM<br>α5 1.13μM |
| Piperidine-4-sulphonic acid | agonist | α1, α2, α3, α5 | α1 13μM<br>α2 20μM<br>α3 8.33μM<br>α5 14.2μM |
| 3-alpha, 21-dihydroxy-5-alpha-pregnan-20-one | neurosteroid (agonist) | α1, α2, α3, α5 | α1 382nM<br>α2 123nM<br>α3 80.2nM<br>α5 17.3nM |
| 5-alpha-Pregnan-3alpha-ol-11,20-dione | neurosteroid (agonist) | α1, α2, α3, α5 | α1 762nM<br>α2 338nM<br>α3 168nM<br>α5 122nM |
| 5-alpha-Pregnan-3alpha-ol-20-one | neurosteroid (agonist) | α1, α2, α3, α5 | α1 692nM<br>α2 140nM<br>α3 80.0nM<br>α5 33.6nM |
| Tracazolate | agonist | α1, α2, α3, α5 | α1 10.6μM<br>α2 8.9μM<br>α3 4.3μM<br>α5 762nM |

(continued)

| Compound | Description | Chromocell Target | $EC_{50}$ Values |
|---|---|---|---|
| Androsterone | Steroid with $GABA_A$ receptor activity | α1, α2, α3, α5 | α1 1.48μM<br>α2 1.52μM<br>α3 1.12μM<br>α5 337nM |
| Ivermectin | Phosphodiesterase inhibitor: Known GABAergic | α1, α2, α3, α5 | α1 4.26μM<br>α2 767nM<br>α3 798nM<br>α5 687nM |
| Chlormezanone | Muscle relaxant: known GABA ligand | α1, α2, α3, α5 | α1 1.74nM<br>α2 5.42nM<br>α3 7.0nM<br>α5 14.1nM |
| Etazolate | Anti-parasitic: known effector of chlorine channels | α1, α2, α3, α5 | α1 2.54μM<br>α2 790nM<br>α3 569nM<br>α5 281nM |
| Dipyridamole | Adenosine inhibitor known to effect GABA release in neurons (not known to bind to $GABA_A$) | a1, a2, α3, α5 | α1 7.16μM<br>α2 3.68μM<br>α3 3.69μM<br>α5 1.37μM |
| Niclosamide | Anti parasitic (side effects include drowsiness and dizziness) | α1, α2, α3, α5 | α1 1.2μM<br>α2 1.26μM<br>α3 0.55μM<br>α5 0.69μM |
| Tyrphostin A9 | PDGFR inhibitor | α1, α2, α3, α5 | α1 1.8μM<br>α2 0.88μM<br>α3 5.0 μM<br>α5 54.0 μM |
| I-OMe Tyrphostin 538 | IGF RTK inhibitor | α1, α2, α3, α5 | α1 3.5μM<br>α2 1.5μM<br>α3 2.2μM<br>α5 Not active |

**Example 5 Characterization $GABA_A$-CHO cells for native $GABA_A$ function using Electrophysiological Assay** (Reference Example)

The following voltage-clamp protocol was used: the membrane potential was clamped to a holding potential of -60 mV. Currents were evoked by 2-sec applications of increasing concentrations of GABA (0.10-100 μM) with inter-

mediate wash with buffer.

Whole cell receptor current traces for the α2, α3, and α5 $GABA_A$ cell lines in response to 100uM GABA, and the α1 $GABA_A$ cell line in response to increasing concentrations of GABA (0.10-100μM in log increments), confirm that the $GABA_A$ cell lines can be used in traditional electrophysiology assays in addition to the High-Throughput Screening assays described above. These electrophysiology assay results, along with the membrane potential assay of Example 2, confirm the physiological and pharmacological relevance of the $GABA_A$ cell lines produced herein. Electrophysiology is accepted as a reliable method of detecting modulators of $GABA_A$ receptors. Our data indicate that the cell lines disclosed herein can produce similarly reliable results using a membrane potential assay. Cell lines of the prior art are not reliable or sensitive enough to effectively utilize this membrane potential assay, which is cheaper and faster than electrophysiology. Thus, the cell lines disclosed herein allow screening on a much larger scale than is available using electrophysiology (10,000's of assays per day using the membrane potential assay compared to less than 100 per day using electrophysiology).

**Example 6 Characterization of an in-cell readout assay for native $GABA_A$ function using halide-sensitive meYFP** (Reference Example)

The response of $GABA_A$ (subunit combinations of α1β3γ2s (A1), α2β3γ2s (A2), α3β3γ2s (A3) and α5β3γ2s (A5)) expressing CHO cells disclosed herein to test compounds was evaluated using the following protocol for an in-cell readout assay.

Cells were plated 24 hours prior to assay at 10-25,000 cells per well in 384 well plates in growth media (Ham's F-12 media plus FBS and glutamine). Media removal was followed by the addition of loading buffer (135mM NaCl, 5mM KCl, 2mM $CaCl_2$, 1 mM $MgCl_2$, 10mM HEPES, 10mM glucose) and incubation for 1 hour. The assay plates were then loaded on the FDSS (Hamamatsu Corporation). Test compounds (e.g. GABA ligand) were diluted in assay buffer (150mM NaI, 5mMKCl, 1.25mM $CaCl_2$, 1 mM $MgCl_2$, 25mM HEPES, 10mM glucose) to the desired concentration (when needed, serial dilutions of each test compound were generated, effective concentrations used: 3nM, 10nM, 30nM, 100nM, 300nM, 1uM, 3uM, 10uM) and added to each well. The plates were read for 90 seconds.

In response to increasing concentrations of GABA ligand, $GABA_A$-meYFP-CHO cells show increasing quench of meYFP signal. This quench can be used to calculate dose response curves for GABA activation. The GABA dose response curves generated by the in-cell readout assay are similar to the curves generated by the Membrane Potential Blue assay described in Example 3. These data demonstrate that the cells disclosed herein can be used in an in-cell readout assay to determine modulators of $GABA_A$.

**Example 7 Generating a stable GC-C-expressing cell line** (Reference Example)

293T cells were transfected with a plasmid encoding the human GC-C gene (SEQ ID NO: 15) using standard techniques. (Examples of reagents that may be used to introduce nucleic acids into host cells include, but are not limited to, LIPOFECTAMINE™, LIPOFECTAMINE™ 2000, OLIGOFECTAMINE™, TFX™ reagents, FUGENE®6, DOTAP/DOPE, Metafectine or FECTURIN™.)

Although drug selection is optional in the methods disclosed herein, we included one drug resistance marker in the plasmid encoding the human GC-C gene. The GC-C sequence was under the control of the CMV promoter. An untranslated sequence encoding a tag for detection by a signaling probe was also present along with a sequence encoding a drug resistance marker. The target sequence utilized was GC-C Target Sequence 1 (SEQ ID NO: 13). In this example, the GC-C gene-containing vector contained GC-C Target Sequence 1.

Transfected cells were grown for 2 days in DMEM-FBS, followed by 10 days in 500μg/ml hygromycin-containing DMEM-FBS, then in DMEM-FBS for the remainder of the time, totaling between 4 and 5 weeks (depending on which independent isolation) in DMEM/10% FBS, prior to the addition of the signaling probe.

Following enrichment on antibiotic, cells were passaged 8-10 times in the absence of antibiotic selection to allow time for expression that is not stable over the selected period of time to subside.

Cells were harvested and transfected with GC-C Signaling Probe 1 (SEQ ID NO: 14) using standard techniques. (Examples of reagents that may be used to introduce nucleic acids into host cells include, but are not limited to, LIPOFECTAMINE™, LIPOFECTAMINE™ 2000, OLIGOFECTAMINE™, TFX™ reagents, FUGENE®6, DOTAP/DOPE, Metafectine or FECTURIN™.) The cells were then dissociated and collected for analysis and sorted using a fluorescence activated cell sorter (Beckman Coulter, Miami, FL).

**GC-C Target Sequence 1 detected by GC-C Signaling probe 1**

5'-GTTCTTAAGGCACAGGAACTGGGAC-3' (SEQ ID NO: 13)

**GC-C Signaling probe 1**

(Supplied as 100μM stock)

5'- Cy5 GCCAGTCCCAGTTCCTGTGCCTTAAGAACCTCGC BHQ2 -3' (SEQ ID NO: 14)

In addition, a similar probe using a QUASAR® Dye (BioSearch) with spectral properties similar to Cy5 was used in certain experiments. In some experiments, 5-MedC and 2-amino dA mixmers were used rather than DNA probes.

The cells were dissociated and collected for analysis and sorting using a fluorescence activated cell sorter (Beckman Coulter, Miami, FL). Standard analytical methods were used to gate cells fluorescing above background and to isolate individual cells falling within the gate into bar-coded 96-well plates. The following gating hierarchy was used:

coincidence gate → singlets gate → live gate → Sort gate in plot FAM vs. Cy5: 0.3% of live cells

The above steps were repeated to obtain a greater number of cells. Two rounds of all the above steps were performed. In addition, the cell passaging, exposure to the signaling probe and isolation of positive cells by the fluorescence activated cell sorter sequence of steps was performed a total of two times for one of the independent transfection rounds.

The plates were transferred to a MICROLAB STAR™ (Hamilton Robotics). Cells were incubated for 9 days in 100μl of 1:1 mix of fresh complete growth medium and 2-day-conditioned growth medium, supplemented with 100U penicillin and 0.1mg/ml streptomycin, dispersed by trypsinization twice to minimize clumps and transferred to new 96-well plates. Plates were imaged to determine confluency of wells (Genetix). Each plate was focused for reliable image acquisition across the plate. Reported confluencies of greater than 70% were not relied upon. Confluency measurements were obtained on 3 consecutive days and used to calculate growth rates.

Cells were binned (independently grouped and plated as a cohort) according to growth rate 3 days following the dispersal step. Each of the 4 growth bins was separated into individual 96-well plates; some growth bins resulted in more than one 96-well plate. Bins were calculated by considering the spread of growth rates and bracketing a range covering a high percentage of the total number of populations of cells. Bins were calculated to capture 12-hour differences in growth rate.

Cells can have doubling times from less than 1 day to more than 2 weeks. In order to process the most diverse clones that at the same time can be reasonably binned according to growth rate, it is preferable to use 3-9 bins with a 0.25 to 0.7 day doubling time per bin. One skilled in the art will appreciate that the tightness of the bins and number of bins can be adjusted for the particular situation and that the tightness and number of bins can be further adjusted if cells were synchronized for their cell cycle.

The plates were incubated under standardized and fixed conditions (DMEM/FBS, 37ºC, 5% $CO_2$) without antibiotics. The plates of cells were split to produce 5 sets of 96-well plates (3 sets for freezing, 1 for assay and 1 for passage). Distinct and independent tissue culture reagents, incubators, personnel and carbon dioxide sources were used downstream in the workflow for each of the sets of plates. Quality control steps were taken to ensure the proper production and quality of all tissue culture reagents: each component added to each bottle of media prepared for use was added by one designated person in one designated hood with only that reagent in the hood while a second designated person monitors to avoid mistakes. Conditions for liquid handling were set to eliminate cross contamination across wells. Fresh tips were used for all steps, or stringent tip washing protocols were used. Liquid handling conditions were set for accurate volume transfer, efficient cell manipulation, washing cycles, pipetting speeds and locations, number of pipetting cycles for cell dispersal, and relative position of tip to plate.

One set of plates was frozen at -70 to -80°C. Plates in the set were first allowed to attain confluencies of 70 to 100%. Medium was aspirated and 90% FBS and 10% DMSO was added. The plates were individually sealed with Parafilm, surrounded by 1 to 5cm of foam and placed into a freezer.

The remaining two sets of plates were maintained under standardized and fixed conditions as described above. All cell splitting was performed using automated liquid handling steps, including media removal, cell washing, trypsin addition and incubation, quenching and cell dispersal steps.

The consistency and standardization of cell and culture conditions for all populations of cells was controlled. Differences across plates due to slight differences in growth rates were controlled by normalization of cell numbers across plates and occurred 3 passages after the rearray. Populations of cells that are outliers were detected and eliminated.

The cells were maintained for 3 to 6 weeks to allow for their *in vitro* evolution under these conditions. During this time, we observed size, morphology, tendency towards microconfluency, fragility, response to trypsinization and average circularity post-trypsinization, or other aspects of cell maintenance such as adherence to culture plate surfaces and resistance to blow-off upon fluid addition.

Populations of cells were tested using functional criteria. The Direct Cyclic GMP Enzyme Immunoassay Kit (Cat. 900-014; AssayDesigns, Inc.) was used according to manufacturer's instructions:

(http://www.assaydesigns.com/objects/catalog/Iproduct/extras/900-014.pdf). Cells were tested at 4 different densities in 96- or 384-well plates and responses were analyzed. The following conditions were used for the GC-C-expressing cell lines disclosed herein.

•Clone screening: 1:2 and 1:3 splits of confluent 96-well plates 48 hour prior to assay, 30 minutes guanylin treatment.
•Dose-response studies: densities of 20,000, 40,000, 60,000, 80,000, 120,000 and 160,000 per well, 30 minutes guanylin treatment (see Example 8).
•Z' studies: densities of 160,000 and 200,000 per well were used, 30 minutes guanylin treatment (see Example 9).

The functional responses from experiments performed at low and higher passage numbers were compared to identify cells with the most consistent responses over defined periods of time, ranging from 4 to 10 weeks. Other characteristics of the cells that changed over time were also noted.

Populations of cells meeting functional and other criteria were further evaluated to determine those most amenable to production of viable, stable and functional cell lines. Selected populations of cells were expanded in larger tissue culture vessels, and the characterization steps described above were continued or repeated under these conditions. At this point, additional standardization steps, such as different cell densities; time of plating, length of cell culture passage; cell culture dishes format and coating; fluidics optimization, including speed and shear force; time of passage; and washing steps, were introduced for consistent and reliable passages. Also, viability of cells at each passage was determined. Manual intervention was increased, and cells were more closely observed and monitored. This information was used to help identify and select final cell lines that retain the desired properties. Final cell lines and back-up cell lines (20 clones total) were selected that showed appropriate adherence/stickiness and growth rate and even plating (lack of microconfluency) when produced following this process and under these conditions.

The initial frozen stock of 3 vials per each of the selected 20 clones was generated by expanding the non-frozen populations from the re-arrayed 96-well plates via 24-well, 6-well and 10cm dishes in DMEM/10%FBS/HEPES/L-Glu. The low passage frozen stocks corresponding to the final cell line and back-up cell lines were thawed at 37°C, washed two times with DMEM containing FBS and incubated in the same manner. The cells were then expanded for a period of 2 to 4 weeks. Two final clones were selected.

One vial from one clone of the initial freeze was thawed and expanded in culture. The resulting cells were tested to confirm that they met the same characteristics for which they were originally selected. Cell banks for each cell line consisting of 20 to over 100 vials may be established.

The following step can also be conducted to confirm that the cell lines are viable, stable and functional: At least one vial from the cell bank is thawed and expanded in culture; the resulting cells are tested to determine if they meet the same characteristics for which they were originally selected.

**Example 8 Characterizing the cell lines for native GC-C function** (Reference Example)

A competitive ELISA for detection of cGMP was used to characterize native GC-C function in the produced GC-C-expressing cell line. Cells expressing GC-C were maintained under standard cell culture conditions in Dulbecco's Modified Eagles medium (DMEM) supplemented with 10% fetal bovine serum, glutamine and HEPES and grown in T175cm flasks. For the ELISA, the cells were plated into coated 96-well plates (poly-D-lysine).

Cell treatment and cell lysis protocol

Cells were washed twice with serum-free medium and incubated with 1 mM IBMX for 30 minutes. Desired activators (*i.e.,* guanylin, 0.001-40 μM) were then added to the cells and incubated for 30-40 minutes. Supernatant was removed, and the cells were washed with TBS buffer. The cells were lysed with 0.1 N HCl. This was followed by lysis with 0.1 N HCl and a freeze/thaw cycle at -20°C/room temperature. Defrosted lysates (samples were spun in Eppendorf tubes at 10,000rpm) were centrifuged to pellet cell debris. The cleared supernatant lysate was then transferred to ELISA plates.

ELISA protocol

All of the following steps were performed at room temperature, unless otherwise indicated. ELISA plates were coated with anti-IgG antibodies in coating buffer (Na-carbonate/bi-carbonate buffer, 0.1 M final, pH 9.6) overnight at 4°C. Plates were then washed with wash buffer (TBS-Tween 20,0.05%), followed by blocking reagent addition. Incubation for 1 hour with blocking reagent at 37°C was followed by a wash of the plates with wash buffer. A rabbit anti-cGMP polyclonal antibody (Chemicon) was then added, followed by incubation for 1 hour and a subsequent wash with wash buffer. Cell lysate was then added, and incubated for 1 hour before the subsequent addition of a cGMP-biotin conjugate (1 and 10nM of 8-Biotin-AET-cGMP (Biolog)). Plates were incubated for 2 hours and then washed with wash buffer. Streptavidin-alkaline phosphate was then added and incubated for 1 hour, then washed with wash buffer. Plates were incubated for at least 1 hour (preferably 2-5 hours) with PNPP substrate (Sigma). The absorbance was then read at 405nm on a SAFIRE$^{2™}$ plate reader (Tecan).

Maximum absorbance was seen when no cell lysate was used in the ELISA (Control). Reduction in absorbance (corresponding to increased cGMP levels) was observed with cell lysate from the produced GC-C-expressing cell line treated with 100 nM guanylin (Clone).

The cGMP level in the produced GC-C-expressing cell line treated with 100 nM guanylin was also compared to that of parental cell line control samples not expressing GC-C (not shown) using the Direct Cyclic GMP Enzyme Immunoassay Kit (Cat. 900-014; AssayDesigns, Inc.). The GC-C-expressing cell line showed a greater reduction in absorbance (corresponding to increased cGMP levels) than parental cells treated and untreated with guanylin.

For guanylin dose-response experiments, cells of the produced GC-C-expressing cell line, plated at densities of 20,000, 40,000, 60,000, 80,000, 120,000 and 160,000 cells/well in a 96-well plate, were challenged with increasing concentration of guanylin for 30 minutes. The cellular response (*i.e.,* absorbance) as a function of changes in cGMP levels (as measured using the Direct Cyclic GMP Enzyme Immunoassay Kit (Cat. 900-014; AssayDesigns, Inc.) was detected using a SAFIRE$^{2™}$ plate reader (Tecan). Data were then plotted as a function of guanylin concentration and analyzed using non-linear regression analysis using GraphPad Prism 5.0 software, resulting in an $EC_{50}$ value of 1.1 nM. The produced GC-C-expressing cell line shows a higher level of cGMP (6 pmol/ml) when treated with low concentrations of guanylin in comparison to that previously reported in other cell lines (3.5 pmol/ml) (Forte et al., Endocr. 140(4):1800-1806 (1999)), indicating the potency of the clone.

**<u>Example 9 Generation of GC-C-expressing cell line Z' value</u>** (Reference Example)

Z' for the produced GC-C-expressing cell line was calculated using a direct competitive ELISA assay. The ELISA was performed using the Direct Cyclic GMP Enzyme Immunoassay Kit (Cat. 900-014; AssayDesigns, Inc.). Specifically, for the Z' assay, 24 positive control wells in a 96-well assay plate (plated at a density of 160,000 or 200,000 cells/well) were challenged with a GC-C activating cocktail of 40μM guanylin and IBMX in DMEM media for 30 minutes. Considering the volume and surface area of the 96-well assay plate, this amount of guanylin created a concentration comparable to the 10μM used by Forte et al. (1999) Endocr. 140(4), 1800-1806. An equal number of wells containing clonal cells in DMEM/IMBX were challenged with vehicle alone (in the absence of activator). Absorbance (corresponding to cGMP levels) in the two conditions was monitored using a SAFIRE$^{2™}$ plate reader (Tecan). Mean and standard deviations in the two conditions were calculated and Z' was computed using the method of Zhang et al., J Biomol Screen, 4(2):67-73 (1999)). The Z' value of the produced GC-C-expressing cell line was determined to be 0.72.

**<u>Example 10 Short-circuit current measurements</u>** (Reference Example)

Using chamber experiments are performed 7-14 days after plating GC-C-expressing cells (primary or immortalized epithelial cells, for example, lung, intestinal, mammary, uterine, or renal) on culture inserts (Snapwell, Corning Life Sciences). Cells on culture inserts are rinsed, mounted in an Using type apparatus (EasyMount Chamber System, Physiologic Instruments) and bathed with continuously gassed Ringer solution (5%$CO_2$ in $O_2$, pH 7.4) maintained at 37°C containing (in mM) 120 NaCl, 25 $NaHCO_3$, 3.3 $KH_2PO_4$, 0.8 $K_2HPO_4$, 1.2 $CaCl_2$, 1.2 $MgCl_2$, and 10 glucose. The hemichambers are connected to a multichannel voltage and current clamp (VCC-MC8, Physiologic Instruments). Electrodes [agar bridged (4% in 1 M KCl) Ag-AgCl] are used, and the inserts are voltage clamped to 0 mV. Transepithelial current, voltage and resistance are measured every 10 seconds for the duration of the experiment. Membranes with a resistance of <200mOhms are discarded. This secondary assay can provide confirmation that in the appropriate cell type (*i.e.,* cell that form tight junctions) the introduced GC-C is altering CFTR activity and modulating a transepithelial current.

**<u>Example 11 Generating a Stable CFTR-Expressing Cell Line</u>** (Reference Example)

***Generating** Expression **Constructs***

Plasmid expression vectors that allowed streamlined cloning were generated based on pCMV-SCRIPT (Stratagene) and contained various necessary components for transcription and translation of a gene of interest, including:

CMV and SV40 eukaryotic promoters; SV40 and HSV-TK polyadenylation sequences; multiple cloning sites; Kozak sequences; and drug resistance cassettes (*i.e.,* puromycin). Ampicillin or neomycin resistance cassettes can also be used to subsitute puromycin. A tag sequence containing Target Sequence 2 (SEQ ID NO: 138) was then inserted into the multiple cloning site of the plasmid. A cDNA cassette encoding a human CFTR was then subcloned into the multiple cloning site upstream of the tag sequence, using Asc1 and Pac1 restriction endonucleases.

***Generating Cell Lines***

***Step 1: Transfection***

CHO cells were transfected with a plasmid encoding a human CFTR (SEQ ID NO: 16) using standard techniques. (Examples of reagents that may be used to introduce nucleic acids into host cells include, but are not limited to, LIPOFECTAMINE™, LIPOFECTAMINE™ 2000, OLIGOFECTAMINE™, TFX™ reagents, FUGENE®6, DOTAP/DOPE, Metafectine or FECTURIN™.)

Although drug selection is optional to produce the cells or cell lines disclosed herein, we included one drug resistance marker in the plasmid *(i.e.,* puromycin). The CFTR sequence was under the control of the CMV promoter. An untranslated sequence encoding a Target Sequence for detection by a signaling probe was also present along with the sequence encoding the drug resistance marker. The target sequence utilized was Target Sequence 2 (SEQ ID NO: 138), and in this example, the CFTR gene-containing vector comprised Target Sequence 2 (SEQ ID NO: 138).

***Step 2: Selection***

Transfected cells were grown for 2 days in Ham's F12-FBS media (Sigma Aldrich, St. Louis, MO) without antibiotics, followed by 10 days in 12.5 μg/ml puromycin-containing Ham's F12-FBS media. The cells were then transferred to Ham's F12-FBS media without antibiotics for the remainder of the time, prior to the addition of the signaling probe.

***Step 3: Cell passaging***

Following enrichment on antibiotic, cells were passaged 5-14 times in the absence of antibiotic selection to allow time for expression that was not stable over the selected period of time to subside.

***Step 4: Exposure of cells to fluorogenic probes***

Cells were harvested and transfected with Signaling Probe 2 (SEQ ID NO: 139) using standard techniques. (Examples of reagents that may be used to introduce nucleic acids into host cells include, but are not limited to, LIPOFECTAMINE™, LIPOFECTAMINE™ 2000, OLIGOFECTAMINE™, TFX™ reagents, FUGENE®6, DOTAP/DOPE, Metafectine or FECTURIN™.) CFTR Signaling Probe 2 (SEQ ID NO: 139) bound Target Sequence 2 (SEQ ID NO: 138). The cells were then collected for analysis and sorted using a fluorescence activated cell sorter.

<u>**Target Sequence detected by signaling probe**</u>

CFTR Target Sequence 1
5'- GTTCTTAAGGCACAGGAACTGGGAC -3' (SEQ ID NO: 17)
CFTR Target Sequence 2
5'- GAAGTTAACCCTGTCGTTCTGCGAC -3' (SEQ ID NO: 138)

<u>**Signaling probe**</u>

CFTR Signaling probe 1 (Supplied as 100μM stock)
5'- **Cy5** GCCAGTCCCAGTTCCTGTGCCTTAAGAACCTCGC **BHQ2** -3' (SEQ ID NO: 18)
CFTR Signaling probe 2 (Supplied as 100μM stock)
5' - **CY5.5** GCGAGTCGCAGAACGACAGGGTTAACTTCCTCGC **BHQ2** -3' (SEQ ID NO: 139)
BHQ2 in Signaling Probe 2 can be substituted with BHQ3 or a gold particle. Target Sequence 2 and Signaling Probe 2 can be replaced by Target Sequence 1 (SEQ ID NO: 17) and Signaling Probe 1 (SEQ ID NO: 18), respectively. BHQ2 in Signaling Probe 1 can be substituted with BHQ3 or a gold particle.
In addition, a similar probe using a Quasar® Dye (BioSearch) with spectral properties similar to Cy5 is used in certain experiments against Target Sequence 1 (SEQ ID NO: 17). In some experiments, 5-MedC and 2-amino dA mixmers were used rather than DNA probes. A non-targeting FAM labeled probe is also used as a loading control.

*Step 5: Isolation of positive cells*

The cells were dissociated and collected for analysis and sorting using a fluorescence activated cell sorter (Beckman Coulter, Miami, FL). Standard analytical methods were used to gate cells fluorescing above background and to isolate individual cells falling within the gate into bar-coded 96-well plates. The following gating hierarchy was used:

coincidence gate → singlets gate → live gate → Sort gate in plot FAM vs. Cy5.5: 0.1 - 0.4 % of cells according to standard procedures in the field.

*Step 6: Additional cycles of steps 1-5 and/or 3-5*

Steps 1-5 and/or 3-5 were repeated to obtain a greater number of cells. Two rounds of steps 1-5 were performed, and for each of these rounds, two internal cycles of steps 3-5 were performed.

*Step 7: Estimation of growth rates for the populations of cells*

The plates were transferred to a Microlab Star (Hamilton Robotics). Cells were incubated for 9 days in 100 μl of 1:1 mix of fresh complete growth media and 2 to 3 day-conditioned growth media, supplemented with 100 units/ml penicillin and 0.1 1 mg/ml streptomycin. Then the cells were dispersed by trypsinization once or twice to minimize clumps and later transferred to new 96-well plates. Plates were imaged to determine confluency of wells (Genetix). Each plate was focused for reliable image acquisition across the plate. Reported confluencies of greater than 70% were not relied upon. Confluency measurements were obtained on consecutive days between days 1 and 10 post-dispersal and used to calculate growth rates.

*Step 8: Binning populations of cells according to growth rate estimates*

Cells were binned (independently grouped and plated as a cohort) according to growth rate less than two weeks following the dispersal step in step 7. Each of the three growth bins was separated into individual 96 well plates; some growth bins resulted in more than one 96 well plate. Bins were calculated by considering the spread of growth rates and bracketing a high percentage of the total number of populations of cells. Bins were calculated to capture 12-16 hour differences in growth rate.

Cells can have doubling times from less than 1 day to more than 2 week. In order to process the most diverse clones that at the same time can be reasonably binned according to growth rate, it may be preferable to use 3-9 bins with a 0.25 to 0.7 day doubling time per bin. One skilled in the art will appreciate that the tightness of the bins and number of bins can be adjusted for the particular situation and that the tightness and number of bins can be further adjusted if cells are synchronized for their cell cycle.

*Step 9: Replica plating to speed parallel processing and provide stringent quality control*

The plates were incubated under standardized and fixed conditions (*i.e.,* Ham's F12-FBS media, 37°C/5%CO2) without antibiotics. The plates of cells were split to produce 4 sets of 96 well plates (3 sets for freezing, 1 set for assay and passage). Distinct and independent tissue culture reagents, incubators, personnel, and carbon dioxide sources were used for each of the sets of the plates. Quality control steps were taken to ensure the proper production and quality of all tissue culture reagents: each component added to each bottle of media prepared for use was added by one designated person in one designated hood with only that reagent in the hood while a second designated person monitored to avoid mistakes. Conditions for liquid handling were set to eliminate cross contamination across wells. Fresh tips were used for all steps or stringent tip washing protocols were used. Liquid handling conditions were set for accurate volume transfer, efficient cell manipulation, washing cycles, pipetting speeds and locations, number of pipetting cycles for cell dispersal, and relative position of tip to plate.

*Step 10: Freezing early passage stocks of populations of cells*

Three sets of plates were frozen at -70 to -80°C. Plates in the set were first allowed to attain confluencies of 70 to 100%. Medium was aspirated and 90%FBS and 10% DMSO was added. The plates were individually sealed with Parafilm, individually surrounded by 1 to 5cm of foam, and then placed into a -80 °C freezer.

***Step 11: Methods and conditions for initial trans formative steps to produce viable, stable and functional (VSF) cell lines***

The remaining set of plates was maintained as described in step 9. All cell splitting was performed using automated liquid handling steps, including media removal, cell washing, trypsin addition and incubation, quenching and cell dispersal steps.

***Step 12: Normalization methods to correct any remaining variability of growth rates***

The consistency and standardization of cell and culture conditions for all populations of cells was controlled. Differences across plates due to slight differences in growth rates were controlled by normalization of cell numbers across plates and occurred every 8 passages after the rearray. Populations of cells that were outliers were detected and eliminated.

***Step 13: Characterization of population of cells***

The cells were maintained for 6 to 10 weeks post rearray in culture. During this time, we observed size, morphology, tendency towards microconfluency, fragility, response to trypsinization and average circularity post-trypsinization, or other aspects of cell maintenance such as adherence to culture plate surfaces and resistance to blow-off upon fluid addition as part of routine internal quality control to identify robust cells. Such benchmarked cells were then admitted for functional assessment.

***Step 14: Assessment of potential functionality of populations of cells under VSF conditions***

Populations of cells were tested using functional criteria. Membrane potential dye kits (Molecular Devices, MDS) were used according to manufacturer's instructions.

Cells were tested at varying densities in 384-well plates (*i.e.,* 12.5 x $10^3$ to 20 x $10^3$ cells/per well) and responses were analyzed. Time between cell plating and assay read was tested. Dye concentration was also tested. Dose response curves and Z' scores were both calculated as part of the assessment of potential functionality.

The following steps (*i.e.,* steps 15-18) can also be conducted to select final and back-up viable, stable and functional cell lines.

***Step 15:***

The functional responses from experiments performed at low and higher passage numbers are compared to identify cells with the most consistent responses over defined periods of time *(e.g.,* 3-9 weeks). Other characteristics of the cells that change over time are also noted.

***Step 16:***

Populations of cells meeting functional and other criteria are further evaluated to determine those most amenable to production of viable, stable and functional cell lines. Selected populations of cells are expanded in larger tissue culture vessels and the characterization steps described above are continued or repeated under these conditions. At this point, additional standardization steps, such as different cell densities; time of plating, length of cell culture passage; cell culture dishes format and coating; fluidics optimization, including speed and shear force; time of passage; and washing steps, are introduced for consistent and reliable passages.

In addition, viability of cells at each passage is determined. Manual intervention is increased and cells are more closely observed and monitored. This information is used to help identify and select final cell lines that retain the desired properties. Final cell lines and back-up cell lines are selected that show appropriate adherence/stickiness, growth rate, and even plating (lack of microconfluency) when produced following this process and under these conditions.

***Step 17: Establishment of cell banks***

The low passage frozen stocks corresponding to the final cell line and back-up cell lines are thawed at 37°C, washed two times with Ham's F12-FBS and then incubated in Ham's F12-FBS. The cells are then expanded for a period of 2 to 4 weeks. Cell banks of clones for each final and back-up cell line are established, with 25 vials for each clonal cells being cryopreserved.

***Step 18:***

At least one vial from the cell bank is thawed and expanded in culture. The resulting cells are tested to determine if they meet the same characteristics for which they are originally selected.

**Example 12 Characterizing Stable Cell Lines for Native CFTR Function** (Reference Example)

We used a high-throughput compatible fluorescence membrane potential assay to characterize native CFTR function in the produced stable CFTR-expressing cell lines.

CHO cell lines stably expressing CFTR were maintained under standard cell culture conditions in Ham's F12 medium supplemented with 10% fetal bovine serum and glutamine. On the day before assay, the cells were harvested from stock plates and plated into black clear-bottom 384 well assay plates. The assay plates were maintained in a 37°C cell culture incubator under 5% $CO_2$ for 22-24 hours. The media was then removed from the assay plates and blue membrane potential dye (Molecular Devices Inc.) diluted in loading buffer (137 mM NaCl, 5 mM KCl, 1.25 mM $CaCl_2$, 25 mM HEPES, 10 mM glucose) was added and allowed to incubate for 1 hour at 37°C. The assay plates were then loaded on a fluorescent plate reader (Hamamatsu FDSS) and a cocktail of forskolin and IBMX dissolved in compound buffer (137 mM sodium gluconate, 5 mM potassium gluconate, 1.25 mM $CaCl_2$, 25 mM HEPES, 10mM glucose) was added.

Representative data from the fluorescence membrane potential assay showed that the ion flux attributable to functional CFTR in stable CFTR-expressing CHO cell lines (cell line 1, M11, J5, E15, and 015) were all higher than control cells lacking CFTR as indicated by the assay response.

The ion flux attributable to functional CFTR in stable CFTR-expressing CHO cell lines (cell line 1, M11, J5, E15, and 015) were also all higher than transiently CFTR-transfected CHO cells. The transiently CFTR-transfected cells were generated by plating CHO cells at 5-16 million per 10cm tissue culture dish and incubating them for 18-20 hours before transfection. A transfection complex consisting of lipid transfection reagent and plasmids encoding CFTR was directly added to each dish. The cells were then incubated at 37°C in a $CO_2$ incubator for 6-12 hours. After incubation, the cells were lifted, plated into black clear-bottom 384 well assay plates, and assayed for function using the above-described fluorescence membrane potential assay.

For forskolin dose-response experiments, cells of the produced stable CFTR-expressing cell lines, plated at a density of 15,000 cells/well in a 384-well plate were challenged with increasing concentration of forskolin, a known CFTR agonist. The cellular response as a function of changes in cell fluorescence was monitored over time by a fluorescent plate reader (Hamamatsu FDSS). Data were then plotted as a function of forskolin concentration and analyzed using non-linear regression analysis using GraphPad Prism 5.0 software, resulting in an $EC_{50}$ of 256 nM. The produced CFTR-expressing cell line shows a $EC_{50}$ value of forskolin within the ranges of $EC_{50}$ if forskolin previously reported in other cell lines (between 250 and 500 nM) (Galietta et al., Am J Physiol Cell Physiol. 281(5): C1734-1742 (2001)), indicating the potency of the clone.

**Example 13 Determination of Z' Value for CFTR Cell-Based Assay** (Reference Example)

Z' value for the produced stable CFTR-expressing cell line was calculated using a high-throughput compatible fluorescence membrane potential assay. The fluorescence membrane potential assay protocol was performed substantially according to the protocol in **Example 12**. Specifically for the Z' assay, 24 positive control wells in a 384-well assay plate (plated at a density of 15,000 cells/well) were challenged with a CFTR activating cocktail of forskolin and IBMX. An equal number of wells were challenged with vehicle alone and containing DMSO (in the absence of activators). Cell responses in the two conditions were monitored using a fluorescent plate reader (Hamamatsu FDSS). Mean and standard deviations in the two conditions were calculated and Z' was computed using the method disclosed in Zhang et al., J Biomol Screen, 4(2): 67-73, (1999). The Z' value of the produced stable CFTR-expressing cell line was determined to be higher than or equal to 0.82.

**Example 14 High-Throughput Screening and Identification of CFTR**

**Modulators** (Reference Example)

A high-throughput compatible fluorescence membrane potential assay is used to screen and identify CFTR modulator. On the day before assay, the cells are harvested from stock plates into growth media without antibiotics and plated into black clear-bottom 384 well assay plates. The assay plates are maintained in a 37°C cell culture incubator under 5% $CO_2$ for 19-24 hours. The media is then removed from the assay plates and blue membrane potential dye (Molecular Devices Inc. ) diluted in load buffer (137 mM NaCl, 5 mM KCl, 1.25 mM $CaCl_2$, 25 mM HEPES, 10 mM glucose) is added and the cells are incubated for 1 hr at 37°C. Test compounds are solubilized in dimethylsulfoxide, diluted in assay buffer (137 mM sodium gluconate, 5 mM potassium gluconate, 1.25 mM $CaCl_2$, 25 mM HEPES, 10mM glucose) and then loaded into 384 well polypropylene micro-titer plates. The cell and compound plates are loaded into a fluorescent plate reader (Hamamatsu FDSS) and run for 3 minutes to identify test compound activity. The instrument will then add a forskolin solution at a concentration of 300 nM - 1 μM to the cells to allow either modulator or blocker activity of the previously added compounds to be observed. The activity of the compound is determined by measuring the change in fluorescence produced following the addition of the test compounds to the cells and/or following the subsequent agonist addition.

**Example 15 Characterizing Stable CFTR-Expressing Cell Lines for Native CFTR Function using Short-Circuit Current Measurements** (Reference Example)

Using chamber experiments are performed 7-14 days after plating CFTR-expressing cells (primary or immortalized epithelial cells including but not limited to lung and intestinal) on culture inserts (Snapwell, Corning Life Sciences). Cells on culture inserts are rinsed, mounted in an Using type apparatus (EasyMount Chamber System, Physiologic Instruments) and bathed with continuously gassed Ringer solution (5% $CO_2$ in $O_2$, pH 7.4) maintained at 37°C containing 120 mM NaCl, 25 mM $NaHCO_3$, 3.3 mM $KH_2PO_4$, 0.8 mM $K_2HPO_4$, 1.2 mM $CaCl_2$, 1.2 mM $MgCl_2$, and 10 mM glucose. The hemichambers are connected to a multichannel voltage and current clamp (VCC-MC8 Physiologic Instruments). Electrodes [agar bridged (4% in 1 M KCl) Ag-AgCl] are used and the inserts are voltage clamped to 0 mV. Transepithelial current, voltage and resistance are measured every 10 seconds for the duration of the experiment. Membranes with a resistance of < 200 mΩs are discarded.

**Example 16 Characterizing Stable CFTR-expressing Cell Lines for Native CFTR Function using Electrophysiological Assay** (Reference Example)

While both manual and automated electrophysiology assays have been developed and both can be applied to assay this system, described below is the protocol for manual patch clamp experiments.

Cells are seeded at low densities and are used 2-4 days after plating. Borosilicate glass pipettes are fire-polished to obtain tip resistances of 2-4 mega Ω. Currents are sampled and low pass filtered. The extracellular (bath) solution contains: 150 mM NaCl, 1 mM $CaCl_2$, 1 mM $MgCl_2$, 10 mM glucose, 10 mM mannitol, and 10 mM TES, pH 7.4. The pipette solution contains: 120 mM CsCl, 1 mM $MgCl_2$, 10 mM TEA-Cl, 0.5 mM EGTA, 1 mM Mg-ATP, and 10 mM HEPES (pH 7.3). Membrane conductances are monitored by alternating the membrane potential between -80 mV and -100 mV. Current-voltage relationships are generated by applying voltage pulses between -100 mV and +100 mV in 20-mV steps.

**Example 17 Generating a Stable NaV 1.7 Heterotrimer-Expressing Cell Line** (Reference Example)

***Generating Expression Constructs***

Plasmid expression vectors that allowed streamlined cloning were generated based on pCMV-SCRIPT (Stratagene) and contained various necessary components for transcription and translation of a gene of interest, including: CMV and SV40 eukaryotic promoters; SV40 and HSV-TK polyadenylation sequences; multiple cloning sites; Kozak sequences; and Neomycin/Kanamycin resistance cassettes (or Ampicillin, Hygromycin, Puromycin, Zeocin resistance cassettes).

***Generation of Cell Lines***

***Step 1: Transfection***

293T cells were cotransfected with three separate plasmids, one encoding a human NaV 1.7 α subunit (SEQ ID NO: 19), one encoding a human NaV 1.7 β1 subunit (SEQ ID NO: 20) and one encoding a human NaV 1.7 β2 subunit (SEQ ID NO: 21), using standard techniques. (Examples of reagents that may be used to introduce nucleic acids into host cells include, but are not limited to, LIPOFECTAMINE™, LIPOFECTAMINE™ 2000, OLIGOFECTAMINE™, TFX™ reagents, FUGENE®6, DOTAP/DOPE, Metafectine or FECTURIN™.)

Although drug selection is optional to produce the cells or cell lines disclosed herein, we included one drug resistance marker per plasmid. The sequences were under the control of the CMV promoter. An untranslated sequence encoding a NaV Target Sequence for detection by a signaling probe was also present along with the sequence encoding the drug resistance marker. The NaV Target Sequences utilized were NaV Target Sequence 1 (SEQ ID NO: 22), NaV Target Sequence 2 (SEQ ID NO: 23) and NaV Target Sequence 3 (SEQ ID NO: 24). In this example, the NaV 1.7 α subunit gene-containing vector comprised NaV Target Sequence 1 (SEQ ID NO: 22); the NaV 1.7 β1 subunit gene-containing vector comprised NaV Target Sequence 2 (SEQ ID NO: 23); and the NaV 1.7 β2 subunit gene-containing vector comprised NaV Target Sequence 3 (SEQ ID NO: 24).

***Step 2: Selection***

Transfected cells were grown for 2 days in DMEM-FBS media, followed by 10 days in antibiotic-containing DMEM-FBS media. During the antibiotic containing period, antibiotics were added to the media as follows: puromycin (0.1 μg/ml), hygromycin (100 μg/ml), and zeocin (200 μg/ml).

***Step 3: Cell passaging***

Following enrichment on antibiotic, cells were passaged 6-18 times in the absence of antibiotic selection to allow time for expression that was not stable over the selected period of time to subside.

***Step 4: Exposure of cells to fluorogenic probes***

Cells were harvested and transfected with signaling probes (SEQ ID NOS: 25, 26, 27) using standard techniques. (Examples of reagents that may be used to introduce nucleic acids into host cells include, but are not limited to, LIPOFECTAMINE™, LIPOFECTAMINE™ 2000, OLIGOFECTAMINE™, TFX™ reagents, FUGENE®6, DOTAP/DOPE, Metafectine or FECTURIN™.)

NaV Signaling Probe 1(SEQ ID NO: 25) bound NaV Target Sequence 1 (SEQ ID NO: 22); NaV Signaling Probe 2 (SEQ ID NO: 26) bound NaV Target Sequence 2 (SEQ ID NO:23); and NaV Signaling Probe 3 (SEQ ID NO: 27) bound NaV Target Sequence 3 (SEQ ID NO: 24). The cells were then dissociated and collected for analysis and sorted using a fluorescence activated cell sorter (Beckman Coulter, Miami, FL).

<u>**Target Sequences detected by signaling probes**</u>

The following target sequences were used for the NaV 1.7 subunit transgenes.
**NaV Target Sequence 1**
5'-GTTCTTAAGGCACAGGAACTGGGAC-3' (SEQ ID NO: 22) (NaV 1.7 α subunit) NaV Target Sequence 2
5'-GAAGTTAACCCTGTCGTTCTGCGAC-3' (SEQ ID NO: 23) (NaV 1.7 β1 subunit)
NaV Target Sequence 3
5'-GTTCTATAGGGTCTGCTTGTCGCTC-3' (SEQ ID NO: 24) (NaV 1.7 β2 subunit)

<u>Signaling probes</u>

Supplied as 100 μM stocks.
NaV Signaling probe 1 - This probe binds target sequence 1.
5'- Cy5 GCCAGTCCCAGTTCCTGTGCCTTAAGAACCTCGC BHQ3 quench -3' (SEQ ID NO: 25)
NaV Signaling probe 2 - This probe binds target sequence 2.
5'- Cy5.5 CGAGTCGCAGAACGACAGGGTTAACTTCCTCGC BHQ3 quench -3' (SEQ ID NO: 26)
NaV Signaling probe 3 - This probe binds target sequence 3.
5'- Fam CGAGAGCGACAAGCAGACCCTATAGAACCTCGC BHQ1 quench -3' (SEQ ID NO: 27)

BHQ3 in NaV Signaling probes 1 and 2 can be replaced by BHQ2 or gold particle. BHQ1 in NaV Signaling probe 3 can be replaced by BHQ2, gold particle, or DABCYL.

In addition, a similar probe using a Quasar® Dye (BioSearch) with spectral properties similar to Cy5 was used in certain experiments. In some experiments, 5-MedC and 2-amino dA mixmer probes were used rather than DNA probes.

***Step 5: Isolation of positive cells***

Standard analytical methods were used to gate cells fluorescing above background and to isolate cells falling within the defined gate directly into 96-well plates. Flow cytometric cell sorting was operated such that a single cell was deposited per well. After selection, the cells were expanded in media lacking drug.

The following gating hierarchy was used:

coincidence gate → singlets gate → live gate → Sort gate in plot FAM vs. Cy5: 0.1-1.0% of live cells.

*Step 6: Additional cycles of steps 1-5 andlor 3-5*

Steps 1-5 and/or 3-5 were repeated to obtain a greater number of cells. At least four independent rounds of steps 1-5 were completed, and for each of these cycles, at least two internal cycles of steps 3-5 were performed for each independent round.

*Step 7: Estimation of growth rates for the populations of cells*

The plates were transferred to a Microlabstar automated liquid handler (Hamilton Robotics). Cells were incubated for 5-7 days in a 1:1 mix of fresh complete growth medium (DMEM/10% FBS) and 2-3 day conditioned growth medium, supplemented with 100 units/ml penicillin and 0.1mg/ml streptomycin. Then the cells were dispersed by trypsinization to minimize clumps and transferred to new 96-well plates. After the clones were dispersed, plates were imaged to determine confluency of wells (Genetix). Each plate was focused for reliable image acquisition across the plate. Reported confluencies of greater than 70% were not relied upon. Confluency measurements were obtained at days every 3 times over 9 days (*i.e*, between days 1 and 10 post-dispersal) and used to calculate growth rates.

*Step 8: Binning populations of cells according to growth rate estimates*

Cells were binned (independently grouped and plated as a cohort) according to growth rate between 10-11 days following the dispersal step in step 7. Bins were independently collected and plated on individual 96 well plates for downstream handling; some growth bins resulted in more than one 96-well plate. Bins were calculated by considering the spread of growth rates and bracketing a high percentage of the total number of populations of cells. Depending on the sort iteration described in Step 5, between 5 and 9 growth bins were used with a partition of 1- 4 days. Therefore, each bin corresponded to a growth rate or population doubling time difference between 8 and 14.4 hours depending on the iteration.

Cells can have doubling times from less 1 day to more than 2 weeks. In order to process the most diverse clones that at the same time can be reasonably binned according to growth rate, it is preferable to use 3-9 bins with a 0.25 to 0.7 day doubling time per bin. One skilled in the art will appreciate that the tightness of the bins and number of bins can be adjusted for the particular situation and that the tightness and number of bins can be further adjusted if cells are synchronized for their cell cycle.

*Step 9: Replica plating to speed parallel processing and provide stringent quality control*

The plates were incubated under standard and fixed conditions (humidified 37°C, 5%CO2) in antibiotics-free DMEM-10%FBS media. The plates of cells were split to produce 4 sets of target plates. These 4 sets of plates comprised all plates with all growth bins to ensure there were 4 replicates of the initial set. Up to 3 target plate sets were committed for cryopreservation (described in step 10), and the remaining set was scaled and further replica plated for passage and functional assay experiments. Distinct and independent tissue culture reagents, incubators, personnel, and carbon dioxide sources were used for downstream replica plates. Quality control steps were taken to ensure the proper production and quality of all tissue culture reagents: each component added to each bottle of media prepared for use was added by one designated person in one designated hood with only that reagent in the hood while a second designated person monitored to avoid mistakes. Conditions for liquid handling were set to eliminate cross contamination across wells. Fresh tips were used for all steps, or stringent tip washing protocols were used. Liquid handling conditions were set for accurate volume transfer, efficient cell manipulation, washing cycles, pipetting speeds and locations, number of pipetting cycles for cell dispersal, and relative position of tip to plate.

*Step 10: Freezing early passage stocks of populations of cells*

Three sets of plates were frozen at -70 to -80°C. Plates in each set were first allowed to attain confluencies of 70 to 80%. Medium was aspirated and 90%FBS and 5%-10% DMSO was added. The plates were sealed with Parafilm, individually surrounded by 1 to 5cm of foam, and then placed into a -80°C freezer.

*Step 11: Methods and conditions for initial trans formative steps to produce viable, stable and functional (VSF) cell lines*

The remaining set of plates was maintained as described in step 9. All cell splitting was performed using automated liquid handling steps, including media removal, cell washing, trypsin addition and incubation, quenching and cell dispersal steps. For some assay plating steps, cells were dissociated with cell dissociation buffer (*e.g.*, CDB, Invitrogen or CellStripper, CellGro) rather than trypsin.

***Step 12: Normalization methods to correct any remaining variability of growth rates***

The consistency and standardization of cell and culture conditions for all populations of cells was controlled. Differences across plates due to slight differences in growth rates were controlled by periodic normalization of cell numbers across plates every 2 to 8 passages. Populations of cells that were outliers were detected and eliminated.

***Step 13: Characterization of population of cells***

The cells were maintained for 3 to 8 weeks to allow for their *in vitro* evolution under these conditions. During this time, we observed size, morphology, fragility, response to trypsinization or dissociation, roundness/average circularity post-dissociation, percentage viability, tendency towards microconfluency, or other aspects of cell maintenance such as adherence to culture plate surfaces.

***Step 14: Assessment of potential functionality of populations of cells*** *under* ***VSF conditions***

Populations of cells were tested using functional criteria. Membrane potential assay kits (Molecular Devices/MDS) were used according to manufacturer's instructions. Cells were tested at multiple different densities in 96- or 384-well plates and responses were analyzed. A variety of post-plating time points were used, *e.g.*, 12-48 hours post plating. Different densities of plating were also tested for assay response differences.

***Step 15:***

The functional responses from experiments performed at low and higher passage numbers were compared to identify cells with the most consistent responses over defined periods of time, ranging from 3 to 9 weeks. Other characteristics of the cells that changed over time were also noted.

***Step 16:***

Populations of cells meeting functional and other criteria were further evaluated to determine those most amenable to production of viable, stable and functional cell lines. Selected populations of cells were expanded in larger tissue culture vessels and the characterization steps described above were continued or repeated under these conditions. At this point, additional standardization steps, such as different plating cell densities; time of passage; culture dish size/format and coating); fluidics optimization; cell dissociation optimization (*e.g.*, type, volume used, and length of time); and washing steps, were introduced for consistent and reliable passages. Temperature differences were also used for standardization (*i.e.,* 30°C vs 37°C).
n addition, viability of cells at each passage was determined. Manual intervention was increased and cells were more closely observed and monitored. This information was used to help identify and select final cell lines that retained the desired properties. Final cell lines and back-up cell lines were selected that showed consistent growth, appropriate adherence, and functional response.

***Step 17: Establishment of cell banks***

The low passage frozen plates described above corresponding to the final cell line and back-up cell lines were thawed at 37°C, washed two times with DMEM-10% FBS and incubated in humidified 37°C /5% CO2 conditions. The cells were then expanded for a period of 2-3 weeks. Cell banks for each final and back-up cell line consisting of 15-20 vials were established.

***Step 18:***

The following step can also be conducted to confirm that the cell lines are viable, stable, and functional. At least one vial from the cell bank is thawed and expanded in culture. The resulting cells are tested to determine if they meet the same characteristics for which they were originally selected.

**Example 18 Characterizing Relative Expression of Heterolocious NaV 1.7 Subunits in Stable NaV 1.7-Expressing Cell Lines** (Reference Example)

Quantitative RT-PCR (qRT-PCR) was used to determine the relative expression of the heterologous human NaV 1.7 α, β1, and β2 subunits in the produced stable NaV 1.7-expressing cell lines. Total RNA was purified from 1-3x$10^6$ mammalian cells using an RNA extraction kit (RNeasy Mini Kit, Qiagen). DNase treatment was done according to rigorous DNase treatment protocol (TURBO DNA-free Kit, Ambion). First strand cDNA synthesis was performed using a reverse transcriptase kit (SuperScript III, Invitrogen) in 20 μL reaction volume with 1 μg DNA-free total RNA and 250 ng Random Primers (Invitrogen). Samples without reverse transcriptase and sample without RNA were used as negative controls for this reaction. Synthesis was done in a thermal cycler (Mastercycler, Eppendorf) at the following conditions: 5 min at 25°C, 60 min at 50°C; reaction termination was conducted for 15 min at 70°C.

For analysis of gene expression, primers and probes for qRT-PCR (MGB TaqMan probes, Applied Biosystems) were designed to specifically anneal to the target sequences (SEQ ID NOS: 22, 23, 24). For sample normalization, control (glyceraldehyde 3-phosphate dehydrogenase (GAPDH)) Pre-Developed Assay reagents (TaqMaN, Applied Biosystems) were used. Reactions, including negative controls and positive controls (plasmid DNA), were set up in triplicates with 40 ng of cDNA in 50 μL reaction volume. The relative amounts of each of the three NaV 1.7 subunits being expressed were determined. All three subunits were successfully expressed in the produced stable NaV 1.7-expressing cell line.

**Example 19 Characterizing Stable NaV 1.7-Expressing Cell Lines for Native NaV Function using Electrophysiological Assay** (Reference Example)

Automated patch-clamp system was used to record sodium currents from the produced stable HEK293T cell lines expressing NaV 1.7 α, β1, and β2 subunits. The following illustrated protocol can also be used for QPatch, Sophion or Patchliner, Nanion systems. The extracellular Ringer's solution contained 140 mM NaCl, 4.7 mM KCl, 2.6 mM $MgCl_2$, 11 mM glucose and 5 mM HEPES, pH 7.4 at room temperature. The intracellular Ringer's solution contained 120 mM CsF, 20 mM Cs-EGTA, 1 mM $CaCl_2$, 1 mM $MgCl_2$, and 10 mM HEPES, pH 7.2. Experiments were conducted at room temperature.

Cells stably expressing NaV 1.7 α, β1, and β2 subunits were grown under standard culturing protocols as described in **Example 17**. Cells were harvested and kept in suspension with continuous stirring for up to 4 hours with no significant change in quality or ability to patch. Electrophysiological experiment (whole-cell) was performed using the standard patch plate. The patch-clamp hole (micro-etched in the chip) is approximately 1 μm in diameter and has a resistance of ~2 MΩ. The membrane potential was clamped to a holding potential of -100 mV.

Current-voltage relation and inactivation characteristics of voltage-gated human NaV 1.7 sodium channel stably expressed in HEK293T cells were characterized. Sodium currents were measured in response to 20 ms depolarization pulses from - 80 mV to +50 mV with a holding potential of -100 mV. The resulting current-voltage (I-V) relationship for peak sodium channel currents was characterized. The activation threshold was - 35 mV (midpoint of activation, Va = - 24.9 mV +/- 3.7 mV), and the maximal current amplitude was obtained at -10 mV. The inactivation graph for the sodium channel was plotted. The membrane potential was held at a holding potential of - 100 mV, subsequently shifted to conditioning potentials ranging from -110 mV to +10 mV for 1000 ms, and finally the current was measured upon a step to 0 mV. The resulting current amplitude indicates the fraction of sodium channels in the inactivated state. At potentials more negative than - 85 mV the channels were predominantly in the closed state, whereas at potentials above -50 mV they were predominantly in the inactivated state. The curve represents the Boltzmann fit from which the $V_{1/2}$ for steady-state inactivation was estimated to be - 74 mV. The current-voltage profile for the produced stable NaV 1.7-expressing cell lines is consistent with previously reported current-voltage profile (Va= - 28.0 mV ±1.1 mV; $V_{1/2}$ = -71.3 mV ± 0.8 mV) (Sheets et al., J Physiol. 581 (Pt 3):1019-1031. (2007)).

**Example 20 Characterizing Stable NaV 1.7-Expressing Cell Lines for Native NaV Function using Membrane Potential Assay** (Reference Example)

The produced stable cells expressing NaV 1.7 α, β1, and β2 subunits were maintained under standard cell culture conditions in Dulbecco's Modified Eagles medium supplemented with 10% fetal bovine serum, glutamine and HEPES. On the day before assay, the cells were harvested from stock plates using cell dissociation buffer, *e.g.*, CDB (GIBCO) or cell-stripper (Mediatech), and plated at 10,000 - 25,000 cells per well in 384 well plates in growth media. The assay plates were maintained in a 37°C cell culture incubator under 5%CO2 for 22-24 hours. The media were then removed from the assay plates and blue fluorescence membrane potential dye (Molecular Devices Inc.) diluted in load buffer (137 mM NaCl, 5 mM KCl,1.25 mM $CaCl_2$, 25 mM HEPES, 10 mM glucose) was added. The cells were incubated with blue membrane potential dye for 1 hour at 37°C. The assay plates were then loaded onto the high-throughput fluorescent plate reader (Hamamastu FDSS). The fluorescent plate reader measures cell fluorescence in images taken of the cell plate once per second and displays the data as relative florescence units.

The assay response of stable NaV 1.7-expressing cells and control cells *(i.e.,* HEK293T parental cells) to addition of buffer and channel activators (*i.e.,* veratridine and scorpion venom (SV)) were measured. In a first addition step (*i.e.,* Addition 1), only buffer was added, with no test compounds added. If desired, test compounds can be added in this step. In a second addition step, veratridine and scorpion venom, which are sodium channels activators, were diluted in assay buffer to the desired concentration (*i.e.,* 25 μM veratridine and 5-25 μg/ml scorpion venom) and added into 384 well polypropylene microtiter plates. Once bound, veratridine and scorpion venom proteins modulate the activity of voltage-gated sodium channels through a combination of mechanisms, including an alteration of the activation and inactivation kinetics. The resulted activation of sodium channels in stable NaV 1.7-expressing cells changes cells membrane potential and the fluorescent signal increases. The above-described functional assay can also be used to characterize the relative potencies of test compounds at NaV 1.7 ion channels.

**Example 21 Characterizing Regulation of NaV 1.7 Alpha Subunit by Beta Subunits** (Reference Example)

***Regulation of Alpha Subunit Gene Expression by Beta Subunits***

Pools of HEK293T cells were engineered to express various ratios of α and β subunits by manipulating the molar ratios of independent plasmid DNAs or α and control plasmids (*e.g.*, α:β1:β2 = 1:1:1). After drug selection the subunits expression in six different cell pools were evaluated with qRT-PCR as described in **Example 18.** Comparative qRT-PCR indicated that α subunit expression in drug-selected cells detection was increased when all three human NaV 1.7 subunits (*i.e.,* α, β1, and β2) were co-transfected in compared to only α subunit and control plasmid transfected. The presence of the β subunit transcripts affects α subunit gene expression, demonstrating the importance of co-expressing all three NaV 1.7 subunits for a physiologically relevant functional assay.

***Regulation of Pharmacological Properties by Beta Subunits***

A membrane potential cell-based assay was used to measure the response to test compounds of the cells stably co-expressing all three NaV 1.7 subunits (*i.e.,* α, β1, and β2) and control cells stably expressing only a NaV 1.7 α subunit. Two compounds (*i.e.,* C18 and K21) were tested in the membrane potential assay performed substantially according to the protocol in **Example 20**. Specifically for this example, the test compounds were added in the first addition step.

C18 and K21 potentiated the response of clone C44 (expressing NaV 1.7 α, β1, and β2 subunits) and blocked the response of clone C60 (expressing NaV 1.7 α subunit only). The assay response of the two test compounds was normalized to the response of buffer alone for each of the two clones.

**Example 22 Characterizing Different Subunit Combinations of NaV 1.7** (Reference Example)

A membrane potential cell-based assay was used to measure the response to test compounds of different cell lines stably co-expressing all three NaV 1.7 subunits (*i.e.,* α, β1, and β2). Dose-response analysis (DRC) for a set of compounds tested on four cell lines generated from cells positive for the expression of NaV 1.7 alpha, beta1 and beta2 subunits resulted in distinct functional profiles (Fig. 4). Multiple cell lines were found to comprise profiles similar to each of the four profiles shown in Fig. 4.

~90 NaV 1.7 cell lines co-expressing all three NaV 1.7 subunits (*i.e.,* α, β1, and β2) were grouped based on their functional pharmacological profile in cell based assays where each clonal cell line was tested using the same compounds (Fig. 5). Each cluster in Fig. 5 (Bin 1-5) represents the subset of clones with similar activity. Activity was calculated as a percentage of maximum signal inhibition, as shown for each clonal cell line in Fig. 5. Negative numbers in Fig. 5 represent percentages of potentiation.

**Table 7 Mammalian G proteins, their families and descriptions**

| **Class** | **Family**/*Subtype* | **Protein # (UniProt)** | **Description** |
|---|---|---|---|
| **G-alpha** | **$G_s$** | | |
| | *Gs* | P04896 | Galpha-s-Bos taurus |
| | *Gs* | P16052 | Galpha-s-Cricetulus longicaudatus |
| | *Gs* | P63092 | Galpha-s-Homo sapiens-2 |
| | *Gs* | P63091 | Galpha-s-Canis familiaris |

(continued)

| Class | Family/*Subtype* | Protein # (UniProt) | Description |
|---|---|---|---|
| | *Gs* | P63093 | Galpha-s-Mesocricetus auratus |
| | *Gs* | P63094 | Galpha-s-Mus musculus-2 |
| | *Gs* | P63095 | Galpha-s-Rattus norvegicus-2 |
| | *Gs* | P29797 | Galpha-s-Sus scrofa |
| | *Gs* | 060726 | Galpha-s-Homo sapiens-4 |
| | *Gs* | 075632 | Galpha-s-Homo sapiens-5 |
| | *Gs* | 075633 | Galpha-s-Homo sapiens-6 |
| | *Gs* | Q14433 | Galpha-s-Homo sapiens-7 |
| | *Gs* | Q14455 | Galpha-s-Homo sapiens |
| | *Gs* | Q8R4A8 | Galpha-s-Cricetulus griseus |
| | *Gs* | Q9JJ33 | Galpha-s-Mus musculus |
| | *Gs* | Q9JLG1 | Galpha-s-Rattus norvegicus-1 |
| | *Gs* | Q5JWF2 | Galpha-s-Homo sapiens-3 |
| | *Golf* | P38405 | Galpha-olf-Homo sapiens-2 |
| | *Golf* | Q8CGK7 | Galpha-olf-Mus musculus |
| | *Golf* | P38406 | Galpha-olf-Rattus norvegicus |
| | *Golf* | Q86XU3 | Galpha-olf-Homo sapiens-1 |
| | **$G_{i/o}$** | | |
| | *Gi* | Q29047 | Galpha-i-Sus scrofa |
| | *Gi1* | P38401 | Galpha-i1-Cavia porcellus |
| | *Gi1* | P50146 | Galpha-i1-Gallus gallus |
| | *Gi1* | P63096 | Galpha-i1-Homo sapiens-1 |
| | *Gi1* | P63097 | Galpha-i1-Bos taurus |
| | *Gi1* | P10824 | Galpha-i1-Rattus norvegicus |
| | *Gi1* | 043383 | Galpha-i1-Homo sapiens-2 |
| | *Gi1* | Q61018 | Galpha-i1-Mus musculus |
| | *Gi2* | P38400 | Galpha-i2-Canis familiaris |
| | *Gi2* | P38402 | Galpha-i2-Cavia porcellus |
| | *Gi2* | P50147 | Galpha-i2-Gallus gallus |
| | *Gi2* | P04899 | Galpha-i2-Homo sapiens-2 |
| | *Gi2* | P08752 | Galpha-i2-Mus musculus-2 |
| | *Gi2* | P04897 | Galpha-i2-Rattus norvegicus |
| | *Gi2* | Q7M3G8 | Galpha-i2-Sus scrofa |
| | *Gi2* | Q7M3G9 | Galpha-i2-Bos taurus-2 |
| | *Gi2* | Q7M3H0 | Galpha-i2-Bos taurus-1 |
| | *Gi2* | Q8JZT4 | Galpha-i2-Mus musculus-1 |
| | *Gi2* | Q96C71 | Galpha-i2-Homo sapiens-1 |
| | *Gi3* | P38403 | Galpha-i3-Cavia porcellus |

(continued)

| Class | Family/*Subtype* | Protein # (UniProt) | Description |
|---|---|---|---|
| | *Gi3* | Q60397 | Galpha-i3-Cricetulus griseus |
| | *Gi3* | P08754 | Galpha-i3-Homo sapiens |
| | *Gi3* | P08753 | Galpha-i3-Rattus norvegicus |
| | *Gi3* | Q9DC51 | Galpha-i3-Mus musculus |
| | *Go* | P59215 | Galpha-o-Rattus norvegicus |
| | *Go* | Q8N6I9 | Galpha-o-Homo sapiens |
| | *Go1* | P08239 | Galpha-o1-Bos taurus |
| | *Go1* | P59216 | Galpha-o1-Cricetulus longicaudatus |
| | *Go1* | P09471 | Galpha-o1-Homo sapiens |
| | *Go1* | P18872 | Galpha-o1-Mus musculus |
| | *Gz* | P19086 | Galpha-z-Homo sapiens-2 |
| | *Gz* | 070443 | Galpha-z-Mus musculus |
| | *Gz* | P19627 | Galpha-z-Rattus norvegicus |
| | *Gz* | Q8IY73 | Galpha-z-Homo sapiens-3 |
| | *Gz* | Q8N652 | Galpha-z-Homo sapiens-1 |
| | *Gz* | Q95LC0 | Galpha-z-Sus scrofa |
| | *Gt* | Q16162 | Galpha-t-Homo sapiens |
| | *Gt* | Q9D7B3 | Galpha-t-Mus musculus |
| | *Gt1* | P04695 | Galpha-t1-Bos taurus |
| | *Gt1* | Q28300 | Galpha-t1-Canis familiaris |
| | *Gt1* | P11488 | Galpha-t1-Homo sapiens |
| | *Gt1* | P20612 | Galpha-t1-Mus musculus |
| | *Gt2* | P04696 | Galpha-t2-Bos taurus |
| | *Gt2* | P19087 | Galpha-t2-Homo sapiens |
| | *Gt2* | P50149 | Galpha-t2-Mus musculus-2 |
| | *Gt2* | Q8BSY7 | Galpha-t2-Mus musculus-1 |
| | *Ggust* | P29348 | Galpha-gust-Rattus norvegicus |
| | **$G_{q/11}$** | | |
| | *Gq* | Q6NT27 | Galpha-q-Homo sapiens-2 |
| | *Gq* | Q28294 | Galpha-q-Canis familiaris |
| | *Gq* | P50148 | Galpha-q-Homo sapiens-1 |
| | *Gq* | P21279 | Galpha-q-Mus musculus |
| | *Gq* | P82471 | Galpha-q-Rattus norvegicus |
| | *G11* | Q71RI7 | Galpha-11-Gallus gallus |
| | *G11* | P38409 | Galpha-11-Bos taurus |
| | *G11* | P52206 | Galpha-11-Canis familiaris |
| | *G11* | P29992 | Galpha-11-Homo sapiens |
| | *G11* | P45645 | Galpha-11-Meleagris gallopavo |

(continued)

| Class | Family/*Subtype* | Protein # (UniProt) | Description |
|---|---|---|---|
| | *G11* | P21278 | Galpha-11-Mus musculus-2 |
| | *G11* | Q9JID2 | Galpha-11-Rattus norvegicus |
| | *G11* | Q8SPP3 | Galpha-11-Macaca mulatta |
| | *G11* | Q91X95 | Galpha-11-Mus musculus-1 |
| | *G14* | P38408 | Galpha-14-Bos taurus |
| | *G14* | 095837 | Galpha-14-Homo sapiens |
| | *G14* | P30677 | Galpha-14-Mus musculus-2 |
| | *G14* | Q8C3M7 | Galpha-14-Mus musculus-3 |
| | *G14* | Q8CBT5 | Galpha-14-Mus musculus-4 |
| | *G14* | Q8R2X9 | Galpha-14-Mus musculus-1 |
| | *G15* | P30678 | Galpha-15-Mus musculus |
| | *G15* | 088302 | Galpha-15-Rattus norvegicus |
| | *G16* | P30679 | Galpha-16-Homo sapiens |
| | **$G_{12/13}$** | | |
| | *G12* | Q03113 | Galpha-12-Homo sapiens |
| | *G12* | P27600 | Galpha-12-Mus musculus |
| | *G12* | Q63210 | Galpha-12-Rattus norvegicus |
| | *G13* | Q14344 | Galpha-13-Homo sapiens |
| | *G13* | P27601 | Galpha-13-Mus musculus-2 |
| | *G13* | Q8C5L2 | Galpha-13-Mus musculus-3 |
| | *G13* | Q9D034 | Galpha-13-Mus musculus-1 |
| **G-beta** | **$B_{1-5}$** | | |
| | *B1* | Q6TMK6 | Gbeta-1-Cricetulus griseus |
| | *B1* | P62871 | Gbeta-1-Bos taurus |
| | *B1* | P62872 | Gbeta-1-Canis familiaris |
| | *B1* | P62873 | Gbeta-1-Homo sapiens |
| | *B1* | P62874 | Gbeta-1-Mus musculus |
| | *B1* | P54311 | Gbeta-1-Rattus norvegicus-2 |
| | *B1* | Q9QX36 | Gbeta-1-Rattus norvegicus-1 |
| | *B2* | P11017 | Gbeta-2-Bos taurus |
| | *B2* | P62879 | Gbeta-2-Homo sapiens |
| | *B2* | P62880 | Gbeta-2-Mus musculus |
| | *B2* | P54313 | Gbeta-2-Rattus norvegicus-2 |
| | *B2* | Q9QX35 | Gbeta-2-Rattus norvegicus-1 |
| | *B3* | P79147 | Gbeta-3-Canis familiaris |
| | *B3* | P16520 | Gbeta-3-Homo sapiens-1 |
| | *B3* | Q61011 | Gbeta-3-Mus musculus |
| | *B3* | P52287 | Gbeta-3-Rattus norvegicus |

(continued)

| Class | Family/*Subtype* | Protein # (UniProt) | Description |
|---|---|---|---|
| | *B3* | Q96B71 | Gbeta-3-Homo sapiens-2 |
| | *B4* | Q9HAV0 | Gbeta-4-Homo sapiens |
| | *B4* | P29387 | Gbeta-4-Mus musculus |
| | *B4* | 035353 | Gbeta-4-Rattus norvegicus |
| | *B5* | 014775 | Gbeta-5-Homo sapiens-2 |
| | *B5* | P62881 | Gbeta-5-Mus musculus-2 |
| | *B5* | P62882 | Gbeta-5-Rattus norvegicus |
| | *B5* | Q60525 | Gbeta-5-Mesocricetus auratus |
| | *B5* | Q96F32 | Gbeta-5-Homo sapiens-1 |
| | *B5* | Q9CSQ0 | Gbeta-5-Mus musculus-3 |
| | *B5* | Q9CU21 | Gbeta-5-Mus musculus-1 |
| | $B_{unclassified}$ | | |
| | *B unclassified* | Q61621 | unclassified_Gbeta-Mus musculus-1 |
| | *B unclassified* | Q8BMQ1 | unclassified_Gbeta-Mus musculus-2 |
| | *B unclassified* | Q9UFT3 | unclassified_Gbeta-Homo sapiens |
| G-gamma | $\gamma_{1-12}$ | | |
| | *γ1* | Q8R1U6 | Ggamma-1-Mus musculus |
| | *γ2* | P59768 | Ggamma-2-Homo sapiens |
| | *γ2* | P63212 | Ggamma-2-Bos taurus |
| | *γ2* | P63213 | Ggamma-2-Mus musculus |
| | *γ2* | 035355 | Ggamma-2-Rattus norvegicus |
| | *γ3* | P63214 | Ggamma-3-Bos taurus |
| | *γ3* | P63215 | Ggamma-3-Homo sapiens |
| | *γ3* | P63216 | Ggamma-3-Mus musculus |
| | *γ3* | 035356 | Ggamma-3-Rattus norveqicus |
| | *γ4* | P50150 | Ggamma-4-Homo sapiens |
| | *γ4* | P50153 | Ggamma-4-Mus musculus |
| | *γ4* | 035357 | Ggamma-4-Rattus norvegicus |
| | *γ5* | P63217 | Ggamma-5-Bos taurus |
| | *γ5* | P63218 | Ggamma-5-Homo sapiens-2 |
| | *γ5* | Q80SZ7 | Ggamma-5-Mus musculus |
| | *γ5* | P63219 | Ggamma-5-Rattus norvegicus |
| | *γ5* | Q9Y3K8 | Ggamma-5-Homo sapiens-1 |
| | *γ7* | P30671 | Ggamma-7-Bos taurus |
| | *γ7* | 060262 | Ggamma-7-Homo sapiens |
| | *γ7* | Q61016 | Ggamma-7-Mus musculus |
| | *γ7* | P43425 | Ggamma-7-Rattus norvegicus |
| | *γ8* | Q9UK08 | Ggamma-8-Homo sapiens-2 |

(continued)

| Class | Family/*Subtype* | Protein # (UniProt) | Description |
|---|---|---|---|
| | *γ8* | P63078 | Ggamma-8-Mus musculus-2 |
| | *γ8* | P63077 | Ggamma-8-Rattus norvegicus |
| | *γ8* | P50154 | Ggamma-8-Bos taurus |
| | *γ8* | 014610 | Ggamma-8-Homo sapiens-1 |
| | *γ8* | Q61017 | Ggamma-8-Mus musculus-1 |
| | *γ10* | P50151 | Ggamma-10-Homo sapiens-2 |
| | *γ10* | 035358 | Ggamma-10-Rattus norvegicus |
| | *γ10* | Q96BN9 | Ggamma-10-Homo sapiens-1 |
| | *γ10* | Q9CXP8 | Ggamma-10-Mus musculus |
| | *γ11* | P61952 | Ggamma-11-Homo sapiens |
| | *γ11* | P61953 | Ggamma-11-Mus musculus |
| | *γ11* | P61954 | Ggamma-11-Rattus norvegicus |
| | *γ12* | Q28024 | Ggamma-12-Bos taurus |
| | *γ12* | Q9UBI6 | Ggamma-12-Homo sapiens |
| | *γ12* | Q9DAS9 | Ggamma-12-Mus musculus |
| | *γ12* | 035359 | Ggamma-12-Rattus norvegicus |
| | *γ13* | Q9P2W3 | Ggamma-13-Homo sapiens |
| | *γ13* | Q9JMF3 | Ggamma-13-Mus musculus |
| | *γ11* | P02698 | Ggamma-t1-Bos taurus |
| | *γ11* | P63211 | Ggamma-t1-Homo sapiens |
| | *γ11* | P63210 | Ggamma-t1-Canis familiaris |
| | *γ11* | Q61012 | Ggamma-t1-Mus musculus |
| | $\gamma$**unclassified** | | |
| | *γ unclassified* | Q7M3H1 | unclassified_Ggamma-Bos indicus |
| Note: Cells may express various combinations of any of these proteins. | | | |

**Table 8 Human orphan GPCRs including their gene symbols and NCBI gene ID numbers**

| Family | Human Gene Symbol | Human Gene ID |
|---|---|---|
| Bombesin | BRS3 | 680 |
| Free fatty acid | GPR42P | 2866 |
| N-Formylpeptide family | FPRL2 | 2359 |
| Nicotinic acid | GPR81 | 27198 |
| Opsin-like | OPN3 | 23596 |
| OrphanA2 | GPR52 | 9293 |
| OrphanA2 | GPR21 | 2844 |
| OrphanA3 | GPR78 | 27201 |
| OrphanA3 | GPR26 | 2849 |

(continued)

| Family | Human Gene Symbol | Human Gene ID |
|---|---|---|
| OrphanA4 | GPR37 | 2861 |
| OrphanA4 | GPR37L1 | 9283 |
| OrphanA6 | GPR63 | 81491 |
| OrphanA6 | GPR45 | 11250 |
| OrphanA7 | GPR83 | 10888 |
| OrphanA9 | GRCAe | 27239 |
| OrphanA9 | GPR153 | 387509 |
| OrphanA12 | P2RY5 | 10161 |
| OrphanA13 | P2RY10 | 27334 |
| Orphan13 | GPR174 | 84636 |
| OrphanA14 | GPR142 | 350383 |
| Orphan14 | GPR139 | 124274 |
| OrphanA5 | ADMR | 11318 |
| OrphanA15 | CMKOR1 | 57007 |
| OrphanLGR | LGR4 | 55366 |
| OrphanLGR | LGR5 | 8549 |
| OrphanLGR | LGR6 | 59352 |
| OrphanSREB | GPR85 | 54329 |
| OrphanSREB | GPR27 | 2850 |
| OrphanSREB | GPR173 | 54328 |
| Orphan (chemokine receptor-like) | CCRL2 | 9034 |
| Orphan (Mas-related) | MAS1 | 4142 |
| Orphan (Mas-related) | MAS1 L | 116511 |
| Orphan (Mas-related) | MRGPRE | 116534 |
| Orphan (Mas-related) | MRGPRF | 116535 |
| Orphan (Mas-related) | MRGPRG | 386746 |
| Orphan (Mas-related) | MRGX3e | 117195 |
| Orphan (Mas-related) | MRGX4e | 117196 |
| Orphan (melatonin-like) | GPR50 | 9248 |
| Orphan (P2Y-like) | GPR87 | 53836 |
| Orphan (trace amine-like) | TRAR3f | 134860 |
| Orphan (trace amine-like) | TRAR4 | 319100 |
| Orphan (trace amine-like) | TRAR5 | 83551 |
| Orphan (trace amine-like) | PNRe | 9038 |
| Orphan (trace amine-like) | GPR57g | 9288 |
| Orphan (trace amine-like) | GPR58 | 9287 |
| Other orphan genes | EBI2 | 1880 |
| Other orphan genes | GPR160 | 26996 |

(continued)

| Family | Human Gene Symbol | Human Gene ID |
|---|---|---|
| Other orphan genes | GPRe | 11245 |
| Other orphan genes | GPR1 | 2825 |
| Other orphan genes | GPR101 | 83550 |
| Other orphan genes | GPR135 | 64582 |
| Other orphan genes | OPN5 | 221391 |
| Other orphan genes | GPR141 | 353345 |
| Other orphan genes | GPR146 | 115330 |
| Other orphan genes | GPR148 | 344561 |
| Other orphan genes | GPR149 | 344758 |
| Other orphan genes | GPR15 | 2838 |
| Other orphan genes | GPR150 | 285601 |
| Other orphan genes | GPR152 | 390212 |
| Other orphan genes | GPR161 | 23432 |
| Other orphan genes | GPR17 | 2840 |
| Other orphan genes | GPR171 | 29909 |
| Other orphan genes | GPR18 | 2841 |
| Other orphan genes | GPR19 | 2842 |
| Other orphan genes | GPR20 | 2843 |
| Other orphan genes | GPR22 | 2845 |
| Other orphan genes | GPR25 | 2848 |
| Other orphan genes | GPR31 | 2853 |
| Other orphan genes | GPR32 | 2854 |
| Other orphan genes | GPR33 | 2856 |
| Other orphan genes | GPR34 | 2857 |
| Other orphan genes | GPR55 | 9290 |
| Other orphan genes | GPR61 | 83873 |
| Other orphan genes | GPR62 | 118442 |
| Other orphan genes | GPR79h | 27200 |
| Other orphan genes | GPR82 | 27197 |
| Other orphan genes | GPR84 | 53831 |
| Other orphan genes | GPR88 | 54112 |
| Other orphan genes | GPR92 | 57121 |
| Other orphan genes | P2RY8 | 286530 |
| Other orphan genes | GPR151 | 134391 |
| LNB7TM | GPR64 | 10149 |
| LNB7TM | GPR56 | 9289 |
| LNB7TM | GPR115 | 221393 |
| LNB7TM | GPR114 | 221188 |

(continued)

| Family | Human Gene Symbol | Human Gene ID |
|---|---|---|
| LNB7TM:Brain specific angiogenesis inhibitor | BAI1 | 575 |
| LNB7TM:Brain specific angiogenesis inhibitor | BAI2 | 576 |
| LNB7TM:Brain specific angiogenesis inhibitor | BAI3 | 577 |
| LNB7TM:Proto-cadherin | CELSR1 | 9620 |
| LNB7TM:Proto-cadherin | CELSR2 | 1952 |
| LN B7TM:Proto-cadh erin | CELSR3 | 1951 |
| LNB7TM:EGF, mucin-like receptor | EMR1 | 2015 |
| LNB7TM:EGF, mucin-like receptor | EMR2 | 30817 |
| LNB7TM | GPR97 | 222487 |
| LNB7TM | GPR110 | 266977 |
| LNB7TM | GPR111 | 222611 |
| LNB7TM | GPR112 | 139378 |
| LNB7TM | GPR113 | 165082 |
| LNB7TM | GPR116 | 221395 |
| LNB7TM | MASS1 | 84059 |
| LNB7TM | ELTD1 | 64123 |
| LNB7TM | GPR123 | 84435 |
| LNB7TM | GPR124 | 25960 |
| LNB7TM | GPR125 | 166647 |
| LNB7TM | GPR126 | 57211 |
| LNB7TM | GPR128 | 84873 |
| LNB7TM | GPR144 | 347088 |
| LNB7TM:EGF, mucin-like receptor | EMR3 | 84658 |
| LNB7TM:EGF, mucin-like receptor | EMR4b | 326342 |
| LNB7TM | CD97 | 976 |
| LNB7TM:Latrophilin substrate | LPHN2 | 23266 |
| LNB7TM:Latrophilin substrate | LPHN3 | 23284 |
| LNB7TM:Latrophilin substrate | LPHN1 | 22859 |
| Unclassified | GPR157 | 80045 |
| GABAB | GPR51 | 9568 |
| GABAB | GPR156 | 165829 |
| Calcium sensor | GPRC6A | 222545 |
| GPRC5 | GPRC5A | 9052 |
| GPRC5 | GPRC5B | 51704 |
| GPRC5 | GPRC5C | 55890 |
| GPRC5 | GPRC5D | 55507 |
| Unclassified | GPR158 | 57512 |
| Unclassified | GPR158L1 | 342663 |

**Table 9 List of Human opioid receptors**

| Name |
|---|
| Opioid receptors, which can include the following subunits: Mu (OPRM1), spliced forms 1 and 2; Delta (OPRD1), spliced form 1; Kappa (OPRK1), spliced form 1; Sigma (Oprs1), spliced forms 1, 2, 3, 4, 5 |
| Opioid like Receptor (OPRL1), spliced forms 1 and 2; |
| Opioid binding protein/cell adhesion molecule-like (OPCML), sliced form 1; |
| Opioid growth factor receptor (OGFR), spliced forms 1 and 2; |
| Opioid growth factor receptor-like 1 (OGFRL1), spliced form 1 |

**Table 10 List of Human olfactory receptors**

| Name/Common Name |
|---|
| ORL1003/; ORL1004/; ORL1011/OR6W1; ORL1015/OR3A3; ORL1016/; ORL1017/OR1 D4; ORL1018/; ORL1019/OR2B2; ORL1020/OR6W1; ORL1022/OR1 F2; ORL1023/OR1 F1; ORL1025/OR3A3; ORL1026/OR8D2; ORL1027/OR7D2; ORL1028/OR7A17; ORL1029/OR5V1; ORL1030/OR12D3; ORL1032/OR2J2; ORL1033/; ORL154/OR2H3; ORL165/OR511; ORL166/OR2F1; ORL167/; ORL19/OR1 D2; ORL1L1/OR1L1; ORL20/OR1E5; ORL203/OR2M4; ORL204/; ORL205/OR7E19P; ORL206/OR8G2; ORL207/OR4D1; ORL208/; ORL209/OR8G1; ORL21/OR10J1; ORL210/OR7E18P; ORL211/OR1C1; ORL212/; ORL213/; ORL214/OR7A5; ORL229/; ORL230/OR7A5; ORL231/; ORL249/; ORL253/OR2T1; ORL254/OR1N3; ORL255/OR1E6; ORL256/OR1F10; ORL257/; ORL260/; ORL262/; ORL263/; ORL264/OR1E2; ORL265/; ORL266/; ORL267/; ORL268/OR3A2; ORL269/; ORL27Q/OR1F10; ORL271/OR2B6; |

(continued)

| Name/Common Name |
| --- |
| ORL272/OR2A5; ORL273/OR2F3; ORL274/OR2F3; ORL282/OR2H7; ORL283/; ORL3001/OR10K1; ORL3002/OR6Y1; ORL3003/OR2T4; ORL3004/OR10Z1; ORL3005/OR6N2; ORL3006/OR5BF1; ORL3007/OR5AV1; ORL3008/OR5AT1; ORL3009/OR11L1; ORL3010/OR6K6; ORL3011/OR10T2; ORL3012/OR10R2; ORL3013/OR2T5; ORL3014/OR6P1; ORL3015/OR2L8; ORL3016/OR13G1; ORL3017/OR2L8; ORL3018/OR10J5; ORL3019/OR6N1; ORL3020/OR6F1; ORL3023/OR10K2; ORL3024/OR6K2; ORL3025/OR5AX1; ORL3026/OR2C4; ORL3027/OR5AY1; ORL3028/; ORL3029/OR1C1; ORL3030/; ORL3032/; ORL3033/OR10J6; ORL3034/OR6K3; ORL3037/OR2L4P; ORL3038/OR2T6P; ORL3039/OR2L3; ORL3040/OR2T3; ORL3041/OR5AY1; ORL3042/OR2G2; ORL3043/OR2G3; ORL3044/; ORL3045/; ORL3046/; ORL3047/; ORL3048/; ORL3049/; ORL305/; ORL3050/; ORL3051/; ORL3052/; ORL3053/; ORL3054/OR2G2; ORL3055/OR2T2P; ORL3056/OR10T1P; ORL3057/OR10R1P; ORL3058/OR10R3P; ORL3059/OR2W3P; ORL3060/OR2AS1P; ORL3061/OR2AK1P; ORL3062/OR10X1P; ORL3063/OR6K1P; ORL3064/OR6K4P; ORL3065/OR6K5P; ORL3066/OR2AQ1P; ORL3067/OR2L5P; ORL3068/OR10AA1P; ORL3069/OR10J2P; ORL307/; ORL3070/OR10J3P; ORL3071/OR2L7P; ORL3072/OR2L9P; ORL3073/OR2AJ1P; ORL3074/OR2T8P; ORL3075/OR6R1P; ORL3076/OR2L6P; ORL3077/OR2T7P; ORL3078/OR7E26P; ORL3079/OR11I1P; ORL308/OR214P; ORL3080/OR10AE1P; ORL3081/OR9H1P; ORL3082/OR7E102; ORL3083/OR7E89P; ORL3084/OR7E90P; ORL3085/OR7E91 P; ORL3086/OR7E62P; ORL3087/OR7E46P; ORL3088/OR7E107P; ORL3089/OR6B2P; ORL309/; ORL3090/OR5S1P; ORL3091/OR6B3P; ORL3092/OR4G6P; ORL3093/OR5H2; ORL3094/OR5H6; ORL3095/OR5K2; ORL3096/OR7E55P; ORL3097/OR7E66P; ORL3098/OR5H4P; ORL3099/OR5H5P; ORL310/; ORL3100/OR5H7P; ORL3101/OR5H8P; ORL3102/OR7E29P; ORL3103/OR7E93P; ORL3104/OR7E53P; ORL3105/OR7E97P; ORL3106/OR5BM1P; ORL3107/OR5H3P; ORL3108/OR5AC1P; ORL3109/OR7E121P; ORL311/; ORL3110/OR7E122P; ORL3111/OR7E127P; ORL3112/OR7E129P; ORL3113/OR5G1P; ORL3114/OR7E131P; ORL3115/OR7E132P; ORL3116/OR7E100P; ORL3117/OR5B5P; ORL3118/OR7E83P; ORL3119/OR7E84P; ORL312/OR10H2; ORL3120/OR7E85P; ORL31202/OR2A3P; ORL3121/OR7E86P; ORL3122/OR7E43P; ORL3123/OR7E94P; ORL3124/OR7E99P; ORL3125/OR7E103P; ORL3126/OR4H11P; ORL3127/OR8N1P; ORL3128/OR7E35P; ORL3129/OR5M14P; ORL313/OR10H3; ORL3130/OR7E130P; ORL3131/OR2Y1; ORL3132/OR2V3; ORL3133/OR2AI1P; ORL3134/OR1X1P; ORL3135/OR2V1P; ORL3136/OR4H5P; ORL3137/OR5U1; ORL3138/OR4F12; ORL3138/OR4F12; ORL3139/OR1F12; ORL314/OR1I1; ORL314/; ORL3140/OR4F14; ORL3141/OR4F16; ORL3143/OR4F15; ORL3145/OR2B8; ORL3146/OR2W6P; ORL3147/OR2I2; ORL3148/; ORL3149/OR4F2P; ORL315/; ORL3150/OR2P1P; ORL3151/OR4F1P; ORL3152/OR7E22P; ORL3153/OR2U2P; ORL3154/OR2U1P; ORL3155/OR2H5P; ORL3156/OR2G1P; ORL3157/OR2AD1P; ORL3158/OR12D1P; ORL3159/OR2W4P; ORL316/; ORL3160/OR2W2P; ORL3161/OR2B7P; ORL3162/OR4F13P; ORL3163/OR2W7P; ORL3164/OR5B7P; ORL3165/OR2J1P; |

(continued)

| Name/Common Name |
| --- |
| ORL3166/OR2N1P; ORL3167/OR2J4P; ORL3168/OR2H4P; ORL3169/OR2E1P; ORL317/; ORL3170/OR2B4P; ORL3171/OR2AE1; ORL3172/OR6V1; ORL3173/OR9A2; ORL3174/OR9A4; ORL3175/OR2A6; ORL3176/OR2A16P; ORL3177/OR2A12P; ORL3178/; ORL3179/OR2F2; ORL3181/OR2A7; ORL3183/OR2Q1P; ORL3184/OR7E38P; ORL3185/OR7E7P; ORL3186/OR2R1P; ORL3187/OR10AC1P; ORL3188/OR4G4P; ORL3189/OR4F7P; ORL3190/OR9P1P; ORL3191/OR9A1P; ORL3192/OR2A11P; ORL3193/OR2A2P; ORL3194/OR2A13P; ORL3195/OR2A14P; ORL3196/OR2A15P; ORL3197/OR9A3P; ORL3198/OR9N1P; ORL3199/OR7E118P; ORL32/; ORL3200/OR7E9P; ORL3201/OR2A17P; ORL3203/OR2A9P; ORL3204/OR2V2; ORL3205/OR9L1P; ORL3206/OR4D4P; ORL3207/OR4K8P; ORL3208/OR7E96P; ORL3209/OR5B1P; ORL3210/OR5D11P; ORL3211/OR7E50P; ORL3212/OR7E8P; ORL3213/OR7E80P; ORL3214/OR7E10P; ORL3215/OR7E125P; ORL3216/OR1L8; ORL3218/OR1L3; ORL3219/OR1K1; ORL3220/OR2AR1P; ORL3221/OR2K2; ORL3222/OR13C3; ORL3225/OR13C8; ORL3226/OR13C9; ORL3227/OR5C2P; ORL3228/OR13C2; ORL3229/OR13F1; ORL3231/OR13J1; ORL3232/OR1J1; ORL3233/OR13C7; ORL3234/OR1B1; ORL3235/; ORL3236/; ORL3237/; ORL3238/; ORL3239/; ORL3240/OR2S2; ORL3242/OR13D1; ORL3243/OR1Q1; ORL3244/OR1L4; ORL3245/OR5C1; ORL3246/OR1N2; ORL3247/; ORL3248/OR13C1P; ORL3249/OR1311P; ORL325/OR7E108P; ORL326/OR13D2P; ORL3264/OR13D3P; ORL3265/OR13A1; ORL3266/OR6D1P; ORL3267/OR7E110P; ORL3268/OR7E68P; ORL3269/OR7E115P; ORL3270/OR6L1P; ORL3271/OR6L2P; ORL3272/OR7M1P; ORL3273/OR6D2P; ORL3274/OR10G6P; ORL3275/OR10G6P; ORL3277/OR9G4; ORL3278/OR9Q1; ORL3279/OR9G5; ORL3281/OR2AG1; ORL3282/OR52E1; ORL3283/OR56A1; ORL3284/OR5P3; ORL3285/OR52L1; ORL3286/OR52L2; ORL3287/OR52J3; ORL3288/OR10G4; ORL3289/OR8D4; ORL3290/OR10G7; ORL3291/OR51M1; ORL3292/OR4D5; ORL3293/OR52E4; ORL3294/OR52E5; ORL3295/OR5M10; ORL3296/OR5T2; ORL3297/OR52N4; ORL3298/OR56A6; ORL3299/OR51E1; ORL33/OR1D4; ORL3300/OR51 A7; ORL3301/OR5A1; ORL3302/OR5A2; ORL3303/OR5A2; ORL3305/OR5112; ORL3306/OR4A4; ORL3307/OR5AS1; ORL3308/OR4A5; ORL3309/OR1S2; ORL3310/OR5B13; ORL3311/OR4P4; ORL3312/OR10V1; ORL3313/OR4C15; ORL3314/OR5M3; ORL3315/OR1S1; ORL3316/OR5M3; ORL3317/OR8D1; ORL3318/OR52M1P; ORL3319/OR10D4; ORL3320/OR56A4; ORL3321/OR8K1; ORL3322/OR5M8; ORL3323/OR4X1; ORL3324/OR52N2; ORL3325/OR51S1; ORL3326/OR52B4; ORL3327/OR5AK3; ORL3328/OR5F1; ORL3329/OR8J3; ORL3330/OR8K5; ORL3331/OR52A1; ORL3332/OR8A1; ORL3333/OR8B12; ORL3334/OR52E8; ORL3335/OR4C12; ORL3336/OR4C13; ORL3337/OR5G3; ORL3338/OR5T3; ORL3339/OR1A2; ORL3339/OR1A2; ORL334/; ORL3340/OR5AU1; ORL3342/OR52H1; ORL3343/OR4F17; ORL3344/OR5R1P; ORL3345/; ORL3346/OR11.18.02; ORL3347/; ORL3348/; ORL3349/; ORL335/; ORL3350/; ORL3351/; ORL3352/; ORL3353/; ORL3354/; ORL3355/; ORL3356/; ORL3357/; ORL3358/; ORL3359/; ORL336/; ORL3360/; ORL3361/; ORL3362/; ORL3363/; ORL3364/; ORL3365/; ORL3366/; ORL3367/OR9I1; ORL3368/OR6B1; ORL3369/OR6M1; ORL337/; ORL3370/OR51L1; ORL3371/OR51A2; ORL3372/OR52E2; ORL3373/OR5P2; ORL3374/OR10S1; ORL3375/OR10S1; |

(continued)

| Name/Common Name |
|---|
| ORL3376/OR51H1; ORL3377/OR10G8; ORL3378/OR6T1; ORL3379/OR4B1; ORL3380/OR51Q1; ORL3381/OR52N1; ORL3383/OR4X2; ORL3384/OR5M9; ORL3385/OR8K3; ORL3386/OR52E6; ORL3387/OR2AG1; ORL3388/OR56B2; ORL3389/OR1M1; ORL339/; ORL3390/OR51G2; ORL3391/OR51F2; ORL3392/OR5D16; ORL3393/OR10Q1; ORL3394/OR5D18; ORL3395/OR5D18; ORL3396/OR5L1; ORL3397/OR51 E2; ORL3398/OR51D1; ORL3399/OR5AR1; ORL34/OR7D2; ORL3400/OR5M1; ORL3401/OR5AP2; ORL34010/OR8H3; ORL3403/OR52B2; ORL3404/OR52K2; ORL3405/OR52K2; ORL3406/OR52B4; ORL3407/OR51I1; ORL3409/OR8I2; ORL341/; ORL3411/OR4A15; ORL3412/OR4D9; ORL3413/OR5B16; ORL3414/OR10A6; ORL3415/OR5B17; ORL3416/OR8H1; ORL3417/OR52P1; ORL3418/OR51T1; ORL3419/OR52R1; ORL342/; ORL3420/OR56B4; ORL3421/OR4D6; ORL3422/OR8B8; ORL3423/OR8B4; ORL3424/OR52B6; ORL3425/OR4C6; ORL3426/OR5D14; ORL3427/OR6Q1; ORL3428/OR52I1; ORL3429/OR52I2; ORL343/; ORL3430/OR2D3; ORL3431/OR52W1P; ORL3432/OR2D2; ORL3433/OR5M11; ORL3434/OR8G3P; ORL3435/OR4C16; ORL3436/OR52N5; ORL3438/OR6X1; ORL3439/OR4A16; ORL3440/OR51C1 P; ORL3441/OR51J1P; ORL3442/OR51R1P; ORL3443/OR9I2P; ORL3444/OR51 A4; ORL3445/OR51G1; ORL3446/OR5D13; ORL3447/OR8J1; ORL3449/OR9G1; ORL345/OR8B3; ORL3450/OR52K1; ORL3451/OR5B2; ORL3452/OR52D1; ORL3453/OR5AN1; ORL3454/OR5AK2; ORL3456/OR8B2; ORL3457/; ORL3458/; ORL3459/; ORL3460/; ORL3461/; ORL3462/; ORL3463/; ORL3464/; ORL3465/; ORL3466/; ORL3467/; ORL3469/; ORL347/; ORL3470/; ORL3471/OR10D3P; ORL3472/OR10N1P; ORL3473/OR8F1P; ORL3474/OR10D1P; ORL3476/OR7E5P; ORL3477/OR51 B3P; ORL3478/OR7E87P; ORL3479/OR7E4P; ORL3480/OR2AL1P; ORL3481/OR6M2P; ORL3482/OR5D2P; ORL3483/OR4V1P; ORL3484/OR8B10P; ORL3485/OR4P1P; ORL3486/OR51N1P; ORL3487/OR52J1P; ORL3488/OR51P1P; ORL3489/OR4C7P; ORL349/; ORL3490/OR5P1P; ORL3491/OR56A2P; ORL3492/OR5E1P; ORL3493/OR56A3P; ORL3494/OR52X1P; ORL3495/OR56A5P; ORL3496/OR52E3P; ORL3497/OR51A3P; ORL3498/OR4C9P; ORL3499/OR52J2P; ORL35/; ORL350/; ORL3500/OR4R1P; ORL3501/OR4C10P; ORL3502/OR51A5P; ORL3503/OR5M2P; ORL3504/OR10AB1P; ORL3505/OR52S1P; ORL3506/OR5M4P; ORL3507/OR5M5P; ORL3508/OR10G5P; ORL3509/OR5M6P; ORL351/; ORL3510/OR5M7P; ORL3511/OR5T1P; ORL3512/OR811P; ORL3513/OR8K2P; ORL3514/OR10D5P; ORL3515/OR5BD1P; ORL3516/OR5AL1P; ORL3517/OR5AL2P; ORL3518/OR10A2P; ORL3519/OR8L1P; ORL352/; ORL3520/OR5BP1P; ORL3521/OR8J2P; ORL3522/OR52N3P; ORL3523/OR4B2P; ORL3524/OR51K1P; ORL3525/OR52Q1P; ORL3526/OR52E7P; ORL3527/OR6A2P; ORL3528/OR52U1P; ORL3529/OR6M3P; ORL353/; ORL3530/OR5D3P; ORL3531/OR8B9P; ORL3532/OR56B1P; ORL3533/OR2AG2P; ORL3534/OR52Y1P; ORL3535/OR51 A6P; ORL3536/OR51F1P; ORL3537/OR7E1P; ORL3538/OR51 H2P; ORL3539/OR5BG1P; ORL354/; ORL3540/OR5W1P; ORL3541 /OR5W2P; ORL3542/OR51A8P; ORL3543/OR5D15P; ORL3544/OR9L2P; ORL3545/OR5D17P; ORL3546/OR9Q2P; ORL3547/OR5W3P; ORL3548/OR913P; ORL3549/OR51A9P; ORL355/; |

(continued)

| Name/Common Name |
|---|
| ORL3550/OR5BL1 P; ORL3551/OR9M1P; ORL3552/OR52M2P; ORL3553/OR52M3P; ORL3554/OR2AH1P; ORL3555/OR56B3P; ORL3557/OR5AM1 P; ORL3558/OR52B1P; ORL3559/OR5M12P; ORL356/OR10A4; ORL3560/OR5AP1 P; ORL3561/OR5M13P; ORL3562/OR52K3P; ORL3563/OR52B3P; ORL3564/OR5BB1P; ORL3565/OR9G2P; ORL3566/OR9G3P; ORL3567/OR51A10P; ORL3568/OR52P2P; ORL3569/OR4A2P; ORL3570/OR5AK1P; ORL3571/OR5BQ1P; ORL3572/OR4A3P; ORL3573/OR4R2P; ORL3574/OR7E117P; ORL3575/OR5F2P; ORL3576/OR5AQ1P; ORL3577/OR5J1P; ORL3578/OR5BE1P; ORL3579/OR5BN1P; ORL3580/OR8K4P; ORL3581/OR7E11P; ORL3582/OR7A3P; ORL3583/OR7E3P; ORL3584/OR4A6P; ORL3585/OR4A7P; ORL3586/OR8C1P; ORL3587/OR4A8P; ORL3588/OR7E15P; ORL3589/OR4A9P; ORL359/; ORL3590/OR4A10P; ORL3591/OR4A1P; ORL3592/OR4A12P; ORL3593/OR4A13P; ORL3594/OR4A14P; ORL3595/OR51 C3P; ORL3596/OR51B1P; ORL3597/OR8B6P; ORL3598/OR8B5P; ORL3599/OR8B7P; ORL36/OR2L2; ORL360/OR2A1; ORL3600/OR10D6P; ORL3601/OR8C3P; ORL3602/OR4P3P; ORL3603/OR8B1P; ORL3604/OR4D7P; ORL3605/OR4D8P; ORL3606/OR2AT1P; ORL3607/OR4D10P; ORL3608/OR4C11P; ORL3609/OR4D11P; ORL361 /; ORL3610/OR55C1P; ORL3611/OR55B1P; ORL3612/OR52V1P; ORL3613/OR52T1P; ORL3614/OR52H2P; ORL3615/OR52B5P; ORL3616/OR5BA1P; ORL3617/OR5AZ1P; ORL3618/OR5B14P; ORL3619/OR5B15P; ORL362/; ORL3620/OR51A11P; ORL3621/OR8R1P; ORL3622/OR5AN2P; ORL3623/OR5BR1 P; ORL3624/OR10W1P; ORL3625/OR5B18P; ORL3626/OR56A7P; ORL3627/OR5BC1P; ORL3628/OR10Q2P; ORL3629/OR5B19P; ORL363/OR10C1; ORL3630/OR4A17P; ORL3631/OR10V2P; ORL3632/OR5AK4P; ORL3633/OR10Y1P; ORL3634/OR7E14P; ORL3635/OR4R3P; ORL3636/OR4A18P; ORL3637/OR4A19P; ORL3638/OR4A20P; ORL3639/OR10V3P; ORL364/; ORL3640/OR7E2P; ORL3641/OR7E13P; ORL3642/OR7E126P; ORL3643/OR801P; ORL3644/OR7E128P; ORL3645/OR5P4P; ORL3646/OR5G4P; ORL3647/OR4S2P; ORL3648/OR5G5P; ORL3649/OR8A2P; ORL3650/OR7E12P; ORL3651/OR4A1P; ORL3652/OR4A21 P; ORL3653/OR4C1P; ORL3654/OR4C14P; ORL3655/OR10A7; ORL3656/OR9K2; ORL3657/OR10P1P; ORL3658/OR10AD1P; ORL3659/OR9K1P; ORL366/; ORL3660/OR10P3P; ORL3661/; ORL3662/; ORL3663/; ORL3664/OR7E95P; ORL3665/OR5BK1P; ORL3666/OR11M1 P; ORL3667/OR9R1P; ORL3668/OR10P2P; ORL3669/OR2A18P; ORL367/OR12D2; ORL3670/OR7A19P; ORL3671 /OR2AP1 P; ORL3672/OR6U1P; ORL3673/OR10U1P; ORL3674/OR11 H2P; ORL3675/OR7E101P; ORL3676/OR7E104P; ORL3677/OR7E111P; ORL3678/OR7E37P; ORL3679/OR7E33P; ORL368/; ORL3680/OR5B10P; ORL3681/OR4K2; ORL3682/OR4K3; ORL3683/OR6J2; ORL3684/OR4K5; ORL3685/OR4N5; ORL3686/OR11 H4; ORL3687/OR11G2; ORL3688/OR4L1; ORL369/; ORL3690/OR4K15; ORL3691 /OR4K17; ORL3692/; ORL3693/OR4N2; ORL3694/OR6S1; ORL3695/OR10G3; ORL3697/OR10G2; ORL3698/OR4E2; ORL3699/OR11H6; ORL37/; ORL3700/OR4K14; ORL3701/OR4K1; |

(continued)

| Name/Common Name |
|---|
| ORL3702/OR6J1P; ORL3703/OR6E1P; ORL3704/OR4N1P; ORL3705/OR4K4P; ORL3706/OR4K6P; ORL3707/OR7E105P; ORL3708/OR7E106P; ORL3709/OR11G1P; ORL371/; ORL3710/OR11H5P; ORL3711/OR4U1P; ORL3712/OR4L2P; ORL3713/OR4Q2P; ORL3714/OR4K16P; ORL3715/OR4T1P; ORL3716/OR4H8P; ORL3717/OR4E1P; ORL3718/OR10G1P; ORL3719/OR7K1P; ORL372/; ORL3720/OR7A12P; ORL3721/OR4N4; ORL3722/OR4N4; ORL3723/OR4M2; ORL3725/; ORL3726/OR4Q1P; ORL3727/OR11K1P; ORL3728/OR4N3P; ORL3729/OR4H6P; ORL373/; ORL3730/OR11H3P; ORL3731/OR4H10P; ORL3732/OR11J1 P; ORL3733/OR11J2P; ORL3734/OR8B11P; ORL3735/OR11I2P; ORL3736/OR4C5P; ORL3737/OR4S1; ORL3738/OR4C3; ORL3739/OR4C2P; ORL374/OR1E9P; ORL3740/OR4C4P; ORL3741/OR4F11P; ORL3742/OR4G5P; ORL3743/OR2C2P; ORL3744/OR4G1P; ORL3745/OR4D2; ORL3746/OR3A2; ORL37465/OR7G3; ORL3747/OR1G1; ORL3748/; ORL3749/OR1E3P; ORL375/OR1R3P; ORL375/; ORL3750/OR1 R1 P; ORL3751/OR4K7P; ORL3752/OR1R2P; ORL3753/OR1D3P; ORL3756/OR4K9P; ORL3757/OR4K10P; ORL3758/OR5D12P; ORL3759/OR5D5P; ORL376/; ORL3760/OR10H4; ORL3761/OR10H5; ORL3762/OR7G1; ORL3763/OR2Z1; ORL3764/OR2Z1; ORL3766/OR7D4P; ORL3767/OR7C1; ORL3768/OR4F19; ORL3769/; ORL377/; ORL3770/OR7A2; ORL3771/OR7G2; ORL3772/OR4F18; ORL3773/OR7A10; ORL3774/; ORL3775/; ORL3776/OR5AH1 P; ORL3777/OR7D1P; ORL3778/OR7E24P; ORL3779/OR7E19P; ORL378/; ORL3780/OR7D4P; ORL3781/OR7E25P; ORL3782/OR7E16P; ORL3783/OR4F8P; ORL3784/OR4F9P; ORL3785/OR7E98P; ORL3786/OR1AB1P; ORL3787/OR7A18P; ORL3788/OR7A1P; ORL3789/OR10B1P; ORL379/; ORL3790/OR7H1P; ORL3791/OR7A11P; ORL3792/OR7A15P; ORL3793/OR7A8P; ORL3794/OR7A14P; ORL3795/OR4G3P; ORL3796/OR4G7P; ORL3797/OR4G8P; ORL3798/OR7E92P; ORL3799/OR4K11P; ORL38/OR1J4; ORL38/; ORL380/; ORL3800/OR4K12P; ORL3801/OR7E23P; ORL3802/OR11H1; ORL3805/OR2D1; ORL3806/OR1F11; ORL3807/OR2A19; ORL3808/OR7E120; ORL3809/OR2M1; ORL381/; ORL3810/OR5AC2; ORL3811/OR5B3; ORL3812/OR6C2; ORL3813/OR52A2; ORL3814/OR4Q3; ORL3815/OR6C1; ORL3816/OR2A20; ORL3817/OR2M2; ORL3818/OR2A21; ORL3819/OR6C3; ORL382/; ORL3820/OR1E7; ORL3821/; ORL3822/; ORL3823/; ORL3824/; ORL3825/; ORL3826/; ORL3827/; ORL3828/; ORL3829/; ORL383/; ORL3830/; ORL3831/; ORL3832/; ORL3833/; ORL3834/; ORL3835/; ORL3836/; ORL3837/; ORL3838/; ORL3839/; ORL384/; ORL3840/; ORL3841/; ORL3842/; ORL3843/; ORL3844/; ORL3845/; ORL3846/; ORL3847/; ORL3848/; ORL3849/; ORL385/; ORL3850/; ORL3851/; ORL3852/; ORL3853/; ORL3854/; ORL3855/; ORL3856/; ORL3857/; ORL3858/; ORL3859/; ORL386/; ORL3860/; ORL3861/; ORL3862/; ORL3863/; ORL3864/; ORL3865/; ORL3866/; ORL3867/; ORL3868/; ORL3869/; ORL387/; ORL3870/; ORL3871/; ORL3872/; ORL3873/; ORL3874/; ORL3875/; ORL3876/; ORL3877/; ORL3878/; ORL3879/; ORL388/; ORL3880/; ORL3881/; ORL3882/; ORL3883/; ORL3884/; ORL3885/; ORL3886/; ORL3887/; ORL3888/; ORL3889/; ORL389/; ORL3890/; ORL3891/; ORL3892/; ORL3893/; ORL3894/; ORL3895/; ORL3896/; ORL3897/; ORL3898/; ORL3899/; ORL39/OR2L1; ORL390/; ORL3900/; ORL3901/; ORL3902/; ORL3903/; ORL3904/; ORL3905/; ORL3906/; ORL3907/; ORL3908/; ORL3909/; |

(continued)

| Name/Common Name |
|---|
| ORL391/; ORL3910/; ORL3911/; ORL3912/; ORL3913/; ORL3914/; ORL3915/; ORL3916/; ORL3917/; ORL3918/; ORL3919/; ORL392/; ORL3920/; ORL3921/; ORL3922/; ORL3923/; ORL3924/; ORL3925/; ORL3926/; ORL3927/; ORL3928/; ORL3929/; ORL393/; ORL3930/; ORL3931/; ORL3932/; ORL3933/; ORL3934/; ORL3935/; ORL3936/; ORL3937/; ORL3938/; ORL3939/; ORL394/; ORL3940/; ORL3941/; ORL3942/; ORL3943/; ORL3944/; ORL3945/; ORL3946/; ORL3947/; ORL3948/; ORL3949/; ORL395/OR1E6; ORL395/OR1E5; ORL3950/; ORL3951/; ORL3952/; ORL3953/; ORL3954/; ORL3955/; ORL3956/; ORL3957/; ORL3958/; ORL3959/; ORL396/OR2A5; ORL3960/; ORL3961/; ORL3962/; ORL3963/; ORL3964/; ORL3965/; ORL3966/; ORL3967/; ORL3968/; ORL3969/; ORL3970/; ORL3971/; ORL3972/; ORL3973/; ORL3974/; ORL3975/; ORL3976/; ORL3977/; ORL3978/; ORL3979/; ORL3980/; ORL3981/; ORL3982/; ORL3983/; ORL3984/; ORL3985/; ORL3986/; ORL3987/; ORL3988/; ORL3989/; ORL3990/; ORL3991 /; ORL3992/; ORL3993/; ORL3994/; ORL3995/; ORL3996/; ORL3997/; ORL3998/; ORL3999/; ORL40/; ORL400/; ORL4000/; ORL4001/; ORL4002/; ORL4003/; ORL4004/; ORL4005/; ORL4006/; ORL4007/; ORL4008/; ORL4009/; ORL401/; ORL4010/; ORL4011 /; ORL4012/; ORL4013/; ORL4014/; ORL4015/; ORL4016/; ORL4017/; ORL4018/; ORL4019/; ORL4020/; ORL4021/; ORL4022/; ORL4023/; ORL4024/; ORL4025/; ORL4026/; ORL4027/; ORL4028/; ORL4029/; ORL4030/; ORL4031/; ORL4032/; ORL4033/; ORL4034/; ORL4035/; ORL4036/; ORL4037/; ORL4038/; ORL4039/; ORL4040/; ORL4041/; ORL4042/; ORL4043/; ORL4044/; ORL4045/; ORL4046/; ORL4047/; ORL4048/; ORL4049/; ORL4050/; ORL4051/; ORL4052/; ORL4053/; ORL4054/; ORL4055/; ORL4056/; ORL4057/; ORL4058/; ORL4059/; ORL4060/; ORL4061/; ORL4062/; ORL4063/; ORL4064/; ORL4065/; ORL4066/; ORL4067/; ORL4068/; ORL4069/; ORL4070/; ORL4071/OR7E88P; ORL4072/OR2AF1P; ORL4073/OR13K1P; ORL4074/OR1AA1P; ORL4075/OR7L1P; ORL4076/OR2AF2P; ORL4077/OR3B1P; ORL4078/OR5AW1P; ORL4079/OR5BH1P; ORL4080/OR2W5P; ORL4081 /OR51 C2P; ORL4082/OR5BJ1P; ORL4083/OR2C5P; ORL4084/OR5B12P; ORL4085/OR7E39P; ORL4086/OR7E27P; ORL4087/OR5D10P; ORL4088/OR213P; ORL4089/OR7E119P; ORL4090/OR7E47P; ORL4091/OR7E42P; ORL4092/OR2M3P; ORL4093/OR7E57P; ORL4094/OR7E34P; ORL4095/OR7E56P; ORL4096/OR7E21 P; ORL4097/OR7E45P; ORL4098/OR7E77P; ORL4099/OR7E81 P; ORL41/OR9A1P; ORL4100/OR7E44P; ORL4101/OR2I5P; ORL4102/OR7E59P; ORL4103/OR7E28P; ORL4104/OR7E54P; ORL4105/OR7E48P; ORL4106/OR51 E3P; ORL4107/OR7E40P; ORL4109/OR2I7P; ORL4110/OR7E30P; ORL4111/OR2I8P; ORL4112/OR52A3P; ORL4113/OR2I9P; ORL4114/OR7E20P; ORL4115/OR2A22P; ORL4116/OR5BH2P; ORL4117/OR1E8P; ORL4118/OR4W1P; ORL4119/OR7E124P; ORL4120/OR10J4P; ORL4121/OR7E123P; ORL4122/OR7E36P; ORL4123/OR4G2P; ORL4124/OR2H2; ORL4125/; ORL4128/OR13E2; ORL42/; ORL420/; ORL423/OR2C1; ORL43/OR5K1; ORL430/; ORL45/OR10A3; ORL4501/; ORL4502/; ORL4503/; ORL4504/; ORL4505/; ORL4506/; ORL4507/; ORL4508/; ORL4509/; ORL4510/; ORL4511/; ORL4512/; ORL4513/; ORL4514/; ORL4515/; ORL4516/; ORL4517/; ORL4518/; ORL4519/; ORL4520/; ORL4521/; ORL4522/; ORL4523/; ORL4524/; ORL4525/; ORL4526/; ORL4527/; ORL4528/; ORL4529/; ORL4530/; ORL4531/; ORL4533/; ORL4535/; ORL4536/; ORL4537/; ORL4538/; |

(continued)

| Name/Common Name |
| --- |
| ORL4539/; ORL4540/; ORL4541 /; ORL4542/; ORL4543/; ORL4544/; ORL4545/; ORL4546/; ORL4547/; ORL4548/; ORL4549/; ORL4550/; ORL4551/; ORL4552/; ORL4553/; ORL4554/; ORL4555/; ORL4556/; ORL4557/; ORL4558/; ORL4559/; ORL4560/; ORL4561/; ORL4562/; ORL4563/; ORL4565/; ORL4566/; ORL4567/; ORL4568/; ORL4569/; ORL4570/; ORL4571/; ORL4572/; ORL4573/; ORL4574/; ORL4575/; ORL4576/; ORL4577/; ORL4578/; ORL4580/; ORL4581/; ORL4582/; ORL4583/; O R L4584/; ORL4585/; ORL4586/; ORL4587/; ORL4588/; ORL4589/; ORL459/OR1D5; ORL4590/; ORL4591/; ORL4592/; ORL4594/; ORL4595/; ORL4597/; ORL4598/; ORL4599/; ORL46/OR1F2; ORL4600/; ORL4601/; ORL4602/; ORL4603/; ORL4604/; ORL4605/; ORL4606/; ORL4607/; ORL4608/; ORL4609/; ORL4610/; ORL4611/; ORL4612/; ORL4613/; ORL4614/; ORL4615/; ORL4616/; ORL4617/; ORL4618/; ORL4619/; ORL462/; ORL4620/; ORL4621/; ORL4622/; ORL4623/; ORL4624/; ORL4625/; ORL4626/; ORL4628/; ORL4629/; ORL4630/; ORL4631/; ORL4632/; ORL4633/; ORL4634/; ORL4635/; ORL4636/; ORL4637/; ORL4638/; ORL4639/; ORL4640/; ORL4641/; ORL4642/; ORL4643/; ORL4644/; ORL4645/OR7E52P; ORL4646/; ORL4647/; ORL4648/; ORL4649/; ORL4650/; ORL4651/; ORL4652/; ORL4653/; ORL4654/; ORL4655/; ORL4656/; ORL4657/; ORL4658/; ORL4659/; ORL4660/; ORL4661/; ORL4662/; ORL4663/; ORL4664/; ORL4665/; ORL4666/; ORL4667/; ORL4668/; ORL4669/; ORL4670/; ORL4671/; ORL4672/; ORL4673/; ORL4674/; ORL4675/; ORL4676/; ORL4677/; ORL4678/; ORL4679/; ORL4680/; ORL4681/; ORL4682/; ORL4683/; ORL4684/; ORL4685/; ORL4686/; ORL4687/; ORL4688/; ORL4689/; ORL4690/; ORL4691 /; ORL4692/; ORL4693/; ORL4694/; ORL4695/; ORL4696/; ORL4697/; ORL4698/; ORL4699/; ORL47/OR5H1; ORL4700/; ORL4701/; ORL4702/; ORL4703/; ORL4704/; ORL4705/; ORL4706/; ORL4707/; ORL4708/; ORL4709/; ORL4710/; ORL4711/; ORL4712/; ORL4713/; ORL4714/; ORL4715/; ORL4716/; ORL4717/; ORL4718/; ORL4719/; ORL4720/; ORL4721/; ORL4722/; ORL4723/; ORL4724/; ORL4725/; ORL4726/; ORL4727/; ORL4728/; ORL4729/; ORL4730/; ORL4731/; ORL4732/; ORL4733/; ORL4734/; ORL4735/; ORL4736/; ORL4737/; ORL4738/; ORL4739/; ORL4740/; ORL4741/; ORL4742/; ORL4743/; ORL4744/; ORL4745/; ORL4746/; ORL4747/; ORL4748/; ORL4749/; ORL4750/; ORL4751/; ORL4752/; ORL4753/; ORL4754/; ORL4755/; ORL4756/; ORL4757/; ORL4758/; ORL4759/; ORL4760/; ORL4761/; ORL4762/; ORL4763/; ORL4764/; ORL4765/; ORL4766/; ORL4767/; ORL4768/; ORL4769/; ORL4770/; ORL4771/; ORL4772/; ORL4773/; ORL4774/; ORL4775/; ORL4776/; ORL4777/; ORL4778/; ORL4779/; ORL4780/; ORL4781/; ORL4782/; ORL4783/; ORL4784/; ORL4785/; ORL4786/; ORL4787/; ORL4788/; ORL4789/; ORL4790/; ORL4791/; ORL4792/; ORL4793/; ORL4794/; ORL4795/; ORL4796/; ORL4797/; ORL4798/; ORL4799/; ORL48/OR1J2; ORL4800/; ORL4801/; ORL4802/; ORL4803/; ORL4804/; ORL4805/; ORL4806/; ORL4807/; ORL4808/; ORL4809/; ORL481/OR51 B2; ORL4810/; ORL4811/; ORL4812/; ORL4813/; ORL4814/; ORL4815/; ORL4816/; ORL4817/; ORL4818/; ORL4819/; ORL482/OR51 B4; ORL4820/; ORL4821/; ORL4822/; ORL4823/; ORL4824/; ORL4825/; ORL4826/; ORL4827/; ORL4828/; ORL4829/; ORL483/OR2D2; ORL4830/; ORL4831/; ORL4832/; ORL4833/; ORL4834/; ORL4835/; ORL4836/; ORL4837/; ORL4838/; ORL4839/; ORL484/OR10A5; ORL4840/; ORL4841/; ORL4842/; ORL4843/; ORL4844/; ORL4845/; ORL4846/; ORL4847/; ORL4848/; ORL4849/; ORL485/; ORL4850/; ORL4851/; ORL4852/; ORL4853/; ORL4854/; ORL4855/; ORL4856/; ORL4857/; ORL4858/; ORL4859/; ORL486/; ORL4860/; ORL4861/; ORL4862/; ORL4862/; ORL4863/; ORL4864/; |

(continued)

| Name/Common Name |
| --- |
| ORL4865/; ORL4866/; ORL4867/; ORL4868/; ORL4869/; ORL487/; ORL4870/; ORL4871/; ORL4872/; ORL4873/; ORL4874/; ORL4875/; ORL4876/; ORL4877/; ORL4878/; ORL4879/; ORL4880/; ORL4881/; ORL4882/; ORL4883/; ORL4884/; ORL4885/; ORL4886/; ORL4887/; ORL4888/; ORL4889/; ORL4890/; ORL4891/; ORL4892/; ORL4893/; ORL4894/; ORL4895/; ORL4896/; ORL4897/; ORL4898/; ORL4899/; ORL49/OR5L2; ORL4900/; ORL4901/; ORL4902/; ORL4903/; ORL4904/; ORL4905/; ORL4906/; ORL4907/; ORL4908/; ORL4909/; ORL491/; ORL4910/; ORL4911 /; ORL4912/; ORL4913/; ORL4914/; ORL4915/; ORL4916/; ORL4917/; ORL4918/; ORL4919/; ORL492/OR1P1P; ORL4920/; ORL4921/; ORL4922/; ORL4923/; ORL4924/; ORL4925/; ORL4926/; ORL4927/; ORL4928/; ORL4929/; ORL493/OR5M11; ORL4930/; ORL4931/; ORL4932/; ORL4933/; ORL4934/; ORL4935/; ORL4936/; ORL4937/; ORL4938/; ORL4939/; ORL4940/; ORL4941/; ORL4942/; ORL4943/; ORL4944/; ORL4945/; ORL4946/; ORL4947/; ORL4948/; ORL4949/; ORL4950/; ORL4951/; ORL4952/; ORL4953/; ORL4954/; ORL4955/; ORL4956/; ORL4957/; ORL4958/; ORL4959/; ORL496/; ORL4960/; ORL4961/; ORL4962/; ORL4963/; ORL4965/; ORL4966/OR4M1; ORL497/; ORL498/; ORL499/; ORL50/OR2K1; ORL500/; ORL501/; ORL504/; ORL506/OR51A1P; ORL507/; ORL508/; ORL509/OR2I6; ORL51/; ORL510/; ORL511 /OR1 D5; ORL512/OR1A1; ORL513/OR6A1; ORL520/; ORL521/OR1D2; ORL522/; ORL523/OR12D2; ORL524/OR11A1; ORL525/OR10H1; ORL526/OR10C1; ORL527/OR10H3; ORL528/OR10H2; ORL536/; ORL589/OR1E6; ORL590/; ORL593/; ORL594/OR3A1; ORL671/OR2J3; ORL672/; ORL675/OR2H1; ORL675/; ORL677/; ORL678/OR2W1; ORL68/OR1D6P; ORL680/OR2B2; ORL681/OR2B9; ORL682/OR2J2; ORL683/OR2J2; ORL684/OR2J2; ORL685/; ORL686/; ORL687/; ORL688/; ORL689/; ORL69/OR3A4; ORL690/; ORL691/; ORL692/OR2J3; ORL693/OR2B3; ORL694/OR2B3; ORL697/; ORL70/OR1E5; ORL71/; ORL72/; ORL73/; ORL732/OR2A10; ORL733/OR10H1; ORL735/; ORL737/; ORL738/OR1E2; ORL739/OR1E5; ORL74/OR3A8P; ORL740/OR1 A2; ORL741/OR1A1; ORL742/; ORL743/OR12D2; ORL75/; ORL76/; ORL77/; ORL78/OR1E5; ORL79/; /OR4F6; /OR4F3; /OR1S1; /OR10G9; /OR13H1 |

**Table 11 List of Canine olfactory receptors (their gene names)**

| Name |
|---|
| ORL147/CfOLF1; ORL148/CfOLF2; ORL149/CfOLF3; ORL150/CfOLF4; ORL168/TPCR62; ORL169/TPCR63; ORL170/TPCR64; ORL171/ TPCR71; ORL172/TPCR72; ORL173/TPCR79; ORL18/DTMT; ORL22 / DOPCRH01; ORL23/DOPCRH02; ORL24/DOPCRH07; ORL25/ DOPCRX01; ORL26/DOPCRX04; ORL27/DOPCRX07; ORL28/ DOPCRX09; ORL29/DOPCRX16; ORL30/DTPCRH02; ORL31/ DTPCRH09; ORL3439/OR4A16; ORL4130/cOR7C50P; ORL4132/ cOR7C49P; ORL4133/cOR7H8P; ORL4134/cOR13C22P; ORL4135/ cOR5BW2P; ORL4136/cOR13C20P; ORL4137/cOR5AN4P; ORL4138/ cOR10Q4P; ORL4139/cOR13Q2P; ORL4140/cOR5L3P; ORL4141/ cOR10J17P; ORL4142/cOR10J15P; ORL4143/cOR2AG5P; ORL4144/ cOR1D9P; ORL4145/cOR2AG4P; ORL4146/cOR13N1P; ORL4147/ cOR5B22P; ORL4148/cOR7C52; ORL4149/cOR4Z5; ORL4150/ cOR7D10; ORL4151/cOR1E12; ORL4152/cOR4X6; ORL4153/cOR7G14; ORL4154/cOR7H9; ORL4155/cOR5F3; ORL4156/cOR915; ORL4157/ cOR4Z4; ORL4158/cOR5M22; ORL4159/cOR9S20; ORL4160/ cOR2M12; ORL4161/cOR2L19; ORL4162/cOR7C46; ORL4163/ cOR4H14; ORL4164/cOR13C21; ORL4165/cOR7C45; ORL4166/ cOR7C44; ORL4167/cOR5D23; ORL4168/cOR4K23; ORL4169/cOR8C6; ORL4170/cOR5L7; ORL4171/cOR2A40; ORL4172/cOR11M3; ORL4173 /cOR7H7; ORL4174/cOR7C43; ORL4175/cOR3A13; ORL4176/ cOR10J21; ORL4177/cOR3A12; ORL4178/cOR8S16; ORL4179/cOR8J6; ORL4180 / cOR7C40; ORL4181/cOR2A36; ORL4182/cOR7C39; ORL4183 / cOR7H6; ORL4184/cOR12F3; ORL4185/cOR7H4; ORL4186/ cOR2AY1; ORL4187/cOR2AG6; ORL4188/cOR2A31; ORL4189/ cOR7C14; ORL4190/cOR10J16; ORL4191/cOR7H2; ORL4192/ cOR7C13; ORL4193/cOR10A9; ORL4194/cOR2D6; ORL4195/cOR7A21; ORL4196/cOR10J13; ORL4197/cOR1D7; ORL4198/cOR1L9; ORL4199/ cOR4Z1; ORL4200/cOR7C4; ORL4201/cOR13D4; ORL4202/cOR7C3; ORL4203/cOR7G4; ORL4204/CfOLF4; ORL4205/CfOLF3; ORL4206 / CfOLF2; ORL4207/CfOLF1; ORL6033/cOR10A10; ORL6034/ cOR10A11P; ORL6035/cOR10A12P; ORL6036/cOR10A13; ORL6037/ cOR10A14; ORL6038/cOR10A3; ORL6040/COR10A4P; ORL6041/ cOR10A5; ORL6041/cOR10A4P; ORL6042/cOR10A8P; ORL6042/ cOR10A5; ORL6043/cOR10A9; ORL6045/cOR10A8P; ORL6046/ cOR10A9; ORL604/cOR10AB2; ORL604/cOR10AD1; ORL6049/ COR10AD2; ORL6050/cOR10AD3; ORL6051/cOR10AG2P; ORL6052/ cOR10AH1P; ORL6053/cOR10AI1; ORL6054/cOR10AJ1P; ORL6055/ cOR10B1P; ORL6056/cOR10D1P; ORL6057/cOR10D4P; ORL6058/ cOR10D5P; ORL6059/cOR10D7; ORL6060/cOR10D8; ORL6061/ cOR10D9; ORL6062/cOR10G11; ORL6063/cOR10G12; ORL6064/ cOR10G13P; ORL6065/cOR10G11; ORL6065/cOR10G7; ORL6066/ cOR10H10; ORL6066/cOR10G12; ORL6067/cOR10G13P; ORL6068/ cOR10G7; ORL6069/cOR10H10; ORL6070/cOR10H11P; ORL6071/ cOR10H12P; ORL6072/cOR10H13; ORL6073/cOR10H14P; ORL6075/ cOR10H6P; ORL6076/cOR10H7; ORL6077/cOR10H8; ORL6078/ cOR10H9; ORL6079/cOR10J10P; ORL6080/cOR10J11P; ORL6081/ |

(continued)

| Name |
| --- |
| cOR10J12; ORL6082/cOR10J13; ORL6083/cOR10J14; ORL6084/ cOR10J15P; ORL6085/cOR10J16; ORL6086/cOR10J17P; ORL6087/ cOR10J18P; ORL6088/cOR10J19; ORL6089/cOR10J20; ORL6090/ cOR10J21; ORL6091/cOR10J22; ORL6092/cOR10J23; ORL6093/ cOR10J7P; ORL6094/cOR10K2; ORL6095/cOR10K3; ORL6096/ cOR10K4; ORL6097/cOR10n; ORL6098/cOR10N1P; ORL6099 / cOR10P4P; ORL6100/cOR10Q1; ORL6101/cOR10Q4P; ORL6101 / cOR10Q3; ORL6102/cOR10Q5; ORL6103/cOR10R4; ORL6104/ cOR10R5; ORL6105/cOR10R6P; ORL6106/cOR10R7; ORL6107 / cOR10S2P; ORL6108/cOR10T3; ORL6109/cOR10T4P; ORL6110 / cOR10V4P; ORL6111/cOR10V5; ORL6112/cOR10V6; ORL6113 / cOR10X2; ORL6114/cOR10Z1; ORL6115/cOR11G10; ORL6116 / cOR11G11; ORL6117/cOR11G1P; ORL6118/cOR11G3P; ORL6119 / cOR11G4; ORL6120/cOR11G5P; ORL6121 / cOR11 G6; ORL6122 / cOR11G7; ORL6123/cOR11G8; ORL6124 / cOR11 G9P; ORL6125/ cOR11H10; ORL6126/cOR11H11P; ORL6127 / cOR11 H7P; ORL6128 / cOR11H8; ORL6129/cOR1 H9; ORL6130 / cOR11I3; ORL6131 / cOR11 J3; ORL6132/cOR11J4; ORL6133/cOR11K3; ORL6134/cOR11K4; ORL6135 / cOR11 L2; ORL6136/cOR11M2; ORL6137/cOR11M3; ORL6138 / cOR11S1; ORL6139/cOR11S2; ORL6140/cOR12E1; ORL6141 / cOR12E2; ORL6142/cOR12E3; ORL6143/cOR12E4P; ORL6144/ cOR12E5; ORL6145/cOR12E7P; ORL6146/cOR12E8; ORL6147/ cOR12F1; ORL6148/cOR12F2P; ORL6149/cOR12G1; ORL6150 / cOR12H1P; ORL6151/cOR12J1; ORL6152/cOR13C10; ORL6153 / cOR13C11; ORL6154/cOR13C12; ORL6155/cOR13C13P; ORL6156 / cOR13C14; ORL6157/cOR13C15; ORL6158/cOR13C16; ORL6159 / cOR13C17; ORL6160/cOR13C18; ORL6161/cOR13C19; ORL6162 / cOR13C20P; ORL6163/cOR13C21; ORL6164/cOR13C22P; ORL6165 / cOR13C23; ORL6166/cOR13C9; ORL6167/cOR13D1; ORL6168 / COR13D4; ORL6169/cOR13D5; ORL6170/cOR13D6P; ORL6171 / COR13D7P; ORL6172/cOR13E3; ORL6173/cOR13F2P; ORL6174 / cOR13F3; ORL6175/cOR13F4; ORL6176/cOR13G1; ORL6177 / cOR13L1; ORL6178/cOR13L2; ORL6179/cOR13M1; ORL6180 / cOR13M2P; ORL6181/cOR13M3; ORL6182/cOR13M4; ORL6183 / cOR13N1P; ORL6184/cOR13N2; ORL6185/cOR13N3P; ORL6186 / cOR13N4; ORL6187/cOR13N5; ORL6188/cOR13P1; ORL6189 / cOR13P2P; ORL6190/cOR13P3; ORL6191/cOR13P4; ORL6192 / cOR13P5; ORL6193/cOR13Q1P; ORL6194/cOR13Q2P; ORL6195 / COR13Q3; ORL6196/cOR13R1; ORL6197/cOR13R2; ORL6198 / cOR13S1P; ORL6199/cOR1A3P; ORL6200/cOR1AB2; ORL6201 / cOR1AB3; ORL6202/cOR1AD1; ORL6203/cOR1AE1; ORL6204 / cOR1AF1; ORL6205/cOR1AG1P; ORL6206/cOR1D10; ORL6207 / cOR1D11P; ORL6208/cOR1D12; ORL6209/cOR1D7; ORL6210 / cOR1D8; ORL6211/cOR1D9P; ORL6212/cOR1E10; ORL6213/cOR1E11; ORL6214/cOR1E12; ORL6215/cOR1F14P; ORL6216 / cOR1 F15; ORL6217/cOR112; ORL6218/cOR1J6; ORL6219/cOR1K2; ORL6220/ cOR1L6; ORL6221/cOR1L8; ORL6222/cOR1L9; ORL6223/cOR1M1P; ORL6224 / cOR1 M2; ORL6225/cOR1P1P; ORL6226 / cOR1 P2; ORL6227 / cOR1R4; ORL6228/cOR1S3P; ORL6229/cOR1X2; ORL6230 / |

(continued)

| Name |
|---|
| cOR2A13P; ORL6231/cOR2A29; ORL6232/cOR2A30; ORL6233 / cOR2A31; ORL6234/cOR2A32; ORL6235/cOR2A33; ORL6236 / cOR2A34P; ORL6237/cOR2A35; ORL6238/cOR2A36; ORL6239 / cOR2A37; ORL6240/cOR2A38; ORL6241/cOR2A39; ORL6242 / cOR2A40; ORL6243/COR2A7; ORL6244/cOR2AG1; ORL6245/ cOR2AG4P; ORL6246/cOR2AG5P; ORL6247/cOR2AG6; ORL6248/ cOR2AG7; ORL6249/cOR2AG8; ORL6250/cOR2AG9; ORL6251 / cOR2AI2; ORL6252/cOR2AK3; ORL6253/cOR2AT5P; ORL6254/ cOR2AT6; ORL6255/cOR2AT7; ORL6256/cOR2AT8P; ORL6258/ cOR2AV1; ORL6259/cOR2AV2; ORL6260/cOR2AV3; ORL6261 / cOR2AX1P; ORL6262/cOR2AX2; ORL6263/cOR2AZ1; ORL6264/ cOR2B10P; ORL6265/cOR2B2P; ORL6266/cOR2B7P; ORL6267/ cOR2B9; ORL6268/cOR2BA1P; ORL6269 / cOR2C1; ORL6270 / cOR2C6; ORL6271/cOR2D10P; ORL6272 / cOR2D2; ORL6273/cOR2D4; ORL6274 /cOR2D5P; ORL6275/cOR2D6; ORL6276/cOR2D7P; ORL6277/ cOR2D8; ORL6278/cOR2D9; ORL6279/cOR2G4; ORL6280/cOR2G5; ORL6281/cOR2H8; ORL6282 / cOR2H9P; ORL6283/cOR2K2; ORL6284 / cOR2L15P; ORL6285/cOR2L16; ORL6286/cOR2L17; ORL6288/ cOR2L18; ORL6289/cOR2L19; ORL6290/cOR2M10; ORL6291/ cOR2M11; ORL6292/cOR2M12; ORL6293/cOR2M8; ORL6294/ cOR2M9P; ORL6295/cOR2Q1P; ORL6296/cOR2S3P; ORL6297/ cOR2T1; ORL6298/cOR2T13; ORL6299/cOR2T14P; ORL6300/ cOR2T15; ORL6301/cOR2T16P; ORL6302/cOR2T17; ORL6303 / cOR2T18P; ORL6304/cOR2T19; ORL6305/cOR2T20; ORL6306/ cOR2T21; ORL6307/cOR2T22; ORL6308/cOR2T23; ORL6309/ cOR2T24; ORL6310/cOR2T25; ORL6311/cOR2T26; ORL6312/cOR2V4; ORL6313/cOR2W10; ORL6314/cOR2W11; ORL6315/cOR2W12; ORL6316/cOR2W13P; ORL6317/cOR2W14; ORL6318/cOR2W15; ORL6319/cOR2W16P; ORL6320 / cOR2W9; ORL6321/cOR2Y2; ORL6322/cOR2Z2; ORL6323/cOR2Z3; ORL6324/cOR2Z4; ORL6325 / cOR3A10; ORL6326/cOR3A11; ORL6327/cOR3A12; ORL6328 / cOR3A13; ORL6329/cOR3A9; ORL6330 / cOR3n; ORL6331 / cOR4A26; ORL6332/cOR4A27; ORL6333/cOR4A28; ORL6334/cOR4A29; ORL6335 /COR4A30; ORL6336/cOR4A31P; ORL6337/cOR4A32P; ORL6338 / cOR4A33P; ORL6339/cOR4A34; ORL6340/cOR4A35; ORL6341 / cOR4A36; ORL6342/cOR4A37P; ORL6343/cOR4A38; ORL6344/ cOR4A39; ORL6345/cOR4A4P; ORL6346/cOR4B1; ORL6347/cOR4B3P; ORL6348/cOR4B4; ORL6349/cOR4C11 P; ORL6350/cOR4C18; ORL6351/cOR4C19; ORL6352/cOR4C1P; ORL6353/cOR4C20P; ORL6354/cOR4C21; ORL6355/cOR4C22P; ORL6356/cOR4C23P; ORL6357/cOR4C24; ORL6358/cOR4C25P; ORL6359/cOR4C26; ORL6360/cOR4C27; ORL6361/cOR4C28; ORL6362/cOR4C29; ORL6363/cOR4C3; ORL6364/cOR4C30; ORL6365/cOR4C31; ORL6366 /cOR4C32; ORL6367/cOR4C33P; ORL6368/cOR4C34; ORL6369 / cOR4C35; ORL6371/cOR4C36; ORL6372/cOR4C37; ORL6373 / cOR4C38; ORL6374/cOR4C39P; ORL6375/cOR4C40; ORL6376 / cOR4C41P; ORL6377/cOR4C42; ORL6378/cOR4C43; ORL6379 / cOR4C44; ORL6380/cOR4D11P; ORL6381/cOR4D13; ORL6382 / cOR4D14P; ORL6383/cOR4D15; ORL6384/cOR4D2P; ORL6385/ |

(continued)

| Name |
|---|
| cOR4D5; ORL6386/cOR4E1P; ORL6387/cOR4E3P; ORL6388/cOR4F22; ORL6389/cOR4F23P; ORL6390/cOR4F24P; ORL6391/cOR4F25; ORL6392/cOR4F26P; ORL6393/cOR4F27P; ORL6394/cOR4G10; ORL6395/cOR4G7P; ORL6396/cOR4G8; ORL6397/cOR4G9; ORL6398/ cOR4H13; ORL6399/cOR4H14; ORL6400/cOR4K15P; ORL6401/ cOR4K18; ORL6402/cOR4K19P; ORL6403/cOR4K20; ORL6404/ cOR4K21P; ORL6405/cOR4K22; ORL6406/cOR4K23; ORL6407/ cOR4K24; ORL6408/cOR4K6P; ORL6409/cOR4L1; ORL6410/cOR4L3P; ORL6411/cOR4L4; ORL6412/cOR4M3P; ORL6413/cOR4M3P; ORL6414 / cOR4N5; ORL6415/cOR4N6; ORL6416/cOR4P10; ORL6417/cOR4P5P; ORL6418/cOR4P6; ORL6419/cOR4P7; ORL6420/cOR4P8; ORL6421 / cOR4P9; ORL6422/cOR4Q4; ORL6423/cOR4Q5; ORL6424/cOR4Q6; ORL6425/cOR4Q7; ORL6426/cOR4S3; ORL6427/cOR4S4; ORL6428 / cOR4S5; ORL6429/cOR4S6; ORL6430/cOR4S7P; ORL6431/cOR4T2P; ORL6432/cOR4X3; ORL6433/cOR4X4; ORL6434/cOR4X5P; ORL6435/ cOR4X6; ORL6436/cOR4Y1; ORL6437/cOR4Y2; ORL6438/cOR4Y3P; ORL6439/cOR4Y4; ORL6440/cOR4Y5; ORL6441/cOR4Z1; ORL6442 / cOR4Z2; ORL6443/cOR4Z3; ORL6444/cOR4Z4; ORL6445 / cOR4Z5; ORL6446/cOR51A14P; ORL6447/cOR51A15P; ORL6448/cOR51A16; ORL6449/cOR51A17; ORL6450/cOR51A18; ORL6451/cOR51A19; ORL6452/cOR51A20P; ORL6453/cOR51 A21; ORL6454/cOR51 AA1; ORL6455/cOR51B10; ORL6456/cOR51 B4; ORL6457/cOR51B7; ORL6458/cOR51 B8P; ORL6459/cOR51 B9; ORL6460/cOR51C4; ORL6461/cOR51C5; ORL6462/cOR51C6P; ORL6463/cOR51C7P; ORL6464/cOR51D2P; ORL6465/cOR51 E2P; ORL6466/cOR51 E4; ORL6467/cOR51F2P; ORL6468/cOR51 F2P; ORL6469/cOR51G2; ORL6470/cOR51G4; ORL6471/cOR51 H3; ORL6472/cOR51 H4; ORL6473/cOR51H5; ORL6474/cOR51I1P; ORL6475/cOR51I2; ORL6476 / cOR5113; ORL6477/cOR51J3; ORL6478/cOR51 K1P; ORL6479 / cOR51I4P; ORL6480/cOR51K2; ORL6481/cOR51 L2; ORL6482 / cOR51L2; ORL6483/cOR51 M1; ORL6484/cOR51 P3; ORL6485/ cOR5101P; ORL6486/cOR51 Q2P; ORL6487/cOR51Q3; ORL6488/ cOR51R2; ORL6489/cOR51T2; ORL6490/cOR51 V2; ORL6491 / cOR51V3; ORL6492/cOR51 V4; ORL6494/cOR51 V5P; ORL6494/ cOR51V5P; ORL6495/cOR51 V6; ORL6495/cOR51 V6; ORL6496/ cOR51V7; ORL6497/cOR51W1; ORL6498/cOR51X1; ORL6499 / cOR51X2; ORL6500/cOR51X3P; ORL6501/cOR51X4; ORL6502 / cOR51Z1P; ORL6503/cOR52A10; ORL6504/cOR52A11; ORL6505 / cOR52A12; ORL6506/cOR52A13; ORL6507/cOR52A14; ORL6508 / cOR52A15; ORL6509/cOR52A16P; ORL6510/cOR52A17; ORL6511/ cOR52A6; ORL6512/cOR52A7; ORL6513/cOR52A8; ORL6514/ cOR52A9; ORL6515/COR52AA1P; ORL6516/cOR52AB1; ORL6517/ cOR52AB2; ORL6518/cOR52AB3; ORL6519/cOR52AB4; ORL6520/ cOR52AC1; ORL6521/cOR52AD1; ORL6522/cOR52AE1; ORL6523/ cOR52B10P; ORL6524/cOR52B2; ORL6525/cOR52B6; ORL6526/ cOR52B7; ORL6527/cOR52B8; ORL6528/cOR52B9P; ORL6529/ cOR52D1P; ORL6530/cOR52D2; ORL6531/cOR52D3; ORL6532/ cOR52D4P; ORL6533/cOR52E10P; ORL6534/cOR52E11P; ORL6535 / cOR52E12: ORL6536/cOR52E13: ORL6537/cOR52E14; ORL6538/ |

(continued)

| Name |
| --- |
| cOR52E15P; ORL6539/cOR52E16P; ORL6540/cOR52E17; ORL6541/ cOR52E18; ORL6542/cOR52E19P; ORL6543/cOR52E2; ORL6544/ cOR52E20P; ORL6545/cOR52E4; ORL6546/cOR52E8; ORL6547/ cOR52E9; ORL6548/cOR52H1; ORL6550/cOR52H10P; ORL6551/ cOR52H11; ORL6552/cOR52H2P; ORL6553/cOR52H3P; ORL6554/ cOR52H4; ORL6555/cOR52H5; ORL6556/cOR52H6; ORL6557/ cOR52H7; ORL6558/cOR52H8; ORL6559/cOR52H9; ORL6560/ cOR52I2; ORL6561/cOR52J5; ORL6562/cOR52J6P; ORL6563/ cOR52J7; ORL6564/cOR52J8; ORL6565/cOR52J9P; ORL6566/ cOR52K4; ORL6567/cOR52K5; ORL6568/cOR52K6; ORL6569/ cOR52L3; ORL6570/cOR52M1P; ORL6571/cOR52M5; ORL6572/ cOR52M6P; ORL6573/cOR52N10; ORL6574/cOR52N11; ORL6575/ cOR52N12P; ORL6576/cOR52N2P; ORL6577/cOR52N6P; ORL6578/ cOR52N7P; ORL6579/cOR52N8; ORL6580/cOR52N9; ORL6581/ cOR52P1P; ORL6582/cOR52P2P; ORL6583/cOR52P3; ORL6584/ cOR52R2; ORL6585/cOR52R3P; ORL6586/cOR52S2; ORL6587/ cOR52S3; ORL6588/cOR52S4P; ORL6589/cOR52S5; ORL6590/ cOR52U2; ORL6591/cOR52U3P; ORL6592/cOR52V2; ORL6593/ cOR52W2; ORL6594/cOR52X2; ORL6595/cOR52X3; ORL6596/ cOR52Z2; ORL6597/cOR52Z3; ORL6598/cOR52Z4; ORL6599 / cOR52Z5; ORL6600/cOR55B3; ORL6601/cOR55D1; ORL6602 / cOR56A10; ORL6603/cOR56A11; ORL6604/cOR56A12; ORL6605/ cOR56A13P; ORL6606/cOR56A14; ORL6607/cOR56A15; ORL6608/ cOR56A16; ORL6609/cOR56A17; ORL6610/cOR56A18; ORL6611/ ; cOR56A19P; ORL6612/COR56A20; ORL6613/cOR56A21P; ORL6614/ cOR56A22; ORL6615/cOR56A23; ORL6616/ cOR56A24; ORL6617 / cOR56A4; ORL6618/cOR56A6; ORL6619/cOR56A8; ORL6620 / cOR56A9; ORL6621/cOR56B10P; ORL6622/cOR56B11; ORL6623 / cOR56B12P; ORL6624/cOR56B2; ORL6625/cOR56B5; ORL6626/ cOR56B6; ORL6627/cOR56B7; ORL6628/cOR56B8P; ORL6629/ cOR56B9P; ORL6630/cOR5A2; ORL6631/cOR5A3; ORL6632/cOR5A4P; ORL6633/cOR5AC3; ORL6634/cOR5AK6; ORL6635/cOR5AK7; ORL6636/cOR5AL1P; ORL6637/cOR5AL3; ORL6638/cOR5AN2P; ORL6639/cOR5AN3; ORL6640/cOR5AN4P; ORL6641/cOR5AP3; ORL6642/cOR5AP4P; ORL6643/cOR5AR1 P; ORL6644/cOR5B22P; ORL6645/cOR5B23; ORL6646/cOR5B24; ORL6647/cOR5B25; ORL6648 /cOR5B26; ORL6649/cOR5B27P; ORL6650/cOR5B28; ORL6651 / cOR5B29; ORL6652/cOR5B30P; ORL6653/cOR5B31; ORL6654/ cOR5B32; ORL6655/cOR5BA2; ORL6656/cOR5BC2; ORL6658/ cOR5BC3; ORL6659/cOR5BG2; ORL6660/cOR5BH3; ORL6661 / cOR5BU2; ORL6662/cOR5BV1P; ORL6663/cOR5BW1P; ORL6664 / cOR5BW2P; ORL6666/cOR5C1G; ORL6667/cOR5D14; ORL6668 / cOR5D19; ORL6669/cOR5D20; ORL6670/cOR5D21; ORL6671/ cOR5D22; ORL6672/cOR5D23; ORL6673/cOR5E1P; ORL6674/cOR5F3; ORL6675/cOR5G1P; ORL6676/cOR5G3P; ORL6677/cOR5G7P; ORL6678/cOR5G8P; ORL6679/cOR5G9; ORL6680/cOR5H10; ORL6681 /cOR5H11; ORL6682/cOR5H12; ORL6683/cOR5H13P; ORL6684/ cOR5H9; ORL6685/cOR5I1; ORL6686/cOR5I2; ORL6687/cOR5J1P; ORL6688/cOR5J3; ORL6689/cOR5J4; ORL6690/cOR5K5; ORL6691/ |

(continued)

| Name |
| --- |
| cOR5K6; ORL6692 / cOR5K7; ORL6693/cOR5L1P; ORL6694/cOR5L3P; ORL6695/cOR5L4P; ORL6696 / cOR5L5; ORL6697/cOR5L6P; ORL6698 / cOR5L7; ORL6699/cOR5M12P; ORL6700/cOR5M13P; ORL6701 / cOR5M17P; ORL6701/COR5M16; ORL6702 / cOR5M18P; ORL6703 / cOR5M19P; ORL6704/cOR5M20; ORL6705/cOR5M21; ORL6706 / cOR5M22; ORL6707 / cOR5M8; ORL6708/cOR5P4P; ORL6709/cOR5P5; ORL6710/COR5P6P; ORL6711 / cOR5R2; ORL6712 / cOR5T4; ORL6713/ cOR5T5; ORL6714 / cOR5T6; ORL6715 / cOR5T7; ORL6716 / cOR5W4; ORL6717/COR5W5; ORL6718 / cOR5W6; ORL6719 / cOR5W7; ORL6720 / cOR5W8; ORL6721/cOR6A2P; ORL6722 / cOR6AA1P; ORL6723 / cOR6AB1P; ORL6724/cOR6B4; ORL6725/cOR6B5P; ORL6726 / COR6B6; ORL6727/cOR6B7; ORL6728/cOR6B8; ORL6729/cOR6C10P; ORL6730 / cOR6C11; ORL6731 / cOR6C12; ORL6732 / cOR6C13P; ORL6733/cOR6C14; ORL6734 / cOR6C15; ORL6735 / cOR6C16P; ORL6736/cOR6C17; ORL6737/cOR6C18P; ORL6739/cOR6C19P; ORL6740 / cOR6C20P; ORL6741 / cOR6C21; ORL6742 / cOR6C22; ORL6743/cOR6C23; ORL6744/cOR6C25; ORL6745/cOR6C27; ORL6745/cOR6C26; ORL6746/cOR6C28; ORL6747/cOR6C29; ORL6748/cOR6C30; ORL6749/cOR6C31; ORL6750/cOR6C32P; ORL6751/cOR6C33; ORL6752/cOR6C34P; ORL6753/cOR6C35; ORL6754/cOR6C36; ORL6755/cOR6C37; ORL6756/cOR6C38; ORL6757/cOR6C39P; ORL6758/cOR6C4; ORL6759/cOR6C40P; ORL6760/cOR6C41P; ORL6761/cOR6C42P; ORL6762/cOR6C43; ORL6763/cOR6C44P; ORL6764/cOR6C45P; ORL6765/ cOR6C46; ORL6766/cOR6C47P; ORL6767/cOR6C48P; ORL6768/ cOR6C49P; ORL6769/cOR6C50P; ORL6770/cOR6C51; ORL6771/cOR6C52P; ORL6772/cOR6C53P; ORL6773/cOR6C54P; ORL6775/cOR6C55; ORL6776/cOR6C56P; ORL6777/ cOR6C57P; ORL6778/cOR6C58P; ORL6779/cOR6C59P; ORL6780/cOR6C5P; ORL6781 / cOR6C6; ORL6782/cOR6C60; ORL6783/cOR6C61 P; ORL6784/cOR6C62; ORL6785/cOR6C63;ORL6786/cOR6C7; ORL6787/cOR6C8; ORL6788 / cOR6C9; ORL6789 /cOR6D3P; ORL6790/cOR6D4; ORL6791 / cOR6D5; ORL6792/cOR6D6P; ORL6793/cOR6D7P; ORL6795/ cOR6K2P; ORL6796/cOR6K5P; ORL6797/cOR6K7P; ORL6798/cOR6K8; ORL6799 / cOR6K9; ORL6800/COR6M4; ORL6801/cOR6M5; ORL6802/cOR6M6; ORL6803/cOR6M7; ORL6804/cOR6M8; ORL6805/cOR6n; ORL6806 / cOR6N1; ORL6807/cOR6P1; ORL6808/cOR6Q2; ORL6809/cOR6T2; ORL6811/cOR6U3; ORL6812/cOR6V2; ORL6813/cOR6W1; ORL6814/ cOR6Y3; ORL6815/cOR6Z1; ORL6816/cOR6Z2; ORL6817/cOR6Z3; ORL6818/cOR7A21; ORL6819/cOR7A22P; ORL6820/cOR7A23; ORL6821/cOR7A24P; ORL6822/cOR7A25P; ORL6823/cOR7A26; ORL6824/cOR7A27; ORL6825/cOR7A28; ORL6826 /cOR7C10P; ORL6827/cOR7C11; ORL6828/cOR7C12P; ORL6829/cOR7C13; ORL6830/cOR7C14P; ORL6831/cOR7C15P; ORL6832/cOR7C16; ORL6833/cOR7C17; ORL6835/cOR7C18P; ORL6836/cOR7C19; ORL6837/cOR7C20; ORL6838/cOR7C21; ORL6839/cOR7C22; ORL6840/cOR7C23P; ORL6841/cOR7C24; ORL6842/cOR7C25; ORL6843/cOR7C26; ORL6844/cOR7C27; ORL6845/cOR7C28; ORL6846/cOR7C29P; ORL6847 / cOR7C3; ORL6848/cOR7C30; |

(continued)

| Name |
|---|
| ORL6849 / cOR7C31; ORL6850 / cOR7C32; ORL6852 / cOR7C33P; ORL6853 / cOR7C34; ORL6854 / cOR7C35; ORL6855 / cOR7C36; ORL6856 / cOR7C37; ORL6857 / cOR7C38; ORL6860 / cOR7C39; ORL6861 / cOR7C4; ORL6862 / cOR7C40; ORL6863 / cOR7C41; ORL6864 / cOR7C42; ORL6865 / cOR7C43; ORL6866 / cOR7C44; ORL6867 / cOR7C45; ORL6868 / cOR7C46; ORL6869 / cOR7C47; ORL6870 / cOR7C48; ORL6871 / cOR7C49P; ORL6872 / cOR7C50P; ORL6873 / cOR7C51; ORL6874 / cOR7C52; ORL6875 / cOR7C53; ORL6876 / cOR7C5P; ORL6877 / cOR7C6; ORL6878 / cOR7C7; ORL6879 / cOR7C8; ORL6880 / cOR7C9P; ORL6881 / cOR7D10; ORL6882 / cOR7D4P; ORL6883 / cOR7D5; ORL6884 / cOR7D7; ORL6885 / cOR7D8; ORL6886 / COR7D9P; ORL6887 / cOR7E152; ORL6888 / cOR7E153; ORL6889 / cOR7E154; ORL6890 / cOR7G10; ORL6891 / cOR7G11; ORL6892 / cOR7G12; ORL6893 / cOR7G13; ORL6894 / cOR7G14; ORL6895 / cOR7G4; ORL6896 / cOR7G5; ORL6898 / cOR7G6; ORL6899 / cOR7G7; ORL6900 / cOR7G8; ORL6901 / cOR7G9; ORL6902 / cOR7H2; ORL6903 / cOR7H3P; ORL6904 / cOR7H4; ORL6905 / cOR7H5P; ORL6908 / cOR7H6; ORL6909 / cOR7H7; ORL6910 / cOR7H8P; ORL6911 / cOR7H9; ORL6912 / cOR7P1; ORL6913 / cOR7R1; ORL6914 / cOR8A1 P; ORL6915 / cOR8B14; ORL6916 / cOR8B15; ORL6917 / cOR8B16; ORL6918 / cOR8B17; ORL6919 / cOR8B18; ORL6920 / cOR8B19; ORL6921 / cOR8B1P; ORL6922 / cOR8B20; ORL6923 / cOR8B21; ORL6924 / cOR8B3; ORL6925 / cOR8B8; ORL6926 / cOR8C4; ORL6927 / cOR8C5; ORL6928 / cOR8C6; ORL6929 / COR8D2P; ORL6930 / cOR8D4; ORL6931 / cOR8D5; ORL6932 / cOR8D6; ORL6933 / cOR8F2; ORL6934 / cOR8F3; ORL6935 / cOR8F4; ORL6936 / cOR8G8P; ORL6937 / cOR8G9P; ORL6938 / cOR8H4; ORL6939 / cOR813P; ORL6940 / cOR8J4; ORL6941 / cOR8J5; ORL6942 / cOR8J6; ORL6943 / cOR8J7; ORL6944 / cOR8K1; ORL6945 / cOR8K6P; ORL6946 / cOR8S10; ORL6947 / cOR8S11; ORL6948 / cOR8S12; ORL6950 / cOR8S13; ORL6951 / cOR8S14; ORL6952 / cOR8S15; ORL6953 / cOR8S16; ORL6954 / cOR8S17; ORL6955 / cOR8S18P; ORL6956 / cOR8S19P; ORL6957 / cOR8S20; ORL6958 / cOR8S2P; ORL6959 / cOR8S3P; ORL6960 / cOR8S4; ORL6961 / cOR8S5; ORL6962 / cOR8S6P; ORL6963 / cOR8S7; ORL6964 / cOR8S8; ORL6965 / cOR8S9; ORL6966 / cOR8T2; ORL6967 / cOR8T3P; ORL6968 / cOR8T4; ORL6969 / cOR8T5; ORL6970 / cOR8U2; ORL6971 / cOR8U3; ORL6973 / cOR8U4P; ORL6974 / cOR8U5; ORL6975 / cOR8U6; ORL6976 / cOR8U7; ORL6977 / cOR8V10; ORL6978 / cOR8V11; ORL6979 / cOR8V2; ORL6980 / cOR8V3; ORL6981 / cOR8V4; ORL6982 / cOR8V5; ORL6983 / cOR8V6; ORL6984 / cOR8V7P; ORL6985 / cOR8V8P; ORL6986 / cOR8V9; ORL6987 / cOR9A7; ORL6988 / cOR9A8; ORL6989 / cOR9G1; ORL6990 / cOR9G4; ORL6991 / cOR9G7; ORL6993 / cOR9G8P; ORL6995/ cOR912P; ORL6996 / cOR914P; ORL6997 / cOR915; ORL6998 / cOR9K3; ORL6999 / cOR9K4; ORL7000 / cOR9K5P; ORL7001 / cOR9K6; ORL7002 / cOR9Q3; ORL7003 / cOR9R2; ORL7004 / cOR9R3P; ORL7005 / cOR9R4; ORL7006 / cOR9S10; ORL7007 / cOR9S11; ORL7008 / cOR9S12; ORL7009 / cOR9S13; ORL7010 / cOR9S14; ORL7011 / cOR9S15; ORL7012 / cOR9S16; ORL7013 / cOR9S17; ORL7014 / cOR9S18; ORL7015/ cOR9S19; ORL7016 / cOR9S1P; ORL7017 / cOR9S2; ORL7018 / cOR9S20; ORL7019 / cOR9S21P; ORL7020 / cOR9S22P; ORL7021 / cOR9S23; |
| ORL7022 / cOR9S3P; ORL7023 / cOR9S4; ORL7024 / cOR9S5P; ORL7025 / cOR9S6; ORL7026 / cOR9S7P; ORL7027 / cOR9S8P; ORL7028 / cOR9S9P; |

**Table 12 Anopheles Gambiae Olfactory Receptors**

| Name/Common Name |
|---|
| IOR100 / GPRor53; IOR101 / GPRor54; IOR102 / GPRor55; IOR103 / GPRor56; IOR104 / GPRor57; IOR105 / GPRor58; IOR106 / GPRor59; IOR107 / GPRor60; IOR108 / GPRor61; IOR109 / GPRor62; IOR110 / GPRor63; IOR111 / GPRor64; IOR112 / GPRor65; IOR113 / GPRor66; IOR114 / GPRor67; IOR115 / GPRor68; IOR116 / GPRor69; IOR117 / GPRor70; IOR118 / GPRor71; IOR119 / GPRor72; IOR120 / GPRor73; IOR121 / GPRor74; IOR122 / GPRor75; IOR123 / GPRor76; IOR124 / GPRor77; IOR125 / GPRor78; IOR126 / GPRor79; IOR127 / GPRor12; IOR49 / GPRor1; IOR50 / GPRor2; IOR51 / GPRor3; IOR52 / GPRor4; IOR53 / GPRor5; IOR54 / GPRor6; IOR55 / GPRor7; IOR56 / GPRor8; IOR57 / GPRor9; IOR58 / GPRor10; IOR59 / GPRor11; IOR60 / GPRor13; IOR61 / GPRor14; IOR62 / GPRor15; IOR63 / GPRor16; IOR64 / GPRor17; IOR65 / GPRor18; IOR66 / GPRor19; IOR67 / GPRor20; IOR68 / GPRor21; IOR69 / GPRor22; IOR70 / GPRor23; IOR71 / GPRor24; IOR72 / GPRor25; IOR73 / GPRor26; IOR74 / GPRor27; IOR75 / GPRor28; IOR76 / GPRor29; IOR77 / GPRor30; IOR78 / GPRor31; IOR79 / GPRor32; IOR80 / GPRor33; IOR81 / GPRor34; IOR82 / GPRor35; IOR83 / GPRor36; IOR84 / GPRor37; IOR85 / GPRor38; IOR86 / GPRor39; IOR87 / GPRor40; IOR88 / GPRor41; IOR89 / GPRor42; IOR90 / GPRor43; IOR91 / GPRor44; IOR92 / GPRor45; IOR93 / GPRor46; IOR94 / GPRor47; IOR95 / GPRor48; IOR96 / GPRor49; IOR97 / GPRor50; IOR98 / GPRor51; IOR99 / GPRor52; ORL7077 / GPRor38; ORL7078 / GPRor38; ORL7079 / GPRor38; ORL7080 / GPRor38; ORL7081 / GPRor38; ORL7082 / GPRor38; ORL7083 / GPRor38; ORL7084 / GPRor38; ORL7085 / GPRor38; ORL7086 / GPRor38; ORL7087 / GPRor38; ORL7088 / GPRor38; ORL7089 / GPRor38; ORL7090 / GPRor38; ORL7091 / GPRor38; ORL7092 / GPRor38; ORL7093 / GPRor38; ORL7094 / GPRor38; ORL7095 / GPRor38; ORL7096 / GPRor38; ORL7097 / GPRor38; ORL7098 / GPRor38; ORL7099 / GPRor38; ORL7100 / GPRor38; ORL7101 / GPRor38; ORL7102 / GPRor38; ORL7103 / GPRor38; ORL7104 / GPRor38; ORL7105 / GPRor38; ORL7106 / GPRor38; ORL7107 / GPRor38; ORL7108 / GPRor38; ORL7109 / GPRor38; ORL7110 / GPRor38; ORL7111 / GPRor38; ORL7112 / GPRor38; ORL7113 / GPRor38; ORL7114 / GPRor38; ORL7115 / GPRor38; ORL7116 / GPRor38; ORL7117 / GPRor38; ORL7118 / GPRor38; ORL7119 / GPRor38; ORL7120 / GPRor38; ORL7121 / GPRor38; ORL7122 / GPRor38; ORL7123 / GPRor38; ORL7124 / GPRor38; ORL7125 / GPRor38 |
| Note: GPRor7/ IOR55 can be co-expressed with each of the other ORs. |

**Table 13 List of Human receptors**

| Human receptors and ion channels, including examples of heteromultimeric receptors and ion channels (gene names) |
|---|
| GABA-A including: GABRA1; GABRA2; GABRA3; GABRA4; GABRA5; GABRA6; GABRB1; GABRB2; GABRB3; GABRG1; GABRG2; GABRG3; GABRD; GABRE;<br>GABRP;GABRQ |
| GABA-C including: GABRR1; GABRR2; GABRR3 |
| nAChR including: CHRNA1; CHRNA2; CHRNA3; CHRNA4; CHRNA5; CHRNA6; CHRNA7; CHRNA9; CHRNA10; CHRNB1; CHRNB2; CHRNB3; CHRNB4; CHRNG;CHRND;CHRNE |
| 5-HT3 including: HTR3A; HTR3B; HTR3C; HTR3D; HTR3E |
| Glycine (GlyR) including: GLRA1; GLRA2; GLRA3; GLRA4; GLRB |
| Glutamate receptors including: GRIA1; GRIA2; GRIA3; GRIA4; GRIK1; GRIK2; GRIK3; GRIK4; GRIK5; GRIN1; GRINL1A; GRINL1B; GRIN2A; GRIN2B; GRIN2C; GRIN2D; GRIN3A; GRIN3B |
| ATP-gated channels including: P2RX1; P2RX2; P2RX3; P2RX4; P2RX5; P2RX6; P2RX7 |
| ENaC/DEG including: SCNN1 A; SCNN1 B; SCNN1 G; SCNN1D; ACCN2; ACCN1; ACCN3; ACCN4; ACCN5 |
| TRP family including: TRPA1; TRPC1; TRPC2; TRPC3; TRPC4; TRPC4AP; TRPC5; TRPC6; TRPC6P; TRPC7; TRPM1; TRPM2; TRPM3; TRPM4; TRPM5; TRPM6; TRPM7; TRPM8; TRPS1; TRPT1; TRPV1; TRPV2; TRPV3; TRPV4; TRPV5; TRPV6 |
| CNG family including: CNGA1; CNGA2; CNGA3; CNGA4; CNGB1; CNGB3; |
| HCN including: HCN1; HCN2; HCN3; HCN4 |

(continued)

| Human receptors and ion channels, including examples of heteromultimeric receptors and ion channels (gene names) |
|---|
| KCN family including: KCNA1; KCNA2; KCNA3; KCNA4; KCNA5; KCNA6; KCNA7; KCNA10; KCNAB1; KCNAB2; KCNAB3; KCNB1; KCNB2; KCNC1; KCNC2; KCNC3; KCNC4; KCND1; KCND2; KCND3; KCNE1; KCNE1 L; KCNE2; KCNE3; KCNE4; KCNF1; KCNG1; KCNG2; KCNG3; KCNG4; KCNH1; KCNH2; KCNH3; KCNH4; KCNH5; KCNH6; KCNH7; KCNH8; KCNIP1; KCNIP2; KCNIP3; KCNIP4; KCNJ1; KCNJ2; KCNJ3; KCNJ4; KCNJ5; KCNJ6; KCNJ8; KCNJ9; KCNJ10; KCNJ11; KCNJ12; KCNJ13; KCNJ14; KCNJ15; KCNJ16; KCNK1; KCNK2; KCNK3; KCNK4; KCNK5; KCNK6; KCNK7; KCNK9; KCNK10; KCNK12; KCNK13; KCNK15; KCNK16; KCNK17; KCNK18; KCNMA1; KCNMB1; KCNMB2; KCNMB3; KCNMB3L; KCNMB4; KCNN1; KCNN2; KCNN3; KCNN4; KCNQ1; KCNQ1 DN; KCNQ1OT1; KCNQ2; KCNQ3; KCNQ4; KCNQ5; KCNRG; KCNS1; KCNS2; KCNS3; KCNT1; KCNT2; KCNU1; KCNV1; KCNV2 |
| Receptor tyrosine kinases including: FGFR1; FGFR2; FGFR3; FGFR4; FGFR6; PDGFRA; PDGFRB; EGFR; ERBB2; ERBB3; ERBB4 |
| Nuclear steroid receptors including: ESR1; ESR2; THRA; THRB; RARA; RARB; RARG; PPARA; PPARD; PPARG; NR1D1; NR1D2; RORA; RORB; RORC; NR1 H3; NR1 H2; NR1 H4; VDR; NR1I2; NR1I3 |
| TGFbeta superfamily receptors including: BMPR1 A; BMPR1 B; BMPR2; ACVR2A; ACVR1 B; ACVR2B; ACVR1 C; TGFBRI; TGFBRII; TGFBRIII |
| T- cell receptors and B-cell receptors |
| Note: Cells can also be made with corresponding sequences from other species; this is an exemplary list of classes of receptors, not all families or family members are listed, and others can also be expressed in cells using our methods. |

**Table 14 GABA subunits from various species.**

| Receptor subunit | Gene name | Spicies |
|---|---|---|
| **GABAA:** | | |
| gamma-aminobutyric acid (GABA) A receptor, alpha 1 | GABRA1 | Homo sapiens |
| gamma-aminobutyric acid (GABA) A receptor, alpha 1 | Gabra1 | Mus musculus |
| gamma-aminobutyric acid (GABA) A receptor, alpha 1 | gabra1 | Danio rerio |
| gamma-aminobutyric acid (GABA) A receptor, alpha 1 | GABRA1 | Pan troglodytes |
| gamma-aminobutyric acid (GABA) A receptor, alpha 1 | GABRA1 (variant 1) | Bos taurus |
| gamma-aminobutyric acid (GABA) A receptor, alpha 1 | GABRA1 (variant 2) | Bos taurus |
| gamma-aminobutyric acid (GABA) A receptor, alpha 1 | GABRA1 | Gallus gallus |
| gamma-aminobutyric acid (GABA) A receptor, alpha 1 | GABRA1 | Canis familiaris |
| gamma-aminobutyric acid (GABA) A receptor, alpha 1 | Gabra1 | Rattus norvegicus |
| | | |
| gamma-aminobutyric acid (GABA) A receptor, alpha 2 | GABRA2 | Homo sapiens |
| gamma-aminobutyric acid (GABA) A receptor, alpha 2 | Gabra2 | Mus musculus |
| gamma-aminobutyric acid (GABA) A receptor, alpha 2 | LOC100150704 | Danio rerio |
| gamma-aminobutyric acid (GABA) A receptor, alpha 2 | GABRA2 | Pan troglodytes |
| gamma-aminobutvric acid (GABA) A receptor, alpha 2 | GABRA2 | Bos taurus |
| gamma-aminobutyric acid (GABA) A receptor, alpha 2 | GABRA2 | Gallus gallus |
| gamma-aminobutyric acid (GABA) A receptor, alpha 2 | GABRA2 | Canis familiaris |
| gamma-aminobutyric acid (GABA) A receptor, alpha 2 | LOC289606 | Rattus norvegicus |

(continued)

| Receptor subunit | Gene name | Spicies |
|---|---|---|
| **GABAA:** | | |
| | | |
| gamma-aminobutyric acid (GABA) A receptor, alpha 3 | GABRA3 | Homo sapiens |
| gamma-aminobutyric acid (GABA) A receptor, alpha 3 | Gabra3 | Mus musculus |
| gamma-aminobutyric acid (GABA) A receptor, alpha 3 | Grd | Drosophila melanogaster |
| gamma-aminobutyric acid (GABA) A receptor, alpha 3 | GABRA3 | Bos taurus |
| gamma-aminobutyric acid (GABA) A receptor, alpha 3 | GABRA3 | Gallus gallus |
| gamma-aminobutyric acid (GABA) A receptor, alpha 3 | GABRA3 | Canis familiaris |
| gamma-aminobutyric acid (GABA) A receptor, alpha 3 | Gabra3 | Rattus norvegicus |
| | | |
| gamma-aminobutyric acid (GABA) A receptor, alpha 4 | GABRA4 | Homo sapiens |
| gamma-aminobutyric acid (GABA) A receptor, alpha 4 | Gabra4 | Mus musculus |
| gamma-aminobutyric acid (GABA) A receptor, alpha 4 | zgc:110204 | Danio rerio |
| gamma-aminobutyric acid (GABA) A receptor, alpha 4 | GABRA4 | Pan troglodytes |
| gamma-aminobutyric acid (GABA) A receptor, alpha 4 | GABRA4 | Bos taurus |
| gamma-aminobutyric acid (GABA) A receptor, alpha 4 | GABRA4 | Gallus gallus |
| gamma-aminobutyric acid (GABA) A receptor, alpha 4 | GABRA4 | Canis familiaris |
| gamma-aminobutyric acid (GABA) A receptor, alpha 4 | Gabra4 | Rattus norvegicus |
| | | |
| gamma-aminobutyric acid (GABA) A receptor, alpha 5 | GABRA5 | Homo sapiens |
| gamma-aminobutyric acid (GABA) A receptor, alpha 5 | Gabra5 | Mus musculus |
| gamma-aminobutyric acid (GABA) A receptor, alpha 5 | CG8916 | Drosophila melanogaster |
| gamma-aminobutyric acid (GABA) A receptor, alpha 5 | lgc-37 | Caenorhabditis elegans |
| gamma-aminobutyric acid (GABA) A receptor, alpha 5 | LOC799124 | Danio rerio |
| gamma-aminobutyric acid (GABA) A receptor, alpha 5 | GABRA5 | Bos taurus |
| gamma-aminobutyric acid (GABA) A receptor, alpha 5 | GABRA5 | Gallus gallus |
| gamma-aminobutyric acid (GABA) A receptor, alpha 5 | GABRA5 | Canis familiaris |
| gamma-aminobutyric acid (GABA) A receptor, alpha 5 | Gabra5 | Rattus norvegicus |
| | | |
| gamma-aminobutyric acid (GABA) A receptor, alpha 6 | GABRA6 | Homo sapiens |
| gamma-aminobutyric acid (GABA) A receptor, alpha 6 | Gabra6 | Mus musculus |
| gamma-aminobutyric acid (GABA) A receptor, alpha 6 | Rdl | Drosophila melanogaster |
| gamma-aminobutyric acid (GABA) A receptor, alpha 6 | lgc-38 | Caenorhabditis elegans |
| gamma-aminobutyric acid (GABA) A receptor, alpha 6 | gabra6a | Danio rerio |
| gamma-aminobutyric acid (GABA) A receptor, alpha 6 | gabra6b | Danio rerio |
| gamma-aminobutyric acid (GABA) A receptor, alpha 6 | GABRA6 | Pan troglodytes |
| gamma-aminobutyric acid (GABA) A receptor, alpha 6 | GABRA6 | Bos taurus |
| gamma-aminobutyric acid (GABA) A receptor, alpha 6 | GABRA6 | Gallus gallus |

(continued)

| Receptor subunit | Gene name | Spicies |
|---|---|---|
| **GABAA:** | | |
| gamma-aminobutyric acid (GABA) A receptor, alpha 6 | GABRA6 | Canis familiaris |
| gamma-aminobutyric acid (GABA) A receptor, alpha 6 | Gabra6 | Rattus norvegicus |
| | | |
| gamma-aminobutyric acid (GABA) A receptor, beta 1 | GABRB1 | Homo sapiens |
| gamma-aminobutyric acid (GABA) A receptor, beta 1 | Gabrb1 | Mus musculus |
| gamma-aminobutyric acid (GABA) A receptor, beta 1 | GABRB1 | Pan troglodytes |
| gamma-aminobutyric acid (GABA) A receptor, beta 1 | GABRB1 | Bos taurus |
| gamma-aminobutyric acid (GABA) A receptor, beta 1 | GABRB1 | Gallus gallus |
| gamma-aminobutyric acid (GABA) A receptor, beta 1 | Gabrb1 | Rattus norvegicus |
| | | |
| gamma-aminobutyric acid (GABA) A receptor, beta 2 | GABRB2 | Homo sapiens |
| gamma-aminobutyric acid (GABA) A receptor, beta 2 | Gabrb2 | Mus musculus |
| gamma-aminobutyric acid (GABA) A receptor, beta 2 | gabrb2 | Danio rerio |
| gamma-aminobutyric acid (GABA) A receptor, beta 2 | GABRB2 | Pan troglodytes |
| gamma-aminobutyric acid (GABA) A receptor, beta 2 | GABRB2 | Bos taurus |
| gamma-aminobutyric acid (GABA) A receptor, beta 2 | GABRB2 | Gallus gallus |
| gamma-aminobutyric acid (GABA) A receptor, beta 2 | GABRB2 | Canis familiaris |
| gamma-aminobutyric acid (GABA) A receptor, beta 2 | Gabrb2 | Rattus norvegicus |
| | | |
| gamma-aminobutyric acid (GABA) A receptor, beta 3 | GABRB3 | Homo sapiens |
| gamma-aminobutyric acid (GABA) A receptor, beta 3 | Gabrb3 | Mus musculus |
| gamma-aminobutyric acid (GABA) A receptor, beta 3 | Lcch3 | Drosophila melanogaster |
| gamma-aminobutyric acid (GABA) A receptor, beta 3 | gab-1 | Caenorhabditis elegans |
| gamma-aminobutyric acid (GABA) A receptor, beta 3 | LOC566922 | Danio rerio |
| gamma-aminobutyric acid (GABA) A receptor, beta 3 | GABRB3 | Pan troglodytes |
| gamma-aminobutyric acid (GABA) A receptor, beta 3 | GABRB3 | Gallus gallus |
| gamma-aminobutyric acid (GABA) A receptor, beta 3 | GABRB3 | Canis familiaris |
| gamma-aminobutyric acid (GABA) A receptor, beta 3 | Gabrb3 | Rattus norvegicus |
| | | |
| gamma-aminobutyric acid (GABA) A receptor, gamma 1 | GABRG1 | Homo sapiens |
| gamma-aminobutyric acid (GABA) A receptor, gamma 1 | Gabrg1 | Mus musculus |
| gamma-aminobutyric acid (GABA) A receptor, gamma 1 | LOC556202 | Danio rerio |
| gamma-aminobutyric acid (GABA) A receptor, gamma 1 | GABRG1 | Pan troglodytes |
| gamma-aminobutyric acid (GABA) A receptor, gamma 1 | GABRG1 | Bos taurus |
| gamma-aminobutyric acid (GABA) A receptor, gamma 1 | GABRG1 | Gallus gallus |
| gamma-aminobutyric acid (GABA) A receptor, gamma 1 | GABRG1 | Canis familiaris |
| gamma-aminobutyric acid (GABA) A receptor, gamma 1 | Gabrg1 | Rattus norvegicus |

(continued)

| Receptor subunit | Gene name | Spicies |
|---|---|---|
| **GABAA:** | | |
| | | |
| gamma-aminobutyric acid (GABA) A receptor, gamma 2 | GABRG2 | Homo sapiens |
| gamma-aminobutyric acid (GABA) A receptor, gamma 2 | Gabrg2 | Mus musculus |
| gamma-aminobutyric acid (GABA) A receptor, gamma 2 | LOC553402 | Danio rerio |
| gamma-aminobutyric acid (GABA) A receptor, gamma 2 | GABRG2 | Bos taurus |
| gamma-aminobutyric acid (GABA) A receptor, gamma 2 | GABRG2 | Gallus gallus |
| gamma-aminobutyric acid (GABA) A receptor, gamma 2 | GABRG2 | Canis familiaris |
| gamma-aminobutyric acid (GABA) A receptor, gamma 2 | Gabrg2 | Rattus norvegicus |
| | | |
| gamma-aminobutyric acid (GABA) A receptor, gamma 3 | GABRG3 | Homo sapiens |
| gamma-aminobutyric acid (GABA) A receptor, gamma 3 | Gabrg3 | Mus musculus |
| gamma-aminobutyric acid (GABA) A receptor, gamma 3 | LOC567057 | Danio rerio |
| gamma-aminobutyric acid (GABA) A receptor, gamma 3 | GABRG3 | Gallus gallus |
| gamma-aminobutyric acid (GABA) A receptor, gamma 3 | Gabrg3 | Rattus norvegicus |
| | | |
| gamma-aminobutyric acid (GABA) A receptor, delta | GABRD | Homo sapiens |
| gamma-aminobutyric acid (GABA) A receptor, delta | Gabrd | Mus musculus |
| gamma-aminobutyric acid (GABA) A receptor, delta | DKEYP-87A12.2 | Danio rerio |
| gamma-aminobutyric acid (GABA) A receptor, delta | GABRD | Pan troglodytes |
| gamma-aminobutyric acid (GABA) A receptor, delta | GABRD | Bos taurus |
| gamma-aminobutyric acid (GABA) A receptor, delta | GABRD | Gallus gallus |
| gamma-aminobutyric acid (GABA) A receptor, delta | GABRD | Canis familiaris |
| gamma-aminobutyric acid (GABA) A receptor, delta | Gabrd | Rattus norvegicus |
| | | |
| gamma-aminobutyric acid (GABA) A receptor, epsilon | GABRE | Homo sapiens |
| gamma-aminobutyric acid (GABA) A receptor, epsilon | Gabre | Mus musculus |
| gamma-aminobutyric acid (GABA) A receptor, epsilon | GABRE | Pan troglodytes |
| gamma-aminobutyric acid (GABA) A receptor, epsilon | GABRE | Bos taurus |
| gamma-aminobutyric acid (GABA) A receptor, epsilon | GABRE | Canis familiaris |
| gamma-aminobutyric acid (GABA) A receptor, epsilon | Gabre | Rattus norvegicus |
| | | |
| gamma-aminobutyric acid (GABA) A receptor, pi | GABRP | Homo sapiens |
| gamma-aminobutyric acid (GABA) A receptor, pi | Gabrp | Mus musculus |
| gamma-aminobutyric acid (GABA) A receptor, pi | GABRP | Pan troglodytes |
| gamma-aminobutyric acid (GABA) A receptor, pi | GABRP | Bos taurus |
| gamma-aminobutyric acid (GABA) A receptor, pi | GABRP | Gallus gallus |
| gamma-aminobutyric acid (GABA) A receptor, pi | GABRP | Canis familiaris |

(continued)

| Receptor subunit | Gene name | Spicies |
|---|---|---|
| **GABAA:** | | |
| gamma-aminobutyric acid (GABA) A receptor, pi | Gabrp | Rattus norvegicus |
| | | |
| gamma-aminobutyric acid (GABA) A receptor, theta | GABRQ | Homo sapiens |
| gamma-aminobutyric acid (GABA) A receptor, theta | Gabrg | Mus musculus |
| gamma-aminobutyric acid (GABA) A receptor, theta | GABRQ | Pan trogiodytes |
| gamma-aminobutyric acid (GABA) A receptor, theta | GABRQ | Bos taurus |
| gamma-aminobutyric acid (GABA) A receptor, theta | GABRQ | Canis familiaris |
| gamma-aminobutyric acid (GABA) A receptor, theta | Gabrq | Rattus norvegicus |
| | | |
| **GABAB:** | | |
| gamma-aminobutyric acid (GABA) B receptor, 1 | GABBR1 | Homo sapiens |
| gamma-aminobutyric acid (GABA) B receptor, 1 | Gabbr1 | Mus musculus |
| gamma-aminobutyric acid (GABA) B receptor, 1 | GABA-B-R1 | Drosophila melanogaster |
| gamma-aminobutyric acid (GABA) B receptor, 1 | Y41G9A.4 | Caenorhabditis elegans |
| gamma-aminobutyric acid (GABA) B receptor, 1 | gabbr1 | Danio rerio |
| gamma-aminobutyric acid (GABA) B receptor, 1 | GABBR1 | Pan troglodytes |
| gamma-aminobutyric acid (GABA) B receptor, 1 | GABBR1 | Bos taurus |
| gamma-aminobutyric acid (GABA) B receptor, 1 | GABBR1 | Canis familiaris |
| gamma-aminobutyric acid (GABA) B receptor, 1 | Gabbr1 | Rattus norvegicus |
| | | |
| gamma-aminobutyric acid (GABA) B receptor, 2 | GABBR2 | Homo sapiens |
| gamma-aminobutyric acid (GABA) B receptor, 2 | Gabbr2 | Mus musculus |
| gamma-aminobutyric acid (GABA) B receptor, 2 | GABA-B-R2 | Drosophila melanogaster |
| gamma-aminobutyric acid (GABA) B receptor, 2 | si:dkey-19011.2 | Danio rerio |
| gamma-aminobutyric acid (GABA) B receptor, 2 | GABBR2 | Pan troglodytes |
| gamma-aminobutyric acid (GABA) B receptor, 2 | GABBR2 | Bos taurus |
| gamma-aminobutyric acid (GABA) B receptor, 2 | GABBR2 | Gallus gallus |
| gamma-aminobutyric acid (GABA) B receptor, 2 | GABBR2 | Canis familiaris |
| gamma-aminobutyric acid (GABA) B receptor, 2 | Gabbr2 | Rattus norvegicus |
| | | |
| **GABAC:** | | |
| gamma-aminobutyric acid (GABA) A receptor, rho1 | GABRR1 | Homo sapiens |
| gamma-aminobutyric acid (GABA) A receptor, rho1 | Gabrr1 | Mus musculus |
| gamma-aminobutyric acid (GABA) A receptor, rho1 | gabrr1 | Danio rerio |
| gamma-aminobutyric acid (GABA) A receptor, rho1 | GABRR1 | Pan troglodytes |
| gamma-aminobutyric acid (GABA) A receptor, rho1 | GABRR1 | Bos taurus |
| gamma-aminobutyric acid (GABA) A receptor, rho1 | GABRR1 | Gallus gallus |

(continued)

| GABAC: | | |
|---|---|---|
| gamma-aminobutyric acid (GABA) A receptor, rho1 | GABRR1 | Canis familiaris |
| gamma-aminobutyric acid (GABA) A receptor, rho1 | Gabrr1 | Rattus norvegicus |
| **Receptor subunit** | **Gene name** | **Spicies** |
| | | |
| gamma-aminobutyric acid (GABA) A receptor, rho2 | GABRR2 | Homo sapiens |
| gamma-aminobutyric acid (GABA) A receptor, rho2 | Gabrr2 | Mus musculus |
| gamma-aminobutyric acid (GABA) A receptor, rho2 | si:dkey-181 i3.1 | Danio rerio |
| gamma-aminobutyric acid (GABA) A receptor, rho2 | GABRR2 | Pan troglodytes |
| gamma-aminobutyric acid (GABA) A receptor, rho2 | GABRR2 | Gallus gallus |
| gamma-aminobutyric acid (GABA) A receptor, rho2 | GABRR2 | Canis familiaris |
| gamma-aminobutyric acid (GABA) A receptor, rho2 | Gabrr2 | Rattus norvegicus |
| | | |
| gamma-aminobutyric acid (GABA) A receptor, rho3 | GABRR3 | Homo sapiens |
| gamma-aminobutyric acid (GABA) A receptor, rho3 | Gabrr3 | Mus musculus |
| gamma-aminobutvric acid (GABA) A receptor, rho3 | zgc:194845 | Danio rerio |
| gamma-aminobutyric acid (GABA) A receptor, rho3 | GABRR3 | Pan troglodytes |
| gamma-aminobutyric acid (GABA) A receptor, rho3 | GABRR3 | Bos taurus |
| gamma-aminobutyric acid (GABA) A receptor, rho3 | GABRR3 | Gallus gallus |
| gamma-aminobutyric acid (GABA) A receptor, rho3 | Gabrr3 | Rattus norvegicus |

**Table 15. List of Human bitter receptors**

| Human bitter receptors |
|---|
| hTAS2R1; hTAS2R3; hTAS2R4; hTAS2R5; hTAS2R7; hTAS2R8; hTAS2R9; hTAS2R10; hTAS2R13; hTAS2R14; hTAS2R16; hTAS2R38; hTAS2R39; hTAS2R40; hTAS2R41; hTAS2R43; hTAS2R44; hTAS2R45; hTAS2R46; hTAS2R47; hTAS2R48; hTAS2R49; hTAS2R50; hTAS2R55; hTAS2R60 |

Preferred G proteins in making bitter receptor cell lines include, but are not limited to Mouse G$\alpha$15 and Human GNA15.

**Table 16 Sweet and Umami receptors**

| Type | Subunit | Gene Symbol | Splice form | NCBI Gene ID | Synonyms |
|---|---|---|---|---|---|
| Umami Taste | T1R1 | T1R1 | 1 | 80835 | TAS1R1, TR1, GPR70 |
| | | | 2 | | |
| | | | 3 | | |
| | | | 4 | | |
| Sweet Taste | T1R2 | T1R2 | 1 | 80834 | TAS1R2, TR2, GPR71 |
| Umami/Sweet Taste | T1R3 | T1R3 | 1 | 83756 | TAS1R3 |

**Table 17 Cystic Fibrosis Transmembrane-conductance Regulator**

| Class | Protein # (UniProt) | Description |
|---|---|---|
| | | *Homo sapiens* cystic fibrosis transmembrane conductance regulator (CFTR) *Homo sapiens* cystic fibrosis transmembrane conductance regulator (CFTR) mutant (ΔF508) |

**Table 18. Guanylyl Cyclases**

| Class | Family/ *Subtype* | Protein # (UniProt) | Description |
|---|---|---|---|
| **Guanylyl cyclases** | | | Guanylate cyclase-A/ natriuretic peptide receptor A |
| | | | Guanylate cyclase-B/ natriuretic peptide receptor B |
| | | | Guanylate cyclase-C |
| | | | Guanylate cyclase-D |
| | | | Guanylate cyclase-E |
| | | | Guanylate cyclase-F |
| | | | Guanylate cyclase-G |
| Related receptor lacking guanylyl cyclase domain | | | Natriuretic peptide receptor C (NPR3) |

**Table 19. List of Mouse Olfactory Receptors**

| Name/Common Name |
|---|
| ORL1000 / GABBR1; ORL1001 / GABBR1; ORL1002 / GABBR1; ORL1034 / STE20-like; ORL1035 / Olfr74; ORL1036 Olfr73; ORL1047 / RA1c; ORL1056 / Olfr74; ORL1057 / Olfr73; ORL1058 / Olfr160; ORL1065 / Olfr263-ps1; ORL1071 / Olfr71 ; ORL1072 / Olfr70; ORL1073 / Olfr37e; ORL1074 / Olfr37c; ORL1076 / Olfr37b; ORL1077 / Olfr37a; ORL1078 / Olfr17; ORL1079 / Olfr72; ORL1080 / Olfr69; ORL1099 / MORPC6; ORL110 / K10; ORL1100 / MORPC7; ORL1101 / MORPC8; ORL1102 / MORPC9; ORL1103 / MORPC10; ORL1104 / MOR103-3/W/88U1-3184557-3185495; ORL1104 / MOR103-2/W88U1-31530; ORL1105 / MOR103-2/W88U1-3153066-3154004; ORL1105 / MOR103-2/W88U1-31530; ORL1106 / MOR208-1/W88U1-3093458-3092487; ORL1107 / MOR208-3/ W88U1-3070171-3069200; ORL1108 / MOR267-4/W5Q1 F-362880-363812; ORL1109 / MOR267-5/W5Q1 F-311029-311961; ORL111 / K17; ORL1110 / MOR103-10/W3B3M-353171-354124; ORL1111 / MOR267-2/W5Q1F-279827-278886; ORL1112 / MOR267-3/W5Q1F-292301-291360; ORL1113 / MOR123-1 /W3B3M-20750-21694; ORL1114 / MOR123-2/W3B3M-29057-29995; ORL1115 / MOR267-8/W5Q1 F-22839; ORL1116 / MOR105-1 /W3B3M-63512-64450; ORL1117 / MOR105-2/W3B3M-109341-110288; ORL1118 / MOR105-3/W3B3M-124081-125028; ORL1119 / MOR105-4/W3B3M-96659-95703; ORL112 / K20; ORL1120 / MOR267-6/W3B3M-225358-224417; ORL1121 / MOR208-2/W88U1-3058853-3057885; ORL1122 / MOR105-10/W3B3M-131350-132315; ORL1123 / MOR208-4/W88U1-3078360-3077440; ORL1124 / MOR208-5/W88U1-3046840-3045839; ORL1125 / MOR103-12/W88U1-3173422-3174358; ORL1126 / MOR105-5P/W3B3M-45128-46082; ORL1127 / MOR105-6P/W3B3M-114822-115785; ORL1128 / MOR105-8/W3B3M-86212-87163; ORL1129 / MOR103-13P/W3B3M-371191-172136; ORL113 / K21; ORL1132 / MOR213-3/W531 T-1683482-1684420; ORL1133 / MOR213-5/W531 T-1674684-1675601; |

(continued)

| Name/Common Name |
|---|
| ORL1134 / MOR213-2/W531T-1667893-1668810; ORL1135 / MOR213-6/W531T-1658501-1657566; ORL1136 / MOR213-4/W531T-1580279-1579341; ORL1138 / MOR203-1/W531T-1307420-1306491; ORL1139 / MOR248-3/W4T2P-365261-366199; ORL114 / L45; ORL1140 / MOR248-5/W4T2P-343048-343986; ORL1141 / MOR248-4/W4T2P-216559-215621; ORL1142 / MOR248-10/W4T2P-160623-159685; ORL1143 / MOR248-7/W4T2P-13374; ORL1144 / MOR136-4/W4QPD-3066754-3065816; ORL1145 / MOR136-7/W4QPD-2956102-2955167; ORL1146 / MOR136-11/W4QPD-2913147-2912218; ORL1146 / MOR136-11 /W4QPD-2913; ORL1147 / MOR127-2/W4QPD-2741555-2742487; ORL1148 / MOR159-1/W4QPD-2539504-2538551; ORL1149 / MOR178-1/W4QPD-2304821-2303838; ORL115 / M 15; ORL1150 / MOR230-4/W62NC-9830-8904; ORL1151 / MOR231-7/W62NC-922173-921229; ORL1152 / MOR231-6/W62NC-874856-873912; ORL1153 / MOR231-4/W62NC-791416-790499; ORL1154 / MOR230-8/W62NC-77563-76631; ORL1155 / MOR231-5/W62NC-755986-755066; ORL1156 / MOR231-8/W62NC-702155-701211; ORL1157 / MOR231-3/W62NC-686470-685553; ORL1158 / MOR231-2/W62NC-631817-630873; ORL1159 / MOR238-1/W62NC-614193-613276; ORL116 / M25; ORL1160 / MOR233-1/W62NC-5997; ORL1161 / MOR233-1/W62NC-525789-524854; ORL1163 / MOR233-5/W62NC-415599-414664; ORL1164 / MOR233-4/W62NC-340658-339766;ORL1165 / MOR233-13/W62NC-3190; ORL1166 / MOR233-7/W62NC-289851-288916; ORL1167 / MOR230-7/W62NC-226803-225871; ORL1168 / MOR225-4/W62NC-213006-212080; ORL1169 / MOR230-3/W62NC-180723-181646; ORL117 / M3; ORL1170 / MOR230-1/W62NC-15070; ORL1171 / MOR227-5/W62NC-1422035-1421118; ORL1172 / MOR227-1/W62NC-1396979-1396065; ORL1173 / MOR232-7/W62NC-136733-137662; ORL1174 / MOR234-2/W62NC-1267349-1268269; ORL1175 / MOR234-1/W62NC-1254809-1255723; ORL1176 / MOR234-3/W62NC-1245738-1246658; ORL1177 / MOR232-2/W62NC-1230360-1231292; ORL1178 / MOR232-9/W62NC-1196441-1195512; ORL1179 / MOR232-3/W62NC-1182131-1183066; ORL118 / M30; ORL1181 / MOR231 - 1/W62NC-1103743-1102823; ORL1182 / MOR232-4/W62NC-10840; ORL1183 / MOR231-13/W62NC-1057100-1056156; ORL1184 / MOR248-9/W4T2P-84764-83847; ORL1185 / MOR136-12/W4QPD-3026443-3025514; ORL1186 / MOR248-1/W6TE3-44139-43210; ORL1187 / MOR233-11/W62NC-483800-482865; ORL1188 / MOR233-3/W62NC-427216-426281; ORL1189 / MOR233-6/W62NC-391592-390657; ORL119 / M31; ORL1190 / MOR233-12 W62NC-3723; ORL1191 / MOR231-14/W62NC-744896-743952; ORL1192 / MOR235-2/W62NC-577185-576244; ORL1193 / MOR227-4/W62NC-1451074-1450151; ORL1194 / MOR233-8/W62NC-304951-304016; ORL1195 / MOR233-9/W62NC-330821-329886; ORL1196 / MOR231-9/W62NC-858333-857389; ORL1197 / MOR231-12/W62NC-843376-842453; ORL1198/MOR228-2/W62NC-1289142-1290071; ORL1199 / MOR237-1/W62NC-570764-569847; ORL120 / M41; ORL1200 / MOR232-6/W62NC-168995-169924; ORL1201 / MOR230-2/W62NC-115145-116068; ORL1202 / MOR231-11/W62NC-675497-674550; ORL1203 / MOR136-2/W4QPD-3113340-3114281; ORL1204 / MOR158-1/W4QPD-2502147-2503100; ORL1205 / MOR228-3/W62NC-1367122-1366193; ORL1206 / MOR136-5/W4QPD-3214526-3213606; ORL1207 / MOR136-9/W4QPD-2866927-2865989; ORL1208 / MOR229-1 /W62NC-1358909-1357980; ORL1209 / MOR127-3/W4QPD-2711413-2712348; ORL121 / M49; ORL1210 / MOR136-10/W4QPD-2732342-2731395; |

(continued)

| Name/Common Name |
| --- |
| ORL1211 / MOR127-4/W4QPD-2695455-2694502; ORL1212 / MOR248-6/W4T2P-240375-239437; ORL1213 / MOR187-3/W531 T-1594763-1595704; ORL1214 / OR136-13/W4QPD-2751886-2750948; ORL1215 / MOR138-2/W4QPD-2674279-2675211; ORL1216 / MOR134-1/W4QPD-2665277-2664330; ORL1217 / MOR138-3/W4QPD-2611994-2611068; ORL1218 / MOR175-2/W531T-1553871-1552915; ORL1219 / MOR175-3/W531T-1619872-1620816; ORL122 / M5; ORL1220 / MOR245-2/W3P0G-777153-778091; ORL1221 / MOR245-3/W3P0G-854234-855172; ORL1222 / MOR245-4/W3P0G-731373-730423; ORL1223 / MOR227-3/W62NC-1436829-1435903; ORL1224 / MOR175-4/W531T-1485097-1486041; ORL1225 / MOR175-5/W531T-14963; ORL1226 / MOR203-2/W531T-1321744-1320800; ORL1229 / MOR203-3/W531T-1345467-1344520; ORL123 / M64; ORL1230 / MOR245-6/W4T2P-78061-78957; ORL1231 / MOR245-8/W4T2P-440890-439952; ORL1232 / MOR159-3/W4QPD-2522092-2523060; ORL1233 / MOR245-21/W3P0G-841549-842487; ORL1234 / MOR225-5/W62NC-27501-26557; ORL1235 / MOR136-3/W4QPD-3170050-3169121; ORL1236 / MOR248-2/W6TE3-24810-25727; ORL1237 / MOR245-5/W3P0G-879404-880342; ORL1238 / MOR245-7/W4T2P-407907-406969; ORL1239 / MOR245-9/W4T2P-73414-74352; ORL124 / M71; ORL1240 / MOR248-11/W6TE3-88524-89435; ORL1241 / MOR245-10/W6TE3-101768-100830; ORL1242 / MOR245-11/W6TE3-67348-66407; ORL1244 / MOR245-20/W3P0G-820471-821424; ORL1245 / MOR225-12/W62NC-89308-88400; ORL1246 / MOR233-16P/W62NC-247006-246231; ORL1247 / MOR228-1/W62NC-1333651-1332722; ORL1248 / MOR233-17/W62NC-275418-275894; ORL1249 / MOR138-1/W4QPD-2418303-2417395; ORL125 / M76; ORL1250 / MOR248-8/W4T2P-257789-258728; ORL1251 / MOR231-16P/W62NC-895529-895143; ORL1252 / MOR136-16P/W4QPD-3227386-3226940; ORL1253 / MOR248-14P/W6TE3-31501-30818; ORL1254 / MOR213-7P/W531T-1651677-1652563; ORL1255 / MOR175-9/W531 T-1514386-1513443; ORL1256 / MOR175-9/W531 T-15143; ORL1257 / MOR136-8/W4QPD-2815255-2814309; ORL1258 / MOR233-10/W62NC-4597; ORL1258 / MOR233-10/W62NC-459745-458834; ORL1258 / MOR233-10/W62NC-4597; ORL1259 / MOR203-6P/W531 T-1295922-1295018; ORL126 / M93; ORL1260 / MOR248-13P/W4T2P-58109-58363; ORL1261 / MOR191-2P/W4CQV-5593-6007; ORL1262 / MOR136-15P/W4QPD-3240667-3239723; ORL1263 / MOR175-6/W531T-1539488-1540432; ORL1264 / MOR213-8/W531 T-1562275-1561814; ORL1265 / MOR175-7P/W531 T-1508205-1507258; ORL1266 / MOR245-15/W3P0G-961708-962647; ORL1267 / MOR245-16/W3P0G-717498-716535; ORL1268 / MOR245-17P/W3P0G-975627-976512; ORL1269 / MOR136-17P/W4QPD-2757545-2756808; ORL127 / M50; ORL1270 / MOR175-8P/W531 T-1490963-1491900; ORL1271 / MOR248-15/W4T2P-24085-25003; ORL1272 / MOR245-23/W3P0G-796342-797214; ORL1273 / MOR231-10/W62NC-992588-991951; ORL1274 / W62NC-556546-555710; ORL1275 / MOR233-15/W62NC-469928-468992; ORL1276 / MOR233-14/W62NC-440433-439497; ORL1277 / MOR248-12/W4T2P-284649-285564; ORL1278 / MOR159-2P/W4QPD-2588673-2589603; ORL1279 / MOR137-1 P/W4QPD-3259989-3259128; ORL128 / K18; ORL1280 / MOR248-13P/W4T2P-188606-188028; ORL1281 / MOR245-18/W6TE3-127243-128182; ORL1282 / MOR248-17P/W4T2P-67251-68078; ORL1283 / MOR128-1/W4QPD-2364495-2363561; ORL1284 / MOR245-22/W3P0G-901703-902599; ORL1285 / |

(continued)

| Name/Common Name |
|---|
| MOR136-1; ORL1286 / MOR138-4P; ORL1287 / MOR248-16; ORL1288 / W4QPD-3054974-305468; ORL1289 / W4CQV-78329-78805; ORL129 / K4; ORL1291 / W3Y5M-1033291-103377; ORL1292 / W3P0G-705890-705633; ORL1293 / MOR177-3/W3Y5M-988304-987372; ORL1294 / MOR211-5P/W3Y5M-847869-846998; ORL1295 / MOR273-4P/W3Y5M-574562-574891; ORL1296 / MOR40-9P/W3Y5M-10006; ORL1297 / MOR191-1 /W3Y5M-2793-1852; ORL1298 / MOR189-1 /W4CQV-69826-70767; ORL1299 / MOR192-1/W4CQV-54584-55525; ORL1300 / MOR190-1/W4CQV-159242-160183; ORL1301 / MOR189-3/W4CQV-121801-122742; ORL1302 / MOR177-2/W3Y5M-973343-972411; ORL1303 / MOR176-1/W3Y5M-957878-956940; ORL1304 / MOR176-2/W3Y5M-947235-946294; ORL1305 / MOR177-1/W3Y5M-936964-936038; ORL1306 / MOR177-4/W3Y5M-929723-930652; ORL1307 / MOR264-4/W3Y5M-908625-909569; ORL1308 / MOR264-9P/W3Y5M-898130-898624; ORL1309 / MOR176-3/W3Y5M-868624-867689; ORL1310 / MOR177-5/W3Y5M-855017-855940; ORL1311 / MOR264-5/W3Y5M-783242-784186; ORL1312 / MOR264-2/W3Y5M-681084-682004; ORL1313 / MOR264-20/W3Y5M-578493-579473; ORL1314 / MOR181-2/W3Y5M-487999-487061; ORL1315 / MOR172-4/W3Y5M-473705-472767; ORL1316 / MOR172-2/W3Y5M-407847-406909; ORL1317 / MOR188-5/W3Y5M-36573-35632; ORL1318 / MOR206-4/W3Y5M-315220-314255; ORL1319 / MOR206-1/W3Y5M-261359-260433; ORL1320 / MOR179-2/W3Y5M-23329-22397; ORL1321 / MORPC10; ORL1322 / MOR206-2/W3Y5M-229046-228099; ORL1323 / MOR206-6/W3Y5M-211534-210588; ORL1324 / MOR206-6/W3Y5M-211534-210588; ORL1325 / MOR174-16/W3Y5M-1397217-1396267; ORL1326 / MOR174-8/W3Y5M-1319379-1318429; ORL1327 / MOR179-6/W3Y5M-131445-132362; ORL1328 / MOR174-1/W3Y5M-1209167-1208229; ORL1329 / MOR177-12/W3Y5M-1153117-1154050; ORL1330 / MOR177-14/W3Y5M-1087729-1088661; ORL1330 / MOR268-5/W5M25-32054; ORL1331 / MOR177-10/W3Y5M-1042284-1041349; ORL1332 / MOR177-6/W3Y5M-1034488-1035423; ORL1333 / MOR177-8/W3Y5M-1026516-1025581; ORL1334 / MOR187-2/W3KVV-73368-74309; ORL1335 / MOR187-1/W3KVV-54811-55737; ORL1336 / MOR260-5/W3KVV-49882; ORL1337 / MOR198-3P/W3KVV-348455-347641; ORL1338 / MOR198-1 P/W3KVV-324361-323428; ORL1339 / MOR196-2/W3KVV-315800-314862; ORL1340 / MOR171-31 P/W3KVV-214374-215339; ORL1341 / MOR185-3/W3KVV-182345-181386; ORL1342 / MOR185-10/W3KVV-159319-160261; ORL1343 / MOR185-2/W3KVV-123798-124745; ORL1344 / MOR185-4/W3KVV-147900-148844; ORL1345 / MOR172-1/W3KVV-4772-3834; ORL1346 / MOR188-3/W3KVV-86109-87068; ORL1347 / MOR194-1/W3KVV-98261-99211; ORL1348 / MOR185-5/W3KVV-172878-173837; ORL1349 / MOR262-12/W3KVV-224823-224347; ORL1350 / MOR227-8P/W3KVV-259526-258592; ORL1351 / MOR199-2/W3KVV-264357-263425; ORL1352 / MOR198-2P/W3KVV-366917-366171; ORL1353 / MOR196-4/W3KVV-375806-374883; ORL1354 / MOR197-1/W3KVV-393843-394826; ORL1355 / MOR196-3/W3KVV-412059-411124; ORL1356 / MOR201-2/W3KVV-456703-455750; ORL1357 / MOR180-1/W3KVV-474678-475610; ORL1358 / MOR213-9/W3KVV-536600-537496; ORL1359 / MOR188-4/W3Y5M-100338-99397; ORL1360 / MOR179-1/W3Y5M-189516-188590; ORL1361 / MOR179-3/W3Y5M-325746-324814; ORL1362 / MOR179-4/W3Y5M-337640-338449; ORL1363 / MOR206-5P/W3Y5M-346436-345499; ORL1364 / MOR0-6P/W3Y5M-369203-368240; |

(continued)

| Name/Common Name |
|---|
| ORL1365 / MOR172-6/W3Y5M-384055-383117; ORL1366 / MOR172-5/W3Y5M-428639-427701; ORL1367 / MOR172-3/W3Y5M-443979-443041; ORL1368 / MOR264-6/W3Y5M-531718-532674; ORL1369 / MOR264-15P/W3Y5M-676374-677272; ORL1370 / MOR264-18/W3Y5M-695715-696659; ORL1371 / MOR264-1/W3Y5M-711622-712602; ORL1372 / MOR264-17/W3Y5M-740400-741371; ORL1373 / MOR264-3/W3Y5M-756846-757802; ORL1374 / MOR264-13P/W3Y5M-1077364-1078166; ORL1375 / MOR264-14P/W3Y5M-1124370-1125280; ORL1376 / MOR264-11/W3Y5M-1131126-1132077; ORL1377 / MOR173-2/W3Y5M-1167169-1166237; ORL1378 / MOR174-10/W3Y5M-1189477-1188533; ORL1379 / MOR173-3/W3Y5M-1235061-1236002; ORL1380 / MOR174-2/W3Y5M-1273418-1274383; ORL1381 / MOR174-4/W3Y5M-1298702-1297758; ORL1382 / MOR174-7/W3Y5M-1350208-1349258; ORL1383 / MOR174-6/W3Y5M-1372914-1371964; ORL1384 / MOR189-4P/W4CQV-82139-83056; ORL1385 / MOR188-6P/W4CQV-116400-117309; ORL1386 / MOR174-3/W3Y5M-1196210-1195248; ORL1387 / MOR206-3/W3Y5M-296158-295220; ORL1388 / MOR207-1 /W3Y5M-356813-355910; ORL1389 / MOR173-1 /W3Y5M-1255560-1254628; ORL1390 / MOR181-1 /W3Y5M-1066159-1067103; ORL1391 / MOR174-11/W3Y5M-1342110-1341187; ORL1392 / MOR177-7/W3Y5M-1159666-1160598; ORL1393 / MOR196-1/W3KVV-428780-427833; ORL1394 / MOR201-1/W3KVV-433882-432941; ORL1395 / MOR185-7/W3KVV-156736-157680; ORL1396 / MOR189-2/W4CQV-99803-98862; ORL1397 / MOR179-7/W3Y5M-174300-175226; ORL1398 / MOR264-7/W3Y5M-1117881-1118826; ORL1399 / MOR264-8P/W3Y5M-596904-597851; ORL1400 / MOR264-10P/W3Y5M-612781-613150; ORL1401 / MOR264-13P/W3Y5M-1107873-1108760; ORL1402 / MOR196-5P/W3KVV-380839-380015; ORL1403 / MOR135-23P/W3Y5M-1248002-1247109; ORL1404 / MOR264-19/W3Y5M-1095234-1096178; ORL1405 / MOR200-1/W3KVV-307786-306848; ORL1406 / MOR122-2/W451A-6144145-6145095; ORL1407 / MOR217-1/W35G5-988484-987537; ORL1408 / MOR262-8/W4TKW-7027342-7028295; ORL1409 / MOR262-7/W4TKW-6976918-6977877; ORL1410 / MOR259-8/W3HRA-2376401-2375454; ORL1411 / MOR259-1/W3HRA-2332174-2333115; ORL1412 / MOR259-10/W3BSW-49456-50403; ORL1413 / MOR258-3/W3BSW-138616-139563; ORL1414 / MOR262-9/W4TKW-6919504-6918566; ORL1415 / MOR262-1/W4TKW-1225680-1226621; ORL1416 / MOR262-2/W4TKW-7058418-7057459; ORL1417 / MOR262-3/W4TKW-6953433-6954383; ORL1418 / MOR258-2/W3BSW-96920-95973; ORL1419 / MOR258-6/W3BSW-105227-104280; ORL1420 / MOR259-5/W3BSW-18873-19823; ORL1421 / MOR259-2/W3HRA-2352027-2352973; ORL1422 / MOR259-3P/W3HRA-2401814-2400953; ORL1423 / MOR259-6/W3BSW-2-544; ORL1424 / MOR258-4P/W3BSW-150977-151874; ORL1425 / MOR262-10/W5BNN-830472-829522; ORL1426 / MOR259-4P/W3BSW-76695-77099; ORL1427 / MOR258-1/W3BSW-119835-118896; ORL1428 / MOR0-10P/W56CV-14410; ORL1429 / MOR177-9/W5D7G-25051; ORL1430 / MOR257-3/W6337-896331-897263; ORL1431 / MOR261-10/W6337-1300168-1301133; ORL1432 / MOR261-11/W6337-1251210-1252142; ORL1433 / MOR261-4/W6337-1237520-1238452; ORL1434 / MOR261-3/W6337-1199855-1200787; ORL1435 / MOR261-12/W6337-1006302-1007228; ORL1436 / MOR253-9/W3DA0-1192971-1192042; ORL1437 / MOR119-1/W3DA0-1130568-1129606; ORL1438 / MOR119-2/W3DA0-1103877-1104809; ORL1439 / MOR261-1/W6337-990672-991604; ORL1440 / MOR257-2/W6337-841981-842934 |

(continued)

| Name/Common Name |
|---|
| ; ORL1441 / MOR261-2/W6337-1035552-1036484; ORL1442 / MOR257-4/W6337-541732-540791; ORL1443 / MOR120-2/W7BV6-767813-766869; ORL1444 / MOR120-3/W7BV6-793646-794587; ORL1445 / MOR261-13/W6337-975599-976531; ORL1446 / MOR103-1/W6337-916741-917676; ORL1447 / MOR261-5/W6337-1284926-1285867; ORL1448 / MOR261 - 8P/W6337-1178511-1179425; ORL1449 / MOR119-3/W3DA0-1146303-1145319; ORL1450 / MOR119-4/W3DA0-1170993-1170009; ORL1451 / MOR257-5P/W6337-868900-869857; ORL1452 / MOR257-6/W6337-879606-880541; ORL1453 / MOR257-8P/W6337-824025-824919; ORL1454 / MOR115-3P/W84WR-8659523-8660427; ORL1455 / MOR261-7P/W6337-1206526-1207460; ORL1456 / MOR0-2P; ORL1457 / MOR257-7P; ORL1458 / MOR254-2/W89HK-7834386-7833439; ORL1459 / MOR254-1/W89HK-7779099-7778152; ORL146 / OR23; ORL1460 / MOR104-1 /W89HK-6435605-6436531; ORL1461 / MOR220-2/W89HK-6095600-6094689; ORL1462 / MOR251-3/W72BC-93289-94203; ORL1463 / MOR104-4/W72BC-315801-314866; ORL1464 / MOR253-7/W72BC-20708-19776; ORL1465 / MOR260-4/W6B6J-1617728-1616772; ORL1466 / MOR283-1 /W6B6J-1611192-1612142; ORL1467 / MOR283-6/W6B6J-1507816-1506869; ORL1468 / MOR283-9/W6B6J-1482894-1481947; ORL1469 / MOR34-6/W6B6J-125214-126167; ORL1470 / MOR204-5/W5Q32-9313-10245; ORL1471 / MOR204-3/W5Q32-73803-74735; ORL1472 / MOR204-20/W5Q32-60944-61876; ORL1473 / MOR204-37/W5Q32-502853-503845; ORL1474 / MOR204-11/W5Q32-425269-426201; ORL1475 / MOR204-4/W5Q32-39832-40764; ORL1476 / MOR204-16/W5Q32-230339-229374; ORL1477 / MOR204-14/W5Q32-200734-199763; ORL1478 / MOR204-2/W5Q32-186348-187286; ORL1479 / MOR204-13/W5Q32-138874-137930; ORL1480 / MOR204-6/W5M25-69366-70298; ORL1481 / MOR195-1/W5M25-192156-193085; ORL1482 / MOR253-3/W3EKE-4539929-4538997; ORL1483 / MOR103-7/W3DQU-665972-665028; ORL1484 / MOR103-11/W3DQU-637729-638667; ORL1485 / MOR222-3/W3DQU-555630-554704; ORL1486 / MOR26-2/W2YG2-89463; ORL1486 / MOR26-2/W2YG2-89463; ORL1486 / MOR26-2/W2YG2-894635-895579; ORL1487 / MOR17-1/W2YG2-878555-879514; ORL1487 / MOR26-2/W2YG2-89463; ORL1488 / MOR31-7/W2YG2-445990-446928; ORL1488 / MOR26-2/W2YG2-89463; ORL1488 / MOR26-2/W2YG2-89463; ORL1489 / MOR2-1/W2YG2-696319-695366; ORL1490 / MOR103-9/W3DQU-624353-625291; ORL1491 / MOR3-1/W2YG2-704876-703917; ORL1492 / MOR204-1/W5Q32-96696-97628; ORL1493 / MOR204-12/W5Q32-2084; ORL1493 / MOR204-8/W5Q32-631067-630123; ORL1494 / MOR260-2/W6B6J-1911482-1912426; ORL1495 / MOR283-7/W6B6J-1637655-1638614; ORL1496 / MOR219-5/W89HK-6164162-6165127; ORL1497 / MOR221-3/W89HK-6118619-6117609; ORL1498 / MOR22-1/W2YG2-903739-902789; ORL1499 / MOR39-1/W31N3-51474-52418; ORL1500 / MOR23-1/W31N3-60307-59360; ORL1501 / MOR1-4/W2YG2-973301-974254; ORL1502 / MOR13-1/W2YG2-566293-567237; ORL1503 / MOR7-1/W5P47-244947-245888; ORL1504 / MOR14-1 /W5P47-468894-469838; ORL1505 / MOR11-1 /W5P47-498289-499245; ORL1506 / MOR8-3/W5P47-330002-329064; ORL1507 / MOR16-1/W5P47-640469-639519; ORL1508 / MOR10-1/W5P47-563341-562394; ORL1509 / MOR28-1/W5P47-614770-613817; ORL1510 / MOR31-3/W5P47-663320-662370; ORL1511 / MOR20-1/W2YG2-1089552-1088599; ORL1512 / MOR19-1/W2YG2-1076170-1077123; ORL1513 / MOR34-1/W6B6J-80607-79651; |

(continued)

| Name/Common Name |
| --- |
| ORL1514 / MOR32-1/W6B6J-220125-221078; ORL1515 / MOR17-2/W2YG2-1177780-1176821; ORL1516 / MOR18-1 /W5P47-513494-512541; ORL1517 / MOR14-2/W5P47-448411-447467; ORL1518 / MOR14-3/W5P47-353138-352188; ORL1519 / MOR10-2/W5P47-302610-301654; ORL1520 / MOR30-1/W5P47-342121-343065; ORL1521 / MOR14-5/W5P47-441398-440448; ORL1522 / MOR21-1/W5P47-320524-321492; ORL1523 / MOR12-1/W2YG2-621694-622644; ORL1524 / MOR26-1/W2YG2-994891 - 995844; ORL1525 / MOR9-2/W2YG2-1129132-1130079; ORL1526 / MOR19-2/W2YG2-1080811-1079870; ORL1527 / MOR31-4/W5P47-680927-679983; ORL1528 / MOR13-2/W2YG2-575728-576672; ORL1529 / MOR31-5/W2YG2-1042430-1041468; ORL1530 / MOR13-3/W2YG2-595422-594484; ORL1531 / MOR35-1/W6B6J-352593-351640; ORL1532 / MOR101-1/W5P47-3485411 - 3484461; ORL1533 / MOR283-1 /W6B6J-1611192-1612142; ORL1534 / MOR31 - 6/W6B6J-490608-489640; ORL1535 / MOR34-3/W6B6J-112780-111824; ORL1536 / MOR32-3/W5P47-85034-85987; ORL1537 / MOR34-4/W3SPQ-88607-87639; ORL1538 / MOR34-5/W3SPQ-121168-122139; ORL1539 / MOR31-9/W2YG2-1048816-1047860; ORL1540 / MOR40-2/W6B6J-270785-271732; ORL1541 / MOR283-2/W6B6J-1667355-1666405; ORL1542 / MOR251-2/W72BC-171390-170461; ORL1543 / MOR103-5/W72BC-327936-328874; ORL1544 / MOR31-10/W2YG2-1061993-1061037; ORL1545 / MOR13-4/W2YG2-612113-613057; ORL1546 / MOR38-1 /W3SPQ-159448-160374; ORL1547 / MOR34-7/W6B6J-68112-69062; ORL1548 / MOR31-11 /W3SPQ-204520-203579; ORL1549 / MOR4-1/W2YG2-1033841-1034782; ORL1550 / MOR32-5/W6B6J-252208-253149; ORL1551 / MOR26-3/W2YG2-1159360-1158410; ORL1552 / MOR29-1/W5P47-630273-631220; ORL1553 / MOR41-1 /W5P47-552780-551806; ORL1554 / MOR31-12/W2YG2-455797-456747; ORL1555 / MOR40-3/W6B6J-466902-467864; ORL1556 / MOR204-9/W5Q32-530116-531060; ORL1557 / MOR204-10/W5Q32-475736-476680; ORL1558 / MOR204-15/W5Q32-364637-363693; ORL1559 / MOR268-1/W5M25-264554-263622; ORL1560 / MOR268-2/W5M25-284570-283626; ORL1561 / MOR104-2/W89HK-6361792-6362751; ORL1562 / MOR219-1/W89HK-6420907-6421848; ORL1563 / MOR219-2/W89HK-6392934-6393893; ORL1564 / MOR283-4/W6B6J-1599662-1598712; ORL1565 / MOR283-5/W6B6J-1534956-1534003; ORL1566 / MOR204-18/W5Q32-432045-431101; ORL1567 / MOR204-19/W5Q32-276676-275732; ORL1568 / MOR283-8/W6B6J-1471717-1470767; ORL1569 / MOR204-21/W6B6J-2565475-2564531; ORL1570 / MOR204-22/W6B6J-2584245-2583301; ORL1571 / MOR267-14/W5M25-85950-84985; ORL1572 / MOR18-3/W5P47-524511-523543; ORL1573 / MOR14-10/W5P47-426061-425069; ORL1574 / MOR32-11/W6B6J-239082-240020; ORL1575 / MOR12-5/W2YG2-687383-686445; ORL1576 / MOR283-11/W6B6J-1686614-1685664; ORL1577 / MOR40-13/W5M25-5796-4837; ORL1578 / MOR204-34/W5M25-106672-107616; ORL1579 / MOR204-35/W5Q32-455235-454291; ORL1580 / MOR25-1/W5P47-581982-581029; ORL1581 / MOR24-2/W5P47-30107-29124; ORL1582 / MOR5-1/W2YG2-631099-630152; ORL1583 / MOR5-2/W2YG2-655151-654204; ORL1584 / MOR8-1/W5P47-274598-275533; ORL1585 / MOR7-2/W5P47-256131-257039; ORL1586 / MOR14-4/W5P47-135621-134671; ORL1587 / MOR9-1/W2YG2-1143235-1144194; ORL1588 / MOR27-1/W3SPQ-138685-137720; ORL1589 / MOR33-2/W2YG2-529814-528876; ORL1590 / MOR30-2/W5P47-147829-146885; ORL1591 / MOR30-3/W5P47-190842-189898; |

(continued)

| Name/Common Name |
| --- |
| ORL1592 / MOR31-8/W3SPQ-192551-191604; ORL1593 / MOR253-4/W3EKE-4576729-4575797; ORL1594 / MOR14-6/W5P47-181031-180081; ORL1595 / MOR204-7/W5M25-61183-62133; ORL1596 / MOR268-3/W5M25-151217-152161; ORL1597 / MOR38-2/W3SPQ-168888-167914; ORL1598 / MOR14-9/W5P47-281703-280732; ORL1599 / MOR36-1/W6B6J-511388-512350; ORL1600 / MOR204-36/W5Q32-573047-574039; ORL1601 / MOR268-5/W5M25-320546-319602; ORL1602 / MOR6-1/W5P47-97167-96226; ORL1603 / MOR283-3/W6B6J-1546343-1545396; ORL1604 / MOR251-4P/W89HK-6450423-6451351; ORL1605 / MOR220-3/W89HK-6243915-6242982; ORL1606 / MOR232-8/W3DQU-611021-611809; ORL1607 / MOR252-1/W72BC-8919-9851; ORL1608 / MOR252-2/W72BC-120727-121659; ORL1609 / MOR204-24P/W5M25-14287-15085; ORL1610 / MOR204-25P/W5Q32-593548-594481; ORL1611 / MOR252-3P/W72BC-46594-45631; ORL1612 / MOR283-12P/W6B6J-1658091-1659040; ORL1613 / MOR267-15/W5Q32-160656-161603; ORL1614 / MOR220-1/W89HK-6195373-6194378; ORL1615 / MOR40-10P/W2P1E-19614-20465; ORL1616 / MOR34-2/W6B6J-104312-103364; ORL1617 / MOR103-6/W3DQU-652373-651429; ORL1618 / MOR31-13/W5P47-673958-673015; ORL1619 / MOR227-6P/W89HK-6487850-6488818; ORL1620 / MOR31-14/W2YG2-430136-429156; ORL1621 / MOR4-2P/W2YG2-964004-964935; ORL1622 / MOR177-11 P/W2YG2-947213-948100; ORL1623 / MOR40-6P/W6B6J-276628-275779; ORL1624 / MOR283-10P/W6B6J-1583910-1582958; ORL1625 / MOR32-6P/W5P47-6290-5337; ORL1626 / MOR32-7P/W6B6J-191967-191091; ORL1627 / MOR0-1 P/W5P47-75410-74513; ORL1628 / MOR31-15P/W2YG2-950882-949921; ORL1629 / MOR37-1/W6B6J-329762-328752; ORL1630 / MOR32-8/W6B6J-148632-148009; ORL1631 / MOR202-22P/W2YG2-1185910-1186564; ORL1632 / MOR0-3P/W5P47-54886-54160; ORL1633 / MOR8-5/W5P47-264777-264052; ORL1634 / MOR32-9P/W6B6J-209903-209472; ORL1635 / MOR260-8P/W6B6J-1692034-1690968; ORL1636 / MOR13-5/W2YG2-550580-551164; ORL1637 / MOR14-8P/W5P47-411244-412187; ORL1638 / MOR24-1P/W5P47-67612-68573; ORL1639 / MOR0-5P/W2YG2-467767-468776; ORL1640 / MOR219-3P/W89HK-6370632-6371598; ORL1641 / MOR103-14P/W72BC-289736-290309; ORL1642 / MOR204-27P/W5Q32-615; ORL1642 / MOR204-27P/W5Q32-615175-616120; ORL1643 / MOR204-31 P/W5Q32-322610-322079; ORL1644 / MOR204-29P/W5Q32-314948-314110; ORL1645 / MOR12-4/W2YG2-677537-676583; ORL1646 / MOR40-11/W6B6J-428225-429190; ORL1647 / MOR251-5/W72BC-158383-157465; ORL1648 / MOR33-3P/W2YG2-1168052-1169035; ORL1649 / MOR32-12/W6B6J-161291-160337; ORL1650 / MOR124-1/W3DQU-686120-685154; ORL1651 / MOR204-17/W5Q32-395664-394720; ORL1652 / MOR10-3P/W5P47-287680-288551; ORL1653 / MOR204-23/W5M25-51685-52620; ORL1654 / MOR8-4P/W5P47-216733-217416; ORL1655 / MOR204-26P/W5Q32-385295-386253; ORL1656 / MOR12-2P/W2YG2-644327-645240; ORL1657 / MOR12-3P/W2YG2-638568-637702; ORL1658 / MOR32-10/W5P47-21851-21168; ORL1659 / MOR171-29P/W5P47-225462-226037; ORL1660 / MOR30-4P/W5P47-372489-373321; ORL1661 / MOR4-3P/W2YG2-1005391-1006203; ORL1662 / MOR268-4/W5M25-308165-307222; ORL1663 / MOR221-1P/W89HK-6342791-6342279; ORL1664 / MOR234-4P/W89HK-6320831-6319861; ORL1665 / MOR204-28P/W5Q32-524681-525575; ORL1666 / MOR204-30P/W5Q32-263948-263325; |

(continued)

| Name/Common Name |
|---|
| ORL1667 / MOR101-2/W5P47-3453253-3452420; ORL1668 / MOR42-2/W5P47-737770-738778; ORL1669 / MOR221-2/W89HK-6298490-6297552; ORL1670 / MOR34-10P/W3SPQ-36679-37599; ORL1671 / MOR40-12/W3SPQ-56026-55068; ORL1672 / MOR219-4/W89HK-6274592-6275590; ORL1673 / MOR40-5; ORL1674 / MOR22-4P; ORL1675 / MOR8-6P; ORL1676 / MOR0-4P; ORL1677 / MOR40-8P; ORL1678 / MOR260-9P; ORL1679 / MOR268-6; ORL1680 / MOR15-2P; ORL1681 / MOR267-16/W3E7T-1122; ORL1682 / MOR152-3/W6RL5-13729544-13728600; ORL1683 / MOR149-2/W6RL5-13479215-13480153; ORL1684 / MOR146-2/W6RL5-13033383-13034336; ORL1685 / MOR145-6/W6RL5-12762912-12761983; ORL1686 / MOR145-3/W6RL5-12641128-12640199; ORL1687 / MOR145-2/W6RL5-12600931-12599972; ORL1688 / MOR165-7/W60AJ-997074-996124; ORL1689 / MOR167-4/W60AJ-957289-958221; ORL1690 / MOR168-1 /W60AJ-946440-947372; ORL1691 / MOR171-23/W60AJ-906653-907588; ORL1692 / MOR165-6/W60AJ-849038-848106; ORL1693 / MOR165-8/W60AJ-829433-828492; ORL1694 / MOR161-3/W60AJ-789779-788844; ORL1695 / MOR171-11/W60AJ-632583-633509; ORL1696 / MOR161-4/W60AJ-1838848-1837916; ORL1697 / MOR161-5/W60AJ-1756545-1755610; ORL1698 / MOR162-7/W60AJ-1618515-1617571; ORL1699 / MOR162-3/W60AJ-1468557-1467613; ORL1700 / MOR164-1/W60AJ-1203283-1202339; ORL1701 / MOR167-3/W/60AJ-1146353-1145421; ORL1702 / MOR167-2/W60AJ-1112443-1111508; ORL1703 / MOR165-5/W60AJ-1101738-1100806; ORL1704 / MOR165-3/W60AJ-1061362-1060430; ORL1705 / MOR165-4/W60AJ-1021597-1020665; ORL1706 / MOR165-3/W60AJ-1021597-1020665; ORL1707 / MOR162-1/W3WPH-400817-399870; ORL1708 / MOR171-1 /W3WPH-265152-266084; ORL1709 / MOR224-1/W3WPH-23217-22300; ORL1710 / MOR171-7/W3WPH-138374-139309; ORL1711 / MOR171-15/W3WPH-108802-107858; ORL1712 / MOR165-2/W60AJ-10842; ORL1713 / MOR170-3/W60AJ-12608; ORL1714 / MOR152-2/W6RL5-13713; ORL1715 / MOR170-7/W60AJ-12973; ORL1716 / MOR162-6/W60AJ-15850; ORL1717 / MOR164-3/W60AJ-93138; ORL1718 / MOR224-9/W60AJ-11080; ORL1719 / MOR170-1 /W60AJ-13340; ORL1720 / MOR171-4/W3WPH-43551; ORL1721 / MOR223-1/W3WPH-32030; ORL1722 / MOR223-3/W3WPH-31288; ORL1723 / MOR224-2/W3WPH-27608; ORL1724 / MOR167-1 /W60AJ-11376; ORL1725 / MOR169-1 /W60AJ-1161422-1160493; ORL1726 / MOR167-3/W60AJ-1146353-1145421; ORL1727 / MOR104-3/W3WPH-497635-498573; ORL1728 / MOR171-14/W3WPH-239372-240295; ORL1729 / MOR239-6/W3WPH-409342-408398; ORL1730 / MOR155-2/W6RL5-13602165-13603106; ORL1731 / MOR151-1/W6RL5-13416193-13415255; ORL1732 / MOR148-1/W6RL5-13273718-13272780; ORL1733 / MOR223-8/W3WPH-34116; ORL1734 / MOR149-3/W6RL5-13493742-13494680; ORL1735 / MOR223-2/W3WPH-326022-325090; ORL1736 / MOR147-1/W6RL5-13982660-13981722; ORL1737 / MOR154-1/W6RL5-13319675-13320592; ORL1738 / MOR155-1/W6RL5-13361011-13360073; ORL1739 / MOR170-2/W60AJ-1229518-1228577; ORL174 / TPCR05; ORL1740 / MOR223-4/W3WPH-382023-382976; ORL1741 / MOR164-2/W60AJ-777570-776635; ORL1742 / MOR149-1/W6RL5-14022842-14023780; ORL1743 / MOR152-1/W6RL5-13964024-13963086; ORL1744 / MOR145-5/W6RL5-12875053-12875991; ORL1745 / MOR147-2/W6RL5-13379299-13380237; ORL1746 / MOR147-3/W6RL5-13290934-13291872; ORL1747 / MOR170-8/W60AJ-1240019-1239045; |

(continued)

| Name/Common Name |
| --- |
| ORL1748 / MOR224-10/W60AJ-1240019-1239045; ORL1749 / MOR171-28/W3WPH-56024-56963; ORL175 / TPCR06; ORL1750 / MOR171-30P/W3WPH-86848-87611; ORL1751 / MOR153-2/W6RL5-13860892-13859953; ORL1752 / MOR146-4/W6RL5-12897602-12896673; ORL1753 / MOR146-6P/W6RL5-13138523-13137594; ORL1754 / MOR146-7P/W6RL5-13162713-13162216; ORL1755 / MOR170-10P/W60AJ-1311845-1310918; ORL1756 / MOR166-1/W60AJ-1053184-1052245; ORL1757 / MOR162-8/W60AJ-1180041-1179105; ORL1758 / MOR171-43/W60AJ-349827-350766; ORL1759 / MOR171-37/W60AJ-585982-586536; ORL176 / TPCR07; ORL1760 / MOR171-35P/W60AJ-528829-529773; ORL1761 / MOR171-33P/W60AJ-250779-250246; ORL1762 / MOR165-1/W60AJ-1076516-1075581; ORL1763 / MOR144/W6RL5-12615238-12614346; ORL1764 / MOR146-1/W6RL5-12995621-12996541; ORL1765 / MOR170-9/W60AJ-1428689-1429648; ORL1766 / MOR165-9P/W60AJ-1008428-1007562; ORL1767 / MOR171-26P/W60AJ-280095-279129; ORL1768 / MOR168-2P/W60AJ-938982-939913; ORL1769 / MOR224-7P/W3WPH-5148-6094; ORL177 / TPCR09; ORL1770 / MOR266-7P/W6RL5-13631800-13632281; ORL1771 / MOR171-34/W60AJ-190077-190616; ORL1772 / MOR171-36P/W60AJ-743262-742475; ORL1773 / MOR171-38P/W3WPH-60472-61323; ORL1774 / MOR162-9P/W60AJ-1563660-1562955; ORL1775 / MOR162-10P/W60AJ-1607698-1606937; ORL1776 / MOR0-12P/W60AJ-1540903-1540427; ORL1777 / MOR171-40P/W60AJ-186080-186905; ORL1778 / MOR170-12/W60AJ-1421479-1420616; ORL1779 / MOR162-12/W60AJ-1549861-1548931; ORL178 / TPCR11; ORL1780 / MOR145-4/W6RL5-12799498-12798575; ORL1781 / MOR171-27P/TW57D-35-838; ORL1782 / MOR153-1 /W6RL5-13896777-13895841; ORL1783 / MOR154-2P/W6RL5-13619312-13619642; ORL1784 / MOR146-5P/W6RL5-12942448-12943376; ORL1785 / MOR167-5P/W60AJ-1153822-1152921; ORL1786 / MOR153-3/W6RL5-13542197-13541268; ORL1787 / MOR224-11/W3WPH-299691-298749; ORL1788 / MOR143-2/W6RL5-12847842-12848782; ORL1789 / MOR150-3/W6RL5-13814160-13813309; ORL179 /TPCR15; ORL1790 / MOR152-4P; ORL1791 / MOR0-7P; ORL1792 / W6RL5-13695863-13696754; ORL1793 / W6RL5-13645512-13646; ORL1794 / W60AJ-925630-926226; ORL1795 / W60AJ-719102-719883; ORL1796 / W60AJ-716549-716307; ORL1797 / W60AJ-703922-704828; ORL1798 / W60AJ-666029-666595; ORL1799 / W60AJ-540286-540740; ORL180 / TPCR18; ORL1800 / W60AJ-4787-4010; ORL1801 / W60AJ-354484-355428; ORL1802 / W60AJ-1667802-166691; ORL1803 / W3WPH-39574-39485; ORL1804 / MOR210-2/W6HFP-3757312-3756371; ORL1805 / MOR210-2/W6HFP-3757312-3756371; ORL1806 / MOR210-1/W6HFP-3739754-3738792; ORL1807 / MOR116-1/W6HFP-3595463-3596401; ORL1808 / MOR110-2/W6HFP-3549701-3548745; ORL1809 / MOR110-1 /W6HFP-3450232-3449300; ORL181 / TPCR33; ORL1810 / MOR113-4/W6HFP-3399224-3398286; ORL1811 / MOR108-4/W6HFP-3383649-3384590; ORL1812 / MOR112-2/W6HFP-3375233-3376171; ORL1813 / MOR117-1/W6HFP-3363319-3362384; ORL1814 / MOR110-5/W6HFP-3347336-3346395; ORL1815 / MOR110-4/W6HFP-3331957-3330998; ORL1816 / MOR111-1/W6HFP-3291668-3290730; ORL1817 / MOR110-10/W6HFP-3279455-3278499; ORL1818 / MOR114-7/W6HFP-3268746-3269681; ORL1819 / MOR114-2/W6HFP-3256141-3257076; ORL182 / TPCR34; ORL1820 / MOR108-1/W6HFP-3234783-3233848; ORL1821 / MOR114-11 /W6HFP-3190827-3191789; |

(continued)

| Name/Common Name |
|---|
| ORL1822 / MOR111-5/W6HFP-3057560-3058498; ORL1823 / MOR111-5/W6HFP-3057560-3058498; ORL1824 / MOR114-9/W6HFP-2942174-2941239; ORL1825 / MOR111-7/W6HFP-2933787-2934725; ORL1826 / MOR111-6/W6HFP-2933787-2934725; ORL1827 / MOR114-8/W6HFP-2867861-2866908; ORL1828 / MOR114-5/W6HFP-2843027-2842092; ORL1829 / MOR269-1/W6HFP-3217337-3216405; ORL183 / TPCR35; ORL1830 / MOR110-6/W6HFP-3410153-3409194; ORL1831 / MOR114-1/W6HFP-3146902-3147840; ORL1832 / MOR114-3/W6HFP-2882441-2881506; ORL1833 / MOR114-4/W6HFP-2803187-2802249; ORL1834 / MOR269-2/W6HFP-2721811-2722740; ORL1835 / MOR269-3/W6HFP-2701088-2702026; ORL1836 / MOR109-1/W6HFP-3611745-3612677; ORL1837 / MOR115-1/W6HFP-2789174-2788245; ORL1838 / MOR110-3/W6HFP-3009185-3010144; ORL1839 / MOR210-3/W6HFP-3703726-3702779; ORL184 / TPCR50; ORL1840 / MOR210-4/W6HFP-3689603-3688656; ORL1841 / MOR113-2/W6HFP-3651269-3652207; ORL1842 / MOR111-4/W6HFP-3093779-3094714; ORL1843 / MOR115-4/W6HFP-2757264-2756335; ORL1844 / MOR210-5/W6HFP-3784755-3783787; ORL1845 / MOR114-12/W6HFP-2976813-2977751; ORL1846 / MOR267-9/W4DUT-349638-348697; ORL1847 / MOR112-1 /W6HFP-3121737-3122681; ORL1848 / MOR113-1 /W6HFP-3518090-3517152; ORL1849 / MOR113-3/W6HFP-3508522-3507590; ORL185 / TPCR51; ORL1850 / MOR111-3/W6HFP-3300973-3300047; ORL1851 / MOR111-6/W6HFP-2923529-2924467; ORL1852 / MOR110-7/W6HFP-3313619-3314564; ORL1853 / MOR114-6/W6HFP-2988069-2989004; ORL1854 / MOR111-2/W6HFP-2911245-2912145; ORL1855 / MOR108-3/W6HFP-3463799-3464389; ORL1856 / MOR115-2/W6HFP-2772746-2771814; ORL1857 / MOR128-3/W4DUT-256495-257527; ORL1858 / MOR139-1/W5685-288383-287430; ORL1859 / MOR111-8P/W6HFP-3030497-3031412; ORL186 / TPCR52; ORL1860 / MOR108-2/W6HFP-3161206-3162115; ORL1861 / MOR111-10/W6HFP-3083812-3084751; ORL1862 / MOR110-11 P/W6HFP-2966353-2967297; ORL1863 / MOR139-5P/W4DUT-1226; ORL1864 / MOR111-9P/W6HFP-3108111-3108545; ORL1865 / MOR113-5; ORL1866 / MOR0-8P; ORL1867 / MOR281-2P; ORL1868 / MOR110-9/W6HFP-3108111-3108545; ORL1869 / MOR277-1 /W6C8B-388180-387227; ORL187 / TPCR53; ORL1870 / MOR256-23/W6C8B-32217-31261; ORL1871 / MOR135-1 /W6C8B-32217-31261; ORL1872 / MOR135-2/W5FBR-2021278-2020334; ORL1873 / MOR135-13/W5FBR-1991788-1990844; ORL1874 / MOR238-2/W4NS2-78020-77115; ORL1875 / MOR174-13/W4NS2-357052-357990; ORL1876 / MOR240-2/W4MDW-2817370-2818311; ORL1877 / MOR135-6/W3YLM-7607774-7608712; ORL1878 / MOR135-26/W3YLM-7598349-7597414; ORL1879 / MOR135-9/W3YLM-7572462-7573400; ORL188 / TPCR55; ORL1880 / MOR157-1/W3YLM-7412114-7413058; ORL1881 / MOR255-6/W3YLM-7279975-7279028; ORL1882 / MOR255-3/W3YLM-7073316-7074251; ORL1883 / MOR256-30/W30/M0-983997-984956; ORL1884 / MOR285-1/W3KVV-2757303-2758223; ORL1885 / MOR275-2/W3KVV-2625833-2624901; ORL1886 / MOR275-1/W3KVV-2597870-2596941; ORL1887 / MOR129-2/W31JB-138145-137198; ORL1888 / MOR256-1/W6C8B-110021-109086; ORL1889 / MOR256-2/W6C8B-197238-196303; ORL189 / TPCR56; ORL1890 / MOR135-3/W5FBR-2185278-2184340; ORL1891 / MOR255-5/W3YLM-7085597-7086544; ORL1892 / MOR278-1/W3UM0-1803300-1802371; ORL1893 / MOR126-2/W31JB-68197-67256; |

(continued)

| Name/Common Name |
|---|
| ORL1894 / MOR256-22/W6C8B-80908-79973; ORL1895 / MOR256-24/W6C8B-257802-256867; ORL1896 / MOR256-26/W6C8B-13397-12462; ORL1897 / MOR256-27/W6C8B-210363-209428; ORL1898 / MOR256-28/W6C8B-96644-95709; ORL1899 / MOR280-1/W6C8B-376427-375489; ORL1900 / MOR285-2/W3KVV-2772718-2771798; ORL1901 / MOR255-1 /W3YLM-7346099-7347034; ORL1902 / MOR174-12/W4NS2-419234-420184; ORL1903 / MOR225-1/W4NS2-221457-222386; ORL1904 / MOR225-2/W4NS2-231090-232013; ORL1905 / MOR225-3/W4NS2-149072-148137; ORL1906 / MOR237-2/W4NS2-45766-44846; ORL1907 / MOR174-5/W4NS2-293140-292193; ORL1908 / MOR222-1 /W3UM0-1727780-1726854; ORL1909 / MOR230-6/W4NS2-174182-173271; ORL1910 / MOR240-1/W4MDW-2837164-2838099; ORL1911 / MOR240-3/W4MDW-2841132-2842067; ORL1912 / MOR107-1/W3KVV-2701653-2700682; ORL1913 / MOR264-17/W3Y5M-740400-741371; ORL1914 / MOR135-12/W5FBR-1970531-1971484; ORL1915 / MOR230-5/W4NS2-106566-105670; ORL1916 / MOR284-2/W3KVV-2563526-2562579; ORL1917 / MOR256-50/W6C8B-70577-69647; ORL1918 / MOR135-16P/W5FBR-2142302-2141401; ORL1919 / MOR135-15/W5FBR-1984802-1983859; ORL1920 / MOR225-9P/W4NS2-210736-211663; ORL1921 / MOR174-17P/W4NS2-390869-391831; ORL1922 / MOR275-5/TR0HK-2-718; ORL1923 / MOR225-6P/W4NS2-249373-248464; ORL1924 / MOR125-2P/W3YLM-7108216-7109156; ORL1925 / MOR125-3P/W3YLM-7151569-7152504; ORL1926 / MOR256-41P/W6C8B-46771-46067; ORL1927 / MOR135-25/W5FBR-2029534-2028601; ORL1928 / MOR256-47/W3KVV-2745169-2746152; ORL1929 / MOR133-5P/W3YLM-7168037-7167066; ORL1930 / MOR278-2/W3UM0-1795241-1794306; ORL1931 / MOR102-2/W3KVV-2673069-2674016; ORL1932 / MOR256-51/W3UM0-960317-959358; ORL1933 / MOR255-7P/W3YLM-7291109-7290503; ORL1934 / MOR222-2/W3UM0-1747565-1747342; ORL1935 / MOR275-3/W3KVV-2640964-2640017; ORL1936 / MOR135-22/W3YLM-7657215-7658155; ORL1937 / MOR275-4/W3KVV-2574755-2573810; ORL1938 / MOR285-3P/W3UM0-1821481-1820555; ORL1939 / MOR174-15P/W4NS2-308539-309478; ORL1940 / MOR225-8P/W4NS2-1882; ORL1941 / MOR230-9/W4NS2-1357-998; ORL1942 / MOR230-10P/W4NS2-132092-131726; ORL1943 / MOR285-4/W3KVV-2783733-2782812; ORL1944 / MOR275-7P/W3KVV-2648951-2647977; ORL1945 / MOR177-13P/W3Y5M-1077364-1078166; ORL1946 / MOR225-11 /W4NS2-2196; ORL1947 / MOR174-19/W4NS2-287980-288915; ORL1948 / MOR256-50/W6C8B-70577-69647; ORL1949 / MOR256-54P/W6C8B-22132-21737; ORL1950 / MOR133-3P/W3YLM-7118727-7117675; ORL1951 / MOR125-4P/W3YLM-7125586-7126621; ORL1952 / MOR222-4P/W3UM0-1737519-1736827; ORL1953 / MOR284-1P/W3KVV-2551151-2550208; ORL1954 / MOR256-45P/W3UM0-1002102-1002975; ORL1955 / MOR174-18/W4NS2-320420-321358; ORL1956 / MOR5-3; ORL1957 / MOR275-6P; ORL1958 / MOR256-34P; ORL1959 / MOR264-1/W3Y5M-711622-712602; ORL1960 / MOR264-3/W3Y5M-756846-757802; ORL1961 / MOR180-1/W3KVV-474678-475610; ORL1962 / MOR188-3/W3KVV-86109-87068; ORL1963 / MOR172-1 /W3KVV-4772-3834; ORL1964 / MOR174-2/W3Y5M-1273418-1274383; ORL1965 / MOR188-4/W3Y5M-100338-99397; ORL1966 / MOR172-3/W3Y5M-443979-443041; ORL1967 / MOR172-5/W3Y5M-428639-427701; ORL1968 / MOR172-6/W3Y5M-384055-383117; ORL1969 / MOR179-1/W3Y5M-384055-383117; |

(continued)

| Name/Common Name |
| --- |
| ORL1970 / MOR264-6/W3Y5M-531718-532674; ORL1971 / MOR174-6/W3Y5M-1372914-1371964; ORL1972 / MOR174-7/W3Y5M-1350208-1349258; ORL1973 / MOR173-2/W3Y5M-1167169-1166237; ORL1974 / MOR173-3/W3Y5M-12350; ORL1975 / MOR174-10/W3Y5M-1189477-1188533; ORL1976 / MOR177-9/W5D7G-2505139-2505348; ORL1977 / MOR179-3/W3Y5M-325746-324814; ORL1978 / MOR196-3/W3KVV-412059-411124; ORL1979 / MOR185-5/W3KVV-172878-173837; ORL1980 / MOR196-4/W3KVV-375806-374883; ORL1981 / MOR194-1/W3KVV-98261-99211; ORL1982 / MOR201-2/W3KVV-456703-455750; ORL1983 / MOR199-1/W3KVV-292755-291823; ORL1984 / MOR199-2/W3KVV-264357-263425; ORL1985 / MOR174-14/W3Y5M-1298702-1297758; ORL1986 / MOR189-1/W4CQV-69826-70767; ORL1987 / MOR192-1/W4CQV-54584-55525; ORL1988 / MOR190-1/W4CQV-159242-160183; ORL1989 / MOR189-3/W4CQV-121801-122742; ORL1990 / MOR177-2/W3Y5M-973343-972411; ORL1991 / MOR176-1 /W3Y5M-957878-956940; ORL1992 / MOR176-2/W3Y5M-94723; ORL1993 / MOR177-1/W3Y5M-936964-936038; ORL1994 / MOR177-4/W3Y5M-929723-930652; ORL1995 / MOR264-4/W3Y5M-908625-909569; ORL1996 / MOR264-9P/W3Y5M-898130-898624; ORL1997 / MOR176-3/W3Y5M-868624-867689; ORL1998 / MOR177-5/W3Y5M-855017-855940; ORL1999 / MOR264-5/W3Y5M-783242-784186; ORL2000 / MOR264-2/W3Y5M-681084-682004; ORL2001 / MOR264-20/W3Y5M-578493-579473; ORL2002 / MOR181-2/W3Y5M-487999-487061; ORL2003 / MOR172-4/W3Y5M-473705-472767; ORL2004 / MOR172-2/W3Y5M-407847-406909; ORL2005 / MOR188-5/W3Y5M-36573-35632; ORL2006 / MOR206-4/W3Y5M-315220-314255; ORL2007 / MOR206-1/W3Y5M-261359-260433; ORL2008 / MOR179-2/W3Y5M-23329-22397; ORL2009 / MOR206-2/W3Y5M-229046-228099; ORL2010 / MOR206-6/W3Y5M-211534-210588; ORL2011 / MOR174-16/W3Y5M-1397217-1396267; ORL2012 / MOR174-8/W3Y5M-1319379-1318429; ORL2013 / MOR179-6/W3Y5M-131445-132362; ORL2014 / MOR174-1/W3Y5M-1209167-1208229; ORL2015 / MOR177-12/W3Y5M-1153117-1154050; ORL2016 / MOR177-14/W3Y5M-1087729-1088661; ORL2017 / MOR177-10/W3Y5M-1042284-1041349; ORL2018 / MOR177-6/W3Y5M-1034488-1035423; ORL2019 / MOR177-8/W3Y5M-1026516-1025581; ORL2020 / MOR187-2/W3KVV-73368-74309; ORL2021 / MOR187-1/W3KVV-54811-55737; ORL2022 / MOR260-5/ W3KVV-498829-497894; ORL2023 / MOR198-3P/W3KVV-348455-347641; ORL2024 / MOR198-1 P/W3KVV-324361-323428; ORL2025 / MOR196-2/W3KVV-315800-314862; ORL2026 / MOR171-31 P/W3KVV-214374-215339; ORL2027 / MOR185-3/W3KVV-182345-181386; ORL2028 / MOR185-10/W3KVV-159319-160261; ORL2029 / MOR185-2/W3KVV-123798-124745; ORL2030 / MOR135-8/W3YLM-7511751-7512683; ORL2031 / MOR177-3/W3Y5M-988304-987372; ORL2032 / MOR191-1/ W3Y5M-2793-1852; ORL2033 / MOR273-4P/W3Y5M-574562-574891; ORL2034 / MOR211-5P/W3Y5M-847869-846998; ORL2035 / MOR40-9P/W3Y5M-1000696-999764; ORL2036 / MOR191-2P/W4CQV-5593-6007; ORL2037 / MOR185-4/W3KVV-147900-148844; ORL2038 / MOR264-11/W3Y5M-1131126-1132077; ORL2039 / MOR264-12P/W3Y5M-1101754-1102679; ORL2040 / MOR264-14P/W3Y5M-1124370-1125280; ORL2041 / MOR179-4/W3Y5M-337640-338449; ORL2042 / MOR0-6P/W3Y5M-369203-368240; ORL2043 / MOR206-5P/W3Y5M-346436-345499; ORL2044 / MOR198-2P/W3KVV-366917-366171; ORL2045 / MOR227-8P/W3KVV-259526-258592; |

(continued)

| Name/Common Name |
| --- |
| ORL2046 / MOR262-13/W3KVV-224823-224347; ORL2047 / MOR264-18/W3Y5M-695715-696659; ORL2048 / MOR264-15P/W3Y5M-676374-677272; ORL2049 / MOR189-4P/W4CQV-82139-83056; ORL2050 / MOR188-6P/W4CQV-116400-117309; ORL2051 / MOR197-1/W3KVV-393843-394826; ORL2052 / MOR213-9/W3KVV-536600-537496; ORL2053 / MOR174-3/W3Y5M-1196210-1195248; ORL2054 / MOR206-3/W3Y5M-296158-295220; ORL2055 / MOR207-1/W3Y5M-356813-355910; ORL2056 / MOR173-1/W3Y5M-1255560-1254628; ORL2057 / MOR181-1/W3Y5M-1066159-1067103; ORL2058 / MOR174-11/W3Y5M-1342110-1341187; ORL2059 / MOR177-7/W3Y5M-1159666-1160598; ORL2060 / MOR196-1/W3KVV-428780-427833; ORL2061 / MOR201-1/W3KVV-433882-432941; ORL2062 / MOR185-7/W3KVV-156736-157680; ORL2063 / MOR189-2/W4CQV-99803-98862; ORL2064 / MOR179-7/W3Y5M-174300-175226; ORL2065 / MOR200-1/W3KVV-307786-306848; ORL2066 / MOR135-23P/W3Y5M-1248002-1247109; ORL2067 / MOR264-10P/W3Y5M-612781-613150; ORL2068 / MOR264-13P/W3Y5M-1107873-1108760; ORL2069 / MOR196-5P/W3KVV-380839-380015; ORL2070 / MOR264-7/W3Y5M-1117881-1118826; ORL2071 / MOR264-7/W3Y5M-596904-597851; ORL2072 / MOR264-19/W3Y5M-1095234-1096178; ORL2073 / MOR256-13/W36P1-1564537-1563617; ORL2074 / MOR256-15/W36P1-1134904-1133960; ORL2075 / MOR256-8/W36P1-1599610-1598684; ORL2076 / MOR256-16/W36P1-1335929-1336879; ORL2077 / MOR256-10/W36P1-1655207-1654266; ORL2078 / MOR256-14/W36P1-1064499-1063549; ORL2079 / MOR218-3/W53BW-2302306-2303253; ORL2080 / MMOR130-2/W36P1-1527364-1526423; ORL2081 / MOR209-1/W53BW-2310315-2311241; ORL2082 / MOR256-37P/W36P1-1124698-1125621; ORL2083 / MOR256-35/W36P1-1682806-1683748; ORL2084 / MOR256-36/W36P1-1545399-1544456; ORL2085 / MOR136-18P/W79P7-387984-388473; ORL2086 / MOR246-4/W5WBF-5318159-5317194; ORL2087 / MOR223-5/W3FNU-207743-208684; ORL2088 / MOR223-6/W3FNU-176078-177034; ORL2089 / MOR223-9/W3FNU-194395-195351; ORL2090 / MOR106-2/W5WBF-5658583-5659512; ORL2091 / MOR106-4/W5WBF-5714619-5715554; ORL2092 / MOR106-5/W5WBF-5627068-5628006; ORL2093 / MOR241-1/W5WBF-5513401-5512475; ORL2094 / MOR242-1/W5WBF-5551698-5550757; ORL2095 / MOR241-2/W5WBF-5530471-5529545; ORL2096 / MOR241-3/W5WBF-5125867-5124941; ORL2097 / MOR205-1/W3FNU-548557-549492; ORL2098 / MOR274-2/W5K7W-1421153-1420203; ORL2099 / MOR24-3/W31 N3-39647-40591; ORL2100 / MOR106-11/W5WBF-5844920-5845906; ORL2101 / MOR106-12/W5WBF-5855806-5856750; ORL2102 / MOR246-6/W5WBF-5382849-5381878; ORL2103 / MOR106-3/W5WBF-5647630-5648565; ORL2104 / MOR247-1/W5WBF-5424209-5423256; ORL2105 / MOR246-5/W5WBF-5477112-5476171; ORL2106 / MOR247-2/W5WBF-5193002-5192073; ORL2107 / MOR106-7/W5WBF-5881541-5880633; ORL2108 / MOR243-1/W5WBF-5579770-5578823; ORL2109 / MOR274-3P/W5K7W-1625757-1626158; ORL2110 / MOR246-3/W5K7W-1625757-1626158; ORL2111 / MOR106-8P/W5WBF-5756058-5757016; ORL2112 / MOR246-1 P/W5WBF-5373821-5373242; ORL2113 / MOR106-9P/TY7JT-1-683; ORL2114 / MOR247-3P; ORL2115 / MOR223-7P/W3FNU-229666-230239; ORL2116 / MOR0-11 P/W5WBF-5645423-5645169; ORL2117 / MOR256-43P/TT5SB-1055-1508; ORL2118 / MOR160-3/W76D0-7743435-7742476; ORL2119 / MOR286-1/W76D0-7636058-7635105; |

(continued)

| Name/Common Name |
|---|
| ORL2120 / MOR160-5/W76D0-7880527-7881462; ORL2121 / MOR122-1/W76D0-7918592-7919524; ORL2122 / MOR160-4/W76D0-7768109-7767192; ORL2123 / MOR184-6/W8B52-422231-423190; ORL2124 / MOR184-4/W8B52-384545-385495; ORL2125 / MOR183-2/W8B52-321033-320104; ORL2126 / MOR183-8/W8B52-284949-285875; ORL2127 / MOR184-5/W8B52-173804-172878; ORL2128 / MOR256-17/W5U4U-231954-232892; ORL2129 / MOR184-2/W8B52-583655-584584; ORL2130 / MOR184-3/W8B52-547947-548912; ORL2131 / MOR182-1/W8B52-60811-61734; ORL2132 / MOR182-2/W8B52-75656-76582; ORL2133 / MOR184-8/W8B52-480129-481085; ORL2134 / MOR182-3/W8B52-30619-31536; ORL2135 / MOR182-4/W8B52-5538-6458; ORL2136 / MOR273-1/W2Y5S-20490-19549; ORL2137 / MOR273-2/W2Y5S-2819-1881; ORL2138 / MOR183-1/W8B52-154784-155713; ORL2139 / MOR182-5/W8B52-42276-41356; ORL2140 / MOR183-3/W8B52-143560-144489; ORL2141 / MOR279-1/W2P81-48667-47726; ORL2142 / MOR279-2/W55B7-3561423-3560482; ORL2143 / MOR183-9/W8B52-293337-294266; ORL2144 / MOR184-9/W8B52-395557-396511; ORL2145 / MOR182-8/W8B52-127561-128482; ORL2146 / MOR131-2P/W5U4U-10625-11514; ORL2147 / MOR182-7P/W8B52-195313-196278; ORL2148 / MOR183-5P/W8B52-228430-229144; ORL2149 / MOR183-6P/W8B52-271329-272258; ORL2150 / MOR113-6P/W8B52-258524-259018; ORL2151 / MOR184-10P/W8B52-497314-497744; ORL2152 / MOR113-7P/W8B52-203902-204307; ORL2153 / MOR0-9P/W8B52-313496-314070; ORL2154 / MOR273-3P/W3JHV-27735-28674; ORL2155 / MOR270-1/W3JHV-107245-106307; ORL2156 / MOR271-1 /W3JHV-63301-64239; ORL2157 / MOR272-1/W3JHV-78430-79368; ORL2158 / MOR263-3/W5KGR-775383-774418; ORL2159 / MOR263-4/W5KGR-746570-745605; ORL2160 / MOR263-10/W5KGR-726917-725952; ORL2161 / MOR256-9/W5KGR-703347-702418; ORL2162 / MOR256-3/W5KGR-693647-692700; ORL2163 / MOR218-8/W5KGR-676482-675520; ORL2164 / MOR218-1/W5KGR-660767-659808; ORL2165 / MOR263-9/W5KGR-461862-462827; ORL2166 / MOR121-1/W5KGR-1281194-1280253; ORL2167 / MOR156-2/W5KGR-1273954-1274886; ORL2168 / MOR250-3/W5KGR-1143634-1144575; ORL2169 / MOR156-3/W5KGR-1086717-1085788; ORL2170 / MOR250-1/W5KGR-1046884-1045940; ORL2171 / MOR267-1/W4CLS-16353-15403; ORL2172 / MOR250-2/W5KGR-1180313-1181239; ORL2173 / MOR156-1/W5KGR-1226275-1227192; ORL2174 / MOR263-6/W5KGR-1295903-1296841; ORL2175 / MOR156-4/W5KGR-1242603-1243532; ORL2176 / MOR218-7/W5KGR-612161-611239; ORL2177 / MOR256-4/W5KGR-434304-435248; ORL2178 / MOR156-3/W5KGR-1086717-1085788; ORL2179 / MOR256-18/W5KGR-211794-212732; ORL2180 / MOR256-19/W5KGR-450259-449306; ORL2181 / MOR256-49/W5KGR-386358-387299; ORL2182 / MOR256-5/W5KGR-342798-341860; ORL2183 / MOR121-2P/W5KGR-1256057-1255391; ORL2184 / MOR256-6/W5KGR-369065-368127; ORL2185 / MOR256-48/W5KGR-310277-309339; ORL2186 / MOR250-4/W5KGR-1166473-1167400; ORL2187 / MOR263-7/W5KGR-800552-799585; ORL2188 / MOR256-39P/W5KGR-1355293-1356153; ORL2189 / MOR121-4P/W5KGR-1083645-1084433; ORL2190 / MOR256-40P/W5KGR-246292-245417; ORL2191 / MOR250-5/W5KGR-1130296-1129370; ORL2192 / MOR250-6/W5KGR-1097715-1096789; ORL2193 / MOR218-5P/W5KGR-563681-564305; ORL2194 / |

(continued)

| Name/Common Name |
|---|
| MOR121-3P/W5KGR-1229239-1228602; ORL2195 / MOR250-7/W5KGR-1109751-1108825; ORL2196 / MOR249-1 P; ORL2197 / MOR273-3P/W3JHV-27735-28674; ORL2198 / MOR272-1/W3JHV-78430-79368; ORL2199 / MOR271-1/W3JHV-63301-64239; ORL2200 / MOR270-1/W3JHV-107245-106307; ORL2201 / MOR239-3/W6KF8-6925606-6926538; ORL2202 / MOR239-1/W6KF8-6873911-6874846; ORL2203 / MOR239-5/W6KF8-6853437-6854381; ORL2204 / MOR214-3/W6KF8-6766043-6765105; ORL2205 / MOR215-1/W6KF8-6641850-6640903; ORL2206 / MOR202-9/W6KF8-6379117-6378179; ORL2207 / MOR202-4/W6KF8-6188025-6187066; ORL2208 / MOR202-7/W6KF8-6166309-6165365; ORL2209 / MOR202-2/W6KF8-6159605-6160531; ORL2210 / MOR202-3/W6KF8-6148505-6149434; ORL2211 / MOR202-5/W6KF8-6130006-6130950; ORL2212 / MOR202-34/W6KF8-6115572-6114628; ORL2213 / MOR202-1/W6KF8-6037877-6036945; ORL2214 / MOR202-6/W6KF8-6008524-6009447; ORL2215 / MOR202-14/W6KF8-5973096-5972173; ORL2216 / MOR202-17/W6KF8-5898047-5898970; ORL2217 / MOR202-35/W6KF8-5879692-5878754; ORL2218 / MOR202-13/W6KF8-5854350-5853427; ORL2219 / MOR202-10/W6KF8-5549080-5548133; ORL2220 / MOR202-37/W6KF8-5465976-5465029; ORL2221 / MOR202-18/W6KF8-5432445-5431498; ORL2222 / MOR202-19/W6KF8-5418545-5417604; ORL2223 / MOR266-8/W6KF8-5357748-5356750; ORL2224 / MOR266-1/W6KF8-5312987-5312040; ORL2225 / MOR212-1/W6KF8-5266830-5267774; ORL2226 / MOR212-3/W6KF8-5223686-5224633; ORL2227 / MOR212-2/W6KF8-5174638-5175585; ORL2228 / MOR214-5/W2EKY-6193-5255; ORL2229 / MOR266-2/W6KF8-7136208-7137197; ORL2230 / MOR211-1/W6KF8-5201028-5200078; ORL2231 / MOR202-8/W6KF8-6373215-6372277; ORL2232 / MOR202-11 /W6KF8-5640130-5639201; ORL2233 / MOR202-15/W6KF8-5687513-5686587; ORL2234 / MOR202-16/W6KF8-5596750-5597694; ORL2235 / MOR214-1/W6KF8-6793122-6794060; ORL2236 / MOR214-4/W6KF8-6747417-6746479; ORL2237 / MOR215-2/W6KF8-6613889-6612933; ORL2238 / MOR239-2/W6KF8-6902921-6903862; ORL2239 / MOR239-4/W6KF8-6863359-6864294; ORL2240 / MOR266-4/W6KF8-7069831-7068902; ORL2241 / MOR202-33/W6KF8-6096670-6095693; ORL2242 / MOR266-9/W6KF8-5294102-5293143; ORL2243 / MOR202-36/W6KF8-5571357-557230; ORL2244 / MOR127-1/W6KF8-5282181-5281234; ORL2245 / MOR211-2/W6KF8-5247224-5248168; ORL2246 / MOR266-3/W6KF8-5336116-5335166; ORL2247 / MOR214-2/W6KF8-6731438-6732376; ORL2248 / MOR202-29P/W6KF8-5815779-5814909; ORL2249 / MOR202-12/W6KF8-5709164-5708232; ORL2250 / MOR202-26P/W6KF8-5580664-5579730; ORL2251 / MOR214-8P/W2EKY-36933-37849; ORL2252 / MOR266-5/W6KF8-7109736-7110693; ORL2253 / MOR202-21/W6KF8-5677362-5676438; ORL2254 / MOR239-7/W6KF8-6888043-6888979; ORL2255 / MOR211-4P/W6KF8-5150306-5149315; ORL2256 / MOR202-23/W6KF8-6020754-6019830; ORL2257 / MOR202-24/W6KF8-5943834-5944758; ORL2258 / MOR214-6/W6KF8-6707784-6708723; ORL2259 / MOR215-4/W6KF8-6696578-6697538; ORL2260 / MOR266-6P/W6KF8-5396997-5396257; ORL2261 / MOR212-4P/W6KF8-5234278-5235119; ORL2262 / MOR214-7P/W6KF8-5234278-5235119; ORL2263 / MOR266-10/W6KF8-7094505-7095473; ORL2264 / MOR202-38/W6KF8-5605544-5605301; ORL2265 / MOR211-3P/W6KF8-5257145-5258009; ORL2266 / MOR202-25P/W6KF8-5587632-5588586; |

(continued)

| Name/Common Name |
| --- |
| ORL2267 / MOR202-28/W6KF8-5736341-5737266; ORL2268 / MOR202-30P/W6KF8-5615669-5616603; ORL2269 / MOR202-31P/W6KF8-5886511-5885392; ORL2270 / MOR241-4P/W6KF8-6795961-6796354; ORL2271 / MOR239-8P/W6KF8-6940434-6941231; ORL2272 / MOR133-4P/W6KF8-6740295-6740492; ORL2273 / MOR128-2/W3FEQ-6361-5417; ORL2274 / MOR216-1/W84RH-10784-9855; ORL2275 / MOR102-1/W4PMG-20510-19572; ORL2276 / MOR264-16P/W1L96-28487-27675; ORL2277 / MOR188-2/W4PJY-91768-92706; ORL2278 / MOR185-1 /W4PJY-12586-13542; ORL2279 / MOR228-4/W2BV0-3116-4045; ORL2280 / MOR156-5/W1TQM-1920-991; ORL2281 / MOR190-2/W1SGA-3329-2379; ORL2282 / MOR228-4/W1 PMD-3116-4045; ORL2283 / MOR184-1/W1L2S-17066-17992; ORL2284 / MOR248-9/TTDGA-1170-253; ORL2285 / MOR251-1/W1QYH-3213-2284; ORL2286 / MOR186-1/W4PJY-84384-85331; ORL2287 / MOR188-1 /W4PJY-21990-22931; ORL2288 / MOR186-2/W4PJY-75927-76868; ORL2289 / MOR183-4/TRVRT-922-14; ORL2290 / MOR182-6/W1S1K-1691-774; ORL2291 / MOR245-13/W1KA9-1464-2399; ORL2292 / MOR231-18/TT735-1291-335; ORL2293 / MOR202-20/TRE00-7947-6994; ORL2295 / MOR32-2/TR5Y9-1219-281; ORL2296 / MOR130-1/tYPL5-1098-157; ORL2297 / MOR259-7P/U1U80-3-796; ORL2298 / MOR213-1/U0S1H-46-969; ORL2299 / MOR211-8P/TWG15-383-970; ORL2300 / MOR162-11P/TV8D2-3166-3744; ORL2301 / MOR253-11 P/TT6U6-782-144; ORL2302 / MOR183-7P/TT1VY-417-1232; ORL2303 / MOR265-1/TSF3U-1080-1994; ORL2304 / MOR190-4P/TRSVL-493-17; ORL2305 / MOR192-2/TRSHY-1304-771; ORL2306 / MOR146-8P/TR2AE-715-5; ORL2307 / MOR246-2/TPYSW-56-1030; ORL2308 / MOR139-5P/TNKDU-1250-1574; ORL2309 / MOR282-1/TTVE7-114-990; ORL2310 / MOR253-10P/TT7QE-740-5V; ORL2311 / MOR253-12P/W1TUG-477-3; ORL2312 / MOR190-3P/W4PJY-571-1491; ORL2313 / MOR253-13P/TT5K3-1036-740; ORL2314 / MOR203-8P/TWNK3-27-896; ORL2315 / MOR231-20P/TT7VM-20-924; ORL2316 / MOR256-54P/TR6JS-3-632; ORL2317 / MOR256-54P/TQPTF-249-1138; ORL2318 / MOR267-17/TSFD3-906-4; ORL2319 / MOR245-14P/TTBE0-61-892; ORL2320 / MOR225-7P/TT538-107-843; ORL2321 / MOR150-2/TY9VC-3-581; ORL2322 / MOR23-2/TTTJT-3-875; ORL2323 / MOR263-8P/W27MS-3-218; ORL2324 / MOR110-8/W1MCC-10401-11346; ORL2325 / MOR114-10P/W1QQ1-1996-1409; ORL2326 / MOR256-42P/ TP834-1315-935; ORL2327 / MOR187-4/W4PJY-109732-110675; ORL2328 / MOR182-9/W2AC4-1116-198; ORL2329 / MOR267-12P/U01 VK-1062-153; ORL2330 / MOR202-32P/TSEV3-2-756; ORL2331 / MOR171-41 P/TSL54-727-5; ORL2332 / MOR189-5P/U1YFE-537-3; ORL2333 / MOR106-10/TYL6H-816-232; ORL2334 / MOR203-7P/TRSBM-251-3; ORL2335 / MOR259-9/TSUEL-2710-1814; ORL2336 / MOR137-2/W1UHS-2284-3222; ORL2337 / MOR182-11P/U0479-791-74; ORL2338 / MOR198-4/TTE3C-1209-237; ORL2339 / MOR135-5/U14GL-32-934; ORL2340 / MOR245-1/TUA6W-459-1397; ORL2341 / MOR15-1/TTR4D-1085-114; ORL2342 / MOR106-6/1N2D-18119-19054; ORL2343 / MOR150-1P/TY9VB-3-604; ORL2344 / MOR14-7P/TTTE8-736-3; ORL2345 / MOR203-5P/TRJAJ-14-661; ORL2346 / MOR40-7P/TVWE3-1144-1410; ORL2347 / MOR267-11/TQ6SJ-17-601; ORL2349 / MOR23-4P/TTTJS-1333-672; ORL2350 / MOR23-4P/TTTJU-544-4; ORL2351 / MOR135-24/TYQL0-1472-1005; ORL2352 / MOR256-44P/TSPWM-444-725; ORL2353 / MOR211-6P/U0959-397-888; ORL2354 / MOR211-7P/U14PG-1022-714; ORL2355 / |

(continued)

| Name/Common Name |
|---|
| MOR218-6P/TSNPN-494-44; ORL2356 / MOR188-7/-TT2EP-29-484; ORL2357 / MOR245-19P/TSJCG-676-4; ORL2358 / MOR0-14P/TSFPQ-475-5; ORL2359 / MOR0-15P/TS7JQ-953-1366; ORL2360 / MOR111-11/TNSDQ-9-470; ORL2361 / MOR193-1/TVVWN-32-970; ORL2362 / MOR204-33P/TVY84-268-1119; ORL2363 / MOR245-24P/TT1BL-547-1423; ORL2364 / MOR225-10P; ORL2365 / MOR231-15P; ORL2366 / MOR34-8P; ORL2367 / MOR34-9; ORL2368 / MOR185-8; ORL2369 / MOR105-9; ORL2370 / MOR256-46P; ORL2371 / MOR182-10P; ORL2372 / MOR0-16P; ORL2373 / MOR185-9P; ORL2374 / MOR231-19P; ORL2375 / MOR256-52P; ORL2376 / MOR220-4P; ORL2377 / MOR264-21 P; ORL2378 / MOR256-53P; ORL2379 / MOR15-3; ORL2380 / MOR184-11 P; ORL2381 / W6KF8-5488050-54886; ORL2382 / MOR202-27P/W6KF8-5768024-5768626; ORL2383 / W5KGR-1221410-122214; ORL2384 / W5KGR-1200441-120091; ORL2385 / W5KGR-1115818-111525; ORL2386 / W5KGR-1060859-106142; ORL2387 / TYMFE-883-533; ORL2388 / M25; ORL242 / K7; ORL245 / MOCLOR1; ORL246 / MOCLOR2; ORL248 / SAC36; ORL318 / C77344; ORL344 / OR27-3; ORL345 / OR55-36; ORL346 / OR6-13; ORL348 / AB004397; ORL398 / vr44a12.s1; ORL399 / vv74c09.r1; ORL425 / olfr89; ORL431 / 17; ORL432 / H12; ORL433 / H7; ORL434 / H6; ORL435 / H3; ORL436 / G7; ORL437 / G6; ORL438 / G3; ORL439 / F12; ORL440 / F6; ORL441 / F5; ORL442 / F3; ORL444 / E12; ORL445 / F7; ORL446 / E6; ORL447 / E3; ORL448 / D12; ORL449 / D7; ORL450 / D3; ORL451 / C6; ORL452 / C3; ORL453 / B12; ORL454 / B7; ORL455 / B6; ORL456 / B3; ORL457 / A7; ORL458 / A3; ORL461 / S1; ORL462 / S3; ORL463 / S6; ORL464 / S18; ORL465 / S19; ORL466 / S25; ORL467 / S41; ORL468 / S46; ORL469 / S50; ORL470 / S51; ORL471 / S79; ORL472 / S83; ORL473 / S85; ORL474 / S86; ORL475 / MOR5'beta3; ORL476 / MOR5'beta2; ORL477 / MOR5'beta1; ORL478 / MOR3'beta1; ORL479 / MOR3'beta2; ORL480 / MOR3'beta3; ORL503 /; ORL514 / Olfr17; ORL52 / OR3; ORL529 / Olfr69; ORL530 / Olfr68; ORL531 / Olfr67; ORL532 / Olfr66; ORL533 / Olfr65; ORL534 / Olfr4-3; ORL535 / Olfr41 ; ORL551 / K11; ORL552 / K12; ORL553 / K13; ORL554 / K14; ORL555 / K15; ORL556 / K18; ORL557 / K18h1; ORL558 / K18h2; ORL559 / K21; ORL560 / K21h1; ORL561 / K22; ORL562 / K23; ORL563 / K25; ORL564 / K26; ORL565 / K27; ORL566 / K30; ORL567 / K31; ORL568 / K4; ORL569 / K40; ORL570 / K41; ORL571 / K42; ORL572 / K4h10; ORL573 / K4h11; ORL574 / K4h12; ORL575 / K4h17; ORL576 / K4h19; ORL577 / K6; ORL578 / K8; ORL579 / K9; ORL580 / M15; ORL581 / M30; ORL582 / M32; ORL583 / M34; ORL584 / M36; ORL585 / M37; ORL586 / M71; ORL587 / M72; ORL588 / Olfr72; ORL595 / or37a; ORL596 / OR37B; ORL597 / or37c; ORL598 / or37d; ORL599 / or37e; ORL600 / OR17; ORL601 / or6; ORL606 /7008668; ORL607 /7008667; ORL608 /7008605; ORL609 /7008666; ORL610 /7008665; ORL611 /7008664; ORL612 /7008663; ORL613 /7008662; ORL614 /7008661; ORL615 /7008660; ORL616 /7008659; ORL617 /7008658; ORL618 /7008657; ORL619 /7008656; ORL620 /7008655; ORL621 /7008654; ORL622 /7008653; ORL623 /7008652; ORL624 /7008651; ORL625 /7008650; ORL626 /7008649; ORL627 /7008648; ORL628 /7008647; ORL629 /7008646; ORL630 /7008645; ORL631 /7008644; ORL632 /7008643; ORL633 /7008642; ORL634 /7008641; ORL635 /7008640; ORL636 /7008639; ORL637 /7008638; ORL638 /7008637; ORL639 /7008636; ORL640 /7008635; ORL641 /7008634; ORL642 /7008633; ORL643 /7008632; ORL644 /7008631; ORL645 /7008630; ORL646 /7008629; ORL647 /7008628; ORL649 /7008626; |

(continued)

| Name/Common Name |
|---|
| ORL650 /7008625; ORL651 /7008624; ORL652 /7008623; ORL653 /7008622; ORL654 /7008621; ORL655 /7008620; ORL656 /7008619; ORL657 /7008618; ORL658 /7008617; ORL659 /7008616; ORL660 /7008615; ORL661 /7008614; ORL662 /7008613; ORL663 /7008612; ORL664 /7008611; ORL665 /7008610; ORL666 /7008609; ORL668 /7008607; ORL669 /7008606; ORL670 / Olfr64; ORL695 / mab68c10.x1; ORL696 / s83; ORL698 / mOR11-2e; ORL699 / mOR11-2d(P); ORL700 / mOR11-2c; ORL701 / mOR11-2b(P); ORL702 / mOR11-7c(P); ORL703 / mOR11-7b; ORL704 / mOR11-7a; ORL7043 / Olfr212; ORL7044 / Olfr672; ORL7045 / Olfr672; ORL705 / mOR11-25; ORL706 / mOR11-40b; ORL708 / mOR11-208(P; ORL709 / mOR11-4; ORL730 / Olfr7b; ORL7548 / Olfr1407-ps1; ORL7549 / Olfr1405-ps1; ORL7550 / Olfr253-ps1; ORL7551 / Olfr1319-ps1; ORL7552 / Olfr1315-ps1; ORL7553 / Olfr1292-ps1; ORL7554 / Olfr1235-ps1; ORL7555 / Olfr1172-ps1; ORL7556 / Olfr1169-ps1; ORL7557 / Olfr1088-ps1; ORL7558 / Olfr1081-ps1; ORL7559 / Olfr1069-ps1; ORL7560 / Olfr1059-ps1; ORL7561 / Olfr1050-ps1; ORL7562 / Olfr1005-ps1; ORL7563 / Olfr526-ps1; ORL7564 / Olfr475-ps1; ORL7565 / Olfr1401-ps1; ORL7566 / Olfr454-ps1; ORL7567 / Olfr712-ps1; ORL7568 / Olfr696-ps1; ORL7569 / Olfr682-ps1; ORL7570 / Olfr662-ps1; ORL7571 / Olfr587-ps1; ORL7572 / Olfr581-ps1; ORL7573 / Olfr542-ps1; ORL7574 / Olfr528-ps1; ORL7575 / Olfr511-ps1; ORL7576 / Olfr499-ps11; ORL7577 / Olfr962-ps1; ORL7579 / Olfr946-ps1; ORL7580 / Olfr931-ps1; ORL7581 / Olfr929-ps1; ORL7582 / Olfr928-ps1; ORL7583 / Olfr879-ps1; ORL7584 / Olfr861-ps1; ORL7585 / Olfr848-ps1; ORL7586 / Olfr840-ps1; ORL7587 / Olfr233-ps1; ORL7588 / Olfr795-ps1; ORL7589 / Olfr778-ps1; ORL7590 / Olfr1379-ps1; ORL7591 / Olfr1400-ps1; ORL7592 / Olfr1369-ps1; ORL7593 / Olfr762-ps1; ORL7594 / Olfr760-ps1; ORL7595 / Olfr1503-ps1; ORL7596 / Olfr1482-ps1; ORL7597 / Olfr1456-ps1; ORL7598 / Olfr1455-ps1; ORL7599 / Olfr423-ps1; ORL7600 / Olfr343-ps1; ORL7601 / Olfr249-ps1; ORL7602 / Olfr364-ps1; ORL7603 / Olfr1117-ps1; ORL7604 / Olfr1127-ps1; ORL7605 / Olfr1025-ps1; ORL7606 / Olfr1027-ps1; ORL7607 / Olfr1185-ps1; ORL7608 / Olfr289-ps1; ORL7609 / Olfr269-ps1; ORL7610 / Olfr1332-ps1; ORL7611 / Olfr445-ps1; ORL7612 / Olfr439-ps1; ORL7613 / Olfr455-ps1; ORL7614 / Olfr296-ps1; ORL7615 / Olfr261-ps1; ORL7616 / Olfr260-ps1; ORL7617 / Olfr573-ps1; ORL7618 / Olfr537-ps1; ORL7619 / Olfr579-ps1; ORL7620 / Olfr621-ps1; ORL7621 / Olfr842-ps1; ORL7622 / Olfr863-ps1; ORL7623 / Olfr785-ps1; ORL7624 / Olfr789-ps1; ORL7625 / Olfr327-ps1; ORL7626 / Olfr326-ps1; ORL7627 / Olfr241-ps1; ORL7628 / Olfr277-ps1; ORL7629 / Olfr721-ps1; ORL7630 / Olfr240-ps1; ORL7630 / Olfr240-ps1; ORL7631 / Olfr188-ps1; ORL7632 / Olfr200-ps1; ORL7633 / Olfr189-ps1; ORL7634 / Olfr163-ps1; ORL7635 / Olfr1555-ps1; ORL7636 / Olfr1326-ps1; ORL7637 / Olfr1300-ps1; ORL7638 / Olfr1372-ps1; ORL7639 / Tas2r111-ps2; ORL7640 / Olfr1268-ps1; ORL7641 / Olfr1004-ps1; ORL7642 / Olfr1004-ps1; ORL7643 / Olfr1194-ps1; ORL7644 / Olfr1063-ps1; ORL7645 / Olfr1267-ps1; ORL7646 / Olfr29-ps1; ORL7647 / Olfr1334-ps1; ORL7648 / Olfr563-ps1; ORL7649 / Olfr548-ps1; ORL7650 / Olfr580-ps1; ORL7651 / Olfr650-ps1; ORL7652 / Olfr595-ps1; ORL7653 / Olfr674-ps1; ORL7654 / Olfr590-ps1; ORL7655 / Olfr709-ps1; ORL7656 / Olfr636-ps1; ORL7657 / Olfr565-ps1; ORL7658 / Olfr567-ps1; ORL7659 / Olfr647-ps1; ORL7660 / Olfr964-ps1; ORL7661 / Olfr949-ps1; ORL7662 / Olfr839-ps1; ORL7663 / Olfr22-ps1; ORL7664 / Olfr1397-ps1; |

(continued)

| Name/Common Name |
| --- |
| ORL7665 / Olfr737-ps1; ORL7666 / Olfr1498-ps1; ORL7667 / Olfr1468-ps1; ORL7668 / Olfr1452-ps1; ORL7669 / Olfr1473-ps1; ORL767 / gene_154; ORL7671 / Olfr1150-ps1; ORL7672 / Olfr1146-ps1; ORL7673 / Olfr1147-ps1; ORL7674 / Olfr1103-ps1; ORL7676 / Olfr1035-ps1; ORL7677 / Olfr999-ps1; ORL7678 / Olfr1224-ps1; ORL7679 / Olfr1159-ps1; ORL768 / gene_17; ORL7680 / Olfr990-ps1; ORL7681 / Olfr1142-ps1; ORL7682 / Olfr997-ps1; ORL7683 / Olfr1077-ps1; ORL7684 / Olfr1296-ps1; ORL7685 / Olfr1053-ps1; ORL7686 / Olfr1068-ps1; ORL7687 / Olfr1192-ps1; ORL7688 / Olfr1096-ps1; ORL7689 / Olfr1191-ps1; ORL769 / OR6-8; ORL7690 / Olfr718-ps1; ORL7691 / Olfr602-ps1; ORL7692 / Olfr841-ps1; ORL7693 / Olfr864-ps1; ORL7694 / Olfr858-ps1; ORL7695 / Olfr896-ps1; ORL7696 / Olfr911-ps1; ORL7697 / Olfr882-ps1; ORL7698 / Olfr886-ps1; ORL7699 / Olfr947-ps1; ORL770 / Olfr37e; ORL7700 / Olfr892-ps1; ORL7701 / Olfr977-ps1; ORL7702 / Olfr817-ps1; ORL7703 / Olfr1374-ps1; ORL7704 / Olfr387-ps1; ORL7705 / Olfr717-ps1; ORL7706 / Olfr754-ps1; ORL7707 / Olfr755-ps1; ORL7708 / Olfr752-ps1; ORL7709 / Olfr1464-ps1; ORL771 / Olfr70; ORL7710 / Olfr1460-ps1; ORL7711 / Olfr1438-ps1; ORL7712 / Olfr1435-ps1; ORL7713 / Olfr1430-ps1; ORL7714 / Olfr1429-ps1; ORL7715 / Tas2r145-ps3; ORL7716 / Olfr634-ps1; ORL7717 /; ORL7718 / Olfr396-ps1; ORL7719 / Olfr409-ps1; ORL772 / Olfr71; ORL7720 / Olfr408-ps1; ORL7721 / Olfr753-ps1; ORL7722 / Olfr1409-ps1; ORL7723 / GA_x5J8B7W531T-1241080-1240524; ORL7724 / GA_x5J8B7W62NC-992588-991951; ORL7725 / GA_x5J8B7W4T2P-95628-96096; ORL7726 / GA_x5J8B7W4CQV-18360-18784; ORL7727 / GA_x5J8B7W3KVV-42515-42818; ORL7728 / Olfr1021-ps1; ORL7729 / Olfr1253-ps1; ORL7730 / Olfr1304-ps1; ORL7731 / Olfr1266-ps1; ORL7732 / Olfr1237-ps1; ORL7733 / Olfr1244-ps1; ORL7734 / Olfr1236-ps1; ORL7735 / Olfr1227-ps1; ORL7736 / Olfr1190-ps1; ORL7737 / Olfr1187-ps1; ORL7738 / Olfr1398-ps1; ORL774 / Olfr49; ORL7740 / Olfr271-ps1; ORL7741 / Olfr1343-ps1; ORL7742 / Olfr588-ps1; ORL7743 / Olfr562-ps1; ORL7744 / Olfr1345-ps1; ORL7745 / GA_x5J8B7W60AJ-1667802-1666911; ORL7746 / Olfr897-ps1; ORL7747 / Olfr1375-ps1; ORL7748 / GA_x5J8B7W3UM0-1747565-1747342; ORL7749 / Olfr1376-ps1; ORL775 / D17Tu42; ORL7750 / Olfr1399-ps1; ORL7751 / Olfr465-ps1; ORL7752 / Olfr1363-ps1; ORL7753 / GA_x5J8B7W8B52-350793-350639; ORL7754 / GA_x5J8B7W6KF8-5605544-5605301; ORL7755 / Olfr1493-ps1; ORL7756 / Olfr1492-ps1; ORL7757 / Olfr1488-ps1; ORL7758 / Olfr1486-ps1; ORL7759 / Olfr1485-ps1; ORL776 / olfv1; ORL7760 / Olfr1483-ps1; ORL7761 / Olfr1481-ps1; ORL7762 / Olfr1479-ps1; ORL7763 / Olfr1478-ps1; ORL7764 / Olfr1476-ps1; ORL7765 / Olfr1421-ps1; ORL7766 / Olfr1327-ps1; ORL7767 / GA_x5J8B7W5KGR-1200441-1200915; ORL7768 / GA_x5J8B7W5FBR-2067722-2067518; ORL7769 / Olfr1210-ps1; ORL777 / olfv5; ORL7771 / Olfr431-ps1; ORL7772 / Olfr1144-ps1; ORL7773 / Olfr1139-ps1; ORL7774 / Olfr1114-ps1; ORL7775 / Olfr1108-ps1; ORL7776 / Olfr1092-ps1; ORL7777 / Olfr1091-ps1; ORL7778 / Olfr1078-ps1; ORL7779 / Olfr1075-ps1; ORL778 / olf18a; ORL7780 / Olfr1074-ps1; ORL7781 / Olfr1072-ps1; ORL7782 / Olfr1070-ps1; ORL7783 / Olfr1067-ps1; ORL7784 / Olfr1064-ps1; ORL7785 / Olfr1041-ps1; ORL7786 / Olfr1017-ps1; ORL7787 / Olfr1007-ps1; ORL7788 / Olfr1003-ps1; ORL7789 / Olfr991-ps1; ORL779 / OR10M; ORL7790 / Olfr989-ps1; ORL7791 / Olfr442-ps1; ORL7792 / Olfr436-ps1; ORL7793 / Olfr673-ps1 ; ORL7794 / Olfr596-ps1; ORL7795 / Olfr546-ps1; ORL7796 / Olfr540-ps1; |

(continued)

| Name/Common Name |
|---|
| ORL7797 / Olfr534-ps1; ORL7798 / Olfr529-ps1; ORL7799 / Olfr515-ps1; ORL780 / OR1-72M16; ORL7800 / Olfr468-ps1; ORL7801 / Olfr953-ps1; ORL7802 / Olfr950-ps1; ORL7803 / Olfr942-ps1; ORL7804 / Olfr940-ps1; ORL7805 / Olfr939-ps1; ORL7806 / Olfr932-ps1; ORL7807 / Olfr927-ps1; ORL7808 / Olfr903-ps1; ORL7809 / Olfr880-ps1; ORL781 / OR1-72M15; ORL7810 / Olfr865-ps1; ORL7811 / Olfr852-ps1; ORL7812 / Olfr838-ps1; ORL7813 / Olfr833-ps1; ORL7814 / Olfr831-ps1; ORL7815 / Olfr797-ps1; ORL7816 / Olfr793-ps1; ORL7817 / Olfr758-ps1; ORL7818 / Olfr757-ps1; ORL7819 / Olfr756-ps1; ORL782 / Or912-93; ORL7820 / Olfr751-ps1; ORL7821 / Olfr428-ps1; ORL7822 / Olfr422-ps1; ORL7823 / Olfr416-ps1; ORL7824 / Olfr236-ps1; ORL7825 / Olfr258-ps1; ORL7826 / Olfr413-ps1; ORL7827 / Olfr1521-ps1; ORL7828 / Olfr274-ps1; ORL7829 / Olfr219-ps1; ORL783 / OR7M; ORL7830 / Olfr1524-ps1; ORL7831 / Olfr237-ps1; ORL7832 / Olfr306-ps1; ORL7833 / Olfr626-ps1; ORL7834 / Olfr217-ps1; ORL7835 / Olfr1523-ps1; ORL7836 / Olfr625-ps1; ORL7837 / Olfr505-ps1; ORL7838 / Olfr500-ps1; ORL7839 / Olfr489-ps1; ORL784 / OR6M; ORL7840 / Olfr1525-ps1; ORL7841 / Olfr637-ps1; ORL7842 / Olfr369-ps1; ORL7843 / Olfr856-ps1; ORL7844 / Olfr1520-ps1; ORL7845 / Olfr1522-ps1; ORL7846 / Olfr254-ps1; ORL7847 / Olfr322-ps1; ORL7848 / Olfr377-ps1; ORL7849 / Olfr383-ps1; ORL785 / OR5M; ORL7850 / Olfr226-ps1; ORL7851 / Olfr388-ps1; ORL7852 / Olfr743-ps1; ORL7853 / Olfr280-ps1; ORL7854 / Olfr278-ps1; ORL7855 / Olfr185-ps1; ORL7856 / Olfr182-ps1; ORL7857 / Olfr246-ps1; ORL7858 / Olfr1561-ps1; ORL7859 / Olfr1526-ps1; ORL786 / OR4M; ORL7860 / Olfr1527-ps1; ORL7861 / Olfr1532-ps1; ORL7862 / Olfr1560-ps1; ORL7863 / Olfr1542-ps1; ORL7864 / Olfr1544-ps1; ORL7865 / Olfr1556-ps1; ORL7866 / Olfr1562-ps1; ORL7867 / Olfr1563-ps1; ORL7868 / Olfr1530-ps1; ORL7869 / Olfr1149-ps1; ORL787 / OR3M; ORL7870 / Olfr336-ps1; ORL7871 / Olfr337-ps1; ORL7872 / Olfr359-ps1; ORL7873 / Olfr1533-ps1; ORL7874 / Olfr1534-ps1; ORL7875 / Olfr1001-ps1; ORL7876 / Olfr1536-ps1; ORL7877 / Olfr1541-ps1; ORL7878 / Olfr268-ps1; ORL7879 / Olfr1119-ps1; ORL788 / OR2M; ORL7880 / Olfr349-ps1; ORL7881 / Olfr334-ps1; ORL7882 / Olfr1073-ps1; ORL7883 / Olfr1546-ps1; ORL7884 / Olfr1547-ps1; ORL7885 / Olfr440-ps1; ORL7886 / Olfr443-ps1; ORL7887 / Olfr451-ps1; ORL7888 / Olfr1531-ps1; ORL7889 / Olfr300-ps1; ORL789 / OR29M; ORL7890 / Olfr501-ps1; ORL7891 / Olfr496-ps1; ORL7892 / Olfr1538-ps1; ORL7893 / Olfr680-ps1; ORL7894 / Olfr1537-ps1; ORL7895 / Olfr941-ps1; ORL7896 / Olfr925-ps1; ORL7897 / Olfr1543-ps1; ORL7898 / Olfr966-ps1; ORL7899 / Olfr1545-ps1; ORL790 / OR28M; ORL7900 / Olfr956-ps1; ORL7901 / Olfr783-ps1; ORL7902 / Olfr405-ps1; ORL7903 / Olfr407-ps1; ORL7904 / Olfr321-ps1; ORL7905 / Olfr333-ps1; ORL7906 / Olfr1549-ps1; ORL7907 / Olfr263-ps1; ORL7908 / Olfr162-ps1; ORL7909 / Olfr1539-ps1; ORL791 / OR27M; ORL7910 / Olfr175-ps1; ORL7911 / Olfr1540-ps1; ORL7912 / Olfr184-ps1; ORL7913 / Olfr759-ps1; ORL7914 / Olfr1470-ps1; ORL7915 / Olfr1529-ps1; ORL7916 / Olfr1439-ps1; ORL7917 / Olfr1422-ps1; ORL7919 / Olfr418-ps1; ORL792 / OR25M; ORL7920 / GA_x5J8B7W3B3M-30314-31216; ORL7921 / GA_x5J8B7W3B3M-318013-318414; ORL7922 / Olfr1171-ps1; ORL7923 / GA_x5J8B7TRQ4E-1174-939; ORL7924 / GA_x5J8B7TTH1T-1160-261; ORL7925 / GA_x5J8B7TUS7D-1-861; ORL7926 / GA_x5J8B7TWLR0-1874-2347; ORL7927 / GA_x5J8B7TY7N2-4-429; ORL7928 / GA_x5J8B7W2GLP-600-794; ORL7929 / GA x5J8B7W3KVV-170014-170572; ORL793 / OR22M; |

(continued)

| Name/Common Name |
|---|
| ORL7930 / GA_x5J8B7W3KVV-25848-25992; ORL7931 / GA_x5J8B7W3KVV-518538-519236; ORL7932 / GA_x5J8B7W3P0G-705890-705633; ORL7933 / GA_x5J8B7W3Y5M-1033291-1033773; ORL7934 / GA_x5J8B7W3Y5M-1094074-1093275; ORL7935 / GA_x5J8B7W3Y5M-123838-124302; ORL7936 / GA_x5J8B7W3Y5M-419033-418135; ORL7937 / Olfr375-ps1; ORL7938 / GA_x5J8B7W3BSW-40181-41086; ORL7939 / Olfr210-ps1; ORL794 / OR21 M; ORL7940 / Olfr1553-ps1; ORL7941 / GA_x5J8B7TS19L-6-604; ORL7942 / GA_x5J8B7U22CP-453-1035; ORL7943 / GA_x5J8B7W3939-136239-135295; ORL7944 / GA_x5J8B7W3DQU-679632-679375; ORL7945 / GA_x5J8B7W3EKE-4522784-4521912; ORL7946 / GA_x5J8B7W3EKE-4574231-4574913; ORL7947 / GA_x5J8B7W3SPQ-59383-59030; ORL7948 / GA_x5J8B7W3SPQ-80592-80214; ORL7949 / Olfr1550-ps1; ORL795 / OR1 M; ORL7950 / GA_x5J8B7W3M6R-403436-402959; ORL7951 / GA_x5J8B7TPDN2-1058-1265; ORL7952 / Olfr379-ps1; ORL7953 / Olfr404-ps1; ORL7954 / Olfr400-ps1; ORL7955 / GA_x5J8B7TT7LR-4113-3651; ORL7956 / GA_x5J8B7U1 E65-1-838; ORL7957 / GA_x5J8B7W31 JB-148149-147210; ORL7958 / GA_x5J8B7W3UM0-970645-971221; ORL7959 / GA_x5J8B7TWUYY-328-3; ORL796 / OR18M; ORL7960 / GA_x5J8B7W27DS-1519-2216; ORL7961 / GA_x5J8B7U07Q4-325-757; ORL7962 / Olfr1552-ps1; ORL7963 / Olfr1558-ps1; ORL7964 / Olfr252-ps1; ORL7965 / Olfr1551-ps1; ORL7966 / GA_x5J8B7TSKU6-615-137; ORL7967 / GA_x5J8B7W3B3M-69696-70097; ORL7968 / GA_x5J8B7W3BSW-105992-106801; ORL7969 / GA_x5J8B7W5Q1 F-344462-343889; ORL797 / OR15-71 M24; ORL7970 / GA_x5J8B7W5Q1 F-395172-396104; ORL7971 / GA_x5J8B7W3Y5M-51270-50422; ORL7972 / GA_x5J8B7W4CQV-104617-103730; ORL7973 / GA_x5J8B7W4CQV-107796-107307; ORL7974 / GA_x5J8B7W4CQV-78329-78805; ORL7975 / GA_x5J8B7W4NS2-369154-369459; ORL7976 / GA_x5J8B7W4NS2-380558-381346; ORL7977 / GA_x5J8B7W4NS2-96830-97153; ORL7978 / GA_x5J8B7W4QPD-3054974-3054685; ORL7979 / GA_x5J8B7W531T-1286425-1285652; ORL798 / OR15-71M21; ORL7980 / GA_x5J8B7W531T-1305505-1304608; ORL7981 / GA_x5J8B7W531T-1558688-1559570; ORL7982 / GA_x5J8B7W531T-1577060-1577325; ORL7983 / GA_x5J8B7W531T-1588603-1588734; ORL7984 / GA_x5J8B7W62NC-1313389-1313225; ORL7985 / GA_x5J8B7W62NC-658712-658455; ORL7986 / GA_x5J8B7W62NC-668773-668438; ORL7987 / GA_x5J8B7W62NC-805000-804277; ORL7988 / GA_x5J8B7W62NC-67655-66851; ORL7989 / GA_x5J8B7W66A6-1043662-1044210; ORL799 / OR15-71M20; ORL7990 / GA_x5J8B7W4TKW-7041228-7041658; ORL7991 / GA_x5J8B7W5BNN-892919-892147; ORL7992 / GA_x5J8B7W6337-1183223-1183810; ORL7993 / GA_x5J8B7W6337-1221999-1222591; ORL7994 / GA_x5J8B7W6337-1270568-1271367; ORL7995 / GA_x5J8B7W6337-801345-802245; ORL7996 / GA_x5J8B7W5M25-134931-135139; ORL7997 / GA_x5J8B7W5M25-282037-281897; ORL7998 / GA_x5J8B7W5P47-202028-201789; ORL7999 / GA_x5J8B7W5Q32-585040-584392; ORL800 / OR15-71 M19; ORL8000 / GA_x5J8B7W6B6J-1522840-1521873; ORL8001 / GA_x5J8B7W60AJ-354484-355428; ORL8002 / GA_x5J8B7W60AJ-4787-4010; ORL8003 / GA_x5J8B7W60AJ-540286-540740; ORL8004 / GA_x5J8B7W60AJ-666029-666595; ORL8005 / GA_x5J8B7W60AJ-703922-704828; ORL8006 / GA_x5J8B7W60AJ-719102-719883; ORL8007 / GA_x5J8B7W60AJ-925630-926226; |

(continued)

| Name/Common Name |
|---|
| ORL8008 / GA_x5J8B7W54VK-284379-285078; ORL8009 / GA_x5J8B7W4W3R-189177-188236; ORL801 / OR12M; ORL8010 / GA_x5J8B7W5WBF-6267395-6266441; ORL8011 / GA_x5J8B7W5KGR-1060859-1061426; ORL8012 / GA_x5J8B7W5KGR-1115818-1115257; ORL8013 / GA_x5J8B7W5KGR-1221410-1222149; ORL8014 / GA_x5J8B7W4CQV-140710-140907; ORL8015 / GA_x5J8B7W4T2P-416420-416268; ORL8016 / GA_x5J8B7W5P47-694928-693967; ORL8017 / GA_x5J8B7W62NC-653162-652995; ORL8018 / GA_x5J8B7W6337-1014614-1015483; ORL802 / OR11 M; ORL8020 / Olfr1405-ps1; ORL8021 / GA_x5J8B7W3B3M-312879-313274; ORL8022 / Olfr428-ps1; ORL8023 / Olfr423-ps1; ORL8024 / Olfr422-ps1; ORL8025 / Olfr413-ps1; ORL8027 / GA_x5J8B7W4PJY-571-1491; ORL8028 / GA_x5J8B7W3Y5M-121303-120726; ORL8029 / GA_x5J8B7W4NS2-37772-38277; ORL8030 / Olfr1319-ps1; ORL8031 / Olfr1315-ps1; ORL8032 / Olfr1304-ps1; ORL8033 / Olfr1300-ps1; ORL8034 / Olfr1296-ps1; ORL8035 / Olfr1292-ps1; ORL8036 / Olfr1268-ps1; ORL8037 / Olfr1267-ps1; ORL8038 / Olfr1266-ps1; ORL8039 / Olfr1253-ps1; ORL804 / OR8M; ORL8040 / Olfr1244-ps1; ORL8041 / Olfr1235-ps1; ORL8042 / Olfr1224-ps1; ORL8043 / Olfr1194-ps1; ORL8044 / Olfr1192-ps1; ORL8045 / Olfr1191-ps1; ORL8046 / Olfr1187-ps1; ORL8047 / Olfr1185-ps1; ORL8048 / Olfr1172-ps1; ORL8049 / Olfr1169-ps1; ORL805 / OR912-47M4; ORL8050 / Olfr1159-ps1; ORL8051 / Olfr1150-ps1; ORL8052 / Olfr1147-ps1; ORL8053 / Olfr1146-ps1; ORL8054 / Olfr1142-ps1; ORL8055 / Olfr1127-ps1; ORL8057 / Olfr1117-ps1; ORL8058 / Olfr1103-ps1; ORL8059 / Olfr1096-ps1; ORL806 / OR912-47M6; ORL8060 / Olfr1091-ps1; ORL8061 / Olfr1081-ps1; ORL8062 / Olfr1078-ps1; ORL8063 / Olfr1077-ps1; ORL8064 / Olfr1075-ps1; ORL8065 / Olfr1068-ps1; ORL8066 / Olfr1067-ps1; ORL8067 / Olfr1063-ps1; ORL8068 / Olfr1053-ps1; ORL8069 / Olfr1050-ps1; ORL807 / OR912-47M7; ORL8070 / Olfr1035-ps1; ORL8071 / Olfr1027-ps1; ORL8072 / Olfr1025-ps1; ORL8073 / Olfr1021-ps1; ORL8074 / Olfr1007-ps1; ORL8075 / Olfr1005-ps1; ORL8076 / Olfr1004-ps1; ORL8077 / Olfr1001-ps1; ORL8078 / Olfr999-ps1; ORL8079 / Olfr997-ps1; ORL808 / OR912-47M8; ORL8080 / Olfr990-ps1; ORL8081 / Olfr364-ps1; ORL8082 / Olfr337-ps1; ORL8083 / Olfr336-ps1; ORL8084 / Olfr1398-ps1; ORL8085 / Olfr289-ps1; ORL8086 / GA_x5J8B7TQG34-443-1109; ORL8087 / GA_x5J8B7TQTH0-2-774; ORL8088 / Olfr274-ps1; ORL8089 / Olfr271-ps1; ORL809 / OR9M; ORL8090 / Olfr29-ps1; ORL8091 / Olfr1401-ps1; ORL8092 / GA_x5J8B7W5D7G-2505139-2505348; ORL8093 / Olfr718-ps1; ORL8094 / GA_x5J8B7W6GG0-4757855-4757501; ORL8095 / Olfr455-ps1; ORL8096 / Olfr451-ps1; ORL8097 / Olfr443-ps1; ORL8098 / Olfr440-ps1; ORL8099 / Olfr210-ps1; ORL810 / OR1-72M13; ORL8100 / GA_x5J8B7W6B6J-189947-189022; ORL8101 / GA_x5J8B7W6B6J-2580381-2580079; ORL8102 / GA_x5J8B7W72BC-125691-125311; ORL8103 / GA_x5J8B7W72BC-152913-152037; ORL8104 / GA_x5J8B7W72BC-33522-34417; ORL8105 / GA_x5J8B7W89HK-6403592-6404251; ORL8106 / GA_x5J8B7TU1 BB-380-666; ORL8107 / GA_x5J8B7W5P47-379997-380552; ORL8108 / GA_x5J8B7W6B6J-1784824-1783474; ORL8109 / Olfr712-ps1; ORL811 / vr44a12.x1; ORL8110 / Olfr709-ps1; ORL8111 / Olfr696-ps1; ORL8112 / Olfr674-ps1; ORL8113 / Olfr662-ps1; ORL8114 / Olfr650-ps1; ORL8115 / Olfr647-ps1; ORL8116 / Olfr636-ps1; ORL8117 / Olfr634-ps1; ORL8118 / Olfr621-ps1; ORL8119 / Olfr602-ps1; ORL812 / OR17; ORL8120 / Olfr596-ps1; ORL8121 / Olfr595-ps1; |

(continued)

| Name/Common Name |
|---|
| ORL8122 / Olfr590-ps1; ORL8123 / Olfr588-ps1; ORL8124 / Olfr587-ps1; ORL8125 / Olfr581-ps1; ORL8126 / Olfr580-ps1; ORL8127 / Olfr579-ps1; ORL8128 / Olfr573-ps1; ORL8129 / Olfr567-ps1; ORL813 / OR6; ORL8130 / Olfr565-ps1; ORL8131 / Olfr563-ps1; ORL8132 / Olfr562-ps1; ORL8133 / Olfr548-ps1; ORL8134 / Olfr515-ps1; ORL8135 / Olfr511-ps1; ORL8136 / Olfr505-ps1; ORL8137 / Olfr501-ps1; ORL8138 / Olfr496-ps1; ORL8139 / Olfr489-ps1; ORL814 / Olfr15; ORL8140 / Olfr306-ps1; ORL8141 / Olfr300-ps1; ORL8142 / Olfr261-ps1; ORL8143 / GA_x5J8B7W6RL5-12878266-12878409; ORL8144 / GA_x5J8B7W6RL5-13451244-13452122; ORL8145 / GA_x5J8B7W6RL5-13645512-13646298; ORL8146 / GA_x5J8B7W6RL5-13695863-13696754; ORL8147 / GA_x5J8B7W6RL5-13870817-13871726; ORL8148 / GA_x5J8B7TR73U-24-691; ORL8149 / GA_x5J8B7W60AJ-261378-260429; ORL815 / Olfr16; ORL8150 / GA_x5J8B7W60AJ-480395-479394; ORL8151 / GA_x5J8B7W60AJ-716549-716307; ORL8152 / GA_x5J8B7W6RL5-13004417-13003354; ORL8153 / Olfr977-ps1; ORL8154 / Olfr964-ps1; ORL8155 / Olfr953-ps1; ORL8156 / Olfr950-ps1; ORL8157 / Olfr949-ps1; ORL8158 / Olfr947-ps1; ORL8159 / Olfr942-ps1; ORL816 / Olfr4-3; ORL8160 / Olfr940-ps1; ORL8161 / Olfr931-ps1; ORL8162 / Olfr928-ps1; ORL8163 / Olfr927-ps1; ORL8164 / Olfr911-ps1; ORL8165 / Olfr896-ps1; ORL8166 / Olfr892-ps1; ORL8167 / Olfr886-ps1; ORL8168 / Olfr882-ps1; ORL8169 / Olfr865-ps1; ORL817 / OR912-47M9; ORL8170 / Olfr864-ps1; ORL8171 / Olfr858-ps1; ORL8172 / Olfr848-ps1; ORL8173 / Olfr842-ps1; ORL8174 / Olfr841-ps1; ORL8175 / Olfr840-ps1; ORL8176 / Olfr839-ps1; ORL8177 / Olfr831-ps1; ORL8178 / GA_x5J8B7W6HFP-3198834-3199739; ORL8179 / GA_x5J8B7W6HFP-3231749-3230798; ORL818 / Olfr17; ORL8180 / GA_x5J8B7TNKDU-1250-1574; ORL8181 / GA_x5J8B7W6HFP-3168293-3169194; ORL8182 / Olfr817-ps1; ORL8183 / Olfr789-ps1; ORL8184 / Olfr785-ps1; ORL8185 / Olfr773-ps1; ORL8186 / Olfr1397-ps1; ORL8187 / Olfr1379-ps1; ORL8188 / Olfr1375-ps1; ORL8189 / Olfr1374-ps1; ORL819 / Olfr37a; ORL8190 / Olfr1372-ps1; ORL8191 / Olfr409-ps1; ORL8192 / Olfr408-ps1; ORL8193 / Olfr407-ps1; ORL8194 / Olfr405-ps1; ORL8195 / Olfr404-ps1; ORL8196 / Olfr400-ps1; ORL8197 / Olfr22-ps1; ORL8198 / Olfr396-ps1; ORL8199 / Olfr388-ps1; ORL820 / Olfr37b; ORL8200 / Olfr387-ps1; ORL8201 / Olfr383-ps1; ORL8202 / Olfr379-ps1; ORL8203 / Olfr75-ps1; ORL8204 / Olfr377-ps1; ORL8205 / Olfr327-ps1; ORL8206 / Olfr322-ps1; ORL8207 / Olfr321-ps1; ORL8208 / Olfr1399-ps1; ORL8209 / Olfr1369-ps1; ORL821 / Olfr37c; ORL8210 / Olfr1363-ps1; ORL8211 / GA_x5J8B7W36P1-1107539-1107771; ORL8212 / Olfr717-ps1; ORL8213 / Olfr465-ps1; ORL8214 / GA_x5J8B7TYL6J-3-280; ORL8215 / Olfr743-ps1; ORL8216 / Olfr737-ps1; ORL8217 / GA_x5J8B7W76D0-7895713-7896429; ORL8218 / Olfr278-ps1; ORL8219 / GA_x5J8B7W8B52-298374-299143; ORL8220 / GA_x5J8B7U0478-6-340; ORL8221 / Olfr200-ps1; ORL8222 / Olfr185-ps1; ORL8223 / Olfr184-ps1; ORL8224 / Olfr175-ps1; ORL8225 / Olfr163-ps1; ORL8226 / GA_x5J8B7W5KGR-202501-203873; ORL8227 / Olfr762-ps1; ORL8228 / Olfr758-ps1; ORL8229 / Olfr757-ps1; ORL8230 / Olfr755-ps1; ORL8231 / Olfr754-ps1; ORL8232 / Olfr753-ps1; ORL8233 / Olfr752-ps1; ORL8235 / Olfr1503-ps1; ORL8236 / Olfr1498-ps1; ORL8237 / Olfr1493-ps1; ORL8238 / Olfr1492-ps1; ORL8239 / Olfr1481-ps1; ORL824 / Olfr66; ORL8240 / Olfr1473-ps1; ORL8241 / Olfr1468-ps1; ORL8242 / Olfr1464-ps1; ORL8243 / Olfr1460-ps1; ORL8244 / Olfr1456-ps1; ORL8245 / |

(continued)

| Name/Common Name |
| --- |
| Olfr1455-ps1; ORL8246 / Olfr1452-ps1; ORL8247 / Olfr1438-ps1; ORL8248 / Olfr1435-ps1; ORL8249 / Olfr1430-ps1; ORL8250 / Olfr1429-ps1; ORL8251 / GA_x5J8B7W6KF8-5344390-5343561; ORL8252 / GA_x5J8B7W6KF8-5425983-5425664; ORL8253 / GA_x5J8B7W6KF8-5463415-5462623; ORL8254 / GA_x5J8B7W6KF8-5484703-5484290; ORL8255 / GA_x5J8B7W6KF8-5488050-5488655; ORL8256 / GA_x5J8B7W6KF8-5515896-5515561; ORL8257 / GA_x5J8B7W6KF8-5539992-5539494; ORL8258 / GA_x5J8B7W6KF8-5546505-5545716; ORL8259 / GA_x5J8B7W6KF8-5563315-5562883; ORL8260 / GA_x5J8B7W6KF8-5957071-5957489; ORL8261 / GA_x5J8B7W6KF8-6942124-6941700; ORL8262 / Olfr1326-ps1; ORL8263 / Olfr1334-ps1; ORL8264 / GA_x5J8B7TR6JS-3-632; ORL8265 / GA_x5J8B7W1Q6G-2523-3443; ORL8266 / Olfr1332-ps1; ORL8267 / Olfr526-ps1; ORL8268 / Olfr475-ps1; ORL8269 / Olfr268-ps1; ORL8270 / Olfr263-ps1; ORL8271 / Olfr258-ps1; ORL8272 / Olfr254-ps1; ORL8273 / Olfr252-ps1; ORL8274 / Olfr249-ps1; ORL8275 / Olfr246-ps1; ORL8276 / Olfr237-ps1; ORL8277 / Olfr236-ps1; ORL8278 / Olfr233-ps1; ORL8279 / Olfr226-ps1; ORL828 / mOR-EG; ORL8280 / Olfr219-ps1; ORL8281 / Olfr431-ps1; ORL8282 / Olfr418-ps1; ORL8283 / Olfr416-ps1; ORL8284 / Olfr1409-ps1; ORL8285 / Olfr1407-ps1; ORL8287 / Olfr359-ps1; ORL8288 / Olfr349-ps1; ORL8289 / Olfr343-ps1; ORL829 / mOR-EV; ORL8290 / Olfr334-ps1; ORL8291 / Olfr1237-ps1; ORL8292 / Olfr1236-ps1; ORL8293 / Olfr1227-ps1; ORL8294 / Olfr1210-ps1; ORL8295 / Olfr1190-ps1; ORL8296 / Olfr1171-ps1; ORL8297 / Olfr1149-ps1; ORL8298 / Olfr1144-ps1; ORL8299 / Olfr1139-ps1; ORL8300 / Olfr1119-ps1; ORL8301 / Olfr1114-ps1; ORL8302 / Olfr1108-ps1; ORL8303 / Olfr1092-ps1; ORL8304 / Olfr1088-ps1; ORL8305 / Olfr1074-ps1; ORL8306 / Olfr1073-ps1; ORL8307 / Olfr1072-ps1; ORL8308 / Olfr1070-ps1; ORL8309 / Olfr1069-ps1; ORL831 / S85; ORL8310 / Olfr1064-ps1; ORL8311 / Olfr1059-ps1; ORL8312 / Olfr1041-ps1; ORL8313 / Olfr1017-ps1; ORL8314 / Olfr1003-ps1; ORL8315 / Olfr991-ps1; ORL8316 / Olfr989-ps1; ORL8317 / Olfr375-ps1; ORL8319 / Olfr369-ps1; ORL8320 / Olfr269-ps1; ORL8321 / Olfr454-ps1; ORL8322 / Olfr445-ps1; ORL8323 / Olfr442-ps1; ORL8324 / Olfr439-ps1; ORL8325 / Olfr436-ps1; ORL8326 / Olfr542-ps1; ORL8327 / Olfr540-ps1; ORL8328 / Olfr537-ps1; ORL8329 / Olfr534-ps1; ORL8330 / Olfr529-ps1; ORL8331 / Olfr528-ps1; ORL8332 / Olfr500-ps1; ORL8333 / Olfr499-ps1; ORL8334 / Olfr468-ps1; ORL8335 / Olfr682-ps1; ORL8336 / Olfr680-ps1; ORL8337 / Olfr673-ps1; ORL8338 / Olfr637-ps1; ORL8339 / Olfr626-ps1; ORL834 / ETL1; ORL8340 / Olfr625-ps1; ORL8341 / Olfr546-ps1; ORL8342 / Olfr296-ps1; ORL8343 / Olfr260-ps1; ORL8344 / Olfr966-ps1; ORL8345 / Olfr962-ps1; ORL8346 / Olfr956-ps1; ORL8347 / Olfr946-ps1; ORL8348 / Olfr941-ps1; ORL8349 / Olfr939-ps1; ORL835 / r35; ORL8350 / Olfr932-ps1; ORL8351 / Olfr929-ps1; ORL8352 / Olfr925-ps1; ORL8353 / Olfr903-ps1; ORL8354 / Olfr897-ps1; ORL8355 / Olfr880-ps1; ORL8356 / Olfr879-ps1; ORL8357 / Olfr863-ps1; ORL8358 / Olfr861-ps1; ORL8359 / Olfr856-ps1; ORL836 / r35; ORL8360 / Olfr852-ps1; ORL8361 / Olfr838-ps1; ORL8362 / Olfr833-ps1; ORL8363 / Olfr797-ps1; ORL8364 / Olfr795-ps1; ORL8365 / Olfr793-ps1; ORL8366 / Olfr783-ps1; ORL8367 / Olfr778-ps1; ORL8368 / Olfr391-ps1; ORL8369 / Olfr333-ps1; ORL837 / CELSR3; ORL8370 / Olfr326-ps1; ORL8371 / Olfr1376-ps1; ORL8372 / Olfr1400-ps1; ORL8373 / Olfr721-ps1; ORL8374 / Olfr277-ps1; ORL8375 / Olfr280-ps1; ORL8376 / Olfr189-ps1; ORL8377 / Olfr188-ps1; ORL8378 / |

(continued)

| Name/Common Name |
| --- |
| Olfr182-ps1; ORL8379 / Olfr162-ps1; ORL838 / 573K1.2; ORL8380 / Olfr760-ps1; ORL8381 / Olfr759-ps1; ORL8382 / Olfr756-ps1; ORL8383 / Olfr1488-ps1; ORL8384 / Olfr1486-ps1; ORL8385 / Olfr1485-ps1; ORL8386 / Olfr1483-ps1; ORL8387 / Olfr1482-ps1; ORL8388 / Olfr1479-ps1; ORL8389 / Olfr1478-ps1; ORL839 / 573K1.3; ORL8390 / Olfr1476-ps1; ORL8391 / Olfr1470-ps1; ORL8392 /Olfr1439-ps1; ORL8393 / Olfr1422-ps1; ORL8394 / Olfr1421-ps1; ORL8395 /Olfr1327-ps1; ORL8396 / Olfr751-ps1; ORL8397 / Olfr253-ps1; ORL8398 / Olfr241-ps1; ORL8399 / Olfr240-ps1; ORL840 / 573K1.4; ORL8400 / Olfr230-ps1; ORL8401 / Olfr217-ps1; ORL8402 / Olfr1345-ps1; ORL8403 / Olfr1343-ps1; ORL841 / 573K1.8; ORL842 / 573K1.10; ORL843 / 573K1.15; ORL844 / AAA93354.1; ORL845 / Ors16; ORL846 / Ora16; ORL847 / MOR83; ORL848 / MOR10; ORL849 / MOR28; ORL850 / mM31 m; ORL851 / mK7m; ORL853 / M12; ORL854 / A16; ORL855 / MOR18; ORL856 / dM538M10.5; ORL857 / 7E3/m50; ORL858 / 7E3/B4; ORL859 / dM538M10.6; ORL860 / dM538M10.7; ORL861 / dM538M10.8; ORL862 / dM538M10.1; ORL863 / dM538M10.3; ORL864 / dM538M10.4; ORL865 / dM538M10.2; ORL866 / B3; ORL867 / B5; ORL868 / B6; ORL876 / B2; ORL877 / P4; ORL878 / P2; ORL879 / P3; ORL880 / 17; ORL8805 /; ORL8807 /; ORL8808 /; ORL8809 /; ORL881 / m50; ORL8810 / Olfr627; ORL8811 /; ORL8812 /; ORL8813 /; ORL8814 /; ORL8815 /; ORL882 / B3; ORL883 / B4; ORL884 / B6; ORL885 / T1; ORL886 / T3; ORL887 / m51; ORL888 / B5; ORL889 / T2; ORL890 / T4; ORL891 / Orz6; ORL892 / Orz6; ORL917 / dM538M10.1; ORL918 / dM538M10.2; ORL919 / dM538M10.3; ORL920 / dM538M10.4; ORL921 / dM538M10.5; ORL9214 /; ORL9215 /; ORL9216 /; ORL9217 /; ORL9218 /; ORL9219 /; ORL922 / dM538M10.6; ORL9220 /; ORL9221 /; ORL9222 /; ORL9223 /; ORL9224 /; ORL9226 /; ORL9227 /; ORL9228 /; ORL9229 /; ORL923 / dM538M10.7; ORL9230 /; ORL9231 /; ORL9232 /; ORL9233 /; ORL9234 /; ORL9235 /; ORL9236 /; ORL9237 /; ORL9238 /; ORL9239 /; ORL924 / dM538M10.8; ORL9240 /; ORL9241 /; ORL9242 /; ORL9243 /; ORL9244 /; ORL9245 /; ORL9246 /; ORL9247 /; ORL9248 /; ORL9249 /; ORL9250 /; ORL9251 /; ORL9252 /; ORL9253 /; ORL9254 /; ORL9255 /; ORL9256 /; ORL9257 /; ORL9258 /; ORL9259 /; ORL926 / P4; ORL9260 /; ORL9261 /; ORL9262 /; ORL9263 /; ORL9264 /; ORL9265 /; ORL9266 /; ORL9267 /; ORL9268 /; ORL9269 /; ORL927 / P3; ORL9271 /; ORL9272 /; ORL9273 /; ORL9274 /; ORL9275 /; ORL9276 /; ORL9277 /; ORL9278 /; ORL9279 /; ORL928 / M50; ORL9280 /; ORL9281 /; ORL9282 /; ORL9283 /; ORL9284 /; ORL9285 /; ORL9286 /; ORL9287 /; ORL9288 /; ORL9350 /; ORL9445 /; ORL9446 /; ORL9447 /; ORL9448 /; ORL9449 /; ORL9450 /; ORL9451 /; ORL9452 /; ORL9453 /; ORL9454 /; ORL9455 /; ORL9456 /; ORL9457 /; ORL9458 /; ORL9536 / Olfr455; ORL9537 / Olfr218; ORL9538 /; ORL9538 / Olfr1083; ORL9539 / Olfr58; ORL9540 / Olfr568; ORL9541 / Olfr118; ORL9542 / Olfr845; ORL9542 / Olfr1286; ORL9543 / Olfr814; ORL9544 / Olfr103; ORL9545 / Olfr725; ORL9546 / Olfr890; ORL9547 / Olfr1066; ORL9548 / Olfr220; ORL9550 / Olfr723; ORL9551 / Olfr681; ORL9552 / Olfr924; ORL9553 / Olfr192; ORL9554 / Olfr250; ORL9555 / Olfr1212; ORL9556 / Olfr955; ORL9557 / Olfr153; ORL9558 / Olfr536; ORL9559 / Olfr382; ORL9560 / Olfr1303; ORL9561 / Olfr1367; ORL9562 / Olfr1393; ORL9563 / Olfr954; ORL9564 / Olfr373; ORL962 / OLFR4-3; ORL963 / A16; ORL964 / MOR18; ORL965 / MOR83; ORL966 / MOR10; ORL967 / MOR28; ORL968 / GABBR1; ORI1131 / MOR105-7P; |
| ORI1137 / MOR203-4/W531 T-1337138-1336194; ORI1146 / MOR136-7/W4QPD-29561; ORI1149 / MOR178-1/W4QPD-23048; ORI1153 / MOR231-4/W62NC-79141; ORI1157 / MOR231-3/W62NC-68647; ORI1162 / MOR233-2/W62NC-506540-505608; ORI1165 / MOR233-13/W62NC-319008-318070; ORI1167 / MOR230-7/W62NC-22680; ORI1180 / MOR232-1/W62NC-1142340-1143269; ORI1182 / MOR232-4/W62NC-1084069-1084983; ORI648 /7008627. |

**Table 20. Polymorphisms related to human bitter receptor genes**

(Table 20 contains reference sequence numbers for each of unique single nucleotide polymorphisms (SNP) for human TAS2R genes, position of the SNP in the reference sequence, and description of SNP.)

| Gene | Reference sequence number | Position in reference sequence | SNP |
| --- | --- | --- | --- |
| TAS2R1 | | | |
| | rs10543720 | pos=401 | alleles="- /CTATCTAT" |
| | rs2234228 | pos=101 | alleles="A/G" |
| | rs2234229 | pos=101 | alleles="C/T" |
| | rs2234230 | pos=101 | alleles="A/C" |

(continued)

| (Table 20 contains reference sequence numbers for each of unique single nucleotide polymorphisms (SNP) for human TAS2R genes, position of the SNP in the reference sequence, and description of SNP.) | | | |
|---|---|---|---|
| **Gene** | **Reference sequence number** | **Position in reference sequence** | **SNP** |
| | rs2234231 | pos=101 | alleles="C/T" |
| | rs2234232 | pos=101 | alleles="A/G" |
| | rs2234233 | pos=301 | alleles="C/T" |
| | rs2234234 | pos=101 | alleles="C/T" |
| | rs2234235 | pos=301 | alleles="C/T" |
| | rs34440745 | pos=301 | alleles="A/T" |
| | rs35186690 | pos=301 | alleles="-/G" |
| | rs35524938 | pos=401 | alleles="-/ATCT" |
| | rs36214451 | pos=401 | alleles="- /TATCTATC" |
| | rs41464 | pos=201 | alleles="A/G" |
| | rs41465 | pos=201 | alleles="A/G" |
| | rs41466 | pos=301 | alleles="A/G" |
| | rs41467 | pos=301 | alleles="G/T" |
| | rs41468 | pos=301 | alleles="C/T" |
| | rs41469 | pos=301 | alleles="A/G" |
| | rs41470 | pos=201 | alleles="A/G" |
| | rs56300050 | pos=253 | alleles="-/ATCT" |
| | rs57183738 | pos=101 | alleles="G/T" |
| | rs58046500 | pos=101 | alleles="C/T" |
| | rs58171988 | pos=201 | alleles="A/G" |
| TAS2R3 | rs11514837 | pos=458 | alleles="A/G" |
| | rs11763979 | pos=501 | alleles="G/T" |
| | rs11771020 | pos=501 | alleles="C/T" |
| | rs11771072 | pos=201 | alleles="A/C" |
| | rs12667706 | pos=201 | alleles="A/G" |
| | rs12703406 | pos=277 | alleles="A/G" |
| | rs13311828 | pos=367 | alleles="A/G" |
| | rs13311829 | pos=367 | alleles="C/G" |
| | rs13311831 | pos=342 | alleles="A/G" |
| | rs17162469 | pos=101 | alleles="A/G" |
| | rs17162471 | pos=101 | alleles="A/C" |
| | rs17162473 | pos=101 | alleles="A/G" |
| | rs17162483 | pos=101 | alleles="A/G" |
| | rs2270009 | pos=301 | alleles="C/T" |
| | rs28480612 | pos=201 | alleles="A/G" |

(continued)

| (Table 20 contains reference sequence numbers for each of unique single nucleotide polymorphisms (SNP) for human TAS2R genes, position of the SNP in the reference sequence, and description of SNP.) | | | |
|---|---|---|---|
| **Gene** | **Reference sequence number** | **Position in reference sequence** | **SNP** |
| | rs4726475 | pos=609 | alleles="C/T" |
| | rs56917574 | pos=101 | alleles="G/T" |
| | rs58640454 | pos=101 | alleles="A/G" |
| | rs60922375 | pos=101 | alleles="A/C" |
| | rs6962760 | pos=301 | alleles="C/T" |
| | rs6965618 | pos=259 | alleles="C/T" |
| | rs765007 | pos=301 | alleles="C/T" |
| | rs765008 | pos=301 | alleles="G/T" |
| | rs7793232 | pos=714 | alleles="A/G" |
| TAS2R4 | rs10485837 | pos=101 | alleles="A/G" |
| | rs2233990 | pos=301 | alleles="A/G" |
| | rs2233991 | pos=101 | alleles="C/T" |
| | rs2233992 | pos=101 | alleles="A/G" |
| | rs2233993 | pos=101 | alleles="A/G" |
| | rs2233994 | pos=101 | alleles="A/G" |
| | rs2233995 | pos=301 | alleles="A/G" |
| | rs2233996 | pos=101 | alleles="C/G" |
| | rs2233997 | pos=101 | alleles="A/C" |
| | rs2233998 | pos=301 | alleles="C/T" |
| | rs2233999 | pos=101 | alleles="A/T" |
| | rs2234000 | pos=101 | alleles="C/T" |
| | rs2234001 | pos=301 | alleles="C/G" |
| | rs2234002 | pos=301 | alleles="A/G" |
| | rs2234003 | pos=101 | alleles="A/G" |
| | rs33920115 | pos=301 | alleles="A/G" |
| | rs34855644 | pos=301 | alleles="-/T" |
| | rs3840580 | pos=61 | alleles="-/AA" |
| | rs57597591 | pos=201 | alleles="-/T" |
| | rs59513189 | pos=201 | alleles="G/T" |
| | rs61582517 | pos=201 | alleles="- /TGTAGATA" |
| TAS2R5 | rs10952507 | pos=201 | alleles="A/G" |
| | rs11761380 | pos=301 | alleles="A/C" |
| | rs11769235 | pos=201 | alleles="A/C" |
| | rs2227264 | pos=301 | alleles="G/T" |
| | rs2234004 | pos=101 | alleles="C/T" |

(continued)

| (Table 20 contains reference sequence numbers for each of unique single nucleotide polymorphisms (SNP) for human TAS2R genes, position of the SNP in the reference sequence, and description of SNP.) | | | |
|---|---|---|---|
| **Gene** | **Reference sequence number** | **Position in reference sequence** | **SNP** |
| | rs2234005 | pos=101 | alleles="A/G" |
| | rs2234006 | pos=682 | alleles="C/T" |
| | rs2234007 | pos=494 | alleles="A/G" |
| | rs2234008 | pos=101 | alleles="A/G" |
| | rs2234009 | pos=101 | alleles="C/T" |
| | rs2234010 | pos=101 | alleles="A/G" |
| | rs2234011 | pos=101 | alleles="C/T" |
| | rs2234012 | pos=301 | alleles="A/G" |
| | rs2234013 | pos=101 | alleles="A/G" |
| | rs2234014 | pos=101 | alleles="C/T" |
| | rs2234015 | pos=301 | alleles="A/G" |
| | rs2234016 | pos=101 | alleles="G/T" |
| | rs2234017 | pos=201 | alleles="C/G" |
| | rs2234018 | pos=101 | alleles="A/T" |
| | rs2234019 | pos=101 | alleles="A/G" |
| | rs2234020 | pos=101 | alleles="C/T" |
| | rs34529840 | pos=301 | alleles="A/G" |
| | rs3801001 | pos=61 | alleles="A/C" |
| | rs4726476 | pos=201 | alleles="C/G" |
| | rs60900504 | pos=101 | alleles="C/T" |
| | rs62477710 | pos=251 | alleles="C/T" |
| | rs62477711 | pos=251 | alleles="G/T" |
| TAS2R7 | rs10161483 | pos=201 | alleles="A/G" |
| | rs10772362 | pos=501 | alleles="C/T" |
| | rs11054041 | pos=201 | alleles="A/C" |
| | rs11838055 | pos=301 | alleles="A/G" |
| | rs2418107 | pos=501 | alleles="C/G" |
| | rs2588350 | pos=301 | alleles="C/T" |
| | rs34212148 | pos=301 | alleles="-/G" |
| | rs36067388 | pos=301 | alleles="-/G" |
| | rs3759251 | pos=101 | alleles="A/T" |
| | rs3759252 | pos=61 | alleles="A/C" |
| | rs619381 | pos=519 | alleles="C/T" |
| | rs7303054 | pos=201 | alleles="C/T" |
| TAS2R8 | rs12314840 | pos=224 | alleles="CAT" |

(continued)

| (Table 20 contains reference sequence numbers for each of unique single nucleotide polymorphisms (SNP) for human TAS2R genes, position of the SNP in the reference sequence, and description of SNP.) | | | |
|---|---|---|---|
| **Gene** | **Reference sequence number** | **Position in reference sequence** | **SNP** |
| | rs1548803 | pos=780 | alleles="C/T" |
| | rs1838344 | pos=277 | alleles="C/T" |
| | rs1838345 | pos=322 | alleles="A/G" |
| | rs2537817 | pos=301 | alleles="C/T" |
| | rs40313 | pos=176 | alleles="C/T" |
| | rs41324347 | pos=65 | alleles="G/T" |
| | rs60652912 | pos=201 | alleles="A/C" |
| | rs620878 | pos=283 | alleles="G/T" |
| | rs7972779 | pos=424 | alleles="C/T" |
| TAS2R9 | rs11054042 | pos=201 | alleles="C/G" |
| | rs11054043 | pos=201 | alleles="G/T" |
| | rs11054044 | pos=201 | alleles="C/G" |
| | rs11402198 | pos=401 | alleles="-/G" |
| | rs17207899 | pos=101 | alleles="G/T" |
| | rs17742870 | pos=101 | alleles="A/T" |
| | rs1838346 | pos=301 | alleles="A/G" |
| | rs2159903 | pos=84 | alleles="A/G" |
| | rs36044129 | pos=301 | alleles="-/T" |
| | rs3741845 | pos=179 | alleles="C/T" |
| | rs3944035 | pos=100 | alleles="A/G" |
| | rs40313 | pos=176 | alleles="C/T" |
| | rs60652912 | pos=201 | alleles="A/C" |
| | rs61320953 | pos=201 | alleles="-/T" |
| | rs655046 | pos=301 | alleles="A/G" |
| | rs667123 | pos=301 | alleles="A/G" |
| | rs667128 | pos=201 | alleles="C/T" |
| TAS2R10 | rs10845219 | pos=301 | alleles="C/T" |
| | rs12307411 | pos=301 | alleles="C/T" |
| | rs35370388 | pos=301 | alleles="-/TGTG" |
| | rs58719830 | pos=225 | alleles="-/TGTG" |
| | rs597468 | pos=301 | alleles="A/G" |
| | rs60832178 | pos=101 | alleles="C/T" |
| | rs61912242 | pos=251 | alleles="G/T" |
| | rs689118 | pos=301 | alleles="C/T" |
| TAS2R13 | rs1015442 | pos=519 | alleles="C/T" |

(continued)

| (Table 20 contains reference sequence numbers for each of unique single nucleotide polymorphisms (SNP) for human TAS2R genes, position of the SNP in the reference sequence, and description of SNP.) | | | |
|---|---|---|---|
| **Gene** | **Reference sequence number** | **Position in reference sequence** | **SNP** |
| | rs1015443 | pos=946 | alleles="C/T" |
| | rs10566346 | pos=401 | alleles="-/TG" |
| | rs10591343 | pos=501 | alleles="-/GT" |
| | rs10845238 | pos=258 | alleles="G/T" |
| | rs10845239 | pos=346 | alleles="A/T" |
| | rs10845240 | pos=449 | alleles="C/G" |
| | rs11054070 | pos=2000 | alleles="C/G" |
| | rs11054071 | pos=201 | alleles="C/G" |
| | rs11830286 | pos=301 | alleles="A/G" |
| | rs34885344 | pos=301 | alleles="C/T" |
| | rs35172210 | pos=301 | alleles="-/T" |
| | rs56987993 | pos=101 | alleles="C/G" |
| | rs7308212 | pos=256 | alleles="C/T" |
| | rs7968736 | pos=201 | alleles="A/T" |
| | rs7978678 | pos=201 | alleles="A/G" |
| TAS2R14 | rs10492104 | pos=101 | alleles="C/G" |
| | rs11610105 | pos=201 | alleles="A/G" |
| | rs16925868 | pos=101 | alleles="C/T" |
| | rs3033010 | pos=501 | alleles="-/C/CT/G" |
| | rs34789740 | pos=301 | alleles="A/G" |
| | rs35386049 | pos=301 | alleles="-/C" |
| | rs35405135 | pos=301 | alleles="-/T" |
| | rs35804287 | pos=301 | alleles="A/G" |
| | rs35926739 | pos=301 | alleles="-/T" |
| | rs3741843 | pos=301 | alleles="A/G" |
| | rs3851583 | pos=501 | alleles="A/G" |
| | rs3851584 | pos=500 | alleles="G/T" |
| | rs3851585 | pos=501 | alleles="C/G" |
| | rs3863321 | pos=21 | alleles="C/T" |
| | rs3936285 | pos=537 | alleles="A/T" |
| | rs4140968 | pos=101 | alleles="C/T" |
| | rs56393802 | pos=241 | alleles="-/TG" |
| | rs60186756 | pos=201 | alleles="-/T" |
| | rs60288130 | pos=201 | alleles="-/TT" |
| | rs61659284 | pos=226 | alleles="-/CTCT" |

(continued)

| (Table 20 contains reference sequence numbers for each of unique single nucleotide polymorphisms (SNP) for human TAS2R genes, position of the SNP in the reference sequence, and description of SNP.) | | | |
|---|---|---|---|
| **Gene** | **Reference sequence number** | **Position in reference sequence** | **SNP** |
| | rs7138535 | pos=301 | alleles="A/T" |
| | rs7487884 | pos=239 | alleles="C/T" |
| TAS2R16 | rs10487745 | pos=101 | alleles="A/C" |
| | rs1204014 | pos=201 | alleles="A/G" |
| | rs1357949 | pos=497 | alleles="A/G" |
| | rs1525489 | pos=301 | alleles="A/G" |
| | rs2233988 | pos=301 | alleles="C/T" |
| | rs2233989 | pos=201 | alleles="C/T" |
| | rs2692396 | pos=301 | alleles="C/G" |
| | rs28371571 | pos=94 | alleles="A/G" |
| | rs28371572 | pos=114 | alleles="C/G" |
| | rs28371573 | pos=126 | alleles="C/T" |
| | rs28371574 | pos=133 | alleles="A/G" |
| | rs28371575 | pos=140 | alleles="C/T" |
| | rs28371576 | pos=136 | alleles="C/T" |
| | rs28371577 | pos=140 | alleles="A/C" |
| | rs28371578 | pos=138 | alleles="A/G" |
| | rs28371579 | pos=139 | alleles="C/T" |
| | rs28371580 | pos=139 | alleles="A/G" |
| | rs28371581 | pos=139 | alleles="G/T" |
| | rs34032423 | pos=301 | alleles="-/CT" |
| | rs34215184 | pos=301 | alleles="A/C" |
| | rs34638781 | pos=301 | alleles="-/C" |
| | rs35947098 | pos=301 | alleles="C/T" |
| | rs58410964 | pos=101 | alleles="A/G" |
| | rs59108896 | pos=101 | alleles="G/T" |
| | rs59743922 | pos=101 | alleles="A/G" |
| | rs60714340 | pos=101 | alleles="C/T" |
| | rs6466849 | pos=201 | alleles="C/T" |
| | rs702423 | pos=301 | alleles="A/G" |
| | rs846664 | pos=301 | alleles="G/T" |
| | rs846665 | pos=284 | alleles="C/G" |
| | rs846666 | pos=392 | alleles="G/T" |
| | rs860170 | pos=301 | alleles="A/G" |
| | rs978739 | pos=535 | alleles="A/G" |

(continued)

| (Table 20 contains reference sequence numbers for each of unique single nucleotide polymorphisms (SNP) for human TAS2R genes, position of the SNP in the reference sequence, and description of SNP.) | | | |
|---|---|---|---|
| **Gene** | **Reference sequence number** | **Position in reference sequence** | **SNP** |
| TAS2R38 | rs10246939 | pos=301 | alleles="C/T" |
| | rs1726866 | pos=301 | alleles="C/T" |
| | rs35251805 | pos=301 | alleles="-/G" |
| | rs4613903 | pos=301 | alleles="G/T" |
| | rs61464348 | pos=201 | alleles="A/C" |
| | rs713598 | pos=301 | alleles="C/G" |
| TAS2R39 | rs10608369 | pos=401 | alleles="-/GT" |
| | rs34169190 | pos=301 | alleles="C/T" |
| | rs35474877 | pos=301 | alleles="A/G" |
| | rs4103817 | pos=451 | alleles="A/G" |
| | rs4726600 | pos=301 | alleles="A/G" |
| | rs56782833 | pos=283 | alleles="-/A" |
| | rs59031091 | pos=201 | alleles="C/G" |
| | rs6964922 | pos=227 | alleles="C/T" |
| TAS2R40 | rs10225801 | pos=201 | alleles="A/G" |
| | rs10260248 | pos=301 | alleles="A/C" |
| | rs17164164 | pos=301 | alleles="C/G" |
| TAS2R41 | rs10278721 | pos=301 | alleles="C/T" |
| | rs13243940 | pos=501 | alleles="A/T" |
| | rs13362832 | pos=201 | alleles="C/T" |
| | rs13362858 | pos=301 | alleles="C/G" |
| | rs1404634 | pos=301 | alleles="A/G" |
| | rs1404635 | pos=301 | alleles="A/G" |
| | rs1473653 | pos=301 | alleles="A/G" |
| | rs33922222 | pos=401 | alleles="-/C" |
| | rs34170633 | pos=301 | alleles="-/A" |
| | rs34281448 | pos=301 | alleles="-/A" |
| | rs34863914 | pos=301 | alleles="C/T" |
| | rs5888105 | pos=401 | alleles="-/G" |
| | rs5888106 | pos=401 | alleles="-/C" |
| | rs59826238 | pos=101 | alleles="C/T" |
| | rs60096100 | pos=201 | alleles="A/C" |
| | rs6947971 | pos=5600 | alleles="G/T" |
| | rs6949267 | pos=526 | alleles="C/G" |
| TAS2R43 | rs10556970 | pos=401 | alleles="-/AT" |

(continued)

| (Table 20 contains reference sequence numbers for each of unique single nucleotide polymorphisms (SNP) for human TAS2R genes, position of the SNP in the reference sequence, and description of SNP.) | | | |
|---|---|---|---|
| **Gene** | **Reference sequence number** | **Position in reference sequence** | **SNP** |
| | rs1965231 | pos=265 | alleles="C/T" |
| | rs34115566 | pos=301 | alleles="-/GT" |
| | rs35720106 | pos=301 | alleles="C/G" |
| TAS2R44 | rs10591850 | pos=401 | alleles="-/AAAT" |
| | rs10743938 | pos=201 | alleles="A/T" |
| | rs10772422 | pos=501 | alleles="C/T" |
| | rs10772423 | pos=301 | alleles="C/T" |
| | rs10845293 | pos=301 | alleles="A/G" |
| | rs10845294 | pos=301 | alleles="C/G" |
| | rs10845295 | pos=201 | alleles="A/G" |
| | rs10845296 | pos=371 | alleles="A/G" |
| | rs11522329 | pos=301 | alleles="A/G" |
| | rs11537117 | pos=201 | alleles="A/T" |
| | rs11537118 | pos=218 | alleles="A/G" |
| | rs11560815 | pos=231 | alleles="C/T" |
| | rs11612527 | pos=301 | alleles="A/T" |
| | rs12315036 | pos=201 | alleles="G/T" |
| | rs12318612 | pos=301 | alleles="C/G" |
| | rs12370363 | pos=201 | alleles="A/G" |
| | rs12819202 | pos=301 | alleles="C/T" |
| | rs1965230 | pos=663 | alleles="A/G" |
| | rs2418291 | pos=501 | alleles="C/T" |
| | rs2418292 | pos=500 | alleles="A/G" |
| | rs2418293 | pos=500 | alleles="C/T" |
| | rs2418294 | pos=500 | alleles="C/T" |
| | rs2418295 | pos=500 | alleles="C/G" |
| | rs2418296 | pos=500 | alleles="A/G" |
| | rs2418297 | pos=500 | alleles="C/T" |
| | rs2418298 | pos=500 | alleles="A/C" |
| | rs2418299 | pos=500 | alleles="A/T" |
| | rs2418300 | pos=500 | alleles="A/C" |
| | rs2418301 | pos=500 | alleles="C/T" |
| | rs28409955 | pos=201 | alleles="C/T" |
| | rs28679275 | pos=201 | alleles="C/T" |
| | rs2900583 | pos=501 | alleles="C/T" |

(continued)

| (Table 20 contains reference sequence numbers for each of unique single nucleotide polymorphisms (SNP) for human TAS2R genes, position of the SNP in the reference sequence, and description of SNP.) | | | |
|---|---|---|---|
| **Gene** | **Reference sequence number** | **Position in reference sequence** | **SNP** |
| | rs2900584 | pos=501 | alleles="C/T" |
| | rs2900585 | pos=501 | alleles="C/T" |
| | rs2952703 | pos=201 | alleles="G/T" |
| | rs33998340 | pos=401 | alleles="-/AGT" |
| | rs34066385 | pos=401 | alleles="-/ACAC" |
| | rs34763234 | pos=301 | alleles="A/G" |
| | rs35241999 | pos=301 | alleles="A/G" |
| | rs3759246 | pos=61 | alleles="C/G" |
| | rs3759247 | pos=61 | alleles="A/G" |
| | rs3983336 | pos=500 | alleles="A/G" |
| | rs3983337 | pos=500 | alleles="A/C" |
| | rs3983338 | pos=500 | alleles="A/C" |
| | rs3983339 | pos=500 | alleles="C/T" |
| | rs3983340 | pos=500 | alleles="C/T" |
| | rs3983341 | pos=500 | alleles="A/G" |
| | rs3983342 | pos=500 | alleles="G/T" |
| | rs3983343 | pos=500 | alleles="C/T" |
| | rs5024225 | pos=401 | alleles="A/T" |
| | rs56079155 | pos=201 | alleles="-/CA" |
| | rs56873588 | pos=201 | alleles="-/AATA" |
| | rs5796420 | pos=401 | alleles="-/ACAC" |
| | rs7952952 | pos=301 | alleles="A/G" |
| | rs7953498 | pos=301 | alleles="C/G" |
| TAS2R45 | NA | | |
| TAS2R46 | rs11560816 | pos=201 | alleles="A/G" |
| | rs2244875 | pos=500 | alleles="C/T" |
| | rs2598002 | pos=301 | alleles="A/C" |
| | rs2599402 | pos=201 | alleles="A/G" |
| | rs2708378 | pos=201 | alleles="C/T" |
| | rs2708379 | pos=201 | alleles="A/G" |
| | rs2708380 | pos=301 | alleles="A/T" |
| | rs2708381 | pos=301 | alleles="A/G" |
| | rs2708382 | pos=495 | alleles="A/G" |
| | rs34033169 | pos=301 | alleles="-/G" |
| | rs34164014 | pos=301 | alleles="-/C" |

(continued)

| (Table 20 contains reference sequence numbers for each of unique single nucleotide polymorphisms (SNP) for human TAS2R genes, position of the SNP in the reference sequence, and description of SNP.) | | | |
|---|---|---|---|
| **Gene** | **Reference sequence number** | **Position in reference sequence** | **SNP** |
| | rs35602687 | pos=301 | alleles="-/C" |
| | rs35801645 | pos=301 | alleles="-/T" |
| | rs61912070 | pos=251 | alleles="G/T" |
| | rs62760561 | pos=401 | alleles="-/TCT" |
| | rs63450660 | pos=401 | alleles="-/T" |
| | rs7970996 | pos=201 | alleles="C/T" |
| TAS2R47 | rs10645657 | pos=401 | alleles="-/AC" |
| | rs1669404 | pos=201 | alleles="A/G" |
| | rs1669405 | pos=201 | alleles="G/T" |
| | rs1960613 | pos=502 | alleles="G/T" |
| | rs2218819 | pos=37 | alleles="C/T" |
| | rs2597924 | pos=201 | alleles="A/G" |
| | rs2597925 | pos=201 | alleles="A/G" |
| | rs2597926 | pos=201 | alleles="G/T" |
| | rs2597927 | pos=201 | alleles="G/T" |
| | rs2599396 | pos=301 | alleles="A/G" |
| | rs2599397 | pos=301 | alleles="C/G" |
| | rs2599404 | pos=301 | alleles="A/C" |
| | rs2600355 | pos=301 | alleles="G/T" |
| | rs2600356 | pos=301 | alleles="A/C" |
| | rs2600357 | pos=301 | alleles="C/T" |
| | rs2600358 | pos=301 | alleles="A/G" |
| | rs2708351 | pos=201 | alleles="G/T" |
| | rs2708371 | pos=201 | alleles="C/G" |
| | rs2708372 | pos=201 | alleles="C/T" |
| | rs2923236 | pos=201 | alleles="C/T" |
| | rs2952701 | pos=201 | alleles="C/T" |
| | rs2952702 | pos=201 | alleles="C/T" |
| | rs34383190 | pos=401 | alleles="-/TC" |
| | rs34570579 | pos=301 | alleles="-/C" |
| | rs34656404 | pos=301 | alleles="A/G" |
| | rs34960146 | pos=301 | alleles="-/C" |
| | rs35267335 | pos=301 | alleles="A/G" |
| | rs35413568 | pos=301 | alleles="-/C" |
| | rs35632581 | pos=301 | alleles="-/C" |

(continued)

| (Table 20 contains reference sequence numbers for each of unique single nucleotide polymorphisms (SNP) for human TAS2R genes, position of the SNP in the reference sequence, and description of SNP.) | | | |
|---|---|---|---|
| **Gene** | **Reference sequence number** | **Position in reference sequence** | **SNP** |
| | rs35884825 | pos=401 | alleles="-/AG" |
| | rs36109559 | pos=301 | alleles="-/A" |
| | rs36123978 | pos=301 | alleles="-/AG" |
| | rs3759244 | pos=201 | alleles="C/T" |
| | rs3759245 | pos=201 | alleles="C/T" |
| | rs3863323 | pos=501 | alleles="G/T" |
| | rs4092162 | pos=91 | alleles="A/G" |
| | rs4763238 | pos=201 | alleles="A/C" |
| | rs5796422 | pos=401 | alleles="-/AG" |
| | rs61928449 | pos=251 | alleles="A/C" |
| | rs7296647 | pos=201 | alleles="A/G" |
| | rs7313796 | pos=201 | alleles="A/C" |
| | rs7980677 | pos=301 | alleles="C/T" |
| | rs977473 | pos=209 | alleles="A/T" |
| | rs977474 | pos=512 | alleles="A/G" |
| TAS2R48 | rs10743937 | pos=301 | alleles="C/T" |
| | rs10772419 | pos=301 | alleles="A/C" |
| | rs10772420 | pos=301 | alleles="A/G" |
| | rs11054169 | pos=335 | alleles="A/G" |
| | rs11054170 | pos=337 | alleles="G/T" |
| | rs11054171 | pos=356 | alleles="A/G" |
| | rs12313469 | pos=301 | alleles="A/G" |
| | rs12424373 | pos=301 | alleles="G/T" |
| | rs12578654 | pos=301 | alleles="C/T" |
| | rs1868768 | pos=301 | alleles="A/C" |
| | rs1868769 | pos=312 | alleles="A/G" |
| | rs34254748 | pos=301 | alleles="-/G" |
| | rs35032794 | pos=301 | alleles="-/C" |
| | rs36057973 | pos=301 | alleles="-/G" |
| | rs3863330 | pos=499 | alleles="A/T" |
| | rs3863333 | pos=301 | alleles="G/T" |
| | rs4763235 | pos=201 | alleles="C/G" |
| | rs56985810 | pos=201 | alleles="C/T" |
| | rs60770813 | pos=101 | alleles="C/G" |
| | rs61624520 | pos=201 | alleles="-/T" |

(continued)

| (Table 20 contains reference sequence numbers for each of unique single nucleotide polymorphisms (SNP) for human TAS2R genes, position of the SNP in the reference sequence, and description of SNP.) | | | |
|---|---|---|---|
| **Gene** | **Reference sequence number** | **Position in reference sequence** | **SNP** |
| | rs7131800 | pos=267 | alleles="A/G" |
| | rs7961372 | pos=201 | alleles="A/C" |
| | rs9330646 | pos=301 | alleles="A/T" |
| | rs9777804 | pos=301 | alleles="C/G" |
| | rs9777906 | pos=301 | alleles="A/T" |
| TAS2R49 | rs10772407 | pos=201 | alleles="A/C" |
| | rs10845278 | pos=356 | alleles="C/T" |
| | rs10845279 | pos=301 | alleles="A/C" |
| | rs10845280 | pos=301 | alleles="A/G" |
| | rs10845281 | pos=301 | alleles="C/T" |
| | rs11054139 | pos=501 | alleles="C/T" |
| | rs11054140 | pos=301 | alleles="C/T" |
| | rs11054141 | pos=261 | alleles="C/T" |
| | rs11054142 | pos=301 | alleles="A/G" |
| | rs11054143 | pos=301 | alleles="C/T" |
| | rs12226919 | pos=301 | alleles="G/T" |
| | rs12226920 | pos=301 | alleles="G/T" |
| | rs12311429 | pos=301 | alleles="A/G" |
| | rs12311490 | pos=301 | alleles="A/G" |
| | rs12312963 | pos=201 | alleles="C/T" |
| | rs1450839 | pos=301 | alleles="A/G" |
| | rs1463237 | pos=348 | alleles="C/T" |
| | rs34365504 | pos=301 | alleles="-/T" |
| | rs34579433 | pos=301 | alleles="-/A" |
| | rs34813278 | pos=301 | alleles="-/A" |
| | rs34965724 | pos=301 | alleles="-/A" |
| | rs35021650 | pos=301 | alleles="-/C" |
| | rs35875890 | pos=301 | alleles="-/ATG" |
| | rs4388985 | pos=401 | alleles="A/G" |
| | rs4418898 | pos=401 | alleles="C/T" |
| | rs4506739 | pos=401 | alleles="A/G" |
| | rs4763604 | pos=201 | alleles="G/T" |
| | rs4763605 | pos=201 | alleles="A/G" |
| | rs58133495 | pos=501 | alleles="-/GAT" |
| | rs59686635 | pos=101 | alleles="A/C" |

(continued)

| (Table 20 contains reference sequence numbers for each of unique single nucleotide polymorphisms (SNP) for human TAS2R genes, position of the SNP in the reference sequence, and description of SNP.) | | | |
|---|---|---|---|
| **Gene** | **Reference sequence number** | **Position in reference sequence** | **SNP** |
| | rs61912291 | pos=251 | alleles="G/T" |
| | rs7135018 | pos=251 | alleles="C/T" |
| | rs7135941 | pos=301 | alleles="C/T" |
| | rs7301234 | pos=301 | alleles="A/G" |
| TAS2R50 | rs10772396 | pos=362 | alleles="C/T" |
| | rs10772397 | pos=301 | alleles="C/T" |
| | rs10772398 | pos=201 | alleles="C/T" |
| | rs10772399 | pos=201 | alleles="C/T" |
| | rs11054131 | pos=201 | alleles="C/G" |
| | rs11054132 | pos=201 | alleles="A/G" |
| | rs11054133 | pos=201 | alleles="C/T" |
| | rs11421487 | pos=401 | alleles="-/T" |
| | rs12426805 | pos=301 | alleles="A/G" |
| | rs1376251 | pos=301 | alleles="C/T" |
| | rs2167263 | pos=245 | alleles="C/G" |
| | rs35533340 | pos=301 | alleles="-/C/G" |
| | rs35633248 | pos=301 | alleles="-/T" |
| | rs35638884 | pos=301 | alleles="-/A" |
| | rs35852119 | pos=301 | alleles="-/T" |
| | rs35970171 | pos=301 | alleles="-/T" |
| | rs55748583 | pos=201 | alleles="C/T" |
| | rs58805611 | pos=101 | alleles="C/T" |
| TAS2R55 | NA | | |
| TAS2R60 | rs10241042 | pos=316 | alleles="C/G" |
| | rs10241523 | pos=316 | alleles="A/C" |
| | rs11978402 | pos=337 | alleles="A/G" |
| | rs12534427 | pos=301 | alleles="C/G" |
| | rs12671578 | pos=201 | alleles="A/G" |
| | rs34328217 | pos=301 | alleles="-/C" |
| | rs34465195 | pos=301 | alleles="A/G" |
| | rs34910453 | pos=301 | alleles="C/T" |
| | rs35195910 | pos=301 | alleles="-/TCT" |
| | rs36004042 | pos=301 | alleles="-/G" |
| | rs4541818 | pos=401 | alleles="C/G" |
| | rs4595035 | pos=301 | alleles="C/T" |

(continued)

| (Table 20 contains reference sequence numbers for each of unique single nucleotide polymorphisms (SNP) for human TAS2R genes, position of the SNP in the reference sequence, and description of SNP.) | | | |
|---|---|---|---|
| **Gene** | **Reference sequence number** | **Position in reference sequence** | **SNP** |
| | rs58270521 | pos=251 | alleles="C/T" |

**Table 21. Allelic variations in coding sequences of human bitter receptors**

| Protein | Feature key | Position(s) | Length | Description | Feature identifier |
|---|---|---|---|---|---|
| TAS2R1 | Natural variant | 111 | 1 | R → H: dbSNP rs41469. | VAR_020198 |
| | Natural variant | 141 | 1 | C → Y: dbSNP rs2234232. | VAR_053340 |
| | Natural variant | 206 | 1 | R → W: dbSNP rs2234233. | VAR_020199 |
| TAS2R4 | Natural variant | 3 | 1 | R → Q: dbSNP rs2233995. | VAR_034535 |
| | Natural variant | 7 | 1 | F → S: dbSNP rs2233998. | VAR_034536 |
| | Natural variant | 62 | 1 | F → L: dbSNP rs2233999. | VAR_053341 |
| | Natural variant | 74 | 1 | T → M: dbSNP rs2234000. | VAR_020200 |
| | Natural variant | 96 | 1 | V → L: dbSNP rs2234001. | VAR_020201 |
| | Natural variant | 171 | 1 | S → N: dbSNP rs2234002. | VAR_020202 |
| | Natural variant | 191 | 1 | I → V: dbSNP rs2234003. | VAR_053342 |
| TAS2R5 | Natural variant | 20 | 1 | G → S: dbSNP rs2234013. | VAR_053343 |
| | Natural variant | 26 | 1 | S → I: dbSNP rs2227264. | VAR_020203 |
| | Natural variant | 113 | 1 | P → L: dbSNP rs2234014. | VAR_034537 |
| | Natural variant | 167 | 1 | Y → C: dbSNP rs34529840. | VAR_034538 |
| | Natural variant | 213 | 1 | R → Q: dbSNP rs2234015. | VAR_024184 |
| | Natural variant | 294 | 1 | R → L: dbSNP rs2234016. | VAR_053344 |
| TAS2R7 | Natural variant | 263 | 1 | T → S: dbSNP rs3759251. | VAR_021852 |
| | Natural variant | 304 | 1 | M → I: dbSNP rs619381. | VAR_024185 |

(continued)

| Protein | Feature key | Position(s) | Length | Description | Feature identifier |
|---|---|---|---|---|---|
| TAS2R8 | Natural variant | 308 | 1 | M → V: dbSNP rs2537817. | VAR_024186 |
| TAS2R9 | Natural variant | 170 | 1 | K → Q: dbSNP rs11054043. | VAR_053345 |
| | Natural variant | 187 | 1 | V → A: dbSNP rs3741845. | VAR_020204 |
| | Natural variant | 238 | 1 | L → V: dbSNP rs11054042. | VAR_053346 |
| TAS2R10 | Natural variant | 156 | 1 | M → T: dbSNP rs597468. | VAR_030009 |
| TAS2R13 | Natural variant | 149 | 1 | N → S in a breast cancer sample; somatic mutation. | VAR_036432 |
| | Natural variant | 259 | 1 | N → S: dbSNP rs1015443. | VAR_021853 |
| TAS2R14 | Natural variant | 86 | 1 | T → A: dbSNP rs16925868. | VAR_053347 |
| TAS2R16 | Natural variant | 172 | 1 | N → K Associated with susceptibility to alcoholism. dbSNP rs846664. | VAR_034539 |
| | Natural variant | 222 | 1 | R → H: dbSNP rs860170. | VAR_020205 |
| TAS2R38 | Natural variant | 49 | 1 | A → P: dbSNP rs713598. | VAR_017860 |
| | Natural variant | 262 | 1 | A → V: dbSNP rs1726866. | VAR_017861 |
| | Natural variant | 296 | 1 | I → V: dbSNP rs10246939. | VAR_017862 |
| TAS2R39 | Natural variant | 193 | 1 | S → F: dbSNP rs35474877. | VAR_053348 |
| | Natural variant | 197 | 1 | K → E: dbSNP rs34169190. | VAR_053349 |
| TAS2R40 | Natural variant | 23 | 1 | V → L: dbSNP rs17164164. | VAR_053350 |
| | Natural variant | 187 | 1 | S → Y: dbSNP rs10260248. | VAR_053351 |
| TAS2R41 | NA | | | | |
| TAS2R43 | NA | | | | |
| TAs2R44 | Natural variant | 35 | 1 | R → W: dbSNP rs10845295. | VAR_030684 |
| | Natural variant | 162 | 1 | M → L: dbSNP rs10743938. | VAR_030685 |
| | Natural variant | 217 | 1 | Q → E: dbSNP rs10845294. | VAR_030686 |

(continued)

| Protein | Feature key | Position(s) | Length | Description | Feature identifier |
|---|---|---|---|---|---|
| | Natural variant | 227 | 1 | A → V: dbSNP rs10845293. | VAR_030687 |
| | Natural variant | 240 | 1 | V → I: dbSNP rs10772423. | VAR_030688 |
| TAS2R45 | NA | | | | |
| TAS2R46 | NA | | | | |
| TAS2R47 | NA | | | | |
| TAS2R48 | Natural variant | 126 | 1 | K → Q: dbSNP rs12424373. | VAR_053354 |
| | Natural variant | 299 | 1 | R → C: dbSNP rs10772420 | VAR_053355 |
| TAS2R49 | Natural variant | 79 | 1 | K → E: dbSNP rs7135018. | VAR_053356 |
| | Natural variant | 143 | 1 | H → Q: dbSNP rs12226920. | VAR_053357 |
| | Natural variant | 148 | 1 | H → N: dbSNP rs12226919. | VAR_053358 |
| | Natural variant | 236 | 1 | I → V: dbSNP rs10845281. | VAR_053359 |
| | Natural variant | 252 | 1 | F → S: dbSNP rs10845280. | VAR_053360 |
| | Natural variant | 255 | 1 | R → L: dbSNP rs10845279. | VAR_053361 |
| TAS2R50 | Natural variant | 203 | 1 | Y → C: dbSNP rs1376251 | VAR_024187 |
| TAS2R55 | Natural variant | 196 | 1 | F → S: dbSNP rs5020531. | VAR_053352 |
| | Natural variant | 265 | 1 | Y → C: dbSNP rs1451772. | VAR_053353 |
| TAS2R55 | NA | | | | |
| TAS2R60 | NA | | | | |
| Note: All positional information in Table 21 refers to canonical sequences of human TAS2R proteins. | | | | | |

**Table 22. List of Insect Genes**

| Insect gene names |
|---|
| IR7a; IR7b; IR7c; IR7d; IR7e; IR7f; IR7g; IR8a; IR10a; IR11a; IR20a; IR21a; IR25a; IR31 a; IR40a; IR41a; IR47a; IR48b; IR48c; IR51b; IR52a; IR52b; IR52c; IR52d; IR54a; IR56a; IR56b; IR56c; IR56d; IR60a; IR60b; IR60c; IR60d; IR60e; IR62a; IR64a; IR67a; IR67b; IR67c; IR68a; IR68b; IR75a; IR75b; IR75c; IR75d; IR76a; IR76b; IR84a; IR85a; IR87a; IR92a; IR93a; IR94a; IR94b; IR94c; IR94d; IR94e; IR94f; IR94g; IR94h; IR100a |

Human odorant receptor homologs of the receptors listed in Table 22 can also be used with the methods and compositions disclosed herein.

**<u>Example 23 Generating a stable umami taste receptor-expressing cell line</u>** (Reference Example)

<u>Transfection</u>

HEK293T (ATCC CRL-11268) were cotransfected with three separate plasmids, one encoding T1 R1 (SEQ ID NO: 41), one encoding T1 R3 (SEQ ID NO: 32) and the other encoding a signaling molecule (mouse G$\alpha$15, SEQ ID NO: 33). Although drug selection is optional in the methods disclosed herein, we included one drug resistance marker per plasmid. The sequences were under the control of the CMV promoter. An untranslated sequence encoding a tag for detection by a signaling probe was also present along with a sequence encoding a drug resistance marker. The target sequences utilized were Target Sequence 1 (SEQ ID NO: 28), Target Sequence 2 (SEQ ID NO: 29) and Target Sequence 3 (SEQ ID NO: 30). In these examples, the T1 R1 gene vector contained Target Sequence 3, the T1 R3 gene vector contained Target Sequence 1 and the Gal5 gene vector contained the Target Sequence 2. The cells were conventionally selected in media containing the drug for 10-14 days.

<u>Exposure of cells to fluorogenic probes</u>

Selected cells were harvested and transfected with signaling probes (SEQ ID NO: 38-40). Signaling Probe 1 binds Tag Sequence 1 (SEQ ID NO:126), Signaling Probe 2 binds Tag Sequence 2 (SEQ ID NO:127) and Signaling Probe 3 binds Tag Sequence 3 (SEQ ID NO:128). The cells were then dissociated and collected for analysis and sorted using a fluorescence activated cell sorter (Beckman Coulter, Miami, FL). As will be appreciated by those of skill in the art, any reagent that is suitable for use with a chosen host cell may be used to introduce a nucleic acid, e.g. plasmid, oligonucleotide, labeled oligonucleotide, into a host cell with proper optimization. Examples of reagents that may be used to introduce nucleic acids into host cells include but are not limited to: Lipofectamine, Lipofectamine 2000, Oligofectamine, TFX reagents, Fugene 6, DOTAP/DOPE, Metafectine, or Fecturin.

Signaling probe 1
**5' - Cy5** GCCAGTCCCAGTTCCTGTGCCTTAAGAACCTCGC **BHQ3 quench -3'** (SEQ ID NO: 38)
Signaling probe 2
**5'- Cy5.5** GCGAGTCGCAGAACGACAGGGTTAACTTCCTCGC **BHQ3 quench -3'** (SEQ ID NO: 39)
Signaling probe 3
**5'- Fam** GCGAGAGCGACAAGCAGACCCTATAGAACCTCGC **BHQ1 quench -3'** (SEQ ID NO: 40)

Tag Sequence 1 with target sequence in bold (umami)

AGGGCGAATTCCAGCACACTGGCGGCCGTTACTAGTGGATCCGAGCTCGGAG GCAGGTGGACAGGAAGGTTCTAAT**GTTCTTAAGGCACAGGAACTGGGAC**ATCT GGGCCCGGAAAGCCTTTTTCTCTGTGATCCGGTACAGTCCTTCTGC (SEQ ID NO:126)

Tag Sequence 2 with target sequence in bold (umami)

AGGGCGAATTCCAGCACACTGGCGGCCGTTACTAGTGGATCCGAGCTCGGTAC CAAGCTTCGAGGCAGGTGGACAGCTTGGTTCTAAT**GAAGTTAACCCTGTCGTT CTGCGAC**ATCTGGGCCCGGAAAGCGTTTAACTGATGGATGGAACAGTCCTTCT GC (SEQ ID NO:127)

Tag Sequence 3 with target sequence in bold (umami)

AAGGGCGAATTCGGATCCGCGGCCGCCTTAAGCTCGAGGCAGGTGGACAGGA AGGTTCTAAT**GTTCTATAGGGTCTGCTTGTCGCTC**ATCTGGGCCCGGAGATG (SEQ ID NO:128)

Other target sequences and signaling probes could be used (see, e.g., as described in International Patent Application Publication No. WO2005/079462 published on September 1, 2005 (Application No. PCT/US05/005080). For example, BHQ3 could be substituted with BHQ1 or a gold particle in Probe 1 or Probe 2. Note that BHQ1 could be substituted with BHQ2 or Dabcyl in Probe 3. A similar probe using a Quasar Dye (BioSearch) with spectral properties similar to Cy5 could be used in certain experiments. Note also that 5-MedC and 2-aminodA mixmer probes rather than DNA probes could be used in some instances.

Isolation of positive cells

Standard analytical methods were used to gate cells fluorescing above background and to isolate cells falling within that defined gate directly into 96-well plates. Cell sorting was operated such that a single cell was deposited per well. After selection, the cells were expanded in media lacking drug.

Functional transformation

We maintained the umami taste receptor cells (selected and expanded as above) using both aliquots of the same cells and different cells in various growth conditions including, for example, low-glucose DMEM media or in glucose-free Leibovitz L-15 media with 10% serum or serum- free. Some cells were maintained in media containing various concentrations of other sugars such as galactose We then characterized cells of the umami taste receptor cell lines maintained under multiple conditions for their ability to respond appropriately to umami ligands and not respond to other stimuli (*i.e.*, sugars).

Growth in the various media conditions, without wishing to be bound by theory, may functionally transform the cells, *e.g.* due to changes in gene expression levels, genomic organization and functional expression of receptors at the cell surface. Parameters in the various media that were evaluated and shown to affect a cell's functional assay response include serum concentration (*i.e.* low serum concentrations and serum deprivation), sugar deprivation and assay plate coating (for example, poly D lysine and laminin). These results demonstrated that characteristics of the cells of the umami taste receptor cell lines may be different when the cells are maintained in different media conditions. They also show that different cells selected by the fluorogenic probes may have different characteristics. One example of our characterization of cells selected by the fluorogenic probes is in Figure 6. As shown in Figure 6, aliquots of the same cells grown in different conditions (1, 2 and Final) responded to umami (MSG) and sweet (fructose) ligands significantly differently. Cells grown in condition 1 were plated in serum deprived, low glucose media. Cells grown in condition 2 were plated overnight in low glucose media containing 10% serum, and were then switched to a serum-deprived media before the assay. Cells grown in conditions 1 or 2 were plated on coated plates (Corning #3665). "Final" conditions encompass high density growth on coated plates (Corning #3300) and in low glucose media with serum deprivation. Cells grown in condition 1 respond to sweet agonist more than umami agonist. Cells grown in condition 2 respond to both ligands equally. In contrast, cells grown in the "Final" growth conditions respond robustly to MSG and not to fructose. Thus, these cells produced an umami taste receptor that was physiologically and pharmacologically relevant.

**Example 24 Characterizing the cell line for native umami taste receptor function (Reference Example)**

1. Confirmation and Quantification of Gene Expression.

Using qRT-PCR, we determined the relative amounts (RNA) of each of the umami taste receptor subunits being expressed in the above cells ("Final"). Using qRT-PCR, we determined the relative amounts of each of the umami taste receptor subunits being expressed. Total RNA was purified from 1-3x$10^6$ mammalian cells using a commercially available RNA purification kit (RNeasy Mini Kit,Qiagen). The RNA extract was then treated using a rigorous DNase treatment protocol (TURBO DNA-free Kit; Ambion). First strand cDNA synthesis was performed using a Reverse Transcriptase Kit (SuperScript III, Invitrogen) in 20 uL reaction volume with 1 uG DNA-free total RNA and 250 nG Random Primers (Invitrogen). Negative controls for this reaction included samples in which reverse transcriptase or RNA were left out during the cDNA synthesis step. cDNA and PCR product synthesis was carried out in a thermal cycler (Mastercycler Eppendorf) at the following conditions: 5 min at 25°C, 60 min at 50°C; reaction termination was conducted for 15 min at 70°C.

For analysis of gene expression (RNA) probes against T1 R1, T1 R3 and mouse G$\alpha$15 cDNA (MGB TaqMan probes, Applied Biosystems) were used. For sample normalization control, the GAPDH, Pre-Developed TaqMaN Assay Reagents,Applied Biosystems was utilized. Reactions, including negative controls and positive controls (plasmid DNA), were set up in triplicates with 40 nG of cDNA in 50 uL reaction volume. The relative amounts of each of the umami taste receptor subunits being expressed (RNA) were determined. Figure 7 graphically depicts the results and indicates that all three nucleic acids were expressed (RNA) in the umami taste receptor cell line. The expression levels of T1 R1, T1 R3 and Gα15 were approximately 10,000x, 100x, and 100,000x higher than the levels observed in control cells, respectively.

Standard single endpoint RT-PCR procedures were used to assess T1 R1, T1 R3 and Gα15 gene expression (RNA) in one and nine month cultures of the umami taste receptor cell line generated according to the protocol described above ("Final"). Cells were grown in a 24-well plate format to 80% confluency and harvested and RNA was isolated using a commercially available RNA preparation kit (RNAqueous kit, Ambion). A range of 5pg to 5 ug of purified total RNA was used to perform reverse transcription, according to the protocol of a commercial first strand cDNA synthesis kit (Superscript III kit, Invitrogen), with oligo (dT) 12,18 primers. Following first strand synthesis, oligo sets specific for the subunits of the umami taste receptor nucleic acids (T1 R1, T1 R3), as well as for the mouse Gα15 nucleic acids, were independently assembled in PCR reaction mixtures (HotStart Taq). Following a 45 cycle PCR, amplicon samples were further analyzed by agarose gel electrophoresis.

The results from these single-endpoint RT-PCR experiments are illustrated in Figure 8. Figure 8 depicts representative photographs of agarose gels used in the RT-PCR experiments. Robust expression (RNA) of all umami taste receptor encoding nucleic acids was detected in both one month and older nine month cultures, demonstrating an exceptional level of stability for a cell line grown under "Final" conditions.

2. Cell-Based Assay For Modulators

Cells are seeded (75-125K) per well 24 hours prior to assay in 96 well plates in growth media (Low Glucose DMEM or L15 media, the media being supplemented with serum and standard growth additives). Following incubation, growth media is removed and the cells are placed in growth serum-free media. Cells are incubated for 2-3 hours. The media is then removed and the cells are loaded with a calcium-sensitive fluorescent dye (Calcium-3, Molecular Devices Corp.) which is diluted in umami assay buffer (130mM NaCl, 1.1 mM$KH_2PO_4$, 1.3mM CaCl, 20mM HEPES and 3mM$NaHPO_4$*$7H_20$). Cells are incubated in this media for 1 hour. Plates are loaded onto a high throughput fluorescent plate reader (Hamamatsu FDSS). Test compounds are diluted in umami assay buffer to the desired concentration and added to each well. Calcium flux is detected for 90 seconds. Activators (*i.e.* MSG) diluted in buffer as above are added to each well in final concentrations ranged between 10 uM and 100 mM and change in relative fluorescence is recorded for an additional 90 seconds.

3. Determination of Z' and $EC_{50}$ Values for Umami Cell-Based Assay

In order to test the effectiveness of the umami taste receptor response in these umami taste receptor-expressing cell lines, the established umami taste receptor agonist monosodium glutamate (MSG) is utilized as a test compound in the assay described above. MSG (Sigma, G5889) is added to test wells at a concentration of 33mM, and control wells receive buffer alone. In the final assay conditions, as reported by calcium flux measurements which are measured by a fluorescence plate reader. (FLIPR3 operating system, Molecular Devices). In one several times repeated assay the cell line had a Z' value of 0.8. See Figure 9 (one illustrative assay). This Z' value indicates that the generated umami taste receptor-expressing cells recognize MSG in a cell-based assay, and that these assays can be performed reliably and robustly using these cells.

In order to test the sensitivity of the umami taste receptor response in an umami taste receptor-expressing cell line disclosed herein, a dose response experiment is performed by adding increasing doses of MSG to cells and measuring the responses as described above. In one assay (Figure 10), it was found that the $EC_{50}$ value for MSG in this cell line was 22mM. These results indicate that the umami receptors produced in the cell lines disclosed herein exhibit strong sensitivity to a known umami receptor ligand in the umami receptor-expressing cell lines disclosed herein.

**Example 25 Potentiation of umami cell line response to MSG by known potentiators IMP and sodium cyclamate (Reference Example)**

As mentioned above, MSG is a known ligand of the umami taste receptor. It has also been demonstrated that the nucleotide inosine monophosphate (IMP, Sigma) can serve as a potentiator of umami taste receptor signaling when presented together with MSG. Using the assay protocol described above, a matrix of increasing MSG and increasing IMP concentrations are applied to the cell lines and responses are generated. Figure 11 shows for one such assay that as the IMP concentration increases, stronger responses are detected at each concentration of MSG tested.

Similar to MSG, sodium cyclamate, an artificial sweetener, can serve as an activator of the umami taste receptor as it interacts with the subunit common to both sweet and umami receptors. Using the assay protocol described above, a matrix of increasing cyclamate (Sigma) concentrations are applied and responses are generated. Figure 12 shows, for one assay, that as the cyclamate concentration increases, more significant responses are detected. This is contrast to several other cell lines described in the prior art, which do not detect cyclamate without the presence of MSG.

**Example 26 Venerating a stable bitter receptor-expressing cell line (Reference Example)**

***Step 1- Transfection***

293T cells were cotransfected with two separate plasmids, one encoding a human TAS2R bitter receptor (one of SEQ ID NOS: 77-101), and the other encoding a mouse G$\alpha$15 signaling protein (SEQ ID NO: 102). As will be appreciated by those of skill in the art, any reagent that is suitable for use with a chosen host cell may be used to introduce a nucleic acid, *e.g.,* a plasmid, oligonucleotide, or labeled oligonucleotide, into a host cell with proper optimization. Examples of reagents that may be used to introduce nucleic acids into host cells include, but are not limited to, Lipofectamine, Lipofectamine 2000, Oligofectamine, TFX reagents, Fugene 6, DOTAP/DOPE, Metafectine, and Fecturin. Although drug selection is optional in the methods disclosed herein, we included one mammalian drug resistance marker per plasmid. Plasmids used a CMV promoter for expression of the bitter receptor gene or the G$\alpha$15 gene. An untranslated sequence encoding a tag for detection by a signaling probe was also present in each vector, along with the sequence encoding the drug resistance marker, so that the tag was transcribed along with the protein the vector expressed, *i.e.,* bitter receptor or G$\alpha$15. The target sequences utilized were Target Sequence 1 (SEQ ID NO: 46), and Target Sequence 2 (SEQ ID NO: 47). In these examples, the TAS2R gene-containing vectors contained Target Sequence 1, and the G$\alpha$15 gene-containing vector contained Target Sequence 2.

***Step 2 - Selection step***

Transfected cells were grown for 2 days in Dulbecco's Modified Eagle Medium (DMEM) containing fetal bovine serum (FBS), followed by two weeks in antibiotic-containing DMEM-FBS. The antibiotic containing period had antibiotics added to the media as follows: Puromycin (0.15 ug/ml) and Hygromycin (100 ug/ml).

***Step 3 - Cell passaging***

Following enrichment on antibiotic, and prior to introduction of fluorogenic signaling probes, cells were passaged 8 (p5-p13) more times in the absence of antibiotic selection to allow time for expression that is not stable over the selected period of time to subside.

***Step 4 - Exposure of cells to fluorogenic signaling probes***

Cells were harvested and transfected with signaling probes (SEQ ID NOS: 48 and49). As will be appreciated by those of skill in the art, any reagent that is suitable for use with a chosen host cell may be used to introduce a nucleic acid, e.g. plasmid, oligonucleotide, labeled oligonucleotide, into a host cell with proper optimization. Examples of reagents that may be used to introduce nucleic acids into host cells include but are not limited to: Lipofectamine, Lipofectamine 2000, Oligofectamine, TFX reagents, Fugene 6, DOTAP/DOPE, Metafectine, or Fecturin.

**Target Sequences detected by signaling probes**

**Target 1**

5'-GTTCTTAAGGCACAGGAACTGGGAC-3' (SEQ ID NO: 46)

Target 2

5'-GAAGTTAACCCTGTCGTTCTGCGAC-3' (SEQ ID NO: 47)

A similar probe using a Quasar Dye (BioSearch) with spectral properties similar to Cy5 was used in certain experiments. Note also that 5-MedC and 2-aminodA mixmer probes rather than DNA probes were used in some instances.

A scrambled nontargeting fam probe was used as a delivery control (not shown).

**Signaling probes**

The signaling probes were upplied as 100$\mu$M stocks.

**Signaling probe** 1 - binds Target 1

**5' - Cy5** GCCAGTCCCAGTTCCTGTGCCTTAAGAACCTCGC **BHQ3 quench -3'** (SEQ ID NO: 48)

**Signaling probe 2 -** binds Target 2

**5'- Cy5.5** GCGAGTCGCAGAACGACAGGGTTAACTTCCTCGC **BHQ3 quench -3'** (SEQ ID NO: 49)

Note that BHQ3 could be substituted with BHQ1 or a gold particle in Probe 1 or Probe 2.

BHQ3 could be substituted with BHQ2 or a gold particle in Probe 1 or Probe 2.

***Step 5 - Isolation of positive cells***

The cells were dissociated and collected for analysis and sorting using a fluorescence activated cell sorter (Beckman Coulter, Miami, FL). Standard analytical methods were used to gate cells fluorescing above background and to isolate individual cells falling within the gate into barcoded 96-well plates. The gating hierarchy was as follows: coincidence gate > singlets gate > live gate > sort gate. With this gating strategy, the top 0.1-1.3% of double-positive cells were marked for sorting into barcoding 96-well plates.

***Step 6 - Additional cycles of steps 1-5 and/or 3-5***

The experiments were staged with very tightly timed logistics. As part of this campaign, there were two full cycles of steps 3-5 performed in order to have redundant sorts completed and additional clones obtained.

***Step 7 - Estimation of growth rates for the populations of cells***

The plates were transferred to a Hamilton Microlabstar automated liquid handler. Cells were incubated for up to 20 days in a 1:1 mix of 2-3 day conditioned growth medium: fresh growth medium (DMEM/10% FBS) supplemented with 100 units penicillin/ml plus 0.1mg/ml streptomycin. Plates were imaged between 7 and 20 days postsort to determine confluency of wells (Genetix). Each plate was focused for reliable image acquisition across the plate. Reported confluencies of greater than 70% were not relied upon. Confluency measurements were obtained 3 times over the time specified above and used to calculate growth rates.

***Step 8 - Binning populations of cells according to growth rate estimates***

Cells were binned (independently grouped and plated as a cohort) according to growth rate approximately 20 days postsort. Bins were independently collected and plated on individual 96 well plates for downstream handling, and there could be more than one target plate per specific bin. Bins were calculated by considering the spread of growth rates and bracketing a range covering a high percentage, at least about 90% (as an estimate) of the total number of populations of cells. 5 growth bins were used with average partitions of 1.2-3.5 days over the bins across the different bitter receptor cell lines. Therefore each bin corresponded to a growth rate or population doubling time difference of approximately 11 hours.

Cells can have doubling times from less than 1 day to more than 2 weeks - in order to process the most diverse clones that at the same time can be reasonably binned according to growth rate, we typically prefer to do 3-9 bins with a 0.25 to 0.7 day doubling time per bin. One skilled in the art would understand how to adjust the tightness of the bins and number of bins according to the particular situation, and tightness and number of bins could be further adjusted if cells were synchronized for their cell cycle

***Step 9 - Replica plating to speed parallel processing and provide stringent quality control***

The plates were incubated under standard and fixed conditions (humidified 37, 5% $CO_2$/95% air) in DMEM media/10% FBS without antibiotics. The plates of cells were split to produce 4 sets (the set consists of all plates with all growth bins - these steps ensured there were 4 replicates of the initial set) of target plates. Up to 3 target plate sets were committed for cryopreservation (see below), and the remaining set was scaled and further replica plated for passage and for functional assay experiments. Distinct and independent tissue culture reagents, incubators, personnel and carbon dioxide sources were used for each set of plates. Quality control steps were taken to ensure the proper production and quality of all tissue culture reagents: each component added to each bottle of media prepared for use was added by one designated person in one designated hood with only that reagent in the hood while a second designated person monitored to avoid mistakes. Conditions for liquid handling were set to eliminate cross contamination across wells. Fresh tips were used for all steps or stringent tip washing protocols were used. Liquid handling conditions were set for accurate volume transfer, efficient cell manipulation, washing cycles, pipetting speeds and locations, number of pipetting cycles for cell dispersal, and relative position of tip to plate.

***Step 10 - Freezing early passage stocks of populations of cells***

Three sets of plates were frozen at -70 to -80°C. Plates in each set were first allowed to attain confluencies of 70 to 100%. Media was aspirated and 90% FBS and 10% DMSO was added. The plates were sealed with Parafilm and then individually surrounded by 1 to 5cm of foam and placed into a -80°C freezer.

***Step 11- Methods and conditions for initial transformative steps to produce stable cell lines***

The remaining set of plates were maintained as described above in step 9. All cell splitting was performed using liquid handling steps, including media removal, cell washing, trypsin addition and incubation, quenching and cell dispersal steps.

***Step 12 - Testing of the functionality of cells in re-arrayed plates***

Due to the scale of the receptor cell panel, no normalization was used - instead the growth re-arrayed plates were quickly tested for functionality as the first priority (between 3-5 passages post-rearray), and a subset population of responders for each of the 25 bitter receptors was identified.

***Step 13 - Characterization of population of cells***

Clones were screened between 3.5 and 6 weeks post-sort with top clones being retested functionally and identified 5 to 6 weeks post-sort. The cells were maintained for up to 6 weeks to allow for their *in vitro* evolution under these conditions. During this time, we observed size, morphology, fragility, response to trypsinization or dissociation, roundness/average circularity post-dissociation, percentage viability, tendency towards microconfluency, or other aspects of cell maintenance such as adherence to culture plate surfaces.

***Step 14 - Assessment of potential functionality of populations of cells***

Populations of cells were tested using functional criteria. Calcium mobilization dye kits (Calcium 3, Molecular Devices/MDS) were used according to manufacturer's instructions. Cells were tested at multiple different densities in 96 or 384-well plates and responses were analyzed. A variety of time points post plating were used, for instance 12-48 hours post plating. Different densities of plating were also tested for assay response differences.

The functional responses from experiments performed at low and higher passage numbers were compared to identify cells with the most consistent responses over defined periods of time, ranging from 6 to 11 weeks postsort. Other characteristics of the cells that changed over time were also noted, for example, time for cells to reattach post-dissociation.

***Step 16 - Further evaluation of cells***

Populations of cells meeting functional and other criteria were further evaluated to determine those most amenable to production of viable, stable and functional cell lines. Selected populations of cells were expanded in larger tissue culture vessels and the characterization steps described above were continued or repeated under these conditions. At this point, additional standardization steps were introduced for consistent and reliable passages. These included different plating cell densities, plate coatings, time of passage, culture dish size/format and coating, fluidics optimization, cell dissociation optimization (e.g., dissociation in the presence of Cell Dissociation Buffer (Invitrogen) vs trypsin), volume of dissociation reagent used, and length of time of dissociation), as well as washing steps. Glutamine concentrations were dose ranged for the culture medium. Also, viability of cells at each passage was determined. Manual intervention was increased and cells were more closely observed and monitored. This information was used to help identify and select final cell lines that retained the desired properties Final cell lines and back-up cell lines were selected that showed consistent growth, consistent *(i.e.* unchanging morphology) appropriate adherence, as well as functional response.

***Step 17- Establishment of cell banks***

The low passage frozen plates (see above) corresponding to the final cell line and back-up cell lines were thawed at 37°C, washed two times with DMEM/10% FBS and incubated in humidified 37°C /5% $CO_2$ conditions. The cells were then expanded for a period of 2-3 weeks. Cell banks for each final and back-up cell line consisting of 25-50 vials were established. Cells were cryopreserved in 50% DMEM/10% FBS, 40% FBS, and 10% DMSO, resulting from further optimization experiments.

***Step 18 - Testing of cell bank***

At least one vial from the cell bank including the frozen stocks of the thawed cell lines, expanded and re-frozen, was thawed and expanded in culture. The resulting cells were tested to confirm that they met the same characteristics for which they were originally selected.

**Example 27 Characterizing the cell line for native bitter receptor function** (Reference Example)

In order to identify and measure the ligand-induced responsiveness of bitter receptor-expressing HEK293 cells, receptor activation was monitored by measuring receptor-triggered alterations in intracellular calcium levels. Cells were either grown overnight in black-clear bottom plates in standard growth media or added to the black-clear bottom plates as a suspension in assay buffer (10 mM HEPES, 130 mM NaCl, 2 mM $CaCl_2$, 5 mM KCl, 1.2 mM $MgCl_2$, 10 mM glucose, pH 7.4). The cells were incubated for 1 hr at room temperature with the no-wash calcium-sensitive fluorescent dye Calcium-3 (Molecular Devices Corp.) in buffer. The cell plates and test compounds (0.01 $\mu$M -100 mM) were placed in the high throughput fluorescent plate reader (Hamamatsu FDSS) which collected images of the plate fluorescence before, during and after the instrument added test compounds to the cells. Software (Hamamatsu FDSS) analysis of images reported the change in relative fluorescence for each well in the cell plate.

A variety of compounds and extracts, many of which were reported as bitter-tasting, were assayed across the 25 bitter receptor cell lines as well as control cells in order to determine activity and to deorphan.

**Example 28 Functional difference between transiently transfected native and tagged receptors (Reference Example)**

Transient transfection of native and tagged receptors followed by assay allowed the analysis of the functional difference between native and tagged receptors. As an example, functional assays were performed in human embryonic kidney (HEK) 293T cells 48 hours after transient transfection of the mouse G$\alpha$15 protein and either a native T2R16 bitter receptor or the same receptor carrying an N-terminal tag from the mouse rhodopsin gene. The cells were incubated for 1 hr at room temperature with the no-wash calcium-sensitive fluorescent dye Calcium-3 (Molecular Devices Corp.) in buffer (10 mM HEPES, 130 mM NaCl, 2 mM CaCl2, 5 mM KCL, 1.2 mM MgCl2, 10 mM glucose, pH 7.4). The assay response for bitter receptor activity was measured in a Hamamatsu FDSS fluorescent plate reader which collected images of the plate fluorescence before, during and after the instrument added a range of concentrations (0.01 uM - 100 uM) of a bitter extract.

The FDDS software analyzed images and reported the change in relative fluorescence for each well in the cell plate.

The results were plotted in FIG. 13. As shown in FIG. 13, the bitter extract selectively activated the native bitter receptor to a greater degree than the rhodopsin tag-containing receptor. This result indicated that native and tagged human bitter receptors show distinct functional activity, and emphasized the importance of expressing native protein and physiologically relevant cell based assays for proper receptor assignments and deorphaning.

**Example 29 High success rate of generation of functional clones of bitter receptors (Reference Example)**

The success rate (number of clones expressing functionally active receptors over all clones isolated) is a critical parameter in determining the efficiency of generating stable cell lines. We performed functional assays of clones we isolated using the method described in Example 26, and found that greater than 80% of the clones isolated were functionally active.

As an example, individual clonal cell lines isolated for expression of specific human bitter receptor T2R41 were cultured in individual wells of a 96-well plate, and the presence of viable cells in each well was confirmed by initial calcium-sensitive dye load measurement. Wells with no or low dye loading were considered blank and indicated in black in FIG. 14. Cells were then tested for functional bitter receptor response to the addition of a bitter extract, read out by receptor-mediated calcium mobilization and monitored by changes in dye fluorescence. Wells containing cells but showing less than 2-fold increase in signal above initial fluorescence were considered negative and are indicated in white in FIG. 14, whereas those with signal greater than 2-fold increase are indicated in gray. In this plate, out of 64 of clones isolated for expression of this bitter receptor gene, 57 clones (89%) showed a significant functional bitter receptor response.

**Example 30 Functional real-time imaging of bitter-receptor response** (Reference Example)

Homogeneity is another important parameter in generating stable cell lines. We used functional real-time imaging of bitter receptor response to determine the homogeneity of cells isolated using the method described in Example 26, as compared to those found by drug selection only. Cells expressing a bitter receptor and G protein were plated onto 96-well poly-D-lysine coated black-clear plates (Becton Dickinson) 24 hours prior to the assay. Cells were loaded with a calcium-sensitive fluorescent dye diluted in assay buffer (130 NaCl, 2mM CaCl, 1.2mM MgCl, 5mM KCl,10mM glucose, 10mM HEPES at pH 7.4). Cells were incubated for 1 hour and then a known activator of the bitter receptor was used at an appropriate concentration. Calcium flux responses of cells were recorded using an AxioVert 200 EPI-fluorescent microscope (Zeiss) with appropriate filters for 3 minutes. Data were analyzed using MetaMorph6.3r7 software (Molecular Devices).

Homogeneous bitter receptor responses were detected in an isolated clonal cell line (FIG. 15, upper photos), as indicated by increase in intracellular free calcium resulting from bitter receptor activation. In similarly treated cultures of drug selected cells, large heterogeneity in assay response was observed following application of the same activator (FIG. 15, lower photos). This result showed that the method of generating bitter receptor-expressing cell lines disclosed herein (*i.e.*, Example 26) could select a cell population that was genetically and functionally consistent.

**Example 31 Uniformity of functional response in bitter receptor-expressing cell lines (Reference Example)**

Because bitter receptors are G protein coupled receptors (GPCRs), in order to make meaningful comparison of bitter responses between different bitter receptors in the presence of various compounds, it is important to determine that the G protein coupling functions uniformly in different cell lines. The relative responses of the different cell lines across a range of concentrations of a G protein coupling agonist should be uniform. To confirm this, we tested the uniformity in G$\alpha$15-mediated receptor responses of all 25 cell lines expressing the different human bitter receptors and the mouse G$\alpha$15 protein in dose response curve experiments with varying concentrations of isoproterenol, an agonist of the $\beta$-adrenergic receptor endogenously expressed in the cells. This endogenous receptor, when stimulated, can couple to the expressed G$\alpha$15 and evoke a change in intracellular free calcium. Percent response was plotted as a function of isoproterenol concentration, and the results were curve-fitted to calculate an EC50 value (concentration of half-maximal receptor activation) (see FIG. 16). Comparing across all 25 discrete clonal bitter cell lines, this EC50 value was found to be 4.9 $\pm$ 0.41 nM, in close agreement with published literature values, and showing remarkably little deviation between the cell lines.

**Example 32 Identification of functionally responsive broadly tuned, moderately tuned, and selective receptors (Reference Example)**

Cell lines stably expressing native human bitter receptor genes, generated using the method disclosed herein (*i.e.*, Example 26), were tested in functional cell-based receptor studies to detect their activation by a collection of chemically diverse compounds. Substances 1-12 eliciting dose-dependent activation of the corresponding human bitter receptor are listed alongside the receptor which they were found to activate in FIG. 17. This allowed the identification of broadly tuned receptors, less broadly tuned, and narrowly tuned receptors.

**Example 33 Identification of receptors activated by a library of compounds** (Reference Example)

The activity of a library of compounds against each of the 25 human bitter receptor cell lines was tested. Data were generated in functional cell-based assays for receptor activity upon addition of the compounds, and expressed as percent activity above the basal activity of the receptor *(i.e.,* receptor activity upon addition of buffer alone). Activity of each compound at each receptor was then coded to highlight compounds with low (<100% above basal activity; white), medium (101-500% above basal activity; light gray), high (501-1000% above basal activity; dark gray), or very high (>1001 % above basal activity, black) activity at each receptor (FIG. 18). The resulting pattern provides a graphical representation of compounds active either selectively or broadly across bitter receptors (rows), and of receptors showing either broad or selective response (columns) to a chemically diverse set of compounds.

**Example 34 Identification of analogs of compounds with similar functional activities as antagonists of agonist-induced bitter receptor activity** (Reference Example)

To test the ability of the bitter receptor-expressing cell-based assays to identify analogues of compounds with similar functional activities, twenty-one structural analogues of a known bitter blocking compound are tested for their ability to inhibit an agonist-induced receptor activity of a bitter receptor. The structures of analogues that inhibit the bitter receptor's activity induced by the agonist are compared. Thus, the bitter receptor-expressing cell lines may aid discovery of potent bitter receptor antagonists.

**Example 35 Different receptor assignments using native cell lines and tagged cell lines (Reference Example)**

Functional assays following transient transfections described in Example 28 showed that native and tagged human bitter receptors had distinct functional activity. This was also confirmed using cell lines expressing native bitter receptors as compared to cell lines expressing tagged bitter receptors. Briefly, bitter receptor assignments for saccharin were made using functional cell-based receptor assays measuring calcium mobilization with stable cell lines expressing native bitter receptors. Bitter receptor assignments for saccharin were also made as described in Pronin et al., "Identification of Ligands for Two Human Bitter T2R Receptors," Chem. Senses, 29:583-593 (2004), using insect-produced recombinant receptor proteins carrying N-terminal mouse rhodopsin tag sequences, in a membrane fraction-based cell free assay for GTP hydrolysis. The different assignments are shown in FIG. 19. The discrepancy between the bitter receptor assignments for the same bitter compound highlighted the essential requirement for native bitter receptors for physiologically relevant functional assignments.

## Example 36 Generating a stable sweet taste receptor-expressing cell line

Transfection

HEK293T (ATCC CRL-11268) were co-transfected with three separate plasmids, one encoding T1 R2 (SEQ ID NO: 31), one encoding T1 R3 (SEQ ID NO: 32) and the other encoding a signaling molecule (mouse G$\alpha$15, SEQ ID NO: 33). Although drug selection is optional in the methods of this invention, we included one drug resistance marker per plasmid. The sequences were under the control of the CMV promoter. An untranslated sequence encoding a tag for detection by a signaling probe was also present along with a sequence encoding a drug resistance marker. The target sequences utilized were Target Sequence 1 (SEQ ID NO: 28; using the tag sequence of SEQ ID NO:123), Target Sequence 2 (SEQ ID NO: 29; using the tag sequence of SEQ ID NO:124) and Target Sequence 3 (SEQ ID NO: 30; using the tag sequence of SEQ ID NO:125). In these examples, the T1 R2 gene vector contained Target Sequence 3, the T1 R3 gene vector contained Target Sequence 1 and the G$\alpha$15 gene vector contained the Target Sequence 2. The cells were conventionally selected in media containing the drug for 10-14 days.

Exposure of cells to fluorogenic probes

Selected cells were harvested and transfected with signaling probes (SEQ ID NO:38-40). Signaling Probe 1 binds Target Sequence 1, Signaling Probe 2 binds Target Sequence 2 and Signaling Probe 3 binds Target Sequence 3. The cells were then dissociated and collected for analysis and sorted using a fluorescence activated cell sorter (Beckman Coulter, Miami, FL).

As will be appreciated by those of skill in the art, any reagent that is suitable for use with a chosen host cell may be used to introduce a nucleic acid, e.g. plasmid, oligonucleotide, labeled oligonucleotide, into a host cell with proper optimization. Examples of reagents that may be used to introduce nucleic acids into host cells include but are not limited to: Lipofectamine, Lipofectamine 2000, Oligofectamine, TFX reagents, Fugene 6, DOTAP/DOPE, Metafectine, or Fecturin.

**Signaling probe 1**

**5' - Cy5** GCCAGTCCCAGTTCCTGTGCCTTAAGAACCTCGC **BHQ3 quench -3'** (SEQ ID NO: 38)

**Signaling probe 2**

**5'- Cy5.5** GCGAGTCGCAGAACGACAGGGTTAACTTCCTCGC **BHQ13 quench -3'** (SEQ ID NO: 39)

**Signaling probe 3**

**5'- Fam** GCGAGAGCGACAAGCAGACCCTATAGAACCTCGC **BHQ1 quench -3'** (SEQ ID NO: 40)

Tag Sequence 1 with target sequence in bold (sweet)

AGGGCGAATTCCAGCACACTGGCGGCCGTTACTAGTGGATCCGAGCTCGGAG GCAGGTGGACAGGAAGGTTCTAAT**GTTCTTAAGGCACAGGAACTGGGAC**ATCT GGGCCCGGAAAGCCTTTTTCTCTGTGATCCGGTACAGTCCTTCTGC (SEQ ID NO: 123)

Tag Sequence 2 with target sequence in bold (sweet)

AGGGCGAATTCCAGCACACTGGCGGCCGTTACTAGTGGATCCGAGCTCGGTAC CAAGCTTCGAGGCAGGTGGACAGCTTGGTTCTAAT**GAAGTTAACCCTGTCGTT CTGCGAC**ATCTGGGCCCGGAAAGCGTTTAACTGATGGATGGAACAGTCCTTCT GC (SEQ ID NO: 124)

Tag Sequence 3 with target sequence in bold (sweet)

AAGGGCGAATTCGGATCCGCGGCCGCCTTAAGCTCGAGGCAGGTGGACAGGA AGGTTCTAAT**GTTCTATAGGGTCTGCTTGTCGCTC**ATCTGGGCCCGGAGATG (SEQ ID NO: 125)

Other target sequences and signaling probes could be used (see, e.g., as described in International Patent Application Publication No. WO2005/079462 published on September 1, 2005 (Application No. PCT/US05/005080). For example, BHQ3 could be substituted with BHQ1 or a gold particle in Probe 1 or Probe 2. Note that BHQ1 could be substituted with BHQ2 or Dabcyl in Probe 3. A similar probe using a Quasar Dye (BioSearch) with spectral properties similar to Cy5 could be used in certain experiments. Note also that 5-MedC and 2-aminodA mixmer probes rather than DNA probes could be used in some instances. Isolation of positive cells

Standard analytical methods were used to gate cells fluorescing above background and to isolate cells falling within that defined gate directly into 96-well plates. Cell sorting was operated such that a single cell was deposited per well. After selection, the cells were expanded in media lacking drug.

Functional transformation

We maintained thesweet taste receptor cells (selected and expanded as above) using both aliquots of the same cells and different cells in various growth conditions including, for example, low-glucose DMEM media or in glucose-free Leibovitz L-15 media with 10% serum or serum- free. Some cells were maintained in media containing various concentrations of other sugars such as galactose. We then characterized cells of the sweet taste receptor cell lines maintained under multiple conditions for their ability to respond appropriately to sweet ligands and not respond to other stimuli (MSG).

Growth in the various media conditions, without wishing to be bound by theory, may functionally transform the cells, e.g. due to changes in gene expression levels, genomic organization and functional expression of receptors at the cell surface. Parameters in the various media that were evaluated and shown to affect a cell's functional assay response include serum concentration (*i.e.* low serum concentrations and serum deprivation), sugar deprivation and assay plate coating (for example, poly D lysine and laminin). These results demonstrated that characteristics of the cells of the sweet taste receptor cell lines may be different when the cells are maintained in different media conditions. They also show that different cells selected by the flourogenic probes may have different characteristics under the same growth conditions. One example of our characterization of cells selected by the fluorogenic probes is shown in Figure 20. As shown in Figure 20, aliquots of the same cells grown in different conditions (1, 2 and Final) responded to umami (MSG) and sweet (fructose) ligands significantly differently. Cells grown in condition 1 were plated in serum deprived, low glucose media. Cells grown in condition 2 were plated overnight in low glucose media containing 10% serum, and were then switched to a serum-deprived media before the assay. Cells grown in conditions 1 or 2 were plated on coated plates (Corning #3665). "Final" conditions encompass high density growth on coated plates (Corning #3300) and in low glucose media with serum deprivation. Cells grown in condition 1 and condition 2 respond to umami agonist more than sweet agonist. In contrast, cells grown in the "Final" growth conditions respond robustly to fructose and not to MSG. Thus, these cells produced a sweet taste receptor that was physiologically and pharmacologically relevant.

**Example 37 Characterizing the cell line for native sweet taste receptor function**

1. Confirmation and Quantification of Gene Expression.

Using qRT-PCR, we determined the relative amounts (RNA) of each of the sweet taste receptor subunits being expressed in the above cells ("Final"). Total RNA was purified from 1-3x$10^6$ mammalian cells using a commercially available RNA purification kit (RNeasy Mini Kit,Qiagen). The RNA extract was then treated using a rigorous DNase treatment protocol (TURBO DNA-free Kit; Ambion). First strand cDNA synthesis was performed using a Reverse Transcriptase Kit (SuperScript III, Invitrogen) in 20 uL reaction volume with 1 uG DNA-free total RNA and 250 nG Random Primers (Invitrogen). Negative controls for this reaction included samples in which reverse transcriptase or RNA were left out during the cDNA synthesis step. cDNA and PCR product synthesis was carried out in a thermal cycler (Mastercycler Eppendorf) at the following conditions: 5 min at 25°C, 60 min at 50°C; reaction termination was conducted for 15 min at 70°C.

For analysis of gene expression (RNA) probes against T1 R2, T1 R3 and mouse Gα15 cDNA (MGB TaqMan probes, Applied Biosystems) were used. For sample normalization control, the GAPDH, Pre-Developed TaqMaN Assay Reagents,Applied Biosystems was utilized. Reactions, including negative controls and positive controls (plasmid DNA), were set up in triplicates with 40 nG of cDNA in 50 uL reaction volume. The relative amounts of each of the sweet taste receptor subunits being expressed (RNA) were determined. Figure 21 graphically depicts the results and shows that all three nucleic acids were expressed (RNA) in the sweet taste receptor cells. The expression levels of T1 R2, T1 R3 and Gα15 were approximately 100,000x, 10x, and 100,000x higher than the levels observed in control cells, respectively.

Standard single endpoint RT-PCR procedures were used to assess T1 R2, T1 R3 and Gα15 gene expression (RNA) in one and nine month cultures of the sweet taste receptor cells generated according to the protocol described above ("Final"). Cells were grown in a 24-well plate format to 80% confluency and harvested and RNA was isolated using a commercially available RNA preparation kit (RNAqueous kit, Ambion). A range of 5pg to 5 ug of purified total RNA was used to perform reverse transcription, according to the protocol of a commercial first strand cDNA synthesis kit (Superscript III kit, Invitrogen), with oligo (dT) 12,18 primers. Following first strand synthesis, oligo sets specific for the subunits of the sweet taste receptor nucleic acid (T1 R2, T1 R3), as well as for the mouse Gα15 nucleic acid, were independently assembled in PCR reaction mixtures (HotStart Taq). Following a 45 cycle PCR, amplicon samples were further analyzed by agarose gel electrophoresis.

The results from these single-endpoint RT-PCR experiments are illustrated in Figure 22. Figure 22 depicts representative photographs of agarose gels used in the RT-PCR experiments. Robust expression (RNA) of all sweet taste receptor encoding nucleic acids was detected in both one month and older nine month cultures, demonstrating an exceptional level of stability for a cell line of this invention grown under "Final" conditions.

2. Cell-Based Assay For Modulators

Forty-eight hours prior to assay, cells of this invention are seeded in growth media (Low Glucose DMEM or L15 media, the media being supplemented with serum and standard growth additives) at a high density in 10 cm dishes. Cells are dissociated from the culture matrix and are seeded 24 hours prior to assay in 96 well plates. Media removal is then followed by the addition of a calcium-sensitive fluorescent dye (Calcium-3, Molecular Devices Corp.) which is diluted in sweet assay buffer (130mM NaCl, 1.1mM$KH_2PO_4$, 1.3mM CaCl, 20mM HEPES and 3mM$NaHPO_4$*7$H_2O$). Cells are incubated in this media for 1 hour. Plates are then loaded on a high throughput plate reader (Hamamatsu FDSS). Test compounds (*i.e.* fructose (Sigma), sucrose (Sigma), glucose (Sigma), Acesulfame K (Fluka), Na-saccharin (Sigma), Na-cyclamate (Aldrich), steviva (Steviva Brands Inc.), mogroside (Slim Sweet, Trimedica), and sorbitol (Sigma)) are diluted in sweet assay buffer and added to each well. Calcium flux is detected for 90 seconds. Activators are diluted in buffer as above are added to each well in final concentrations ranged between 10 uM and 100 mM and change in relative fluorescence is recorded for an additional 90 seconds.

3. Determination of Z' and $EC_{50}$ Values for Sweet Cell-Based Assay

In order to test the effectiveness of the sweet taste receptor response in these sweet taste receptor-expressing cells, the established sweet taste receptor agonist fructose is utilized as a test compound in the assay described above. Fructose is added to test wells (odd columns) at a concentration of 75mM, and control wells (even columns) receive buffer alone. In the final assay conditions, as reported by calcium flux measurements which are measured by a fluorescence plate reader (FLIPR3 operating system, Molecular Devices). In one several times repeated assay the cells had a Z' value of 0.8. See Figure 23 (one illustrative assay). This Z' value indicates that the generated sweet taste receptor-expressing cells recognize fructose in a cell-based assay, and that these assays can be performed reliably and robustly using these cells.

In order to test the sensitivity of the sweet taste receptor response in a sweet taste receptor-expressing line of this invention, a series of dose response experiments were performed by adding increasing doses of various sweet receptor agonists to cells and measuring the responses as described above. In one assay (Figure 24) it was found that the $EC_{50}$ values for these tested compounds were as follows: less than 1.5mM for saccharin, less than 3.5mM for sucrose, less than 4.3mM for fructose and less than 34.1 mM for glucose. These results indicate that the sweet receptors produced in the cell lines of this invention exhibit strong sensitivity to a number of established sweet receptor ligands in the sweet receptor-expressing cell lines of this invention.

In this assay, it was also found that the calcium flux response curve was different when the sweet taste receptor-expressing cells were exposed to different sweeteners. As illustrated in Figure 25, the length and intensity of the response by the cells to different ligands varied. For example, steviva displayed a longer and more intense response than many of the other sweeteners tested. These results suggest the cells and cell lines of this invention are useful to assay for sweet lingering, an undesired continuation of sweet taste, seen with certain high intensity sweeteners such as steviva.

**Example 38 Generation and isolation of stable cell lines endogenously expressing at least one sweet receptor subunit**

Design of signaling probes

Signaling probes directed to sequences corresponding to the T1 R2 or T1R3 genomic loci were designed. The signaling probes were directed to coding exons, non-coding introns or non-coding untranslated sequences as described in Table 23.

Signaling probes S21, S22, S23, R2-3U1 and R2-I1 comprise a Cy5.5 fluorescent label at the 5' terminus and a BHQ2 quencher at the 3'terminus. Signaling probes S31, S32, S33, R3-3U1 and R3-I31 comprise a 6-FAM fluorophore at the 5' terminus and a BHQ1 at the 3' terminus. Signaling probes could also comprise other fluorophores or quenchers. Signaling probes were synthesized, conjugated to their respective labels and purified at Genelink (Hawthorne, NY).

**Table 23**

| Name | Probe sequence | Directed to | Target sequence |
|---|---|---|---|
| S21 | **5' - Cy5.5** GCGAGGAGACGGATGAG GTGAAATAGAAGCTCGC **BHQ2 quench – 3'** (SEQ ID NO: 103) | T1 R2 exon 6 | **5' -** CTTCTATTTCAC CTCATCCGTCTC **- 3'** (SEQ ID NO: 113) |
| S22 | **5' - Cy5.5** GCGAGATTGTGAAGCAG AGGGGAAAGAGGCTCGC **BHQ2 quench – 3'** (SEQ ID NO: 104) | T1 R2 exon 6 | **5' -** CCTCTTTCCCCT CTGCTTCACAAT **- 3'** (SEQ ID NO: 114) |
| S23 | **5' - Cy5.5** GCGAGACCTTCACTTCA TACTCCTTGCACCTCGC **BHQ2 quench – 3'** (SEQ ID NO: 105) | T1 R2 exon 1 | **5' -** GTGCAAGGAGTA TGAAGTGAAGGT **- 3'** (SEQ ID NO: 115) |
| R2-3U1 | **5' - Cy5.5** GCGAGCCTTGTCTCCCG CAATACCTTCACCTCGC **BHQ2 quench – 3'** (SEQ ID NO: 106) | T1 R2 3' untranslated region | **5' -** GTGAAGGTATTG CGGGAGACAAGG **- 3'** (SEQ ID NO: 116) |
| R2-I1 | **5' - Cy5.5** GCGAGCTTTCTCCTTGT CTCCCGCAATACCTCGC **BHQ2 quench – 3'** (SEQ ID NO: 107) | T1 R2 intron 1 | **5' -** GTATTGCGGGAG ACAAGGAGAAAG **- 3'** (SEQ ID NO: 117) |

(continued)

| Name | Probe sequence | Directed to | Target sequence |
|---|---|---|---|
| S31 | **5' – 6-FAM** GCGAGGTTTCCCCTGAT TTCCTGTGTTCCGTCGC **BHQ1 quench – 3'** (SEQ ID NO: 108) | T1 R3 exon 6 | **5' -** GGAACACAGGAA ATCAGGGGAAAC **- 3'** (SEQ ID NO: 118) |
| S32 | **5' – 6-FAM** GCGAGCACCCACAGCTT CAGGTCGTACTCCTCGC **BHQ1 quench – 3'** (SEQ ID NO: 109) | T1 R3 exon 4 | **5' -** GAGTACGACCTG AAGCTGTGGGTG **- 3'** (SEQ ID NO: 119) |
| S33 | **5' – 6-FAM** GCGAGGACCACTCATCC TGGCCACAAAAGCTCGC **BHQ1 quench – 3'** (SEQ ID NO: 110) | T1 R3 exon 6 | **5' -** CTTTTGTGGCCA GGATGAGTGGTC **- 3'** (SEQ ID NO: 120) |
| R3-3U1 | **5' – 6-FAM** GCCAGCCTGATTTGGGC TTCCAGCCATCTCTCGC **BHQ1 quench – 3'** (SEQ ID NO: 111) | T1 R3 3' untranslated region | **5' -** AGATGGCTGGAA GCCCAAATCAGG **- 3'** (SEQ ID NO: 121) |
| R3-I31 | **5' – 6-FAM** GCGAGGCAGAGGACAG CACACCCCCATCTCTCGC **BHQ1 quench – 3'** (SEQ ID NO: 112) | T1 R3 intron 3 | **5' -** AGATGGGGGTGT GCTGTCCTCTGC **- 3'** (SEQ ID NO: 122) |

Figures 26 and 27 depict a graphical representation of the T1 R2 and T1 R3 genomic loci and intron-exon coding structures. Other target sequences and signaling probes could be used (see, *e.g.*, as described in International Patent Application Publication No. WO2005/079462 published on September 1, 2005 (Application No. PCT/US05/005080). For example, BHQ1 could be substituted with BHQ2 or Dabcyl in Probe 3. Note also that 5-MedC and 2-aminodA mixmer probes rather than DNA probes could be used in some instances.

Exposure of cells to fluorogenic probes

Either HEK 293T (ATCC CRL-11268), HuTu (ATCC HTB-40) or H716 (ATCC CCL-251) cells were transfected with one of the following combinations of signaling probes in separate experiments: a) S21 and S31, b) S21 and S32, c) S21 and S33, d) S22 and S31, e) S22 and S32, f) S22 and S33, g) S23 and S31, h) S23 and S32, i) S23 and S33, j) R2-3U1 and R3-3U1, k) R2-3U1 and R3-I31, l) R2-I1 and R3-3U1 and m) R2-I1 and R3-I31. HEK 293T cells were maintained in Dulbecco's Modified Eagle media (Sigma #D5796) containing 10% Fetal Bovine Serum (FBS) (Sigma #SAC1203C), 2mM L-Glutamine (Sigma G7513) and 10mM HEPES (Sigma H0887), HuTu cells were maintained in Eagle's Minimal Essential Media (Sigma #D5650) containing 10% FBS (Sigma #2442), 2mM L-Glutamine (Sigma #G7513) and 1 mM Sodium Pyruvate (Sigma #S8636) and H716 cells were maintained in Roswell Park Memorial Institute 1640 (Gibco #11875-093) containing 10% FBS (Sigma #F2442). As will be appreciated by those of skill in the art, any reagent suitable for use with a chosen host cell may be used to introduce a nucleic acid (e.g. plasmid, oligonucleotide, labeled oligonucleotide) into a host cell with proper optimization. Examples of reagents that may be used to introduce nucleic acids into host cells include but are not limited to: Lipofectamine, Lipofectamine 2000, Oligofectamine, TFX reagents, Fugene 6, DOTAP/DOPE, Metafectine, or Fecturin.

In different experiments, HEK 293T cells, HuTu cells and H716 cells either were treated with or without a mutagenic treatment (*e.g.* UV light) to induce random mutagenesis or gene-activation prior to introduction of fluorogenic probes as described below. Cells were resuspended in serum free media at 1.25 x $10^6$ cells per milliliter and exposed to UV radiation to deliver 1 Joule/$m^2$/s from a germicidal bulb (Sylvania) emitting mainly 254 nm wavelengths for 15 minutes. Other mutagenic treatments also could be used (*e.g.*, chemical mutagenesis by exposure to mutagens such as ethyl methane sulfonate (EMS)). Cells could be incubated with a final concentration (*e.g.*, 200 ug/ml) of ethyl methanesulfonate (EMS; Sigma) and incubated prior to their transfer to media lacking EMS.

The cells were then exposed to signaling probes, dissociated and collected for testing using a fluorescence activated cell sorter (Beckman Coulter, Miami, FL).

Isolation of positive cells

Standard analytical methods according to standard practice in the field of flow cytometry and familiar to one skilled in the art were used to gate, or select, positive fluorescing cells. Fluorescence activated cell sorting was used to isolate positive fluorescing cells falling within the desired gate, or having the desired fluorescence, directly into 96-well plates. Cell sorting parameters were set for the isolation of only one cell per well. Transfected cells associated with different absolute or relative fluorescence levels for at least one signaling probe were isolated by gating the top 1% of the cells with the highest signal intensity relative to the entire population.

In separate experiments, transfected cells associated with different absolute or relative fluorescence levels for at least one signaling probe were isolated by gating the top 0.1% of the cells with the highest signal intensity relative to the entire population.

Individual cells were isolated and maintained in media as described below. For each cell type, equal proportions of its respective conditioned and fresh media were combined for use. Conditioned media was prepared by culturing each cell type in its respective fresh media for two days, harvesting and then filtering the media. Individually isolated HEK 293T cells were isolated and maintained in Dulbecco's Modified Eagle media (Sigma #D5796) containing 10% FBS (Sigma #SAC1203C), 2mM L-Glutamine (Sigma G7513), 10mM HEPES (Sigma H0887) and a 1 to 50 dilution of penicillin/streptomycin solution (final concentration 100 units penicillin/100μG streptomycin per mL) (Sigma # P4458). Individually isolated HuTu cells were isolated and maintained in Eagle's Minimal Essential Media (Sigma #D5650) containing 10% FBS (Sigma #2442), 2mM L-Glutamine (Sigma #G7513), 1 mM Sodium Pyruvate (Sigma #S8636) and a 1 to 50 dilution of penicillin/streptomycin solution (final concentration 100 units penicillin/100μG streptomycin per mL) (Sigma # P4458). Individually isolated H716 cells were isolated and maintained in Roswell Park Memorial Institute 1640 media (Gibco #11875-093) containing 10% FBS (Sigma #F2442) and a 1 to 50 dilution of penicillin/streptomycin solution (final concentration 100 units penicillin/100μG streptomycin per mL) (Sigma # P4458).

Robotic cell culture methods were used to passage cells isolated and maintained in 96 well tissue culture plates. In particular, the MICROLAB STAR™ instrument was used to carry out a variety of automated cell culture tasks (e.g., removing media, replacing media, adding reagents, cell washing, removing wash solution, adding a dispersing agent, removing cells from a culture vessel, adding cells to a culture vessel and the like). Other cell culture methods, including manual methods, could also be used. Cells then were expanded into 10 cm tissue culture dishes and cultured using standard manual tissue culture techniques (e.g., removing media, washing cells, adding media).

Isolated cells were expanded in culture to give rise to populations of cells derived from an individually isolated cell. Individually isolated cells were expanded by culturing them in their respective media, as described below. HEK 293T cells were transferred for maintenance in Dulbecco's Modified Eagle media (Sigma #D5796) containing 10% FBS (Sigma #SAC1203C), 2mM L-Glutamine (Sigma G7513) and 10mM HEPES (Sigma H0887), HuTu cells were transferred for maintenance in Eagle's Minimal Essential Media (Sigma #D5650) containing 10% FBS (Sigma #2442), 2mM L-Glutamine (Sigma #G7513) and 1 mM Sodium Pyruvate (Sigma #S8636). H716 cells were transferred for maintenance in Roswell Park Memorial Institute 1640 media (Gibco #11875-093) containing 10% FBS (Sigma #F2442).

Functional testing

Populations of cells each resulting from the expansion of single isolated cells then were characterized for their ability to respond to a sweet ligand using functional cell-based assays. 15mM fructose was used in these experiments. Depending on the cell type, different conditions were used as described below.

Forty-eight hours prior to assay, cells were seeded in growth media (L15 media (Sigma #L5520) supplemented with 10% serum (Sigma #SAC1203C), 4mM L-Glutamine (Sigma G7513), 10mM HEPES (Sigma H0887), and a 1 to 50 dilution of penicillin/streptomycin solution (final concentration 100 units penicillin/100μG streptomycin per mL) (Sigma # P4458) at a high density, from 12 to 20 million cells, in 10 cm dishes. Cells were dissociated from the culture matrix and were seeded at 35,000 cells per well 24 hours prior to assay in 384 well plates. Media was removed after 18 to 24 hours. A calcium-sensitive fluorescent dye (Fluo-4, Invitrogen) diluted in sweet assay buffer (130mM NaCl, 1.1

$mMKH_2PO_4$, 1.3mM CaCl, 20mM HEPES and $3mMNaHPO_4*7H_2O$) was added then to each well. Cells were incubated in this media for 1 hour. Plates then were loaded on a high throughput plate reader (Hamamatsu FDSS). Sweet assay buffer with DMSO was added to each well for a final concentration of 0.2% DMSO. Calcium flux or assay response was detected prior to, during and following addition of sweet assay buffer containing fructose to each well for a final concentration of 15mM fructose. The assay can be optionally performed with a quencher of the calcium-sensitive fluorescent dye. Such Quenchers are well-known in the art and include, *e.g.,* Dipicrylamine (DPA), Acid Violet 17 (AV17), Diazine Black (DB), HLB30818, trypan blue, Bromophenol Blue, HLB30701, HLB30702, HLB30703, Nitrazine Yellow, Nitro Red, DABCYL (Molecular Probes), QSY (Molecular Probes), Metal ion quenchers (e.g., $Co^{2+}$, $Ni^{2+}$, $Cu^{2+}$), and Iodide ion.

Alternatively, cells were dissociated from the culture matrix and were seeded at cell densities from 2,500 to 20,000 per well for 24 or 48 hours prior to assay in Eagle's Minimal Essential Media (Sigma #D5650) containing 10% FBS (Sigma #2442), 2mM L-Glutamine (Sigma #G7513) and 1 mM Sodium Pyruvate (Sigma #S8636) or a mixture of equal parts of this media and L-15 media (Sigma, L5520) in a 384 well plate. After 18 to 52 hours, the media was removed. A calcium-sensitive fluorescent dye (Fluo-4, Invitrogen) diluted in sweet assay buffer (130mM NaCl, $1.1mMKH_2PO_4$, 1.3mM CaCl, 20mM HEPES and $3mMNaHPO_4*7H_2O$) was added then to each well. Cells were incubated in this media for 1 hour. Plates then were loaded on a high throughput plate reader (Hamamatsu FDSS). Sweet assay buffer with DMSO was added to each well for a final concentration of 0.2% DMSO. Calcium flux or assay response was detected prior to, during and following addition of sweet assay buffer containing fructose to each well for a final concentration of 15mM fructose.

Alternatively, cells were pelleted and resuspended in calcium-sensitive fluorescent dye (Fluo-4, Invitrogen) diluted in sweet assay buffer (130mM NaCl, 1.1 $mMKH_2PO_a$, 1.3mM CaCl, 20mM HEPES and $3mMNaHPO_4*7H_2O$). Cells were plated in 384 well plates and incubated in this media for 1 hour. Plates then were loaded on a high throughput plate reader (Hamamatsu FDSS). Sweet assay buffer with DMSO was added to each well for a final concentration of 0.2% DMSO. Calcium flux or assay response was detected prior to, during and following addition of sweet assay buffer containing fructose to each well for a final concentration of 15mM fructose.

Other sweet ligands including high-intensity natural and artificial sweeteners and natural and artificial sweeteners could also be used, for examples sucrose (Sigma), glucose (Sigma), Acesulfame K (Fluka), Na-saccharin (Sigma), Na-cyclamate (Aldrich), steviva (Steviva Brands Inc.), mogroside (Slim Sweet, Trimedica), and sorbitol (Sigma)). In addition, other taste ligands including bitter, umami, fat, cool, hot and sour ligands or tastants, as well as compounds with no known or intrinsic taste could be used to determine selectivity and specificity of the sweet response. In addition, any of the tastants or ligands could be tested in the presence of additional compounds including enhancers or blockers, for instance the sweet enhancer Rebaudioside-C RP44 (RedPoint Bio) and the sucralose enhancer S2383 (Senomyx), and the Sucrose Enhancer S5742 (Senomyx).

In addition, other media and incubation steps could be used and other plate formats, for instance 96 well plates and 1536 well plates could be used.

Numerous cell populations cultured from individually isolated cells and found to express, naturally and/or by gene-activation, at least one subunit of the sweet taste receptor were identified as having a response to fructose by monitoring the response or activation kinetics of the cells in the functional fluorescent calcium flux reporter assay. See, Figure 28. Figure 28A shows HuTu cells responding to fructose. Figure 28B shows H716 cells responding to fructose. Figure 28C shows 293T cells responding to fructose.

<u>High throughput screening</u>

Sweet agonists and/or modulators can be identified by using high throughput screening (HTS) to test the effects of compounds on cells plated according to the above methods. Sweet assay buffer is added to each well with, or without, one or more components or compounds of a natural or synthetic compound library, or extracts or extract fractions. DMSO or other organic solvents such as ethanol also are included to assist in compound or extract solubilization for a final concentration of less than 5% DMSO(e.g., instance 0.2%). Calcium flux is detected for a period of time (e.g., 90 seconds). Fructose, or another sweet ligand, diluted in sweet assay buffer is then added to each well for a final activating concentration (*e.g.,* 15mM). Change in relative fluorescence is recorded for an additional period of time (e.g., 90 seconds). A number of control compounds also will be tested.

This testing is repeated until part or all of a compound library is screened. The data is analyzed to identify active compounds. Agonists and modulators including blockers, potentiators, enhancers or allosteric modulators are identified using this or other assay protocols. Testing of compounds using different sweet ligands are used to identify compounds that modulate the response of the sweet receptor to just one, all or a subset of sweet ligands, for instance compounds that are selective or pan-active. This is done by independent high throughput screening of the same compounds or compound libraries but using one or more different sweet ligands in each screen.

By examining temporal kinetics of the response curve (e.g., rate of decay or magnitude or response), modulating compounds with other qualitative and quantitative effects are identified.

Taste testing

Compounds identified using the sweet cell based assay are tested in human sensory tests according to standard practice in the field and familiar to one skilled in the art. Compounds are tested for their intrinsic taste characteristics *(e.g.* sweet, bitter, umami, salty, sour, cool or hot tastes and off-tastes) as well as other qualities or properties that can be perceived such as mouthfeel, numbing, pain, irritation and tingle. Testing is performed in one or more different formats or matrices including liquids, semi-solids, solids, powders, tablets, gels, gums, sprays, dissolvable strips, food or beverage product formulation. Samples with and without compound are evaluated to detect the sensory properties of the compounds. Different doses of the compounds may be tested. In certain experiments, compounds are tested in samples that additionally comprise other compounds or ingredients having a taste and the change, alteration, enhancement, potentiation or inhibition of the perception of such taste is assessed. The compound may be provided in the presence of an agent desiged to solubilize it, including an alcohol such as ethanol, DMSO or other solvent. The samples may be heated, chilled or tested at room temperature. Compounds also are tested for their ability to enhance or block sweet or other tastes. Sensory testing is performed with or without nose-clips to identify any odor-related effects. Taste testing is performed using the "sip and spit" mode where low concentrations of compounds are used to limit human exposure. Compounds are tasted to determine their effect on the perception of one or more sweet ligands and sweeteners. Human or animal subjects being exposed to or evaluating the compounds may be studied using functional brain imaging or other imaging tests (e.g., magnetic resonance imaging) to correlate patterns of activity in vivo with a compound.

**Example 39 Generating a stable odorant receptor-expressing cell line (Reference Example)**

***Step 1- Transfection***

293T cells were cotransfected with two separate plasmids, one encoding a human odorant receptor (one of SEQ ID NOS: 134-135), and the other encoding a human G$\alpha$15 signaling protein (SEQ ID NO: 133). As will be appreciated by those of skill in the art, any reagent that is suitable for use with a chosen host cell may be used to introduce a nucleic acid, e.g., a plasmid, oligonucleotide, or labeled oligonucleotide, into a host cell with proper optimization. Examples of reagents that may be used to introduce nucleic acids into host cells include, but are not limited to, Lipofectamine, Lipofectamine 2000, Oligofectamine, TFX reagents, Fugene 6, DOTAP/DOPE, Metafectine, and Fecturin. Although drug selection is optional in the methods described herein, we included one mammalian drug resistance marker per plasmid. The plasmids used herein contain a CMV promoter for expression of the odorant receptor gene or the G$\alpha$15 gene. An untranslated sequence encoding a tag, which contains a target sequence, for detection by a signaling probe was also present in each plasmid, such that the tag was co-transcribed and fused with the odorant receptor or G$\alpha$15 transcript. A tag contains a target sequence capable of hybridizing to a signaling probe. The target sequences utilized were Target Sequence 1 (SEQ ID NO: 129), and Target Sequence 2 (SEQ ID NO: 130), and the tag sequences utilized are Tag Sequence 1 (SEQ ID NO: 136) and Tag Sequence 2 (SEQ ID NO: 137). In these examples, the odorant gene-encoding plasmid contains Target Sequence 1, and the G$\alpha$15 gene-encoding plasmid contains Target Sequence 2.

***Step 2 - Selection step***

Transfected cells were grown for 1-4 days in Dulbecco's Modified Eagle Medium (DMEM) containing fetal bovine serum (FBS), followed by two weeks in antibiotic-containing DMEM-FBS. The antibiotic containing period had antibiotics added to the media as follows: Puromycin (0.3 ug/ml) and Hygromycin (110 ug/ml), Puromycin (0.15 ug/ml) and Hygromycin (75 ug/ml), and Puromycin (0.05 ug/ml) and Hygromycin (18 ug/ml).

***Step 3 - Cell passaging***

Following enrichment by antibiotic selection, and prior to introduction of fluorogenic signaling probes, cells were passaged 3 to 15 (p3-p15) more times in the absence of antibiotic selection to allow time for any unstable expression to subside.

***Step 4 - Exposure of cells to fluorogenic signaling probes***

Cells were harvested and transfected with signaling probes (SEQ ID NOS: 131 and 132). As will be appreciated by those of skill in the art, any reagent that is suitable for use with a chosen host cell may be used to introduce a nucleic acid, e.g. plasmid, oligonucleotide, labeled oligonucleotide, into a host cell with proper optimization. Examples of reagents that may be used to introduce nucleic acids into host cells include but are not limited to: Lipofectamine, Lipofectamine 2000, Oligofectamine, TFX reagents, Fugene 6, DOTAP/DOPE, Metafectine, or Fecturin.

**Target Sequences detected by signaling probes**

**Target Sequence 1**

5'-GTTCTTAAGGCACAGGAACTGGGAC-3' (SEQ ID NO: 129) **Tag Sequence 1**, with target sequence in bold (odorant)

5'-AGGGCGAATTCCAGCACACTGGCGGCCGTTACTAGTGGATCCGAGCTCGGAGGCAGGTGGACAGGAAGGTTCTAAT**GTTCTTAAGGCACAGGAACTGGGAC**ATCTGGGCCCGGAAAGCCTTTTTCTCTGTGATCCGGTACAGTCCTTCTGC-3' (SEQ ID NO: 136)

**Target Sequence 2**

5'-GAAGTTAACCCTGTCGTTCTGCGAC-3' (SEQ ID NO: 130) **Tag Sequence 2,** with target sequence in bold (odorant)

5'-AGGGCGAATTCCAGCACACTGGCGGCCGTTACTAGTGGATCCGAGCTCGGTACCAAGCTTCGAGGCAGGTGGACAGCTTGGTTCTAAT**GAAGTTAACCCTGTCGTTCTGCGAC**ATCTGGGCCCGGAAAGCGTTTAACTGATGGATGGAACAGTCCTTCTGC-3' (SEQ ID NO: 137)

**Signaling probes**

The signaling probes were supplied as 100μM stocks.
**Signaling probe 1 -** binds Target 1
**5' - Quasar670** GCCAGTCCCAGTTCCTGTGCCTTAAGAACCTCGC **BHQ2 quench -3'** (SEQ ID NO: 131)
**Signaling probe** 2 - binds Target 2
**5'- Cy5.5** GCGAGTCGCAGAACGACAGGGTTAACTTCCTCGC **BHQ2 quench -3'** (SEQ ID NO: 132)

Note also that 5-MedC and 2-aminodA mixmer probes rather than DNA probes were used in some instances.

A scrambled nontargeting 6-Fam probe was used as a delivery control (not shown) in some experiments.

***Step 5 - Isolation of positive cells***

The cells were dissociated and collected for analysis and sorting using a fluorescence activated cell sorter (Beckman Coulter, Miami, FL). Standard analytical methods were used to gate cells fluorescing above background and to isolate individual cells falling within the gate into barcoded 96-well plates. The gating hierarchy was as follows: coincidence gate > singlets gate > live gate > sort gate. With this gating strategy, the top 0.1-2% of double-positive cells were marked for sorting into barcoding 96-well plates.

***Step 6 - Additional cycles of steps 2-5 and/or 3-5***

The experiments were staged with very tightly timed logistics. As part of this protocol, there were two full cycles of steps 2-5 or 3-5 performed in order to have redundant sorts completed and additional clones obtained.

*Step 7- Estimation of growth rates for the populations of cells*

The plates were transferred to a Hamilton Microlabstar automated liquid handler. Cells were incubated for up to 20 days in a 1:1 mix of 2-3 day conditioned growth medium: fresh growth medium (DMEM/10% FBS) supplemented with 100 units penicillin/ml plus 0.1mg/ml streptomycin. Plates were imaged between 7 and 30 days postsort to determine confluency of wells (Genetix). Each plate was focused for reliable image acquisition across the plate. Reported confluencies of greater than 70% were not relied upon. Confluency measurements were obtained 3 times over the time specified above and used to calculate growth rates.

*Step 8 - Binning populations of cells according to growth rate estimates*

Cells were binned (independently grouped and plated as a cohort) according to growth rate approximately 20 to 30 days postsort. Bins were independently collected and plated on individual 96 well plates for downstream handling, and there could be more than one target plate per specific bin. Bins were calculated by considering the spread of growth rates and bracketing a range covering a high percentage, at least about 30 to 90% (as an estimate) of the total number of populations of cells. 9 growth bins were used with average partitions of approximately 15 to 40 hours over the bins across the different odorant receptor cell lines. Therefore each bin corresponded to a growth rate or population doubling time difference of approximately 1 to 5 hours.

Cells can have doubling times from less than 1 day to more than 2 weeks - in order to process the most diverse clones that at the same time can be reasonably binned according to growth rate; we typically prefer to do 3-9 bins with a 0.25 to 0.7 day doubling time per bin. One skilled in the art would understand how to adjust the tightness of the bins and number of bins according to the particular situation, and tightness and number of bins could be further adjusted if cells were synchronized for their cell cycle. In this example, cell lines with doubling times of 15 to 40 hours were selected for binning to increase tightness of the bins.

*Step 9 - Replica plating to speed parallel processing and provide stringent quality control*

The plates were incubated under standard and fixed conditions (humidified 37 , 5% $CO_2$/95% air) in DMEM media/10% FBS with antibiotics (100 units penicillin/ml plus 0.1mg/ml streptomycin). The plates of cells were split to produce 4 sets (the set consists of all plates with all growth bins - these steps ensured there were 4 replicates of the initial set) of target plates. Up to 3 target plate sets were committed for cryopreservation (see below), and the remaining sets were scaled and further replica plated for passage and for functional assay experiments. Distinct and independent tissue culture reagents, incubators, personnel and carbon dioxide sources were used for each set of plates. Quality control steps were taken to ensure the proper production and quality of all tissue culture reagents: each component added to each bottle of media prepared for use was added by one designated person in one designated hood with only that reagent in the hood while a second designated person monitored to avoid mistakes. Conditions for liquid handling were set to eliminate cross contamination across wells. Fresh tips were used for all steps or stringent tip washing protocols were used. Liquid handling conditions were set for accurate volume transfer, efficient cell manipulation, washing cycles, pipetting speeds and locations, number of pipetting cycles for cell dispersal, and relative position of tip to plate.

*Step 10 - Freezing early passage stocks of populations of cells*

Two sets of plates were frozen at -70 to -80°C. Plates in each set were first allowed to attain a cell confluency of 70 to 100%. Media was aspirated and culture media or 90% FBS and 10% DMSO was added. The plates were sealed with Parafilm® and then individually surrounded by 1 to 5 cm of foam and placed into a - 80 °C freezer.

*Step 11- Methods and conditions for initial transformative steps to produce stable cell lines*

The remaining 2 sets of plates were maintained as described above in step 9. All cell splitting was performed using liquid handling steps, including media removal, cell washing, trypsin addition and incubation, quenching and cell dispersal steps.

*Step 12 - Testing of the functionality of cells in re-arrayed plates*

The consistency and standardization of cell and culture conditions for all populations of cells were controlled. Differences across plates due to slight differences in growth rates were controlled by normalization of cell numbers across plates and occurred every 2 to 8 passages after the rearray. Populations of cells that were outliers were detected and eliminated.

### *Step 13 - Characterization of population of cells*

Clones were screened between 3.5 and 6 weeks post-sort with and top clones were retested functionally and were identified 5 to 6 weeks post-sort. The cells were maintained for up to 6 weeks to allow for their *in vitro* evolution under these conditions. During this time, we observed size, morphology, fragility, response to trypsinization or dissociation, roundness/average circularity post-dissociation, percentage viability, tendency towards microconfluency, or other aspects of cell maintenance such as adherence to culture plate surfaces.

### *Step 14 - Assessment of potential functionality of populations of cells*

Populations of cells were tested using functional criteria. Calcium mobilization dye Fluo-4 (Invitrogen) was used according to manufacturer's instructions. HEK293T cell lines stably expressing h-OR were maintained under standard cell culture conditions in DMEM medium supplemented with 10% fetal bovine serum and glutamine. On the day before assay, the cells were harvested from stock plates and plated into black clear-bottom 384 well assay plates. The assay plates were maintained in a 37°C cell culture incubator under 5% $CO_2$ for 22-48 hours. The media was then removed from the assay plates and calcium mobilization dye (Fluo-4, Invitrogen, Carlsbad, CA), diluted in buffer (130 mM NaCl, 5 mM KCl, 2 mM $CaCl_2$, 1.2 mM $MgCl_2$, 10 mM HEPES, 10 mM glucose, pH 7.0), was added and allowed to incubate for 1 hour at 37°C with Trypan Ultra Blue (ABD Bioquest, CA) as a quencher, and diluted in the above buffer (130 mM NaCl, 5 mM KCl, 2 mM $CaCl_2$, 1.2 mM $MgCl_2$, 10 mM HEPES, 10 mM glucose, pH 7.0). The above-identified quencher may be substituted by a quencher well-known in the art, e.g., Dipicrylamine (DPA), Acid Violet 17 (AV17), Diazine Black (DB), HLB30818, Bromophenol Blue, HLB30701, HLB30702, HLB30703, Nitrazine Yellow, Nitro Red, DABCYL (Molecular Probes), QSY (Molecular Probes), Metal ion quenchers (e.g., $Co^{2+}$, $Ni^{2+}$, $Cu^{2+}$), and Iodide ion. The assay plates were then loaded on a fluorescent plate reader (Hamamatsu FDSS) and agonist (4.5 mM Helional for OR3A1 and 0.3 mM Bourgeonal for OR1 D2- Figs 29A, 29B) dissolved in the above buffer (130 mM NaCl, 5 mM KCl, 2 mM $CaCl_2$, 1.2 mM $MgCl_2$, 10 mM HEPES, 10 mM glucose, pH 7.0) was added. Cells were tested at multiple different densities in 384-well plates and responses were analyzed. A variety of time points post-plating were used, for example 12-48 hours post-plating. Different densities of plating also were tested for assay response differences.

Figure 29A depicts representative traces of the functional cell-based response of cells expressing human odorant receptor OR3A1 to Helional (to a final concentration of 4.5 mM) compared to DMSO vehicle background signal as control. Test and control traces are overlaid. The results presented in Figure 29A of the cell-based assay designed to report calcium flux using a fluorescent calcium signaling dye show that the cells demonstrated a response to Helional over background.

Figure 29B depicts representative traces of the functional cell-based response of cells expressing human odorant receptor OR1 D2 to Bourgeonal (to a final concentration of 0.3 mM) compared to DMSO vehicle background signal as control. Test and control traces are overlaid. The results presented in Figure 29B of the cell-based assay designed to report calcium flux using a fluorescent calcium signaling dye show that the cells demonstrated a response to Bourgeonal over background.

The functional responses from experiments performed at low and higher passage numbers are compared to identify cells with the most consistent responses over defined periods of time, ranging from 6 to 40 weeks post-sort. Other characteristics of the cells that change over time are also noted, for example, time for cells to reattach post-dissociation.

### *Step 16 - Further evaluation of cells*

Populations of cells meeting functional and other criteria are further evaluated to determine those most amenable to production of viable, stable and functional cell lines. Selected populations of cells are expanded in larger tissue culture vessels and the characterization steps described above are continued or repeated under these conditions. At this point, additional standardization steps are introduced for consistent and reliable passages. These included different plating cell densities, plate coatings, time of passage, culture dish size/format and coating, fluidics optimization, cell dissociation optimization (e.g., dissociation in the presence of Cell Dissociation Buffer (Invitrogen) vs trypsin), volume of dissociation reagent used, and length of time of dissociation), as well as washing steps. Glutamine concentrations are dose ranged for the culture medium. Also, viability of cells at each passage are determined. Manual intervention is increased and cells are more closely observed and monitored. This information is used to help identify and select final cell lines that retain the desired properties. Final cell lines and back-up cell lines are selected that showed consistent growth, consistent (*i.e.* unchanging morphology) appropriate adherence, as well as functional response.

***Step 17 -Establishment of cell banks***

The low passage frozen plates (see above) corresponding to the final cell line and back-up cell lines are thawed at 37°C, washed two times with DMEM/10% FBS and incubated in humidified 37°C/5% $CO_2$ conditions. The cells are then expanded for a period of 2-3 weeks. Cell banks for each final and back-up cell line consisting of 25-50 vials are established. Cells are cryopreserved in 50% DMEM/10% FBS, 40% FBS, and 10% DMSO, resulting from further optimization experiments.

***Step 18 - Testing of cell bank***

At least one vial from the cell bank including the frozen stocks of the thawed cell lines, expanded and re-frozen, is thawed and expanded in culture. The resulting cells are tested to confirm that they meet the same characteristics for which they are originally selected.

**Example 40 Characterizing the cell line for native odorant receptor function (Reference Example)**

In order to identify and measure the ligand-induced responsiveness of odorant receptor-expressing HEK293T cells, receptor activation are monitored by measuring receptor-triggered alterations in intracellular calcium levels. Cells are either grown overnight in black-clear bottom plates in standard growth media or added to the black-clear bottom plates as a suspension in assay buffer (130 mM NaCl, 5 mM KCl, 2 mM $CaCl_2$, 1.2 mM $MgCl_2$, 10 mM HEPES, 10 mM glucose, pH 7.0). The cells are incubated for 1 hr at room temperature with the no-wash calcium-sensitive fluorescent dye Fluo-4 (Invitrogen) in buffer with Trypan Ultra Blue (ABD Bioquest, CA) as a quencher, diluted in the above buffer (130 mM NaCl, 5 mM KCl, 2 mM $CaCl_2$, 1.2 mM $MgCl_2$, 10 mM HEPES, 10 mM glucose, pH 7.0). The above-identified quencher can be substituted by a quencher well-known in the art, *e.g.*, Dipicrylamine (DPA), Acid Violet 17 (AV17), Diazine Black (DB), HLB30818, Trypan Blue, Bromophenol Blue, HLB30701, HLB30702, HLB30703, Nitrazine Yellow, Nitro Red, DABCYL (Molecular Probes), QSY (Molecular Probes), Metal ion quenchers *(e.g.,* $Co^{2+}$, $Ni^{2+}$, $Cu^{2+}$), and Iodide ion. The cell plates and test compounds (0.01 $\mu$M -100 mM) are placed in the high throughput fluorescent plate reader (Hamamatsu FDSS) which collects images of the plate fluorescence before, during and after the instrument added test compounds to the cells. Software (Hamamatsu FDSS) analysis of images reports the change in relative fluorescence for each well in the cell plate.

A variety of compounds and extracts, many of which were reported as odorants, are assayed across the odorant receptor cell lines as well as control cells in order to determine activity and to deorphan.

**Example 41 Uniformity of functional response in odorant receptor-expressing cell lines (Reference Example)**

Because odorant receptors are G protein coupled receptors (GPCRs), in order to make meaningful comparison of responses between different odorant receptors in the presence of various compounds, it is important to determine that the G protein coupling receptors functions uniformly in different cell lines. The relative responses of the different cell lines across a range of concentrations of a G protein coupling agonist should be uniform. To confirm this, the uniformity in G$\alpha$15-mediated receptor responses of all cell lines expressing the different odorant receptors and the mouse G$\alpha$15 protein are tested in dose response curve experiments with varying concentrations of isoproterenol, an agonist of the $\beta$-adrenergic receptor endogenously expressed in the cells. This endogenous receptor, when stimulated, can couple to the expressed G$\alpha$15 and evoke a change in intracellular free calcium. Percent response is plotted as a function of isoproterenol concentration, and the results are curve-fitted to calculate an EC50 value (concentration of half-maximal receptor activation).

**Example 42 Identification of functionally responsive broadly tuned,**

**moderately tuned, and selective receptors (Reference Example)**

Cell lines stably expressing native human odorant receptor genes, generated using the method described herein (*i.e.*, Example 39), are tested in functional cell-based receptor studies to detect their activation by a collection of chemically diverse compounds. This allows the identification of broadly tuned receptors, less broadly tuned, and narrowly tuned receptors.

**Example 43 Identification of receptors activated by a library of compounds** (Reference Example)

The activity of a library of compounds against each of the odorant receptor cell lines is tested. Data are generated in functional cell-based assays for receptor activity upon addition of the compounds, and are expressed as percent activity above the basal activity of the receptor *(i.e.,* receptor activity upon addition of buffer alone). Activity of each compound at each receptor is then coded to highlight compounds with low *(e.g.,* <100% above basal activity; white), medium *(e.g.,* 101-500% above basal activity; light gray), high (*e.g.*, 501-1000% above basal activity; dark gray), or very high (*e.g.*, >1001% above basal activity, black) activity at each receptor. The resulting pattern is used to provide a graphical representation of compounds active either selectively or broadly across odorant receptors and of receptors showing either broad or selective response to a chemically diverse set of compounds. Analysis of the data is used to assign or identify particular odorant receptors or pattern of odorant receptor activity with test compounds of interest and to identify modulators including blockers and potentiators. Analysis of this data can also be used to correlate patterns of odorant receptor activity with physiological effects of corresponding odorants tested.

**Example 44 Identification of analogs of compounds with similar functional activities as antagonists of agonist-induced odorant receptor activity** (Reference Example)

To test the ability of the odorant receptor-expressing cell-based assays to identify analogues of compounds with similar functional activities, structural analogues of a known modulator or modulating compound are tested for their ability to inhibit an agonist-induced receptor activity of an odorant receptor. The structures of analogues that inhibit the odorant receptor's activity induced by the agonist are compared. Thus, the odorant receptor-expressing cell lines may aid discovery of potent odorant receptor antagonists. Modulators with different activities (e.g., potentiation, agonism, blocking, *etc.*) are also identified by operating the assay and testing and performing assay result analysis as would be known to one of skill in the art.

The embodiments of the present invention described above are intended to be merely exemplary, and those skilled in the art will recognize, or be able to ascertain using no more than routine experimentation, numerous equivalents to the specific procedures described herein. All such equivalents are considered to be within the scope of the present invention and are covered by the following claims. Additionally, ordinarily skilled artisans will recognize that operational sequences must be set forth in some specific order for the purpose of explanation and claiming, but the present invention contemplates various changes beyond such specific order.

## Claims

**1.** A method for isolating a cell that endogenously expresses a sweet taste receptor T1R2 subunit, wherein the method comprises:

providing a population of cells; wherein said population of cells has not been genetically engineered; wherein said population of cells is not known to endogenously express a T1R2 subunit;
introducing into the cells a molecular beacon that detects expression of a T1R2 subunit; and
isolating cells that express a receptor T1R2 subunit.

**2.** The method of claim 1, wherein said population of cells also is not known to endogenously express a T1R3 subunit; and wherein said method further comprises:

introducing into the population of cells a molecular beacon that detects expression of a T1R3 subunit; and
isolating cells that express a T1R3 subunit.

**3.** A method for isolating a cell that endogenously expresses a T1R2 subunit,
wherein the method comprises:

providing a population of cells; wherein said population of cells has not been genetically engineered;
introducing into the cells a molecular beacon that detects expression of a T1R2 subunit; and
isolating cells that express a T1R2 subunit;

wherein any expression level of the T1R2 subunit in the isolated cell is at least 10x, 50x, 100x, 250x, 500x, 750x, 1000x, 2500x, 5000x, 7500x, 10000x, 50000x, or at least 100000x higher than in an average cell of the population of cells.

**4.** The method of claim 1, wherein said population of cells also is not known to endogenously express a T1R3 subunit; and wherein said method comprises the further steps of:

introducing into the population of cells a molecular beacon that detects expression of a T1R3 subunit; and isolating cells that express a T1R3 subunit;
wherein any expression level of the T1R3 subunit in the isolated cell is at least 10x, 50x, 100x, 250x, 500x, 750x, 1000x, 2500x, 5000x, 7500x, 10000x, 50000x, or at least 100000x higher than in an average cell of the population of cells.

**5.** The method of claim 1 or 3, wherein said molecular beacon is directed to non-coding introns or non-coding untranslated sequences of a T1R2 RNA.

**6.** The method of claim 2 or 4, wherein said molecular beacon is directed to non-coding introns or non-coding untranslated sequences of a T1R3 RNA.

**7.** The method of any one of claims 1-2 or 5-6, wherein any expression level of T1R2 or T1R3 in the isolated cell is at least 10x, 50x, 100x, 250x, 500x, 750x, 1000x, 2500x, 5000x, 7500x, 10000x, 50000x, or at least 100000X higher than in an average cell of the population of cells.

**8.** The method of any one of claims 1-7, wherein genetic variability in the population of cells had been increased prior to said isolating step.

**9.** The method of any one of claims 1-8, wherein said molecular beacon is labeled.

**10.** The method of claim 9, wherein said molecular beacon is labeled with a fluorophore and wherein said molecular beacon is conjugated to a quencher.

**11.** The method of claim 10, wherein said molecular beacon comprises an oligonucleotide comprising the nucleotide sequence of any one of SEQ ID NOs: 103-112.

**12.** The method of claim 11, wherein the cells are isolated by utilizing fluorescence-activated cell sorting ("FACS™") or magnetic activated cell sorting ("MACS™").

**13.** The method of claim 12, wherein the cells are isolated by utilizing fluorescence-activated cell sorting ("FACS™").

**14.** The method of claim 8, wherein said genetic variability is increased by exposure of said population of cells to: ultraviolet light, x-rays, mutagens, carcinogens, chemical agents, growth conditions that are sub-optimal, proteins that result in DNA damage, and/or proteins that decrease the fidelity of DNA replication or repair.

**15.** The method of claim 14, wherein said genetic variability is increased by exposure of said population of cells to ultraviolet light.

**16.** The method of claim 14, wherein said genetic variability is increased by exposure of said population of cells to a mutagen.

**17.** The method of claim 16, wherein said mutagen is ethyl methanesulfonate.

**18.** The method of any one of claims 1-17, wherein said population of cells comprises: mixed cell types of different origin, cells of one cell type that are genetically heterologous, or cells of one particular cell line.

**19.** The method of claim 18, wherein said population of cells comprises cells of one particular cell line.

**Patentansprüche**

**1.** Verfahren zum Isolieren einer Zelle, die endogen eine T1R2-Untereinheit eines Rezeptors für süßen Geschmack exprimiert, wobei das Verfahren umfasst:

Bereitstellen einer Population von Zellen; wobei die Population von Zellen nicht gentechnisch verändert worden ist; wobei die Population von Zellen nicht dafür bekannt ist, endogen eine T1R2-Untereinheit zu exprimieren;
Einführen eines Molecular Beacons in die Zellen, welcher die Expression einer T1R2-Untereinheit detektiert; und
Isolieren von Zellen, die eine T1R2-Untereinheit des Rezeptors exprimieren.

**2.** Verfahren nach Anspruch 1, wobei die Population von Zellen auch nicht dafür bekannt ist, eine T1R3-Untereinheit endogen zu exprimieren; und wobei das Verfahren ferner umfasst:

Einführen eines Molecular Beacons in die Population von Zellen, welcher die Expression einer T1R3-Untereinheit detektiert; und
Isolieren von Zellen, die eine T1R3-Untereinheit exprimieren.

**3.** Verfahren zum Isolieren einer Zelle, die endogen eine T1R2-Untereinheit exprimiert, wobei das Verfahren umfasst:

Bereitstellen einer Population von Zellen; wobei die Population von Zellen nicht gentechnisch verändert worden ist;
Einführen eines Molecular Beacons in die Zellen, welcher die Expression einer T1R2-Untereinheit detektiert; und
Isolieren von Zellen, die eine T1R2-Untereinheit exprimieren;
wobei jedwedes Expressionsniveau der T1R2-Untereinheit in der isolierten Zelle mindestens 10x, 50x, 100x, 250x, 500x, 750x, 1000x, 2500x, 5000x, 7500x, 10000x, 50000x oder mindestens 100000x höher als in einer durchschnittlichen Zelle der Population von Zellen ist.

**4.** Verfahren nach Anspruch 1, wobei die Population von Zellen auch nicht dafür bekannt ist, eine T1R3-Untereinheit endogen zu exprimieren; und wobei das Verfahren folgende weiteren Schritte umfasst:

Einführen eines Molecular Beacons in die Population von Zellen, welcher die Expression einer T1R3-Untereinheit detektiert; und
Isolieren von Zellen, die eine T1R3-Untereinheit exprimieren;
wobei jedwedes Expressionsniveau der T1R3-Untereinheit in der isolierten Zelle mindestens 10x, 50x, 100x, 250x, 500x, 750x, 1000x, 2500x, 5000x, 7500x, 10000x, 50000x oder mindestens 100000x höher als in einer durchschnittlichen Zelle der Population von Zellen ist.

**5.** Verfahren nach Anspruch 1 oder 3, wobei der Molecular Beacon auf nicht-kodierende Introns oder nicht-kodierende nicht-translatierte Sequenzen einer T1R2-RNA gerichtet ist.

**6.** Verfahren nach Anspruch 2 oder 4, wobei der Molecular Beacon auf nicht-kodierende Introns oder nicht-kodierende nicht-translatierte Sequenzen einer T1R3-RNA gerichtet ist.

**7.** Verfahren nach einem der Ansprüche 1-2 oder 5-6, wobei jedwedes Expressionsniveau von T1R2 oder T1R3 in der isolierten Zelle mindestens 10x, 50x, 100x, 250x, 500x, 750x, 1000x, 2500x, 5000x, 7500x, 10000x, 50000x oder mindestens 100000x höher als in einer durchschnittlichen Zelle der Population von Zellen ist.

**8.** Verfahren nach einem der Ansprüche 1-7, wobei die genetische Variabilität in der Population von Zellen vor dem Isolationsschritt erhöht worden ist.

**9.** Verfahren nach einem der Ansprüche 1-8, wobei der Molecular Beacon markiert ist.

**10.** Verfahren nach Anspruch 9, wobei der Molecular Beacon mit einem Fluorophor markiert ist und wobei der Molecular Beacon an einen Quencher konjugiert ist.

**11.** Verfahren nach Anspruch 10, wobei der Molecular Beacon ein Oligonucleotid umfasst, das die Nucleotidsequenz nach irgendeinem der SEQ ID Nos: 103-112 umfasst.

**12.** Verfahren nach Anspruch 11, wobei die Zellen durch Nutzung von Fluoreszenzaktiviertem Zellsortieren ("FACS™") oder magnetisch aktiviertem Zellsortieren ("MACS™") isoliert werden.

**13.** Verfahren nach Anspruch 12, wobei die Zellen durch Nutzung von Fluoreszenzaktiviertem Zellsortieren ("FACS™") isoliert werden.

**14.** Verfahren nach Anspruch 8, wodurch die genetische Variabilität durch Einwirkung von Ultraviolettlicht, Röntgenstrahlen, Mutagenen, Carcinogenen, Chemikalien, suboptimalen Wachstumsbedingungen, Proteinen, die zu DNA-Schäden führen, und/oder Proteinen, die die Genauigkeit der DNA-Replikation oder -Reparatur herabsetzen, auf die Population von Zellen erhöht wird.

**15.** Verfahren nach Anspruch 14, wobei die genetische Variabilität durch Einwirkung von Ultraviolettlicht auf die Population von Zellen erhöht wird.

**16.** Verfahren nach Anspruch 14, wobei die genetische Variabilität durch Einwirkung eines Mutagens auf die Population von Zellen erhöht wird.

**17.** Verfahren nach Anspruch 16, wobei das Mutagen Ethylmethansulfonat ist.

**18.** Verfahren nach einem der Ansprüche 1-17, wobei die Population von Zellen gemischte Zelltypen unterschiedlichen Ursprungs, Zellen eines Zelltyps, die genetisch heterolog sind, oder Zellen von einer speziellen Zelllinie umfasst.

**19.** Verfahren nach Anspruch 18, wobei die Population von Zellen Zellen von einer speziellen Zelllinie umfasst.

## Revendications

**1.** Procédé d'isolation d'une cellule qui exprime de manière endogène une sous-unité T1R2 du récepteur de goût sucré, ce procédé comprenant :

l'utilisation d'une population de cellules ; ladite population de cellules n'ayant pas été génétiquement modifiée ;
ladite population de cellules n'étant pas connue pour exprimer de manière endogène une sous-unité T1R2 ;
l'introduction dans les cellules d'une balise moléculaire qui détecte l'expression d'une sous-unité T1R2 ; et
l'isolation des cellules qui expriment une sous-unité T1R2.

**2.** Procédé selon la revendication 1, dans lequel ladite population de cellules n'est pas non plus connue pour exprimer de manière endogène une sous-unité T1R3 ; et dans lequel ledit procédé comprenant en outre :

l'introduction dans la population de cellules d'une balise moléculaire qui détecte l'expression d'une sous-unité T1R3 ; et
l'isolation des cellules qui expriment une sous-unité T1R3.

**3.** Procédé d'isolation d'une cellule qui exprime de manière endogène une sous-unité T1R2 du récepteur de goût sucré, ce procédé comprenant :

l'utilisation d'une population de cellules ; ladite population de cellules n'ayant pas été génétiquement modifiée ;
l'introduction dans les cellules d'une balise moléculaire qui détecte l'expression d'une sous-unité T1R2 ; et
l'isolation des cellules qui expriment une sous-unité T1R2 ;
tout niveau d'expression de la sous-unité T1R2 dans la cellule isolée étant au moins 10 x, 50 x, 100 x, 250 x, 500 x, 750 x, 1 000 x, 2 500 x, 5 000 x, 7 500 x, 10 000 x, 50 000 x ou au moins 100 000 x plus élevé que dans une cellule moyenne de la population de cellules.

**4.** Procédé selon la revendication 1, dans lequel ladite population de cellules n'est pas connue pour exprimer de manière endogène une sous-unité T1R3 ; et dans lequel ledit procédé comprend les étapes supplémentaires suivantes :

l'introduction dans la population de cellules d'une balise moléculaire qui détecte l'expression d'une sous-unité T1R3 ; et
l'isolation des cellules qui expriment une sous-unité T1R3 ;

tout niveau d'expression de la sous-unité T1R3 dans la cellule isolée étant au moins 10 x, 50 x, 100 x, 250 x, 500 x, 750 x, 1 000 x, 2 500 x, 5 000 x, 7 500 x, 10 000 x, 50 000 x ou au moins 100 000 x plus élevé que dans une cellule moyenne de la population de cellules.

**5.** Procédé selon la revendication 1 ou 3, dans lequel ladite balise moléculaire est dirigée vers des introns non codants ou dans des séquences non codantes non traduites d'un ARN de T1R2.

**6.** Procédé selon la revendication 2 ou 4, dans lequel ladite balise moléculaire est dirigée vers des introns non codants ou dans des séquences non codantes non traduites d'un ARN de T1R3.

**7.** Procédé selon l'une quelconque des revendications 1 à 2 ou 5 à 6, dans lequel tout niveau d'expression de T1R2 ou de T1R3 dans la cellule isolée est au moins 10 x, 50 x, 100 x, 250 x, 500 x, 750 x, 1 000 x, 2 500 x, 5 000 x, 7 500 x, 10 000 x, 50 000 x ou au moins 100 000 x plus élevé que dans une cellule moyenne de la population de cellules.

**8.** Procédé selon l'une quelconque des revendications 1 à 7, dans lequel la variabilité génétique dans la population de cellules a été augmentée avant ladite étape d'isolation.

**9.** Procédé selon l'une quelconque des revendications 1 à 8, dans lequel ladite balise moléculaire est marquée.

**10.** Procédé selon la revendication 9, dans lequel ladite balise moléculaire est marquée avec un fluorophore et où ladite balise moléculaire est conjuguée à un extincteur.

**11.** Procédé selon la revendication 10, dans lequel ladite balise moléculaire comprend un oligonucléotide contenant la séquence de nucléotides de l'une quelconque des SEQ ID NO:103 à 112.

**12.** Procédé selon la revendication 11, dans lequel les cellules sont isolées par emploi d'un tri cellulaire activé par fluorescence (« FACS™ ») ou d'un tri cellulaire activé par un champ magnétique (« MACS™ »).

**13.** Procédé selon la revendication 12, dans lequel les cellules sont isolées par emploi d'un tri cellulaire activé par fluorescence (« FACS™ »).

**14.** Procédé selon la revendication 8, dans lequel ladite variabilité génétique est augmentée par exposition de ladite population de cellules à : une lumière ultraviolette, des rayons X, des mutagènes, des carcinogènes, des agents chimiques, des conditions de croissance qui sont sous-optimales, des protéines qui entraînent une dégradation de l'ADN et/ou des protéines qui réduisent la fidélité de la réplication ou la réparation de l'ADN.

**15.** Procédé selon la revendication 14, dans lequel ladite variabilité génétique est augmentée par exposition de ladite population de cellules à la lumière ultraviolette.

**16.** Procédé selon la revendication 14, dans lequel ladite variabilité génétique est augmentée par exposition de ladite population de cellules à un mutagène.

**17.** Procédé selon la revendication 16, dans lequel ledit mutagène est le méthanesulfonate d'éthyle.

**18.** Procédé selon l'une quelconque des revendications 1 à 17, dans lequel ladite population de cellules comprend : des types mixtes de cellules de différente origine, des cellules d'un seul type de cellules qui sont génétiquement hétérologues ou des cellules d'une lignée cellulaire particulière.

**19.** Procédé selon la revendication 18, dans lequel ladite population de cellules comprend des cellules d'une lignée cellulaire particulière.

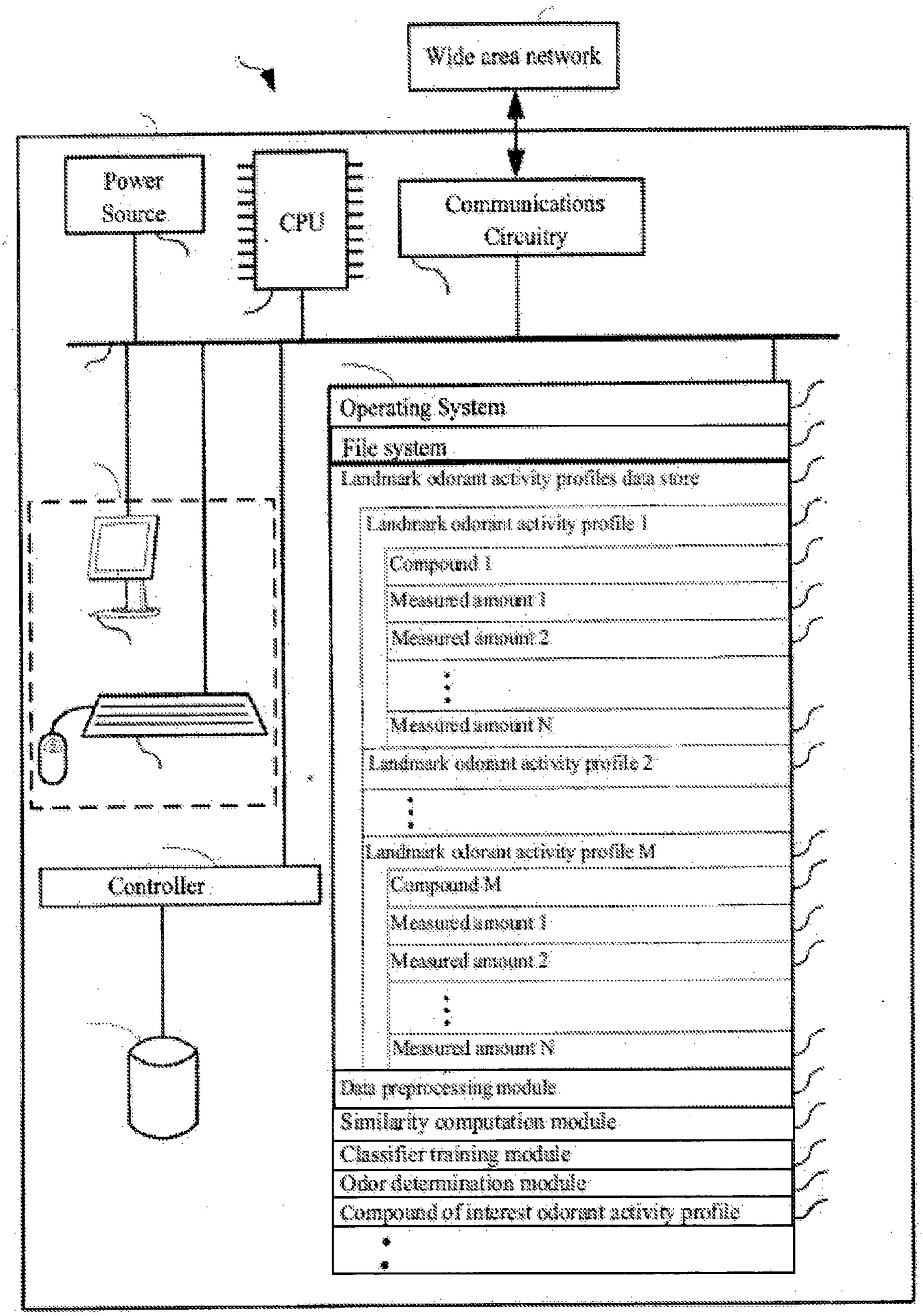
Wide area network
Power Source
CPU
Communications Circuitry
Operating System
File system
Landmark odorant activity profiles data store
Landmark odorant activity profile 1
Compound 1
Measured amount 1
Measured amount 2
Measured amount N
Landmark odorant activity profile 2
Landmark odorant activity profile M
Compound M
Measured amount 1
Measured amount 2
Measured amount N
Data preprocessing module
Similarity computation module
Classifier training module
Odor determination module
Compound of interest odorant activity profile
Controller

Figure 1

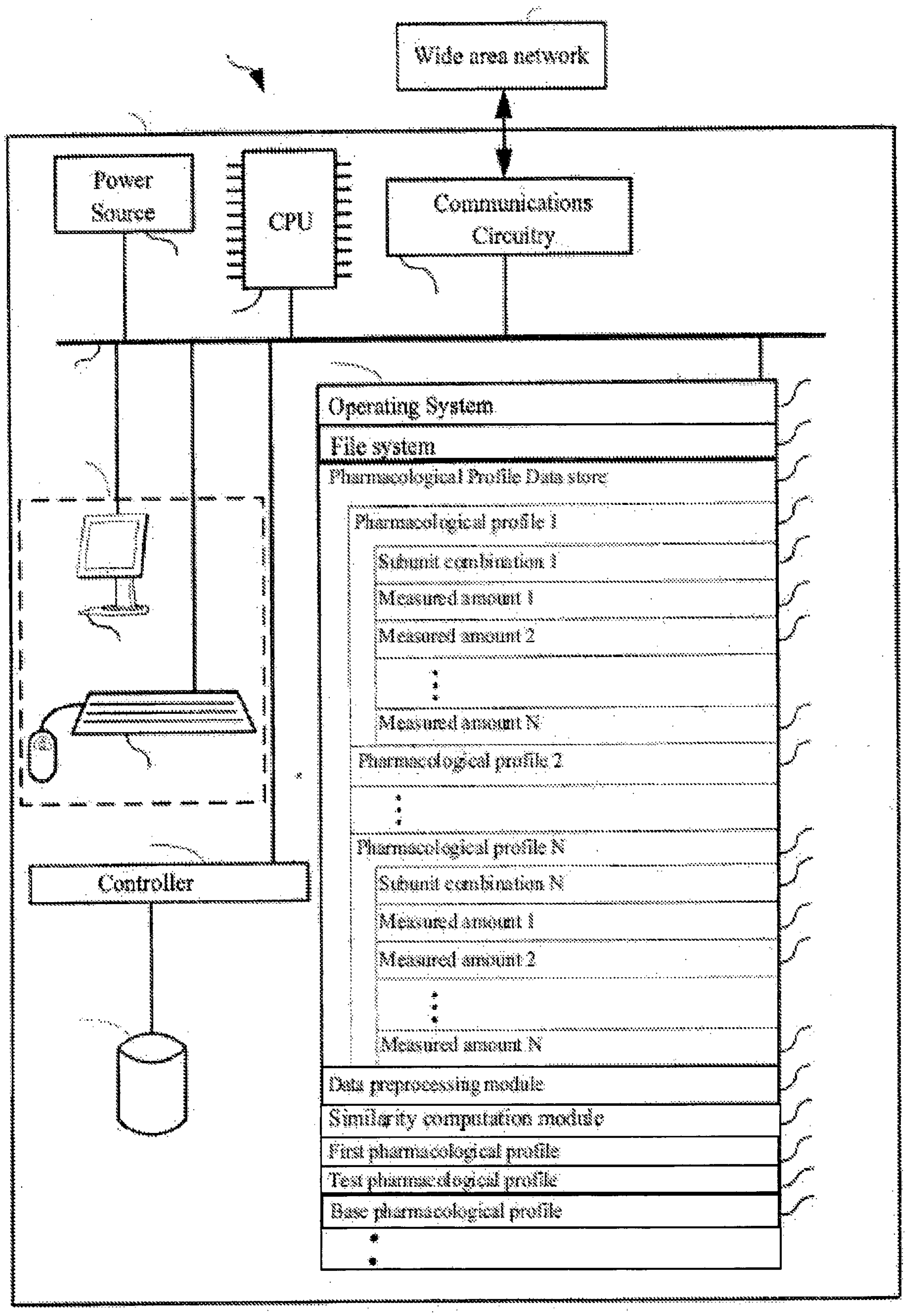
Wide area network
Power Source
CPU
Communications Circuitry
Operating System
File system
Pharmacological Profile Data store
Pharmacological profile 1
Subunit combination 1
Measured amount 1
Measured amount 2
Measured amount N
Pharmacological profile 2
Pharmacological profile N
Subunit combination N
Measured amount 1
Measured amount 2
Measured amount N
Data preprocessing module
Similarity computation module
First pharmacological profile
Test pharmacological profile
Base pharmacological profile
Controller

Figure 2

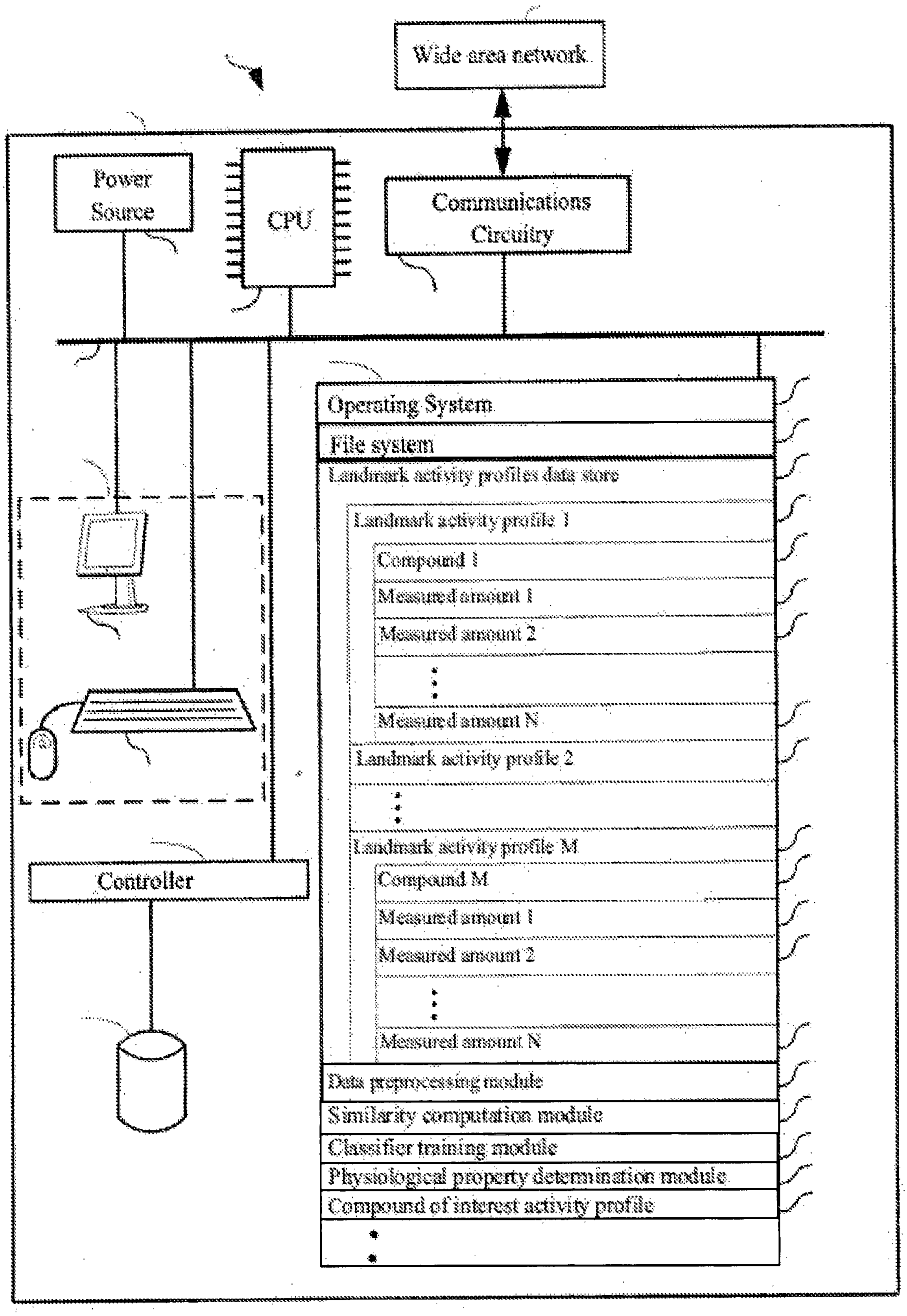
Wide area network
Power Source
CPU
Communications Circuitry
Operating System
File system
Landmark activity profiles data store
Landmark activity profile 1
Compound 1
Measured amount 1
Measured amount 2
Measured amount N
Landmark activity profile 2
Landmark activity profile M
Compound M
Measured amount 1
Measured amount 2
Measured amount N
Data preprocessing module
Similarity computation module
Classifier training module
Physiological property determination module
Compound of interest activity profile
Controller

Figure 3

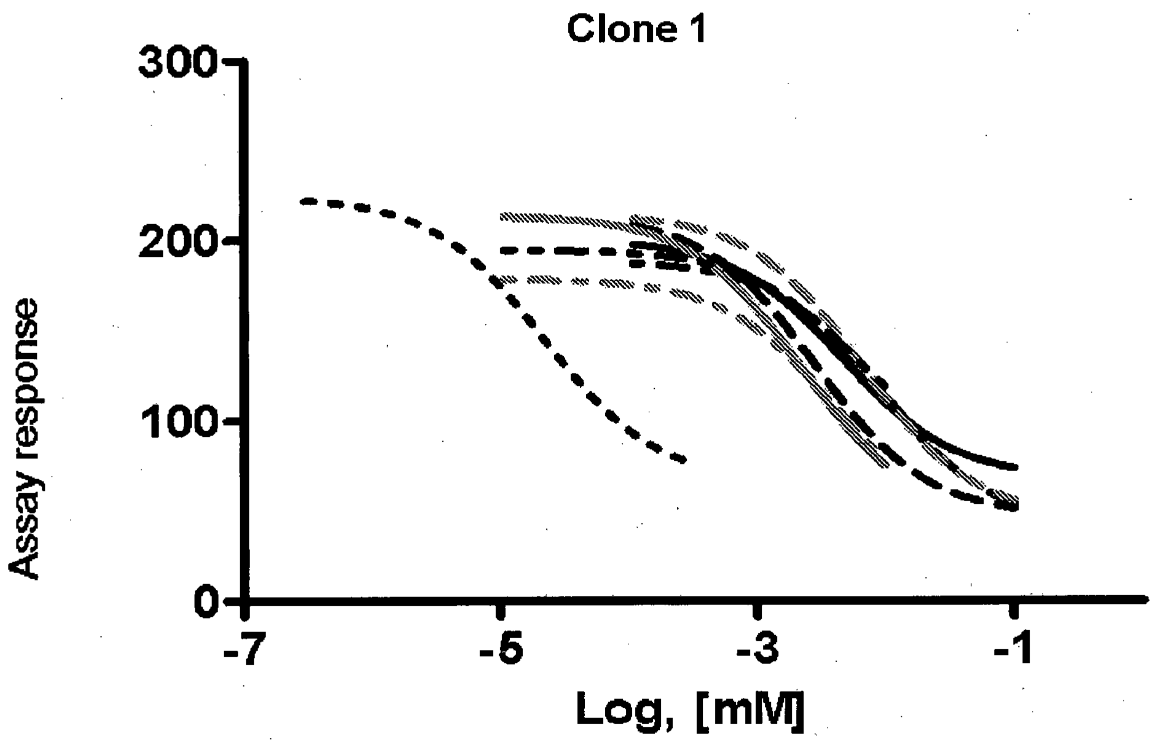


Figure 4A

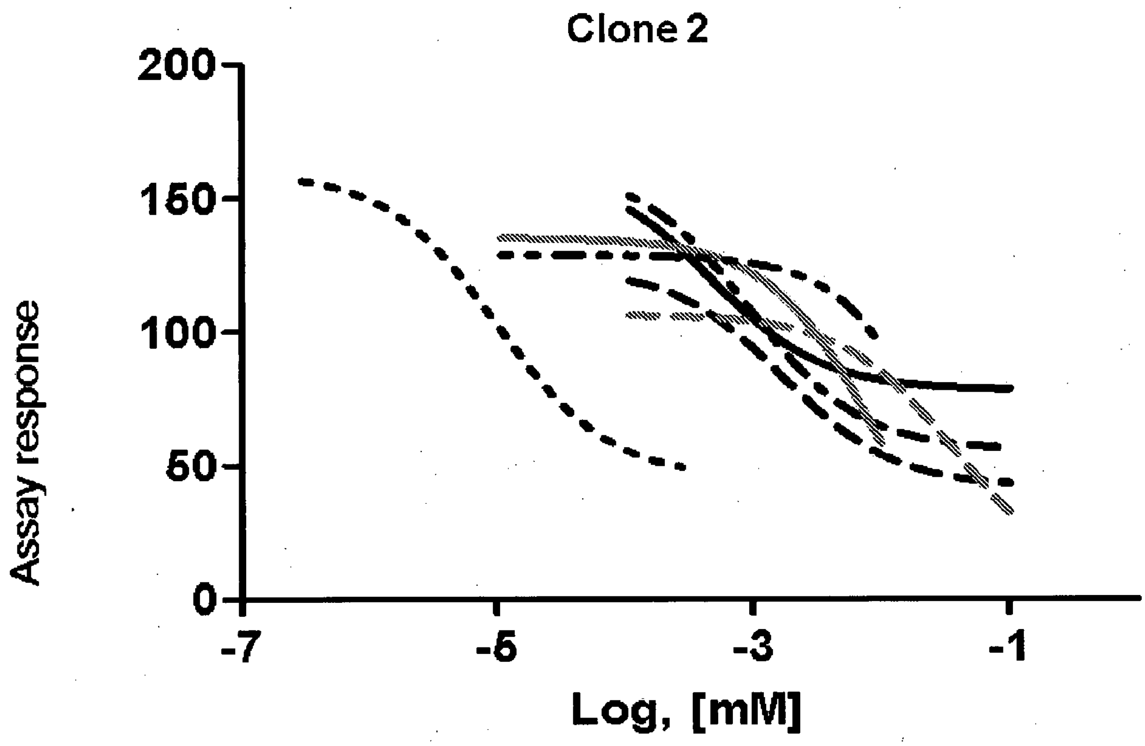


Figure 4B

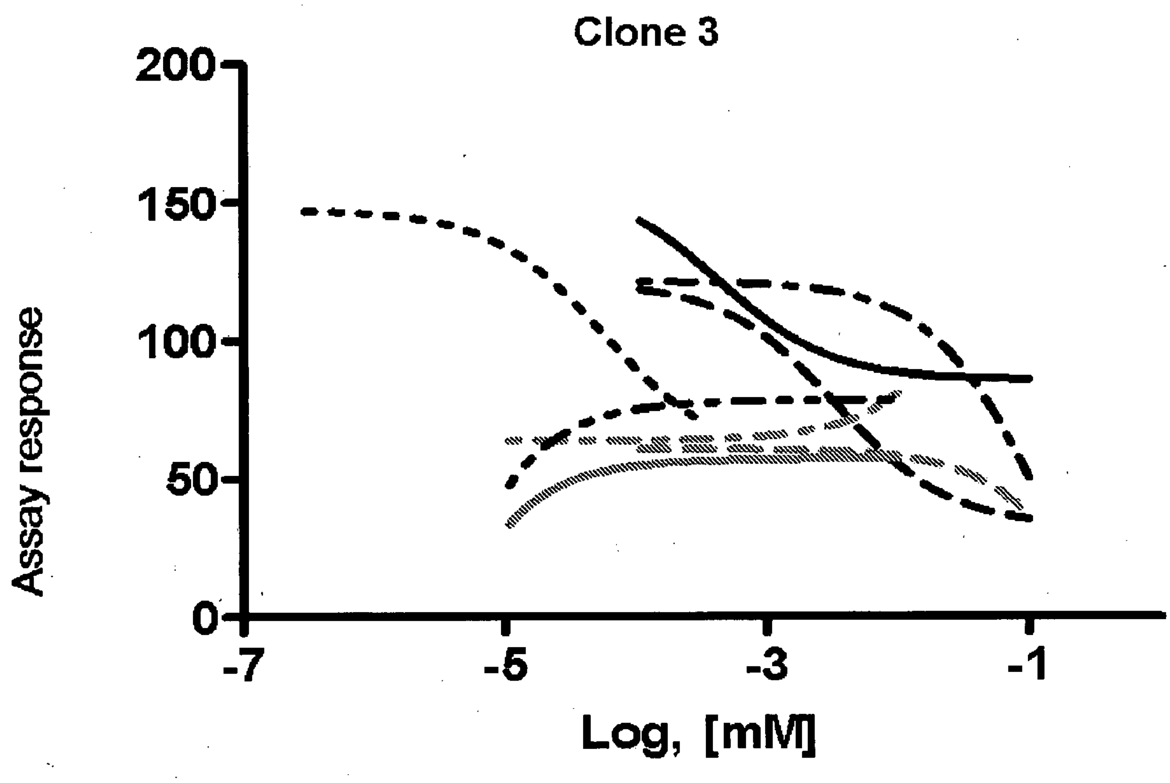


Figure 4C

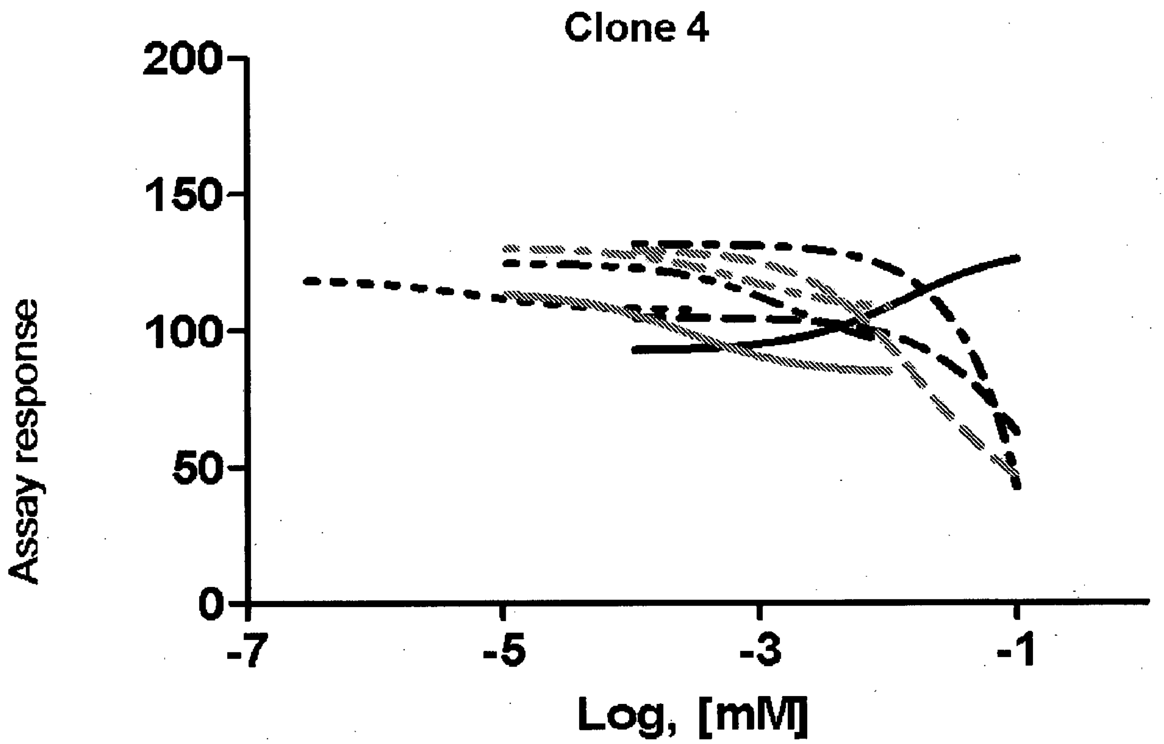


Figure 4D

# Bin 1

| | Compound 1 | | Compound 2 | | Compound 3 | | Compound 4 | | Compound 5 | | Compound 6 | | Compound 7 | | Compound 8 | |
|---|---|---|---|---|---|---|---|---|---|---|---|---|---|---|---|---|
| Clone | Dose 1 | Dose 2 | Dose 1 | Dose 2 | Dose 1 | Dose 2 | Dose 1 | Dose 2 | Dose 1 | Dose 2 | Dose 1 | Dose 2 | Dose 1 | Dose 2 | Dose 1 | Dose 2 |
| 1 | 54 | 61 | 76 | 69 | 82 | 86 | 76 | 61 | 62 | 53 | 61 | 69 | 57 | 58 | 48 | 33 |
| 2 | 64 | 58 | 81 | 69 | 76 | 62 | 78 | 55 | 83 | 28 | 87 | 76 | 54 | 49 | 56 | 40 |
| 3 | 63 | 57 | 53 | 74 | 81 | 65 | 63 | 59 | 34 | 20 | 64 | 63 | 38 | 45 | 45 | 27 |
| 4 | -42 | -19 | 86 | 73 | 71 | 74 | 9 | 32 | 78 | 78 | 73 | -6 | 51 | 50 | 79 | 9 |
| 5 | 60 | 65 | 79 | 66 | 77 | 56 | 79 | 41 | 84 | 43 | 63 | 61 | 55 | 44 | 46 | 35 |
| 6 | 69 | 69 | 72 | 61 | 56 | 76 | 85 | 60 | 59 | 68 | 74 | 69 | 86 | 64 | 55 | 74 |
| 7 | 54 | 21 | 86 | 60 | 82 | 48 | 40 | 33 | 44 | 19 | 77 | 64 | 47 | 56 | 43 | 35 |
| 8 | 26 | 46 | 72 | 68 | 76 | 72 | 75 | 71 | 71 | 76 | 94 | 73 | 68 | 60 | 64 | 42 |
| 9 | 36 | 39 | 84 | 28 | 53 | 47 | -14 | -5 | 84 | 92 | 73 | 64 | 52 | 43 | 80 | 84 |
| 10 | 23 | 1 | 81 | 62 | 84 | 49 | 87 | 67 | 88 | 36 | 81 | 69 | 82 | 60 | 42 | 35 |
| 11 | 54 | 38 | 76 | 43 | 42 | 43 | 37 | 35 | 85 | 24 | 77 | 68 | 42 | 43 | 48 | 42 |
| 12 | 80 | 48 | 82 | 73 | 78 | 72 | 72 | 61 | 44 | 28 | 78 | 62 | 20 | 39 | 25 | 65 |
| 13 | 58 | 46 | 53 | 61 | 83 | 34 | 48 | 31 | 44 | 15 | 64 | 55 | 48 | 36 | 41 | 42 |
| 14 | 61 | 55 | 81 | 72 | 68 | 58 | 61 | 47 | 67 | 33 | 71 | 56 | 10 | 41 | 64 | 44 |
| 15 | 75 | 86 | 86 | 86 | 81 | 75 | 67 | 65 | 64 | 24 | 86 | 61 | 61 | 58 | 66 | 54 |
| 16 | 62 | 65 | 65 | 62 | 68 | 50 | 51 | 63 | 83 | 51 | 76 | 58 | 50 | 50 | 49 | 40 |
| 17 | 68 | 61 | 87 | 77 | 88 | 62 | 67 | 73 | 62 | 67 | 96 | 63 | 67 | 60 | 62 | 38 |
| 18 | 57 | 69 | 79 | 71 | 81 | 65 | 63 | 64 | 57 | 43 | 96 | 74 | 38 | 49 | 64 | 39 |

Figure 5A

# Bin 2

| | Compound 1 | | Compound 2 | | Compound 3 | | Compound 4 | | Compound 5 | | Compound 6 | | Compound 7 | | Compound 8 | |
|---|---|---|---|---|---|---|---|---|---|---|---|---|---|---|---|---|
| Clone | Dose 1 | Dose 2 | Dose 1 | Dose 2 | Dose 1 | Dose 2 | Dose 1 | Dose 2 | Dose 1 | Dose 2 | Dose 1 | Dose 2 | Dose 1 | Dose 2 | Dose 1 | Dose 2 |
| 19 | 40 | 46 | 75 | 71 | 71 | 48 | 50 | 59 | 22 | 21 | 61 | 74 | 45 | 86 | 14 | -8 |
| 20 | 27 | 48 | 55 | 76 | 46 | 48 | 66 | 79 | 66 | 13 | 66 | 72 | -57 | -65 | -14 | -8 |
| 21 | 54 | 61 | 62 | 46 | 61 | 67 | 42 | 34 | 26 | 13 | 66 | 23 | -79 | -14 | 7 | 21 |
| 22 | 42 | 46 | 63 | 48 | 75 | 55 | 38 | 60 | 54 | 28 | 65 | 44 | 27 | 40 | 5 | -24 |
| 23 | 46 | 54 | 63 | 61 | 67 | 53 | 64 | 56 | 15 | -12 | 58 | 36 | -20 | 22 | 18 | -6 |
| 24 | 28 | 31 | 69 | 62 | 73 | 40 | 38 | 39 | 49 | 35 | 73 | 52 | 21 | 31 | -16 | -15 |
| 25 | 40 | 36 | 74 | 60 | 62 | 47 | 62 | 44 | 47 | 28 | 74 | 67 | 36 | 40 | 2 | 10 |
| 26 | 42 | 45 | 57 | 43 | 74 | 58 | 46 | 41 | 62 | 35 | 62 | 56 | 9 | 28 | 13 | 17 |
| 27 | 67 | 76 | 35 | 65 | 73 | 46 | 65 | 60 | 36 | -4 | 60 | 64 | 6 | -14 | 26 | 23 |
| 28 | 41 | 49 | 76 | 61 | 45 | 57 | 43 | 63 | 44 | 15 | 71 | 39 | 6 | 11 | -2 | -19 |
| 29 | 73 | 43 | 82 | 64 | 66 | 28 | 33 | 22 | 17 | 11 | 65 | 40 | 10 | 15 | 27 | 17 |
| 30 | 36 | 32 | 81 | 57 | 72 | 45 | 43 | 36 | 24 | 10 | 74 | 56 | 30 | 33 | 40 | 27 |
| 31 | 48 | 42 | 54 | 63 | 73 | 18 | 45 | 37 | 24 | 18 | 69 | 51 | 31 | 26 | 18 | 8 |
| 32 | 25 | 48 | 52 | 42 | 72 | 63 | 64 | 51 | -17 | 7 | 67 | 36 | 27 | 5 | 47 | 35 |
| 33 | 64 | 47 | 59 | 57 | 40 | 19 | 58 | 63 | 9 | -14 | 28 | -6 | 36 | 43 | 14 | 25 |

Figure 5B

Bin 3

| Clone | Compound 1 | | Compound 2 | | Compound 3 | | Compound 4 | | Compound 5 | | Compound 6 | | Compound 7 | | Compound 8 | |
|---|---|---|---|---|---|---|---|---|---|---|---|---|---|---|---|---|
| | Dose 1 | Dose 2 | Dose 1 | Dose 2 | Dose 1 | Dose 2 | Dose 1 | Dose 2 | Dose 1 | Dose 2 | Dose 1 | Dose 2 | Dose 1 | Dose 2 | Dose 1 | Dose 2 |
| 34 | -100 | 8 | 27 | 22 | 33 | 0 | 30 | 35 | 47 | 10 | 66 | 62 | -3 | 58 | 34 | 41 |
| 35 | 16 | 15 | 22 | 1 | 33 | -3 | -20 | -31 | 65 | 66 | 61 | 48 | 45 | 44 | 54 | 69 |
| 36 | -37 | -48 | 44 | 18 | 66 | 10 | 21 | 23 | 19 | 28 | 73 | 58 | 46 | 41 | 33 | 21 |
| 37 | -24 | -24 | 43 | 15 | 43 | -11 | 14 | 1 | 47 | 56 | 74 | 61 | 45 | 63 | 61 | 77 |
| 38 | -52 | -21 | 45 | 16 | 14 | 38 | 30 | 23 | 66 | 27 | 63 | 20 | 14 | 38 | 39 | 49 |
| 39 | -58 | -31 | 38 | 9 | 22 | -10 | 12 | 20 | 10 | 26 | 63 | 61 | 14 | 28 | 21 | 18 |
| 40 | -28 | -50 | 74 | 31 | 56 | -11 | 30 | 18 | 28 | 14 | 62 | 33 | 18 | 29 | 32 | 32 |
| 41 | 19 | -17 | 47 | 28 | 58 | 4 | 6 | -1 | 58 | 46 | 63 | 61 | 33 | 35 | 34 | 33 |
| 42 | -33 | -27 | 45 | 13 | 26 | -10 | 30 | 28 | 11 | 26 | 63 | 42 | 2 | 22 | 26 | 31 |
| 43 | 11 | 0 | 74 | 32 | 32 | -37 | 20 | 14 | 38 | 10 | 55 | 41 | 10 | 23 | 23 | 32 |
| 44 | -10 | -22 | 63 | 10 | 36 | -11 | -6 | -1 | 23 | 36 | 64 | 34 | 10 | 14 | 14 | 27 |
| 45 | 4 | 0 | 74 | 27 | 25 | -14 | 9 | 6 | 32 | 31 | 74 | 58 | 24 | 20 | 22 | 20 |
| 46 | -48 | -44 | 65 | 11 | -18 | -44 | 26 | 18 | 23 | 7 | 62 | 33 | 2 | 16 | 28 | 28 |
| 47 | -34 | -43 | 38 | 17 | 64 | 7 | -2 | -10 | 29 | 19 | 70 | 46 | 13 | 23 | 13 | 11 |

Figure 5C

# Bin 4

| | Compound 1 | | Compound 2 | | Compound 3 | | Compound 4 | | Compound 5 | | Compound 6 | | Compound 7 | | Compound 8 | |
|---|---|---|---|---|---|---|---|---|---|---|---|---|---|---|---|---|
| Clone | Dose 1 | Dose 2 | Dose 1 | Dose 2 | Dose 1 | Dose 2 | Dose 1 | Dose 2 | Dose 1 | Dose 2 | Dose 1 | Dose 2 | Dose 1 | Dose 2 | Dose 1 | Dose 2 |
| 48 | 14 | -22 | 60 | 38 | -6 | -8 | 6 | 37 | 62 | -7 | 25 | -11 | -6 | -8 | -2 | 8 |
| 49 | 30 | 22 | 85 | 58 | 20 | 24 | 30 | 17 | 81 | 25 | 68 | 33 | 30 | 24 | 32 | 25 |
| 50 | -10 | -21 | 73 | 58 | -11 | 16 | 53 | 46 | 55 | -6 | 44 | 21 | -11 | 16 | 27 | 23 |
| 51 | 30 | -5 | 96 | 67 | 5 | 13 | 42 | 33 | 78 | 41 | 56 | 41 | 5 | 13 | 4 | 5 |
| 52 | 49 | 35 | 76 | 64 | -1 | 12 | 38 | 37 | 76 | 7 | 68 | 26 | -1 | 12 | 36 | 31 |
| 53 | 46 | 24 | 86 | 56 | -23 | -18 | 82 | 22 | 80 | -22 | 38 | 18 | -23 | -18 | 6 | 30 |
| 54 | 51 | 41 | 62 | 20 | -4 | -4 | 36 | 23 | 19 | 41 | 56 | 5 | 5 | 27 | 0 | 35 |
| 55 | 63 | 51 | 82 | 78 | 18 | 32 | 63 | 48 | 56 | 5 | 63 | 34 | 18 | 32 | 50 | 48 |
| 56 | 64 | 62 | 97 | 71 | 23 | 12 | 58 | 65 | 81 | 7 | 56 | 27 | 23 | 12 | 20 | 37 |
| 57 | 98 | 88 | 94 | 74 | -2 | 5 | 93 | 88 | 86 | -18 | 81 | 15 | -2 | 5 | 16 | 25 |

Figure 5D

# Bin 5

| | Compound 1 | | Compound 2 | | Compound 3 | | Compound 4 | | Compound 5 | | Compound 6 | | Compound 7 | | Compound 8 | |
|---|---|---|---|---|---|---|---|---|---|---|---|---|---|---|---|---|
| Clone | Dose 1 | Dose 2 | Dose 1 | Dose 2 | Dose 1 | Dose 2 | Dose 1 | Dose 2 | Dose 1 | Dose 2 | Dose 1 | Dose 2 | Dose 1 | Dose 2 | Dose 1 | Dose 2 |
| 58 | 54 | 41 | 90 | 60 | 51 | 42 | 29 | 44 | -111 | -93 | 62 | 62 | -20 | 10 | 14 | 24 |
| 59 | 60 | 46 | 93 | 78 | 77 | 44 | 72 | 62 | -54 | -135 | 38 | 4 | 24 | 23 | 30 | 12 |
| 60 | 47 | 30 | 83 | 65 | 70 | 40 | 58 | 41 | -60 | -51 | 29 | 2 | -17 | 0 | -25 | -32 |

Figure 5E

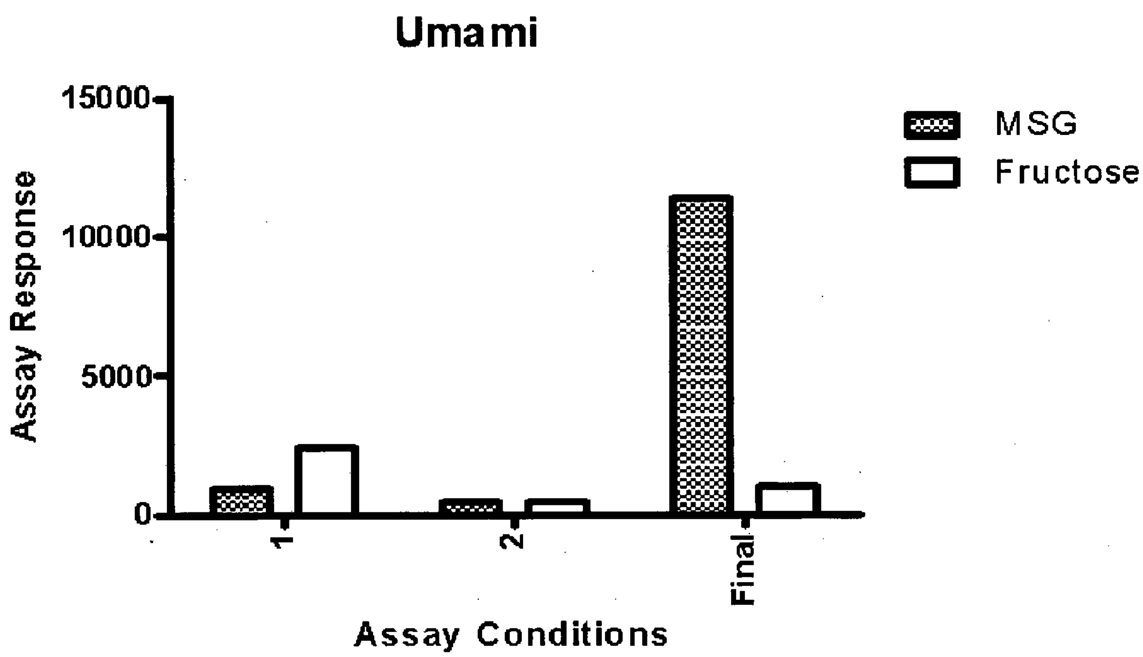
Umami
Assay Response
15000
10000
5000
0
1
2
Final
Assay Conditions
MSG
Fructose

Figure 6

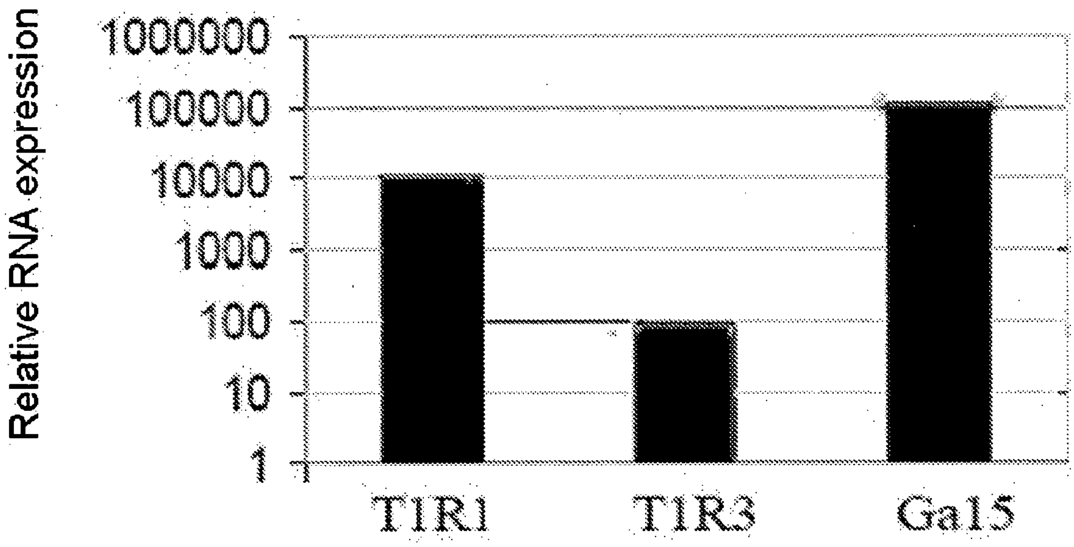
Relative RNA expression
1000000
100000
10000
1000
100
10
1
T1R1
T1R3
Ga15

Figure 7

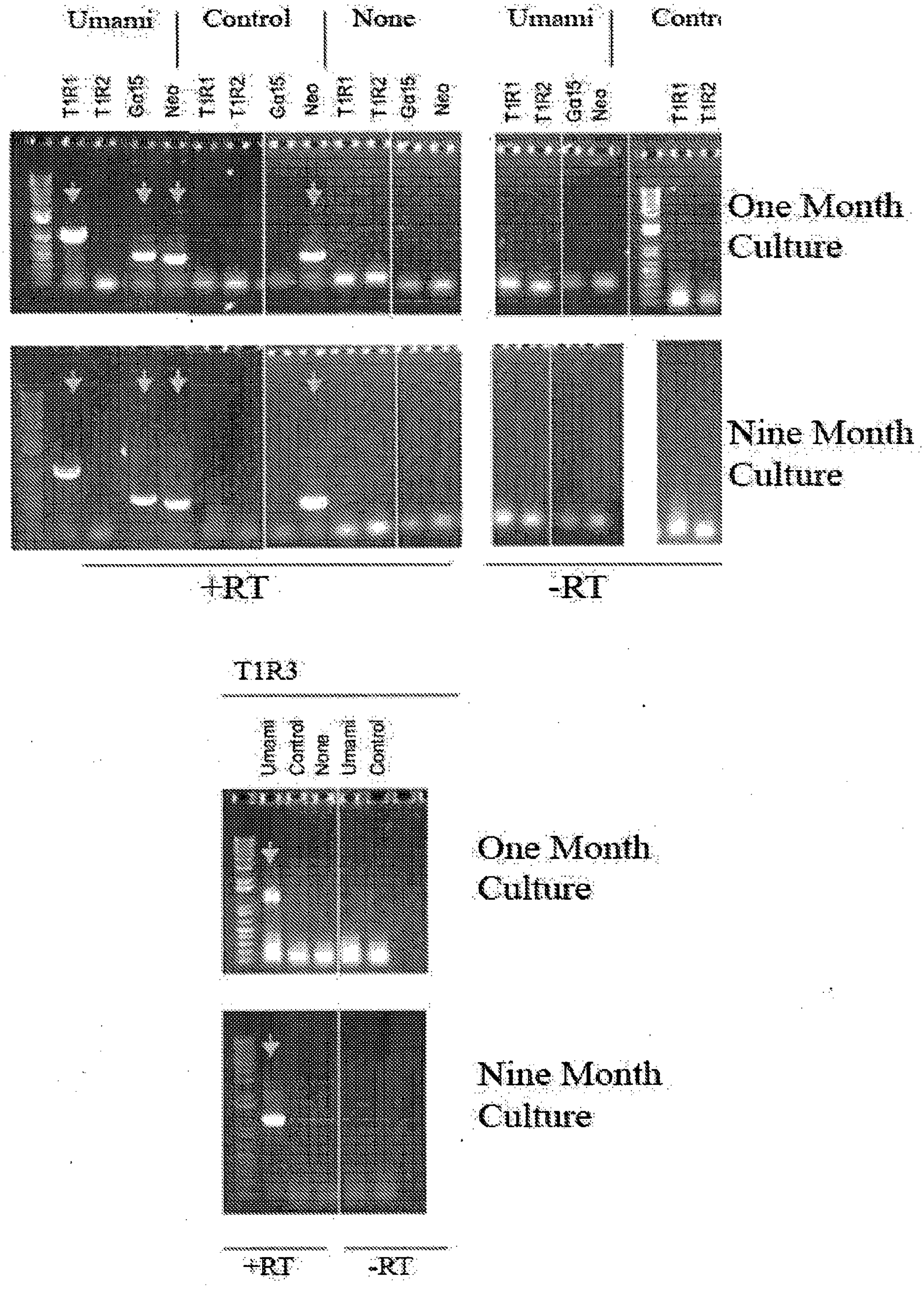
Umami
Control
None
Umami
Contr
T1R1
T1R2
Gα15
Neo
One Month Culture
Nine Month Culture
+RT
-RT
T1R3
Umami
Control
None
Umami
Control
One Month Culture
Nine Month Culture
+RT
-RT

Figure 8

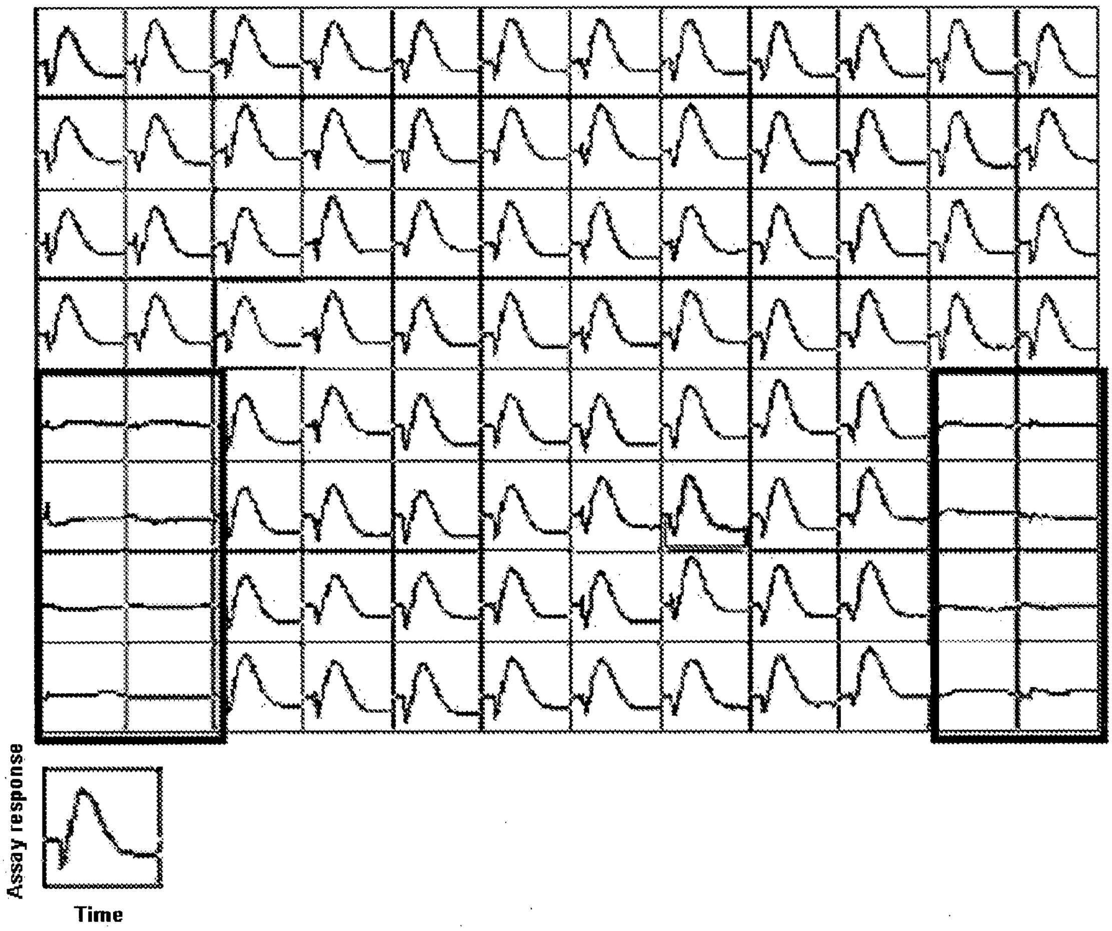


Figure 9

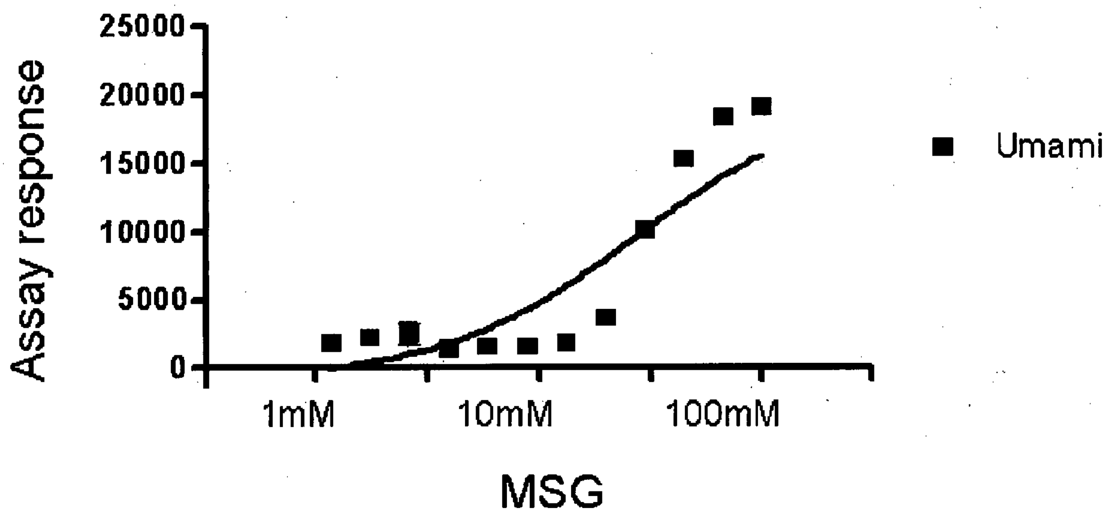
Assay response
25000
20000
15000
10000
5000
0
1mM
10mM
100mM
MSG
Umami

Figure 10

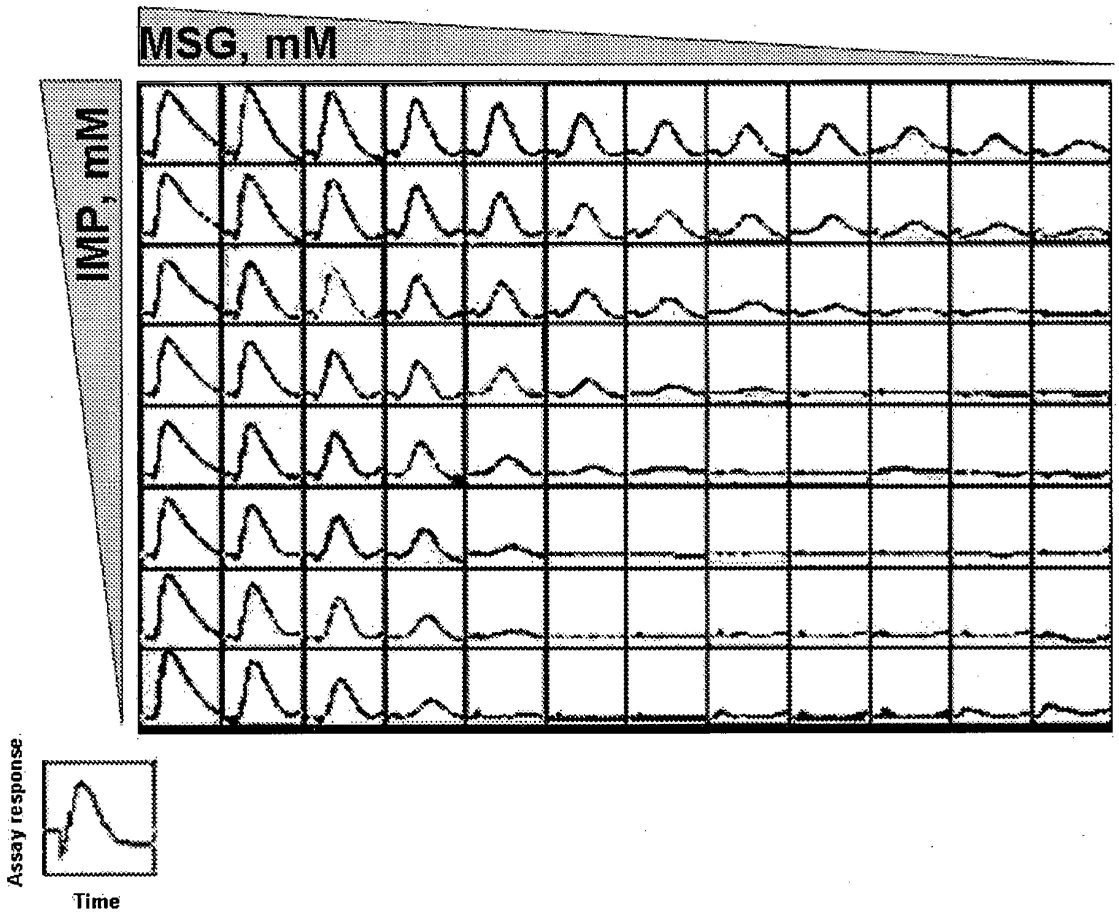


Figure 11

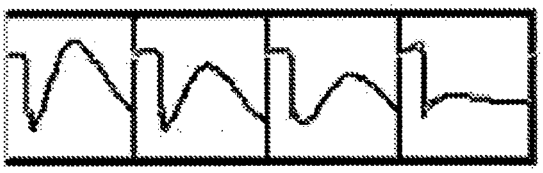


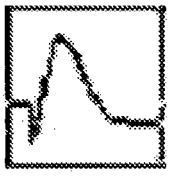


Figure 12

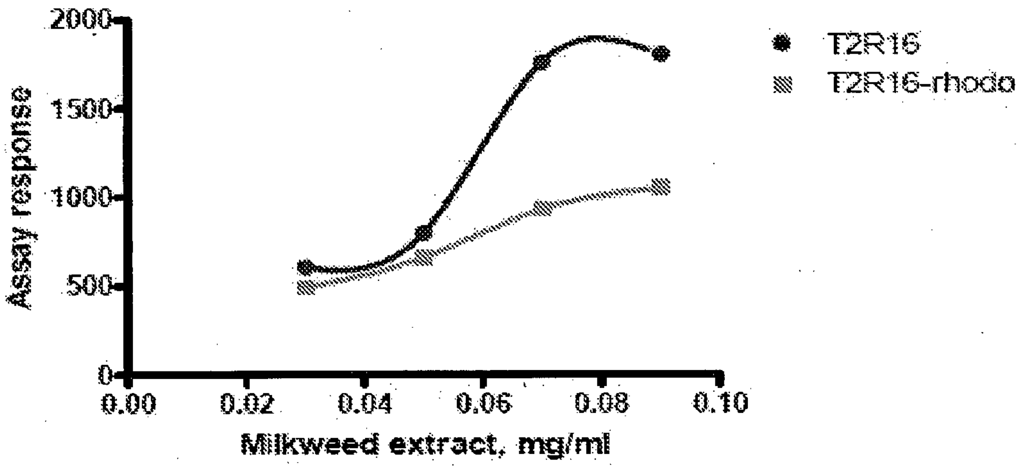
2000
1500
1000
500
0
Assay response
0.00
0.02
0.04
0.06
0.08
0.10
Milkweed extract, mg/ml
T2R16
T2R16-rhodo

Figure 13

| | 1 | 2 | 3 | 4 | 5 | 6 |
|---|---|---|---|---|---|---|
| A | 1400 | | 1013 | - | 1334 | 1029 |
| B | 1145 | 1029 | 1402 | 1567 | | - |
| C | 1529 | 1193 | 1316 | 1331 | 1388 | - |
| D | 1233 | 1231 | - | 1725 | 1288 | 1267 |
| E | 998 | 1358 | 1121 | 1599 | 1356 | |
| F | 1097 | 1310 | - | 1341 | 1515 | 1465 |
| G | 1421 | 1225 | 1053 | 1558 | 1335 | 1430 |
| H | 1172 | 1176 | 1262 | 1036 | 1268 | - |

| | 7 | 8 | 9 | 10 | 11 | 12 |
|---|---|---|---|---|---|---|
| A | - | 1473 | 1373 | | | |
| B | 1293 | 1466 | 1316 | | | |
| C | 1567 | 1371 | 1235 | | | |
| D | 1275 | 1317 | | | | |
| E | 1353 | 1594 | | | | |
| F | 1492 | 1456 | | | | |
| G | 1421 | 1528 | | | | |
| H | 1491 | 1597 | | | | |

Figure 14

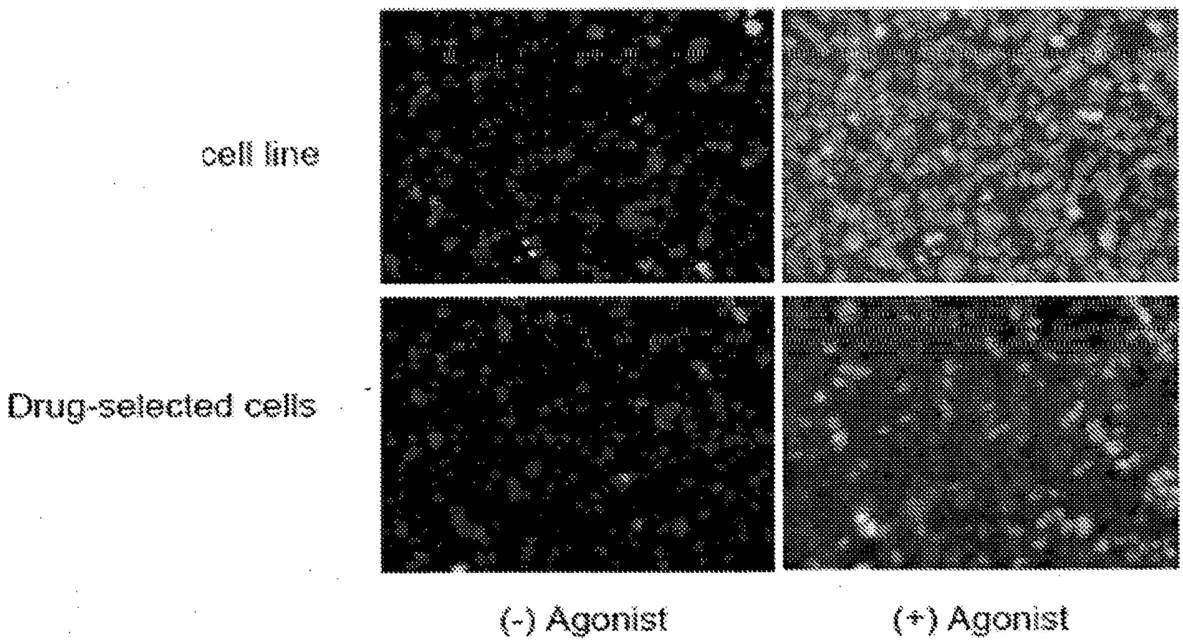


Figure 15

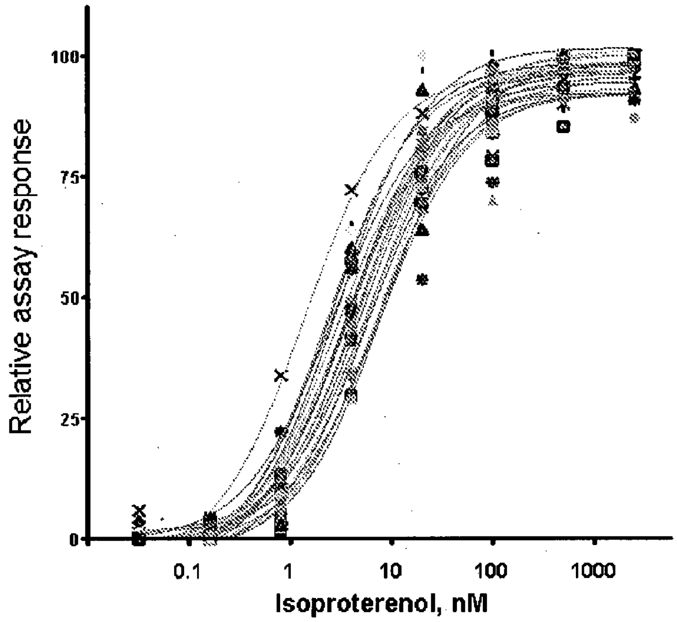


$EC_{50} = 4.9 \pm 0.41$ nM

Figure 16

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| Substance 1 | | | ✓ | | | | | | | | | ✓ | | | ✓ | | | | | ✓ | | | | ✓ | ✓ |
| Substance 2 | | | ✓ | | | | | | ✓ | | | ✓ | | | ✓ | | | | | ✓ | | | | ✓ | ✓ |
| Substance 3 | | | | | | | | ✓ | | | | | | | ✓ | | | | | | ✓ | | | | |
| Substance 4 | ✓ | | ✓ | | | | | | | | | ✓ | | ✓ | ✓ | | | | | | | | | ✓ | ✓ |
| Substance 5 | | | ✓ | | | | | | ✓ | | | | | | | | ✓ | ✓ | | | | | | | |
| Substance 6 | | | | | | | | ✓ | | | | | | | | | | | | | | | | | |
| Substance 7 | | ✓ | | | | ✓ | | | ✓ | | ✓ | | | | | | | | | | | | ✓ | ✓ | |
| Substance 8 | | | | | | | | | | | | | -------- broadly tuned -------- | | | | | | | | | | | | |
| Substance 9 | | | | | | | | | | | | | -------- broadly tuned -------- | | | | | | | | | | | | |
| Substance 10 | | | | | | | | ✓ | | | | | | ✓ | | | | | | | | | | ✓ | |
| Substance 11 | | | ✓ | | | | | ✓ | | | | | | | | | | | | | | | | | |
| Substance 12 | | | | | | | ✓ | | | | | | | ✓ | | | | | | | | | ✓ | | |
| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |

Figure 17

| | F1 | F2 | F3 | F4 | F5 | F6 | F7 | F8 | F9 | F10 | F11 | F12 |
|---|---|---|---|---|---|---|---|---|---|---|---|---|
| 1 | 225% | 59% | 100% | 115% | 1% | 84% | 65% | 107% | 82% | 109% | 1% | 161% |
| 2 | 225% | 93% | 258% | 141% | 123% | 40% | 113% | 97% | 110% | 110% | 5% | 213% |
| 3 | 72% | 76% | 65% | 75% | 86% | 96% | 126% | 27% | 78% | 168% | 68% | 93% |
| 4 | 178% | 44% | 120% | 42% | 87% | 51% | 48% | 76% | 27% | 166% | 63% | 98% |
| 5 | 131% | 38% | 237% | 64% | 143% | 0% | 91% | 63% | 59% | 165% | 9% | 58% |
| 6 | 117% | 30% | 145% | 62% | 156% | 53% | 157% | 94% | 32% | 85% | 6% | 26% |
| 7 | 66% | 77% | 579% | 68% | 147% | 62% | 192% | 28% | 81% | 245% | 2% | 224% |
| 8 | 139% | 0% | 62% | 76% | 50% | 105% | 92% | 72% | 35% | 22% | 25% | 23% |
| 9 | 197% | 425% | 151% | 319% | 473% | 257% | 165% | 40% | 315% | 77% | 681% | 557% |
| 10 | 1076% | 2565% | 1743% | 1339% | 1671% | 215% | 1093% | 741% | 1243% | 1073% | 4532% | 1806% |
| 11 | 2289% | 3441% | 1973% | 1853% | 1658% | 785% | 1196% | 801% | 3788% | 1384% | 2693% | 1842% |
| 12 | 145% | 26% | 207% | 0% | 170% | 0% | 155% | 7% | 13% | 117% | 1% | 57% |
| 13 | 822% | 771% | 963% | 614% | 571% | 642% | 538% | 205% | 1223% | 1253% | 1221% | 377% |
| 14 | 145% | 50% | 263% | 89% | 49% | 35% | 64% | 75% | 98% | 214% | 0% | 103% |
| 15 | 261% | 117% | 730% | 55% | 127% | 396% | 5% | 279% | 195% | 117% | 1201% | 117% |
| 16 | 182% | 27% | 65% | 126% | 70% | 5% | 133% | 116% | 92% | 85% | 11% | 205% |
| 17 | 270% | 42% | 41% | 165% | 89% | 177% | 175% | 81% | 83% | 68% | 34% | 171% |
| 18 | 145% | 62% | 42% | 151% | 133% | 79% | 22% | 9% | 95% | 89% | 0% | 54% |
| 19 | 141% | 3% | 0% | 30% | 95% | 0% | 33% | 46% | 11% | 121% | 0% | 0% |
| 20 | 188% | 112% | 297% | 141% | 180% | 35% | 166% | 19% | 42% | 157% | 11% | 89% |
| 21 | 39% | 0% | 32% | 96% | 41% | 44% | 0% | 4% | 45% | 133% | 9% | 13% |
| 22 | 228% | 0% | 205% | 168% | 114% | 149% | 103% | 6% | 80% | 236% | 0% | 137% |
| 23 | 258% | 27% | 133% | 110% | 130% | 143% | 106% | 114% | 53% | 74% | 108% | 156% |
| 24 | 160% | 175% | 484% | 115% | 172% | 257% | 147% | 98% | 105% | 28% | 163% | 120% |
| 25 | 45% | 0% | 0% | 0% | 28% | 66% | 0% | 3% | 28% | 110% | 0% | 0% |
| 26 | 202% | 30% | 285% | 49% | 123% | 35% | 151% | 125% | 88% | 133% | 4% | 122% |
| 27 | 2335% | 2763% | 2241% | 2302% | 1193% | 1140% | 1303% | 973% | 4042% | 1572% | 2049% | 1998% |
| 28 | 82% | 0% | 185% | 85% | 0% | 63% | 122% | 76% | 4% | 84% | 17% | 60% |
| 29 | 2065% | 2329% | 1028% | 1567% | 832% | 1068% | 793% | 579% | 2091% | 906% | 1146% | 1281% |
| 30 | 325% | 65% | 272% | 175% | 160% | 257% | 334% | 87% | 128% | 259% | 125% | 197% |
| 31 | 119% | 95% | 54% | 109% | 48% | 0% | 49% | 53% | 3% | 32% | 0% | 39% |
| 32 | 69% | 18% | 219% | 4% | 93% | 30% | 48% | 95% | 65% | 156% | 18% | 43% |

Figure 18A

| | F1 | F2 | F3 | F4 | F5 | F6 | F7 | F8 | F9 | F10 | F11 | F12 |
|---|---|---|---|---|---|---|---|---|---|---|---|---|
| 33 | 154% | 3% | 91% | 1% | 0% | 25% | 52% | 50% | 45% | 181% | 0% | 0% |
| 34 | 2119% | 1336% | 2408% | 1987% | 762% | 1376% | 539% | 536% | 1939% | 3795% | 542% | 1206% |
| 35 | 33% | 13% | 123% | 13% | 42% | 15% | 93% | 0% | 12% | 92% | 0% | 35% |
| 36 | 49% | 0% | 129% | 18% | 37% | 316% | 39% | 51% | 0% | 100% | 43% | 74% |
| 37 | 353% | 136% | 145% | 164% | 204% | 254% | 223% | 80% | 139% | 294% | 98% | 222% |
| 38 | 67% | 53% | 76% | 100% | 79% | 90% | 34% | 18% | 70% | 108% | 59% | 37% |
| 39 | 352% | 197% | 71% | 346% | 179% | 184% | 217% | 103% | 138% | 113% | 188% | 90% |
| 40 | 57% | 220% | 198% | 165% | 144% | 214% | 96% | 136% | 47% | 231% | 24% | 96% |
| 41 | 672% | 491% | 1402% | 735% | 513% | 777% | 349% | 432% | 967% | 112% | 832% | 779% |
| 42 | 49% | 0% | 146% | 115% | 0% | 27% | 10% | 66% | 22% | 111% | 103% | 25% |
| 43 | 117% | 26% | 91% | 91% | 198% | 73% | 4% | 90% | 76% | 33% | 0% | 11% |
| 44 | 1205% | 1312% | 1246% | 1360% | 1123% | 865% | 596% | 705% | 880% | 2185% | 1016% | 973% |
| 45 | 662% | 501% | 1377% | 664% | 433% | 536% | 68% | 749% | 805% | 621% | 133% | 862% |
| 46 | 5% | 14% | 39% | 50% | 16% | 290% | 5% | 0% | 22% | 56% | 38% | 38% |
| 47 | 225% | 81% | 173% | 66% | 131% | 227% | 197% | 21% | 81% | 114% | 57% | 88% |
| 48 | 124% | 157% | 492% | 124% | 112% | 439% | 257% | 241% | 173% | 236% | 158% | 210% |
| 49 | 590% | 347% | 611% | 446% | 469% | 253% | 348% | 274% | 412% | 708% | 501% | 347% |
| 50 | 184% | 67% | 134% | 219% | 203% | 93% | 184% | 105% | 44% | 270% | 158% | 199% |
| 51 | 352% | 508% | 1196% | 216% | 288% | 366% | 106% | 421% | 520% | 806% | 227% | 317% |
| 52 | 174% | 123% | 293% | 105% | 111% | 71% | 187% | 128% | 91% | 213% | 0% | 100% |
| 53 | 172% | 92% | 166% | 115% | 123% | 85% | 113% | 157% | 116% | 327% | 54% | 108% |
| 54 | 233% | 397% | 566% | 156% | 360% | 197% | 239% | 408% | 174% | 1020% | 14% | 444% |
| 55 | 189% | 94% | 283% | 98% | 95% | 87% | 90% | 133% | 31% | 185% | 0% | 29% |
| 56 | 170% | 125% | 122% | 179% | 74% | 65% | 55% | 59% | 32% | 153% | 31% | 68% |
| 57 | 112% | 0% | 112% | 130% | 133% | 148% | 186% | 64% | 112% | 167% | 145% | 70% |
| 58 | 102% | 0% | 129% | 69% | 145% | 20% | 54% | 8% | 127% | 269% | 62% | 38% |
| 59 | 109% | 38% | 285% | 86% | 200% | 10% | 179% | 67% | 142% | 296% | 0% | 79% |
| 60 | 2604% | 1068% | 3263% | 2858% | 1247% | 1348% | 1570% | 1181% | 2471% | 1352% | 1917% | 1978% |
| 61 | 305% | 221% | 246% | 320% | 339% | 179% | 284% | 246% | 272% | 1032% | 44% | 169% |
| 62 | 175% | 1% | 155% | 90% | 64% | 32% | 72% | 131% | 207% | 327% | 0% | 58% |
| 63 | 154% | 49% | 206% | 53% | 207% | 51% | 142% | 144% | 140% | 387% | 45% | 70% |

Figure 18B

| | F13 | F14 | F15 | F16 | F17 | F18 | F19 | F20 | F21 | F22 | F23 | F24 | F25 |
|---|---|---|---|---|---|---|---|---|---|---|---|---|---|
| 1 | 90% | 49% | 144% | 103% | 207% | 153% | 161% | 95% | 159% | 53% | 92% | 223% | 160% |
| 2 | 57% | 12% | 177% | 31% | 84% | 141% | 118% | 235% | 55% | 263% | 93% | 275% | 37% |
| 3 | 0% | 0% | 88% | 6% | 0% | 57% | 128% | 5% | 23% | 96% | 49% | 18% | 0% |
| 4 | 142% | 28% | 102% | 192% | 160% | 199% | 9% | 105% | 0% | 39% | 166% | 148% | 27% |
| 5 | 203% | 111% | 38% | 168% | 143% | 137% | 120% | 3% | 34% | 54% | 47% | 156% | 47% |
| 6 | 32% | 0% | 28% | 42% | 52% | 74% | 32% | 0% | 0% | 118% | 165% | 165% | 44% |
| 7 | 59% | 0% | 141% | 36% | 67% | 91% | 45% | 131% | 51% | 153% | 38% | 98% | 93% |
| 8 | 26% | 0% | 0% | 0% | 45% | 79% | 90% | 47% | 4% | 97% | 70% | 140% | 34% |
| 9 | 158% | 196% | 914% | 147% | 615% | 413% | 141% | 318% | 191% | 215% | 513% | 200% | 195% |
| 10 | 2442% | 521% | 1980% | 1076% | 2521% | 769% | 427% | 1219% | 4579% | 900% | 2484% | 656% | 1185% |
| 11 | 1884% | 1892% | 4154% | 1803% | 1390% | 2319% | 623% | 2053% | 1053% | 3252% | 1109% | 1452% | 936% |
| 12 | 36% | 0% | 22% | 70% | 46% | 147% | 14% | 0% | 34% | 92% | 115% | 135% | 49% |
| 13 | 1687% | 1803% | 4472% | 2053% | 1236% | 1185% | 652% | 760% | 1023% | 739% | 625% | 1053% | 710% |
| 14 | 213% | 163% | 281% | 220% | 185% | 172% | 34% | 107% | 30% | 98% | 182% | 167% | 123% |
| 15 | 201% | 0% | 85% | 181% | 288% | 145% | 43% | 106% | 99% | 612% | 592% | 110% | 135% |
| 16 | 2% | 0% | 89% | 30% | 121% | 124% | 46% | 183% | 17% | 150% | 56% | 110% | 39% |
| 17 | 120% | 0% | 50% | 194% | 196% | 113% | 152% | 176% | 52% | 183% | 56% | 145% | 162% |
| 18 | 146% | 39% | 59% | 43% | 113% | 102% | 0% | 141% | 33% | 89% | 113% | 34% | 49% |
| 19 | 92% | 0% | 190% | 47% | 111% | 163% | 26% | 161% | 54% | 12% | 22% | 36% | 35% |
| 20 | 93% | 53% | 113% | 106% | 89% | 338% | 21% | 135% | 34% | 153% | 153% | 80% | 125% |
| 21 | 19% | 51% | 7% | 271% | 39% | 127% | 66% | 77% | 44% | 64% | 164% | 9% | 25% |
| 22 | 417% | 217% | 92% | 524% | 297% | 104% | 250% | 354% | 73% | 179% | 142% | 236% | 165% |
| 23 | 138% | 6% | 91% | 173% | 213% | 181% | 158% | 36% | 142% | 66% | 65% | 129% | 230% |
| 24 | 27% | 0% | 183% | 0% | 51% | 270% | 0% | 170% | 88% | 297% | 89% | 142% | 19% |
| 25 | 91% | 8% | 0% | 35% | 51% | 109% | 69% | 1% | 0% | 0% | 0% | 0% | 0% |
| 26 | 107% | 0% | 198% | 99% | 106% | 216% | 121% | 6% | 103% | 14% | 46% | 136% | 217% |
| 27 | 1833% | 2214% | 4258% | 2410% | 2779% | 2686% | 687% | 2140% | 1347% | 2111% | 705% | 1306% | 1262% |
| 28 | 33% | 29% | 76% | 44% | 84% | 71% | 30% | 50% | 40% | 2% | 60% | 82% | 110% |
| 29 | 1348% | 1804% | 3266% | 1427% | 1953% | 1886% | 342% | 2524% | 815% | 2028% | 289% | 1375% | 1017% |
| 30 | 350% | 82% | 174% | 326% | 219% | 157% | 164% | 114% | 237% | 132% | 195% | 81% | 248% |
| 31 | 14% | 22% | 0% | 13% | 57% | 43% | 19% | 4% | 30% | 60% | 68% | 39% | 106% |
| 32 | 86% | 38% | 27% | 107% | 73% | 167% | 47% | 65% | 0% | 46% | 49% | 182% | 48% |

Figure 18C

| | F13 | F14 | F15 | F16 | F17 | F18 | F19 | F20 | F21 | F22 | F23 | F24 | F25 |
|---|---|---|---|---|---|---|---|---|---|---|---|---|---|
| 33 | 95% | 34% | 79% | 71% | 97% | 0% | 39% | 171% | 9% | 56% | 114% | 95% | 96% |
| 34 | 3945% | 1970% | 820% | 3813% | 2335% | 2132% | 852% | 1388% | 2341% | 1059% | 1436% | 254% | 1032% |
| 35 | 9% | 0% | 51% | 38% | 54% | 63% | 36% | 121% | 35% | 7% | 0% | 211% | 24% |
| 36 | 9% | 0% | 34% | 172% | 50% | 144% | 42% | 29% | 39% | 130% | 135% | 181% | 157% |
| 37 | 340% | 179% | 158% | 429% | 280% | 266% | 195% | 244% | 247% | 67% | 219% | 115% | 210% |
| 38 | 115% | 100% | 46% | 1% | 120% | 170% | 77% | 152% | 35% | 84% | 3% | 47% | 121% |
| 39 | 18% | 28% | 187% | 75% | 145% | 265% | 281% | 13% | 152% | 419% | 59% | 191% | 0% |
| 40 | 161% | 11% | 234% | 73% | 149% | 52% | 0% | 126% | 50% | 437% | 52% | 295% | 101% |
| 41 | 147% | 135% | 1027% | 25% | 224% | 832% | 426% | 719% | 172% | 1134% | 35% | 593% | 776% |
| 42 | 26% | 0% | 56% | 69% | 198% | 82% | 104% | 66% | 39% | 54% | 63% | 58% | 135% |
| 43 | 25% | 641% | 104% | 11% | 545% | 0% | 66% | 141% | 61% | 220% | 26% | 190% | 128% |
| 44 | 2018% | 1368% | 1422% | 2403% | 1842% | 1394% | 838% | 700% | 1286% | 1639% | 1050% | 872% | 896% |
| 45 | 386% | 250% | 811% | 250% | 323% | 303% | 178% | 81% | 395% | 1061% | 403% | 166% | 242% |
| 46 | 23% | 0% | 79% | 249% | 74% | 383% | 395% | 196% | 247% | 183% | 112% | 163% | 106% |
| 47 | 181% | 52% | 66% | 153% | 72% | 372% | 51% | 111% | 78% | 89% | 132% | 108% | 142% |
| 48 | 96% | 198% | 101% | 198% | 172% | 375% | 205% | 51% | 166% | 412% | 142% | 140% | 147% |
| 49 | 440% | 268% | 389% | 517% | 262% | 728% | 579% | 282% | 633% | 332% | 301% | 249% | 731% |
| 50 | 157% | 281% | 298% | 130% | 53% | 157% | 147% | 165% | 145% | 271% | 209% | 87% | 0% |
| 51 | 309% | 147% | 434% | 242% | 227% | 873% | 161% | 184% | 301% | 896% | 316% | 282% | 99% |
| 52 | 180% | 21% | 114% | 85% | 59% | 386% | 245% | 138% | 209% | 41% | 26% | 230% | 182% |
| 53 | 256% | 76% | 169% | 247% | 71% | 293% | 300% | 111% | 169% | 163% | 232% | 95% | 180% |
| 54 | 324% | 240% | 402% | 409% | 52% | 589% | 133% | 141% | 206% | 321% | 162% | 62% | 297% |
| 55 | 85% | 2% | 157% | 89% | 19% | 131% | 141% | 12% | 67% | 90% | 75% | 85% | 168% |
| 56 | 146% | 99% | 139% | 102% | 78% | 271% | 202% | 4% | 178% | 119% | 56% | 232% | 204% |
| 57 | 153% | 86% | 106% | 156% | 101% | 300% | 146% | 7% | 50% | 165% | 104% | 88% | 52% |
| 58 | 50% | 11% | 88% | 75% | 106% | 270% | 74% | 70% | 32% | 71% | 24% | 131% | 2% |
| 59 | 249% | 11% | 3% | 179% | 153% | 395% | 154% | 70% | 8% | 53% | 25% | 41% | 92% |
| 60 | 3144% | 1096% | 1941% | 847% | 1395% | 1737% | 1862% | 923% | 2472% | 3124% | 1080% | 1647% | 2273% |
| 61 | 317% | 376% | 169% | 608% | 368% | 868% | 177% | 117% | 5% | 254% | 320% | 236% | 146% |
| 62 | 130% | 12% | 95% | 102% | 76% | 265% | 225% | 111% | 47% | 32% | 106% | 35% | 118% |
| 63 | 164% | 37% | 58% | 170% | 81% | 341% | 122% | 0% | 27% | 185% | 67% | 77% | 86% |

Figure 18D

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 |
|---|---|---|---|---|---|---|---|---|---|---|---|---|---|---|---|---|---|---|---|---|---|---|---|---|---|
| Untagged | | | ✓ | | | | | | ✓ | | | | | | | | ✓ | ✓ | | | | | | | |
| Tagged | | | | | | ✓ | | | | | | ✓ | | | | | | | | | | | | | |

Figure 19

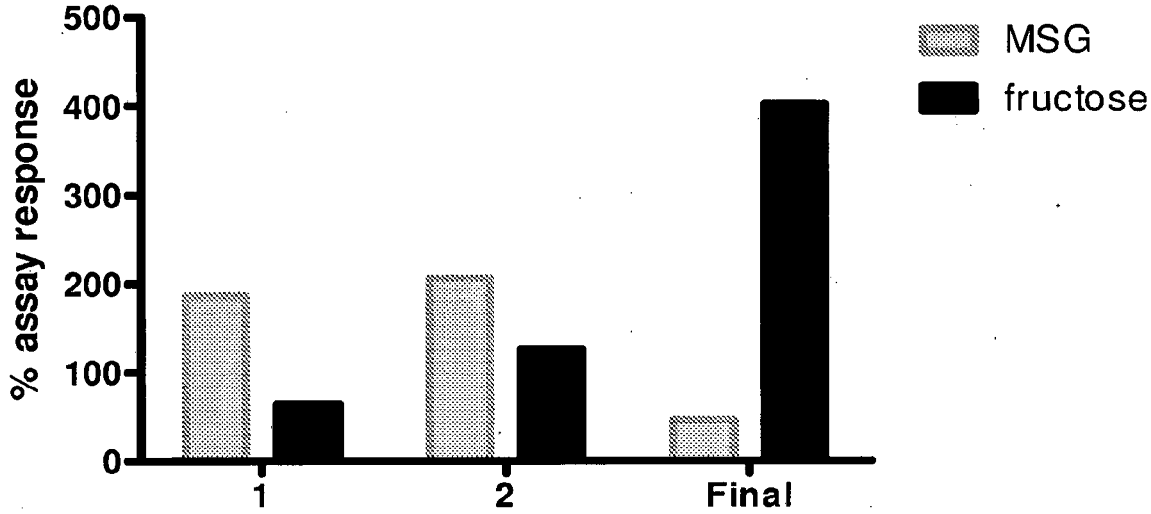


Figure 20

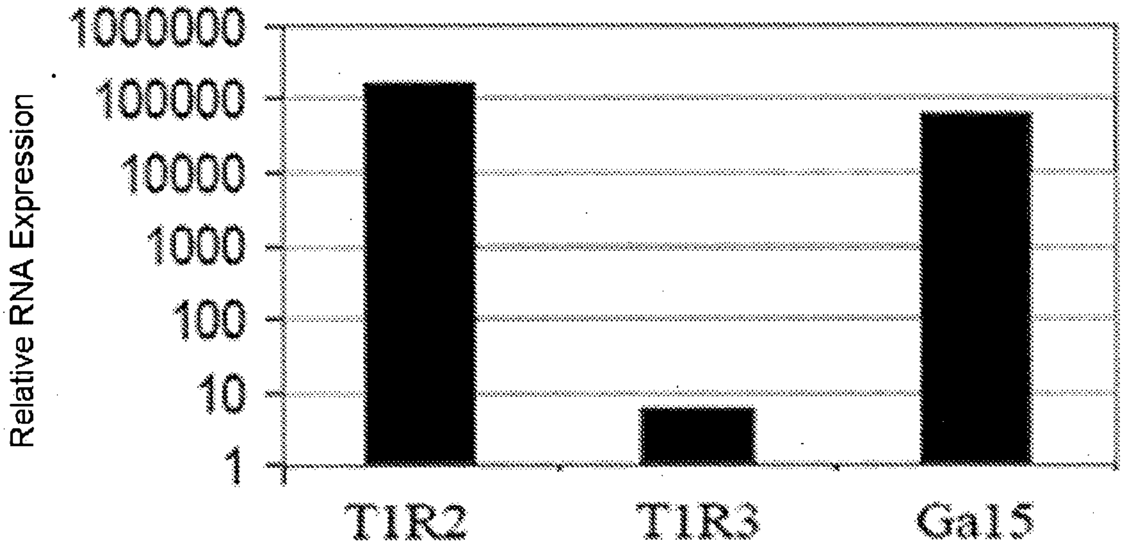
Relative RNA Expression
1000000
100000
10000
1000
100
10
1
T1R2
T1R3
Ga15

Figure 21

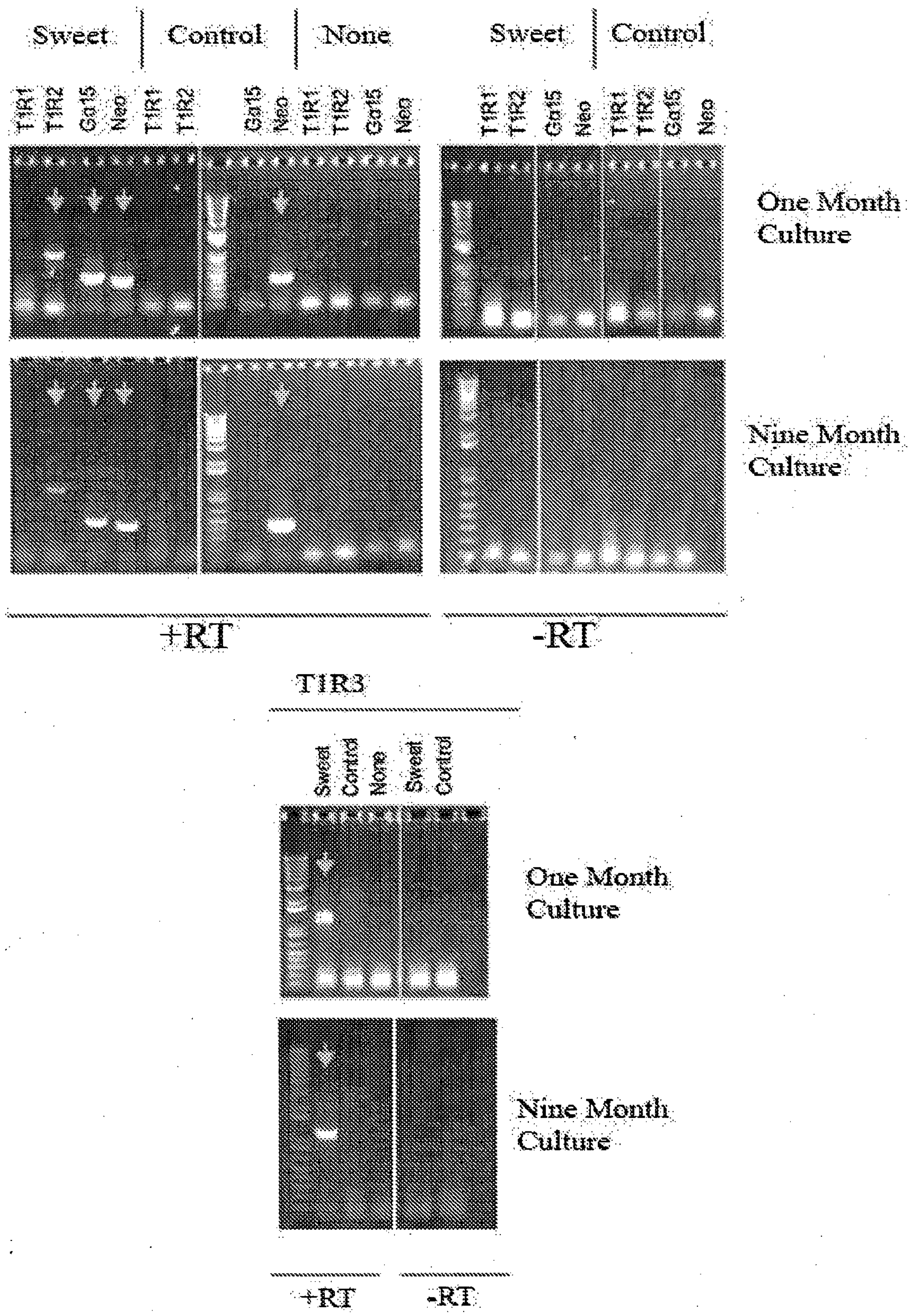

Sweet
Control
None
Sweet
Control
T1R1
T1R2
Gα15
Neo
T1R1
T1R2
Gα15
Neo
T1R1
T1R2
Gα15
Neo
T1R1
T1R2
Gα15
Neo
T1R1
T1R2
Gα15
Neo
One Month Culture
Nine Month Culture
+RT
-RT
T1R3
Sweet
Control
None
Sweet
Control
One Month Culture
Nine Month Culture
+RT
-RT


Figure 22

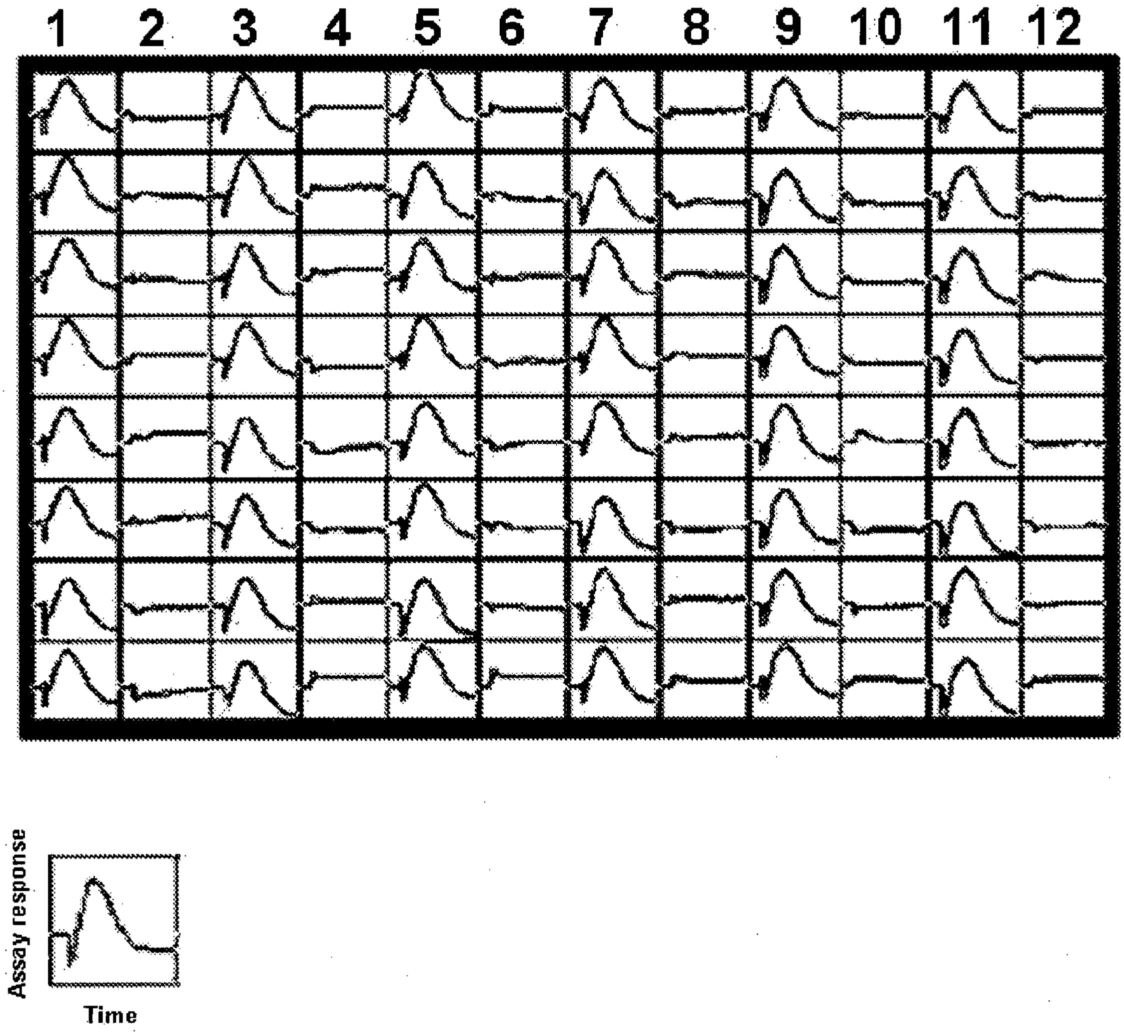


Figure 23

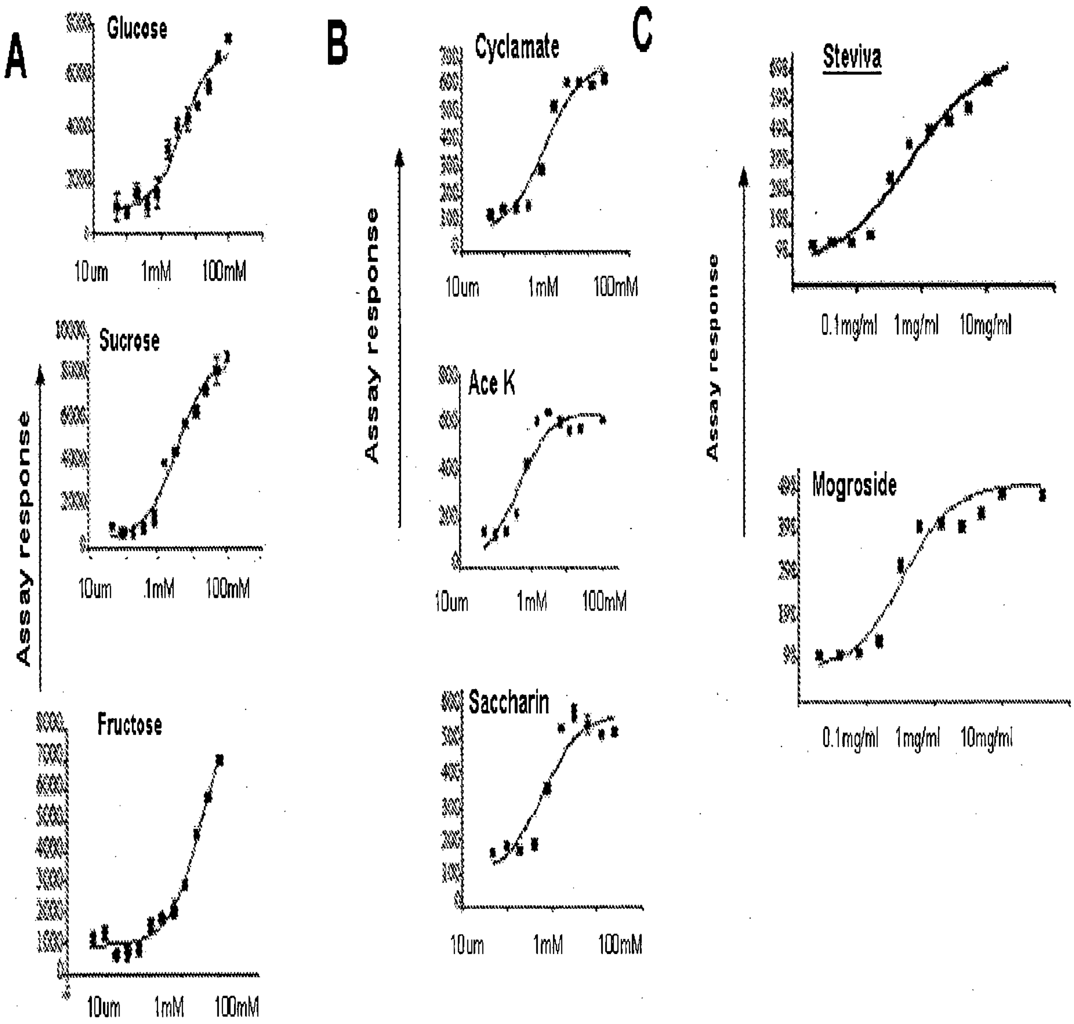
A
Glucose
10um 1mM 100mM
Sucrose
10um 1mM 100mM
Assay response
Fructose
10um 1mM 100mM
B
Cyclamate
10um 1mM 100mM
Assay response
Ace K
10um 1mM 100mM
Saccharin
10um 1mM 100mM
C
Steviva
0.1mg/ml 1mg/ml 10mg/ml
Assay response
Mogroside
0.1mg/ml 1mg/ml 10mg/ml

Figure 24

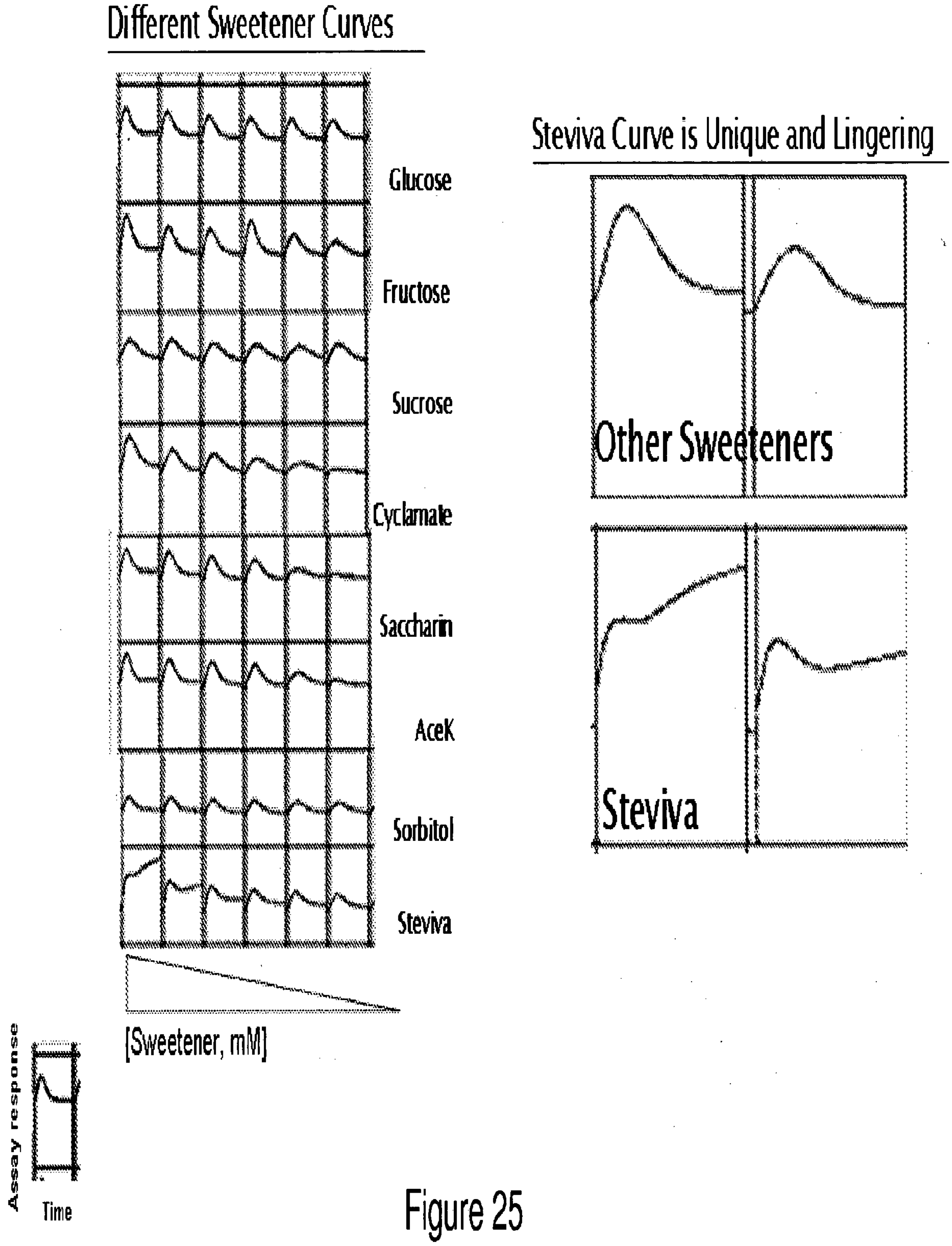
Different Sweetener Curves
Glucose
Fructose
Sucrose
Cyclamate
Saccharin
AceK
Sorbitol
Steviva
[Sweetener, mM]
Assay response
Time
Steviva Curve is Unique and Lingering
Other Sweeteners
Steviva

Figure 25

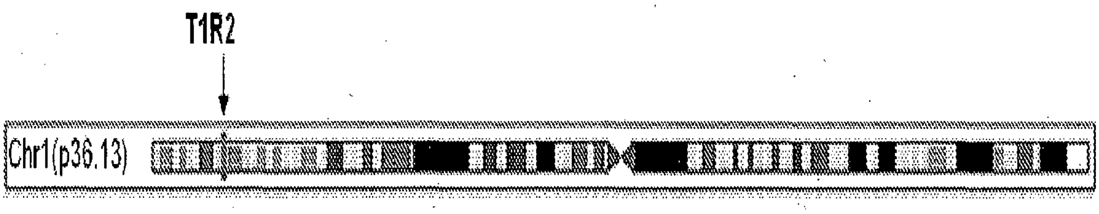


Figure 26A

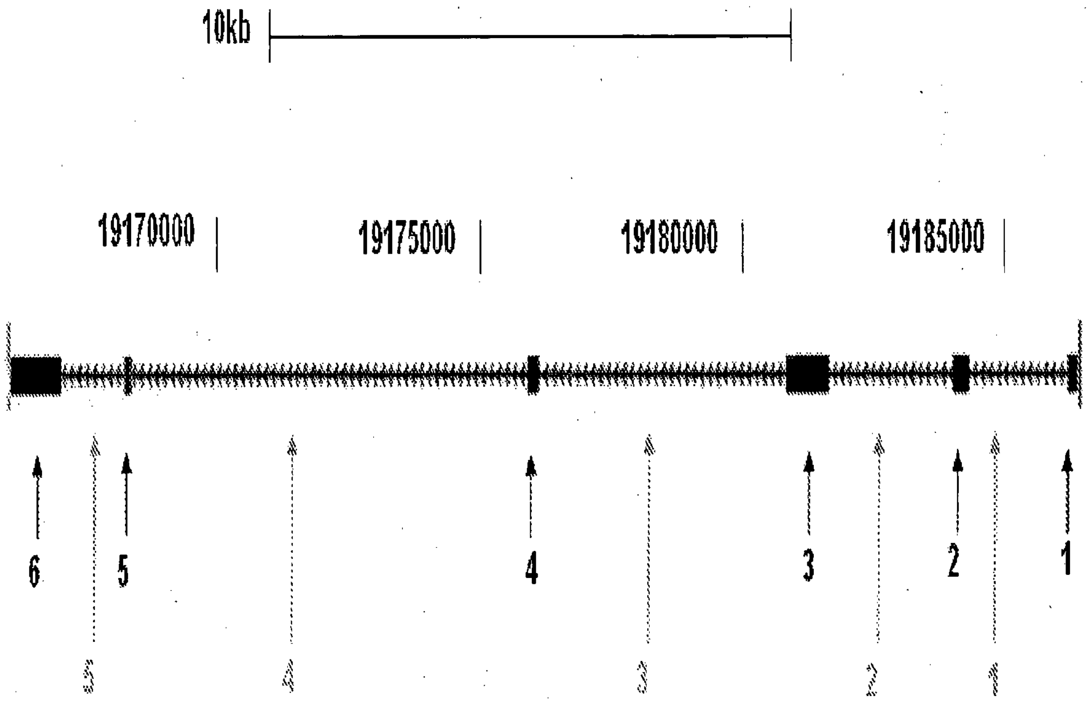


Figure 26B

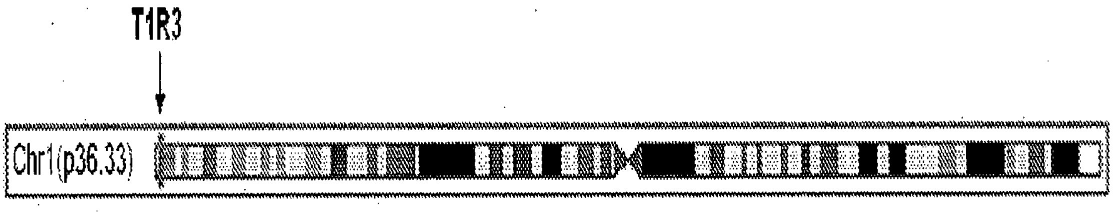


Figure 27A

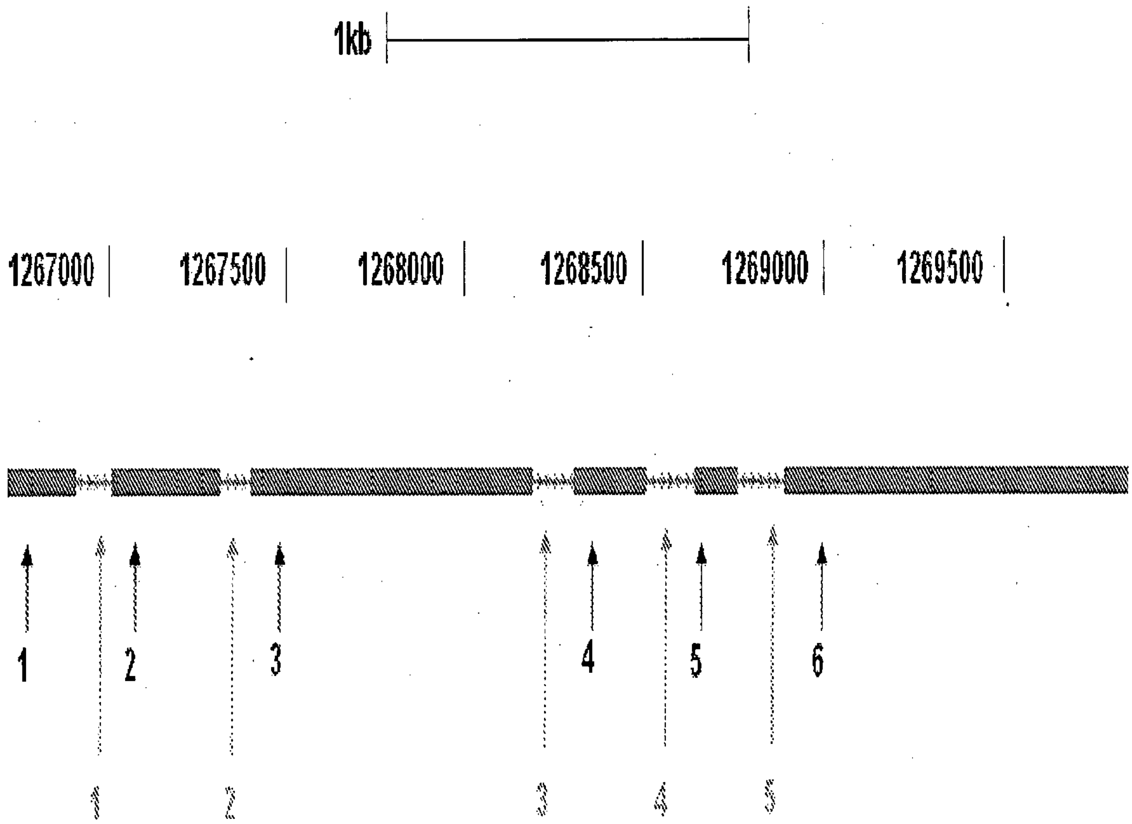
1kb
1267000
1267500
1268000
1268500
1269000
1269500
1
2
3
4
5
6
1
2
3
4
5

Figure 27B

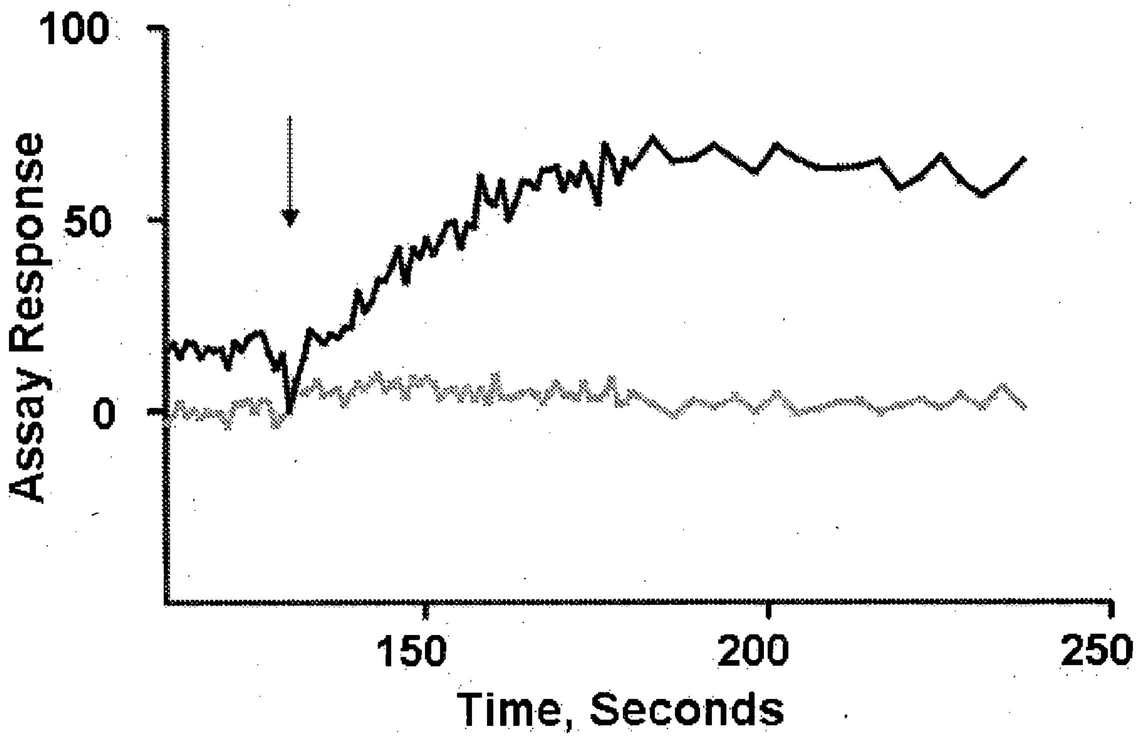


Figure 28A

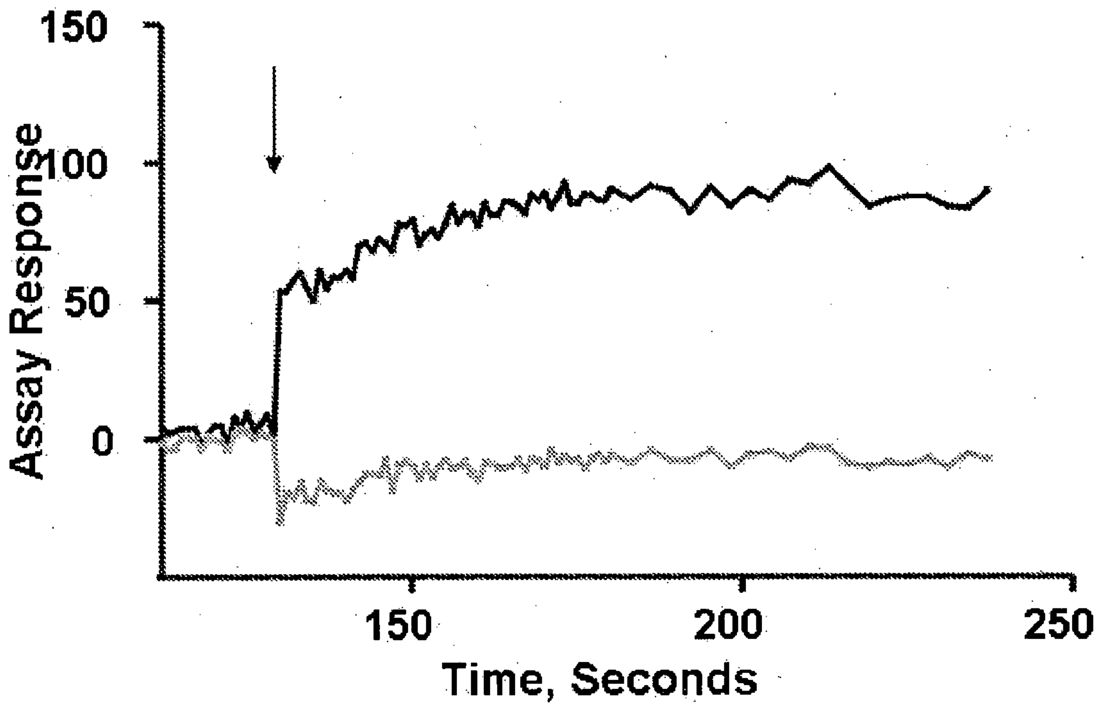


Figure 28B

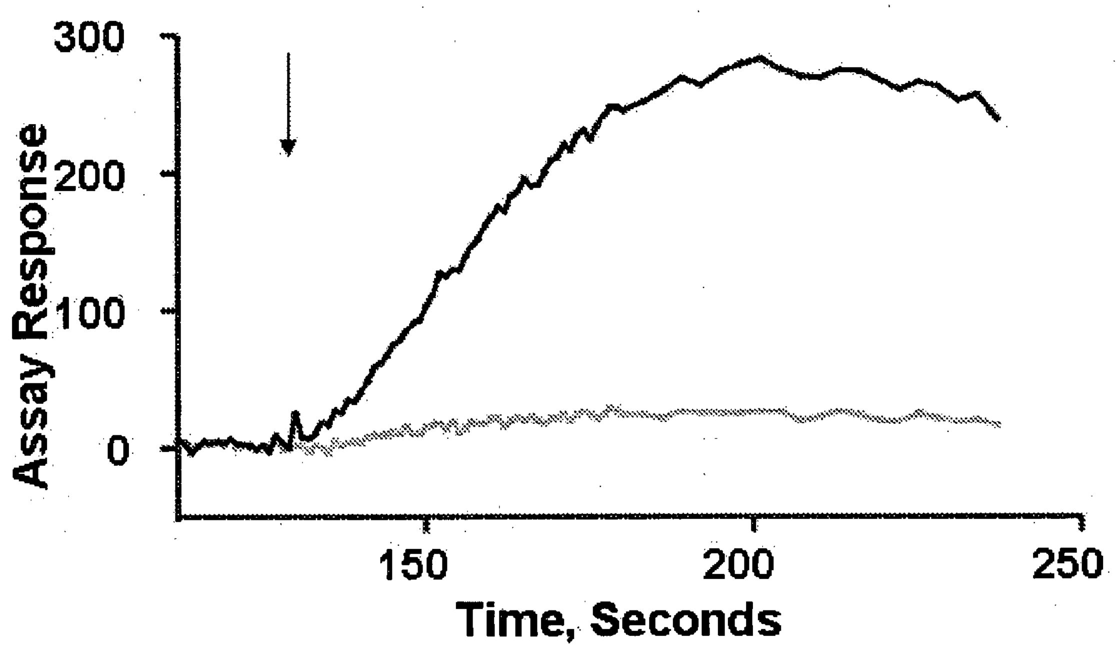


Figure 28C

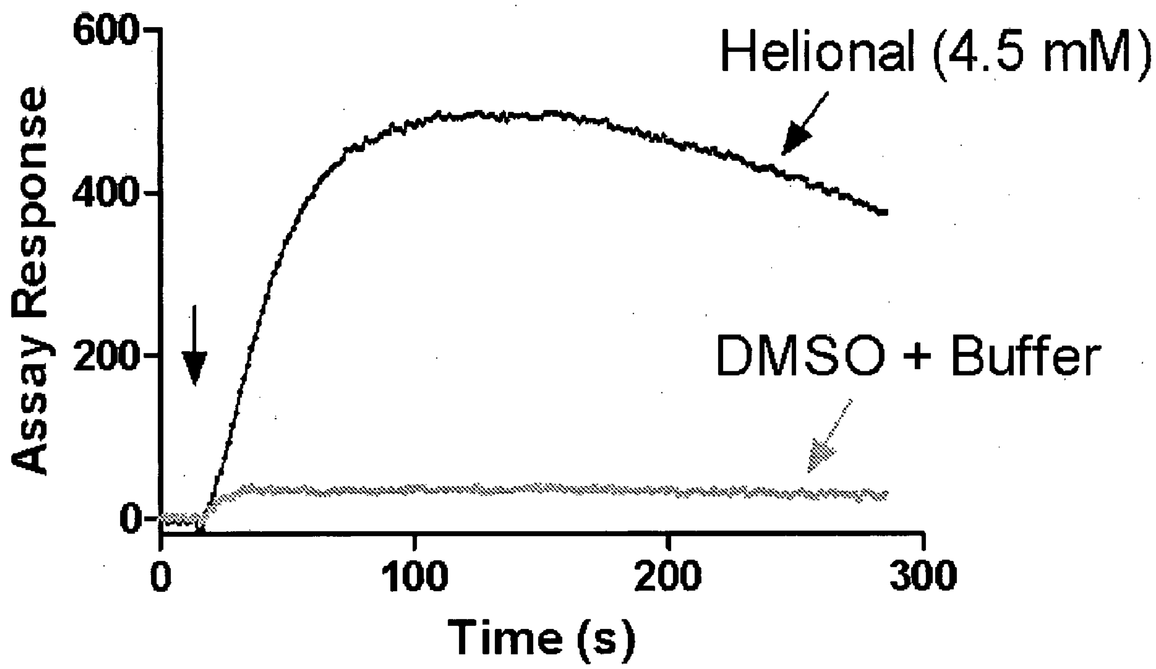
Helional (4.5 mM)
DMSO + Buffer
Assay Response
600
400
200
0
0
100
200
300
Time (s)

Figure 29A

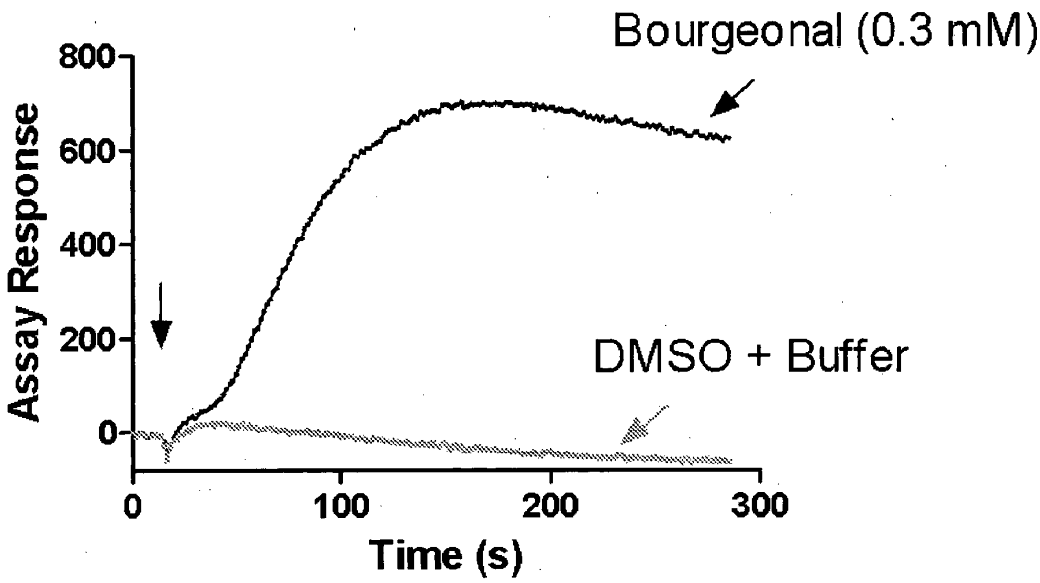
Bourgeonal (0.3 mM)
DMSO + Buffer
Assay Response
800
600
400
200
0
0
100
200
300
Time (s)

Figure 29B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2008262087 A
- WO 2009102569 A
- US 2006147937 A
- US 0931936 W
- US 6692965 B
- WO 2005079462 A
- US 05005080 W
- WO 9412650 A
- US 2005005080 W
- WO 1994012650 A
- US 61149321 B
- US 61149318 B
- US 61149324 B
- US 61149311 B
- US 61230536 B
- US 61235181 B

### Non-patent literature cited in the description


- **TOYONO et al.** CCAAT/Enhancer-binding protein beta regulates expression of human T1R3 taste receptor gene in the bile duct carcinoma cell line, HuCCT1. *Biochemica et Biophysica Acta,* 2007, vol. 1769, 641-648
- Current Protocols in Molecular Biology. John Wiley & Sons, 1989, 6.3.1-6.3.6
- **ALTSHUL et al.** *J. Mol. Biol.,* 1990, vol. 215, 403-410
- **NEEDLEMAN et al.** *J. Mol. Biol.,* 1970, vol. 48, 444-453
- **MEYERS et al.** *Comput. Appl. Biosci.,* 1988, vol. 4, 11-17
- **E. MEYERS ; W. MILLER.** *CABIOS,* 1989, vol. 4, 11-17
- **PEARSON.** *Methods Enzymol.,* 1990, vol. 183, 63-98
- **PEARSON.** *Methods Mol. Biol.,* 2000, vol. 132, 185-219
- **PAUL D. THOMAS ; MICHAEL J. CAMPBELL ; ANISH KEJARIWAL ; HUAIYU MI ; BRIAN KARLAK ; ROBIN DAVERMAN ; KAREN DIEMER ; ANUSHYA MURUGANUJAN ; APURVA NARECHANIA.** PANTHER: a library of protein families and subfamilies indexed by function. *Genome Res.,* 2003, vol. 13, 2129-2141
- **PEARSON.** *Methods Mol. Biol.,* 1994, vol. 243, 307-31
- **GONNET et al.** *Science,* 1992, vol. 256, 1443-45
- **ROBINSON et al.** *Biochemistry,* 1997, vol. 36 (37), 11169-11178
- **DEFFIE et al.** *Cancer Res.,* 1988, vol. 48 (13), 3595-3602
- **THIEBAUT et al.** *J Histochem Cytochem.,* 1990, vol. 38 (5), 685-690
- **ROEPE et al.** *Biochemistry,* 1993, vol. 32 (41), 11042-11056
- **SIMON et al.** *Proc Natl Acad Sci USA.,* 1994, vol. 91 (3), 1128-1132
- **SCHINDLER et al.** *Biochemistry,* 1996, vol. 35 (9), 2811-2817
- **ALTAN et al.** *J Exp Med.,* 1998, vol. 187 (10), 1583-1598
- **GILL et al.** *Cell,* 1992, vol. 71 (1), 23-32
- **ABRAHAM et al.** *Proc Natl Acad Sci USA.,* 1993, vol. 90 (1), 312-316
- **ALTAN et al.** *Proc Natl Acad Sci USA.,* 1999, vol. 96 (8), 4432-4437
- **ZHANG JH ; CHUNG TD ; OLDENBURG KR.** A Simple Statistical Parameter for Use in Evaluation and Validation of High Throughput Screening Assays. *J. Biomol. Screen.,* 1999, vol. 4 (2), 67-73
- **DRAGHICI.** Data Analysis Tools for DNA Microarrays. Chapman & Hall, CRC Press, 2003
- **CONOVER.** Practical Nonparametric Statistics. Wiley, 1971
- **PIERCE.** *An Introduction To Information Theory: Symbols, Signals, and Noise,* 1980
- **DUDA et al.** Pattern Classification. John Wiley & Sons, Inc, 2001
- **HASTIE et al.** The Elements of Statistical Learning. Springer-Verlag, 2001
- **DRAGHICI.** Data Analysis Tools for DNA Microarrays. Chapman & Hall/CRC, 2003
- **MOUNT.** Bioinformatics: sequence and genome analysis. Cold Spring Harbor Laboratory Press, 2001

- **DUDA ; HART.** Pattern Classification and Scene Analysis. John Wiley & Sons, Inc, 1973, 211-256
- **DUDA et al.** Pattern Classification. John Wiley & Sons, Inc, 537-563
- **KAUFMAN ; ROUSSEEUW.** Finding Groups in Data: An Introduction to ClusterAnalysis. Wiley, 1990
- **EVERITT.** Cluster analysis. Wiley, 1993
- **BACKER.** Computer-Assisted Reasoning in Cluster-Analysis. Prentice Hall, 1995
- **JOLLIFFE.** Principal Component Analysis. Springer, 1986
- **KUBINYI.** 3D QSAR in drug design theory methods and applications. Pergamon Press, 1990, 589-638
- **DUDA.** Pattern Classification. John Wiley & Sons, Inc, 2001
- **HASTIE.** The Elements of Statistical Learning. Springer, 2001
- **VENABLES ; RIPLEY.** Modern Applied Statistics with s-plus. Springer, 1997
- **CRISTIANINI ; SHAWE-TAYLOR.** An Introduction to Support Vector Machines. Cambridge University Press, 2000
- A training algorithm for optimal margin classifiers. **BOSER et al.** Proceedings of the 5th Annual ACM Workshop on Computational Learning Theory. ACM Press, 1992, 142-152
- **VAPNIK.** Statistical Learning Theory. Wiley, 1998
- **DUDA.** Pattern Classification. John Wiley & Sons, Inc, 2001
- **FUREY et al.** *Bioinformatics,* 2000, vol. 16, 906-914
- **DUDA.** Pattern Classification. John Wiley & Sons, Inc, 2001, 395-396
- **DUDA.** Pattern Classification. John Wiley & Sons, Inc, 2001, 396-408, 411-412
- Random Forests - Random Features. **BREIMAN.** Technical Report 567. 1999
- **DUDA.** Pattern Classification. John Wiley & Sons, Inc, 2001, 408-409
- **HASTIE et al.** The Elements of Statistical Learning. Springer-Verlag, 2001, 283-295
- **TIBSHIRANI et al.** *Proceedings of the National Academy of Science USA,* 2002, vol. 99, 6567-6572
- **OLSEN ; SIEGHART.** International Union of Pharmacology. LXX. Subtypes of γ-Aminobutyric AcidA Receptors: Classification of the Basis of Subunit Composition, Pharmacology, and Function. Update. *Pharmacological Reviews,* 2008, vol. 60, 243-260
- **GOTTI ; ZOLI ; CLEMENTI.** Brain nicotinic acetylcholine receptors: native subtypes and their relevance. *Trends in Pharmacological Sciences,* 2006, vol. 27, 482-491
- **N. MILLAR.** *Neuropharmacology,* January 2009, vol. 56 (1), 237-246
- **N.M. BARNES et al.** *Neuropharmacology,* 2009, vol. 56, 273-284
- **JW LYNCH.** *Neuropharmacology,* 2009, vol. 56, 303-309
- **PERRAIS.** *Neuropharmacology,* 2009, vol. 56, 131-140
- **JANE.** *Neuropharmacology,* 2009, vol. 56, 90-113
- **W. LU.** *Neuron,* 2009, vol. 62 (2), 254-268
- **M.F. JARVIS ; B.S. KHAKH.** *Neuropharmacology,* 2009, vol. 56, 208-215
- **S ROBERTSON.** *Current Opinion in Neurobiology,* 2001, vol. 11, 378-386
- **PRINSTER ; HAGUE ; HALL.** Heterodimerization of G Protein-Coupled Receptors : Specificity and Functional Significance. *Pharmacological Reviews,* 2005, vol. 57, 289-298
- **WA CATTERALL et al.** *Pharmacol Rev,* 2005, vol. 57, 411-425
- **J ARIKKATH ; K CAMPBELL.** *Current Opinion in Neurobiology,* 2003, vol. 13, 298-307
- **WA CATTERALL et al.** *Pharmacol. Rev.,* 2005, vol. 57, 397-409
- **Y KUBO et al.** *Pharmacol. Rev.,* 2005, vol. 57, 509-526
- **G GUTMAN et al.** *Pharmacol. Rev.,* 2005, vol. 57, 473-508
- **A WEI et al.** *Pharmacol. Rev.,* 2005, vol. 57, 463-472
- **DE CLAPHAM et al.** *Pharmacol. Rev.,* 2005, vol. 57 (4), 427-450
- **F HOFMANN et al.** *Pharmacol. Rev.,* 2005, vol. 57 (4), 455-462
- **A STARUSCHENKO et al.** *Biophys J,* 2005, vol. 88, 3966-3975
- **H YAMAMURA et al.** *European J Pharm.,* 2008, vol. 600, 32-36
- **S KELLENBERGER ; L SCHILD.** *Physiol Rev,* 2002, vol. 82, 735-767
- Unit 2.2. of Short Protocols in Molecular Biology. John Wiley & Sons, Inc, 1999
- Short Protocols in Molecular Biology. John Wiley & Sons, Inc, 1999
- **FARUQI et al.** *Molecular and Cellular Biology,* 2000, vol. 20, 990-1000
- **SCHLEIFMAN et al.** *Methods Molecular Biology,* 2008, vol. 435, 175-90
- **PIERCE.** An Introduction To Information Theory: Symbols, Signals, and Noise. Dover, 1980
- **MARK H. BEERS.** The Merck Manual
- Diagnostic and Statistical Manual of Mental Disorders DSM-IV-TR
- **THOMSEN W et al.** *Curr. Opin. Biotechnol.,* 2005, vol. 16, 655-665

- **ZHONG H ; NEUBIG RR.** *J Pharmacol Exp Ther.,* 2001, vol. 297, 837-45
- **LATTIN J et al.** *J. Leukoc. Biol.,* 2007, vol. 82, 16-32
- **KIMBLE RJ et al.** *Combin. Chem. HTS,* 2003, vol. 6, 1-9
- **CHRISTOPOULOS A ; KENAKIN T.** *Pharmacol Rev.,* 2002, vol. 54, 323-74
- **GERMAIN P et al.** *Pharmacol Rev,* 2006, vol. 58, 685-704
- **GONZÁLEZ JE et al.** *Drug Discovery Tech.,* 1999, vol. 4, 431-439
- **GONZÁLEZ JE.** *Drug Discovery Tech.,* 1999, vol. 4, 431-439
- **LEVI R ; SMITH NC.** *J Pharmacol Exp Ther.,* 2000, vol. 292, 825-830
- **KLIP, A et al.** *FASEB J,* 1994, vol. 8, 43-53
- **LASKOWSKI KR ; RUSSELL RR.** *Curr Heart Fail Rep.,* 2008, vol. 5, 75-9
- **ESTACIO RO.** *Postgrad Med.,* 2009, vol. 121, 33-44
- **WILLSON TM ; MOORE JT.** *Mol Endocrinol.,* 2002, vol. 16, 1135-1144
- **ROSEN J ; MINER JN.** *Endocr. Rev,* 2005, vol. 26, 452-464
- **VON EULER US.** *Pharmacol Rev.,* 1972, vol. 24 (2), 365-369
- **ROMA MG.** *World J Gastroenterol.,* 2008, vol. 14, 6786-6801
- **TANOUE A.** *J Pharmacol Sci.,* 2009, vol. 109, 50-52
- **PODESSER BK ; HALLSTRÖM S.** *Br J Pharmacol.,* 2007, vol. 151 (7), 930-940
- **PROZIALECK WC ; EDWARDS JR.** *Pharmacol Ther.,* 2007, vol. 114, 74-93
- **BRINKMANN M et al.** *Cytotechnology,* 2002, vol. 38, 119-127
- **KELLOFF GJ et al.** *Cancer Epidemiol Biomarkers Prev.,* 2000, vol. 9, 127-137
- **GOODWIN AM.** *Microvasc Res.,* 2007, vol. 74, 172-183
- **SCHMAUSS D ; WEIS M.** *Circulation,* 2008, vol. 117, 2131-2141
- **FABREGAT I.** *World J Gastroenterol.,* 2009, vol. 15, 513-520
- **MOTYL T et al.** *J Physiol Pharmacol.,* 2006, vol. 57 (7), 17-32
- **JACOBSON-KRAM D et al.** *Environ Health Perspect.,* 1993, vol. 101 (3), 121-125
- **KENYON J ; GERSON SL.** *Nucleic Acids Res.,* 2007, vol. 35, 7557-7565
- **PANAGIOTIS A. TSONIS.** Stem Cells from Differentiated Cells. *Molecular Interventions,* 2004, vol. 4, 81-83
- **SMITH.** EMBRYO-DERIVED STEM CELLS: Of Mice and Men. *Annu. Rev. Cell Dev. Biol.,* 2001, vol. 17, 435-62
- **KANG et al.** iPS Cells Can Support Full-Term Development of Tetraploid Blastocyst-Complemented Embryos. *Cell Stem Cell,* 22 July 2009
- **ZHAO.** iPS cells produce viable mice through tetraploid complementation. *Nature,* 23 July 2009
- **ABRAHAM et al.** *Proc Natl Acad Sci U SA.,* 1993, vol. 90 (1), 312-316
- **REICHLING ; MEYERHOF ; BEHRENS.** *J. Neurochem.,* 2008, vol. 106, 1138-1148
- **UEDA et al.** Identification of coding single-nucleotide polymorphisms in human taste receptor genes involving bitter tasting. *Biochem Biophys Res Commun,* 2001, vol. 285, 147-151
- **WOODING et al.** Natural selection and molecular evolution in PTC, a bitter-taste receptor gene. *Am. J. Hum, Genet.,* 2004, vol. 74, 637-646
- **KIM et al.** Worldwide haplotype diversity and coding sequence variation at human bitter taste receptor loci. *Human Mutation,* 2005, vol. 26, 199-204
- **KIM et al.** Positional cloning of the human quantitative trait locus underlying taste sensitivity to phenylthiocarbamide. *Science,* 2003, vol. 299, 1221-1225
- **CHANDRASHEKAR et al.** T2Rs function as bitter taste receptors. *Cell,* 2000, vol. 100, 703-711
- **BUFE et al.** The human TAS2R16 receptor mediates bitter taste in rsponse to β-glucopyranosides. *Na Genet,* vol. 32, 397-401
- **NAHORSKI.** Pharmacology of intracellular signaling pathways. *Brit. J. Pharm.,* 2000, vol. 147, S38-S45
- History of Quantitative Structure-Activity Relationships. **SELASSIE, C.D.** Burger's Medicinal Chemistry and Drug Discovery. vol. 1
- **TOKARSKI ; HOPFINGER.** *J. Chem. Inf. Computer Sci.,* 1997, vol. 37, 792-811
- **DUCA ; HOPFINGER.** *J Chem Inf Comput Sci,* 2001, vol. 41 (5), 1367-87
- **FORTE et al.** *Endocr.,* 1999, vol. 140 (4), 1800-1806
- **ZHANG et al.** *J Biomol Screen,* 1999, vol. 4 (2), 67-73
- **GALIETTA et al.** *Am J Physiol Cell Physiol.,* 2001, vol. 281 (5), C1734-1742
- **SHEETS et al.** *J Physiol.,* 2007, vol. 581, 1019-1031
- **PRONIN et al.** Identification of Ligands for Two Human Bitter T2R Receptors. *Chem. Senses,* 2004, vol. 29, 583-593

**SEQUENCE LISTING**

<110> Shekdar, Kambiz Sawchuk, Dennis J. Shah, Purvi Manoj

<120> NOVEL CELL LINES AND METHODS

<130> 0022980025WO1

<140>
<141>

<150> 61/149,321 <151> 2009-02-02

<150> 61/149,318 <151> 2009-02-02

<150> 61/149,324 <151> 2009-02-02

<150> 61/149,311 <151> 2009-02-02

<150> 61/230,536 <151> 2009-07-31

<150> 61/235,181 <151> 2009-08-19

<160> 139

<170> FastSEQ for Windows Version 4.0

<210> 1

<211> 1371
<212> DNA
<213> Homo sapiens

<220>
<223> Human GABA-A receptor alpha 1 subunit cDNA

<400> 1

```
atgaggaaaa gtccaggtct gtctgactgt ctttgggcct ggatcctcct tctgagcaca 60
ctgactggaa gaagctatgg acagccgtca ttacaagatg aacttaaaga caataccact 120
gtcttcacca ggattttgga cagactccta gatggttatg acaatcgcct gagaccagga 180
ttgggagagc gtgtaaccga agtgaagact gatatcttcg tcaccagttt cggacccgtt 240
tcagaccatg atatggaata tacaatagat gtatttttcc gtcaaagctg gaaggatgaa 300
aggttaaaat ttaaaggacc tatgacagtc ctccggttaa ataacctaat ggcaagtaaa 360
atctggactc cggacacatt tttccacaat ggaaagaagt cagtggccca caacatgacc 420
atgcccaaca aactcctgcg gatcacagag gatggcacct tgctgtacac catgaggctg 480
acagtgagag ctgaatgtcc gatgcatttg gaggacttcc ctatggatgc ccatgcttgc 540
ccactaaaat ttggaagtta tgcttataca agagcagaag ttgtttatga atggaccaga 600
gagccagcac gctcagtggt tgtagcagaa gatggatcac gtctaaacca gtatgacctt 660
cttggacaaa cagtagactc tggaattgtc cagtcaagta caggagaata tgttgttatg 720
accactcatt tccacttgaa gagaaagatt ggctactttg ttattcaaac atacctgcca 780
tgcataatga cagtgattct ctcacaagtc tccttctggc tcaacagaga gtctgtacca 840
gcaagaactg tctttggagt aacaactgtg ctcaccatga caacattgag catcagtgcc 900
agaaactccc tccctaaggt ggcttatgca acagctatgg attggtttat tgccgtgtgc 960
tatgcctttg tgttctcagc tctgattgag tttgccacag taaactattt cactaagaga 1020
ggttatgcat gggatggcaa aagtgtggtt ccagaaaagc caaagaaagt aaaggatcct 1080
cttattaaga aaaacaacac ttacgctcca acagcaacca gctacacccc taatttggcc 1140

aggggcgacc cgggcttagc caccattgct aaaagtgcaa ccatagaacc taaagaggtc 1200
aagcccgaaa caaaaccacc agaacccaag aaaaccttta acagtgtcag caaaattgac 1260
cgactgtcaa gaatagcctt cccgctgcta tttggaatct ttaacttagt ctactgggct 1320
acgtatttaa acagagagcc tcagctaaaa gcccccacac cacatcaata g          1371
```

<210> 2
<211> 1356
<212> DNA
<213> Homo sapiens

<220>
<223> Human GABA-A receptor alpha 2 subunit cDNA

<400> 2

```
atgaagacaa aattgaacat ctacaacatg cagttcctgc tttttgtttt cttggtgtgg 60
gaccctgcca ggttggtgct ggctaacatc caagaagatg aggctaaaaa taacattacc 120
atctttacga gaattcttga cagacttctg gatggttacg ataatcggct tagaccagga 180
ctgggagaca gtattactga agtcttcact aacatctacg tgaccagttt tggccctgtc 240
tcagatacag atatggaata tacaattgat gttttctttc gacaaaaatg gaaagatgaa 300
cgtttaaaat ttaaaggtcc tatgaatatc cttcgactaa acaatttaat ggctagcaaa 360
atctggactc cagatacctt ttttcacaat gggaaaaaat cagtagctca taatatgaca 420
atgccaaata agttgcttcg aattcaggat gatgggactc tgctgtatac catgaggctt 480
acagttcaag ctgaatgccc aatgcacttg gaggatttcc caatggatgc tcattcatgt 540
cctctgaaat ttggcagcta tgcatataca acttcagagg tcacttatat ttggacttac 600
aatgcatctg attcagtaca ggttgctcct gatggctcta ggttaaatca atatgacctg 660
ctgggccaat caatcggaaa ggagacaatt aaatccagta caggtgaata tactgtaatg 720
acagctcatt tccacctgaa aagaaaaatt gggtattttg tgattcaaac ctatctgcct 780
tgcatcatga ctgtcattct ctcccaagtt tcattctggc ttaacagaga atctgtgcct 840
gcaagaactg tgtttggagt aacaactgtc ctaacaatga caactctaag catcagtgct 900
cggaattctc tccccaaagt ggcttatgca actgccatgg actggtttat tgctgtttgt 960
tatgcatttg tgttctctgc cctaattgaa tttgcaactg ttaattactt caccaaaaga 1020
ggatggactt gggatgggaa gagtgtagta aatgacaaga aaaaagaaaa ggcttccgtt 1080
atgatacaga acaacgctta tgcagtggct gttgccaatt atgccccgaa tctttcaaaa 1140
gatccagttc tctccaccat ctccaagagt gcaaccacgc cagaacccaa caagaagcca 1200
gaaaacaagc cagctgaagc aaagaaaact ttcaacagtg ttagcaaaat tgacagaatg 1260
tccagaatag tttttccagt tttgtttggt acctttaatt tagtttactg ggctacatat 1320
ttaaacagag aacctgtatt aggggtcagt ccttga 1356
```

<210> 3
<211> 1479
<212> DNA
<213> Homo sapiens

<220>
<223> Human GABA-A receptor alpha 3 subunit cDNA

<400> 3

```
atgataatca cacaaacaag tcactgttac atgaccagcc ttgggattct tttcctgatt 60
aatattctcc ctggaaccac tggtcaaggg gaatcaagac gacaagaacc cggggacttt 120
gtgaagcagg acattggcgg gctgtctcct aagcatgccc cagatattcc tgatgacagc 180
actgacaaca tcactatctt caccagaatc ttggatcgtc ttctggacgg ctatgacaac 240
cggctgcgac ctgggcttgg agatgcagtg actgaagtga agactgacat ctacgtgacc 300
agttttggcc ctgtgtcaga cactgacatg gagtacacta ttgatgtatt ttttcggcag 360
acatggcatg atgaaagact gaaatttgat ggccccatga agatccttcc actgaacaat 420
ctcctggcta gtaagatctg gacaccggac accttcttcc acaatggcaa gaaatcagtg 480
gctcataaca tgaccacgcc caacaagctg ctcagattgg tggacaacgg aaccctcctc 540
tatacaatga ggttaacaat tcatgctgag tgtcccatgc atttggaaga ttttcccatg 600
gatgtgcatg cctgcccact gaagtttgga agctatgcct atacaacagc tgaagtggtt 660
tattcttgga ctctcggaaa gaacaaatcc gtggaagtgg cacaggatgg ttctcgcttg 720
aaccagtatg accttttggg ccatgttgtt gggacagaga taatccggtc tagtacagga 780
gaatatgtcg tcatgacaac ccacttccat ctcaagcgaa aaattggcta ctttgtgatc 840
cagacctact tgccatgtat catgactgtc attctgtcac aagtgtcgtt ctggctcaac 900
agagagtctg ttcctgcccg tacagtcttt ggtgtcacca ctgtgcttac catgaccacc 960
ttgagtatca gtgccagaaa ttccttacct aaagtggcat atgcgacggc catggactgg 1020
ttcatagccg tctgttatgc ctttgtattt tctgcactga ttgaatttgc cactgtcaac 1080
```

```
tatttcacca agcggagttg ggcttgggaa ggcaagaagg tgccagaggc cctggagatg 1140
aagaagaaaa caccagcagc cccagcaaag aaaaccagca ctaccttcaa catcgtgggg 1200
accacctatc ccatcaacct ggccaaggac actgaatttt ccaccatctc caagggcgct 1260
gctcccagtg cctcctcaac cccaacaatc attgcttcac ccaaggccac ctacgtgcag 1320
gacagcccga ctgagaccaa gacctacaac agtgtcagca aggttgacaa aatttcccgc 1380
atcatctttc ctgtgctctt tgccatattc aatctggtct attgggccac atatgtcaac 1440
cgggagtcag ctatcaaggg catgatccgc aaacagtag                        1479
```

<210> 4
<211> 1389
<212> DNA
<213> Homo sapiens

<220>
<223> Human GABA-A receptor alpha 5 subunit cDNA

<400> 4

```
atggacaatg gaatgttctc tggttttatc atgatcaaaa acctccttct cttttgtatt 60
tccatgaact tatccagtca ctttggcttt tcacagatgc caaccagttc agtgaaagat 120
gagaccaatg acaacatcac gatatttacc aggatcttgg atgggctctt ggatggctac 180
gacaacagac ttcggcccgg gctgggagag cgcatcactc aggtgaggac cgacatctac 240
gtcaccagct tcggcccggt gtccgacacg gaaatggagt acaccataga cgtgtttttc 300
cgacaaagct ggaaagatga aaggcttcgg tttaagggcc ccatgcagcg cctccctctc 360
aacaacctcc ttgccagcaa gatctggacc ccagacacgt tcttccacaa cgggaagaag 420
tccatcgctc acaacatgac cacgcccaac aagctgctgc ggctggagga cgacggcacc 480
ctgctctaca ccatgcgctt gaccatctct gcagagtgcc ccatgcagct tgaggacttc 540
ccgatggatg cgcacgcttg ccctctgaaa tttggcagct atgcgtaccc taattctgaa 600
gtcgtctacg tctggaccaa cggctccacc aagtcggtgg tggtggcgga agatggctcc 660
agactgaacc agtaccacct gatggggcag acggtgggca ctgagaacat cagcaccagc 720
acaggcgaat acacaatcat gacagctcac ttccacctga aaaggaagat tggctacttt 780
gtcatccaga cctaccttcc ctgcataatg accgtgatct tatcacaggt gtcctttggg 840
ctgaaccggg aatcagtccc agccaggaca gtttttgggg tcaccacggt gctgaccatg 900
acgaccctca gcatcagcgc caggaactct ctgcccaaag tggcctacgc caccgccatg 960
gactggttca tagccgtgtg ctatgccttc gtcttctcgg cgctgataga gtttgccacg 1020
gtcaattact ttaccaagag aggctgggcc tgggatggca aaaaagcctt ggaagcagcc 1080
aagatcaaga aaaagcgtga agtcatacta aataagtcaa caaacgcttt tacaactggg 1140
aagatgtctc acccccccaa cattccgaag gaacagaccc cagcagggac gtcgaataca 1200
acctcagtct cagtaaaacc ctctgaagag aagacttctg aaagcaaaaa gacttacaac 1260
agtatcagca aaattgacaa aatgtcccga atcgtattcc cagtcttgtt cggcactttc 1320
aacttagttt actgggcaac gtatttgaat agggagccgg tgataaaagg agccgcctct 1380
ccaaaataa                                                         1389
```

<210> 5
<211> 1422
<212> DNA
<213> Homo sapiens

<220>
<223> Human GABA-A receptor beta 3 variant 1 subunit cDNA

<400> 5

atgtggggcc ttgcgggagg aaggcttttc ggcatcttct cggccccggt gctggtggct 60
gtggtgtgct gcgcccagag tgtgaacgat cccgggaaca tgtcctttgt gaaggagacg 120
gtggacaagc tgttgaaagg ctacgacatt cgcctaagac ccgacttcgg gggtcccccg 180
gtctgcgtgg ggatgaacat cgacatcgcc agcatcgaca tggtttccga agtcaacatg 240
gattatacct taaccatgta ttttcaacaa tattggagag ataaaaggct cgcctattct 300
gggatccctc tcaacctcac gcttgacaat cgagtggctg accagctatg ggtgcccgac 360
acatatttct taaatgacaa aaagtcattt gtgcatggag tgacagtgaa aaaccgcatg 420
atccgtcttc accctgatgg gacagtgctg tatgggctca gaatcaccac gacagcagca 480
tgcatgatgg acctcaggag ataccccctg gacgagcaga actgcactct ggaaattgaa 540
agctatggct acaccacgga tgacattgag ttttactggc gaggcgggga caaggctgtt 600
accggagtgg aaaggattga gctcccgcag ttctccatcg tggagcaccg tctggtctcg 660
aggaatgttg tcttcgccac aggtgcctat cctcgactgt cactgagctt tcggttgaag 720
aggaacattg gatacttcat tcttcagact tatatgccct ctatactgat aacgattctg 780
tcgtgggtgt ccttctggat caattatgat gcatctgctg ctagagttgc cctcgggatc 840

acaactgtgc tgacaatgac aaccatcaac acccaccttc gggagacctt gcccaaaatc 900
ccctatgtca aagccattga catgtacctt atgggctgct tcgtctttgt gttcctggcc 960
cttctggagt atgcctttgt caactacatt ttctttggaa gaggccctca aaggcagaag 1020
aagcttgcag aaaagacagc caaggcaaag aatgaccgtt caaagagcga aagcaaccgg 1080
gtggatgctc atggaaatat tctgttgaca tcgctggaag ttcacaatga aatgaatgag 1140
gtctcaggcg gcattggcga taccaggaat tcagcaatat cctttgacaa ctcaggaatc 1200
cagtacagga aacagagcat gcctcgagaa gggcatgggc gattcctggg ggacagaagc 1260
ctcccgcaca agaagaccca tctacggagg aggtcttcac agctcaaaat taaaatacct 1320
gatctaaccg atgtgaatgc catagacaga tggtccagga tcgtgtttcc attcactttt 1380
tctcttttca acttagttta ctggctgtac tatgttaact ga 1422

<210> 6
<211> 1404
<212> DNA
<213> Homo sapiens

<220>
<223> Human GABA-A receptor gamma 2 transcript variant 1 (short) subunit cDNA

<400> 6

```
atgagttcgc caaatatatg gagcacagga agctcagtct actcgactcc tgtattttca 60
cagaaaatga cggtgtggat tctgctcctg ctgtcgctct accctggctt cactagccag 120
aaatctgatg atgactatga agattatgct tctaacaaaa catgggtctt gactccaaaa 180
gttcctgagg gtgatgtcac tgtcatctta aacaacctgc tggaaggata tgacaataaa 240
cttcggcctg atataggagt gaagccaacg ttaattcaca cagacatgta tgtgaatagc 300
attggtccag tgaacgctat caatatggaa tacactattg atatattttt tgcgcaaacg 360
tggtatgaca gacgtttgaa atttaacagc accattaaag tcctccgatt gaacagcaac 420
atggtgggga aaatctggat tccagacact ttcttcagaa attccaaaaa agctgatgca 480
cactggatca ccacccccaa caggatgctg agaatttgga atgatggtcg agtgctctac 540
accctaaggt tgacaattga tgctgagtgc caattacaat tgcacaactt tccaatggat 600
gaacactcct gccccttgga gttctcaagt tatggctatc cacgtgaaga aattgtttat 660
caatggaagc gaagttctgt tgaagtgggc gacacaagat cctggaggct ttatcaattc 720
tcatttgttg gtctaagaaa taccaccgaa gtagtgaaga caacttccgg agattatgtg 780
gtcatgtctg tctactttga tctgagcaga agaatgggat actttaccat ccagacctat 840
atcccctgca cactcattgt cgtcctatcc tgggtgtctt tctggatcaa taaggatgct 900
gttccagcca gaacatcttt aggtatcacc actgtcctga caatgaccac cctcagcacc 960
attgcccgga aatcgctccc caaggtctcc tatgtcacag cgatggatct ctttgtatct 1020
gtttgtttca tctttgtctt ctctgctctg gtggagtatg gcaccttgca ttattttgtc 1080
agcaaccgga aaccaagcaa ggacaaagat aaaaagaaga aaaaccctgc ccctaccatt 1140
gatatccgcc caagatcagc aaccattcaa atgaataatg ctacacacct tcaagagaga 1200
gatgaagagt acggctatga gtgtctggac ggcaaggact gtgccagttt tttctgctgt 1260
tttgaagatt gtcgaacagg agcttggaga catgggagga tacatatccg cattgccaaa 1320
atggactcct atgctcggat cttcttcccc actgccttct gcctgtttaa tctggtctat 1380
tgggtctcct acctctacct gtga                                        1404
```

<210> 7
<211> 25
<212> DNA
<213> Artificial Sequence

<220>
<223> GABA Target Sequence 1

<400> 7
gttcttaagg cacaggaact gggac 25

<210> 8
<211> 25
<212> DNA
<213> Artificial Sequence

<220>
<223> GABA Target Sequence 2

<400> 8
gaagttaacc ctgtcgttct gcgac 25

<210> 9
<211> 25
<212> DNA
<213> Artificial Sequence

<220>
<223> GABA Target Sequence 3

<400> 9
gttctatagg gtctgcttgt cgctc 25

<210> 10

<211> 34
<212> DNA
<213> Artificial Sequence

<220>
<223> GABA Signal Probe 1

<400> 10
gccagtccca gttcctgtgc cttaagaacc tcgc 34

<210> 11
<211> 34
<212> DNA
<213> Artificial Sequence

<220>
<223> GABA Signal Probe 2

<400> 11
gcgagtcgca gaacgacagg gttaacttcc tcgc 34

<210> 12
<211> 34
<212> DNA
<213> Artificial Sequence

<220>
<223> GABA Signal Probe 3

<400> 12
gcgagagcga caagcagacc ctatagaacc tcgc 34

<210> 13
<211> 25
<212> DNA
<213> Artificial Sequence

<220>
<223> GC-C Target Sequence 1

<400> 13
gttcttaagg cacaggaact gggac 25

<210> 14
<211> 34
<212> DNA
<213> Artificial Sequence

<220>
<223> GC-C Signaling Probe 1

<400> 14
gccagtccca gttcctgtgc cttaagaacc tcgc 34

<210> 15
<211> 3222
<212> DNA
<213> homo sapiens

<220>
<223> GUCY2C (guanylate cyclase 2C)

<400> 15

```
atgaagacgt tgctgttgga cttggctttg tggtcactgc tcttccagcc cgggtggctg 60
tcctttagtt cccaggtgag tcagaactgc cacaatggca gctatgaaat cagcgtcctg 120
atgatgggca actcagcctt tgcagagccc ctgaaaaact tggaagatgc ggtgaatgag 180
gggctggaaa tagtgagagg acgtctgcaa aatgctggcc taaatgtgac tgtgaacgct 240
actttcatgt attcggatgg tctgattcat aactcaggcg actgccggag tagcacctgt 300
gaaggcctcg acctactcag gaaaatttca aatgcacaac ggatgggctg tgtcctcata 360
gggccctcat gtacatactc caccttccag atgtaccttg acacagaatt gagctacccc 420
atgatctcag ctggaagttt tggattgtca tgtgactata aagaaacctt aaccaggctg 480
atgtctccag ctagaaagtt gatgtacttc ttggttaact tttggaaaac caacgatctg 540
cccttcaaaa cttattcctg gagcacttcg tatgtttaca agaatggtac agaaactgag 600
gactgtttct ggtaccttaa tgctctggag gctagcgttt cctatttctc ccacgaactc 660
ggctttaagg tggtgttaag acaagataag gagtttcagg atatcttaat ggaccacaac 720
aggaaaagca atgtgattat tatgtgtggt ggtccagagt tcctctacaa gctgaagggt 780
gaccgagcag tggctgaaga cattgtcatt attctagtgg atcttttcaa tgaccagtac 840
ttggaggaca atgtcacagc ccctgactat atgaaaaatg tccttgttct gacgctgtct 900
cctgggaatt cccttctaaa tagctctttc tccaggaatc tatcaccaac aaaacgagac 960
tttgctcttg cctatttgaa tggaatcctg ctctttggac atatgctgaa gatatttctt 1020
gaaaatggag aaaatattac cacccccaaa tttgctcatg ctttcaggaa tctcactttt 1080
gaagggtatg acggtccagt gaccttggat gactgggggg atgttgacag taccatggtg 1140
cttctgtata cctctgtgga caccaagaaa tacaaggttc ttttgaccta tgatacccac 1200
gtaaataaga cctatcctgt ggatatgagc cccacattca cttggaagaa ctctaaactt 1260
cctaatgata ttacaggccg gggccctcag atcctgatga ttgcagtctt caccctcact 1320
ggagctgtgg tgctgctcct gctcgtcgct ctcctgatgc tcagaaaata tagaaaagat 1380
tatgaacttc gtcagaaaaa atggtcccac attcctcctg aaaatatctt tcctctggag 1440
accaatgaga ccaatcatgt tagcctcaag atcgatgatg acaaaagacg agatacaatc 1500
cagagactac gacagtgcaa atacgacaaa aagcgagtga ttctcaaaga tctcaagcac 1560
aatgatggta atttcactga aaaacagaag atagaattga acaagttgct tcagattgac 1620
tattacaacc tgaccaagtt ctacggcaca gtgaaacttg ataccatgat cttcggggtg 1680
atagaatact gtgagagagg atccctccgg gaagttttaa atgacacaat ttcctaccct 1740
gatggcacat tcatggattg ggagtttaag atctctgtct tgtatgacat tgctaaggga 1800
atgtcatatc tgcactccag taagacagaa gtccatggtc gtctgaaatc taccaactgc 1860
gtagtggaca gtagaatggt ggtgaagatc actgattttg gctgcaattc cattttacct 1920
ccaaaaaagg acctgtggac agctccagag cacctccgcc aagccaacat ctctcagaaa 1980
ggagatgtgt acagctatgg gatcatcgca caggagatca ttctgcggaa agaaaccttc 2040
tacactttga gctgtcggga ccggaatgag aagattttca gagtggaaaa ttccaatgga 2100
atgaaaccct tccgcccaga tttattcttg gaaacagcag aggaaaaaga gctagaagtg 2160
tacctacttg taaaaaactg ttgggaggaa gatccagaaa agagaccaga tttcaaaaaa 2220
attgagacta cacttgccaa gatatttgga ctttttcatg accaaaaaaa tgaaagctat 2280
atggatacct tgatccgacg tctacagcta tattctcgaa acctggaaca tctggtagag 2340
gaaaggacac agctgtacaa ggcagagagg gacagggctg acagacttaa ctttatgttg 2400
cttccaaggc tagtggtaaa gtctctgaag gagaaaggct ttgtggagcc ggaactatat 2460
gaggaagtta caatctactt cagtgacatt gtaggtttca ctactatctg caaatacagc 2520
acccccatgg aagtggtgga catgcttaat gacatctata agagttttga ccacattgtt 2580
gatcatcatg atgtctacaa ggtggaaacc atcggtgatg cgtacatggt ggctagtggt 2640
ttgcctaaga gaaatggcaa tcggcatgca atagacattg ccaagatggc cttggaaatc 2700
ctcagcttca tggggacctt tgagctggag catcttcctg gcctcccaat atggattcgc 2760
attggagttc actctggtcc ctgtgctgct ggagttgtgg gaatcaagat gcctcgttat 2820
tgtctatttg gagatacggt caacacagcc tctaggatgg aatccactgg cctccctttg 2880
agaattcacg tgagtggctc caccatagcc atcctgaaga gaactgagtg ccagttcctt 2940
tatgaagtga gaggagaaac atacttaaag ggaagaggaa atgagactac ctactggctg 3000
actgggatga aggaccagaa attcaacctg ccaacccctc ctactgtgga gaatcaacag 3060
cgtttgcaag cagaattttc agacatgatt gccaactctt tacagaaaag acaggcagca 3120

gggataagaa gccaaaaacc cagacgggta gccagctata aaaaaggcac tctggaatac 3180
ttgcagctga ataccacaga caaggagagc acctattttt aa                    3222
```

<210> 16
<211> 4443
<212> DNA
<213> homo sapiens

<220>
<223> human cystic fibrosis transmembrane conductance regulator (CFTR)

<400> 16

atgcagaggt cgcctctgga aaaggccagc gttgtctcca aacttttttt cagctggacc 60
agaccaattt tgaggaaagg atacagacag cgcctggaat tgtcagacat ataccaaatc 120
ccttctgttg attctgctga caatctatct gaaaaattgg aaagagaatg ggatagagag 180
ctggcttcaa agaaaaatcc taaactcatt aatgcccttc ggcgatgttt tttctggaga 240
tttatgttct atggaatctt tttatattta ggggaagtca ccaaagcagt acagcctctc 300
ttactgggaa gaatcatagc ttcctatgac ccggataaca aggaggaacg ctctatcgcg 360
atttatctag gcataggctt atgccttctc tttattgtga ggacactgct cctacaccca 420
gccatttttg gccttcatca cattggaatg cagatgagaa tagctatgtt tagtttgatt 480
tataagaaga ctttaaagct gtcaagccgt gttctagata aaataagtat tggacaactt 540
gttagtctcc tttccaacaa cctgaacaaa tttgatgaag gacttgcatt ggcacatttc 600
gtgtggatcg ctcctttgca agtggcactc ctcatggggc taatctggga gttgttacag 660
gcgtctgcct tctgtggact tggtttcctg atagtccttg ccctttttca ggctgggcta 720
gggagaatga tgatgaagta cagagatcag agagctggga agatcagtga aagacttgtg 780
attacctcag aaatgattga aaatatccaa tctgttaagg catactgctg ggaagaagca 840
atggaaaaaa tgattgaaaa cttaagacaa acagaactga aactgactcg gaaggcagcc 900
tatgtgagat acttcaatag ctcagccttc ttcttctcag ggttctttgt ggtgttttta 960
tctgtgcttc cctatgcact aatcaaagga atcatcctcc ggaaaatatt caccaccatc 1020
tcattctgca ttgttctgcg catggcggtc actcggcaat ttccctgggc tgtacaaaca 1080
tggtatgact ctcttggagc aataaacaaa atacaggatt tcttacaaaa gcaagaatat 1140
aagacattgg aatataactt aacgactaca gaagtagtga tggagaatgt aacagccttc 1200
tgggaggagg gatttgggga attatttgag aaagcaaaac aaaacaataa caatagaaaa 1260
acttctaatg gtgatgacag cctcttcttc agtaatttct cacttcttgg tactcctgtc 1320
ctgaaagata ttaatttcaa gatagaaaga ggacagttgt tggcggttgc tggatccact 1380
ggagcaggca agacttcact tctaatggtg attatgggag aactggagcc ttcagagggt 1440
aaaattaagc acagtggaag aatttcattc tgttctcagt tttcctggat tatgcctggc 1500
accattaaag aaaatatcat ctttggtgtt tcctatgatg aatatagata cagaagcgtc 1560
atcaaagcat gccaactaga agaggacatc tccaagtttg cagagaaaga caatatagtt 1620
cttggagaag gtggaatcac actgagtgga ggtcaacgag caagaatttc tttagcaaga 1680
gcagtataca aagatgctga tttgtattta ttagactctc cttttggata cctagatgtt 1740
ttaacagaaa aagaaatatt tgaaagctgt gtctgtaaac tgatggctaa caaaactagg 1800
attttggtca cttctaaaat ggaacattta aagaaagctg acaaaatatt aattttgcat 1860
gaaggtagca gctattttta tgggacattt tcagaactcc aaaatctaca gccagacttt 1920
agctcaaaac tcatgggatg tgattctttc gaccaattta gtgcagaaag aagaaattca 1980
atcctaactg agaccttaca ccgtttctca ttagaaggag atgctcctgt ctcctggaca 2040
gaaacaaaaa aacaatcttt taaacagact ggagagtttg gggaaaaaag gaagaattct 2100
attctcaatc caatcaactc tatacgaaaa ttttccattg tgcaaaagac tccccttacaa 2160
atgaatggca tcgaagagga ttctgatgag cctttagaga gaaggctgtc cttagtacca 2220
gattctgagc agggagaggc gatactgcct cgcatcagcg tgatcagcac tggccccacg 2280
cttcaggcac gaaggaggca gtctgtcctg aacctgatga cacactcagt taaccaaggt 2340
cagaacattc accgaaagac aacagcatcc acacgaaaag tgtcactggc ccctcaggca 2400
aacttgactg aactggatat atattcaaga aggttatctc aagaaactgg cttggaaata 2460
agtgaagaaa ttaacgaaga agacttaaag gagtgctttt ttgatgatat ggagagcata 2520
ccagcagtga ctacatggaa cacatacctt cgatatatta ctgtccacaa gagcttaatt 2580
tttgtgctaa tttggtgctt agtaattttt ctggcagagg tggctgcttc tttggttgtg 2640
ctgtggctcc ttggaaacac tcctcttcaa gacaaaggga atagtactca tagtagaaat 2700
aacagctatg cagtgattat caccagcacc agttcgtatt atgtgtttta catttacgtg 2760
ggagtagccg acactttgct tgctatggga ttcttcagag gtctaccact ggtgcatact 2820
ctaatcacag tgtcgaaaat tttacaccac aaaatgttac attctgttct tcaagcacct 2880
atgtcaaccc tcaacacgtt gaaagcaggt gggattctta atagattctc caaagatata 2940
gcaattttgg atgaccttct gcctcttacc atatttgact tcatccagtt gttattaatt 3000
gtgattggag ctatagcagt tgtcgcagtt ttacaaccct acatctttgt tgcaacagtg 3060
ccagtgatag tggcttttat tatgttgaga gcatatttcc tccaaacctc acagcaactc 3120
aaacaactgg aatctgaagg caggagtcca attttcactc atcttgttac aagcttaaaa 3180

```
ggactatgga cacttcgtgc cttcggacgg cagccttact ttgaaactct gttccacaaa 3240
gctctgaatt tacatactgc caactggttc ttgtacctgt caacactgcg ctggttccaa 3300
atgagaatag aaatgatttt tgtcatcttc ttcattgctg ttaccttcat ttccatttta 3360
acaacaggag aaggagaagg aagagttggt attatcctga ctttagccat gaatatcatg 3420
agtacattgc agtgggctgt aaactccagc atagatgtgg atagcttgat gcgatctgtg 3480
agccgagtct ttaagttcat tgacatgcca acagaaggta aacctaccaa gtcaaccaaa 3540
ccatacaaga atggccaact ctcgaaagtt atgattattg agaattcaca cgtgaagaaa 3600
gatgacatct ggccctcagg gggccaaatg actgtcaaag atctcacagc aaaatacaca 3660
gaaggtggaa atgccatatt agagaacatt tccttctcaa taagtcctgg ccagagggtg 3720
ggcctcttgg gaagaactgg atcagggaag agtactttgt tatcagcttt tttgagacta 3780
ctgaacactg aaggagaaat ccagatcgat ggtgtgtctt gggattcaat aactttgcaa 3840
cagtggagga aagcctttgg agtgatacca cagaaagtat ttattttttc tggaacattt 3900
agaaaaaact tggatcccta tgaacagtgg agtgatcaag aaatatggaa agttgcagat 3960
gaggttgggc tcagatctgt gatagaacag tttcctggga agcttgactt tgtccttgtg 4020
gatgggggct gtgtcctaag ccatggccac aagcagttga tgtgcttggc tagatctgtt 4080
ctcagtaagg cgaagatctt gctgcttgat gaacccagtg ctcatttgga tccagtaaca 4140
taccaaataa ttagaagaac tctaaaacaa gcatttgctg attgcacagt aattctctgt 4200
gaacacagga tagaagcaat gctggaatgc caacaatttt tggtcataga agagaacaaa 4260
gtgcggcagt acgattccat ccagaaactg ctgaacgaga ggagcctctt ccggcaagcc 4320
atcagcccct ccgacagggt gaagctcttt ccccaccgga actcaagcaa gtgcaagtct 4380
aagccccaga ttgctgctct gaaagaggag acagaagaag aggtgcaaga tacaaggctt 4440
tga                                                               4443
```

<210> 17
<211> 25
<212> DNA
<213> Artificial Sequence

<220>
<223> CFTR Target Sequence 1

<400> 17
gttcttaagg cacaggaact gggac 25

<210> 18
<211> 34
<212> DNA
<213> Artificial Sequence

<220>
<223> CFTR Signaling probe 1

<400> 18
gccagtccca gttcctgtgc cttaagaacc tcgc 34

<210> 19
<211> 5934
<212> DNA
<213> Homo sapiens

<220>
<223> human SCN9A

<400> 19

```
atggcaatgt tgcctccccc aggacctcag agctttgtcc atttcacaaa acagtctctt 60
gccctcattg aacaacgcat tgctgaaaga aaatcaaagg aacccaaaga agaaaagaaa 120
gatgatgatg aagaagcccc aaagccaagc agtgacttgg aagctggcaa acaactgccc 180
ttcatctatg gggacattcc tcccggcatg gtgtcagagc ccctggagga cttggacccc 240
tactatgcag acaaaaagac tttcatagta ttgaacaaag ggaaaacaat cttccgtttc 300
aatgccacac ctgctttata tatgctttct cctttcagtc ctctaagaag aatatctatt 360
aagattttag tacactcctt attcagcatg ctcatcatgt gcactattct gacaaactgc 420
atatttatga ccatgaataa cccgccggac tggaccaaaa atgtcgagta cacttttact 480
ggaatatata cttttgaatc acttgtaaaa atccttgcaa gaggcttctg tgtaggagaa 540
ttcacttttc ttcgtgaccc gtggaactgg ctggattttg tcgtcattgt ttttgcgtat 600
ttaacagaat ttgtaaacct aggcaatgtt tcagctcttc gaactttcag agtattgaga 660
```

gctttgaaaa ctatttctgt aatcccaggc ctgaagacaa ttgtaggggc tttgatccag 720
tcagtgaaga agctttctga tgtcatgatc ctgactgtgt tctgtctgag tgtgtttgca 780
ctaattggac tacagctgtt catgggaaac ctgaagcata aatgttttcg aaattcactt 840
gaaaataatg aaacattaga aagcataatg aataccctag agagtgaaga agactttaga 900
aaatattttt attacttgga aggatccaaa gatgctctcc tttgtggttt cagcacagat 960
tcaggtcagt gtccagaggg gtacacctgt gtgaaaattg gcagaaaccc tgattatggc 1020
tacacgagct ttgacacttt cagctgggcc ttcttagcct tgtttaggct aatgacccaa 1080
gattactggg aaaaccttta ccaacagacg ctgcgtgctg ctggcaaaac ctacatgatc 1140
ttctttgtcg tagtgatttt cctgggctcc ttttatctaa taaacttgat cctggctgtg 1200
gttgccatgg catatgaaga acagaaccag gcaaacattg aagaagctaa acagaaagaa 1260
ttagaatttc aacagatgtt agaccgtctt aaaaaagagc aagaagaagc tgaggcaatt 1320
gcagcggcag cggctgaata tacaagtatt aggagaagca gaattatggg cctctcagag 1380
agttcttctg aaacatccaa actgagctct aaaagtgcta aagaaagaag aaacagaaga 1440
aagaaaaaga atcaaaagaa gctctccagt ggagaggaaa agggagatgc tgagaaattg 1500
tcgaaatcag aatcagagga cagcatcaga agaaaaagtt tccaccttgg tgtcgaaggg 1560
cataggcgag cacatgaaaa gaggttgtct acccccaatc agtcaccact cagcattcgt 1620
ggctccttgt ttctgcaagg gcgaagcagc agaacaagtc tttttagttt caaaggcaga 1680
ggaagagata taggatctga gactgaattt gccgatgatg agcacagcat tttggagac 1740
aatgagagca gaaggggctc actgtttgtg ccccacagac cccaggagcg acgcagcagt 1800
aacatcagcc aagccagtag gtccccacca atgctgccgg tgaacgggaa aatgcacagt 1860
gctgtggact gcaacggtgt ggtctccctg gttgatggac gctcagccct catgctcccc 1920
aatggacagc ttctgccaga gggcacgacc aatcaaatac acaagaaaag gcgttgtagt 1980
tcctatctcc tttcagagga tatgctgaat gatcccaacc tcagacagag agcaatgagt 2040
agagcaagca tattaacaaa cactgtggaa gaacttgaag agtccagaca aaaatgtcca 2100
ccttggtggt acagatttgc acacaaattc ttgatctgga attgctctcc atattggata 2160
aaattcaaaa agtgtatcta ttttattgta atggatcctt ttgtagatct tgcaattacc 2220
atttgcatag ttttaaacac attatttatg gctatggaac accacccaat gactgaggaa 2280
ttcaaaaatg tacttgctat aggaaatttg gtctttactg gaatctttgc agctgaaatg 2340
gtattaaaac tgattgccat ggatccatat gagtatttcc aagtaggctg gaatattttt 2400
gacagcctta ttgtgacttt aagtttagtg gagctctttc tagcagatgt ggaaggattg 2460
tcagttctgc gatcattcag actgctccga gtcttcaagt tggcaaaatc ctggccaaca 2520
ttgaacatgc tgattaagat cattggtaac tcagtagggg ctctaggtaa cctcacctta 2580
gtgttggcca tcatcgtctt cattttttgct gtggtcggca tgcagctctt tggtaagagc 2640
tacaaagaat gtgtctgcaa gatcaatgat gactgtacgc tcccacggtg gcacatgaac 2700
gacttcttcc actccttcct gattgtgttc cgcgtgctgt gtggagagtg gatagagacc 2760
atgtgggact gtatggaggt cgctggtcaa gctatgtgcc ttattgttta catgatggtc 2820
atggtcattg gaaacctggt ggtcctaaac ctatttctgg ccttattatt gagctcattt 2880
agttcagaca atcttacagc aattgaagaa gaccctgatg caaacaacct ccagattgca 2940
gtgactagaa ttaaaaaggg aataaattat gtgaaacaaa ccttacgtga atttattcta 3000
aaagcatttt ccaaaaagcc aaagatttcc agggagataa gacaagcaga agatctgaat 3060
actaagaagg aaaactatat ttctaaccat acacttgctg aaatgagcaa aggtcacaat 3120
ttcctcaagg aaaaagataa aatcagtggt tttggaagca gcgtggacaa acacttgatg 3180
gaagacagtg atggtcaatc atttattcac aatcccagcc tcacagtgac agtgccaatt 3240
gcacctgggg aatccgattt ggaaaatatg aatgctgagg aacttagcag tgattcggat 3300
agtgaataca gcaaagtgag attaaaccgg tcaagctcct cagagtgcag cacagttgat 3360
aaccctttgc ctggagaagg agaagaagca gaggctgaac ctatgaattc cgatgagcca 3420
gaggcctgtt tcacagatgg ttgtgtacgg aggttctcat gctgccaagt taacatagag 3480
tcagggaaag gaaaaatctg gtggaacatc aggaaaacct gctacaagat tgttgaacac 3540
agttggtttg aaagcttcat tgtcctcatg atcctgctca gcagtggtgc cctggctttt 3600
gaagatattt atattgaaag gaaaaagacc attaagatta tcctggagta tgcagacaag 3660
atcttcactt acatcttcat tctggaaatg cttctaaaat ggatagcata tggttataaa 3720
acatatttca ccaatgcctg gtgttggctg gatttcctaa ttgttgatgt ttctttggtt 3780
actttagtgg caaacactct tggctactca gatcttggcc ccattaaatc ccttcggaca 3840
ctgagagctt taagacctct aagagcctta tctagatttg aaggaatgag ggtcgttgtg 3900
aatgcactca taggagcaat tccttccatc atgaatgtgc tacttgtgtg tcttatattc 3960
tggctgatat tcagcatcat gggagtaaat ttgtttgctg gcaagttcta tgagtgtatt 4020
aacaccacag atgggtcacg gtttcctgca agtcaagttc caaatcgttc cgaatgtttt 4080
gcccttatga atgttagtca aaatgtgcga tggaaaaacc tgaaagtgaa ctttgataat 4140
gtcggacttg gttacctatc tctgcttcaa gttgcaactt ttaagggatg gacgattatt 4200
atgtatgcag cagtggattc tgttaatgta gacaagcagc ccaaatatga atatagcctc 4260
tacatgtata tttattttgt cgtctttatc atctttgggt cattcttcac tttgaacttg 4320
ttcattggtg tcatcataga taatttcaac caacagaaaa agaagcttgg aggtcaagac 4380
atctttatga cagaagaaca gaagaaatac tataatgcaa tgaaaaagct ggggtccaag 4440
aagccacaaa agccaattcc tcgaccaggg aacaaaatcc aaggatgtat atttgaccta 4500
gtgacaaatc aagcctttga tattagtatc atggttctta tctgtctcaa catggtaacc 4560

```
atgatggtag aaaaggaggg tcaaagtcaa catatgactg aagttttata ttggataaat 4620
gtggttttta taatcctttt cactggagaa tgtgtgctaa aactgatctc cctcagacac 4680
tactacttca ctgtaggatg gaatattttt gattttgtgg ttgtgattat ctccattgta 4740
ggtatgtttc tagctgattt gattgaaacg tattttgtgt cccctaccct gttccgagtg 4800
atccgtcttg ccaggattgg ccgaatccta cgtctagtca aaggagcaaa ggggatccgc 4860
acgctgctct ttgctttgat gatgtccctt cctgcgttgt ttaacatcgg cctcctgctc 4920
ttcctggtca tgttcatcta cgccatcttt ggaatgtcca actttgccta tgttaaaaag 4980
gaagatggaa ttaatgacat gttcaatttt gagacctttg gcaacagtat gatttgcctg 5040
ttccaaatta caacctctgc tggctgggat ggattgctag cacctattct taacagtaag 5100
ccacccgact gtgacccaaa aaaagttcat cctggaagtt cagttgaagg agactgtggt 5160
aacccatctg ttggaatatt ctactttgtt agttatatca tcatatcctt cctggttgtg 5220
gtgaacatgt acattgcagt catactggag aattttagtg ttgccactga agaaagtact 5280
gaacctctga gtgaggatga ctttgagatg ttctatgagg tttgggagaa gtttgatccc 5340
gatgcgaccc agtttataga gttctctaaa ctctctgatt ttgcagctgc cctggatcct 5400
cctcttctca tagcaaaacc caacaaagtc cagctcattg ccatggatct gcccatggtt 5460
agtggtgacc ggatccattg tcttgacatc ttatttgctt ttacaaagcg tgttttgggt 5520
gagagtgggg agatggattc tcttcgttca cagatggaag aaaggttcat gtctgcaaat 5580
ccttccaaag tgtcctatga acccatcaca accacactaa aacggaaaca agaggatgtg 5640
tctgctactg tcattcagcg tgcttataga cgttaccgct taaggcaaaa tgtcaaaaat 5700
atatcaagta tatacataaa agatggagac agagatgatg atttactcaa taaaaaagat 5760
atggcttttg ataatgttaa tgagaactca agtccagaaa aaacagatgc cacttcatcc 5820
accacctctc caccttcata tgatagtgta acaaagccag acaaagagaa atatgaacaa 5880
gacagaacag aaaaggaaga caaagggaaa gacagcaagg aaagcaaaaa atag       5934
```

<210> 20
<211> 657
<212> DNA
<213> Homo sapiens

<220>
<223> human SCN1B

<400> 20

```
atgggggaggc tgctggcctt agtggtcggc gcggcactgg tgtcctcagc ctgcgggggc 60
tgcgtggagg tggactcgga gaccgaggcc gtgtatggga tgaccttcaa aattctttgc 120
atctcctgca agcgccgcag cgagaccaac gctgagacct tcaccgagtg gaccttccgc 180
cagaagggca ctgaggagtt tgtcaagatc ctgcgctatg agaatgaggt gttgcagctg 240
gaggaggatg agcgcttcga gggccgcgtg gtgtggaatg gcagccgggg caccaaagac 300
ctgcaggatc tgtctatctt catcaccaat gtcacctaca accactcggg cgactacgag 360
tgccacgtct accgcctgct cttcttcgaa aactacgagc acaacaccag cgtcgtcaag 420
aagatccaca ttgaggtagt ggacaaagcc aacagagaca tggcatccat cgtgtctgag 480
atcatgatgt atgtgctcat tgtggtgttg accatatggc tcgtggcaga gatgatttac 540
tgctacaaga agatcgctgc cgccacggag actgctgcac aggagaatgc ctcggaatac 600
ctggccatca cctctgaaag caaagagaac tgcacgggcg tccaggtggc cgaatag    657
```

<210> 21
<211> 648
<212> DNA
<213> Homo sapiens

<220>
<223> human SCN2B

<400> 21

atgcacagag atgcctggct acctcgccct gccttcagcc tcacggggct cagtctcttt 60
ttctctttgg tgccaccagg acggagcatg gaggtcacag tacctgccac cctcaacgtc 120
ctcaatggct ctgacgcccg cctgccctgc accttcaact cctgctacac agtgaaccac 180
aaacagttct ccctgaactg gacttaccag gagtgcaaca actgctctga ggagatgttc 240
ctccagttcc gcatgaagat cattaacctg aagctggagc ggtttcaaga ccgcgtggag 300
ttctcaggga accccagcaa gtacgatgtg tcggtgatgc tgagaaacgt gcagccggag 360
gatgagggga tttacaactg ctacatcatg aacccccctg accgccaccg tggccatggc 420
aagatccatc tgcaggtcct catggaagag ccccctgagc gggactccac ggtggccgtg 480
attgtgggtg cctccgtcgg gggcttcctg gctgtggtca tcttggtgct gatggtggtc 540
aagtgtgtga ggagaaaaaa agagcagaag ctgagcacag atgacctgaa gaccgaggag 600
gagggcaaga cggacggtga aggcaacccg gatgatggcg ccaagtag 648

<210> 22
<211> 25
<212> DNA
<213> Artificial Sequence

<220>
<223> NaV Target sequence 1

<400> 22
gttcttaagg cacaggaact gggac 25

<210> 23
<211> 25
<212> DNA
<213> Artificial Sequence

<220>
<223> NaV Target sequence 2

<400> 23
gaagttaacc ctgtcgttct gcgac 25

<210> 24
<211> 25
<212> DNA
<213> Artificial Sequence

<220>
<223> NaV Target sequence 3

<400> 24
gttctatagg gtctgcttgt cgctc 25

<210> 25
<211> 34
<212> DNA
<213> Artificial Sequence

<220>
<223> NaV Signaling probe 1 (binds target 1)

<400> 25
gccagtccca gttcctgtgc cttaagaacc tcgc 34

<210> 26
<211> 34

<212> DNA
<213> Artificial Sequence

<220>
<223> NaV Signaling probe 2 (binds target 2)

<400> 26
gcgagtcgca gaacgacagg gttaacttcc tcgc 34

<210> 27
<211> 34
<212> DNA
<213> Artificial Sequence

<220>
<223> NaV Signaling probe 3 (binds target 3)

<400> 27
gcgagagcga caagcagacc ctatagaacc tcgc 34

<210> 28
<211> 25
<212> DNA
<213> Artificial Sequence

<220>
<223> Target Sequence 1 used in generating a stable umami taste receptor-expressing cell line

<400> 28
gttcttaagg cacaggaact gggac 25

<210> 29
<211> 25
<212> DNA
<213> Artificial Sequence

<220>
<223> Target Sequence 2 used in generating a stable umami taste receptor-expressing cell line

<400> 29
gaagttaacc ctgtcgttct gcgac 25

<210> 30
<211> 25
<212> DNA
<213> Artificial Sequence

<220>
<223> Target Sequence 3 used in generating a stable umami taste receptor-expressing cell line

<400> 30
gttctatagg gtctgcttgt cgctc 25

<210> 31
<211> 2520
<212> DNA
<213> Homo sapiens

<220>
<223> Human T1R2 DNA sequence

<400> 31

```
atggggccca gggcaaagac catctgctcc ctgttcttcc tcctatgggt cctggctgag 60
ccggctgaga actcggactt ctacctgcct ggggattacc tcctgggtgg cctcttctcc 120
ctccatgcca acatgaaggg cattgttcac cttaacttcc tgcaggtgcc catgtgcaag 180
gagtatgaag tgaaggtgat aggctacaac ctcatgcagg ccatgcgctt tgcggtggag 240
gagatcaaca atgacagcag cctgctgcct ggtgtgctgc tgggctatga gatcgtggat 300
gtgtgctaca tctccaacaa tgtccagccg gtgctctact tcctggcaca cgaggacaac 360
ctccttccca tccaagagga ctacagtaac tacatttccc gtgtggtggc tgtcattggc 420
cctgacaact ccgagtctgt catgactgtg gccaacttcc tctccctatt tctccttcca 480
cagatcacct acagcgccat cagcgatgag ctgcgagaca aggtgcgctt cccggctttg 540
ctgcgtacca cacccagcgc cgaccaccac gtcgaggcca tggtgcagct gatgctgcac 600
ttccgctgga actggatcat tgtgctggtg agcagcgaca cctatggccg cgacaatggc 660
cagctgcttg gcgagcgcgt ggcccggcgc gacatctgca tcgccttcca ggagacgctg 720
cccacactgc agcccaacca gaacatgacg tcagaggagc gccagcgcct ggtgaccatt 780
gtggacaagc tgcagcagag cacagcgcgc gtcgtggtcg tgttctcgcc cgacctgacc 840
ctgtaccact tcttcaatga ggtgctgcgc cagaacttca cgggcgccgt gtggatcgcc 900
tccgagtcct gggccatcga cccggtcctg cacaacctca cggagctggg ccacttgggc 960
accttcctgg gcatcaccat ccagagcgtg cccatcccgg gcttcagtga gttccgcgag 1020
tggggcccac aggctgggcc gccacccctc agcaggacca gccagagcta tacctgcaac 1080

caggagtgcg acaactgcct gaacgccacc ttgtccttca acaccattct caggctctct 1140
ggggagcgtg tcgtctacag cgtgtactct gcggtctatg ctgtggccca tgccctgcac 1200
agcctcctcg gctgtgacaa aagcacctgc accaagaggg tggtctaccc ctggcagctg 1260
cttgaggaga tctggaaggt caacttcact ctcctggacc accaaatctt cttcgacccg 1320
caaggggacg tggctctgca cttggagatt gtccagtggc aatgggaccg gagccagaat 1380
cccttccaga gcgtcgcctc ctactacccc ctgcagcgac agctgaagaa catccaagac 1440
atctcctggc acaccgtcaa caacacgatc cctatgtcca tgtgttccaa gaggtgccag 1500
tcagggcaaa agaagaagcc tgtgggcatc cacgtctgct gcttcgagtg catcgactgc 1560
cttcccggca ccttcctcaa ccacactgaa gatgaatatg aatgccaggc ctgcccgaat 1620
aacgagtggt cctaccagag tgagacctcc tgcttcaagc ggcagctggt cttcctggaa 1680
tggcatgagg cacccaccat cgctgtggcc ctgctggccg ccctgggctt cctcagcacc 1740
ctggccatcc tggtgatatt ctggaggcac ttccagacac ccatagttcg ctcggctggg 1800
ggccccatgt gcttcctgat gctgacactg ctgctggtgg catacatggt ggtcccggtg 1860
tacgtggggc cgcccaaggt ctccacctgc ctctgccgcc aggccctctt tcccctctgc 1920
ttcacaatct gcatctcctg tatcgccgtg cgttctttcc agatcgtctg cgccttcaag 1980
atggccagcc gcttcccacg cgcctacagc tactgggtcc gctaccaggg gccctacgtc 2040
tctatggcat ttatcacggt actcaaaatg gtcattgtgg taattggcat gctggccacg 2100
ggcctcagtc ccaccacccg tactgacccc gatgacccca agatcacaat tgtctcctgt 2160
aaccccaact accgcaacag cctgctgttc aacaccagcc tggacctgct gctctcagtg 2220
gtgggtttca gcttcgccta catgggcaaa gagctgccca ccaactacaa cgaggccaag 2280
ttcatcaccc tcagcatgac cttctatttc acctcatccg tctccctctg caccttcatg 2340
tctgcctaca gcggggtgct ggtcaccatt gtggacctct tggtcactgt gctcaacctc 2400
ctggccatca gcctgggcta cttcggcccc aagtgctaca tgatcctctt ctacccggag 2460
cgcaacacgc ccgcctactt caacagcatg atccagggct acaccatgag gagggactag 2520
```

<210> 32
<211> 2559
<212> DNA
<213> Homo sapiens

<220>
<223> Human T1R3 DNA sequence

<400> 32

atgctgggcc ctgctgtcct gggcctcagc ctctgggctc tcctgcaccc tgggacgggg 60
gccccattgt gcctgtcaca gcaacttagg atgaaggggg actacgtgct gggggggctg 120
ttccccctgg gcgaggccga ggaggctggc ctccgcagcc ggacacggcc cagcagccct 180
gtgtgcacca ggttctcctc aaacggcctg ctctgggcat tggccatgaa aatggccgtg 240
gaggagatca acaacaagtc ggatctgctg cccgggctgc gcctgggcta cgacctcttt 300
gatacgtgct cggagcctgt ggtggccatg aagcccagcc tcatgttcct ggccaaggca 360
ggcagccgcg acatcgccgc ctactgcaac tacacgcagt accagccccg tgtgctggct 420
gtcatcgggc ccactcgtc agagctcgcc atggtcaccg gcaagttctt cagcttcttc 480
ctcatgcccc aggtcagcta cggtgctagc atggagctgc tgagcgcccg ggagaccttc 540
ccctccttct tccgcaccgt gcccagcgac cgtgtgcagc tgacggccgc cgcggagctg 600
ctgcaggagt tcggctggaa ctgggtggcc gccctgggca gcgacgacga gtacggccgg 660
cagggcctga gcatcttctc ggccctggcc gcggcacgcg gcatctgcat cgcgcacgag 720
ggcctggtgc cgctgccccg tgccgatgac tcgcggctgg ggaaggtgca ggacgtcctg 780
caccaggtga accagagcag cgtgcaggtg gtgctgctgt tcgcctccgt gcacgccgcc 840
cacgccctct tcaactacag catcagcagc aggctctcgc ccaaggtgtg ggtggccagc 900
gaggcctggc tgacctctga cctggtcatg ggcctgcccg gcatggccca gatgggcacg 960
gtgcttggct tcctccagag gggtgcccag ctgcacgagt tcccccagta cgtgaagacg 1020
cacctggccc tggccaccga cccggccttc tgctctgccc tgggcgagag ggagcagggt 1080
ctggaggagg acgtggtggg ccagcgctgc ccgcagtgtg actgcatcac gctgcagaac 1140
gtgagcgcag ggctaaatca ccaccagacg ttctctgtct acgcagctgt gtatagcgtg 1200
gcccaggccc tgcacaacac tcttcagtgc aacgcctcag gctgccccgc gcaggacccc 1260
gtgaagccct ggcagctcct ggagaacatg tacaacctga ccttccacgt gggcgggctg 1320
ccgctgcggt tcgacagcag cggaaacgtg gacatggagt acgacctgaa gctgtgggtg 1380
tggcagggct cagtgcccag gctccacgac gtgggcaggt tcaacggcag cctcaggaca 1440
gagcgcctga agatccgctg gcacacgtct gacaaccaga agcccgtgtc ccggtgctcg 1500
cggcagtgcc aggagggcca ggtgcgccgg gtcaaggggt tccactcctg ctgctacgac 1560
tgtgtggact gcgaggcggg cagctaccgg caaaacccag acgacatcgc ctgcaccttt 1620
tgtggccagg atgagtggtc cccggagcga agcacacgct gcttccgccg caggtctcgg 1680
ttcctggcat ggggcgagcc ggctgtgctg ctgctgctcc tgctgctgag cctggcgctg 1740
ggccttgtgc tggctgcttt ggggctgttc gttcaccatc gggacagccc actggttcag 1800

gcctcggggg ggcccctggc ctgctttggc ctggtgtgcc tgggcctggt ctgcctcagc 1860
gtcctcctgt tccctggcca gcccagccct gcccgatgcc tggcccagca gcccttgtcc 1920
cacctcccgc tcacgggctg cctgagcaca ctcttcctgc aggcggccga gatcttcgtg 1980
gagtcagaac tgcctctgag ctgggcagac cggctgagtg gctgcctgcg ggggccctgg 2040
gcctggctgg tggtgctgct ggccatgctg gtggaggtcg cactgtgcac ctggtacctg 2100
gtggccttcc cgccggaggt ggtgacggac tggcacatgc tgcccacgga ggcgctggtg 2160
cactgccgca cacgctcctg ggtcagcttc ggcctagcgc acgccaccaa tgccacgctg 2220
gcctttctct gcttcctggg cactttcctg gtgcggagcc agccgggccg ctacaaccgt 2280
gcccgtggcc tcacctttgc catgctggcc tacttcatca cctgggtctc ctttgtgccc 2340
ctcctggcca atgtgcaggt ggtcctcagg cccgccgtgc agatgggcgc cctcctgctc 2400
tgtgtcctgg gcatcctggc tgccttccac ctgcccaggt gttacctgct catgcggcag 2460
ccagggctca acacccccga gttcttcctg ggagggggcc ctggggatgc ccaaggccag 2520
aatgacggga acacaggaaa tcaggggaaa catgagtga 2559

<210> 33
<211> 858
<212> DNA
<213> Mus musculus

<220>
<223> Mouse G-alpha 15 sequence

<220>
<221> misc_feature
<222> 589, 595, 616, 623, 636, 654
<223> n = A,T,C or G

<400> 33

```
atggcccggt ccctgacttg gggctgctgt ccctggtgcc tgacagagga ggagaagact 60
gccgccagaa tcgaccagga gatcaacagg attttgttgg aacagaaaaa acaagagcgc 120
gaggaattga aactcctgct gttggggcct ggtgagagcg ggaagagtac gttcatcaag 180
cagatgcgca tcattcacgg tgtgggctac tcggaggagg accgcagagc cttccggctg 240
ctcatctacc agaacatctt cgtctccatg caggccatga tagatgcgat ggaccggctg 300
cagatcccct tcagcaggcc tgacagcaag cagcacgcca gcctagtgat gacccaggac 360
ccctataaag tgagcacatt cgagaagcca tatgcagtgg ccatgcagta cctgtggcgg 420
gacgcgggca tccgtgcatg ctacgagcga aggcgtgaat tccaccttct ggactccgcg 480
gtgtattacc tgtcacacct ggagcgcata tcagaggaca gctacatccc cactgcgcaa 540
gacgtgctgc gcagtcgcat gcccaccaca ggcatcaatg agtactgcnc acacntccca 600
cctggccaca tacttncccc agnttccagg gacccnggcg agacgcagag gccncccaag 660
agcttcatct tggacatgta tgcgcgcgtg tacgcgagct gcgcagagcc ccaggacggt 720
ggcaggaaag gctcccgcgc gcgccgcttc ttcgcacact tcacctgtgc cacggacacg 780
caaagcgtcc gcagcgtgtt caaggacgtg cgggactcgg tgctggcccg gtacctggac 840
gagatcaacc tgctgtga                                                858
```

<210> 34
<211> 839
<212> PRT
<213> Homo sapiens

<220>
<223> Human T1R2

<400> 34

```
Met Gly Pro Arg Ala Lys Thr Ile Ser Ser Leu Phe Phe Leu Leu Trp
1               5                   10                  15
Val Leu Ala Glu Pro Ala Glu Asn Ser Asp Phe Tyr Leu Pro Gly Asp
            20                  25                  30
Tyr Leu Leu Gly Gly Leu Phe Ser Leu His Ala Asn Met Lys Gly Ile
        35                  40                  45
Val His Leu Asn Phe Leu Gln Val Pro Met Cys Lys Glu Tyr Glu Val
    50                  55                  60
Lys Val Ile Gly Tyr Asn Leu Met Gln Ala Met Arg Phe Ala Val Glu
65                  70                  75                  80
Glu Ile Asn Asn Asp Ser Ser Leu Leu Pro Gly Val Leu Leu Gly Tyr
                85                  90                  95
```

```
Glu Ile Val Asp Val Cys Tyr Ile Ser Asn Asn Val Gln Pro Val Leu
            100                 105                 110
Tyr Phe Leu Ala His Glu Asp Asn Leu Leu Pro Ile Gln Glu Asp Tyr
        115                 120                 125
Ser Asn Tyr Ile Ser Arg Val Val Ala Val Ile Gly Pro Asp Asn Ser
    130                 135                 140
Glu Ser Val Met Thr Val Ala Asn Phe Leu Ser Leu Phe Leu Leu Pro
145                 150                 155                 160
Gln Ile Thr Tyr Ser Ala Ile Ser Asp Glu Leu Arg Asp Lys Val Arg
                165                 170                 175
Phe Pro Ala Leu Leu Arg Thr Thr Pro Ser Ala Asp His His Ile Glu
            180                 185                 190
Ala Met Val Gln Leu Met Leu His Phe Arg Trp Asn Trp Ile Ile Val
        195                 200                 205
Leu Val Ser Ser Asp Thr Tyr Gly Arg Asp Asn Gly Gln Leu Leu Gly
    210                 215                 220
Glu Arg Val Ala Arg Arg Asp Ile Cys Ile Ala Phe Gln Glu Thr Leu
225                 230                 235                 240
Pro Thr Leu Gln Pro Asn Gln Asn Met Thr Ser Glu Glu Arg Gln Arg
                245                 250                 255
Leu Val Thr Ile Val Asp Lys Leu Gln Gln Ser Thr Ala Arg Val Val
            260                 265                 270
Val Val Phe Ser Pro Asp Leu Thr Leu Tyr His Phe Phe Asn Glu Val
        275                 280                 285
Leu Arg Gln Asn Phe Thr Gly Ala Val Trp Ile Ala Ser Glu Ser Trp
    290                 295                 300
Ala Ile Asp Pro Val Leu His Asn Leu Thr Glu Leu Arg His Leu Gly
305                 310                 315                 320
Thr Phe Leu Gly Ile Thr Ile Gln Ser Val Pro Ile Pro Gly Phe Ser
                325                 330                 335
Glu Phe Arg Glu Trp Gly Pro Gln Ala Gly Pro Pro Pro Leu Ser Arg
            340                 345                 350
Thr Ser Gln Ser Tyr Thr Cys Asn Gln Glu Cys Asp Asn Cys Leu Asn
        355                 360                 365
Ala Thr Leu Ser Phe Asn Thr Ile Leu Arg Leu Ser Gly Glu Arg Val
    370                 375                 380
Val Tyr Ser Val Tyr Ser Ala Val Tyr Ala Val Ala His Ala Leu His
385                 390                 395                 400
Ser Leu Leu Gly Cys Asp Lys Ser Thr Cys Thr Lys Arg Val Val Tyr
                405                 410                 415
Pro Trp Gln Leu Leu Glu Glu Ile Trp Lys Val Asn Phe Thr Leu Leu
            420                 425                 430
Asp His Gln Ile Phe Phe Asp Pro Gln Gly Asp Val Ala Leu His Leu
        435                 440                 445
Glu Ile Val Gln Trp Gln Trp Asp Arg Ser Gln Asn Pro Phe Gln Ser
    450                 455                 460
Val Ala Ser Tyr Tyr Pro Leu Gln Arg Gln Leu Lys Asn Ile Gln Asp
465                 470                 475                 480
Ile Ser Trp His Thr Ile Asn Asn Thr Ile Pro Met Ser Met Cys Ser
                485                 490                 495
Lys Arg Cys Gln Ser Gly Gln Lys Lys Lys Pro Val Gly Ile His Val
            500                 505                 510
Cys Cys Phe Glu Cys Ile Asp Cys Leu Pro Gly Thr Phe Leu Asn His
        515                 520                 525
Thr Glu Asp Glu Tyr Glu Cys Gln Ala Cys Pro Asn Asn Glu Trp Ser
    530                 535                 540
Tyr Gln Ser Glu Thr Ser Cys Phe Lys Arg Gln Leu Val Phe Leu Glu
545                 550                 555                 560
Trp His Glu Ala Pro Thr Ile Ala Val Ala Leu Leu Ala Ala Leu Gly
                565                 570                 575
Phe Leu Ser Thr Leu Ala Ile Leu Val Ile Phe Trp Arg His Phe Gln
            580                 585                 590
Thr Pro Ile Val Arg Ser Ala Gly Gly Pro Met Cys Phe Leu Met Leu
        595                 600                 605
Thr Leu Leu Leu Val Ala Tyr Met Val Val Pro Val Tyr Val Gly Pro
```

```
        610                 615                 620
    Pro Lys Val Ser Thr Cys Leu Cys Arg Gln Ala Leu Phe Pro Leu Cys
    625                 630                 635                 640
    Phe Thr Ile Cys Ile Ser Cys Ile Ala Val Arg Ser Phe Gln Ile Val
                    645                 650                 655
    Cys Ala Phe Lys Met Ala Ser Arg Phe Pro Arg Ala Tyr Ser Tyr Trp
                660                 665                 670
    Val Arg Tyr Gln Gly Pro Tyr Val Ser Met Ala Phe Ile Thr Val Leu
            675                 680                 685
    Lys Met Val Ile Val Val Ile Gly Met Leu Ala Thr Gly Leu Ser Pro
        690                 695                 700
    Thr Thr Arg Thr Asp Pro Asp Asp Pro Lys Ile Thr Ile Val Ser Cys
    705                 710                 715                 720
    Asn Pro Asn Tyr Arg Asn Ser Leu Leu Phe Asn Thr Ser Leu Asp Leu
                    725                 730                 735
    Leu Leu Ser Val Val Gly Phe Ser Phe Ala Tyr Met Gly Lys Glu Leu
                740                 745                 750
    Pro Thr Asn Tyr Asn Glu Ala Lys Phe Ile Thr Leu Ser Met Thr Phe
            755                 760                 765
    Tyr Phe Thr Ser Ser Val Ser Leu Cys Thr Phe Met Ser Ala Tyr Ser
        770                 775                 780
    Gly Val Leu Val Thr Ile Val Asp Leu Leu Val Thr Val Leu Asn Leu
    785                 790                 795                 800
    Leu Ala Ile Ser Leu Gly Tyr Phe Gly Pro Lys Cys Tyr Met Ile Leu
                    805                 810                 815
    Phe Tyr Pro Glu Arg Asn Thr Pro Ala Tyr Phe Asn Ser Met Ile Gln
                820                 825                 830
    Gly Tyr Thr Met Arg Arg Asp
            835
```

<210> 35
<211> 852
<212> PRT
<213> Homo sapiens

<220>
<223> Human T1R3 protein sequence

<400> 35

```
Met Leu Gly Pro Ala Val Leu Gly Leu Ser Leu Trp Ala Leu Leu His
 1               5                  10                  15
Pro Gly Thr Gly Ala Pro Leu Cys Leu Ser Gln Gln Leu Arg Met Lys
            20                  25                  30
Gly Asp Tyr Val Leu Gly Gly Leu Phe Pro Leu Gly Glu Ala Glu Glu
        35                  40                  45
Ala Gly Leu Arg Ser Arg Thr Arg Pro Ser Ser Pro Val Cys Thr Arg
    50                  55                  60
Phe Ser Ser Asn Gly Leu Leu Trp Ala Leu Ala Met Lys Met Ala Val
65                  70                  75                  80
Glu Glu Ile Asn Asn Lys Ser Asp Leu Leu Pro Gly Leu Arg Leu Gly
                85                  90                  95
Tyr Asp Leu Phe Asp Thr Cys Ser Glu Pro Val Val Ala Met Lys Pro
            100                 105                 110
Ser Leu Met Phe Leu Ala Lys Ala Gly Ser Arg Asp Ile Ala Ala Tyr
        115                 120                 125
Cys Asn Tyr Thr Gln Tyr Gln Pro Arg Val Leu Ala Val Ile Gly Pro
    130                 135                 140
His Ser Ser Glu Leu Ala Met Val Thr Gly Lys Phe Phe Ser Phe Phe
145                 150                 155                 160
Leu Met Pro Gln Val Ser Tyr Gly Ala Ser Met Glu Leu Leu Ser Ala
                165                 170                 175
Arg Glu Thr Phe Pro Ser Phe Phe Arg Thr Val Pro Ser Asp Arg Val
            180                 185                 190
Gln Leu Thr Ala Ala Ala Glu Leu Leu Gln Glu Phe Gly Trp Asn Trp
```

```
        195                 200                 205
Val Ala Ala Leu Gly Ser Asp Asp Glu Tyr Gly Arg Gln Gly Leu Ser
    210                 215                 220
Ile Phe Ser Ala Leu Ala Ala Ala Arg Gly Ile Cys Ile Ala His Glu
225                 230                 235                 240
Gly Leu Val Pro Leu Pro Arg Ala Asp Asp Ser Arg Leu Gly Lys Val
                245                 250                 255
Gln Asp Val Leu His Gln Val Asn Gln Ser Ser Val Gln Val Val Leu
            260                 265                 270
Leu Phe Ala Ser Val His Ala Ala His Ala Leu Phe Asn Tyr Ser Ile
        275                 280                 285
Ser Ser Arg Leu Ser Pro Lys Val Trp Val Ala Ser Glu Ala Trp Leu
    290                 295                 300
Thr Ser Asp Leu Val Met Gly Leu Pro Gly Met Ala Gln Met Gly Thr
305                 310                 315                 320
Val Leu Gly Phe Leu Gln Arg Gly Ala Gln Leu His Glu Phe Pro Gln
                325                 330                 335
Tyr Val Lys Thr His Leu Ala Leu Ala Thr Asp Pro Ala Phe Cys Ser
            340                 345                 350
Ala Leu Gly Glu Arg Glu Gln Gly Leu Glu Glu Asp Val Val Gly Gln
        355                 360                 365
Arg Cys Pro Gln Cys Asp Cys Ile Thr Leu Gln Asn Val Ser Ala Gly
    370                 375                 380
Leu Asn His His Gln Thr Phe Ser Val Tyr Ala Ala Val Tyr Ser Val
385                 390                 395                 400
Ala Gln Ala Leu His Asn Thr Leu Gln Cys Asn Ala Ser Gly Cys Pro
                405                 410                 415
Ala Gln Asp Pro Val Lys Pro Trp Gln Leu Leu Glu Asn Met Tyr Asn
            420                 425                 430
Leu Thr Phe His Val Gly Gly Leu Pro Leu Arg Phe Asp Ser Ser Gly
        435                 440                 445
Asn Val Asp Met Glu Tyr Asp Leu Lys Leu Trp Val Trp Gln Gly Ser
    450                 455                 460
Val Pro Arg Leu His Asp Val Gly Arg Phe Asn Gly Ser Leu Arg Thr
465                 470                 475                 480
Glu Arg Leu Lys Ile Arg Trp His Thr Ser Asp Asn Gln Lys Pro Val
                485                 490                 495
Ser Arg Cys Ser Arg Gln Cys Gln Glu Gly Gln Val Arg Arg Val Lys
            500                 505                 510
Gly Phe His Ser Cys Cys Tyr Asp Cys Val Asp Cys Glu Ala Gly Ser
        515                 520                 525
Tyr Arg Gln Asn Pro Asp Asp Ile Ala Cys Thr Phe Cys Gly Gln Asp
    530                 535                 540
Glu Trp Ser Pro Glu Arg Ser Thr Arg Cys Phe Arg Arg Arg Ser Arg
545                 550                 555                 560
Phe Leu Ala Trp Gly Glu Pro Ala Val Leu Leu Leu Leu Leu Leu Leu
                565                 570                 575
Ser Leu Ala Leu Gly Leu Val Leu Ala Ala Leu Gly Leu Phe Val His
            580                 585                 590
His Arg Asp Ser Pro Leu Val Gln Ala Ser Gly Gly Pro Leu Ala Cys
        595                 600                 605
Phe Gly Leu Val Cys Leu Gly Leu Val Cys Leu Ser Val Leu Leu Phe
    610                 615                 620
Pro Gly Gln Pro Ser Pro Ala Arg Cys Leu Ala Gln Gln Pro Leu Ser
625                 630                 635                 640
His Leu Pro Leu Thr Gly Cys Leu Ser Thr Leu Phe Leu Gln Ala Ala
                645                 650                 655
Glu Ile Phe Val Glu Ser Glu Leu Pro Leu Ser Trp Ala Asp Arg Leu
            660                 665                 670
Ser Gly Cys Leu Arg Gly Pro Trp Ala Trp Leu Val Val Leu Leu Ala
        675                 680                 685
Met Leu Val Glu Val Ala Leu Cys Thr Trp Tyr Leu Val Ala Phe Pro
    690                 695                 700
Pro Glu Val Val Thr Asp Trp His Met Leu Pro Thr Glu Ala Leu Val
705                 710                 715                 720
```

```
His Cys Arg Thr Arg Ser Trp Val Ser Phe Gly Leu Ala His Ala Thr
                725                 730                 735
Asn Ala Thr Leu Ala Phe Leu Cys Phe Leu Gly Thr Phe Leu Val Arg
            740                 745                 750
Ser Gln Pro Gly Cys Tyr Asn Arg Ala Arg Gly Leu Thr Phe Ala Met
        755                 760                 765
Leu Ala Tyr Phe Ile Thr Trp Val Ser Phe Val Pro Leu Leu Ala Asn
    770                 775                 780
Val Gln Val Val Leu Arg Pro Ala Val Gln Met Gly Ala Leu Leu Leu
785                 790                 795                 800
Cys Val Leu Gly Ile Leu Ala Ala Phe His Leu Pro Arg Cys Tyr Leu
                805                 810                 815
Leu Met Arg Gln Pro Gly Leu Asn Thr Pro Glu Phe Phe Leu Gly Gly
            820                 825                 830
Gly Pro Gly Asp Ala Gln Gly Gln Asn Asp Gly Asn Thr Gly Asn Gln
        835                 840                 845
Gly Lys His Glu
    850
```

<210> 36
<211> 236
<212> PRT
<213> Mus musculus

<220>
<223> Mouse G-alpha 15 protein sequence

<400> 36

```
Met Ala Arg Ser Thr Trp Gly Cys Cys Trp Cys Thr Lys Thr Ala Ala
 1               5                  10                  15
Arg Asp Asn Arg Lys Lys Arg Lys Gly Gly Ser Gly Lys Ser Thr Lys
            20                  25                  30
Met Arg His Gly Val Gly Tyr Ser Asp Arg Arg Ala Arg Tyr Asn Val
        35                  40                  45
Ser Met Ala Met Asp Ala Met Asp Arg Ser Arg Asp Ser Lys His Ala
    50                  55                  60
Ser Val Met Thr Asp Tyr Lys Val Ser Thr Lys Tyr Ala Val Ala Met
65                  70                  75                  80
Tyr Trp Arg Asp Ala Gly Arg Ala Cys Tyr Arg Arg Arg His Asp Ser
                85                  90                  95
Ala Val Tyr Tyr Ser His Arg Ser Asp Ser Tyr Thr Ala Asp Val Arg
            100                 105                 110
Ser Arg Met Thr Thr Gly Asn Tyr Cys Ser Val Lys Lys Thr Lys Arg
        115                 120                 125
Val Asp Val Gly Gly Arg Ser Arg Arg Lys Trp His Cys Asn Val Ala
    130                 135                 140
Tyr Ala Ser Ser Tyr Asp Cys Asn Asp Asn Arg Met Ser Ala Ser Thr
145                 150                 155                 160
Trp Lys Ser Thr Ser Val Asn Lys Thr Asp Asp Lys His Thr Ser His
                165                 170                 175
Ala Thr Tyr Ser Gly Arg Arg Asp Ala Ala Ala Lys Ser Asp Met Tyr
            180                 185                 190
Ala Arg Val Tyr Ala Ser Cys Ala Asp Gly Gly Arg Lys Gly Ser Arg
        195                 200                 205
Ala Arg Arg Ala His Thr Cys Ala Thr Asp Thr Ser Val Arg Ser Val
    210                 215                 220
Lys Asp Val Arg Asp Ser Val Ala Arg Tyr Asp Asn
225                 230                 235
```

<210> 37
<211> 374
<212> PRT
<213> Homo sapiens

<220>
<223> Human G-alpha 15 sequence

<400> 37

```
Met Ala Arg Ser Leu Thr Trp Arg Cys Cys Pro Trp Cys Leu Thr Glu
1               5                   10                  15
Asp Glu Lys Ala Ala Ala Arg Val Asp Gln Glu Ile Asn Arg Ile Leu
            20                  25                  30
Leu Glu Gln Lys Lys Gln Asp Arg Gly Glu Leu Lys Leu Leu Leu Leu
        35                  40                  45
Gly Pro Gly Glu Ser Gly Lys Ser Thr Phe Ile Lys Gln Met Arg Ile
    50                  55                  60
Ile His Gly Ala Gly Tyr Ser Glu Glu Glu Arg Lys Gly Phe Arg Pro
65                  70                  75                  80
Leu Val Tyr Gln Asn Ile Phe Val Ser Met Arg Ala Met Ile Glu Ala
                85                  90                  95
Met Glu Arg Leu Gln Ile Pro Phe Ser Arg Pro Glu Ser Lys His His
            100                 105                 110
Ala Ser Leu Val Met Ser Gln Asp Pro Tyr Lys Val Thr Thr Phe Glu
        115                 120                 125
Lys Arg Tyr Ala Ala Ala Met Gln Trp Leu Trp Arg Asp Ala Gly Ile
    130                 135                 140
Arg Ala Cys Tyr Glu Arg Arg Arg Glu Phe His Leu Leu Asp Ser Ala
145                 150                 155                 160
Val Tyr Tyr Leu Ser His Leu Glu Arg Ile Thr Glu Glu Gly Tyr Val
                165                 170                 175
Pro Thr Ala Gln Asp Val Leu Arg Ser Arg Met Pro Thr Thr Gly Ile
            180                 185                 190
Asn Glu Tyr Cys Phe Ser Val Gln Lys Thr Asn Leu Arg Ile Val Asp
        195                 200                 205
Val Gly Gly Gln Lys Ser Glu Arg Lys Lys Trp Ile His Cys Phe Glu
    210                 215                 220
Asn Val Ile Ala Leu Ile Tyr Leu Ala Ser Leu Ser Glu Tyr Asp Gln
225                 230                 235                 240
Cys Leu Glu Glu Asn Asn Gln Glu Asn Arg Met Lys Glu Ser Leu Ala
                245                 250                 255
Leu Phe Gly Thr Ile Leu Glu Leu Pro Trp Phe Lys Ser Thr Ser Val
            260                 265                 270
Ile Leu Phe Leu Asn Lys Thr Asp Ile Leu Glu Glu Lys Ile Pro Thr
        275                 280                 285
Ser His Leu Ala Thr Tyr Phe Pro Ser Phe Gln Gly Pro Lys Gln Asp
    290                 295                 300
Ala Glu Ala Ala Lys Arg Phe Ile Leu Asp Met Tyr Thr Arg Met Tyr
305                 310                 315                 320
Thr Gly Cys Val Asp Gly Pro Glu Gly Ser Lys Lys Gly Ala Arg Ser
                325                 330                 335
Arg Arg Leu Phe Ser His Tyr Thr Cys Ala Thr Asp Thr Gln Asn Ile
            340                 345                 350
Arg Lys Val Phe Lys Asp Val Arg Asp Ser Val Leu Ala Arg Tyr Leu
        355                 360                 365
Asp Glu Ile Asn Leu Leu
    370
```

<210> 38
<211> 34

<212> DNA
<213> Artificial Sequence

<220>
<223> Signaling probe 1 (binds Tag Sequence 1 (SEQ ID NO:126))

<400> 38
gccagtccca gttcctgtgc cttaagaacc tcgc 34

<210> 39
<211> 34
<212> DNA
<213> Artificial Sequence

<220>
<223> Signaling probe 2 (binds Tag Sequence 2 (SEQ ID NO:127))

<400> 39
gcgagtcgca gaacgacagg gttaacttcc tcgc 34

<210> 40
<211> 34
<212> DNA
<213> Artificial Sequence

<220>
<223> Signaling probe 3 (binds Tag Sequence 3 (SEQ ID NO:128))

<400> 40
gcgagagcga caagcagacc ctatagaacc tcgc 34

<210> 41
<211> 2556
<212> DNA
<213> Homo sapiens

<220>
<223> Umami T1R1 Nucleic Acid sequnece

<400> 41

```
atgctgctct gcacggctcg cctggtcggc ctgcagcttc tcatttcctg ctgctgggcc 60
tttgcctgcc atagcacgga gtcttctcct gacttcaccc tccccggaga ttacctcctg 120
gcaggcctgt tccctctcca ttctggctgt ctgcaggtga ggcacagacc cgaggtgacc 180
ctgtgtgaca ggtcttgtag cttcaatgag catggctacc acctcttcca ggctatgcgg 240
cttggggttg aggagataaa caactccacg gccctgctgc ccaacatcac cctggggtat 300
cagctgtatg atgtgtgttc tgactctgcc aatgtgtatg ccacactgag agtgctctcc 360
ctgccagggc aacaccacat agagctccaa ggagaccttc tccactattc ccctacggtg 420
ctggcagtga ttgggcctga cagcaccaac cgtgctgcca ccacagccgc cctgctgagc 480
cctttcctgg tgcccatgat tagctatgcg gccagcagcg agacgctcag cgtgaagcgg 540
cagtatccct ctttcctgcg caccatcccc aatgacaagt accaggtgga gaccatggtg 600
ctgctgctgc agaagttcgg gtggacctgg atctctctgg ttggcagcag tgacgactat 660
gggcagctag ggggtgcaggc actggagaac caggccactg gtcaggggat ctgcattgct 720
ttcaaggaca tcatgccctt ctctgcccag gtgggcgatg agaggatgca gtgcctcatg 780
cgccacctgg cccaggccgg ggccaccgtc gtggttgttt tttccagccg gcagttggcc 840
agggtgtttt tcgagtccgt ggtgctgacc aacctgactg gcaaggtgtg ggtcgcctca 900
gaagcctggg ccctctccag gcacatcact ggggtgcccg ggatccagcg cattgggatg 960
gtgctgggcg tggccatcca gaagagggct gtccctggcc tgaaggcgtt tgaagaagcc 1020
tatgcccggg cagacaagaa ggcccctagg ccttgccaca agggctcctg gtgcagcagc 1080
aatcagctct gcagagaatg ccaagctttc atggcacaca cgatgcccaa gctcaaagcc 1140
ttctccatga gttctgccta caacgcatac cgggctgtgt atgcggtggc ccatggcctc 1200
caccagctcc tgggctgtgc ctctggagct tgttccaggg gccgagtcta cccctggcag 1260
cttttggagc agatccacaa ggtgcatttc cttctacaca aggacactgt ggcgtttaat 1320
gacaacagag atcccctcag tagctataac ataattgcct gggactggaa tggacccaag 1380
tggaccttca cggtcctcgg ttcctccaca tggtctccag ttcagctaaa cataaatgag 1440
accaaaatcc agtggcacgg aaaggacaac caggtgccta agtctgtgtg ttccagcgac 1500
tgtcttgaag ggcaccagcg agtggttacg ggtttccatc actgctgctt tgagtgtgtg 1560
ccctgtgggg ctgggacctt cctcaacaag agtgacctct acagatgcca gccttgtggg 1620
aaagaagagt gggcacctga gggaagccag acctgcttcc cgcgcactgt ggtgtttttg 1680
gctttgcgtg agcacacctc ttgggtgctg ctggcagcta acacgctgct gctgctgctg 1740
ctgcttggga ctgctggcct gtttgcctgg cacctagaca cccctgtggt gaggtcagca 1800
gggggccgcc tgtgctttct tatgctgggc tccctggcag caggtagtgg cagcctctat 1860
ggcttctttg gggaacccac aaggcctgcg tgcttgctac gccaggccct ctttgccctt 1920
ggtttcacca tcttcctgtc ctgcctgaca gttcgctcat tccaactaat catcatcttc 1980

aagttttcca ccaaggtacc tacattctac cacgcctggg tccaaaacca cggtgctggc 2040
ctgtttgtga tgatcagctc agcggcccag ctgcttatct gtctaacttg gctggtggtg 2100
tggaccccac tgcctgctag ggaataccag cgcttccccc atctggtgat gcttgagtgc 2160
acagagacca actccctggg cttcatactg gccttcctct acaatggcct cctctccatc 2220
agtgcctttg cctgcagcta cctgggtaag gacttgccag agaactacaa cgaggccaaa 2280
tgtgtcacct tcagcctgct cttcaacttc gtgtcctgga tcgccttctt caccacggcc 2340
agcgtctacg acggcaagta cctgcctgcg gccaacatga tggctgggct gagcagcctg 2400
agcagcggct tcggtgggta ttttctgcct aagtgctacg tgatcctctg ccgcccagac 2460
ctcaacagca cagagcactt ccaggcctcc attcaggact acacgaggcg ctgcggctcc 2520
acctgaaggg cgaattcgga tccgcggccg ccttaa                           2556
```

<210> 42
<211> 841
<212> PRT
<213> Homo sapiens

<220>
<223> Human Umami T1R1 Isoform 1 Amino Acid sequnece

<400> 42

Met Leu Leu Cys Thr Ala Arg Leu Val Gly Leu Gln Leu Leu Ile Ser
1 5 10 15
Cys Cys Trp Ala Phe Ala Cys His Ser Thr Glu Ser Ser Pro Asp Phe
20 25 30
Thr Leu Pro Gly Asp Tyr Leu Leu Ala Gly Leu Phe Pro Leu His Ser
35 40 45
Gly Cys Leu Gln Val Arg His Arg Pro Glu Val Thr Leu Cys Asp Arg
50 55 60
Ser Cys Ser Phe Asn Glu His Gly Tyr His Leu Phe Gln Ala Met Arg
65 70 75 80
Leu Gly Val Glu Glu Ile Asn Asn Ser Thr Ala Leu Leu Pro Asn Ile
85 90 95
Thr Leu Gly Tyr Gln Leu Tyr Asp Val Cys Ser Asp Ser Ala Asn Val
100 105 110
Tyr Ala Thr Leu Arg Val Leu Ser Leu Pro Gly Gln His His Ile Glu
115 120 125
Leu Gln Gly Asp Leu Leu His Tyr Ser Pro Thr Val Leu Ala Val Ile
130 135 140
Gly Pro Asp Ser Thr Asn Arg Ala Ala Thr Thr Ala Ala Leu Leu Ser
145 150 155 160
Pro Phe Leu Val Pro Met Ile Ser Tyr Ala Ala Ser Ser Glu Thr Leu
165 170 175
Ser Val Lys Arg Gln Tyr Pro Ser Phe Leu Arg Thr Ile Pro Asn Asp
180 185 190
Lys Tyr Gln Val Glu Thr Met Val Leu Leu Leu Gln Lys Phe Gly Trp
195 200 205
Thr Trp Ile Ser Leu Val Gly Ser Ser Asp Asp Tyr Gly Gln Leu Gly
210 215 220
Val Gln Ala Leu Glu Asn Gln Ala Thr Gly Gln Gly Ile Cys Ile Ala
225 230 235 240
Phe Lys Asp Ile Met Pro Phe Ser Ala Gln Val Gly Asp Glu Arg Met
245 250 255
Gln Cys Leu Met Arg His Leu Ala Gln Ala Gly Ala Thr Val Val Val
260 265 270
Val Phe Ser Ser Arg Gln Leu Ala Arg Val Phe Phe Glu Ser Val Val
275 280 285
Leu Thr Asn Leu Thr Gly Lys Val Trp Val Ala Ser Glu Ala Trp Ala
290 295 300
Leu Ser Arg His Ile Thr Gly Val Pro Gly Ile Gln Arg Ile Gly Met
305 310 315 320
Val Leu Gly Val Ala Ile Gln Lys Arg Ala Val Pro Gly Leu Lys Ala
325 330 335
Phe Glu Glu Ala Tyr Ala Arg Ala Asp Lys Lys Ala Pro Arg Pro Cys
340 345 350
His Lys Gly Ser Trp Cys Ser Ser Asn Gln Leu Cys Arg Glu Cys Gln

355 360 365
Ala Phe Met Ala His Thr Met Pro Lys Leu Lys Ala Phe Ser Met Ser
370 375 380
Ser Ala Tyr Asn Ala Tyr Arg Ala Val Tyr Ala Val Ala His Gly Leu
385 390 395 400
His Gln Leu Leu Gly Cys Ala Ser Gly Ala Cys Ser Arg Gly Arg Val
405 410 415
Tyr Pro Trp Gln Leu Leu Glu Gln Ile His Lys Val His Phe Leu Leu
420 425 430
His Lys Asp Thr Val Ala Phe Asn Asp Asn Arg Asp Pro Leu Ser Ser
435 440 445
Tyr Asn Ile Ile Ala Trp Asp Trp Asn Gly Pro Lys Trp Thr Phe Thr
450 455 460
Val Leu Gly Ser Ser Thr Trp Ser Pro Val Gln Leu Asn Ile Asn Glu
465 470 475 480
Thr Lys Ile Gln Trp His Gly Lys Asp Asn Gln Val Pro Lys Ser Val
485 490 495
Cys Ser Ser Asp Cys Leu Glu Gly His Gln Arg Val Val Thr Gly Phe
500 505 510
His His Cys Cys Phe Glu Cys Val Pro Cys Gly Ala Gly Thr Phe Leu
515 520 525
Asn Lys Ser Asp Leu Tyr Arg Cys Gln Pro Cys Gly Lys Glu Glu Trp
530 535 540
Ala Pro Glu Gly Ser Gln Thr Cys Phe Pro Arg Thr Val Val Phe Leu
545 550 555 560
Ala Leu Arg Glu His Thr Ser Trp Val Leu Leu Ala Ala Asn Thr Leu
565 570 575
Leu Leu Leu Leu Leu Leu Gly Thr Ala Gly Leu Phe Ala Trp His Leu
580 585 590
Asp Thr Pro Val Val Arg Ser Ala Gly Gly Arg Leu Cys Phe Leu Met
595 600 605
Leu Gly Ser Leu Ala Ala Gly Ser Gly Ser Leu Tyr Gly Phe Phe Gly
610 615 620
Glu Pro Thr Arg Pro Ala Cys Leu Leu Arg Gln Ala Leu Phe Ala Leu
625 630 635 640
Gly Phe Thr Ile Phe Leu Ser Cys Leu Thr Val Arg Ser Phe Gln Leu
645 650 655
Ile Ile Ile Phe Lys Phe Ser Thr Lys Val Pro Thr Phe Tyr His Ala
660 665 670
Trp Val Gln Asn His Gly Ala Gly Leu Phe Val Met Ile Ser Ser Ala
675 680 685
Ala Gln Leu Leu Ile Cys Leu Thr Trp Leu Val Val Trp Thr Pro Leu
690 695 700
Pro Ala Arg Glu Tyr Gln Arg Phe Pro His Leu Val Met Leu Glu Cys
705 710 715 720
Thr Glu Thr Asn Ser Leu Gly Phe Ile Leu Ala Phe Leu Tyr Asn Gly
725 730 735
Leu Leu Ser Ile Ser Ala Phe Ala Cys Ser Tyr Leu Gly Lys Asp Leu
740 745 750
Pro Glu Asn Tyr Asn Glu Ala Lys Cys Val Thr Phe Ser Leu Leu Phe
755 760 765
Asn Phe Val Ser Trp Ile Ala Phe Phe Thr Thr Ala Ser Val Tyr Asp
770 775 780
Gly Lys Tyr Leu Pro Ala Ala Asn Met Met Ala Gly Leu Ser Ser Leu
785 790 795 800
Ser Ser Gly Phe Gly Gly Tyr Phe Leu Pro Lys Cys Tyr Val Ile Leu
805 810 815
Cys Arg Pro Asp Leu Asn Ser Thr Glu His Phe Gln Ala Ser Ile Gln
820 825 830
Asp Tyr Thr Arg Arg Cys Gly Ser Thr
835 840

<210> 43
<211> 586
<212> PRT
<213> Homo sapiens

<220>
<223> Human Umami T1R1 Isoform 2 Amino Acid sequnece

<400> 43

Leu Leu Cys Thr Ala Arg Leu Val Gly Leu Gln Leu Leu Ile Ser Cys
1 5 10 15
Cys Trp Ala Phe Ala Cys His Ser Thr Glu Ser Ser Pro Asp Phe Thr
20 25 30
Leu Pro Gly Asp Tyr Leu Leu Ala Gly Leu Phe Pro Leu His Ser Gly
35 40 45
Cys Leu Gln Val Arg His Arg Pro Glu Val Thr Leu Cys Asp Arg Ser
50 55 60
Cys Ser Phe Asn Glu His Gly Tyr His Leu Phe Gln Ala Met Arg Leu
65 70 75 80
Gly Val Glu Glu Ile Asn Asn Ser Thr Ala Leu Leu Pro Asn Ile Thr
85 90 95
Leu Gly Tyr Gln Leu Tyr Asp Val Cys Ser Asp Ser Ala Asn Val Tyr
100 105 110
Ala Thr Leu Arg Val Leu Ser Leu Pro Gly Gln His His Ile Glu Leu
115 120 125
Gln Gly Asp Leu Leu His Tyr Ser Pro Thr Val Leu Ala Val Ile Gly
130 135 140
Pro Asp Ser Thr Asn Arg Ala Ala Thr Thr Ala Ala Leu Leu Ser Pro
145 150 155 160
Phe Leu Val Pro Met Leu Leu Glu Gln Ile His Lys Val His Phe Leu
165 170 175
Leu His Lys Asp Thr Val Ala Phe Asn Asp Asn Arg Asp Pro Leu Ser
180 185 190
Ser Tyr Asn Ile Ile Ala Trp Asp Trp Asn Gly Pro Lys Trp Thr Phe
195 200 205
Thr Val Leu Gly Ser Ser Thr Trp Ser Pro Val Gln Leu Asn Ile Asn
210 215 220
Glu Thr Lys Ile Gln Trp His Gly Lys Asp Asn Gln Val Pro Lys Ser
225 230 235 240
Val Cys Ser Ser Asp Cys Leu Glu Gly His Gln Arg Val Val Thr Gly
245 250 255
Phe His His Cys Cys Phe Glu Cys Val Pro Cys Gly Ala Gly Thr Phe
260 265 270
Leu Asn Lys Ser Asp Leu Tyr Arg Cys Gln Pro Cys Gly Lys Glu Glu
275 280 285
Trp Ala Pro Glu Gly Ser Gln Thr Cys Phe Pro Arg Thr Val Val Phe
290 295 300
Leu Ala Leu Arg Glu His Thr Ser Trp Val Leu Leu Ala Ala Asn Thr
305 310 315 320
Leu Leu Leu Leu Leu Leu Leu Gly Thr Ala Gly Leu Phe Ala Trp His
325 330 335
Leu Asp Thr Pro Val Val Arg Ser Ala Gly Gly Arg Leu Cys Phe Leu
340 345 350
Met Leu Gly Ser Leu Ala Ala Gly Ser Gly Ser Leu Tyr Gly Phe Phe
355 360 365
Gly Glu Pro Thr Arg Pro Ala Cys Leu Leu Arg Gln Ala Leu Phe Ala
370 375 380
Leu Gly Phe Thr Ile Phe Leu Ser Cys Leu Thr Val Arg Ser Phe Gln
385 390 395 400
Leu Ile Ile Ile Phe Lys Phe Ser Thr Lys Val Pro Thr Phe Tyr His
405 410 415
Ala Trp Val Gln Asn His Gly Ala Gly Leu Phe Val Met Ile Ser Ser
420 425 430
Ala Ala Gln Leu Leu Ile Cys Leu Thr Trp Leu Val Val Trp Thr Pro
435 440 445
Leu Pro Ala Arg Glu Tyr Gln Arg Phe Pro His Leu Val Met Leu Glu
450 455 460

```
Cys Thr Glu Thr Asn Ser Leu Gly Phe Ile Leu Ala Phe Leu Tyr Asn
465                 470                 475                 480
Gly Leu Leu Ser Ile Ser Ala Phe Ala Cys Ser Tyr Leu Gly Lys Asp
                485                 490                 495
Leu Pro Glu Asn Tyr Asn Glu Ala Lys Cys Val Thr Phe Ser Leu Leu
            500                 505                 510
Phe Asn Phe Val Ser Trp Ile Ala Phe Phe Thr Thr Ala Ser Val Tyr
        515                 520                 525
Asp Gly Lys Tyr Leu Pro Ala Ala Asn Met Met Ala Gly Leu Ser Ser
    530                 535                 540
Leu Ser Ser Gly Phe Gly Gly Tyr Phe Leu Pro Lys Cys Tyr Val Ile
545                 550                 555                 560
Leu Cys Arg Pro Asp Leu Asn Ser Thr Glu His Phe Gln Ala Ser Ile
                565                 570                 575
Gln Asp Tyr Thr Arg Arg Cys Gly Ser Thr
            580                 585
```

<210> 44
<211> 480
<212> PRT
<213> Homo sapiens

<220>
<223> Human Umami T1R1 Isoform 3 Amino Acid sequnece

<400> 44

```
Met Leu Leu Cys Thr Ala Arg Leu Val Gly Leu Gln Leu Leu Ile Ser
 1               5                  10                  15
Cys Cys Trp Ala Phe Ala Cys His Ser Thr Glu Ser Ser Pro Asp Phe
            20                  25                  30
Thr Leu Pro Gly Asp Tyr Leu Leu Ala Gly Leu Phe Pro Leu His Ser
        35                  40                  45
Gly Cys Leu Gln Val Arg His Arg Pro Glu Val Thr Leu Cys Asp Arg
    50                  55                  60
Ser Cys Ser Phe Asn Glu His Gly Tyr His Leu Phe Gln Ala Met Arg
65                  70                  75                  80
Leu Gly Val Glu Glu Ile Asn Asn Ser Thr Ala Leu Leu Pro Asn Ile
                85                  90                  95
Thr Leu Gly Tyr Gln Leu Tyr Asp Val Cys Ser Asp Ser Ala Asn Val
            100                 105                 110
Tyr Ala Thr Leu Arg Val Leu Ser Leu Pro Gly Gln His His Ile Glu
        115                 120                 125
Leu Gln Gly Asp Leu Leu His Tyr Ser Pro Thr Val Leu Ala Val Ile
    130                 135                 140
Gly Pro Asp Ser Thr Asn Arg Ala Ala Thr Thr Ala Ala Leu Leu Ser
145                 150                 155                 160
Pro Phe Leu Val Pro Met Leu Ser Tyr Ala Ala Ser Ser Glu Thr Leu
                165                 170                 175
Ser Val Lys Arg Gln Tyr Pro Ser Phe Leu Arg Thr Ile Pro Asn Asp
            180                 185                 190
Lys Tyr Gln Val Glu Thr Met Val Leu Leu Leu Gln Lys Phe Gly Trp
        195                 200                 205
Thr Trp Ile Ser Leu Val Gly Ser Ser Asp Asp Tyr Gly Gln Leu Gly
    210                 215                 220
Val Gln Ala Leu Glu Asn Gln Ala Thr Gly Gln Gly Ile Cys Ile Ala
225                 230                 235                 240
Phe Lys Asp Ile Met Pro Phe Ser Ala Gln Val Gly Asp Glu Arg Met
                245                 250                 255
Gln Cys Leu Met Arg His Leu Ala Gln Ala Gly Ala Thr Val Val Val
            260                 265                 270
Val Phe Ser Ser Arg Gln Leu Ala Arg Val Phe Phe Glu Ser Val Val
        275                 280                 285
Leu Thr Asn Leu Thr Gly Lys Val Trp Val Ala Ser Glu Ala Trp Ala
    290                 295                 300

Leu Ser Arg His Ile Thr Gly Val Pro Gly Ile Gln Arg Ile Gly Met
305                 310                 315                 320
Val Leu Gly Val Ala Ile Gln Lys Arg Ala Val Pro Gly Leu Lys Ala
                325                 330                 335
Phe Glu Glu Ala Tyr Ala Arg Ala Asp Lys Lys Ala Pro Arg Pro Cys
            340                 345                 350
His Lys Gly Ser Trp Cys Ser Ser Asn Gln Leu Cys Arg Glu Cys Gln
        355                 360                 365
Ala Phe Met Ala His Thr Met Pro Lys Leu Lys Ala Phe Ser Met Ser
    370                 375                 380
Ser Ala Tyr Asn Ala Tyr Arg Ala Val Tyr Ala Val Ala His Gly Leu
385                 390                 395                 400
His Gln Leu Leu Gly Cys Ala Ser Gly Ala Cys Ser Arg Gly Arg Val
                405                 410                 415
Tyr Pro Trp Gln Thr Ser Thr Asp Ala Ser Leu Val Gly Lys Lys Ser
            420                 425                 430
Gly His Leu Arg Glu Ala Arg Pro Ala Ser Arg Ala Leu Trp Cys Phe
        435                 440                 445
Trp Leu Cys Val Ser Thr Pro Leu Gly Cys Cys Trp Gln Leu Thr Arg
    450                 455                 460
Cys Cys Cys Cys Cys Cys Leu Gly Leu Leu Ala Cys Leu Pro Gly Thr
465                 470                 475                 480
```

<210> 45
<211> 218
<212> PRT
<213> Homo sapiens

<220>
<223> Human Umami T1R1 Isoform 3 Amino Acid sequnece

<220>
<221> VARIANT
<222> 216
<223> Xaa = Any Amino Acid

<400> 45

```
Met Cys Thr Ala Arg Val Gly Ser Cys Cys Trp Ala Ala Cys His Ser
 1               5                  10                  15
Thr Ser Ser Asp Thr Gly Asp Tyr Ala Gly His Ser Gly Cys Val Arg
            20                  25                  30
His Arg Val Thr Cys Asp Arg Ser Cys Ser Asn His Gly Tyr His Ala
        35                  40                  45
Met Arg Gly Val Asn Asn Ser Thr Ala Asn Thr Gly Tyr Tyr Asp Val
    50                  55                  60
Cys Ser Asp Ser Ala Asn Val Tyr Ala Thr Arg Val Ser Gly His His
65                  70                  75                  80
Gly Asp His Tyr Ser Thr Val Ala Val Gly Asp Ser Thr Asn Arg Ala
                85                  90                  95
Ala Thr Thr Ala Ala Ser Val Met His Lys Val His His Lys Asp Thr
            100                 105                 110
Val Ala Asn Asp Asn Arg Asp Ser Ser Tyr Asn Ala Trp Asp Trp Asn
        115                 120                 125
Gly Lys Trp Thr Thr Val Gly Ser Ser Thr Trp Ser Val Asn Asn Thr
    130                 135                 140
Lys Trp His Gly Lys Asp Asn Val Lys Ser Val Cys Ser Ser Asp Cys
145                 150                 155                 160
Gly His Arg Val Val Thr Gly His His Cys Cys Cys Val Cys Gly Ala
                165                 170                 175
Gly Thr Asn Lys Ser Ala Thr Trp Val Arg Thr Cys Arg Thr Thr Thr
            180                 185                 190
Arg Thr Asn Val Ser Ser Ala Cys Ser Ser Thr Ser Cys Gly Ser Ser
        195                 200                 205
Ser Arg Ala Ser Thr Thr Ala Xaa Thr Cys


                210                 215
```

<210> 46
<211> 25
<212> DNA
<213> Artificial Sequence

<220>
<223> Target Sequence 1 used in generating a stable bitter receptor-expressing cell line

<400> 46
gttcttaagg cacaggaact gggac 25

<210> 47
<211> 25

<212> DNA
<213> Artificial Sequence

<220>
<223> Target Sequence 2 used in generating a stable bitter receptor-expressing cell line

<400> 47
gaagttaacc ctgtcgttct gcgac 25

<210> 48
<211> 34
<212> DNA
<213> Artificial Sequence

<220>
<223> Signaling Probe 1 used in generating a stable bitter receptor-expressing cell line

<400> 48
gccagtccca gttcctgtgc cttaagaacc tcgc 34

<210> 49
<211> 34
<212> DNA
<213> Artificial Sequence

<220>
<223> Signaling Probe 2 used in generating a stable bitter receptor-expressing cell line

<400> 49
gcgagtcgca gaacgacagg gttaacttcc tcgc 34

<210> 50
<211> 374
<212> PRT
<213> Homo sapiens

<220>
<223> Human GNA15

<400> 50

```
Met Ala Arg Ser Leu Thr Trp Arg Cys Cys Pro Trp Cys Leu Thr Glu
 1               5                  10                  15
Asp Glu Lys Ala Ala Ala Arg Val Asp Gln Glu Ile Asn Arg Ile Leu
            20                  25                  30
Leu Glu Gln Lys Lys Gln Asp Arg Gly Glu Leu Lys Leu Leu Leu Leu
```

```
        35                  40                  45
Gly Pro Gly Glu Ser Gly Lys Ser Thr Phe Ile Lys Gln Met Arg Ile
    50                  55                  60
Ile His Gly Ala Gly Tyr Ser Glu Glu Glu Arg Lys Gly Phe Arg Pro
65                  70                  75                  80
Leu Val Tyr Gln Asn Ile Phe Val Ser Met Arg Ala Met Ile Glu Ala
                85                  90                  95
Met Glu Arg Leu Gln Ile Pro Phe Ser Arg Pro Glu Ser Lys His His
            100                 105                 110
Ala Ser Leu Val Met Ser Gln Asp Pro Tyr Lys Val Thr Thr Phe Glu
        115                 120                 125
Lys Arg Tyr Ala Ala Ala Met Gln Trp Leu Trp Arg Asp Ala Gly Ile
    130                 135                 140
Arg Ala Cys Tyr Glu Arg Arg Arg Glu Phe His Leu Leu Asp Ser Ala
145                 150                 155                 160
Val Tyr Tyr Leu Ser His Leu Glu Arg Ile Thr Glu Glu Gly Tyr Val
                165                 170                 175
Pro Thr Ala Gln Asp Val Leu Arg Ser Arg Met Pro Thr Thr Gly Ile
            180                 185                 190
Asn Glu Tyr Cys Phe Ser Val Gln Lys Thr Asn Leu Arg Ile Val Asp
        195                 200                 205
Val Gly Gly Gln Lys Ser Glu Arg Lys Lys Trp Ile His Cys Phe Glu
    210                 215                 220
Asn Val Ile Ala Leu Ile Tyr Leu Ala Ser Leu Ser Glu Tyr Asp Gln
225                 230                 235                 240
Cys Leu Glu Glu Asn Asn Gln Glu Asn Arg Met Lys Glu Ser Leu Ala
                245                 250                 255
Leu Phe Gly Thr Ile Leu Glu Leu Pro Trp Phe Lys Ser Thr Ser Val
            260                 265                 270
Ile Leu Phe Leu Asn Lys Thr Asp Ile Leu Glu Glu Lys Ile Pro Thr
        275                 280                 285
Ser His Leu Ala Thr Tyr Phe Pro Ser Phe Gln Gly Pro Lys Gln Asp
    290                 295                 300
Ala Glu Ala Ala Lys Arg Phe Ile Leu Asp Met Tyr Thr Arg Met Tyr
305                 310                 315                 320
Thr Gly Cys Val Asp Gly Pro Glu Gly Ser Lys Lys Gly Ala Arg Ser
                325                 330                 335
Arg Arg Leu Phe Ser His Tyr Thr Cys Ala Thr Asp Thr Gln Asn Ile
            340                 345                 350
Arg Lys Val Phe Lys Asp Val Arg Asp Ser Val Leu Ala Arg Tyr Leu
        355                 360                 365
Asp Glu Ile Asn Leu Leu
    370
```

<210> 51
<211> 900
<212> DNA
<213> Homo sapiens

<220>
<223> human TAS2R1(F1) coding sequence

<400> 51

```
atgctagagt ctcacctcat tatctatttt cttcttgcag tgatacaatt tcttcttggg 60
attttcacaa atggcatcat tgtggtggtg aatggcattg acttgatcaa gcacagaaaa 120
atggctccgc tggatctcct tctttcttgt ctggcagttt ctagaatttt tctgcagttg 180
ttcatcttct acgttaatgt gattgttatc ttcttcatag aattcatcat gtgttctgcg 240
aattgtgcaa ttctcttatt tataaatgaa ttggaacttt ggcttgccac atggctcggc 300
gttttctatt gtgccaaggt tgccagcgtc cgtcacccac tcttcatctg gttgaagatg 360
aggatatcca agctggtccc atggatgatc ctggggtctc tgctatatgt atctatgatt 420
tgtgttttcc atagcaaata tgcagggttt atggtcccat acttcctaag gaaatttttc 480
tcccaaaatg ccacaattca aaaagaagat acactggcta tacagatttt ctcttttgtt 540
gctgagttct cagtgccatt gcttatcttc ctttttgctg ttttgctctt gattttctct 600
ctggggaggc acacccggca aatgagaaac acagtggccg gcagcagggt tcctggcagg 660

ggtgcaccca tcagcgcgtt gctgtctatc ctgtccttcc tgatcctcta cttctcccac 720
tgcatgataa aagtttttct ctcttctcta aagtttcaca tcagaaggtt catctttctg 780
ttcttcatcc ttgtgattgg tgtataccct tctggacact ctctcatctt aattttagga 840
aatcctaaat tgaaacaaaa tgcaaaaaag ttcctcctcc acagtaagtg ctgtcagtga 900
```

<210> 52
<211> 951
<212> DNA
<213> Homo sapiens

<220>
<223> human TAS2R3 (F5) coding sequence

<400> 52

```
atgatgggac tcaccgaggg ggtgttcctg attctgtctg gcactcagtt cacactggga 60
attctggtca attgtttcat tgagttggtc aatggtagca gctggttcaa gaccaagaga 120
atgtctttgt ctgacttcat catcaccacc ctggcactct tgaggatcat tctgctgtgt 180
attatcttga ctgatagttt tttaatagaa ttctctccca acacacatga ttcagggata 240
ataatgcaaa ttattgatgt ttcctggaca tttacaaacc atctgagcat ttggcttgcc 300
acctgtcttg gtgtcctcta ctgcctgaaa atcgccagtt tctctcaccc cacattcctc 360
tggctcaagt ggagagtttc tagggtgatg gtatggatgc tgttgggtgc actgctctta 420
tcctgtggta gtaccgcatc tctgatcaat gagtttaagc tctattctgt ctttagggga 480
attgaggcca ccaggaatgt gactgaacac ttcagaaaga agaggagtga gtattatctg 540
atccatgttc ttgggactct gtggtacctg cctcccttaa ttgtgtccct ggcctcctac 600
tctttgctca tcttctccct ggggaggcac acacggcaga tgctgcaaaa tgggacaagc 660
tccagagatc caaccactga ggcccacaag agggccatca gaatcatcct ttccttcttc 720
tttctcttct tactttactt tcttgctttc ttaattgcat catttggtaa tttcctacca 780
aaaaccaaga tggctaagat gattggtgaa gtaatgacaa tgttttatcc tgctggccac 840
tcatttattc tcattctggg gaacagtaag ctgaagcaga catttgtagt gatgctccgg 900
tgtgagtctg gtcatctgaa gcctggatcc aaggacccca ttttctctta g          951
```

<210> 53
<211> 900
<212> DNA
<213> Homo sapiens

<220>
<223> human TAS2R4 (F25) coding sequence

<400> 53

```
atgcttcggt tattctattt ctctgctatt attgcctcag ttattttaaa ttttgtagga 60
atcattatga atctgtttat tacagtggtc aattgcaaaa cttgggtcaa aagccataga 120
atctcctctt ctgataggat tctgttcagc ctgggcatca ccaggtttct tatgctggga 180
ctatttctgg tgaacaccat ctacttcgtc tcttcaaata cggaaaggtc agtctacctg 240
tctgcttttt ttgtgttgtg tttcatgttt ttggactcga gcagtgtctg gtttgtgacc 300
ttgctcaata tcttgtactg tgtgaagatt actaacttcc aacactcagt gtttctcctg 360
ctgaagcgga atatctcccc aaagatcccc aggctgctgc tggcctgtgt gctgatttct 420
gctttcacca cttgcctgta catcacgctt agccaggcat caccttttcc tgaacttgtg 480
actacgagaa ataacacatc atttaatatc agtgagggca tcttgtcttt agtggtttct 540
ttggtcttga gctcatctct ccagttcatc attaatgtga cttctgcttc cttgctaata 600
cactccttga ggagacatat acagaagatg cagaaaaatg ccactggttt ctggaatccc 660
cagacggaag ctcatgtagg tgctatgaag ctgatggtct atttcctcat cctctacatt 720
ccatattcag ttgctaccct ggtccagtat ctcccctttt atgcagggat ggatatgggg 780
accaaatcca tttgtctgat ttttgccacc ctttactctc caggacattc tgttctcatt 840
attatcacac atcctaaact gaaaacaaca gcaaagaaga ttctttgttt caaaaaatag 900
```

<210> 54
<211> 900
<212> DNA
<213> Homo sapiens

<220>
<223> human TAS2R5 (F11) coding sequence

<400> 54

```
atgctgagcg ctggcctagg actgctgatg ctggtggcag tggttgaatt tctcatcggt 60
ttaattggaa atggaagcct ggtggtctgg agttttagag aatggatcag aaaattcaac 120
tggtcctcat ataacctcat tatcctgggc ctggctggct gccgatttct cctgcagtgg 180
ctgatcattt tggacttaag cttgtttcca cttttccaga gcagccgttg gcttcgctat 240
cttagtatct tctgggtcct ggtaagccag gccagcttat ggtttgccac cttcctcagt 300
gtcttctatt gcaagaagat cacgaccttc gatcgcccgg cctacttgtg gctgaagcag 360
agggcctata acctgagtct ctggtgcctt ctgggctact ttataatcaa tttgttactt 420
acagtccaaa ttggcttaac attctatcat cctccccaag gaaacagcag cattcggtat 480
ccctttgaaa gctggcagta cctgtatgca tttcagctca attcaggaag ttatttgcct 540
ttagtggtgt ttcttgtttc ctctgggatg ctgattgtct ctttgtatac acaccacaag 600
aagatgaagg tccattcagc tggtaggagg gatgtccggg ccaaggctca catcactgcg 660
ctgaagtcct tgggctgctt cctcttactt cacctggttt atatcatggc cagccccttc 720
tccatcacct ccaagactta tcctcctgat ctcaccagtg tcttcatctg ggagacactc 780
atggcagcct atccttctct tcattctctc atattgatca tggggattcc tagggtgaag 840
cagacttgtc agaagatcct gtggaagaca gtgtgtgctc ggagatgctg ggcccatga 900
```

<210> 55
<211> 957
<212> DNA
<213> Homo sapiens

<220>
<223> human TAS2R7 (F4) coding sequence

<400> 55

```
atggcagata aagtgcagac tactttattg ttcttagcag ttggagagtt ttcagtgggg 60
atcttaggga atgcattcat tggattggta aactgcatgg attgggtcaa gaagaggaaa 120
attgcctcca ttgatttaat cctcacaagt ctggccatat ccagaatttg tctattgtgc 180
gtaatactat tagattgttt tatattggtg ctatatccag atgtctatgc cactggtaaa 240
gaaatgagaa tcattgactt cttctggaca ctaaccaatc atttaagtat ctggtttgca 300
acctgcctca gcatttacta tttcttcaag ataggtaatt tctttcaccc acttttcctc 360
tggatgaagt ggagaattga cagggtgatt tcctggattc tactggggtg cgtggttctc 420
tctgtgttta ttagccttcc agccactgag aatttgaacg ctgatttcag gttttgtgtg 480
aaggcaaaga ggaaaacaaa cttaacttgg agttgcagag taaataaaac tcaacatgct 540
tctaccaagt tatttctcaa cctggcaacg ctgctcccct tttgtgtgtg cctaatgtcc 600
tttttcctct tgatcctctc cctgcggaga catatcaggc gaatgcagct cagtgccaca 660
gggtgcagag accccagcac agaagcccat gtgagagccc tgaaagctgt catttccttc 720
cttctcctct ttattgccta ctatttgtcc tttctcattg ccacctccag ctactttatg 780
ccagagacgg aattagctgt gatttttggt gagtccatag ctctaatcta cccctcaagt 840
cattcattta tcctaatact ggggaacaat aaattaagac atgcatctct aaaggtgatt 900
tggaaagtaa tgtctattct aaaaggaaga aaattccaac aacataaaca aatctga    957
```

<210> 56
<211> 930
<212> DNA
<213> Homo sapiens

<220>
<223> human TAS2R8 (F2) coding sequence

<400> 56

```
atgttcagtc ctgcagataa catctttata atcctaataa ctggagaatt catactagga 60
atattgggga atggatacat tgcactagtc aactggattg actggattaa gaagaaaaag 120
atttccacag ttgactacat ccttaccaat ttagttatcg ccagaatttg tttgatcagt 180
gtaatggttg taaatggcat tgtaatagta ctgaacccag atgtttatac aaaaaataaa 240
caacagatag tcatttttac cttctggaca tttgccaact acttaaatat gtggattacc 300
acctgcctta atgtcttcta ttttctgaag atagccagtt cctctcatcc actttttctc 360
tggctgaagt ggaaaattga tatggtggtg cactggatcc tgctgggatg ctttgccatt 420
tccttgttgg tcagccttat agcagcaata gtactgagtt gtgattatag gtttcatgca 480
attgccaaac ataaaagaaa cattactgaa atgttccatg tgagtaaaat accatacttt 540
gaacccttga ctctctttaa cctgtttgca attgtcccat ttattgtgtc actgatatca 600
tttttccttt tagtaagatc tttatggaga cataccaagc aaataaaact ctatgctacc 660

ggcagtagag accccagcac agaagttcat gtgagagcca ttaaaactat gacttcattt 720
atcttctttt ttttcctata ctatatttct tctattttga tgacctttag ctatcttatg 780
acaaaataca agttagctgt ggagtttgga gagattgcag caattctcta cccccttgggt 840
cactcactta ttttaattgt tttaaataat aaactgaggc agacatttgt cagaatgctg 900
acatgtagaa aaattgcctg catgatatga                                  930
```

<210> 57
<211> 939
<212> DNA
<213> Homo sapiens

<220>
<223> human TAS2R9 (F24) coding sequence

<400> 57

atgccaagtg caatagaggc aatatatatt attttaattg ctggtgaatt gaccataggg 60
atttggggaa atggattcat tgtactagtt aactgcattg actggctcaa aagaagagat 120
atttccttga ttgacatcat cctgatcagc ttggccatct ccagaatctg tctgctgtgt 180
gtaatatcat tagatggctt ctttatgctg ctctttccag gtacatatgg caatagcgtg 240
ctagtaagca ttgtgaatgt tgtctggaca tttgccaata attcaagtct ctggtttact 300
tcttgcctca gtatcttcta tttactcaag atagccaata tatcgcaccc atttttcttc 360
tggctgaagc taaagatcaa caaggtcatg cttgcgattc ttctggggtc ctttcttatc 420
tctttaatta ttagtgttcc aaagaatgat gatatgtggt atcacctttt caaagtcagt 480
catgaagaaa acattacttg gaaattcaaa gtgagtaaaa ttccaggtac tttcaaacag 540
ttaaccctga acctgggggt gatggttccc tttatccttt gcctgatctc atttttcttg 600
ttactttct ccctagttag acacaccaag cagattcgac tgcatgctac agggttcaga 660
gaccccagta cagaggccca catgagggcc ataaaggcag tgatcatctt tctgctcctc 720
ctcatcgtgt actacccagt ctttcttgtt atgacctcta gcgctctgat tcctcaggga 780
aaattagtgt tgatgattgg tgacatagta actgtcattt tcccatcaag ccattcattc 840
attctaatta tgggaaatag caagttgagg gaagcttttc tgaagatgtt aagatttgtg 900
aagtgtttcc ttagaagaag aaagcctttt gttccatag 939

<210> 58
<211> 924
<212> DNA
<213> Homo sapiens

<220>
<223> human TAS2R10 (F16) coding sequence

<400> 58

atgctacgtg tagtggaagg catcttcatt tttgttgtag ttagtgagtc agtgtttggg 60
gttttgggga atggatttat tggacttgta aactgcattg actgtgccaa gaataagtta 120
tctacgattg gctttattct caccggctta gctatttcaa gaatttttct gatatggata 180
ataattacag atggatttat acagatattc tctccaaata tatatgcctc cggtaaccta 240
attgaatata ttagttactt ttgggtaatt ggtaatcaat caagtatgtg gtttgccacc 300
agcctcagca tcttctattt cctgaagata gcaaattttt ccaactacat atttctctgg 360
ttgaagagca gaacaaatat ggttcttccc ttcatgatag tattcttact tatttcatcg 420
ttacttaatt ttgcatacat tgcgaagatt cttaatgatt ataaaatgaa gaatgacaca 480
gtctgggatc tcaacatgta taaaagtgaa tactttatta aacagatttt gctaaatctg 540
ggagtcattt tcttctttac actatcccta attacatgta ttttttaat catttccctt 600
tggagacaca acaggcagat gcaatcaaat gtgacaggat tgagagactc caacacagaa 660
gctcatgtga aggcaatgaa agttttgata tctttcatca tcctctttat cttgtatttt 720
ataggcatgg ccatagaaat atcatgtttt actgtgcgag aaaacaaact gctgcttatg 780
tttggaatga caaccacagc catctatccc tggggtcact catttatctt aattctagga 840
aacagcaagc taaagcaagc ctctttgagg gtactgcagc aattgaagtg ctgtgagaaa 900
aggaaaaatc tcagagtcac atag 924

<210> 59
<211> 912
<212> DNA
<213> Homo sapiens

<220>
<223> human TAS2R13 (F3) coding sequence

<400> 59

```
atggaaagtg ccctgccgag tatcttcact cttgtaataa ttgcagaatt cataattggg 60
aatttgagca atggatttat agtactgatc aactgcattg actgggtcag taaaagagag 120
ctgtcctcag tcgataaact cctcattatc ttggcaatct ccagaattgg gctgatctgg 180
gaaatattag taagttggtt tttagctctg cattatctag ccatatttgt gtctggaaca 240
ggattaagaa ttatgatttt tagctggata gtttctaatc acttcaatct ctggcttgct 300
acaatcttca gcatctttta tttgctcaaa atagcgagtt tctctagccc tgcttttctc 360
tatttgaagt ggagagtaaa caaagtgatt ctgatgatac tgctaggaac cttggtcttc 420
ttattttttaa atctgataca aataaacatg catataaaag actggctgga ccgatatgaa 480
agaaacacaa cttggaattt cagtatgagt gactttgaaa cattttcagt gtcggtcaaa 540
ttcactatga ctatgttcag tctaacacca tttactgtgg ccttcatctc ttttctcctg 600
ttaatttctt ccctgcagaa acatctccag aaaatgcaac tcaattacaa aggacacaga 660
gaccccagga ccaaggtcca tacaaatgcc ttgaaaattg tgatctcatt cctttttattc 720
tatgctagtt tctttctatg tgttctcata tcatggattt ctgagctgta tcagaacaca 780
gtgatctaca tgctttgtga gacgattgga gtcttctctc cttcaagcca ctcctttctt 840
ctgattctag gaaacgctaa gttaagacag gcctttcttt tggtggcagc taaggtatgg 900
gctaaacgat ga                                                     912
```

<210> 60
<211> 954
<212> DNA
<213> Homo sapiens

<220>
<223> human TAS2R14 (F15) coding sequence

<400> 60

```
atgggtggtg tcataaagag catatttaca ttcgttttaa ttgtggaatt tataattgga 60
aatttaggaa atagtttcat agcactggtg aactgtattg actgggtcaa gggaagaaag 120
atctcttcgg ttgatcggat cctcactgct ttggcaatct ctcgaattag cctggtttgg 180
ttaatattcg gaagctggtg tgtgtctgtg tttttcccag ctttatttgc cactgaaaaa 240
atgttcagaa tgcttactaa tatctggaca gtgatcaatc attttagtgt ctggttagct 300
acaggcctcg gtactttta ttttctcaag atagccaatt tttctaactc tatttttctc 360
tacctaaagt ggagggttaa aaaggtggtt ttggtgctgc ttcttgtgac ttcggtcttc 420
ttgtttttaa atattgcact gataaacatc catataaatg ccagtatcaa tggatacaga 480
agaaacaaga cttgcagttc tgattcaagt aactttacac gattttccag tcttattgta 540
ttaaccagca ctgtgttcat tttcataccc tttactttgt ccctggcaat gtttcttctc 600
ctcatcttct ccatgtggaa acatcgcaag aagatgcagc acactgtcaa aatatccgga 660
gacgccagca ccaaagccca cagaggagtt aaaagtgtga tcactttctt cctactctat 720
gccatttctt ctctgtcttt tttcatatca gtttggacct ctgaaaggtt ggaggaaaat 780
ctaattattc tttcccaggt gatgggaatg gcttatcctt catgtcactc atgtgttctg 840
attcttggaa acaagaagct gagacaggcc tctctgtcag tgctactgtg gctgaggtac 900
atgttcaaag atggggagcc ctcaggtcac aaagaattta gagaatcatc ttga       954
```

<210> 61
<211> 876
<212> DNA
<213> Homo sapiens

<220>
<223> human TAS2R16 (F14) coding sequence

<400> 61

```
atgataccca tccaactcac tgtcttcttc atgatcatct atgtgcttga gtccttgaca 60
attattgtgc agagcagcct aattgttgca gtgctgggca gagaatggct gcaagtcaga 120
aggctgatgc ctgtggacat gattctcatc agcctgggca tctctcgctt ctgtctacag 180
tgggcatcaa tgctgaacaa tttttgctcc tattttaatt tgaattatgt actttgcaac 240
ttaacaatca cctgggaatt ttttaatatc cttacattct ggttaaacag cttgcttacc 300
gtgttctact gcatcaaggt ctcttctttc acccatcaca tctttctctg gctgaggtgg 360
agaattttga ggttgtttcc ctggatatta ctgggttctc tgatgattac ttgtgtaaca 420
atcatccctt cagctattgg gaattacatt caaattcagt tactcaccat ggagcatcta 480
ccaagaaaca gcactgtaac tgacaaactt gaaaatttc atcagtatca gttccaggct 540
catacagttg cattggttat tcctttcatc ctgttcctgg cctccaccat ctttctcatg 600
gcatcactga ccaagcagat acaacatcat agcactggtc actgcaatcc aagcatgaaa 660

gcgcgcttca ctgccctgag gtcccttgcc gtcttattta ttgtgtttac ctcttacttt 720
ctaaccatac tcatcaccat tataggtact ctatttgata agagatgttg gttatgggtc 780
tgggaagctt ttgtctatgc tttcatctta atgcattcca cttcactgat gctgagcagc 840
cctacgttga aaaggattct aaagggaaag tgctag                           876
```

<210> 62
<211> 1002
<212> DNA
<213> Homo sapiens

<220>
<223> human TAS2R38 (F7) coding sequence

<400> 62

```
atgttgactc taactcgcat ccgcactgtg tcctatgaag tcaggagtac atttctgttc 60
atttcagtcc tggagtttgc agtggggttt ctgaccaatg ccttcgtttt cttggtgaat 120
ttttgggatg tagtgaagag gcaggcactg agcaacagtg attgtgtgct gctgtgtctc 180
agcatcagcc ggcttttcct gcatggactg ctgttcctga gtgctatcca gcttacccac 240
ttccagaagt tgagtgaacc actgaaccac agctaccaag ccatcatcat gctatggatg 300
attgcaaacc aagccaacct ctggcttgct gcctgcctca gcctgcttta ctgctccaag 360
ctcatccgtt tctctcacac cttcctgatc tgcttggcaa gctgggtctc caggaagatc 420
tcccagatgc tcctgggtat tattctttgc tcctgcatct gcactgtcct ctgtgtttgg 480
tgctttttta gcagacctca cttcacagtc acaactgtgc tattcatgaa taacaataca 540
aggctcaact ggcagattaa agatctcaat ttattttatt cctttctctt ctgctatctg 600
tggtctgtgc ctcctttcct attgtttctg gtttcttctg ggatgctgac tgtctccctg 660
ggaaggcaca tgaggacaat gaaggtctat accagaaact ctcgtgaccc cagcctggag 720
gcccacatta aagccctcaa gtctcttgtc tcctttttct gcttctttgt gatatcatcc 780
tgtgctgcct tcatctctgt gcccctactg attctgtggc gcgacaaaat aggggtgatg 840
gtttgtgttg ggataatggc agcttgtccc tctgggcatg cagccatcct gatctcaggc 900
aatgccaagt tgaggagagc tgtgatgacc attctgctct gggctcagag cagcctgaag 960
gtaagagccg accacaaggc agattcccgg acactgtgct ga                   1002
```

<210> 63
<211> 1014
<212> DNA
<213> Homo sapiens

<220>
<223> human TAS2R39 (F23) coding sequence

<400> 63

```
atgctaggga gatgttttcc tccagacacc aaagagaagc aacagctcag aatgactaaa 60
ctctgcgatc ctgcagaaag tgaattgtcg ccatttctca tcaccttaat tttagcagtt 120
ttacttgctg aatacctcat tggtatcatt gcaaatggtt tcatcatggc tatacatgca 180
gctgaatggg ttcaaaataa ggcagtttcc acaagtggca ggatcctggt tttcctgagt 240
gtatccagaa tagctctcca aagcctcatg atgttagaaa ttaccatcag ctcaacctcc 300
ctaagttttt attctgaaga cgctgtatat tatgcattca aaataagttt tatattctta 360
aatttttgta gcctgtggtt tgctgcctgg ctcagtttct tctactttgt gaagattgcc 420
aatttctcct acccccttttt cctcaaactg aggtggagaa ttactggatt gataccctgg 480
cttctgtggc tgtccgtgtt tatttccttc agtcacagca tgttctgcat caacatctgc 540
actgtgtatt gtaacaattc tttccctatc cactcctcca actccactaa gaaaacatac 600
ttgtctgaga tcaatgtggt cggtctggct tttttcttta acctggggat tgtgactcct 660
ctgatcatgt tcatcctgac agccaccctg ctgatcctct ctctcaagag acacacccta 720
cacatgggaa gcaatgccac agggtccaac gaccccagca tggaggctca catgggggcc 780
atcaaagcta tcagctactt tctcattctc tacattttca atgcagttgc tctgtttatc 840
tacctgtcca acatgtttga catcaacagt ctgtggaata atttgtgcca gatcatcatg 900
gctgcctacc ctgccagcca ctcaattcta ctgattcaag ataaccctgg gctgagaaga 960
gcctggagcg gcttcagctt cgacttcatc tttacccaaa agagtggact ctga       1014
```

<210> 64
<211> 972
<212> DNA
<213> Homo sapiens

<220>
<223> human TAS2R40 (F19) coding sequence

<400> 64

```
atggcaacgg tgaacacaga tgccacagat aaagacatat ccaagttcaa ggtcaccttc 60
actttggtgg tctccggaat agagtgcatc actggcatcc ttgggagtgg cttcatcacg 120
gccatctatg gggctgagtg ggccaggggc aaaacactcc ccactggtga ccgcattatg 180
ttgatgctga gcttttccag gctcttgcta cagatttgga tgatgctgga gaacattttc 240
agtctgctat tccgaattgt ttataaccaa aactcagtgt atatcctctt caaagtcatc 300
actgtctttc tgaaccattc caatctctgg tttgctgcct ggctcaaagt cttctattgt 360
cttagaattg caaacttcaa tcatcctttg ttcttcctga tgaagaggaa aatcatagtg 420
ctgatgcctt ggcttctcag gctgtcagtg ttggtttcct taagcttcag ctttcctctc 480
tcgagagatg tcttcaatgt gtatgtgaat agctccattc ctatcccctc ctccaactcc 540
acggagaaga agtacttctc tgagaccaat atggtcaacc tggtattttt ctataacatg 600
gggatcttcg ttcctctgat catgttcatc ctggcagcca ccctgctgat cctctctctc 660
aagagacaca ccctacacat gggaagcaat gccacagggt ccagggaccc cagcatgaag 720
gctcacatag gggccatcaa agccaccagc tactttctca tcctctacat tttcaatgca 780
attgctctat ttctttccac gtccaacatc tttgacactt acagttcctg gaatattttg 840
tgcaagatca tcatggctgc ctaccctgcc ggccactcag tacaactgat cttgggcaac 900
cctgggctga gaagagcctg gaagcggttt cagcaccaag ttcctcttta cctaaaaggg 960
cagactctgt ga                                                    972
```

<210> 65
<211> 924
<212> DNA
<213> Homo sapiens

<220>
<223> human TAS2R41 (F18) coding sequence

<400> 65

```
atgcaagcag cactgacggc cttcttcgtg ttgctcttta gcctgctgag tcttctgggg 60
attgcagcga atggcttcat tgtgctggtg ctgggcaggg agtggctgcg atatggcagg 120
ttgctgccct tggatatgat cctcattagc ttgggtgcct cccgcttctg cctgcagttg 180
gttgggacgg tgcacaactt ctactactct gcccagaagg tcgagtactc tggggtctc 240
ggccgacagt tcttccatct acactggcac ttcctgaact cagccacctt ctggttttgc 300
agctggctca gtgtcctgtt ctgtgtgaag attgctaaca tcacacactc caccttcctg 360
tggctgaagt ggaggttccc agggtgggtg ccctggctcc tgttgggctc tgtcctgatc 420
tccttcatca taaccctgct gtttttttgg gtgaactacc ctgtatatca agaattttta 480
attagaaaat tttctgggaa catgacctac aagtggaata caaggataga aacatactat 540
ttcccatccc tgaaactggt catctggtca attccttttt ctgtttttct ggtctcaatt 600
atgctgctga ttaattctct gaggaggcat actcagagaa tgcagcacaa cgggcacagc 660
ctgcaggacc ccagcaccca ggctcacacc agagctctga agtccctcat ctccttcctc 720
attctttatg ctctgtcctt tctgtccctg atcattgatg ccgcaaaatt tatctccatg 780
cagaacgact tttactggcc atggcaaatt gcagtctacc tgtgcatatc tgtccatccc 840
ttcatcctca tcttcagcaa cctcaagctt cgaagcgtgt tctcacagct cctgttgttg 900
gcaaggggct tctgggtggc ctga                                        924
```

<210> 66
<211> 930
<212> DNA
<213> Homo sapiens

<220>
<223> human TAS2R43 (F6) coding sequence

<400> 66

```
atgataactt ttctgcccat catttttcc agtctggtag tggttacatt tgttattgga 60
aattttgcta atggcttcat agcactggta aattccattg agtggttcaa gagacaaaag 120
atctcctttg ctgaccaaat tctcactgct ctggcggtct ccagagttgg tttgctctgg 180
gtattattat taaactggta ttcaactgtg ttgaatccag cttttaatag tgtagaagta 240
agaactactg cttataatat ctgggcagtg atcaaccatt tcagcaactg gcttgctact 300
accctcagca tattttattt gctcaagatt gccaatttct ccaactttat ttttcttcac 360
ttaaagagga gagttaagag tgtcattctg gtgatgttgt tggggccttt gctatttttg 420
gcttgtcatc tttttgtgat aaacatgaat gagattgtgc ggacaaaaga atttgaagga 480
aacatgactt ggaagatcaa attgaagagt gcaatgtact tttcaaatat gactgtaacc 540

atggtagcaa acttagtacc cttcactctg accctactat cttttatgct gttaatctgt 600
tctttgtgta aacatctcaa gaagatgcag ctccatggta aaggatctca agatcccagc 660
accaaggtcc acataaaagc tttgcaaact gtgatctcct tcctcttgtt atgtgccatt 720
tactttctgt ccataatgat atcagtttgg agttttggaa gtctggaaaa caaacctgtc 780
ttcatgttct gcaaagctat tagattcagc tatccttcaa tccacccatt catcctgatt 840
tggggaaaca agaagctaaa gcagactttt ctttcagttt tttggcaaat gaggtactgg 900
gtgaaaggag agaagacttc atctccatga                                  930
```

<210> 67
<211> 930
<212> DNA
<213> Homo sapiens

<220>
<223> human TAS2R44 (F12) coding sequence

<400> 67

```
atgacaactt ttatacccat catttttcc agtgtggtag tggttctatt tgttattgga 60
aattttgcta atggcttcat agcattggta aattccattg agcgggtcaa gagacaaaag 120
atctcttttg ctgaccagat tctcactgct ctggcggtct ccagagttgg tttgctctgg 180
gtattattat taaattggta ttcaactgtg tttaatccag ctttttatag tgtagaagta 240
agaactactg cttataatgt ctgggcagta accggccatt tcagcaactg gcttgctact 300
agcctcagca tattttattt gctcaagatt gccaatttct ccaaccttat ttttcttcac 360
ttaaagagga gagttaagag tgtcattctg gtgatgctgt tggggccttt actatttttg 420
gcttgtcaac tttttgtgat aaacatgaaa gagattgtac ggacaaaaga atatgaagga 480
aacttgactt ggaagatcaa attgaggagt gcagtgtacc tttcagatgc gactgtaacc 540
acgctaggaa acttagtgcc cttcactctg accctgctat gtttttgct gttaatctgt 600
tctctgtgta aacatctcaa gaagatgcag ctccatggta aaggatctca agatcccagc 660
accaaggtcc acataaaagc tttgcaaact gtgatctttt tcctcttgtt atgtgccgtt 720
tactttctgt ccataatgat atcagtttgg agttttggga gtctggaaaa caaacctgtc 780
ttcatgttct gcaaagctat tagattcagc tatccttcaa tccacccatt catcctgatt 840
tggggaaaca agaagctaaa gcagactttt ctttcagttt tgcggcaagt gaggtactgg 900
gtgaaaggag agaagccttc atctccatga                                   930
```

<210> 68
<211> 900
<212> DNA
<213> Homo sapiens

<220>
<223> human TAS2R45 (F8) coding sequence

<400> 68

```
atgataactt ttctgcccat catattttcc attctagtag tggttacatt tgttattgga 60
aattttgcta atggcttcat agcgttggta aattccaccg agtgggtgaa gagacaaaag 120
atctcctttg ctgaccaaat tgtcactgct ctggcggtct ccagagttgg tttgctctgg 180
gtgttattat taaattggta ttcaactgtg ttgaatccag ctttttgtag tgtagaatta 240
agaactactg cttataatat ctgggcagta accggccatt tcagcaactg gcctgctact 300
agcctcagca tattttattt gctcaagatt gccaatttct ccaaccttat ttttcttcgc 360
ttaaagagga gagttaagag tgtcattctg gtgatgctgt tggggccttt gctatttttg 420
gcttgtcatc tttttgtggt aaacatgaat cagattgtat ggacaaaaga atatgaagga 480
aacatgactt ggaagatcaa attgaggcgt gcaatgtacc tttcagatac gactgtaacc 540
atgctagcaa acttagtacc ctttactgta accctgatat cttttctgct gttagtctgt 600
tctctgtgta aacatctcaa gaagatgcac ctccatggca aaggatctca agatcccagt 660
accaaggtcc acataaaagt tttgcaaact gtgatctcct tcctcttgtt atgtgccatt 720
tactttgtgt ctgtaataat atcagtttgg agttttaaga atctggaaaa caaacctgtc 780
ttcatgttct gccaagctat tggattcagc tgttcttcag cccacccgtt catcctgatt 840
tggggaaaca agaagctaaa gcagacttat ctttcagttt tgtggcaaat gaggtactga 900
```

<210> 69
<211> 900
<212> DNA
<213> Homo sapiens

<220>
<223> human TAS2R46 (F9) coding sequence

<400> 69

atgataactt ttctgcccat cattttttcc attctaatag tggttacatt tgtgattgga 60
aattttgcta atggcttcat agcattggta aattccattg agtggtttaa gagacaaaag 120
atctcttttg ctgaccaaat tctcactgct ctggcagtct ccagagttgg tttactctgg 180
gtattagtat taaattggta tgcaactgag ttgaatccag cttttaacag tatagaagta 240
agaattactg cttacaatgt ctgggcagta atcaaccatt tcagcaactg gcttgctact 300
agcctcagca tattttattt gctcaagatt gccaatttct ccaaccttat ttttcttcac 360
ttaaagagga gagttaagag tgttgttctg gtgatactat tggggccttt gctatttttg 420
gtttgtcatc tttttgtgat aaacatgaat cagattatat ggacaaaaga atatgaagga 480
aacatgactt ggaagatcaa actgaggagt gcaatgtacc tttcaaatac aacggtaacc 540
atcctagcaa acttagttcc cttcactctg accctgatat cttttctgct gttaatctgt 600
tctctgtgta aacatctcaa aaagatgcag ctccatggca aaggatctca agatcccagc 660
atgaaggtcc acataaaagc tttgcaaact gtgacctcct tcctcttgtt atgtgccatt 720
tactttctgt ccataatcat gtcagtttgg agttttgaga gtctggaaaa caaacctgtc 780
ttcatgttct gcgaagctat tgcattcagc tatccttcaa cccacccatt catcctgatt 840
tggggaaaca agaagctaaa gcagactttt ctttcagttt tgtggcaaat gaggtactga 900

<210> 70
<211> 960
<212> DNA
<213> Homo sapiens

<220>
<223> human TAS2R47 (F22) coding sequence

<400> 70

atgataactt ttctgcccat cattttttcc attctaatag tggttatatt tgttattgga 60
aattttgcta atggcttcat agcattggta aattccattg agtgggtcaa gagacaaaag 120
atctcctttg ttgaccaaat tctcactgct ctggcggtct ccagagttgg tttgctctgg 180
gtgttattac tacattggta tgcaactcag ttgaatccag ctttttatag tgtagaagta 240
agaattactg cttataatgt ctgggcagta accaaccatt tcagcagctg gcttgctact 300
agcctcagca tgttttattt gctcaggatt gccaatttct ccaaccttat ttttcttcgc 360
ataaagagga gagttaagag tgttgttctg gtgatactgt tggggccttt gctatttttg 420
gtttgtcatc tttttgtgat aaacatggat gagactgtat ggacaaaaga atatgaagga 480
aacgtgactt ggaagatcaa attgaggagt gcaatgtacc attcaaatat gactctaacc 540
atgctagcaa actttgtacc cctcactctg accctgatat cttttctgct gttaatctgt 600
tctctgtgta aacatctcaa gaagatgcag ctccatggca aaggatctca agatcccagc 660
accaaggtcc acataaaagc tttgcaaact gtgacctcct ttcttctgtt atgtgccatt 720
tactttctgt ccatgatcat atcagtttgt aattttggga ggctggaaaa gcaacctgtc 780
ttcatgttct gccaagctat tatattcagc tatccttcaa cccacccatt catcctgatt 840
ttgggaaaca agaagctaaa gcagattttt ctttcagttt tgcggcatgt gaggtactgg 900
gtgaaagaca gaagccttcg tctccataga ttcacaagag ggcattgtg tgtcttctga 960

<210> 71
<211> 900
<212> DNA
<213> Homo sapiens

<220>
<223> human TAS2R48 (F17) coding sequence

<400> 71

atgatgtgtt ttctgctcat catttcatca attctggtag tgtttgcatt tgttcttgga 60
aatgttgcca atggcttcat agccctagta aatgtcattg actgggttaa cacacgaaag 120
atctcctcag ctgagcaaat tctcactgct ctggtggtct ccagaattgg tttactctgg 180
gtcatgttat tcctttggta tgcaactgtg tttaattctg ctttatatgg tttagaagta 240
agaattgttg cttctaatgc ctgggctgta acgaaccatt tcagcatgtg gcttgctgct 300
agcctcagca tattttgttt gctcaagatt gccaatttct ccaaccttat ttctctccac 360
ctaaagaaga gaattaagag tgttgttctg gtgatactgt tggggccctt ggtatttctg 420
atttgtaatc ttgctgtgat aaccatggat gagagagtgt ggacaaaaga atatgaagga 480

aatgtgactt ggaagatcaa attgaggaat gcaatacacc tttcaagctt gactgtaact 540
actctagcaa acctcatacc ctttactctg agcctaatat gttttctgct gttaatctgt 600
tctctttgta aacatctcaa gaagatgcgg ctccatagca aaggatctca agatcccagc 660
accaaggtcc atataaaagc tttgcaaact gtgacctcct tcctcatgtt atttgccatt 720
tactttctgt gtataatcac atcaacttgg aatcttagga cacagcagag caaacttgta 780
ctcctgcttt gccaaactgt tgcaatcatg tatccttcat tccactcatt catcctgatt 840
atgggaagta ggaagctaaa acagaccttt ctttcagttt tgtggcagat gacacgctga 900

<210> 72
<211> 930
<212> DNA
<213> Homo sapiens

<220>
<223> human TAS2R49 (F21) coding sequence

<400> 72

atgatgagtt ttctacacat tgtttttttcc attctagtag tggttgcatt tattcttgga 60
aattttgcca atggctttat agcactgata aatttcattg cctgggtcaa gagacaaaag 120
atctcctcag ctgatcaaat tattgctgct ctggcagtct ccagagttgg tttgctctgg 180
gtaatattat tacattggta ttcaactgtg ttgaatccaa cttcatctaa tttaaaagta 240
ataattttta tttctaatgc ctgggcagta accaatcatt tcagcatctg gcttgctact 300
agcctcagca tattttattt gctcaagatc gtcaatttct ccagacttat ttttcatcac 360
ttaaaaagga aggctaagag tgtagttctg gtgatagtgt tggggtcttt gttcttttg 420
gtttgtcacc ttgtgatgaa acacacgtat ataaatgtgt ggacagaaga atgtgaagga 480
aacgtaactt ggaagatcaa actgaggaat gcaatgcacc tttccaactt gactgtagcc 540
atgctagcaa acttgatacc attcactctg accctgatat cttttctgct gttaatctac 600
tctctgtgta aacatctgaa gaagatgcag ctccatggca aaggatctca agatcccagc 660
accaagatcc acataaaagc tctgcaaact gtgacctcct tcctcatatt acttgccatt 720
tactttctgt gtctaatcat atcgttttgg aattttaaga tgcgaccaaa agaaattgtc 780
ttaatgcttt gccaagcttt tggaatcata tatccatcat tccactcatt cattctgatt 840
tgggggaaca agacgctaaa gcagaccttt ctttcagttt tgtggcaggt gacttgctgg 900
gcaaaaggac agaaccagtc aactccatag 930

<210> 73
<211> 900
<212> DNA
<213> Homo sapiens

<220>
<223> human TAS2R50 (F10) coding sequence

<400> 73

```
atgataactt ttctatacat tttttttca attctaataa tggttttatt tgttctcgga 60
aactttgcca atggcttcat agcactggta aatttcattg actgggtgaa gagaaaaaag 120
atctcctcag ctgaccaaat tctcactgct ctggcggtct ccagaattgg tttgctctgg 180
gcattattat taaattggta tttaactgtg ttgaatccag ctttttatag tgtagaatta 240
agaattactt cttataatgc ctgggttgta accaaccatt tcagcatgtg gcttgctgct 300
aacctcagca tattttattt gctcaagatt gccaatttct ccaaccttct ttttcttcat 360
ttaaagagga gagttaggag tgtcattctg gtgatactgt tggggacttt gatatttttg 420
gtttgtcatc ttcttgtggc aaacatggat gagagtatgt gggcagaaga atatgaagga 480
aacatgactg ggaagatgaa attgaggaat acagtacatc tttcatattt gactgtaact 540
accctatgga gcttcatacc ctttactctg tccctgatat cttttctgat gctaatctgt 600
tctctgtgta aacatctcaa gaagatgcag ctccatggag aaggatcgca agatctcagc 660
accaaggtcc acataaaagc tttgcaaact ctgatctcct tcctcttgtt atgtgccatt 720
ttctttctat tcctaatcgt ttcggtttgg agtcctagga ggctgcggaa tgacccggtt 780
gtcatggtta gcaaggctgt tggaaacata tatcttgcat tcgactcatt catcctaatt 840
tggagaacca agaagctaaa acacaccttt cttttgattt tgtgtcagat taggtgctga 900
```

<210> 74
<211> 945
<212> DNA
<213> Homo sapiens

<220>
<223> human TAS2R55 (F13) coding sequence

<400> 74

```
atggccaccg aattggacaa aatctttctg attctggcaa tagcagaatt catcatcagc 60
atgctgggga atgtgttcat tggactggta aactgctctg aagggatcaa gaaccaaaag 120
gtcttctcag ctgacttcat cctcacctgc ttggctatct ccacaattgg acaactgttg 180
gtgatactgt ttgattcatt tctagtggga cttgcttcac atttatatac cacatataga 240
ctaggaaaaa ctgttattat gctttggcac atgactaatc acttgacaac ctggcttgcc 300
acctgcctaa gcattttcta tttctttaag atagcccact tcccccactc cctttcctc 360
tggctgaggt ggaggatgaa cggaatgatt gttatgcttc ttatattgtc tttgttctta 420
ctgattttg acagtttagt gctagaaata tttattgata tctcactcaa tataatagat 480
aaaagtaatc tgactttata tttagatgaa agtaaaactc tctttgataa actctctatt 540
ttaaaaactc ttctcagctt gaccagtttt atcccctttt ctctgtccct gacctccttg 600
ctttttttat ttctgtcctt ggtgagacat actagaaatt tgaagctcag ttccttgggc 660
tctagagact ccagcacaga ggcccatagg agggccatga aaatggtgat gtctttcctt 720
ttcctcttca tagttcattt tttttcctta caagtggcca attggatatt ttttatgttg 780
tggaacaaca agtacataaa gtttgtcatg ttagccttaa atgcctttcc ctcgtgccac 840
tcatttattc tcattctggg aaacagcaag ctgcgacaga cagctgtgag gctactgtgg 900
catcttagga actatacaaa aacaccaaat gctttacctt tgtga 945
```

<210> 75
<211> 957
<212> DNA
<213> Homo sapiens

<220>
<223> human TAS2R60 (F20) coding sequence

<400> 75

```
atgaatggag accacatggt tctaggatct tcggtgactg acaagaaggc catcatcttg 60
gttaccattt tactcctttt acgcctggta gcaatagcag gcaatggctt catcactgct 120
gctctgggcg tggagtgggt gctacggaga atgttgttgc cttgtgataa gttattggtt 180
agcctagggg cctctcgctt ctgtctgcag tcagtggtaa tgggtaagac catttatgtt 240
ttcttgcatc cgatggcctt cccatacaac cctgtactgc agtttctagc tttccagtgg 300
gacttcctga atgctgccac cttatggtcc tctacctggc tcagtgtctt ctattgtgtg 360
aaaattgcta ccttcaccca ccctgtcttc ttctggctaa agcacaagtt gtctgggtgg 420
ctaccatgga tgctcttcag ctctgtaggg ctctccagct tcaccaccat tctatttttc 480
ataggcaacc acagaatgta tcagaactat ttaaggaacc atctacaacc ttggaatgtc 540
actggcgata gcatacggag ctactgtgag aaattctatc tcttccctct aaaaatgatt 600
acttggacaa tgcccactgc tgtctttttc atttgcatga ttttgctcat cacatctctg 660
ggaagacaca ggaagaaggc tctccttaca acctcaggat tccgagagcc cagtgtgcag 720
gcacacataa aggctctgct ggctctcctc tcttttgcca tgctcttcat ctcatatttc 780
ctgtcactgg tgttcagtgc tgcaggtatt tttccacctc tggactttaa attctgggtg 840
tgggagtcag tgatttatct gtgtgcagca gttcacccca tcattctgct cttcagcaac 900
tgcaggctga gagctgtgct gaagagtcgt cgttcctcaa ggtgtgggac accttga    957
```

<210> 76
<211> 1125
<212> DNA
<213> Homo sapiens

<220>
<223> human GNA15 coding sequence

<400> 76

```
atggcccggt ccctgacttg ggctgctgt ccctggtgcc tgacagagga ggagaagact 60
gccgccagaa tcgaccagga gatcaacagg attttgttgg aacagaaaaa acaagagcgc 120
gaggaattga aactcctgct gttggggcct ggtgagagcg ggaagagtac gttcatcaag 180
cagatgcgca tcattcacgg tgtgggctac tcggaggagg accgcagagc cttccggctg 240
ctcatctacc agaacatctt cgtctccatg caggccatga tagatgcgat ggaccggctg 300
cagatcccct tcagcaggcc tgacagcaag cagcacgcca gcctagtgat gacccaggac 360
ccctataaag tgagcacatt cgagaagcca tatgcagtgg ccatgcagta cctgtggcgg 420
gacgcgggca tccgtgcatg ctacgagcga aggcgtgaat tccaccttct ggactccgcg 480

gtgtattacc tgtcacacct ggagcgcata tcagaggaca gctacatccc cactgcgcaa 540
gacgtgctgc gcagtcgcat gcccaccaca ggcatcaatg agtactgctt ctccgtgaag 600
aaaaccaaac tgcgcatcgt ggatgttggt ggccagaggt cagagcgtag gaaatggatt 660
cactgtttcg agaacgtgat tgccctcatc tacctggcct ccctgagcga gtatgaccag 720
tgcctagagg agaacgatca ggagaaccgc atggaggaga gtctcgctct gttcagcacg 780
atcctagagc tgccctggtt caagagcacc tcggtcatcc tcttcctcaa caagacggac 840
atcctggaag ataagattca cacctcccac ctggccacat acttccccag cttccaggga 900
ccccggcgag acgcagaggc cgccaagagc ttcatcttgg acatgtatgc gcgcgtgtac 960
gcgagctgcg cagagcccca ggacggtggc aggaaaggct cccgcgcgcg ccgcttcttc 1020
gcacacttca cctgtgccac ggacacgcaa agcgtccgca gcgtgttcaa ggacgtgcgg 1080
gactcggtgc tggcccggta cctggacgag atcaacctgc tgtga                1125
```

<210> 77
<211> 299
<212> PRT
<213> Homo sapiens

<220>
<223> human TAS2R1 (F1)

<400> 77

```
Met Leu Glu Ser His Leu Ile Ile Tyr Phe Leu Leu Ala Val Ile Gln
 1               5                  10                  15
Phe Leu Leu Gly Ile Phe Thr Asn Gly Ile Ile Val Val Val Asn Gly
            20                  25                  30
Ile Asp Leu Ile Lys His Arg Lys Met Ala Pro Leu Asp Leu Leu Leu
        35                  40                  45
Ser Cys Leu Ala Val Ser Arg Ile Phe Leu Gln Leu Phe Ile Phe Tyr
    50                  55                  60
Val Asn Val Ile Val Ile Phe Phe Ile Glu Phe Ile Met Cys Ser Ala
65                  70                  75                  80
Asn Cys Ala Ile Leu Leu Phe Ile Asn Glu Leu Glu Leu Trp Leu Ala
                85                  90                  95
Thr Trp Leu Gly Val Phe Tyr Cys Ala Lys Val Ala Ser Val Arg His
            100                 105                 110
Pro Leu Phe Ile Trp Leu Lys Met Arg Ile Ser Lys Leu Val Pro Trp
        115                 120                 125
Met Ile Leu Gly Ser Leu Leu Tyr Val Ser Met Ile Cys Val Phe His
    130                 135                 140
Ser Lys Tyr Ala Gly Phe Met Val Pro Tyr Phe Leu Arg Lys Phe Phe
145                 150                 155                 160
Ser Gln Asn Ala Thr Ile Gln Lys Glu Asp Thr Leu Ala Ile Gln Ile
                165                 170                 175
Phe Ser Phe Val Ala Glu Phe Ser Val Pro Leu Leu Ile Phe Leu Phe
            180                 185                 190
Ala Val Leu Leu Leu Ile Phe Ser Leu Gly Arg His Thr Arg Gln Met
        195                 200                 205
Arg Asn Thr Val Ala Gly Ser Arg Val Pro Gly Arg Gly Ala Pro Ile
    210                 215                 220
Ser Ala Leu Leu Ser Ile Leu Ser Phe Leu Ile Leu Tyr Phe Ser His
225                 230                 235                 240
Cys Met Ile Lys Val Phe Leu Ser Ser Leu Lys Phe His Ile Arg Arg
                245                 250                 255
Phe Ile Phe Leu Phe Phe Ile Leu Val Ile Gly Ile Tyr Pro Ser Gly
            260                 265                 270
His Ser Leu Ile Leu Ile Leu Gly Asn Pro Lys Leu Lys Gln Asn Ala
        275                 280                 285
Lys Lys Phe Leu Leu His Ser Lys Cys Cys Gln
    290                 295
```

<210> 78
<211> 316
<212> PRT
<213> Homo sapiens

<220>
<223> Human TAS2R3 (F5)

<400> 78

```
Met Met Gly Leu Thr Glu Gly Val Phe Leu Ile Leu Ser Gly Thr Gln
 1               5                  10                  15
Phe Thr Leu Gly Ile Leu Val Asn Cys Phe Ile Glu Leu Val Asn Gly
            20                  25                  30
Ser Ser Trp Phe Lys Thr Lys Arg Met Ser Leu Ser Asp Phe Ile Ile
        35                  40                  45
Thr Thr Leu Ala Leu Leu Arg Ile Ile Leu Leu Cys Ile Ile Leu Thr
    50                  55                  60
Asp Ser Phe Leu Ile Glu Phe Ser Pro Asn Thr His Asp Ser Gly Ile
65                  70                  75                  80
Ile Met Gln Ile Ile Asp Val Ser Trp Thr Phe Thr Asn His Leu Ser
                85                  90                  95
Ile Trp Leu Ala Thr Cys Leu Gly Val Leu Tyr Cys Leu Lys Ile Ala
            100                 105                 110
Ser Phe Ser His Pro Thr Phe Leu Trp Leu Lys Trp Arg Val Ser Arg
        115                 120                 125
Val Met Val Trp Met Leu Leu Gly Ala Leu Leu Leu Ser Cys Gly Ser
    130                 135                 140
Thr Ala Ser Leu Ile Asn Glu Phe Lys Leu Tyr Ser Val Phe Arg Gly
145                 150                 155                 160
Ile Glu Ala Thr Arg Asn Val Thr Glu His Phe Arg Lys Lys Arg Ser
                165                 170                 175
Glu Tyr Tyr Leu Ile His Val Leu Gly Thr Leu Trp Tyr Leu Pro Pro
            180                 185                 190
Leu Ile Val Ser Leu Ala Ser Tyr Ser Leu Leu Ile Phe Ser Leu Gly
        195                 200                 205
Arg His Thr Arg Gln Met Leu Gln Asn Gly Thr Ser Ser Arg Asp Pro
    210                 215                 220
Thr Thr Glu Ala His Lys Arg Ala Ile Arg Ile Ile Leu Ser Phe Phe
225                 230                 235                 240
Phe Leu Phe Leu Leu Tyr Phe Leu Ala Phe Leu Ile Ala Ser Phe Gly
                245                 250                 255
Asn Phe Leu Pro Lys Thr Lys Met Ala Lys Met Ile Gly Glu Val Met
            260                 265                 270
Thr Met Phe Tyr Pro Ala Gly His Ser Phe Ile Leu Ile Leu Gly Asn
        275                 280                 285
Ser Lys Leu Lys Gln Thr Phe Val Val Met Leu Arg Cys Glu Ser Gly
    290                 295                 300
His Leu Lys Pro Gly Ser Lys Gly Pro Ile Phe Ser
305                 310                 315
```

<210> 79
<211> 299
<212> PRT
<213> Homo sapiens

<220>
<223> Human TAS2R4 (F25)

<400> 79

```
Met Leu Arg Leu Phe Tyr Phe Ser Ala Ile Ile Ala Ser Val Ile Leu
 1               5                  10                  15
Asn Phe Val Gly Ile Ile Met Asn Leu Phe Ile Thr Val Val Asn Cys
            20                  25                  30
Lys Thr Trp Val Lys Ser His Arg Ile Ser Ser Ser Asp Arg Ile Leu
        35                  40                  45
Phe Ser Leu Gly Ile Thr Arg Phe Leu Met Leu Gly Leu Phe Leu Val
    50                  55                  60
Asn Thr Ile Tyr Phe Val Ser Ser Asn Thr Glu Arg Ser Val Tyr Leu
```

```
65                  70                  75                  80
Ser Ala Phe Phe Val Leu Cys Phe Met Phe Leu Asp Ser Ser Ser Val
                85                  90                  95
Trp Phe Val Thr Leu Leu Asn Ile Leu Tyr Cys Val Lys Ile Thr Asn
            100                 105                 110
Phe Gln His Ser Val Phe Leu Leu Leu Lys Arg Asn Ile Ser Pro Lys
        115                 120                 125
Ile Pro Arg Leu Leu Leu Ala Cys Val Leu Ile Ser Ala Phe Thr Thr
    130                 135                 140
Cys Leu Tyr Ile Thr Leu Ser Gln Ala Ser Pro Phe Pro Glu Leu Val
145                 150                 155                 160
Thr Thr Arg Asn Asn Thr Ser Phe Asn Ile Ser Glu Gly Ile Leu Ser
                165                 170                 175
Leu Val Val Ser Leu Val Leu Ser Ser Ser Leu Gln Phe Ile Ile Asn
            180                 185                 190
Val Thr Ser Ala Ser Leu Leu Ile His Ser Leu Arg Arg His Ile Gln
        195                 200                 205
Lys Met Gln Lys Asn Ala Thr Gly Phe Trp Asn Pro Gln Thr Glu Ala
    210                 215                 220
His Val Gly Ala Met Lys Leu Met Val Tyr Phe Leu Ile Leu Tyr Ile
225                 230                 235                 240
Pro Tyr Ser Val Ala Thr Leu Val Gln Tyr Leu Pro Phe Tyr Ala Gly
                245                 250                 255
Met Asp Met Gly Thr Lys Ser Ile Cys Leu Ile Phe Ala Thr Leu Tyr
            260                 265                 270
Ser Pro Gly His Ser Val Leu Ile Ile Ile Thr His Pro Lys Leu Lys
        275                 280                 285
Thr Thr Ala Lys Lys Ile Leu Cys Phe Lys Lys
    290                 295
```

<210> 80
<211> 299
<212> PRT
<213> Homo sapiens

<220>
<223> Human TAS2R5 (F11)

<400> 80

```
Met Leu Ser Ala Gly Leu Gly Leu Leu Met Leu Val Ala Val Val Glu
 1               5                  10                  15
Phe Leu Ile Gly Leu Ile Gly Asn Gly Ser Leu Val Val Trp Ser Phe
            20                  25                  30
Arg Glu Trp Ile Arg Lys Phe Asn Trp Ser Ser Tyr Asn Leu Ile Ile
        35                  40                  45
Leu Gly Leu Ala Gly Cys Arg Phe Leu Leu Gln Trp Leu Ile Ile Leu
    50                  55                  60
Asp Leu Ser Leu Phe Pro Leu Phe Gln Ser Ser Arg Trp Leu Arg Tyr
65                  70                  75                  80
Leu Ser Ile Phe Trp Val Leu Val Ser Gln Ala Ser Leu Trp Phe Ala
                85                  90                  95
Thr Phe Leu Ser Val Phe Tyr Cys Lys Lys Ile Thr Thr Phe Asp Arg
            100                 105                 110
Pro Ala Tyr Leu Trp Leu Lys Gln Arg Ala Tyr Asn Leu Ser Leu Trp
        115                 120                 125
Cys Leu Leu Gly Tyr Phe Ile Ile Asn Leu Leu Leu Thr Val Gln Ile
    130                 135                 140
Gly Leu Thr Phe Tyr His Pro Pro Gln Gly Asn Ser Ser Ile Arg Tyr
145                 150                 155                 160
Pro Phe Glu Ser Trp Gln Tyr Leu Tyr Ala Phe Gln Leu Asn Ser Gly
                165                 170                 175
Ser Tyr Leu Pro Leu Val Val Phe Leu Val Ser Ser Gly Met Leu Ile
            180                 185                 190
Val Ser Leu Tyr Thr His His Lys Lys Met Lys Val His Ser Ala Gly

        195                 200                 205
Arg Arg Asp Val Arg Ala Lys Ala His Ile Thr Ala Leu Lys Ser Leu
    210                 215                 220
Gly Cys Phe Leu Leu Leu His Leu Val Tyr Ile Met Ala Ser Pro Phe
225                 230                 235                 240
Ser Ile Thr Ser Lys Thr Tyr Pro Pro Asp Leu Thr Ser Val Phe Ile
                245                 250                 255
Trp Glu Thr Leu Met Ala Ala Tyr Pro Ser Leu His Ser Leu Ile Leu
            260                 265                 270
Ile Met Gly Ile Pro Arg Val Lys Gln Thr Cys Gln Lys Ile Leu Trp
        275                 280                 285
Lys Thr Val Cys Ala Arg Arg Cys Trp Gly Pro
    290                 295
```

<210> 81
<211> 318
<212> PRT
<213> Homo sapiens

<220>
<223> Human TAS2R7 (F4)

<400> 81

Met Ala Asp Lys Val Gln Thr Thr Leu Leu Phe Leu Ala Val Gly Glu
1               5                   10                  15
Phe Ser Val Gly Ile Leu Gly Asn Ala Phe Ile Gly Leu Val Asn Cys
            20                  25                  30
Met Asp Trp Val Lys Lys Arg Lys Ile Ala Ser Ile Asp Leu Ile Leu
        35                  40                  45
Thr Ser Leu Ala Ile Ser Arg Ile Cys Leu Leu Cys Val Ile Leu Leu
    50                  55                  60
Asp Cys Phe Ile Leu Val Leu Tyr Pro Asp Val Tyr Ala Thr Gly Lys
65                  70                  75                  80
Glu Met Arg Ile Ile Asp Phe Phe Trp Thr Leu Thr Asn His Leu Ser
                85                  90                  95
Ile Trp Phe Ala Thr Cys Leu Ser Ile Tyr Tyr Phe Phe Lys Ile Gly
            100                 105                 110
Asn Phe Phe His Pro Leu Phe Leu Trp Met Lys Trp Arg Ile Asp Arg
        115                 120                 125
Val Ile Ser Trp Ile Leu Leu Gly Cys Val Val Leu Ser Val Phe Ile
    130                 135                 140
Ser Leu Pro Ala Thr Glu Asn Leu Asn Ala Asp Phe Arg Phe Cys Val
145                 150                 155                 160
Lys Ala Lys Arg Lys Thr Asn Leu Thr Trp Ser Cys Arg Val Asn Lys
                165                 170                 175
Thr Gln His Ala Ser Thr Lys Leu Phe Leu Asn Leu Ala Thr Leu Leu
            180                 185                 190
Pro Phe Cys Val Cys Leu Met Ser Phe Phe Leu Leu Ile Leu Ser Leu
        195                 200                 205
Arg Arg His Ile Arg Arg Met Gln Leu Ser Ala Thr Gly Cys Arg Asp
    210                 215                 220
Pro Ser Thr Glu Ala His Val Arg Ala Leu Lys Ala Val Ile Ser Phe
225                 230                 235                 240
Leu Leu Leu Phe Ile Ala Tyr Tyr Leu Ser Phe Leu Ile Ala Thr Ser
                245                 250                 255
Ser Tyr Phe Met Pro Glu Thr Glu Leu Ala Val Ile Phe Gly Glu Ser
            260                 265                 270
Ile Ala Leu Ile Tyr Pro Ser Ser His Ser Phe Ile Leu Ile Leu Gly
        275                 280                 285
Asn Asn Lys Leu Arg His Ala Ser Leu Lys Val Ile Trp Lys Val Met
    290                 295                 300
Ser Ile Leu Lys Gly Arg Lys Phe Gln Gln His Lys Gln Ile
305                 310                 315

<210> 82
<211> 309
<212> PRT
<213> Homo sapiens

<220>
<223> Human TAS2R8 (F24)

<400> 82

Met Phe Ser Pro Ala Asp Asn Ile Phe Ile Ile Leu Ile Thr Gly Glu
1 5 10 15
Phe Ile Leu Gly Ile Leu Gly Asn Gly Tyr Ile Ala Leu Val Asn Trp
20 25 30
Ile Asp Trp Ile Lys Lys Lys Lys Ile Ser Thr Val Asp Tyr Ile Leu
35 40 45
Thr Asn Leu Val Ile Ala Arg Ile Cys Leu Ile Ser Val Met Val Val
50 55 60
Asn Gly Ile Val Ile Val Leu Asn Pro Asp Val Tyr Thr Lys Asn Lys
65 70 75 80
Gln Gln Ile Val Ile Phe Thr Phe Trp Thr Phe Ala Asn Tyr Leu Asn
85 90 95
Met Trp Ile Thr Thr Cys Leu Asn Val Phe Tyr Phe Leu Lys Ile Ala
100 105 110
Ser Ser Ser His Pro Leu Phe Leu Trp Leu Lys Trp Lys Ile Asp Met
115 120 125
Val Val His Trp Ile Leu Leu Gly Cys Phe Ala Ile Ser Leu Leu Val
130 135 140
Ser Leu Ile Ala Ala Ile Val Leu Ser Cys Asp Tyr Arg Phe His Ala
145 150 155 160
Ile Ala Lys His Lys Arg Asn Ile Thr Glu Met Phe His Val Ser Lys
165 170 175
Ile Pro Tyr Phe Glu Pro Leu Thr Leu Phe Asn Leu Phe Ala Ile Val
180 185 190
Pro Phe Ile Val Ser Leu Ile Ser Phe Phe Leu Leu Val Arg Ser Leu
195 200 205
Trp Arg His Thr Lys Gln Ile Lys Leu Tyr Ala Thr Gly Ser Arg Asp
210 215 220
Pro Ser Thr Glu Val His Val Arg Ala Ile Lys Thr Met Thr Ser Phe
225 230 235 240
Ile Phe Phe Phe Phe Leu Tyr Tyr Ile Ser Ser Ile Leu Met Thr Phe
245 250 255
Ser Tyr Leu Met Thr Lys Tyr Lys Leu Ala Val Glu Phe Gly Glu Ile
260 265 270
Ala Ala Ile Leu Tyr Pro Leu Gly His Ser Leu Ile Leu Ile Val Leu
275 280 285
Asn Asn Lys Leu Arg Gln Thr Phe Val Arg Met Leu Thr Cys Arg Lys
290 295 300
Ile Ala Cys Met Ile
305

<210> 83
<211> 312
<212> PRT
<213> Homo sapiens

<220>
<223> Human TAS2R9 (F24)

<400> 83

Met Pro Ser Ala Ile Glu Ala Ile Tyr Ile Ile Leu Ile Ala Gly Glu
1 5 10 15
Leu Thr Ile Gly Ile Trp Gly Asn Gly Phe Ile Val Leu Val Asn Cys
20 25 30

```
Ile Asp Trp Leu Lys Arg Arg Asp Ile Ser Leu Ile Asp Ile Ile Leu
        35                  40                  45
Ile Ser Leu Ala Ile Ser Arg Ile Cys Leu Leu Cys Val Ile Ser Leu
    50                  55                  60
Asp Gly Phe Phe Met Leu Leu Phe Pro Gly Thr Tyr Gly Asn Ser Val
65                  70                  75                  80
Leu Val Ser Ile Val Asn Val Val Trp Thr Phe Ala Asn Asn Ser Ser
                85                  90                  95
Leu Trp Phe Thr Ser Cys Leu Ser Ile Phe Tyr Leu Leu Lys Ile Ala
            100                 105                 110
Asn Ile Ser His Pro Phe Phe Phe Trp Leu Lys Leu Lys Ile Asn Lys
        115                 120                 125
Val Met Leu Ala Ile Leu Leu Gly Ser Phe Leu Ile Ser Leu Ile Ile
    130                 135                 140
Ser Val Pro Lys Asn Asp Asp Met Trp Tyr His Leu Phe Lys Val Ser
145                 150                 155                 160
His Glu Glu Asn Ile Thr Trp Lys Phe Lys Val Ser Lys Ile Pro Gly
                165                 170                 175
Thr Phe Lys Gln Leu Thr Leu Asn Leu Gly Val Met Val Pro Phe Ile
            180                 185                 190
Leu Cys Leu Ile Ser Phe Phe Leu Leu Leu Phe Ser Leu Val Arg His
        195                 200                 205
Thr Lys Gln Ile Arg Leu His Ala Thr Gly Phe Arg Asp Pro Ser Thr
    210                 215                 220
Glu Ala His Met Arg Ala Ile Lys Ala Val Ile Ile Phe Leu Leu Leu
225                 230                 235                 240
Leu Ile Val Tyr Tyr Pro Val Phe Leu Val Met Thr Ser Ser Ala Leu
                245                 250                 255
Ile Pro Gln Gly Lys Leu Val Leu Met Ile Gly Asp Ile Val Thr Val
            260                 265                 270
Ile Phe Pro Ser Ser His Ser Phe Ile Leu Ile Met Gly Asn Ser Lys
        275                 280                 285
Leu Arg Glu Ala Phe Leu Lys Met Leu Arg Phe Val Lys Cys Phe Leu
    290                 295                 300
Arg Arg Arg Lys Pro Phe Val Pro
305                 310
```

<210> 84
<211> 300
<212> PRT
<213> Homo sapiens

<220>
<223> Human TAS2R10 (F16)

<400> 84

```
Met Leu Arg Val Val Glu Gly Ile Phe Ile Phe Val Val Val Ser Glu
 1               5                  10                  15
Ser Val Phe Gly Val Leu Gly Asn Gly Phe Ile Gly Leu Val Asn Cys
            20                  25                  30
Ile Asp Cys Ala Lys Asn Lys Leu Ser Thr Ile Gly Phe Ile Leu Thr
        35                  40                  45
Gly Leu Ala Ile Ser Arg Ile Phe Leu Ile Trp Ile Ile Ile Thr Asp
    50                  55                  60
Gly Phe Ile Gln Ile Phe Ser Pro Asn Ile Tyr Ala Ser Gly Asn Leu
65                  70                  75                  80
Ile Glu Tyr Ile Ser Tyr Phe Trp Val Ile Gly Asn Gln Ser Ser Met
                85                  90                  95
Trp Phe Ala Thr Ser Leu Ser Ile Phe Tyr Phe Leu Lys Ile Ala Asn
            100                 105                 110
Phe Ser Asn Tyr Ile Phe Leu Trp Leu Lys Ser Arg Thr Asn Met Val
        115                 120                 125
Leu Pro Phe Met Ile Val Phe Leu Leu Ile Ser Ser Leu Leu Asn Phe
    130                 135                 140

Ala Tyr Ile Ala Lys Ile Leu Asn Asp Tyr Lys Met Lys Asn Asp Thr
145                 150                 155                 160
Val Trp Asp Leu Asn Met Tyr Lys Ser Glu Tyr Phe Ile Lys Gln Ile
                165                 170                 175
Leu Leu Asn Leu Gly Val Ile Phe Phe Phe Thr Leu Ser Leu Ile Thr
            180                 185                 190
Cys Ile Phe Leu Ile Ile Ser Leu Trp Arg His Asn Arg Gln Met Gln
        195                 200                 205
Ser Asn Val Thr Gly Leu Arg Asp Ser Asn Thr Glu Ala His Val Lys
    210                 215                 220
Ala Met Lys Val Leu Ile Ser Phe Ile Ile Leu Phe Ile Leu Tyr Phe
225                 230                 235                 240
Ile Gly Met Ala Ile Glu Ile Ser Cys Phe Thr Val Arg Glu Asn Lys
                245                 250                 255
Leu Leu Leu Met Phe Gly Met Thr Thr Thr Ala Ile Tyr Pro Trp Gly
            260                 265                 270
His Ser Phe Ile Leu Ile Leu Gly Asn Ser Lys Leu Lys Gln Ala Ser
        275                 280                 285
Leu Arg Val Leu Gln Gln Leu Lys Cys Cys Glu Lys
    290                 295                 300
```

<210> 85
<211> 300
<212> PRT
<213> Homo sapiens

<220>
<223> Human TAS2R13 (F3)

<400> 85

```
Met Glu Ser Ala Leu Pro Ser Ile Phe Thr Leu Val Ile Ile Ala Glu
 1               5                  10                  15
Phe Ile Ile Gly Asn Leu Ser Asn Gly Phe Ile Val Leu Ile Asn Cys
            20                  25                  30
Ile Asp Trp Val Ser Lys Arg Glu Leu Ser Ser Val Asp Lys Leu Leu
        35                  40                  45
Ile Ile Leu Ala Ile Ser Arg Ile Gly Leu Ile Trp Glu Ile Leu Val
    50                  55                  60
Ser Trp Phe Leu Ala Leu His Tyr Leu Ala Ile Phe Val Ser Gly Thr
65                  70                  75                  80
Gly Leu Arg Ile Met Ile Phe Ser Trp Ile Val Ser Asn His Phe Asn
                85                  90                  95
Leu Trp Leu Ala Thr Ile Phe Ser Ile Phe Tyr Leu Leu Lys Ile Ala
            100                 105                 110
Ser Phe Ser Ser Pro Ala Phe Leu Tyr Leu Lys Trp Arg Val Asn Lys
        115                 120                 125
Val Ile Leu Met Ile Leu Leu Gly Thr Leu Val Phe Leu Phe Leu Asn
    130                 135                 140
Leu Ile Gln Ile Asn Met His Ile Lys Asp Trp Leu Asp Arg Tyr Glu
145                 150                 155                 160
Arg Asn Thr Thr Trp Asn Phe Ser Met Ser Asp Phe Glu Thr Phe Ser
                165                 170                 175
Val Ser Val Lys Phe Thr Met Thr Met Phe Ser Leu Thr Pro Phe Thr
            180                 185                 190
Val Ala Phe Ile Ser Phe Leu Leu Leu Ile Phe Ser Leu Gln Lys His
        195                 200                 205
Leu Gln Lys Met Gln Leu Asn Tyr Lys Gly His Arg Asp Pro Arg Thr
    210                 215                 220
Lys Val His Thr Asn Ala Leu Lys Ile Val Ile Ser Phe Leu Leu Phe
225                 230                 235                 240
Tyr Ala Ser Phe Phe Leu Cys Val Leu Ile Ser Trp Ile Ser Glu Leu
                245                 250                 255
Tyr Gln Asn Thr Val Ile Tyr Met Leu Cys Glu Thr Ile Gly Val Phe
            260                 265                 270

Ser Pro Ser Ser His Ser Phe Leu Leu Ile Leu Gly Asn Ala Lys Leu
        275                 280                 285
Arg Gln Ala Phe Leu Leu Val Ala Ala Lys Val Trp
    290                 295                 300
```

<210> 86
<211> 317
<212> PRT
<213> Homo sapiens

<220>
<223> Human TAS2R14 (F15)

<400> 86

```
Met Gly Gly Val Ile Lys Ser Ile Phe Thr Phe Val Leu Ile Val Glu
 1               5                   10                  15
Phe Ile Ile Gly Asn Leu Gly Asn Ser Phe Ile Ala Leu Val Asn Cys
            20                  25                  30
Ile Asp Trp Val Lys Gly Arg Lys Ile Ser Ser Val Asp Arg Ile Leu
        35                  40                  45
Thr Ala Leu Ala Ile Ser Arg Ile Ser Leu Val Trp Leu Ile Phe Gly
    50                  55                  60
Ser Trp Cys Val Ser Val Phe Phe Pro Ala Leu Phe Ala Thr Glu Lys
65                  70                  75                  80
Met Phe Arg Met Leu Thr Asn Ile Trp Thr Val Ile Asn His Phe Ser
                85                  90                  95
Val Trp Leu Ala Thr Gly Leu Gly Thr Phe Tyr Phe Leu Lys Ile Ala
            100                 105                 110
Asn Phe Ser Asn Ser Ile Phe Leu Tyr Leu Lys Trp Arg Val Lys Lys
        115                 120                 125
Val Val Leu Val Leu Leu Leu Val Thr Ser Val Phe Leu Phe Leu Asn
    130                 135                 140
Ile Ala Leu Ile Asn Ile His Ile Asn Ala Ser Ile Asn Gly Tyr Arg
145                 150                 155                 160
Arg Asn Lys Thr Cys Ser Ser Asp Ser Ser Asn Phe Thr Arg Phe Ser
                165                 170                 175
Ser Leu Ile Val Leu Thr Ser Thr Val Phe Ile Phe Ile Pro Phe Thr
            180                 185                 190
Leu Ser Leu Ala Met Phe Leu Leu Leu Ile Phe Ser Met Trp Lys His
        195                 200                 205
Arg Lys Lys Met Gln His Thr Val Lys Ile Ser Gly Asp Ala Ser Thr
    210                 215                 220
Lys Ala His Arg Gly Val Lys Ser Val Ile Thr Phe Phe Leu Leu Tyr
225                 230                 235                 240
Ala Ile Phe Ser Leu Ser Phe Phe Ile Ser Val Trp Thr Ser Glu Arg
                245                 250                 255
Leu Glu Glu Asn Leu Ile Ile Leu Ser Gln Val Met Gly Met Ala Tyr
            260                 265                 270
Pro Ser Cys His Ser Cys Val Leu Ile Leu Gly Asn Lys Lys Leu Arg
        275                 280                 285
Gln Ala Ser Leu Ser Val Leu Leu Trp Leu Arg Tyr Met Phe Lys Asp
    290                 295                 300
Gly Glu Pro Ser Gly His Lys Glu Phe Arg Glu Ser Ser
305                 310                 315
```

<210> 87
<211> 291
<212> PRT
<213> Homo sapiens

<220>
<223> Human TAS2R16 (F14)

<400> 87

```
Met Ile Pro Ile Gln Leu Thr Val Phe Phe Met Ile Ile Tyr Val Leu
 1               5                  10                  15
Glu Ser Leu Thr Ile Ile Val Gln Ser Ser Leu Ile Val Ala Val Leu
            20                  25                  30
Gly Arg Glu Trp Leu Gln Val Arg Arg Leu Met Pro Val Asp Met Ile
        35                  40                  45
Leu Ile Ser Leu Gly Ile Ser Arg Phe Cys Leu Gln Trp Ala Ser Met
    50                  55                  60
Leu Asn Asn Phe Cys Ser Tyr Phe Asn Leu Asn Tyr Val Leu Cys Asn
65                  70                  75                  80
Leu Thr Ile Thr Trp Glu Phe Phe Asn Ile Leu Thr Phe Trp Leu Asn
                85                  90                  95
Ser Leu Leu Thr Val Phe Tyr Cys Ile Lys Val Ser Ser Phe Thr His
            100                 105                 110
His Ile Phe Leu Trp Leu Arg Trp Arg Ile Leu Arg Leu Phe Pro Trp
        115                 120                 125
Ile Leu Leu Gly Ser Leu Met Ile Thr Cys Val Thr Ile Ile Pro Ser
    130                 135                 140
Ala Ile Gly Asn Tyr Ile Gln Ile Gln Leu Leu Thr Met Glu His Leu
145                 150                 155                 160
Pro Arg Asn Ser Thr Val Thr Asp Lys Leu Glu Asn Phe His Gln Tyr
                165                 170                 175
Gln Phe Gln Ala His Thr Val Ala Leu Val Ile Pro Phe Ile Leu Phe
            180                 185                 190
Leu Ala Ser Thr Ile Phe Leu Met Ala Ser Leu Thr Lys Gln Ile Gln
        195                 200                 205
His His Ser Thr Gly His Cys Asn Pro Ser Met Lys Ala Arg Phe Thr
    210                 215                 220
Ala Leu Arg Ser Leu Ala Val Leu Phe Ile Val Phe Thr Ser Tyr Phe
225                 230                 235                 240
Leu Thr Ile Leu Ile Thr Ile Ile Gly Thr Leu Phe Asp Lys Arg Cys
                245                 250                 255
Trp Leu Trp Val Trp Glu Ala Phe Val Tyr Ala Phe Ile Leu Met His
            260                 265                 270
Ser Thr Ser Leu Met Leu Ser Ser Pro Thr Leu Lys Arg Ile Leu Lys
        275                 280                 285
Gly Lys Cys
    290
```

<210> 88
<211> 333
<212> PRT
<213> Homo sapiens

<220>
<223> Human TAS2R38 (F7)

<400> 88

```
Met Leu Thr Leu Thr Arg Ile Arg Thr Val Ser Tyr Glu Val Arg Ser
 1               5                  10                  15
Thr Phe Leu Phe Ile Ser Val Leu Glu Phe Ala Val Gly Phe Leu Thr
            20                  25                  30
Asn Ala Phe Val Phe Leu Val Asn Phe Trp Asp Val Val Lys Arg Gln
        35                  40                  45
Ala Leu Ser Asn Ser Asp Cys Val Leu Leu Cys Leu Ser Ile Ser Arg
    50                  55                  60
Leu Phe Leu His Gly Leu Leu Phe Leu Ser Ala Ile Gln Leu Thr His
65                  70                  75                  80
Phe Gln Lys Leu Ser Glu Pro Leu Asn His Ser Tyr Gln Ala Ile Ile
                85                  90                  95
Met Leu Trp Met Ile Ala Asn Gln Ala Asn Leu Trp Leu Ala Ala Cys
            100                 105                 110
Leu Ser Leu Leu Tyr Cys Ser Lys Leu Ile Arg Phe Ser His Thr Phe
        115                 120                 125
Leu Ile Cys Leu Ala Ser Trp Val Ser Arg Lys Ile Ser Gln Met Leu
    130                 135                 140
Leu Gly Ile Ile Leu Cys Ser Cys Ile Cys Thr Val Leu Cys Val Trp
145                 150                 155                 160
Cys Phe Phe Ser Arg Pro His Phe Thr Val Thr Thr Val Leu Phe Met
                165                 170                 175
Asn Asn Asn Thr Arg Leu Asn Trp Gln Ile Lys Asp Leu Asn Leu Phe
            180                 185                 190
Tyr Ser Phe Leu Phe Cys Tyr Leu Trp Ser Val Pro Pro Phe Leu Leu
        195                 200                 205
Phe Leu Val Ser Ser Gly Met Leu Thr Val Ser Leu Gly Arg His Met
    210                 215                 220
Arg Thr Met Lys Val Tyr Thr Arg Asn Ser Arg Asp Pro Ser Leu Glu
225                 230                 235                 240
Ala His Ile Lys Ala Leu Lys Ser Leu Val Ser Phe Phe Cys Phe Phe
                245                 250                 255
Val Ile Ser Ser Cys Ala Ala Phe Ile Ser Val Pro Leu Leu Ile Leu
            260                 265                 270
Trp Arg Asp Lys Ile Gly Val Met Val Cys Val Gly Ile Met Ala Ala
        275                 280                 285
Cys Pro Ser Gly His Ala Ala Ile Leu Ile Ser Gly Asn Ala Lys Leu
    290                 295                 300
Arg Arg Ala Val Met Thr Ile Leu Leu Trp Ala Gln Ser Ser Leu Lys
305                 310                 315                 320
Val Arg Ala Asp His Lys Ala Asp Ser Arg Thr Leu Cys
                325                 330
```

<210> 89
<211> 338
<212> PRT
<213> Homo sapiens

<220>
<223> Human TAS2R39 (F23)

<400> 89

```
Met Leu Gly Arg Cys Phe Pro Pro Asp Thr Lys Glu Lys Gln Gln Leu
1               5                   10                  15
Arg Met Thr Lys Leu Cys Asp Pro Ala Glu Ser Glu Leu Ser Pro Phe
            20                  25                  30
Leu Ile Thr Leu Ile Leu Ala Val Leu Leu Ala Glu Tyr Leu Ile Gly
        35                  40                  45
Ile Ile Ala Asn Gly Phe Ile Met Ala Ile His Ala Ala Glu Trp Val
    50                  55                  60
Gln Asn Lys Ala Val Ser Thr Ser Gly Arg Ile Leu Val Phe Leu Ser
65                  70                  75                  80
Val Ser Arg Ile Ala Leu Gln Ser Leu Met Met Leu Glu Ile Thr Ile
                85                  90                  95
Ser Ser Thr Ser Leu Ser Phe Tyr Ser Glu Asp Ala Val Tyr Tyr Ala
            100                 105                 110
Phe Lys Ile Ser Phe Ile Phe Leu Asn Phe Cys Ser Leu Trp Phe Ala
        115                 120                 125
Ala Trp Leu Ser Phe Phe Tyr Phe Val Lys Ile Ala Asn Phe Ser Tyr
    130                 135                 140
Pro Leu Phe Leu Lys Leu Arg Trp Arg Ile Thr Gly Leu Ile Pro Trp
145                 150                 155                 160
Leu Leu Trp Leu Ser Val Phe Ile Ser Phe Ser His Ser Met Phe Cys
                165                 170                 175
Ile Asn Ile Cys Thr Val Tyr Cys Asn Asn Ser Phe Pro Ile His Ser
            180                 185                 190
Ser Asn Ser Thr Lys Lys Thr Tyr Leu Ser Glu Ile Asn Val Val Gly
        195                 200                 205
Leu Ala Phe Phe Phe Asn Leu Gly Ile Val Thr Pro Leu Ile Met Phe


    210                 215                 220
Ile Leu Thr Ala Thr Leu Leu Ile Leu Ser Leu Lys Arg His Thr Leu
225                 230                 235                 240
His Met Gly Ser Asn Ala Thr Gly Ser Asn Asp Pro Ser Met Glu Ala
                245                 250                 255
His Met Gly Ala Ile Lys Ala Ile Ser Tyr Phe Leu Ile Leu Tyr Ile
            260                 265                 270
Phe Asn Ala Val Ala Leu Phe Ile Tyr Leu Ser Asn Met Phe Asp Ile
        275                 280                 285
Asn Ser Leu Trp Asn Asn Leu Cys Gln Ile Ile Met Ala Ala Tyr Pro
    290                 295                 300
Ala Ser His Ser Ile Leu Leu Ile Gln Asp Asn Pro Gly Leu Arg Arg
305                 310                 315                 320
Ala Trp Lys Arg Leu Gln Leu Arg Leu His Leu Tyr Pro Lys Glu Trp
                325                 330                 335
Thr Leu
```

<210> 90
<211> 323
<212> PRT
<213> Homo sapiens

<220>
<223> Human TAS2R40 (F19)

<400> 90

```
Met Ala Thr Val Asn Thr Asp Ala Thr Asp Lys Asp Ile Ser Lys Phe
 1               5                  10                  15
Lys Val Thr Phe Thr Leu Val Val Ser Gly Ile Glu Cys Ile Thr Gly
            20                  25                  30
Ile Leu Gly Ser Gly Phe Ile Thr Ala Ile Tyr Gly Ala Glu Trp Ala
        35                  40                  45
Arg Gly Lys Thr Leu Pro Thr Gly Asp Arg Ile Met Leu Met Leu Ser
    50                  55                  60
Phe Ser Arg Leu Leu Leu Gln Ile Trp Met Met Leu Glu Asn Ile Phe
65                  70                  75                  80
Ser Leu Leu Phe Arg Ile Val Tyr Asn Gln Asn Ser Val Tyr Ile Leu
                85                  90                  95
Phe Lys Val Ile Thr Val Phe Leu Asn His Ser Asn Leu Trp Phe Ala
            100                 105                 110
Ala Trp Leu Lys Val Phe Tyr Cys Leu Arg Ile Ala Asn Phe Asn His
        115                 120                 125
Pro Leu Phe Phe Leu Met Lys Arg Lys Ile Ile Val Leu Met Pro Trp
    130                 135                 140
Leu Leu Arg Leu Ser Val Leu Val Ser Leu Ser Phe Ser Phe Pro Leu
145                 150                 155                 160
Ser Arg Asp Val Phe Asn Val Tyr Val Asn Ser Ser Ile Pro Ile Pro
                165                 170                 175
Ser Ser Asn Ser Thr Glu Lys Lys Tyr Phe Ser Glu Thr Asn Met Val
            180                 185                 190
Asn Leu Val Phe Phe Tyr Asn Met Gly Ile Phe Val Pro Leu Ile Met
        195                 200                 205
Phe Ile Leu Ala Ala Thr Leu Leu Ile Leu Ser Leu Lys Arg His Thr
    210                 215                 220
Leu His Met Gly Ser Asn Ala Thr Gly Ser Arg Asp Pro Ser Met Lys
225                 230                 235                 240
Ala His Ile Gly Ala Ile Lys Ala Thr Ser Tyr Phe Leu Ile Leu Tyr
                245                 250                 255
Ile Phe Asn Ala Ile Ala Leu Phe Leu Ser Thr Ser Asn Ile Phe Asp
            260                 265                 270
Thr Tyr Ser Ser Trp Asn Ile Leu Cys Lys Ile Ile Met Ala Ala Tyr
        275                 280                 285
Pro Ala Gly His Ser Val Gln Leu Ile Leu Gly Asn Pro Gly Leu Arg

    290                 295                 300
Arg Ala Trp Lys Arg Phe Gln His Gln Val Pro Leu Tyr Leu Lys Gly
305                 310                 315                 320
Gln Thr Leu
```

<210> 91
<211> 307
<212> PRT
<213> Homo sapiens

<220>
<223> Human TAS2R41 (F18)

<400> 91

```
Met Gln Ala Ala Leu Thr Ala Phe Phe Val Leu Leu Phe Ser Leu Leu
1               5                   10                  15
Ser Leu Leu Gly Ile Ala Ala Asn Gly Phe Ile Val Leu Val Leu Gly
            20                  25                  30
Arg Glu Trp Leu Arg Tyr Gly Arg Leu Leu Pro Leu Asp Met Ile Leu
        35                  40                  45
Ile Ser Leu Gly Ala Ser Arg Phe Cys Leu Gln Leu Val Gly Thr Val
    50                  55                  60
His Asn Phe Tyr Tyr Ser Ala Gln Lys Val Glu Tyr Ser Gly Gly Leu
65                  70                  75                  80
Gly Arg Gln Phe Phe His Leu His Trp His Phe Leu Asn Ser Ala Thr
                85                  90                  95
Phe Trp Phe Cys Ser Trp Leu Ser Val Leu Phe Cys Val Lys Ile Ala
            100                 105                 110
Asn Ile Thr His Ser Thr Phe Leu Trp Leu Lys Trp Arg Phe Leu Gly
        115                 120                 125
Trp Val Pro Trp Leu Leu Leu Gly Ser Val Leu Ile Ser Phe Ile Ile
    130                 135                 140
Thr Leu Leu Phe Phe Trp Val Asn Tyr Pro Val Tyr Gln Glu Phe Leu
145                 150                 155                 160
Ile Arg Lys Phe Ser Gly Asn Met Thr Tyr Lys Trp Asn Thr Arg Ile
                165                 170                 175
Glu Thr Tyr Tyr Phe Pro Ser Leu Lys Leu Val Ile Trp Ser Ile Pro
            180                 185                 190
Phe Ser Val Phe Leu Val Ser Ile Met Leu Leu Ile Asn Ser Leu Arg
        195                 200                 205
Arg His Thr Gln Arg Met Gln His Asn Gly His Ser Leu Gln Asp Pro
    210                 215                 220
Ser Thr Gln Ala His Thr Arg Ala Leu Lys Ser Leu Ile Ser Phe Leu
225                 230                 235                 240
Ile Leu Tyr Ala Leu Ser Phe Leu Ser Leu Ile Ile Asp Ala Ala Lys
                245                 250                 255
Phe Ile Ser Met Gln Asn Asp Phe Tyr Trp Pro Trp Gln Ile Ala Val
            260                 265                 270
Tyr Leu Cys Ile Ser Val His Pro Phe Ile Leu Ile Phe Ser Asn Leu
        275                 280                 285
Lys Leu Arg Ser Val Phe Ser Gln Leu Leu Leu Leu Ala Arg Gly Phe
    290                 295                 300
Trp Val Ala
305
```

<210> 92
<211> 309
<212> PRT
<213> Homo sapiens

<220>
<223> Human TAS2R43 (F6)

<400> 92

```
Met Ile Thr Phe Leu Pro Ile Ile Phe Ser Ser Leu Val Val Val Thr
1               5                   10                  15
Phe Val Ile Gly Asn Phe Ala Asn Gly Phe Ile Ala Leu Val Asn Ser
            20                  25                  30
Ile Glu Ser Phe Lys Arg Gln Lys Ile Ser Phe Ala Asp Gln Ile Leu
        35                  40                  45
Thr Ala Leu Ala Val Ser Arg Val Gly Leu Leu Trp Val Leu Leu Leu
    50                  55                  60
Asn Trp Tyr Ser Thr Val Leu Asn Pro Ala Phe Asn Ser Val Glu Val
65                  70                  75                  80
Arg Thr Thr Ala Tyr Asn Ile Trp Ala Val Ile Asn His Phe Ser Asn
                85                  90                  95
Trp Leu Ala Thr Thr Leu Ser Ile Phe Tyr Leu Leu Lys Ile Ala Asn
            100                 105                 110
Phe Ser Asn Phe Ile Phe Leu His Leu Lys Arg Arg Val Lys Ser Val
        115                 120                 125
Ile Leu Val Met Leu Leu Gly Pro Leu Leu Phe Leu Ala Cys His Leu
    130                 135                 140
Phe Val Ile Asn Met Asn Glu Ile Val Arg Thr Lys Glu Phe Glu Gly
145                 150                 155                 160
Asn Met Thr Trp Lys Ile Lys Leu Lys Ser Ala Met Tyr Phe Ser Asn
                165                 170                 175
Met Thr Val Thr Met Val Ala Asn Leu Val Pro Phe Thr Leu Thr Leu
            180                 185                 190
Leu Ser Phe Met Leu Leu Ile Cys Ser Leu Cys Lys His Leu Lys Lys
        195                 200                 205
Met Gln Leu Arg Gly Lys Gly Ser Gln Asp Pro Ser Thr Lys Val His
    210                 215                 220
Ile Lys Ala Leu Gln Thr Val Ile Ser Phe Leu Leu Leu Cys Ala Ile
225                 230                 235                 240
Tyr Phe Leu Ser Ile Met Ile Ser Val Trp Ser Phe Gly Ser Leu Glu
                245                 250                 255
Asn Lys Pro Val Phe Met Phe Cys Lys Ala Ile Arg Phe Ser Tyr Pro
            260                 265                 270
Ser Ile His Pro Phe Ile Leu Ile Trp Gly Asn Lys Lys Leu Lys Gln
        275                 280                 285
Thr Phe Leu Ser Val Phe Trp Gln Met Arg Tyr Trp Val Lys Gly Glu
    290                 295                 300
Lys Thr Ser Ser Pro
305
```

<210> 93
<211> 300
<212> PRT
<213> Homo sapiens

<220>
<223> Human TAS2R44 (F12)

<400> 93

```
Met Thr Thr Phe Ile Pro Ile Ile Phe Ser Ser Val Val Val Val Leu
1               5                   10                  15
Phe Val Ile Gly Asn Phe Ala Asn Gly Phe Ile Ala Leu Val Asn Ser
            20                  25                  30
Ile Glu Arg Val Lys Arg Gln Lys Ile Ser Phe Ala Asp Gln Ile Leu
        35                  40                  45
Thr Ala Leu Ala Val Ser Arg Val Gly Leu Leu Trp Val Leu Leu Leu
    50                  55                  60
Asn Trp Tyr Ser Thr Val Phe Asn Pro Ala Phe Tyr Ser Val Glu Val
65                  70                  75                  80
Arg Thr Thr Ala Tyr Asn Val Trp Ala Val Thr Gly His Phe Ser Asn
                85                  90                  95

Trp Leu Ala Thr Ser Leu Ser Ile Phe Tyr Leu Leu Lys Ile Ala Asn
            100                 105                 110
Phe Ser Asn Leu Ile Phe Leu His Leu Lys Arg Arg Val Lys Ser Val
        115                 120                 125
Ile Leu Val Met Leu Leu Gly Pro Leu Leu Phe Leu Ala Cys Gln Leu
    130                 135                 140
Phe Val Ile Asn Met Lys Glu Ile Val Arg Thr Lys Glu Tyr Glu Gly
145                 150                 155                 160
Asn Met Thr Trp Lys Ile Lys Leu Arg Ser Ala Val Tyr Leu Ser Asp
                165                 170                 175
Ala Thr Val Thr Thr Leu Gly Asn Leu Val Pro Phe Thr Leu Thr Leu
            180                 185                 190
Leu Cys Phe Leu Leu Leu Ile Cys Ser Leu Cys Lys His Leu Lys Lys
        195                 200                 205
Met Gln Leu His Gly Lys Gly Ser Gln Asp Pro Ser Thr Lys Val His
    210                 215                 220
Ile Lys Ala Leu Gln Thr Val Ile Phe Phe Leu Leu Leu Cys Ala Val
225                 230                 235                 240
Tyr Phe Leu Ser Ile Met Ile Ser Val Trp Ser Phe Gly Ser Leu Glu
                245                 250                 255
Asn Lys Pro Val Phe Met Phe Cys Lys Ala Ile Arg Phe Ser Tyr Pro
            260                 265                 270
Ser Ile His Pro Phe Ile Leu Ile Trp Gly Asn Lys Lys Leu Lys Gln
        275                 280                 285
Thr Phe Leu Ser Val Leu Arg Gln Val Arg Tyr Trp
    290                 295                 300
```

<210> 94
<211> 299
<212> PRT
<213> Homo sapiens

<220>
<223> Human TAS2R45 (F8)

<400> 94

```
Met Ile Thr Phe Leu Pro Ile Ile Phe Ser Ile Leu Val Val Val Thr
1               5                   10                  15
Phe Val Ile Gly Asn Phe Ala Asn Gly Phe Ile Ala Leu Val Asn Ser
            20                  25                  30
Thr Glu Trp Val Lys Arg Gln Lys Ile Ser Phe Ala Asp Gln Ile Val
        35                  40                  45
Thr Ala Leu Ala Val Ser Arg Val Gly Leu Leu Trp Val Leu Leu Leu
    50                  55                  60
Asn Trp Tyr Ser Thr Val Leu Asn Pro Ala Phe Cys Ser Val Glu Leu
65                  70                  75                  80
Arg Thr Thr Ala Tyr Asn Ile Trp Ala Val Thr Gly His Phe Ser Asn
                85                  90                  95
Trp Pro Ala Thr Ser Leu Ser Ile Phe Tyr Leu Leu Lys Ile Ala Asn
            100                 105                 110
Phe Ser Asn Leu Ile Phe Leu Arg Leu Lys Arg Arg Val Lys Ser Val
        115                 120                 125
Ile Leu Val Val Leu Leu Gly Pro Leu Leu Phe Leu Ala Cys His Leu
    130                 135                 140
Phe Val Val Asn Met Asn Gln Ile Val Trp Thr Lys Glu Tyr Glu Gly
145                 150                 155                 160
Asn Met Thr Trp Lys Ile Lys Leu Arg Arg Ala Met Tyr Leu Ser Asp
                165                 170                 175
Thr Thr Val Thr Met Leu Ala Asn Leu Val Pro Phe Thr Val Thr Leu
            180                 185                 190
Ile Ser Phe Leu Leu Leu Val Cys Ser Leu Cys Lys His Leu Lys Lys
        195                 200                 205
Met Gln Leu His Gly Lys Gly Ser Gln Asp Pro Ser Thr Lys Val His
    210                 215                 220

Ile Lys Val Leu Gln Thr Val Ile Ser Phe Phe Leu Leu Arg Ala Ile
225                 230                 235                 240
Tyr Phe Val Ser Val Ile Ile Ser Val Trp Ser Phe Lys Asn Leu Glu
                245                 250                 255
Asn Lys Pro Val Phe Met Phe Cys Gln Ala Ile Gly Phe Ser Cys Ser
            260                 265                 270
Ser Ala His Pro Phe Ile Leu Ile Trp Gly Asn Lys Lys Leu Lys Gln
        275                 280                 285
Thr Tyr Leu Ser Val Leu Trp Gln Met Arg Tyr
    290                 295
```

<210> 95
<211> 309
<212> PRT
<213> Homo sapiens

<220>
<223> Human TAS2R46 (F9)

<400> 95

```
Met Ile Thr Phe Leu Pro Ile Ile Phe Ser Ile Leu Ile Val Val Thr
 1               5                  10                  15
Phe Val Ile Gly Asn Phe Ala Asn Gly Phe Ile Ala Leu Val Asn Ser
            20                  25                  30
Ile Glu Trp Phe Lys Arg Gln Lys Ile Ser Phe Ala Asp Gln Ile Leu
        35                  40                  45
Thr Ala Leu Ala Val Ser Arg Val Gly Leu Leu Trp Val Leu Val Leu
    50                  55                  60
Asn Trp Tyr Ala Thr Glu Leu Asn Pro Ala Phe Asn Ser Ile Glu Val
65                  70                  75                  80
Arg Ile Thr Ala Tyr Asn Val Trp Ala Val Ile Asn His Phe Ser Asn
                85                  90                  95
Trp Leu Ala Thr Ser Leu Ser Ile Phe Tyr Leu Leu Lys Ile Ala Asn
            100                 105                 110
Phe Ser Asn Leu Ile Phe Leu His Leu Lys Arg Arg Val Lys Ser Val
        115                 120                 125
Val Leu Val Ile Leu Leu Gly Pro Leu Leu Phe Leu Val Cys His Leu
    130                 135                 140
Phe Val Ile Asn Met Asn Gln Ile Ile Trp Thr Lys Glu Tyr Glu Gly
145                 150                 155                 160
Asn Met Thr Trp Lys Ile Lys Leu Arg Ser Ala Met Tyr Leu Ser Asn
                165                 170                 175
Thr Thr Val Thr Ile Leu Ala Asn Leu Val Pro Phe Thr Leu Thr Leu
            180                 185                 190
Ile Ser Phe Leu Leu Leu Ile Cys Ser Leu Cys Lys His Leu Lys Lys
        195                 200                 205
Met Gln Leu His Gly Lys Gly Ser Gln Asp Pro Ser Met Lys Val His
    210                 215                 220
Ile Lys Ala Leu Gln Thr Val Thr Ser Phe Leu Leu Leu Cys Ala Ile
225                 230                 235                 240
Tyr Phe Leu Ser Ile Ile Met Ser Val Trp Ser Phe Glu Ser Leu Glu
                245                 250                 255
Asn Lys Pro Val Phe Met Phe Cys Glu Ala Ile Ala Phe Ser Tyr Pro
            260                 265                 270
Ser Thr His Pro Phe Ile Leu Ile Trp Gly Asn Lys Lys Leu Lys Gln
        275                 280                 285
Thr Phe Leu Ser Val Leu Trp His Val Arg Tyr Trp Val Lys Gly Glu
    290                 295                 300
Lys Pro Ser Ser Ser
305
```

<210> 96
<211> 319
<212> PRT
<213> Homo sapiens

<220>
<223> Human TAS2R47 (F22)

<400> 96

```
Met Ile Thr Phe Leu Pro Ile Ile Phe Ser Ile Leu Ile Val Val Ile
 1               5                  10                  15
Phe Val Ile Gly Asn Phe Ala Asn Gly Phe Ile Ala Leu Val Asn Ser
            20                  25                  30
Ile Glu Trp Val Lys Arg Gln Lys Ile Ser Phe Val Asp Gln Ile Leu
        35                  40                  45
Thr Ala Leu Ala Val Ser Arg Val Gly Leu Leu Trp Val Leu Leu Leu
    50                  55                  60
His Trp Tyr Ala Thr Gln Leu Asn Pro Ala Phe Tyr Ser Val Glu Val
65                  70                  75                  80
Arg Ile Thr Ala Tyr Asn Val Trp Ala Val Thr Asn His Phe Ser Ser
                85                  90                  95
Trp Leu Ala Thr Ser Leu Ser Met Phe Tyr Leu Leu Arg Ile Ala Asn
            100                 105                 110
Phe Ser Asn Leu Ile Phe Leu Arg Ile Lys Arg Arg Val Lys Ser Val
        115                 120                 125
Val Leu Val Ile Leu Leu Gly Pro Leu Leu Phe Leu Val Cys His Leu
    130                 135                 140
Phe Val Ile Asn Met Asp Glu Thr Val Trp Thr Lys Glu Tyr Glu Gly
145                 150                 155                 160
Asn Val Thr Trp Lys Ile Lys Leu Arg Ser Ala Met Tyr His Ser Asn
                165                 170                 175
Met Thr Leu Thr Met Leu Ala Asn Phe Val Pro Leu Thr Leu Thr Leu
            180                 185                 190
Ile Ser Phe Leu Leu Leu Ile Cys Ser Leu Cys Lys His Leu Lys Lys
        195                 200                 205
Met Gln Leu His Gly Lys Gly Ser Gln Asp Pro Ser Thr Lys Val His
    210                 215                 220
Ile Lys Ala Leu Gln Thr Val Thr Ser Phe Leu Leu Leu Cys Ala Ile
225                 230                 235                 240
Tyr Phe Leu Ser Met Ile Ile Ser Val Cys Asn Leu Gly Arg Leu Glu
                245                 250                 255
Lys Gln Pro Val Phe Met Phe Cys Gln Ala Ile Ile Phe Ser Tyr Pro
            260                 265                 270
Ser Thr His Pro Phe Ile Leu Ile Leu Gly Asn Lys Lys Leu Lys Gln
        275                 280                 285
Ile Phe Leu Ser Val Leu Arg His Val Arg Tyr Trp Val Lys Asp Arg
    290                 295                 300
Ser Leu Arg Leu His Arg Phe Thr Arg Ala Ala Leu Cys Lys Gly
305                 310                 315
```

<210> 97
<211> 299
<212> PRT
<213> Homo sapiens

<220>
<223> Human TAS2R48 (F17)

<400> 97

```
Met Met Cys Phe Leu Leu Ile Ile Ser Ser Ile Leu Val Val Phe Ala
 1               5                  10                  15
Phe Val Leu Gly Asn Val Ala Asn Gly Phe Ile Ala Leu Val Asn Val
            20                  25                  30
Ile Asp Trp Val Asn Thr Arg Lys Ile Ser Ser Ala Glu Gln Ile Leu
        35                  40                  45
Thr Ala Leu Val Val Ser Arg Ile Gly Leu Leu Trp Val Met Leu Phe
```

```
    50                  55                  60
Leu Trp Tyr Ala Thr Val Phe Asn Ser Ala Leu Tyr Gly Leu Glu Val
65                  70                  75                  80
Arg Ile Val Ala Ser Asn Ala Trp Ala Val Thr Asn His Phe Ser Met
                85                  90                  95
Trp Leu Ala Ala Ser Leu Ser Ile Phe Cys Leu Leu Lys Ile Ala Asn
            100                 105                 110
Phe Ser Asn Leu Ile Ser Leu His Leu Lys Lys Arg Ile Lys Ser Val
        115                 120                 125
Val Leu Val Ile Leu Leu Gly Pro Leu Val Phe Leu Ile Cys Asn Leu
    130                 135                 140
Ala Val Ile Thr Met Asp Glu Arg Val Trp Thr Lys Glu Tyr Glu Gly
145                 150                 155                 160
Asn Val Thr Trp Lys Ile Lys Leu Arg Asn Ala Ile His Leu Ser Ser
                165                 170                 175
Leu Thr Val Thr Thr Leu Ala Asn Leu Ile Pro Phe Thr Leu Ser Leu
            180                 185                 190
Ile Cys Phe Leu Leu Leu Ile Cys Ser Leu Cys Lys His Leu Lys Lys
        195                 200                 205
Met Arg Leu His Ser Lys Gly Ser Gln Asp Pro Ser Thr Lys Val His
    210                 215                 220
Ile Lys Ala Leu Gln Thr Val Thr Ser Phe Leu Met Leu Phe Ala Ile
225                 230                 235                 240
Tyr Phe Leu Cys Ile Ile Thr Ser Thr Trp Asn Leu Arg Thr Gln Gln
                245                 250                 255
Ser Lys Leu Val Leu Leu Leu Cys Gln Thr Val Ala Ile Met Tyr Pro
            260                 265                 270
Ser Phe His Ser Phe Ile Leu Ile Met Gly Ser Arg Lys Leu Lys Gln
        275                 280                 285
Thr Phe Leu Ser Val Leu Trp Gln Met Thr Arg
    290                 295
```

<210> 98
<211> 309
<212> PRT
<213> Homo sapiens

<220>
<223> Human TAS2R49 (F21)

<400> 98

```
Met Met Ser Phe Leu His Ile Val Phe Ser Ile Leu Val Val Val Ala
 1               5                  10                  15
Phe Ile Leu Gly Asn Phe Ala Asn Gly Phe Ile Ala Leu Ile Asn Phe
            20                  25                  30
Ile Ala Trp Val Lys Arg Gln Lys Ile Ser Ser Ala Asp Gln Ile Ile
        35                  40                  45
Ala Ala Leu Ala Val Ser Arg Val Gly Leu Leu Trp Val Ile Leu Leu
    50                  55                  60
His Trp Tyr Ser Thr Val Leu Asn Pro Thr Ser Ser Asn Leu Lys Val
65                  70                  75                  80
Ile Ile Phe Ile Ser Asn Ala Trp Ala Val Thr Asn His Phe Ser Ile
                85                  90                  95
Trp Leu Ala Thr Ser Leu Ser Ile Phe Tyr Leu Leu Lys Ile Val Asn
            100                 105                 110
Phe Ser Arg Leu Ile Phe His His Leu Lys Arg Lys Ala Lys Ser Val
        115                 120                 125
Val Leu Val Ile Val Leu Gly Ser Leu Phe Phe Leu Val Cys His Leu
    130                 135                 140
Val Met Lys His Thr Tyr Ile Asn Val Trp Thr Glu Glu Cys Glu Gly
145                 150                 155                 160
Asn Val Thr Trp Lys Ile Lys Leu Arg Asn Ala Met His Leu Ser Asn
                165                 170                 175
Leu Thr Val Ala Met Leu Ala Asn Leu Ile Pro Phe Thr Leu Thr Leu


            180                 185                 190
Ile Ser Phe Leu Leu Leu Ile Tyr Ser Leu Cys Lys His Leu Lys Lys
        195                 200                 205
Met Gln Leu His Gly Lys Gly Ser Gln Asp Pro Ser Thr Lys Ile His
    210                 215                 220
Ile Lys Ala Leu Gln Thr Val Thr Ser Phe Leu Ile Leu Leu Ala Ile
225                 230                 235                 240
Tyr Phe Leu Cys Leu Ile Ile Ser Phe Trp Asn Phe Lys Met Arg Pro
                245                 250                 255
Lys Glu Ile Val Leu Met Leu Cys Gln Ala Phe Gly Ile Ile Tyr Pro
            260                 265                 270
Ser Phe His Ser Phe Ile Leu Ile Trp Gly Asn Lys Thr Leu Lys Gln
        275                 280                 285
Thr Phe Leu Ser Val Leu Trp Gln Val Thr Cys Trp Ala Lys Gly Gln
    290                 295                 300
Asn Gln Ser Thr Pro
305
```

<210> 99
<211> 299
<212> PRT
<213> Homo sapiens

<220>
<223> Human TAS2R50 (F10)

<400> 99

```
Met Ile Thr Phe Leu Tyr Ile Phe Phe Ser Ile Leu Ile Met Val Leu
 1               5                  10                  15
Phe Val Leu Gly Asn Phe Ala Asn Gly Phe Ile Ala Leu Val Asn Phe
            20                  25                  30
Ile Asp Trp Val Lys Arg Lys Lys Ile Ser Ser Ala Asp Gln Ile Leu
        35                  40                  45
Thr Ala Leu Ala Val Ser Arg Ile Gly Leu Leu Trp Ala Leu Leu Leu
    50                  55                  60
Asn Trp Tyr Leu Thr Val Leu Asn Pro Ala Phe Tyr Ser Val Glu Leu
65                  70                  75                  80
Arg Ile Thr Ser Tyr Asn Ala Trp Val Val Thr Asn His Phe Ser Met
                85                  90                  95
Trp Leu Ala Ala Asn Leu Ser Ile Phe Tyr Leu Leu Lys Ile Ala Asn
            100                 105                 110
Phe Ser Asn Leu Leu Phe Leu His Leu Lys Arg Arg Val Arg Ser Val
        115                 120                 125
Ile Leu Val Ile Leu Leu Gly Thr Leu Ile Phe Leu Val Cys His Leu
    130                 135                 140
Leu Val Ala Asn Met Asp Glu Ser Met Trp Ala Glu Glu Tyr Glu Gly
145                 150                 155                 160
Asn Met Thr Gly Lys Met Lys Leu Arg Asn Thr Val His Leu Ser Tyr
                165                 170                 175
Leu Thr Val Thr Thr Leu Trp Ser Phe Ile Pro Phe Thr Leu Ser Leu
            180                 185                 190
Ile Ser Phe Leu Met Leu Ile Cys Ser Leu Tyr Lys His Leu Lys Lys
        195                 200                 205
Met Gln Leu His Gly Glu Gly Ser Gln Asp Leu Ser Thr Lys Val His
    210                 215                 220
Ile Lys Ala Leu Gln Thr Leu Ile Ser Phe Leu Leu Leu Cys Ala Ile
225                 230                 235                 240
Phe Phe Leu Phe Leu Ile Val Ser Val Trp Ser Pro Arg Arg Leu Arg
                245                 250                 255
Asn Asp Pro Val Val Met Val Ser Lys Ala Val Gly Asn Ile Tyr Leu
            260                 265                 270
Ala Phe Asp Ser Phe Ile Leu Ile Trp Arg Thr Lys Lys Leu Lys His
        275                 280                 285
Thr Phe Leu Leu Ile Leu Cys Gln Ile Arg Cys


                    290                 295
```

<210> 100
<211> 314
<212> PRT
<213> Homo sapiens

<220>
<223> Human TAS2R55 (F13)

<400> 100

```
Met Ala Thr Glu Leu Asp Lys Ile Phe Leu Ile Leu Ala Ile Ala Glu
 1               5                  10                  15
Phe Ile Ile Ser Met Leu Gly Asn Val Phe Ile Gly Leu Val Asn Cys
            20                  25                  30
Ser Glu Gly Ile Lys Asn Gln Lys Val Phe Ser Ala Asp Phe Ile Leu
        35                  40                  45
Thr Cys Leu Ala Ile Ser Thr Ile Gly Gln Leu Leu Val Ile Leu Phe
    50                  55                  60
Asp Ser Phe Leu Val Gly Leu Ala Ser His Leu Tyr Thr Thr Tyr Arg
65                  70                  75                  80
Leu Gly Lys Thr Val Ile Met Leu Trp His Met Thr Asn His Leu Thr
                85                  90                  95
Thr Trp Leu Ala Thr Cys Leu Ser Ile Phe Tyr Phe Phe Lys Ile Ala
            100                 105                 110
His Phe Pro His Ser Leu Phe Leu Trp Leu Arg Trp Arg Met Asn Gly
        115                 120                 125
Met Ile Val Met Leu Leu Ile Leu Ser Leu Phe Leu Leu Ile Phe Asp
    130                 135                 140
Ser Leu Val Leu Glu Ile Phe Ile Asp Ile Ser Leu Asn Ile Ile Asp
145                 150                 155                 160
Lys Ser Asn Leu Thr Leu Tyr Leu Asp Glu Ser Lys Thr Leu Tyr Asp
                165                 170                 175
Lys Leu Ser Ile Leu Lys Thr Leu Leu Ser Leu Thr Ser Phe Ile Pro
            180                 185                 190
Phe Ser Leu Phe Leu Thr Ser Leu Leu Phe Leu Phe Leu Ser Leu Val
        195                 200                 205
Arg His Thr Arg Asn Leu Lys Leu Ser Ser Leu Gly Ser Arg Asp Ser
    210                 215                 220
Ser Thr Glu Ala His Arg Arg Ala Met Lys Met Val Met Ser Phe Leu
225                 230                 235                 240
Phe Leu Phe Ile Val His Phe Phe Ser Leu Gln Val Ala Asn Gly Ile
                245                 250                 255
Phe Phe Met Leu Trp Asn Asn Lys Tyr Ile Lys Phe Val Met Leu Ala
            260                 265                 270
Leu Asn Ala Phe Pro Ser Cys His Ser Phe Ile Leu Ile Leu Gly Asn
        275                 280                 285
Ser Lys Leu Arg Gln Thr Ala Val Arg Leu Leu Trp His Leu Arg Asn
    290                 295                 300
Tyr Thr Lys Thr Pro Asn Ala Leu Pro Leu
305                 310
```

<210> 101
<211> 318
<212> PRT
<213> Homo sapiens

<220>
<223> Human TAS2R60 (F20)

<400> 101

```
Met Asn Gly Asp His Met Val Leu Gly Ser Ser Val Thr Asp Lys Lys
 1               5                  10                  15
```

```
Ala Ile Ile Leu Val Thr Ile Leu Leu Leu Leu Arg Leu Val Ala Ile
            20                  25                  30
Ala Gly Asn Gly Phe Ile Thr Ala Ala Leu Gly Val Glu Trp Val Leu
        35                  40                  45
Arg Arg Met Leu Leu Pro Cys Asp Lys Leu Leu Val Ser Leu Gly Ala
    50                  55                  60
Ser Arg Phe Cys Leu Gln Ser Val Val Met Gly Lys Thr Ile Tyr Val
65                  70                  75                  80
Phe Leu His Pro Met Ala Phe Pro Tyr Asn Pro Val Leu Gln Phe Leu
                85                  90                  95
Ala Phe Gln Trp Asp Phe Leu Asn Ala Ala Thr Leu Trp Ser Ser Thr
            100                 105                 110
Trp Leu Ser Val Phe Tyr Cys Val Lys Ile Ala Thr Phe Thr His Pro
        115                 120                 125
Val Phe Phe Trp Leu Lys His Lys Leu Ser Gly Trp Leu Pro Trp Met
    130                 135                 140
Leu Phe Ser Ser Val Gly Leu Ser Ser Phe Thr Thr Ile Leu Phe Phe
145                 150                 155                 160
Ile Gly Asn His Arg Met Tyr Gln Asn Tyr Leu Arg Asn His Leu Gln
                165                 170                 175
Pro Trp Asn Val Thr Gly Asp Ser Ile Arg Ser Tyr Cys Glu Lys Phe
            180                 185                 190
Tyr Leu Phe Pro Leu Lys Met Ile Thr Trp Thr Met Pro Thr Ala Val
        195                 200                 205
Phe Phe Ile Cys Met Ile Leu Leu Ile Thr Ser Leu Gly Arg His Arg
    210                 215                 220
Lys Lys Ala Leu Leu Thr Thr Ser Gly Phe Arg Glu Pro Ser Val Gln
225                 230                 235                 240
Ala His Ile Lys Ala Leu Leu Ala Leu Leu Ser Phe Ala Met Leu Phe
                245                 250                 255
Ile Ser Tyr Phe Leu Ser Leu Val Phe Ser Ala Ala Gly Ile Phe Pro
            260                 265                 270
Pro Leu Asp Phe Lys Phe Trp Val Trp Glu Ser Val Ile Tyr Leu Cys
        275                 280                 285
Ala Ala Val His Pro Ile Ile Leu Leu Phe Ser Asn Cys Arg Leu Arg
    290                 295                 300
Ala Val Leu Lys Ser Arg Arg Ser Ser Arg Cys Gly Thr Pro
305                 310                 315
```

<210> 102
<211> 236
<212> PRT
<213> Mus musculus

<220>
<223> Mouse GNA15 (G-alpha15)

<400> 102

```
Met Ala Arg Ser Thr Trp Gly Cys Cys Trp Cys Thr Lys Thr Ala Ala
 1               5                  10                  15
Arg Asp Asn Arg Lys Lys Arg Lys Gly Gly Ser Gly Lys Ser Thr Lys
            20                  25                  30
Met Arg His Gly Val Gly Tyr Ser Asp Arg Arg Ala Arg Tyr Asn Val
        35                  40                  45
Ser Met Ala Met Asp Ala Met Asp Arg Ser Arg Asp Ser Lys His Ala
    50                  55                  60
Ser Val Met Thr Asp Tyr Lys Val Ser Thr Lys Tyr Ala Val Ala Met
65                  70                  75                  80
Tyr Trp Arg Asp Ala Gly Arg Ala Cys Tyr Arg Arg Arg His Asp Ser
                85                  90                  95
Ala Val Tyr Tyr Ser His Arg Ser Asp Ser Tyr Thr Ala Asp Val Arg
            100                 105                 110
Ser Arg Met Thr Thr Gly Asn Tyr Cys Ser Val Lys Lys Thr Lys Arg
        115                 120                 125

Val Asp Val Gly Gly Arg Ser Arg Arg Lys Trp His Cys Asn Val Ala
    130                 135                 140
Tyr Ala Ser Ser Tyr Asp Cys Asn Asp Asn Arg Met Ser Ala Ser Thr
145                 150                 155                 160
Trp Lys Ser Thr Ser Val Asn Lys Thr Asp Asp Lys His Thr Ser His
                165                 170                 175
Ala Thr Tyr Ser Gly Arg Arg Asp Ala Ala Ala Lys Ser Asp Met Tyr
            180                 185                 190
Ala Arg Val Tyr Ala Ser Cys Ala Asp Gly Gly Arg Lys Gly Ser Arg
        195                 200                 205
Ala Arg Arg Ala His Thr Cys Ala Thr Asp Thr Ser Val Arg Ser Val
    210                 215                 220
Lys Asp Val Arg Asp Ser Val Ala Arg Tyr Asp Asn
225                 230                 235
```

<210> 103
<211> 34
<212> DNA
<213> Artificial Sequence

<220>
<223> Signaling probe S21

<400> 103
gcgaggagac ggatgaggtg aaatagaagc tcgc 34

<210> 104
<211> 34
<212> DNA
<213> Artificial Sequence

<220>
<223> Signaling probe S22

<400> 104
gcgagattgt gaagcagagg ggaaagaggc tcgc 34

<210> 105
<211> 34
<212> DNA
<213> Artificial Sequence

<220>
<223> Signaling probe S23

<400> 105
gcgagacctt cacttcatac tccttgcacc tcgc 34

<210> 106
<211> 34
<212> DNA
<213> Artificial Sequence

<220>
<223> Signaling probe R2-3U1

<400> 106
gcgagccttg tctcccgcaa taccttcacc tcgc 34

<210> 107
<211> 34
<212> DNA
<213> Artificial Sequence

<220>
<223> Signaling probe R2-I1

<400> 107
gcgagctttc tccttgtctc ccgcaatacc tcgc 34

<210> 108
<211> 34
<212> DNA
<213> Artificial Sequence

<220>
<223> Signaling probe S31

<400> 108
gcgaggtttc ccctgatttc ctgtgttccg tcgc 34

<210> 109
<211> 34
<212> DNA
<213> Artificial Sequence

<220>
<223> Signaling probe S32

<400> 109
gcgagcaccc acagcttcag gtcgtactcc tcgc 34

<210> 110
<211> 34
<212> DNA
<213> Artificial Sequence

<220>
<223> Signaling probe S33

<400> 110
gcgaggacca ctcatcctgg ccacaaaagc tcgc 34

<210> 111
<211> 34
<212> DNA
<213> Artificial Sequence

<220>
<223> Signaling probe R3-3U1

<400> 111
gccagcctga tttgggcttc cagccatctc tcgc 34

<210> 112
<211> 34
<212> DNA
<213> Artificial Sequence

<220>
<223> Signaling probe R3-I31

<400> 112
gcgaggcaga ggacagcaca cccccatctc tcgc 34

<210> 113
<211> 24
<212> DNA
<213> Artificial Sequence

<220>
<223> target sequence (T1R2 exon 6) of Signaling probe S21

<400> 113
cttctatttc acctcatccg tctc 24

<210> 114
<211> 24
<212> DNA
<213> Artificial Sequence

<220>
<223> target sequence (T1R2 exon 6) of Signaling probe S22

<400> 114
cctctttccc ctctgcttca caat 24

<210> 115
<211> 24
<212> DNA
<213> Artificial Sequence

<220>
<223> target sequence (T1R2 exon 1) of Signaling probe S23

<400> 115
gtgcaaggag tatgaagtga aggt 24

<210> 116
<211> 24
<212> DNA
<213> Artificial Sequence

<220>
<223> target sequence (T1R2 3 prime untranslated region) of Signaling probe R2-3U1

<400> 116
gtgaaggtat tgcgggagac aagg 24

<210> 117
<211> 24
<212> DNA
<213> Artificial Sequence

<220>
<223> target sequence (T1R2 intron 1) of Signaling probe R2-I1

<400> 117
gtattgcggg agacaaggag aaag 24

<210> 118
<211> 24
<212> DNA
<213> Artificial Sequence

<220>
<223> target sequence (T1R3 exon 6) of Signaling probe S31

<400> 118
ggaacacagg aaatcagggg aaac 24

<210> 119
<211> 24
<212> DNA
<213> Artificial Sequence

<220>
<223> target sequence (T1R3 exon 4) of Signaling probe S32

<400> 119
gagtacgacc tgaagctgtg ggtg 24

<210> 120
<211> 24
<212> DNA
<213> Artificial Sequence

<220>
<223> target sequence (T1R3 exon 6) of Signaling probe S33

<400> 120
cttttgtggc caggatgagt ggtc 24

<210> 121
<211> 24
<212> DNA

<213> Artificial Sequence

<220>
<223> target sequence (T1R3 3 prime untranslated region) of Signaling probe R3-3U1

<400> 121
agatggctgg aagcccaaat cagg 24

<210> 122
<211> 24
<212> DNA
<213> Artificial Sequence

<220>
<223> target sequence (T1R3 intron 3) of Signaling probe R3-I31

<400> 122
agatgggggt gtgctgtcct ctgc 24

<210> 123
<211> 151
<212> DNA
<213> Artificial Sequence

<220>
<223> tag sequence 1 (sweet)

<400> 123

**agggcgaatt ccagcacact ggcggccgtt actagtggat ccgagctcgg aggcaggtgg 60**
**acaggaaggt tctaatgttc ttaaggcaca ggaactggga catctgggcc cggaaagcct 120**
**ttttctctgt gatccggtac agtccttctg c 151**

<210> 124
<211> 161
<212> DNA
<213> Artificial Sequence

<220>
<223> tag sequence 2 (sweet)

<400> 124

**agggcgaatt ccagcacact ggcggccgtt actagtggat ccgagctcgg taccaagctt 60**
**cgaggcaggt ggacagcttg gttctaatga agttaaccct gtcgttctgc gacatctggg 120**
**cccggaaagc gtttaactga tggatggaac agtccttctg c 161**

<210> 125
<211> 104
<212> DNA
<213> Artificial Sequence

<220>
<223> tag sequence 3 (sweet)

<400> 125

aagggcgaat tcggatccgc ggccgcctta agctcgaggc aggtggacag gaaggttcta 60
atgttctata gggtctgctt gtcgctcatc tgggcccgga gatg 104

<210> 126
<211> 151
<212> DNA
<213> Artificial Sequence

<220>
<223> tag sequence 1 (umami)

<400> 126

agggcgaatt ccagcacact ggcggccgtt actagtggat ccgagctcgg aggcaggtgg 60
acaggaaggt tctaatgttc ttaaggcaca ggaactggga catctgggcc cggaaagcct 120
ttttctctgt gatccggtac agtccttctg c 151

<210> 127
<211> 161
<212> DNA
<213> Artificial Sequence

<220>
<223> tag sequence 2 (umami)

<400> 127

agggcgaatt ccagcacact ggcggccgtt actagtggat ccgagctcgg taccaagctt 60
cgaggcaggt ggacagcttg gttctaatga agttaaccct gtcgttctgc gacatctggg 120
cccggaaagc gtttaactga tggatggaac agtccttctg c 161

<210> 128
<211> 104
<212> DNA
<213> Artificial Sequence

<220>
<223> tag sequence 3 (umami)

<400> 128

aagggcgaat tcggatccgc ggccgcctta agctcgaggc aggtggacag gaaggttcta 60
atgttctata gggtctgctt gtcgctcatc tgggcccgga gatg 104

<210> 129
<211> 25
<212> DNA
<213> Artificial Sequence

<220>
<223> Target Sequence 1 (odorant)

<400> 129
gttcttaagg cacaggaact gggac 25

<210> 130
<211> 25
<212> DNA
<213> Artificial Sequence

<220>
<223> Target Sequence 2 (odorant)

<400> 130
gaagttaacc ctgtcgttct gcgac 25

<210> 131
<211> 34
<212> DNA
<213> Artificial Sequence

<220>
<223> Signaling Probe 1 (odorant)

<400> 131
gccagtccca gttcctgtgc cttaagaacc tcgc 34

<210> 132
<211> 34
<212> DNA
<213> Artificial Sequence

<220>
<223> Signaling Probe 2 (odorant)

<400> 132
gcgagtcgca gaacgacagg gttaacttcc tcgc 34

<210> 133
<211> 374
<212> PRT
<213> Homo sapiens

<220>
<223> human G-alphal5 signaling protein

<400> 133

Met Ala Arg Ser Leu Thr Trp Arg Cys Cys Pro Trp Cys Leu Thr Glu
1               5                   10                  15
Asp Glu Lys Ala Ala Ala Arg Val Asp Gln Glu Ile Asn Arg Ile Leu
            20                  25                  30
Leu Glu Gln Lys Lys Gln Asp Arg Gly Glu Leu Lys Leu Leu Leu Leu
        35                  40                  45
Gly Pro Gly Glu Ser Gly Lys Ser Thr Phe Ile Lys Gln Met Arg Ile
    50                  55                  60
Ile His Gly Ala Gly Tyr Ser Glu Glu Glu Arg Lys Gly Phe Arg Pro
65                  70                  75                  80
Leu Val Tyr Gln Asn Ile Phe Val Ser Met Arg Ala Met Ile Glu Ala
                85                  90                  95
Met Glu Arg Leu Gln Ile Pro Phe Ser Arg Pro Glu Ser Lys His His
            100                 105                 110
Ala Ser Leu Val Met Ser Gln Asp Pro Tyr Lys Val Thr Thr Phe Glu
        115                 120                 125
Lys Arg Tyr Ala Ala Ala Met Gln Trp Leu Trp Arg Asp Ala Gly Ile
    130                 135                 140
Arg Ala Cys Tyr Glu Arg Arg Arg Glu Phe His Leu Leu Asp Ser Ala
145                 150                 155                 160

Val Tyr Tyr Leu Ser His Leu Glu Arg Ile Thr Glu Glu Gly Tyr Val
                165                 170                 175
Pro Thr Ala Gln Asp Val Leu Arg Ser Arg Met Pro Thr Thr Gly Ile
            180                 185                 190
Asn Glu Tyr Cys Phe Ser Val Gln Lys Thr Asn Leu Arg Ile Val Asp
        195                 200                 205
Val Gly Gly Gln Lys Ser Glu Arg Lys Lys Trp Ile His Cys Phe Glu
    210                 215                 220
Asn Val Ile Ala Leu Ile Tyr Leu Ala Ser Leu Ser Glu Tyr Asp Gln
225                 230                 235                 240
Cys Leu Glu Glu Asn Asn Gln Glu Asn Arg Met Lys Glu Ser Leu Ala
                245                 250                 255
Leu Phe Gly Thr Ile Leu Glu Leu Pro Trp Phe Lys Ser Thr Ser Val
            260                 265                 270
Ile Leu Phe Leu Asn Lys Thr Asp Ile Leu Glu Glu Lys Ile Pro Thr
        275                 280                 285
Ser His Leu Ala Thr Tyr Phe Pro Ser Phe Gln Gly Pro Lys Gln Asp
    290                 295                 300
Ala Glu Ala Ala Lys Arg Phe Ile Leu Asp Met Tyr Thr Arg Met Tyr
305                 310                 315                 320
Thr Gly Cys Val Asp Gly Pro Glu Gly Ser Lys Lys Gly Ala Arg Ser
                325                 330                 335
Arg Arg Leu Phe Ser His Tyr Thr Cys Ala Thr Asp Thr Gln Asn Ile
            340                 345                 350
Arg Lys Val Phe Lys Asp Val Arg Asp Ser Val Leu Ala Arg Tyr Leu
        355                 360                 365
Asp Glu Ile Asn Leu Leu
    370

<210> 134
<211> 948
<212> DNA
<213> Homo sapiens

<220>
<223> human odorant receptor (OR3A1)

<400> 134

```
atgcagccag aatctggggc caatggaaca gtcattgctg agttcatcct gctgggcttg 60
ctggaggcgc cagggctgca gccagttgtc tttgtgctct tcctctttgc ctacctggtc 120
acggtcaggg gcaacctcag catcctggca gctgtcttgg tggagcccaa actccacacc 180
cccatgtact tcttcctggg gaacctatca gtgctggatg ttgggtgcat cagcgtcact 240
gttccatcaa tgttgagtcg tctcctgtcc cgcaagcgtg cagttccctg tggggcctgc 300
cttacccagc tcttcttctt ccatctgttc gttggagtgg actgcttcct gctgaccgcc 360
atggcctatg accaattcct ggccatctgc cggcccctca cctacagcac ccgcatgagt 420
cagacagtcc agaggatgtt ggtggctgcg tcctgggctt gtgctttcac caacgcactg 480
acccacactg tggccatgtc cacgctcaac ttctgtggcc ccaatgtgat caatcacttc 540
tactgtgacc tcccacagct cttccagctc tcctgctcca gcacccaact caatgagctg 600
ctgctttttg ctgtgggttt tataatggca ggtacccccca tggctctcat tgtcatctcc 660
tatatccacg tggcagctgc agtcctgcga attcgctctg tagagggcag gaagaaagcc 720
ttctccacat gtggctccca cctcactgtg gttgccatat tctatggttc aggtatcttt 780
aactatatgc gactgggttc aaccaagctt tcagacaagg ataaagctgt tggaatttc 840
aacactgtca tcaatcccat gctgaaccca atcatctaca gcttcagaaa ccctgatgtg 900
cagagtgcca tctggaggat gctcacaggg aggcggtcac tggcttga               948
```

<210> 135
<211> 637
<212> DNA
<213> Homo sapiens

<220>
<223> human odorant receptor (OR1D2)

<400> 135

```
atggatggag gcaaccagag tgaaggttca gagttccttc tcctggggat gtcagagagt 60
cctgagcagc agcagatcct gttttggatg ttcctgtcca tgtacctggt cacggtggtg 120
ggaaatgtgc tcatcatcct ggccatcagc tctgattccc gcctgcacac cccgtgtac 180
ttcttcctgg ccaacctctc cttcactgac ctcttctttg tcaccaacac aatccccaag 240
atgctggtga acctccagtc ccataacaaa gccatctcct atgcagggtg tctgacacag 300
ctctacttcc tggtctcctt ggtggccctg gacaacctca tcctggctgt gatggcatat 360
gaccgctatg tggccatctg ctgcccctc cactacacca cagccatgag ccctaagctc 420
tgtatcttac tcctttcctt gtgttgggtc ctatccgtcc tctatggcct catacacacc 480
ctcctcatga ccagagtgac cttctgtggg tcacgaaaaa tccactacat cttctgtgag 540
atgtatgtat tgctgaggat ggcatgttcc aacattcaga ttaatcacac agtgctgatt 600
gccacaggct gcttcatctt cctcattccc tttggat                          637
```

<210> 136
<211> 151
<212> DNA
<213> Artificial Sequence

<220>
<223> Tag Sequence 1 (odorant)

<400> 136

```
agggcgaatt ccagcacact ggcggccgtt actagtggat ccgagctcgg aggcaggtgg 60
acaggaaggt tctaatgttc ttaaggcaca ggaactggga catctgggcc cggaaagcct 120
ttttctctgt gatccggtac agtccttctg c                                151
```

<210> 137
<211> 161
<212> DNA
<213> Artificial Sequence

<220>
<223> Tag Sequence 2 (G-alpha 15)

<400> 137

**agggcgaatt ccagcacact ggcggccgtt actagtggat ccgagctcgg taccaagctt 60**
**cgaggcaggt ggacagcttg gttctaatga agttaaccct gtcgttctgc gacatctggg 120**
**cccggaaagc gtttaactga tggatggaac agtccttctg c 161**

<210> 138
<211> 25
<212> DNA
<213> Artificial Sequence

<220>
<223> CFTR Target Sequence 2

<400> 138
gaagttaacc ctgtcgttct gcgac 25

<210> 139
<211> 34
<212> DNA
<213> Artificial Sequence

<220>
<223> CFTR Signaling probe 2

<400> 139
gcgagtcgca gaacgacagg gttaacttcc tcgc 34